# EUROPEAN PATENT SPECIFICATION

(11) **EP 2 340 021 B1**
(45) Date of publication and mention of the grant of the patent: **07.11.2012**
(21) Application number: 09782745.5
(22) Date of filing: 08.09.2009
(51) Int. Cl.: C07D 491/107, C07D 405/14, C07D 207/16, C07D 401/06, C07D 401/12, C07D 403/06, C07D 403/12, C07D 405/06, C07D 405/12, C07D 413/12, C07D 409/04, C07D 409/12, C07D 417/12, C07D 401/14, A61K 31/40, A61K 31/4025, A61P 35/00

(54) **SUBSTITUTED PYRROLIDINE-2-CARBOXAMIDES**
SUBSTITUIERTE PYRROLIDIN-2-CARBOXYLAMIDE
PYRROLIDINE-2-CARBOXAMIDES SUBSTITUÉS

(30) Priority: 18.09.2008 US 97884 P; 15.07.2009 US 225633 P
(43) Date of publication of application: 06.07.2011
(73) Proprietor: F. Hoffmann-La Roche AG, 4070 Basel (CH)
(72) Inventor: DING, Qingjie, Bridgewater, NJ 08807 (US); JIANG, Nan, Fairfield, NJ 07004 (US); LIU, Jin-Jun, Warren, NJ 07059 (US); ROSS, Tina, Morgan, Royersford, PA 19468 (US); ZHANG, Jing, Parsippany, NJ 07054 (US); ZHANG, Zhuming, Hillsborough, NJ 08844 (US)
(74) Representative: Beyermann, Jochen Carl
(86) International application number: PCT/EP2009/061610
(87) International publication number: WO 2010/031713

(56) References cited:
- WO-A1-2008/005268
- WO-A2-2006/091646

## Description

p53 is a tumor suppresser protein that plays a central role in protection against development of cancer. It guards cellular integrity and prevents the propagation of permanently damaged clones of cells by the induction of growth arrest or apoptosis. At the molecular level, p53 is a transcription factor that can activate a panel of genes implicated in the regulation of cell cycle and apoptosis. p53 is a potent cell cycle inhibitor which is tightly regulated by MDM2 at the cellular level. MDM2 and p53 form a feedback control loop. MDM2 can bind p53 and inhibit its ability to transactivate p53-regulated genes. In addition, MDM2 mediates the ubiquitin-dependent degradation of p53. p53 can activate the expression of the MDM2 gene, thus raising the cellular level of MDM2 protein. This feedback control loop insures that both MDM2 and p53 are kept at a low level in normal proliferating cells. MDM2 is also a cofactor for E2F, which plays a central role in cell cycle regulation.

The ratio of MDM2 to p53 (E2F) is dysregulated in many cancers. Frequently occurring molecular defects in the p16INK4/p19ARF locus, for instance, have been shown to affect MDM2 protein degradation. Inhibition of MDM2-p53 interaction in tumor cells with wild-type p53 should lead to accumulation of p53, cell cycle arrest and/or apoptosis. MDM2 antagonists, therefore, can offer a novel approach to cancer therapy as single agents or in combination with a broad spectrum of other antitumor therapies. The feasibility of this strategy has been shown by the use of different macromolecular tools for inhibition of MDM2-p53 interaction (e.g. antibodies, antisense oligonucleotides, peptides). MDM2 also binds E2F through a conserved binding region as p53 and activates E2F-dependent transcription of cyclin A, suggesting that MDM2 antagonists might have effects in p53 mutant cells.

WO 2006/091646 and WO 2008/005268 disclose structurally different heterocyclic compounds as inhibitors of the MDM2-p53 interaction.

The present invention provides compounds of the formula 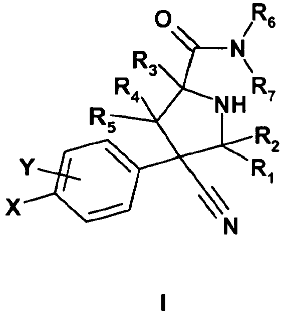 wherein
X is selected from the group consisting ofH, F, Cl, Br, I, cyano, nitro, ethynyl, cyclopropyl, methyl, ethyl, isopropyl, vinyl and methoxy;
Y is one to four group(s) independently selected from the group consisting of H, F, Cl, Br, I, CN, OH, nitro, lower alkyl, cycloalkyl, lower alkoxy, lower alkenyl, cycloalkenyl, lower alkynyl, aryl, heteroaryl, heterocycle, COOR', OCOR', CONR'R", NR'COR", NR"SO₂R', SO₂NR'R" and NR'R", wherein
R' and R" is independently selected from H, substituted or unsubstituted lower alkyl, substituted or unsubstituted lower cycloalkyl, substituted or unsubstituted lower alkenyl, substituted or unsubstituted lower cycloalkenyl, substituted or unsubstituted aryl, substituted or unsubstituted heteroaryl or substituted or unsubstituted heterocycle; and in the case of R' and R" may independently link to form a cyclic structure selected from substituted or unsubstituted cycloalkyl, substituted or unsubstituted cycloalkenyl, substituted or unsubstituted heteroaryl or substituted or unsubstituted heterocycle,
one of R₁ and R₂ is selected from the group consisting of lower alkyl, substituted lower alkyl, lower alkenyl, substituted lower alkenyl, aryl, substituted aryl, heteroaryl, substituted heteroaryl, heterocycle, substituted heterocycle, cycloalkyl, substituted cycloalkyl, cycloalkenyl and substituted cycloalkenyl and the other is hydrogen or lower alkyl;
R₃ is H or lower alkyl;
one of R₄ and R₅ is selected from the group consisting of lower alkyl, substituted lower alkyl, lower alkenyl, substituted lower alkenyl, aryl, substituted aryl, heteroaryl, substituted heteroaryl, heterocycle, substituted heterocycle, cycloalkyl, substituted cycloalkyl, cycloalkenyl, and substituted cycloalkenyl and the other is hydrogen;
R₆ and R₇ are selected from the group consisting of (CH₂)ₙ-R', (CH₂)ₙ-NR'R", (CH₂)ₙ-NR'COR", (CH₂)ₙ-NR'SO₂R", (CH₂)ₙ-COOH, (CH₂)ₙ-COOR', (CH₂)ₙ-CONR'R", (CH₂)ₙ-OR', (CH₂)ₙ-SR', (CH₂)ₙ-SOR', (CH₂)ₙ-SO₂R', (CH₂)ₙ-COR', (CH₂)ₙ-SO₃H, (CH₂)ₙ-SONR'R", (CH₂)ₙ-SO₂NR'R", (CH₂CH₂O)ₘ-(CH₂)ₙ-R', (CH₂CH₂O)ₘ-(CH₂)ₙ-OH, (CH₂CH₂O)ₘ-(CH₂)ₙ-OR', (CH₂CH₂O)ₘ-(CH₂)ₙ-NR'R", (CH₂CH₂O)ₘ-(CH₂)ₙ-NR'COR", (CH₂CH₂O)ₘ-(CH₂)ₙ-NR'SO₂R", (CH₂CH₂O)ₙ-(CH₂)ₙ-COOH, (CH₂CH₂O)ₘ-(CH₂)ₙ-COOR', (CH₂CH₂O)ₘ-(CH₂)ₙ-CONR'R", (CH₂CH₂O)ₘ-(CH₂)ₙ-SO₂R', (CH₂CH₂O)ₘ-(CH₂)ₙ-COR', (CH₂CH₂O)ₘ-(CH₂)ₙ-SONR'R", (CH₂CH₂O)ₘ-(CH₂)ₙ-SO₂NR'R", (CH₂)ₚ-(CH₂CH₂O)ₘ-(CH₂)ₙ-R', (CH₂)ₚ-(CH₂CH₂O)ₘ-(CH₂)ₙ-OH, (CH₂)ₚ-(CH₂CH₂O)ₘ-(CH₂)ₙ-OR', (CH₂)ₚ-(CH₂CH₂O)ₘ-(CH₂)ₙ-NR'R", (CH₂)ₚ-(CH₂CH₂O)ₘ-(CH₂)ₙ-NR'COR", (CH₂)ₚ-(CH₂CH₂O)ₘ-(CH₂)ₙ-NR'SO₂R", (CH₂)ₚ-(CH₂CH₂O)ₘ-(CH₂)ₙ-COOH, (CH₂)ₚ-(CH₂CH₂O)ₘ-(CH₂)ₙ-COOR', (CH₂)ₚ-(CH₂CH₂O)ₘ-(CH₂)ₙ-CONR'R", (CH₂)ₚ-(CH₂CH₂O)ₘ-(CH₂)ₙ-SO₂R', (CH₂)ₚ-(CH₂CH₂O)ₘ-(CH₂)ₙ-COR', (CH₂)ₚ-(CH₂CH₂O)ₘ-(CH₂)ₙ-SONR'R", (CH₂)ₚ-(CH₂CH₂O)ₘ-(CH₂)ₙ-SO₂NR'R", -COR', -SOR' and SO₂R' wherein R' and R" are the same definitions as above;
m, n and p are independently 0 to -6;
and the pharmaceutically acceptable salts and esters thereof.

Preferred are compounds of formula I having a stereochemical structure as shown as formula II 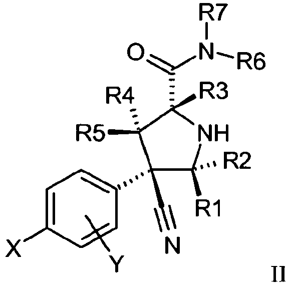 wherein
X is selected from the group consisting ofH, F, Cl, Br, I, cyano, nitro, ethynyl, cyclopropyl, methyl, ethyl, isopropyl, vinyl and methoxy;
Y is one to four group(s) independently selected from the group consisting ofH, F, Cl, Br, I, CN, OH, nitro, lower alkyl, cycloalkyl, lower alkoxy, lower alkenyl, cycloalkenyl, lower alkynyl, aryl, heteroaryl, heterocycle, COOR', OCOR', CONR'R", NR'COR", NR"SO₂R', SO₂NR'R" and NR'R" wherein
R' and R" are independently selected from H or substituted or unsubstituted lower alkyl, substituted or unsubstituted lower cycloalkyl, substituted or unsubstituted lower alkenyl, substituted or unsubstituted lower cycloalkenyl, substituted or unsubstituted aryl, substituted or unsubstituted heteroaryl or substituted or unsubstituted heterocycle, and wherein R' and R" may independently link to form a cyclic structure selected from substituted or unsubstituted cycloalkyl, substituted or unsubstituted cycloalkenyl, substituted or unsubstituted heteroaryl or substituted or unsubstituted heterocycle;
R₁ is selected from the group consisting of lower alkyl, substituted lower alkyl, lower alkenyl, substituted lower alkenyl, aryl, substituted aryl, heteroaryl, substituted heteroaryl, heterocycle, substituted heterocycle, cycloalkyl, substituted cycloalkyl, cycloalkenyl and substituted cycloalkenyl;
R₂ is hydrogen or lower alkyl;
R₃ is H or lower alkyl;
R₅ is selected from the group consisting of lower alkyl, substituted lower alkyl, lower alkenyl, substituted lower alkenyl, aryl, substituted aryl, heteroaryl, substituted heteroaryl, heterocycle, substituted heterocycle, cycloalkyl, substituted cycloalkyl, cycloalkenyl and substituted cycloalkenyl;
R₄ is hydrogen;
R₆ and R₇ are selected from the group consisting of (CH₂)ₙ-R', (CH₂)ₙ-NR'R", (CH₂)ₙ-NR'COR", (CH₂)ₙ-NR'SO₂R", (CH₂)ₙ-COOH, (CH₂)ₙ-COOR', (CH₂)ₙ-CONR'R", (CH₂)ₙ-OR', (CH₂)ₙ-SR', (CH₂)ₙ-SOR', (CH₂)ₙ-SO₂R', (CH₂)ₙ-COR', (CH₂)ₙ-SO₃H, (CH₂)ₙ-SONR'R", (CH₂)ₙ-SO₂NR'R", (CH₂CH₂O)ₘ-(CH₂)ₙ-R', (CH₂CH₂O)ₘ-(CH₂)ₙ-OH, (CH₂CH₂O)ₘ-(CH₂)ₙ-OR', (CH₂CH₂O)ₘ-(CH₂)ₙ-NR'R", (CH₂CH₂O)ₘ-(CH₂)ₙ-NR'COR", (CH₂CH₂O)ₘ-(CH₂)ₙ-NR'SO₂R", (CH₂CH₂O)ₘ-(CH₂)ₙ-COOH, (CH₂CH₂O)ₘ-(CH₂)ₙ-COOR', (CH₂CH₂O)ₘ-(CH₂)ₙ-CONR'R", (CH₂CH₂O)ₘ-(CH₂)ₙ-SO₂R', (CH₂CH₂O)ₘ-(CH₂)ₙ-COR', (CH₂CH₂O)ₘ-(CH₂)ₙ-SONR'R", (CH₂CH₂O)ₘ-(CH₂)ₙ-SO₂NR'R", (CH₂)ₚ-(CH₂CH₂O)ₘ-(CH₂)ₙ-R', (CH₂)ₚ-(CH₂CH₂O)ₘ-(CH₂)ₙ-OH, (CH₂)ₚ-(CH₂CH₂O)ₘ-(CH₂)ₙ-OR', (CH₂)ₚ-(CH₂CH₂O)ₘ-(CH₂)ₙ-NR'R", (CH₂)ₚ-(CH₂CH₂O)ₘ-(CH₂)ₙ-NR'COR", (CH₂)ₚ-(CH₂CH₂O)ₘ-(CH₂)ₙ-NR'SO₂R", (CH₂)ₚ-(CH₂CH₂O)ₘ-(CH₂)ₙ-COOH, (CH₂)ₚ-(CH₂CH₂O)ₘ-(CH₂)ₙ-COOR', (CH₂)ₚ-(CH₂CH₂O)ₘ-(CH₂)ₙ-CONR'R", (CH₂)ₚ-(CH₂CH₂O)ₘ-(CH₂)ₙ-SO₂R', (CH₂)ₚ-(CH₂CH₂O)ₘ-(CH₂)ₙ-COR', (CH₂)ₚ-(CH₂CH₂O)ₘ-(CH₂)ₙ-SONR'R", (CH₂')ₚ-(CH₂CH₂O)ₘ-(CH₂)ₙ-SO₂NR'R", -COR', -SOR' and SO₂R' wherein R' and R" are the same definitions as above;
m, n, and p are independently 0 to-6; and
the pharmaceutically acceptable salts and esters thereof.

Especially preferred are compounds of formula II wherein
X is F, Cl or Br;
Y is one to two group(s) independently selected from the group consisting of H, F, Cl, Br, I, CN, OH, nitro, lower alkyl, cycloalkyl, lower alkoxy, lower alkenyl, lower cycloalkenyl and lower alkynyl;
R₁ is selected from the group consisting of lower alkyl, substituted lower alkyl, lower alkenyl, substituted lower alkenyl, aryl, substituted aryl, heteroaryl, substituted heteroaryl, heterocycle, substituted heterocycle, cycloalkyl, substituted cycloalkyl, cycloalkenyl and substituted cycloalkenyl;
R₂ is hydrogen;
R₃ is H;
R₅ is selected from the group consisting of aryl, substituted aryl, heteroaryl and substituted heteroaryl;
R₄ is hydrogen;
R₆ and R₇ are selected from the group consisting of (CH₂)ₙ-R', (CH₂)ₙ-NR'R", (CH₂)ₙ-NR'COR", (CH₂)ₙ-NR'SO₂R", (CH₂)ₙ-COOH, (CH₂)ₙ-COOR', (CH₂)ₙ-CONR'R", -(CH₂)ₙ-OR', (CH₂)ₙ-SR', (CH₂)ₙ-SOR', (CH₂)ₙ-SO₂R', (CH₂)ₙ-COR', (CH₂)ₙ-SO₃H, (CH₂)ₙ-SONR'R", (CH₂)ₙ-SO₂NR'R", (CH₂CH₂O)ₘ-(CH₂)ₙ-R', (CH₂CH₂O)ₘ-(CH₂)ₙ-OH, (CH₂CH₂O)ₘ-(CH₂)ₙ-OR', (CH₂CH₂O)ₘ-(CH₂)ₙ-NR'R", (CH₂CH₂O)ₘ-(CH₂)ₙ-NR'COR", (CH₂CH₂O)ₘ-(CH₂)ₙ-NR'SO₂R", (CH₂CH₂O)ₘ-(CH₂)ₙ-COOH, (CH₂CH₂O)ₘ-(CH₂)ₙ-COOR', (CH₂CH₂O)ₘ-(CH₂)ₙ-CONR'R", (CH₂CH₂O)ₘ-(CH₂)ₙ-SO₂R', (CH₂CH₂O)ₘ-(CH₂)ₙ-COR', (CH₂CH₂O)ₘ-(CH₂)ₙ-SONR'R", (CH₂CH₂O)ₘ-(CH₂)ₙ-SO₂NR'R", (CH₂)ₚ-(CH₂CH₂O)ₘ-(CH₂)ₙ-R', (CH₂)ₚ-(CH₂CH₂O)ₘ-(CH₂)ₙ-OH, (CH₂)ₚ-(CH₂CH₂O)ₘ-(CH₂)ₙ-OR', (CH₂)ₚ-(CH₂CH₂O)ₘ-(CH₂)ₙ-NR'R", (CH₂)ₚ-(CH₂CH₂O)ₘ-(CH₂)ₙ-NR'COR", (CH₂)ₚ-(CH₂CH₂O)ₘ-(CH₂)ₙ-NR'SO₂R", (CH₂)ₚ-(CH₂CH₂O)ₘ-(CH₂)ₙ-COOH, (CH₂)ₚ-(CH₂CH₂O)ₘ-(CH₂)ₙ-COOR', (CH₂)ₚ-(CH₂CH₂O)ₘ-(CH₂)ₙ-CONR'R", (CH₂)ₚ-(CH₂CH₂O)ₘ-(CH₂)ₙ-SO₂R', (CH₂)ₚ-(CH₂CH₂O)ₘ-(CH₂)ₙ-COR', (CH₂)ₚ-(CH₂CH₂O)ₘ-(CH₂)ₙ-SONR'R", (CH₂)ₚ-(CH₂CH₂O)ₘ-(CH₂)ₙ-SO₂NR'R", -COR', -SOR' and SO₂R' wherein
R' and R" are independently selected from H, lower alkyl, substituted lower alkyl, lower cycloalkyl, substituted lower cycloalkyl, lower alkenyl, substituted lower alkenyl, lower cycloalkenyl, substituted lower cycloalkenyl, aryl, substituted aryl, heteroaryl, substituted heteroaryl, heterocycle, or substituted heterocycle, and wherein R' and R" may also independently link to form a cyclic structure selected from substituted or unsubstituted cycloalkyl, substituted or unsubstituted cycloalkenyl, substituted or unsubstituted heteroaryl or substituted or unsubstituted heterocycle;
m, n and p are independently 0 to 6; and
the pharmaceutically acceptable salts and esters thereof.

Further preferred are compounds of formula II wherein
X is F, Cl or Br;
Y is a mono-substituting group selected from H or F; and
R₁ is selected from the group consisting of lower alkyl, substituted lower alkyl, lower alkenyl, substituted lower alkenyl, heterocycle, substituted heterocycle, cycloalkyl, substituted cycloalkyl, cycloalkenyl and substituted cycloalkenyl.

Further preferred R₁ is a substituted lower alkyl selected from 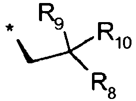 where R₈, R₉ are both methyl, or linked to form a cyclopropyl, cyclobutyl, cyclopentyl or acyclohexyl group;
R₁₀ is (CH₂)ₘ-R₁₁;
m is 0, 1 or 2;
R₁₁ is selected from hydrogen, hydroxyl, lower alkyl, lower alkoxy, aryl, substituted aryl, heteroaryl, substituted heteroaryl, heterocycle or substituted heterocycle,
R₂ is H;
R₃ is H;
R₅ is a substituted phenyl selected from: 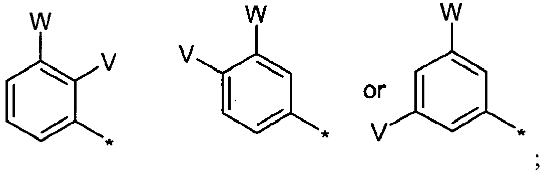 W is F, Cl or Br;
V is H or F;
R₄ is hydrogen;
one of R₆ and R₇ is hydrogen and the other is (CH₂)ₙ-R';
n is 0 or I and
R' is selected from aryl, substituted aryl, heteroaryl, substituted heteroaryl, heterocycle or substituted heterocycle.

Especially preferred are compounds selected from the group consisting of
rac-(2R,3R,4R,5S)-3-(3-chloro-phenyl)4-(4-chloro-phenyl)-4-cyano-5-(2,2-dimethyl-propyl)-pyrrolidine-2-carboxylic acid (2-morpholin-4-yl-ethyl)-amide,
(2R,3R,4R,5S)-3-(3-chloro-phenyl)-4-(4-chloro-phenyl)-4-cyano-5-(2,2-dimethyl-propyl)-pyrrolidine-2-carboxylic acid (2-morpholin-4-yl-ethyl)-amide,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid dimethylamide,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid ((S)-3,4-dihydroxy-butyl)-amide,
(2R,3R,4R,5S)-3-(3-chloro-phenyl)-4-(4-chloro-phenyl)-4-cyano-5-(2,2-dimethyl-propyl)-pyrrolidine-2-carboxylic acid ((S)-3,4-dihydroxy-butyl)-amide,
rac-(2S,3R,4R,5R)-4-(3-chloro-phenyl)-3-(4-chloro-phenyl)-2-(2,2-dimethyl-propyl)-5-[4-(2-morpholin-4-yl-2-oxo-ethyl)-piperazine-1-carbonyl]-pyrrolidine-3-carbonitrile,
rac-(25,3R,4R,5R)-4-(3-chloro-phenyl)-3-(4-chloro-phenyl)-2-(2,2-dimethyl-propyl)-5-[4-(2-oxo-2-pyrrolidin-1-yl-ethyl)-piperazine-1-carbonyl]-pyrrolidine-3-carbonitrile,
rac-(25,3R4R,5R)-4-(3-chloro-phenyl)-3-(4-chloro-phenyl)-2-(2,2-dimethyl-propyl)-5-[4-(2-hydroxy-ethyl)-piperazine-1-carbonyl]-pyrrolidine-3-carbonitrile,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid (4-hydroxy-butyl)-amide,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid (2-pyrrolidin-1-yl-ethyl)-amide,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid (2-piperazin-1-yl-ethyl)-amide,
(S)-2-{[(2R,3R,4R,5S)-3-(3-chloro-phenyl)-4-(4-chloro-phenyl)-4-cyano-5-(2,2-dimethylpropyl)-pyrrolidine-2-carbonyl]-amino}-3-methyl-butyric acid methyl ester,
(S)-2-{[(2R,3R,4R,5S)-3-(3-chloro-phenyl)-4-(4-chloro-phenyl)-4-cyano-5-(2,2-dimethylpropyl)-pyrrolidine-2-carbonyl]-amino}-3-methyl-butyric acid,
rac-(2R,3R,4R,5S)-3-(3-chloro-phenyl)4-(4-chloro-phenyl)-4-cyano-5-(2,2-dimethyl-propyl)-pyrrolidine-2-carboxylic acid (1-hydroxymethyl-cyclopropylmethyl)-amide,
rac-(2R,3R,4R,5S)-3-(3-Chloro-phenyl)-4-(4-chloro-phenyl)-4-cyano-5-(2,2-dimethyl-propyl)-pyrrolidine-2-carboxylic acid (1-hydroxymethyl-cyclobutylmethyl)-amide,
rac-(2R,3R,4R,5S)-3-(3-chloro-phenyl)-4-(4-chloro-phenyl)-4-cyano-5-(2,2-dimethyl-propyl) pyrrolidine-2-carboxylic acid (3,3-dimethyl-butyl)-amide,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid (2,2-dimethyl-propyl)-amide,
(2S,3R,4R,5R)-4-(3-chloro-phenyl)-3-(4-chloro-phenyl)-2-(2,2-dimethyl-propyl)-5-((S)-2-hydroxymethyl-pyrrolidine-1-carbonyl) pyrrolidine-3-carbonitrile,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid 2-(3,4-dimethoxy-phenyl)ethyl amide,
(2S,3S,4S,5R)-3-(3-chloro-phenyl)-4-(4-chloro-phenyl)-4-cyano-5-(2,2-dimethyl-propyl)-pyrrolidine-2-carboxylic acid ((R)-1-hydroxymethyl-3-methyl-butyl)-amide,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid (3-hydroxy-propyl)-amide,
rac-(2R,3S,4R,5S)-3-(3-chloro-2-fluoro-phenyl)-4-(4-chloro-phenyl)-4-cyano-5-(2,2-dimethylpropyl)-pyrrolidine-2-carboxylic acid [2-(cis-2,6-dimethyl-morpholin-4-yl)-ethyl]-amide,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid (2-cyclopropyl-ethyl)-amide,
rac-(3-{[(2R,3R,4R,5S)-3-(3-chloro-phenyl)-4-(4-chloro-phenyl)-4-cyano-5-(2,2-dimethylpropyl)-pyrrolidine-2-carbonyl]-amino}-propyl)-carbamic acid tert-butyl ester,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid (3-amino-propyl)-amide,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid [3-(1-acetyl-piperidin-4-ylamino)-propyl]-amide,
rac-(2R,3R,4R,5S)-3-(3-chloro-phenyl)-4-(4-chloro-phenyl)-4-cyano-5-isobutyl-pyrrolidine-2-carboxylic acid (3-hydroxy-propyl)-amide,
rac-(2R,3R,4R,5R)-5-(3-chloro-phenyl)-4-(4-chloro-phenyl)-4-cyano-3-(2,2-dimethyl-propyl)-pyrrolidine-2-carboxylic acid (3-hydroxy-propyl)-amide,
rac-(2R,3R,4R,5S)-3-(3-chloro-phenyl)-4-(4-chloro-phenyl)-4-cyano-5-(2,2-dimethyl-propyl)-2-methyl-pyrrolidine-2-carboxylic acid (3-hydroxy-propyl)-amide,
rac-(2R,3S,4R,5S)-3-(3-chloro-2-fluoro-phenyl)-4-(4-chloro-phenyl)-4-cyano-5-cyclopentylmethyl-pyrro lidine-2-carboxylic acid ((S)-3,4-dihydroxy-butyl)-amide,
rac-(2R,3S,4R,5S)-3-(3-chloro-2-fluoro-phenyl)-4-(4-chloro-phenyl)-4-cyano-5-(2,2-dimethylpropyl)-pyrrolidine-2-carboxylic acid (2-hydroxy-1,1-dimethyl-ethyl)-amide,
rac-(2R,3S,4R,5S)-3-(3-chloro-2-fluoro-phenyl)-4-(4-chloro-phenyl)-4-cyano-5-(2,2-dimethylpropyl)-pyrrolidine-2-carboxylic acid (3-hydroxy-2,2-dimethyl-propyl)-amide,
rac-(2R,3S,4R,5S)-3-(3-chloro-2-fluoro-phenyl)-4-(4-chloro-phenyl)-4-cyano-5-(2,2-dimethylpropyl)-pyrrolidine-2-carboxylic acid [2-(2-hydroxy-ethoxy)-ethyl]-amide,
rac-(2R,3S,4R,5S)-3-(3-chloro-2-fluoro-phenyl)-4-(4-chloro-phenyl)-4-cyano-5-(2,2-dimethylpropyl)-pyrrolidine-2-carboxylic acid (2-acetylamino-ethyl)-amide,
rac-(2R,3S,4R,5S)-3-(3-chloro-2-fluoro-phenyl)-4-(4-chloro-phenyl)-4-cyano-5-(2,2-dimethylpropyl)-pyrrolidine-2-carboxylic acid (3-imidazol-1-yl-propyl)-amid,
rac-(2R,3S,4R,5S)-3-(3-chloro-2-fluoro-phenyl)-4-(4-chloro-phenyl)-4-cyano-5-(2,2-dimethylpropyl)-pyrrolidine-2-carboxylic acid ((R)-4-hydroxy-3-methyl-butyl)-amide,
rac-(2R,3S,4R,5S)-3-(3-chloro-2-fluoro-phenyl)-4-(4-chloro-phenyl)-4-cyano-5-(2,2-dimethylpropyl)-pyrro lid ine-2-carboxylic acid cyclopropylmethoxy-amide,
rac-(2R,3R,4R,5S)-3,5-bis-(3-chloro-phenyl)-4-(4-chloro-phenyl)-4-cyano-pyrrolidine-2-carboxylic acid [2-((S)-2,2-dimethyl-[1,3]dioxolan-4-yl)-ethyl]-amide,
rac-(2R,3R,4R,5S)-3,5-bis-(3-chloro-phenyl)-4-(4-chloro-phenyl)-4-cyano-pyrrolidine-2-carboxylic acid ((S)-3,4-dihydroxy-butyl)-amide,
rac-(2R,3R,4R,5S)-3-(3-chloro-phenyl)-4-(4-chloro-phenyl)-4-cyano-5-(1-ethyl-propyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-phenyl)-4-cyano-5-(2,2-dimethylpropyl)-pyrrolidine-2-carboxylic acid ((S)-3,4-dihydroxy-butyl)-amide,
(2R,3S,4R,5S)-3-(3-chloro-2-fluoro-phenyl)-4-(4-chloro-phenyl)-4-cyano-5-(2,2-dimethylpropyl)-pyrrolidine-2-carboxylic acid ((S)-3,4-dihydroxy-butyl)-amide,
rac-(2R,3R,4R,5S)-3-(3-chloro-phenyl)-4-(4-chloro-phenyl)-4-cyano-5-isobutyl-pyrrolidine-2-carboxylic acid ((S)-3,4-dihydroxy-butyl)-amide,
rac-(2R,3R,4R,5R)-3-(3-chloro-phenyl)-4-(4-chloro-phenyl)-4-cyano-5-isobutyl-pyrrolidine-2-carboxylic acid ((S)-3,4-dihydroxy-butyl)-amide,
rac-(2R,3R,4R,5R)-3-(3-chloro-phenyl)-4-(4-chloro-phenyl)-4-cyano-5-(1-ethyl-propyl)-pyrrolidine-2-carboxylic acid ((S)-3,4-dihydroxy-butyl)-amide,
rac-(2R,3R,4R)-3-(3-chloro-phenyl)-4-(4-chloro-phenyl)-4-cyano-5,5-diethyl-pyrrolidine-2-carboxylic acid ((S)-3,4-dihydroxy-butyl)-amide,
rac-(2R,3R,4R,5S)-3-(3-chloro-phenyl)-4-(4-chloro-phenyl)-4-cyano-5-isopropyl-pyrrolidine-2-carboxylic acid ((S)-3,4-dihydroxy-butyl)-amide,
rac-(2R,3R,4R,5S)-3-(3-chloro-phenyl)-4-(4-chloro-phenyl)-4-cyano-5-cyclohexyl-pyrrolidine-2-carboxylic acid ((S)-3,4-dihydroxy-butyl)-amide,
rac-(2R,3S,4R,5S)-5-tert-butyl-3-(3-chloro-2-fluoro-phenyl)-4-(4-chloro-phenyl)-4-cyanopyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide,
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide,
rac-(2S,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide,
rac-(2R,3S,4R,5S)-4-(4-bromo-phenyl)-3-(3-chloro-2-fluoro-phenyl)-4-cyano-5-(2,2-dimethylpropyl)-pyrrolidine-2-carboxylic acid ((S)-3,4-dihydroxy-butyl)-amide,
rac-(2R,3S,4R,5S)-3-(3-chloro-2-fluoro-phenyl)-4-cyano-5-(2,2-dimethyl-propyl)-4-(4-fluoro-phenyl)-pyrrolidine-2-carboxylic acid ((S)-3,4-dihydroxy-butyl)-amide
rac-(2R,3S,4R,5S)-3-(3-chloro-2-fluoro-phenyl)-4-cyano-4-(2,4-dichloro-phenyl)-5-(2,2-dimethyl-propyl)-pyrrolidine-2-carboxylic acid ((S)-3,4-dihydroxy-butyl)-amide,
(2R,3S,4R,5S)-3-(3-chloro-2-fluoro-phenyl)-4-cyano-4-(2,4-dichloro-phenyl)-5-(2,2-dimethylpropyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-methyl-phenyl)-4-cyano-5-(2,2-dimethyl-propyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-phenyl)-4-cyano-5-cyclohexylmethyl-pyrrolidine-2-carboxylic acid ((S)-3,4-dihydroxy-butyl)-amide,
rac-(2S,3S,4R,5S)-3-(3-chloro-2-fluoro-phenyl)-4-(4-chloro-phenyl)-4-cyano-5-cyclohexylmethyl-pyrrolidine-2-carboxylic acid ((S)-3,4-dihydroxy-butyl)-amide,
rac-(2R,3S,4R,5S)-4-(4-chloro-2-fluoro-phenyl)-4-cyano-3-(2,3-difluoro-phenyl)-5-(2,2-dimethyl-propyl)-pyrrolidine-2-carboxylic acid ((S)-3,4-dihydroxy-butyl)-amide,
(2R,3S,4R,5S)-4-(4-chloro-2-fluoro-phenyl)-4-cyano-3-(2,3-difluoro-phenyl)-5-(2,2-dimethylpropyl)-pyrrolidine-2-carboxylic acid ((S)-3,4-dihydroxy-butyl)-amide,
rac-(2R,3S,4R,5S)-3-(3-bromo-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide,
(2R,3S,4R,5S)-3-(3-bromo-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide,
rac-(2R,3R,4R,5S)-4-(4-chloro-2-fluoro-phenyl)-3-(3-chloro-phenyl)-4-cyano-5-(2,2-dimethylpropyl)-pyrrolidine-2-carboxylic acid ((S)-3,4-dihydroxy-butyl)-amide
(2R,3R,4R,5S)-4-(4-chloro-2-fluoro-phenyl)-3-(3-chloro-phenyl)-4-cyano-5-(2,2-dimethylpropyl)-pyrrolidine-2-carboxylic acid ((S)-3,4-dihydroxy-butyl)-amide,
rac-(2R,3R,4R,5S)-4-(4-chloro-2-fluoro-phenyl)-4-cyano-5-(2,2-dimethyl-propyl)-3-(3-fluoro-phenyl)-pyrrolidine-2-carboxylic acid ((S)-3,4-dihydroxy-butyl)-amide,
rac-(2R,3R,4R,5S)-3-(3-bromo-phenyl)-4-(4-chloro-2-fluoro-phenyl)-4-cyano-5-(2,2-dimethylpropyl)-pyrrolidine-2-carboxylic acid ((S)-3,4-dihydroxy-butyl)-amide,
rac-(2S,3R,4R,5S)-3-(3-bromo-phenyl)-4-(4-chloro-2-fluoro-phenyl)-4-cyano-5-(2,2-dimethylpropyl)-pyrrolidine-2-carboxylic acid ((S)-3,4-dihydroxy-butyl)-amide.
rac-(2R,3R,4R,5S)-4-(4-chloro-2-fluoro-phenyl)-4-cyano-3-(3,4-dichloro-phenyl)-5-(2,2-dimethyl-propyl)-pyrrolidine-2-carboxylic acid ((S)-3,4-dihydroxy-butyl)-amide,
rac-(2 R,3 R,4R,5 S)-3-(3-chloro-4-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide,
(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide,
rac-(2R,3R,4R,5S)-3-(4-bromo-thiophen-2-yl)-4-(4-chloro-2-fluoro-phenyl)-4-cyano-5-(2,2-dimethyl-propyl)-pyrrolidine-2-carboxylic acid ((S)-3,4-dihydroxy-butyl)-amide,
rac-(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carboxylic acid ((R)-3,4-dihydroxy-butyl)-amide,
(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carboxylic acid ((R)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((R)-3,4-dihydroxy-butyl)-amide;
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((R)-3,4-dihydroxy-butyl)-amide,
rac-(2R,3R,4R,5S)-4-(4-chloro-2-fluoro-phenyl)-3-(3-chloro-phenyl)-4-cyano-5-(2,2-dimethylpropyl)-pyrro lidine-2-carboxylic acid ((R)-3,4-dihydroxy-butyl)-amide,
rac-(2R,3S,4R,5S)-3-(3-chloro-2-fluoro-phenyl)-4-(4-chloro-phenyl)-4-cyano-5-(2,2-dimethylpropyl)-pyrrolidine-2-carboxylic acid ((R)-3,4-dihydroxy-butyl)-amide,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid ((R)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(2,2,3-trimethyl-butyl)-pyrrolidine-2-carboxylic acid ((S)-3,4-dihydroxy-butyl)-amide,
(2R,3S,4R,5S)-3-(3-chloro-2-fluoro-phenyl)-4-(4-chloro-2-fluoro-phenyl)-4-cyano-5-(2,2,3-trimethyl-butyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(2,2,3-trimethyl-butyl)-pyrrolidine-2-carboxylic acid ((R)-3,4-dihydroxy-butyl)-amide,
(2R,3S,4R,5S)-3-(3-chloro-2-fluoro-phenyl)-4-(4-chloro-2-fluoro-phenyl)-4-cyano-5-(2,2,3-trimethyl-butyl)-pyrrolidine-2-carboxylic acid ((R)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(2,2-dimethyl-but-3-enyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(2,2-dimethyl-butyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(2-methyl-2-phenyl-propyl)-pyrrolidine-2-carboxylic acid ((S)-3,4-dihydroxy-butyl)-amide,
rac-(2R,3R,4R,5S)-3-(3-chloro-5-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide,
rac-(2R,3S,4R,5S)-4-(4-chloro-2-fluoro-phenyl)-3-[5-chloro-2-(2-hydroxy-ethoxy)-phenyl]-4-cyano-5-(2,2-dimethyl-propyl)-pyrrolidine-2-carboxylic acid ((S)-3,4-dihydroxy-butyl)-amide,
rac-(2R,3S,4R,5S)-4-(4-chloro-2-fluoro-phenyl)-3-(5-chloro-2-methoxy-phenyl)-4-cyano-5-(2,2-dimethyl-propyl)-pyrrolidine-2-carboxylic acid ((S)-3,4-dihydroxy-butyl)-amide,
rac(2R,3R,4R,5S)-3-(3-chloro-phenyl)-4-(4-chloro-phenyl)-4-cyano-5-(2,2-dimethyl-propyl)-pyrrolidine-2-carboxylic acid (quinolin-3-ylmethyl)-amide,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid 4-hydroxy-benzylamide,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid (2-ethyl-butyl)-amide,
rac-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-piperidine-1-carboxylic acid tert-butyl ester,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid piperidin-4-ylamide trifluoroacetic acid,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-methanesulfonylpiperidin-4-yl)-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-methyl-carbonyl-piperidin-4-yl)-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-benzoyl-piperidin-4-yl)-amide,
rac-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-piperidine-1-carboxylic acid isopropylamide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [1-(2-hydroxy-2-methyl-propyl)-1H-pyrazol-3-yl]-amide,
chiral 2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [1-(2-hydroxy-2-methyl-propyl)-1H-pyrazol-3-yl]-amide,
chiral (2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid [1-(2-hydroxy-2-methyl-propyl)-1H-pyrazol-3-yl]-amide,
rac-(2R,3S,4R,5S)-3-(3-chloro-2-fluoro-phenyl)-4-(4-chloro-2-fluoro-phenyl)-4-cyano-5-(2,2-dimethylpropyl)-pyrrolidine-2-carboxylic acid {1-[2-methyl-2-((R)-1-oxiranylmethoxy)-propyl]-1H-pyrazol-3-yl}-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((*R*)-3-amino-2-hydroxy-propoxy)-2-methyl-propyl]-1H-pyrazol-3-yl}-amide,
rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-{2-[(*R*)-2-hydroxy-3-(3-hydroxypropylamino)-propoxy]-2-methyl-propyl}-1H-pyrazol-3-yl)-amide,
rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-{2-[(*R*)-2-hydroxy-3-(2-hydroxy-1-hydroxymethyl-ethylamino)-propoxy]-2-methyl-propyl}-1H-pyrazol-3-yl)-amide,
rac-(2*R*,3*S*,4*R*,5*S*)-3-(3-chloro-2-fluoro-phenyl)-4-(4-chloro-2-fluoro-phenyl)-4-cyano-5-(2,2-dimethylpropyl)-pyrrolidine-2-carboxylic acid {1-[2-methyl-2-((*S*)-1-oxiranylmethoxy)-propyl]-1H-pyrazol-3-yl}-amide,
rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((*S*)-3-amino-2-hydroxy-propoxy)-2-methyl-propyl]-1H-pyrazol-3-yl}-amide,
rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((*S*)-2,3-dihydroxy-propoxy)-2- methylpropyl]-1H-pyrazol-3-yl}-amide,
of rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((*S*)-3-dimethylamino-2-hydroxy-propoxy)-2-methyl-propyl]-1H-pyrazol-3-yl)-amide,
rac-{(*S*)-3-[2-(3-{[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-amino]-pyrazol-1-yl)-1,1-dimethyl-ethoxy]-2-hydroxy-propylamino) -acetic acid,
rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)pyrrolidine-2-carboxylic acid {1-[2-((*S*)-2-hydroxy-3-methylamino-propoxy)-2-methyl-propyl]-1H-pyrazol-3-yl}-amide,
rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((*R*)-3-dimethylamino-2-hydroxy-propoxy)-2-methyl-propyl]-1H-pyrazol-3-yl)-amide,
rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid [1-((*R*)-2,3-dihydroxy-propyl)-1H-pyrazol-3-yl]-amide,
rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid [1-(2-hydroxy-ethyl)-1H-pyrazol-3-yl]-amide,
rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid ((*S*)-2,3-dihydroxy-propyl)-amide,
rac- (2*S*,3*S*,4*S*,5*R*)-3-(3-chloro-phenyl)-4-(4-chloro-phenyl)-4-cyano-5-(2,2-dimethyl-propyl)-pyrrolidine-2-carboxylic acid ((*S*)-2,3-dihydroxy-propyl)-amide,
rac- (2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid [1-((*R*)-2,2-dimethyl-[1,3]dioxolan-4-ylmethyl)-1H-pyrazol-3-yl]-amide,
rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid 2-trifluoromethyl-benzylamide,
rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid 4-(2-oxo-pyrrolidin-1-yl)-benzylamide;
rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (tetrahydro-pyran-4-ylmethyl)-amide,
rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (3-hydroxy-2-hydroxymethyl-propyl)-amide,
rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid [2-(2-amino-ethoxy)-ethyl]-amide,
rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (3-methanesulfonyl-propyl)-amide,
rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (2-methanesulfonyl-ethyl)-amide,
rac-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-cyclohexylamino-1-carboxylic acid tert-butyl ester,
rac-4-{[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-trans-cyclohexylamine trifluoroacetic acid salt,
rac-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-trans-cyclohexyl-N-methanesulfonamide,
rac-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-trans-cyclohexyl-N-methanesulfonamide,
rac-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-trans-cyclohexyl-(1,1-dioxo)-2-isothiazolidine,
rac-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-trans-cyclohexyl-urea,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid N-[1-(2-hydroxy ethyl)-piperidin-4-yl]amide,
rac-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-piperidine-1-sulfonic acid amide,
rac 3- 4-[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-piperazin-1-yl}-acetic acid,
rac 3-{4-[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-piperazin-1-yl}-N,N-bis-(2-methoxy-ethyl)-acetamide,
rac 3-{4-[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-piperazin-1-yl}-N,N-bis-(2-hydroxy-ethyl)-acetamide,
rac 3-{4-[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-piperazin-1-yl}-N-(3-methoxy-propyl)-acetamide,
rac 2-{4-[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-piperazin-1-yl}-acetamide,
rac (2S,3R,4S,5R)-4-(3-chloro-2-fluoro-phenyl)-3-(4-chloro-2-fluoro-phenyl)-2-(2,2-dimethylpropyl)-5-[4-(2-morpholin-4-yl-2-oxo-ethyl)-piperazine-1-carbonyl]-pyrrolidine-3-carbonitrile,
rac 2-{4-[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-piperazin-1-yl}-N-[2-((S)-2,2-dimethyl-[1,3]dioxolan-4-yl)-ethyl]-acetamide,
rac 2-{4-[(2R,3S,4R,5S)-3-(3-chloro-2-fluoro-phenyl)-4-(4-chloro 2-fluoro-phenyl)-4-cyano-5-(2,2-dimethyl-propyl)-pyrrolidine-2-carbonyl]-piperazin-1-yl}-N-((S)-3,4-dihydroxy-butyl)-acetamide,
rac {1-[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-piperidin-4-yl}-acetic acid methyl ester,
rac {1-[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-piperidin-4-yl}-acetic acid hydrochloride salt, rac 2-{1-[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-piperidin-4-yl}-acetamide,
rac 2-{1-[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-piperidin-4-yl}-N,N-bis-(2-hydroxy-ethyl)-acetamide,
rac 2-{1-[(2R,3S,4R,5S)-3-(3-chloro-2-fluoro-phenyl)-4-(4-chloro 2-fluoro-phenyl)-4-cyano-5-(2,2-dimethyl-propyl)-pyrrolidine-2-carbonyl]-piperidin-4-yl}-N-(2-hydroxy-ethyl)-N-methylacetamide,
rac 2-{1-[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-piperidin-4-yl}-N-(2-hydroxy-propyl)-acetamide,
rac {[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-acetic acid tert-butyl ester,
rac {[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-acetic aid trifluoro acetic acid salt,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid carbamoylmethyl-amide,
{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-acetic aid trifluoro acetic acid salt,
rac 3-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid ethyl ester,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (3-carbamoyl-phenyl)-amide,
rac 3-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid tert-butyl ester,
rac 3-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (3-hydroxymethyl-phenyl)-amide,
rac (3-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-propyl)-carbamic acid tert-butyl ester,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (3-amino-propyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [3-(aminosulfonyl-amino)-propyl)-amide,
rac 2-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid tert-butyl ester,
rac 4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid tert-butyl ester,
rac 4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid ethyl ester,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-carbamoyl-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-carbamoyl-phenyl)-amide,
rac 2-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid ethyl ester,
rac 4- {[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid,
rac 2-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid ,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (3-cyano-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-hydroxy-2-methyl-propyl)-amide,
rac 3-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid 2-hydroxy-2-methyl-propyl ester,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (3-methanesulfonylamino-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (1H-tetrazol-5-ylmethyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (3-ureado-propyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (3-methylsulfanyl-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (3-methanesulfonyl-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (3-methanesulfinyl-phenyl)-amide,
3- {[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid,
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (3-carbamoyl-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [3-(1H-tetrazol-5-yl)-phenyl]-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(1H-tetrazol-5-yl)-phenyl]-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-amino-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-acetylamino-phenyl)-amide,
rac 2-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-thiazole-4-carboxylic acid ethyl este,r
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (1,3-dioxo-2,3-dihydro-1H-isoindol-5-yl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (6-oxo-1,6-dihydro-pyridin-3-yl)-amide,
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (6-oxo-1,6-dihydro-pyridin-3-yl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-methylsulfanyl-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-methanesulfonyl-phenyl)-amide,
4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid tert-butyl ester,
4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid,
4-{[(2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid,
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-carbamoyl-phenyl)-amide,
(2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-carbamoyl-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-methanesulfonylamino-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-trifluoro-methanesulfonylamino-phenyl)-amide,
rac (2R,3R,4R,5S)-4-(4-chloro-2-fluoro-phenyl)-3-(3-chloro-2-methyl-phenyl)-4-cyano-5-(2,2-dimethyl-propyl)-pyrrolidine-2-carboxylic acid [3-(1-methyl-1H-tetrazol-5-yl)-phenyl]-amide,
rac (2R,3R,4R,5S)-4-(4-chloro-2-fluoro-phenyl)-3-(3-chloro-2-methyl-phenyl)-4-cyano-5-(2,2-dimethyl-propyl)-pyrrolidine-2-carboxylic acid [3-(2-methyl-1H-tetrazol-5-yl)-phenyl]-amide,
(2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid (6-oxo-1,6-dihydro-pyridin-3-yl)-amide,
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(1H-tetrazol-5-yl)-phenyl]-amide,
(2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(1H-tetrazol-5-yl)-phenyl]-amide,
rac (2R,3R,4R,5S)-4-(4-chloro-2-fluoro-phenyl)-3-(3-chloro-2-methyl-phenyl)-4-cyano-5-(2,2-dimethyl-propyl)-pyrrolidine-2-carboxylic acid [4-(2-methyl-1H-tetrazol-5-yl)-phenyl]-amide,
rac (2R,3R,4R,5S)-4-(4-chloro-2-fluoro-phenyl)-3-(3-chloro-2-methyl-phenyl)-4-cyano-5-(2,2-dimethyl-propyl)-pyrrolidine-2-carboxylic acid [4-(1-methyl-1H-tetrazol-5-yl)-phenyl]-amide,
rac 5-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-2-fluoro-benzoic acid ethyl ester,
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [3-(1H-tetrazol-5-yl)-phenyl]-amide,
(2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid [3-(1H-tetrazol-5-yl)-phenyl]-amide,
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-carbamoyl-3-chloro-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [3-chloro-4-(1H-tetrazol-5-yl)-phenyl]-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-fluoro-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (3-fluoro-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (3-chloro-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-chloro-phenyl)-amide,
rac 4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-2-fluoro-benzoic acid tert-butyl ester,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-ethylcarbamoyl-3-fluoro-phenyl)-amide,
rac 4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino]-2-fluoro-benzoic acid,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (6-methoxy-pyridin-3-yl)-amide,
rac 3-({[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-methyl)-benzoic acid methyl ester,
rac 4-({[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-methyl)-benzoic acid methyl ester,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-chloro-phenyl)-amide,
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-chloro-phenyl)-amide,
(2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-chloro-phenyl)-amide,
rac 4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-2-methoxy-benzoic acid methyl ester,
rac 3-({[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-methyl)-benzoic acid,
rac 4-({[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-methyl)-benzoic acid,
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-acetylamino-phenyl)-amide,
(2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-acetylamino-phenyl)-amide,
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-methanesulfonyl-phenyl)-amide,
(2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-methanesulfonyl-phenyl)-amide;
rac 4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino]-2-methoxy-benzoic acid ,
rac 5-bromo-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-2-methoxy-benzoic acid methyl ester,
rac 4- {[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-2-methyl-benzoic acid methyl ester,
rac 2-Chloro-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid methyl ester,
rac 4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-2-trifluoromethyl-benzoic acid methyl ester,
rac 4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-2-methyl-benzoic acid,
rac 2-Chloro-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(2H-[1,2,4]triazol-3-yl)-phenyl]-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(5-oxo-2,5-dihydro-1H-[1,2,4]tiazol-3-yl)-phenyl]-amide,
rac-(2R,3S,4R,5S)-3-(5-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(2-cyclopropyl-2-methyl-propyl)-pyrrolidine-2-carboxylic acid ((S)-3,4-dihydroxy-butyl)-amide,
(2R,3S,4R,5S)-3-(3-chloro-2-fluoro-phenyl)-4-(4-chloro-2-fluoro-phenyl)-4-cyano-5-(2-cyclopropyl-2-methyl-propyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-3-methyl-phenyl)-4-cyano-5-(2,2-dimethyl-propyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(tetrahydro-pyran-4-ylmethyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(1-methyl-cyclohexylmethyl)-pyrrolidine-2-carboxylic acid ((S)-3,4-dihydroxy-butyl)-amide,
(2R,3S,4R,5S)-3-(3-chloro-2-fluoro-phenyl)-4-(4-chloro-2-fluoro-phenyl)-4-cyano-5-(1-methylcyclohexylmethyl)-pyrrolidine-2-carboxylic acid ((S)-3,4-dihydroxy-butyl)-amide,
rac-(2R,3S,4R,5S)-5-(2-benzyloxycarbonyl-2-methyl-propyl)-3-(3-chloro-2-fluoro-phenyl)-4-(4-chloro-2-fluoro-phenyl)-4-cyano-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(2-methoxycarbonyl-2-methyl-propyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid ((S)-3,4-dihydroxy-butyl)-amide,
(2R,3S,4R,5S)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(2,2-diethyl-butyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(2-ethyl-2-methyl-butyl)-pyrrolidine-2-carboxylic acid ((S)-3,4-dihydroxy-butyl)-amide,
rac-(2R,3R,4R,5S)-4-(4-chloro-2,6-difluoro-phenyl)-3-(3-chloro-phenyl)-4-cyano-5-(2,2-dimethyl-propyl)-pyrrolidine-2-carboxylic acid ((S)-3,4-dihydroxy-butyl)-amide,
rac-(2R,3R,4R,5S)-4-(4-chloro-2,5-difluoro-phenyl)-3-(3-chloro-phenyl)-4-cyano-5-(2,2-dimethyl-propyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(3-methoxy-2,2-dimethyl-propyl)-pyrrolidine-2-carboxylic acid ((S)-3,4-dihydroxy-butyl)-amide,
(2R,3S,4R,5S)-3-(3-chloro-2-fluoro-phenyl)-4-(4-chloro-2-fluoro-phenyl)-4-cyano-5-(3-methoxy-2,2-dimethyl-propyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(2-ethyl-2-hydroxymethyl-butyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(3-methyl-oxetan-3-ylmethyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(3-ethyl-oxetan-3-ylmethyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(1-hydroxymethyl-cyclopropylmethyl)-pyrrolidine-2-carboxylic acid ((S)-3,4-dihydroxy-butyl)-amide,
(2R,3S,4R,5S)-3-(3-chloro-2-fluoro-phenyl)-4-(4-chloro-2-fluoro-phenyl)-4-cyano-5-(1-hydroxymethyl-cyclopropylmethyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(1-hydroxymethyl-cyclobutylmethyl)-pyrrolidine-2-carboxylic acid ((S)-3,4-dihydroxy-butyl)-amide,
(2R,3S,4R,5S)-3-(3-chloro-2-fluoro-phenyl)-4-(4-chloro-2-fluoro-phenyl)-4-cyano-5-(1-hydroxymethyl-cyclobutylmethyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid ((S)-3,4-dihydroxy-butyl)-amide,
(2R,3S,4R,5S)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(1-hydroxymethyl-cyclohex-3-enylmethyl)-pyrrolidine-2-carboxylic acid ((S)-3,4-dihydroxybutyl)-amide,
rac-(2R,3S,4R,5S)-3-(3-chloro-2-fluoro-phenyl)-4-(4-chloro-2-fluoro-phenyl)-4-cyano-5-(1-hydroxymethyl-cyclohexylmethyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-[4-(2-hydroxy-ethoxy)-2,2-dimethyl-butyl]-pyrrolidine-2-carboxylic acid ((S)-3,4-dihydroxy-butyl)-amide,
rac-(2R,3S,4R,5S)-5-(4-azido-2,2-dimethyl-butyl)-3-(3-chloro-2-fluoro-phenyl)-4-(4-chloro-2-fluoro-phenyl)-4-cyano-pyrrolidine-2-carboxylic acid ((S)-3,4-dihydroxy-butyl)-amide,
rac-(2R,3S,4R,5S)-5-(4-amino-2,2-dimethyl-butyl)-3-(3-chloro-2-fluoro-phenyl)-4-(4-chloro-2-fluoro-phenyl)-4-cyano-pyrrolidine-2-carboxylic acid ((S)-3,4-dihydroxy-butyl)-amide,
rac-(2R,3S,4R,5S)-5-(4-acetylamino-2,2-dimethyl-butyl)-3-(3-chloro-2-fluoro-phenyl)-4-(4-chloro-2-fluoro-phenyl)-4-cyano-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(4-methanesulfonylamino-2,2-dimethyl-butyl)-pyrrolidine-2-carboxylic acid ((S)-3,4-dihydroxybutyl)-amide,
rac-(2R,3S,4R,5S)-5-(4-benzoylamino-2,2-dimethyl-butyl)-3-(3-chloro-2-fluoro-phenyl)-4-(4-chloro-2-fluoro-phenyl)-4-cyano-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-[2-methyl-2-(5-methyl-furan-2-yl)-propyl]-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-[3-(4-methoxy-phenyl)-2,2-dimethyl-propyl]-pyrrolidine-2-carboxylic acid ((S)-3,4-dihydroxy-butyl)-amide,
rac-(2R,3S,4R,5S)-5-[2-(1-benzyl-1,2,3,6-tetrahydro-pyridin-4-yl)-2-methyl-propyl]-3-(3-chloro-2-fluoro-phenyl)-4-(4-chloro-2-fluoro-phenyl)-4-cyano-pyrrolidine-2-carboxylic acid ((S)-3,4-dihydroxy-butyl)-amide,
rac-(2R,3S,4R,5S)-5-[2-(1-benzyl-piperidin-4-yl)-2-methyl-propyl]-3-(3-chloro-2-fluoro-phenyl)-4-(4-chloro-2-fluoro-phenyl)-4-cyano-pyrrolidine-2-carboxylic acid ((S)-3,4-dihydroxybutyl)-amide,
rac-(2R,3S,4R,5S)-3-(3-chloro-2-fluoro-phenyl)-4-(4-chloro-2-fluoro-phenyl)-4-cyano-5-[2-(3,6-dihydro-2H-pyran-4-yl)-2-methyl-propyl]-pyrrolidine-2-carboxylic acid ((S)-3,4-dihydroxybutyl)-amide,
rac-(2R,3S,4R,5S)-3-(3-chloro-2-fluoro-phenyl)-4-(4-chloro-2-fluoro-phenyl)-4-cyano-5-[2-methyl-2-(tetrahydro-pyran-4-yl)-propyl]-pyrrolidine-2-carboxylic acid ((S)-3,4-dihydroxybutyl)-amide,
rac-(2R,3S,4R,5S)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid ((S)-3,4-dihydroxy-4-methylpentyl)-amide,
(2R,3S,4R,5S)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid ((S)-3,4-dihydroxy-4-methylpentyl)-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-4-methyl-pentyl)-amide,
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-4-methyl-pentyl)-amide, rac-(2R,3S,4R,5S)-3-(3-chloro-2-fluoro-phenyl)-4-(4-chloro-2-fluoro-phenyl)-4-cyano-5-(1-methyl-cyclohexylmethyl)-pyrrolidine-2-carboxylic acid ((S)-3,4-dihydroxy-4-methyl-pentyl)-amide,
(2R,3S,4R,5S)-3-(3-chloro-2-fluoro-phenyl)-4-(4-chloro-2-fluoro-phenyl)-4-cyano-5-(1-methylcyclohexylmethyl)-pyrrolidine-2-carboxylic acid ((S)-3,4-dihydroxy-4-methyl-pentyl)-amide,
rac-(2S,3R,4S,5R)-4-(3-cloro-2-fluoro-phenyl)-3-(4-chloro-2-fluoro-phenyl)-2-(2,2-dimethylpropyl)-5-(3-hydroxy-azetidine-1-carbonyl)-pyrrolidine-3-carbonitrile,
rac-(2R,3S,4R,5S)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid [1-(2-hydroxy-2-methyl-propyl)-1H-pyrazol-3-yl]-amide,
(2R,3S,4R,5S)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid [1-(2-hydroxy-2-methyl-propyl)-1H-pyrazol-3-yl]-amide,
rac-(2R,3S,4R,5S)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid [1-(2-hydroxy-2-methyl-propyl)-1H-pyrazol-3-yl]-amide,
(2R,35,4R,55)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid [1-(2-hydroxy-2-methyl-propyl)-1H-pyrazol-3-yl]-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid amide,
rac-6-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-nicotinic acid methyl ester,
of rac-6-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-N-(2-hydroxy-1,1-dimethyl-ethyl)-nicotinamide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (6-acetylamino-pyridin-3-yl)-amide,
rac-(2R,3S.,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-methyl-2-oxo-1,2-dihydro-pyridin-4-yl)-amide,
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-methyl-2-oxo-1,2-dihydro-pyridin-4-yl)-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [5-((S)-1,2-dihydroxy-ethyl)-pyrazin-2-yl]-amide,
rac-(2R,3S,4R,5S)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid (1-methyl-2-oxo-1,2-dihydro-pyridin-4-yl)-amide,
rac-5- {[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-furan-2-carboxylic acid methyl ester,
rac-5-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-furan-2-carboxylic acid,
rac-5- {[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-furan-2-carboxylic acid amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (6-chloro-pyridazin-3-yl)-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-methyl-pyridin-3-yl)-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(2-hydroxy-ethoxy)-phenyl]-amide,
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(2-hydroxy-ethoxy)-phenyl]-amide,
rac-5- {[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-thiophene-2-carboxylic acid methyl ester,
rac-5-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-thiophene-2-carboxylic acid,
5-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-thiophene-2-carboxylic acid
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-methoxy-pyridin-4-yl)-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-hydroxy-pyridin-4-yl)-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-acetyl-phenyl)-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(2-bromo-acetyl)-phenyl]-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(2- dimethylamino-acetyl)-phenyl]-amide,
rac-(5-{[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-4H-[1,2,4]triazol-3-yl)-acetic acid,
rac-(3- {[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-pyrazol-1-yl)-acetic acid,
rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (1H-imidazol-4-ylmethyl)-amide,
rac-(2*S*,3*R*,4*S*,5*R*)-4-(3-chloro-2-fluoro-phenyl)-3-(4-chloro-2-fluoro-phenyl)-2-(2,2-dimethylpropyl)-5-(2-oxa-6-aza-spiro[3.3] heptane-6-carbonyl)-pyrrolidine-3-carbonitrile,
rac-1-[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-azetidine3-carboxylic acid,
rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-[1,2,3]triazol-1-yl-ethyl)-amide,
rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-carbamoylmethyl-1H-pyrazol-3-yl)-amide,
rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid [1-(2-hydroxy-2-methyl-propyl)-1H-pyrazol-3-yl]-amide,
rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (3-methanesulfonylamino-propyl)-amide,
chiral (2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((*S*)-3-dimethylamino-2-hydroxy-propoxy)-2-methy)-propyl]-1H-pyrazol]-3-yl}-amide,
chiral (2*S*,3*R*,4*S*,5*R*)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((*S*)-3-dimethylamino-2-hydroxy-propoxy)-2-methyl-propyl]-1H-pyrazol-3- yl}-amide,
rac-1-{[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-cyclopropane carboxylic acid,
rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid [1-(4-hydroxy-piperidin-4-ylmethyl)-1H-pyrazol-3-yl]-amide,
rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-acetyl-thiophen-3-yl)-amide,
rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-carbamoyl-thiophen-3-yl)-amide,
rac- (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [1-((S)-3-dimethylamino-2-hydroxy-propyl)-1H-pyrazol-3-yl]-amide,
rac-4- {[(2R,3S,4R,5S)-3-(3-chloro-2-fluoro-phenyl)-4-(4-chloro-2-fluoro-phenyl)-4-cyano-5-(1-methyl-cyclohexylmethyl)-pyrrolidine-2-carbonyl]-amino}-benzoic acid,
rac-4-{[(2R,3S,4R,5S)-3-(5-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid methyl ester,
rac-4-{[(2R,3S,4R,5S)-3-(5-ch)oro-2-fluoro-pheny))-4-(4-chloro-2-fluoro-phenyl)-4-cyano-5-(2,2-dimethyl-propyl)-pyrrolidine-2-carbonyl]-amino}-benzoic acid ,
rac-4-{[(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carbonyl]-amino}-benzoic acid methyl ester,
rac-4-{[(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carbonyl]-amino}-benzoic acid,
rac-4-{[(2R,3R,4R,5S)-3-(3-bromo-phenyl)-4-(4-chloro-2-fluoro-phenyl)-4-cyano-5-(2,2-dimethyl-propyl)-pyrrolidine-2-carbonyl]-amino}-benzoic acid methyl ester,
of rac-4- {[(2R,3R,4R,5S)-3-(3-bromo-phenyl)-4-(4-chloro-2-fluoro-phenyl)-4-cyano-5-(2,2-dimethyl-propyl)-pyrrolidine-2-carbonyl]-amino}-benzoic acid,
rac-4-{[(2R,3S,4R,5S)-3-(3-chloro-2-fluoro-phenyl)-4-(4-chloro-phenyl)-4-cyano-5-(2,2-dimethyl-propyl)-pyrrolidine-2-carbonyl]-amino}-benzoic acid methyl ester,
rac-4-{[(2R,3S,4R,5S)-3-(3-chloro-2-fluoro-phenyl)-4-(4-chloro-phenyl)-4-cyano-5-(2,2-dimethyl-propyl)-pyrrolidine-2-carbonyl]-amino}-benzoic acid,
rac-4-{[(2R,3S,4R,5S)-3-(3-chloro-2-fluoro-phenyl)-4-(4-chloro-2-fluoro-phenyl)-4-cyano-5-(1-methyl-cyclohexylmethyl)-pyrrolidine-2-carbonyl]-amino}-benzoic acid methyl ester and
rac-[4-(3-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-pyrazol-1-ylmethyl)-4-hydroxy-piperidin-1-yl]-acetic acid.

In the specification where indicated the various groups may be substituted by 1-5 or, preferably, 1-3 substituents independently selected from the group consisting of lower alkyl, lower-alkenyl, lower-alkynyl, dioxo-lower-alkylene (forming e.g. a benzodioxyl group), halogen, hydroxy, CN, CF₃, NH₂, N(H, lower-alkyl), N(lower-alkyl)₂, aminocarbonyl, carboxy, NO₂, lower-alkoxy, thio-lower-alkoxy, lower-alkylsufonyl, aminosulfonyl, lower-alkylcarbonyl, lower-alkylcarbonyloxy, lower-alkoxycarbonyl, lower-alkyl-carbonyl-NH, fluoro-lower-alkyl, fluoro-lower-alkoxy, lower-alkoxy-carbonyl-lower-alkoxy, carboxy-lower-alkoxy, carbamoyl-lower-alkoxy, hydroxy-lower-alkoxy, NH₂-lower-alkoxy, N(H, lower-alkyl)-lower-alkoxy, N(lower-alkyl)₂-lower-alkoxy, lower-alkyl-1-oxiranyl-lower-alkoxy-lower-alkyl, 2-oxo-pyrrolidin-1-yl, (1,1-dioxo)-2-isothiazolidine, 3-lower-alkyl sulfinyl, a substituted or unsubstituted heterocyclic ring, a substituted or unsubstituted aryl ring, a substituted or unsubstituted heteroaryl ring, trifluoro-lower-alkylsulfonylamino-aryl, lower-alkyl sulfonylaminocarbonyl, lower-alkyl sulfonylaminocarbonyl-aryl, hydroxycarbamoyl-phenyl, benzyloxy-lower-alkoxy, mono- or di-lower alkyl substituted amino-sulfonyl and lower-alkyl which can optionally be substituted with halogen, hydroxy, NH₂, N(H, lower-alkyl) or N(lower-alkyl)₂. Preferred substituents for the cycloalkyl, cycloalkenyl, aryl, heteroaryl and heterocycle rings are halogen, lower alkoxy, lower alkyl, hydroxycarbonyl, carboxy, carboxy lower alkoxy, oxo and CN. Preferred substituents for alkyl are alkoxy and N(lower akyl)₂.

The term "alkyl" refers to straight- or branched-chain saturated hydrocarbon groups having from 1 to about 20 carbon atoms, including groups having from 1 to about 7 carbon atoms. In certain embodiments, alkyl substituents may be lower alkyl substituents. The term "lower alkyl" refers to alkyl groups having from I to 6 carbon atoms, and in certain embodiments from I to 4 carbon atoms. Examples of alkyl groups include, but are not limited to, methyl, ethyl, n-propyl, i-propyl, n-butyl, s-butyl, t-butyl, n-pentyl, and s-pentyl.

As used herein, "cycloalkyl" is intended to refer to any stable monocyclic or polycyclic system which consists of carbon atoms only, any ring of which being saturated, and the term "cycloalkenyl" is intended to refer to any stable monocyclic or polycyclic system which consists of carbon atoms only, with at least one ring thereof being partially unsaturated. Preferably, "cycloalkyl" as used herein consists of 3 to 10 carbon atoms and "cycloalkenyl" of 5 to 10 carbon atoms. Examples of cycloalkyls include, but are not limited to, cyclopropyl, cyclobutyl, cyclopentyl, cyclohexyl, cycloheptyl, adamantyl, cyclooctyl, bicycloalkyls, including bicyclooctanes such as [2.2.2]bicyclooctane or [3.3.0]bicyclooctane, bicyclononanes such as [4.3.0]bicyclononane, and bicyclodecanes such as [4.4.0]bicyclodecane (decalin), or spiro compounds. Examples of cycloalkenyls include, but are not limited to, cyclopentenyl or cyclohexenyl.

The term "alkenyl" as used herein means an unsaturated straight-chain or branched aliphatic hydrocarbon group containing one double bond and having 2 to 6, preferably 2 to 4 carbon atoms. Examples of such "alkenyl group" are vinyl, ethenyl, allyl, isopropenyl, 1-propenyl, 2-methyl-1-propenyl, 1-butenyl, 2-butenyl, 3-butenyl, 2-ethyl-1-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl and 5-hexenyl.

The term "alkynyl" as used herein means an unsaturated straight-chain or branched aliphatic hydrocarbon group containing one triple bond and having 2 to 6, preferably 2 to 4 carbon atoms. Examples of such "alkynyl group" are ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl and 5-hexynyl.

The term "halogen" as used in the definitions means fluorine, chlorine, bromine, or iodine, preferably fluorine and chlorine.

"Aryl" means a monovalent, monocyclic or bicyclic, aromatic carbocyclic hydrocarbon radical, preferably a 6-10 member aromatic ring system. Preferred aryl groups include, but are not limited to, phenyl, naphthyl, tolyl, and xylyl.

"Heteroaryl" means an aromatic, preferably 5 to 10 membered, mono- or bicyclic automatic hydrocarbon, wherein 1 to 4, preferably 1 to 3 carbon atoms are replaced by a hetero atom selected from nitrogen, oxygen or sulfur atom. Preferred heteroaryl groups include, but are not limited to, thienyl, furyl, indolyl, pyrrolyl, pyridinyl, pyrazinyl, oxazolyl, thiaxolyl, quinolinyl, pyrimidinyl, imidazole substituted or unsubstituted triazolyl and substituted or unsubstituted tetrazolyl. Where the heteroaryl group is bicyclic a preferred group is 1,3-dioxo-2,3-dihydro-1H-isoindol-5-yl group.

In the case of aryl or heteroaryl which are bicyclic it should be understood that one ring may be aryl while the other is heteroaryl and both being substituted or unsubstituted. In case of bicyclic ring systems the two rings can be fused (like i.e. in naphthyl) or linked via a single bond (like i.e. in biphenyl).

"Heterocycle" or "heterocyclic ring"means a substituted or unsubstituted 5 to 10-, preferably 5 to 8 membered, mono- or bicyclic, non-aromatic hydrocarbon, wherein 1 to 3 carbon atoms are replaced by a hetero atom selected from nitrogen, oxygen or sulfur atom. Examples include pyrrolidin-2-yl; pyrrolidin-3-yl; piperidinyl; morpholin-4-yl and the like which in turn can be substituted. "Hetero atom" means an atom selected from N, O and S.

"Alkoxy, alkoxyl or lower alkoxy" refers to any of the above lower alkyl groups attached to an oxygen atom. Typical lower alkoxy groups include methoxy, ethoxy, isopropoxy or propoxy, butyloxy and the like. Further included within the meaning of alkoxy are multiple alkoxy side chains, e.g. ethoxy ethoxy, methoxy methoxy, methoxy ethoxy ethoxy and the like and substituted alkoxy side chains, e.g., dimethylamino ethoxy, diethylamino ethoxy, dimethoxy-phosphoryl methoxy and the like.

"Pharmaceutically acceptable," such as pharmaceutically acceptable carrier, excipient, etc., means pharmacologically acceptable and substantially non-toxic to the subject to which the particular compound is administered.

"Pharmaceutically acceptable salt" refers to conventional acid-addition salts or base-addition salts that retain the biological effectiveness and properties of the compounds of the present invention and are formed from suitable non-toxic organic or inorganic acids or organic or inorganic bases. Sample acid-addition salts include those derived from inorganic acids such as hydrochloric acid, hydrobromic acid, hydroiodic acid, sulfuric acid, sulfamic acid, phosphoric acid and nitric acid, and those derived from organic acids such as p-toluenesulfonic acid, salicylic acid, methanesulfonic acid, oxalic acid, succinic acid, citric acid, malic acid, lactic acid, fumaric acid, trifluoro acetic acid and the like. Sample base-addition salts include those derived from ammonium, potassium, sodium and, quaternary ammonium hydroxides, such as for example, tetramethylammonium hydroxide. Chemical modification of a pharmaceutical compound (i.e. drug) into a salt is a technique well known to pharmaceutical chemists to obtain improved physical and chemical stability, hygroscopicity, flowability and solubility of compounds. *See, e.g.*, Ansel *et al.*, Pharmaceutical Dosage Forms and Drug Delivery Systems (6th Ed. 1995) at pp. 196 and 1456- 1457.

The compounds of formula I and II as well as their salts that have at least one asymmetric carbon atom may be present as racemic mixtures or different stereoisomers. The various isomers can be isolated by known separation methods, e.g., chromatography.

Compounds disclosed herein and covered by formula I and II above may exhibit tautomerism or structural isomerism. Also described are any tautomeric or structural isomeric form of these compounds, or mixtures of such forms.

The compounds of the present invention are useful in the treatment or control of cell proliferative disorders, in particular oncological disorders. These compounds and formulations containing said compounds may be particularly useful in the treatment or control of solid tumors, such as, for example, breast, colon, lung and prostate tumors.

Consequently, as a further embodiment according to the present invention there are provided the compounds according to formula I and II for use as medicaments, in particular for the use as medicaments in the treatment of cancer, more particular solid tumors, and most particularly breast, colon, lung and prostate tumors.

A therapeutically effective amount of a compound in accordance with this invention means an amount of compound that is effective to prevent, alleviate or ameliorate symptoms of disease or prolong the survival of the subject being treated. Determination of a therapeutically effective amount is within the skill in the art.

The therapeutically effective amount or dosage of a compound according to this invention can vary within wide limits and may be determined in a manner known in the art. Such dosage will be adjusted to the individual requirements in each particular case including the specific compound(s) being administered, the route of administration, the condition being treated, as well as the patient being treated. In general, in the case of oral or parenteral administration to adult humans weighing approximately 70 Kg, a daily dosage of about 10 mg to about 10,000 mg, preferably from about 200 mg to about 1,000 mg, should be appropriate, although the upper limit may be exceeded when indicated. The daily dosage can be administered as a single dose or in divided doses, or for parenteral administration; it may be given as continuous infusion.

Formulations of the present invention include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, as well as the particular mode of administration. The amount of active ingredient which can be combined with a carrier material to produce a single dosage form will generally be that amount of a formula I compound which produces a therapeutic effect. Generally, out of one hundred percent, this amount will range from about 1 percent to about ninety-nine percent of active ingredient, preferably from about 5 percent to about 70 percent, most preferably from about 10 percent to about 30 percent.

Methods of preparing these formulations or compositions include the step of bringing into association a compound of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules, cachets, sachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary or paste.

"Effective amount" means an amount that is effective to prevent, alleviate or ameliorate symptoms of disease or prolong the survival of the subject being treated.

"IC50" refers to the concentration of a particular compound required to inhibit 50% of a specific measured activity. IC₅₀ can be measured, *inter alia*, as is described subsequently.

The present invention provides methods for the synthesis of pyrrolidine-2-carboxamide. The compounds of the invention can be prepared by processes known in the art. Suitable processes for synthesizing these compounds are provided in the examples.

Compounds of this invention can be synthesized according to the following general schemes. The key transformation is a convergent [2+3] cylcoaddition of emine **II** and activated olefin **III** to generate pyrrolidine-3-carbonitrile compounds **IV** in a stereoselective and efficient manner.

The starting materials are either commercially available or can be synthesized by methods known to those of ordinary skill in the art. Preparations of intermediates **II** and **III** are illustrated in **Scheme 1** and **2**. In general an appropriately selected aldehyde or ketonecan be reacted with glycine tert-butyl ester or glycine methyl ester to generate imine **II** and were used as a crude product **(Scheme 1)**.

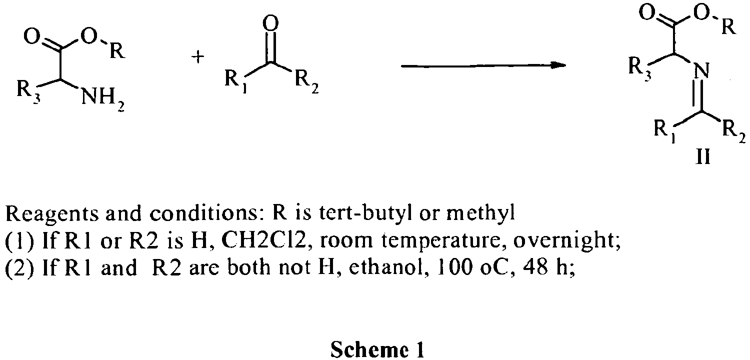

An intermediate of formula **III** can be made from a base-catalyzed condensation reaction of appropriately selected substituted-phenyl acetonitrile and aldehyde The reaction proceedes in a highly stereoselective manner with Z-isomer as the major or exclusive product.

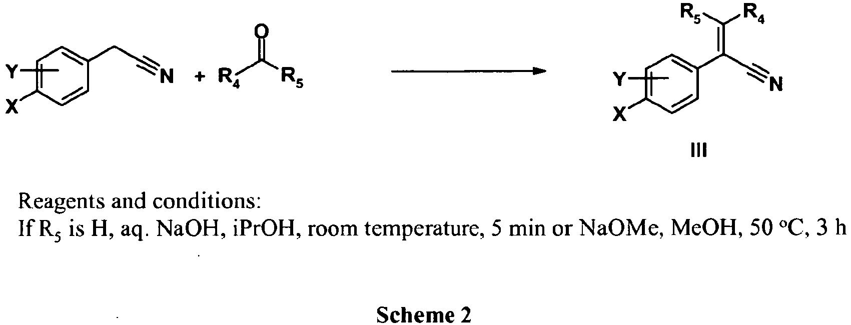

As illustrated in **Scheme 3**, pyrrolidine of formula **IV** can be made from intermediates **II** and **III** by a convergent 1,3-dipolar cylcoaddition reaction mediated by lewis acid AgF and triethylamine. The [2+3] cycloaddition reactions of azomethine ylides 1,3-dipoles with olefinic dipolarphiles to form pyrrolidine ring formation have been described in published procedures including Jorgensen, K. A. et al (Org. Lett. 2005, Vol 7, No. 21, 4569-4572), Grigg, R. et al (Tetrahedron, 1992, Vol 48, No. 47, 10431-10442-, Tetrahedron, 2002, Vol 58, 1719-1737), Schreiber, S. L. et al (J. Am. Chem. Soc., 2003, 125, 10174-10175), and Carretero, J. C. et al (Tetrahedron, 2007, 63, 6587-6602).. Compounds **IV** is subsequently converted to acid **V** followed by amide formation with various amines using HATU as the coupling reagent to give the compounds of formula **I**. The amide formation from **V** to **I** can also be achieved under other conditions using EDCI and HOBt or oxalyl chloride as the coupling reagent to activate the acid **V**.

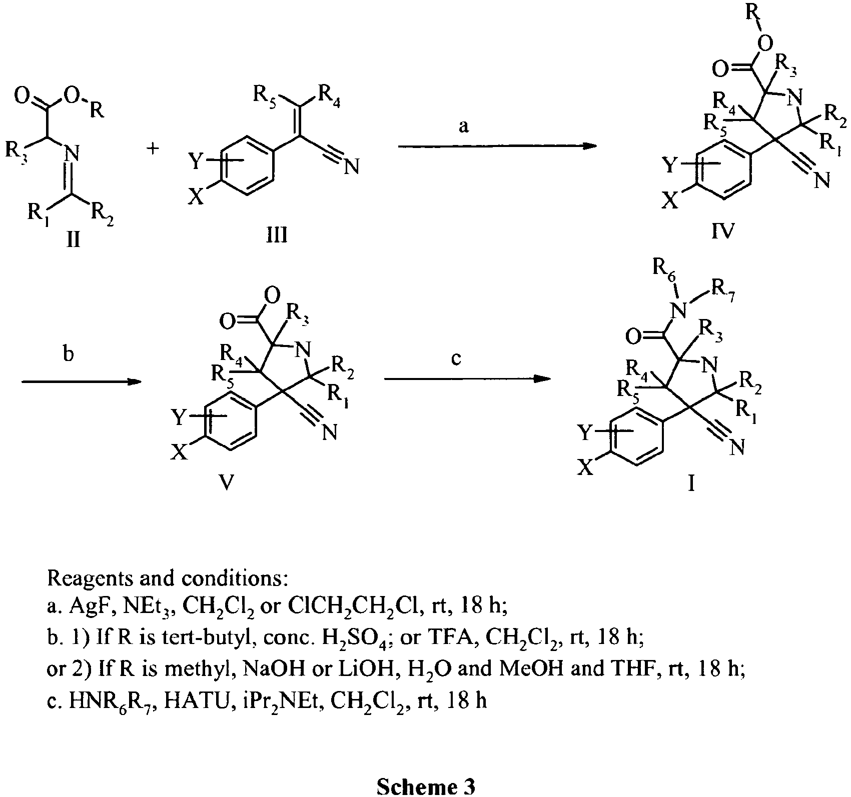

The process according to scheme 3 forms a further embodiment according to the present invention.

The pyrrolidine compounds **I**, **IV**, **V** are prepared initially as a racemic mixture and can be chirally separated using chiral Super Fluid Chromatography (SFC) or chiral HPLC or chiral column chromatography. For example, racemic mixture of compound **Ia** and **Ia'** can be readily resolved into two optically pure or enriched chiral enantiomers by separation using chiral Super Fluid Chromatography (SFC). **(Scheme 4)**.

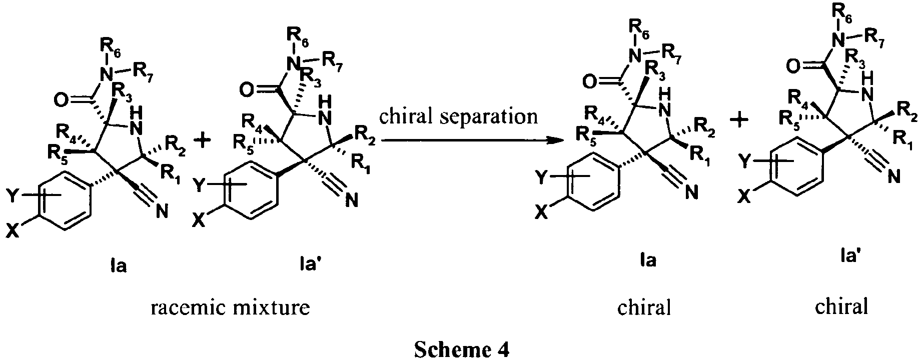

### Examples

The compounds of the present invention may be synthesized according to known techniques. The following examples and references are provided to aid the understanding of the present invention, the true scope of which is set forth in the appended claims.

### Example 1a

### Preparation of intermediate [3,3-dimethyl-but-(E)-ylideneamino]-acetic acid tert-butyl ester

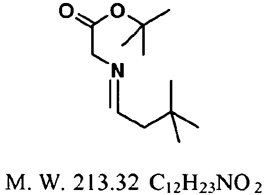

A mixture of glycine tert-butyl ester (Alfa) (2.71 g, 20.0 mmol) and 3,3-dimethyl-butyraldehyde (Alfa) (2.21 g. 21.0 mmol) in CH₂Cl₂ (50 mL) was stirred at rt overnight. The reaction mixture was concentrated and the residue was dried *in vacuo* to give [3,3-dimethyl-but-(E)-ylideneamino]-acetic acid tert-butyl ester (4.29 g, 100%) as colorless oil which was used in the next step without further purification.

### Example 1b

### Preparation of intermediate (Z)-3-(3-chloro-phenyl)-2-(4-chloro-phenyl)-acrylonitrile

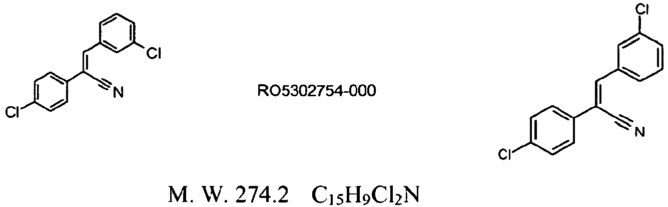

### Method A.

To a solution of 4-chlorobenzyl cyanide (5.62 g, 4.00 mmol) and 3-chloro-benzaldehyde (Aldrich) (6.06 g, 4.00 mmol) in iPrOH (250 mL) was added 4 N NaOH (5 mL) dropwise at rt and the reaction mixture was stirred at rt for 10 min to give a white suspension. The solid was filtered and washed with water and iPrOH and then dried overnight *in vacuum* to give (Z)-3-(3-chloro-phenyl)-2-(4-chloro-phenyl)-acrylonitrile (9.33 g, 85.1%) as a white powder which was used in the next step without further purification.

### Method B.

To a solution of4-chlorobenzyl cyanide (Aldrich) (4.5 g, 30 mmol) and 3-chloro-benzaldehyde (Aldrich) (4 g, 29 mmol) in methanol (150 mL) was slowly added a methanolic solution (Aldrich, 25 wt.%) of sodium methoxide (10 mL, 44 mmol). The reaction mixture was heated and stirred at 50 °C for 3 h. The mixture became cloudy, and was cooled to room temperarure and filtered. The white precipitate was washed with water, cold methanol, and then dried *in vacu* to give the first batch of desired product (5.5 g). The filtrate was concentrated, diluted with water, neutralized by aqueous HCl solution to "pH" 7, then extracted with ethyl acetate. The organic layer was separated, dried over MgSO₄, and concentrated. The residue was purified by chromatography (EtOAc;hexanes = 1;20, then 1:10) to give the second batch of the desired product (1.6 g).The two batches were combined to give (Z)-3-(3-chloro-phenyl)-2-(4-chloro-phenyl)-acrylonitrile as a white powder (7.1 g, 88%).

HRMS (ES⁺) *m*/*z* Calcd for C₁₅H₉Cl₂N [M⁺]; 273.0112, found: 273.0113.

### Example 1c

### Preparation of intermediate rac-(2R,3 R,4R,5S)-3-(3-chloro-phenyl)-4-(4-chloro-phenyl)-4-cyano-5-(2,2-dimethyl-propyl)-pyrrolidine-2-carboxylic acid tert-butyl ester

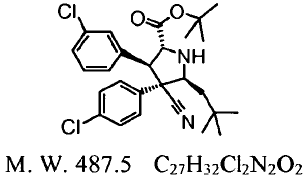

To a solution of [3,3-dimethyl-but-(E)-ylideneamino]-acetic acid tert-butyl ester (4.26 g, 20.00 mmol) and (Z)-3-(3-chloro-phenyl)-2-(4-chloro-phenyl)-acrylonitrile (5.48 g, 20.00 mmol) in ClCH₂CH₂Cl (100 mL) were added triethyl amine (4.2 g, 40.00 mmol) and AgF (2.53 g, 20.00 mmol) in one portion. The mixture was stirred at rt overnight. The mixture was then quenched with sat. NH₄Cl and extracted with CH₂Cl₂. The organic phase was separated, filtered through Celite and dried over Na₂SO₄. The mixture was then separated and concentrated. The residue was triturated with EtOAc and nHexane, and the precipitates were collected by filtration and the mother liquid was concentrated and further purified by flash column (SiO₂, 1-20% of EtOAc in hexanes) to give rac-(2R,3R,4R,5S)-3-(3-chloro-phenyl)-4-(4-chloro-phenyl)-4-cyano-5-(2,2-dimethyl-propyl)-pyrrolidine-2-carboxylic acid tert-butyl ester (6.65 g, 68.2%; HRMS (ES⁺) m/z Calcd for C₂₇H₃₂Cl₂N₂O₂ + H [(M+H)⁺]: 487.1914, found: 487.1910) and rac-(2R,3R,4R,5R)-3-(3-chloro-phenyl)-4-(4-chloro-phenyl)-4-cyano-5-(2,2-dimethyl-propyl)-pyrrolidine-2-carboxylic acid tert-butyl ester (0.86 g, 8.8%).

HRMS (ES⁺) m/z Calcd for C₂₇H₃₂Cl₂N₂O₂ + H [(M+H)⁺]: 487.1914, found: 487.1910).

### Example 1d

### Preparation of intermediate rac-(2R,3R,4R,5S)-3-(3-chloro-phenyl)-4-(4-chloro-phenyl)-4-cyano-S-(2,2-dimethyl-propyl)-pyrrolidine-2-carboxylic acid

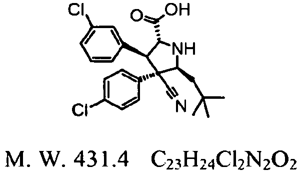

A solution of rac-(2R,3R,4R,5S)-3-(3-chloro-phenyl)-4-(4-chloro-phenyl)-4-cyano-5-(2,2-dimethyl-propyl)-pyrrolidine-2-carboxylic acid tert-butyl ester (3.78 g, 7.75 mmol) in conc. H₂SO₄ (20 mL) was stirred at rt for 2 hrs. The mixture was then poured into ice and extracted with EtOAc. The organic phase was separated, dried over Na₂SO₄, and concentrated. The residue was then triturated with EtOAc and nHexane and the precipitates were collected by filtration and washed with ether to give rac-(2R,3R,4R,5S)-3-(3-chloro-phenyl)-4-(4-chloro-phenyl)-4-cyano-5-(2,2-dimethyl-propyl)-pyrrolidine-2-carboxylic acid (3.60 g, 100%) as a white solid which was used in the next step without further purification: HRMS (ES⁺) m/z Calcd for C₂₃H₂₄Cl₂N₂O₂ + H [(M+H)⁺]:431.1288, found: 431.1287.

### Example 1e

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid (2-morpholin-4-yl-ethyl)-amide

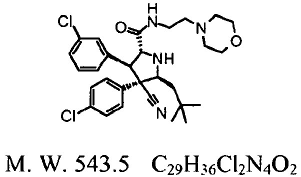

A mixture of rac-(2R,3R,4R,5S)-3-(3-chloro-phenyl)-4-(4-chloro-phenyl)-4-cyano-5-(2,2-dimethyl-propyl)-pyrrolidine-2-carboxylic acid (61.0 mg, 0.14 mmol), 2-morpholin-4-yl-ethylamine (36.0 mg, 0.28 mmol), 2-(7-azabenzotriazol-1-yl)-N,N,N',N'-tetramethyluronium hexafluorophosphate (HATU, 106.0 mg, 0.28 mmol) and iPr₂NEt (38.8 mg, 0.30 mmol) in CH₂Cl₂ (2 mL) was stirred at rt overnight. The mixture was then diluted with CH₂Cl₂ and washed with water, brine. The organic phase was separated, filtered and dried over Na₂SO₄. The mixture was then concentrated and purified by SiO₂ flash column (20-100% ofEtOAc in Hexanes) to give rac-(2R,3R,4R,5S)-3-(3-chloro-phenyl)-4-(4-chloro-phenyl)-4-cyano-5-(2,2-dimethyl-propyl)-pyrrolidine-2-carboxylic acid (2-morpholin-4-yl-ethyl)-amide (60.5 mg, 86.4%) as a white amorphous.

HRMS (ES⁺) m/z Calcd for C₂₉H₃₆Cl₂N₄O₂ + H[(M+H)⁺]: 543.2288, found : 523.2284.

### Example 1f

### Preparation of (2R,3R,4R,5S)-3-(3-chloro-phenyl)-4-(4-chloro-phenyl)-4-cyano-5-(2,2-dimethylpropyl)-pyrrolidine-2-carboxylic acid (2-morpholin-4-yl-ethyl)-amide

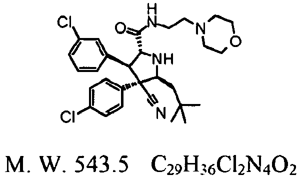

The racemic product obtained above (**Example 1e**, 45 mg) was further separated by SFC chiral column to give-(2R,3R,4R,5S)-3-(3-chloro-phenyl)-4-(4-chloro-phenyl)-4-cyano-5-(2,2-dimethyl-propyl)-pyrrolidine-2-carboxylic acid (2-morpholin-4-yl-ethyl)-amide (13.1 mg, 29.1%) and (2S,3S,4S,5R)-3-(3-chloro-phenyl)-4-(4-chloro-phenyl)-4-cyano-5-(2,2-dimethylpropyl)-pyrrolidine-2-carboxylic acid (2-morpholin-4-yl-ethyl)-amide (14.6 mg, 32.4%).

### Example 2

### Preparation of rac-(2R,3R,4R,5S)-3-(3-chloro-phenyl)-4-(4-chloro-phenyl)-4-cyano-5-(2,2-dimethyl-propyl)-pyrrolidinc-2-carboxylic acid dimethylamide

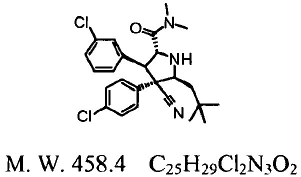

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (61.0 mg, 0.14 mmol) prepared in **Example 1d** was reacted with dimethylamine (1.0 M in THF, 2 mL), HATU (106.0 mg, 0.28 mmol) and iPr₂NEt (38.8 mg, 0.30 mmol) in CH₂Cl₂ (2 mL) at rt overnight to give rac-(2R,3R,4R,5S)-3-(3-chloro-phenyl)-4-(4-chloro-phenyl)-4-cyano-5-(2,2-dimethyl-propyl)-pyrrolidine-2-carboxylic acid dimethyl amide (57.8 mg, 90.0 %).

HRMS (ES⁺) m/z Calcd for C₂₅H₂₉Cl₂N₃O₂ + H [(M+H)⁺]: 458.1761, found: 458.1757.

### Example 3a

### Preparation of intermediate 2-((S)-2,2-dimethyl-[1,3]dioxolan-4-yl)-ethylamine

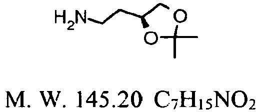

### Step A.

To a solution of (4S)-(+)-4-(2-hydroxyethyl)-2,2-dimethyl-1,3-dioxolane (Aldrich) (21.1 g, 0.14 mol) and triethylamine (40 mL, 0.28 mol) in dichloromethane (250 mL) at 0 °C was added methanesulfonyl chloride (13.4 mL, 0.17 mol) dropwise. The reaction mixture was stirred at 0 °C for 1.5 h, then water was added. The organic layer was separated, washed with water, brine, dried over MgSO₄, concentrated to give methanesulfonic acid 2-((S)-2,2-dimethyl-[1,3]dioxolan-4-yl)-ethyl ester as a yellow oil (31.7 g, 98%).

### Step B.

To a solution of methanesulfonic acid 2-((S)-2,2-dimethyl-[1,3]dioxolan-4-yl)-ethyl ester (31.7 g, 0.14 mol) in N,N-dimethylformamide (200 mL) was added NaN₃ (46 g, 0.71 mol). The reaction mixture was stirred at room temperature for 70 h. Then the mixture was partitioned between ethyl acetate and water. The organic layer was separated, washed with water, brine several times, dried over MgSO₄, concentrated to give (S)-4-(2-azido-ethyl)-2,2-dimethyl-[1,3]dioxolane as a yellow oil (21.3 g, 88%).

### Step C.

A suspension of (S)-4-(2-azido-ethyl)-2,2-dimethyl-[1,3]dioxolane as a yellow oil (18.7 g, 0.11 mol) and PtO₂ (2.5 g) in ethyl acetate (100 mL) was vigorously shaken in a Parr under atmosphere of H₂ (50 psi) for 18 h. The mixture was filtered through a short pad of celite. The filtrate was concentrated to give 2-((S)-2,2-dimethyl-[1,3]dioxolan-4-yl)-ethylamine as a colorless oil (14 g, 88%).

### Example 3b

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid ((S)-3,4-dihydroxy-butyl)-amide

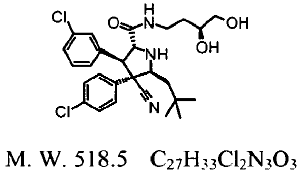

A mixture of rac-(2R,3R,4R,5S)-3-(3-chloro-phenyl)-4-(4-chloro-phenyl)-4-cyano-5-(2,2-dimethyl-propyl)-pyrrolidine-2-carboxylic acid (431.4 mg, 1.00 mmol) prepared in **Example 1d**, 2-((S)-2,2-dimethyl-[1,3]dioxolan-4-yl)-ethylamine (217.5 mg, 1.5 mmol), HATU (570.30 mg, 1.50 mmol) and iPr₂NEt (258.6 mg, 2.00 mmol) in CH₂Cl₂ (20 mL) was stirred at rt for 1 hour. The mixture was then diluted with CH₂Cl₂ and washed with water, brine. The organic phase was separated, filtered and dried over Na₂SO₄. The mixture was then concentrated and the residue was treated with PPTS (cat) in MeOH (20 mL) at 120 °C for 5 min with CEM microwave reactor. The reaction mixture was concentrated and the residue was diluted with EtOAc and washed with water, brine. The organic phase was separated, filtered and dried over Na₂SO₄. The mixture was then concentrated and purified by SiO₂ flash column (5% of MeOH in EtOAc) to give rac-(2R,3R,4R.SS)-3-(3-chloro-phenyl)-4-(4-chloro-phenyl)-4-cyano-5-(2,2-dimethyl-propyl)-pyrrolidine-2-carboxylic acid ((S)-3,4-dihydroxy-butyl)-amide (450.0 mg, 86.7%) as a white amorphous.

HRMS (ES⁺) m/z Calcd for C₂₇H₃₃Cl₂N₃O₃+ H[(M+H)⁺]:518.1972, found: 518.1970.

### Example 3c

### Preparation of (2R,3R,4R,5S)-3-(3-chloro-phenyl)-4-(4-chloro-phenyl)-4-cyano-5-(2,2-dimethylpropyl)-pyrrolidine-2-carboxylic acid ((S)-3,4-dihydroxy-butyl)-amide

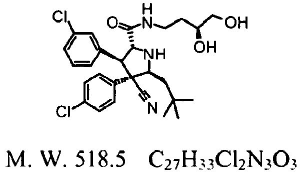

The racemic product obtained above (**Example 3b**, 450.0 mg) was further separated by SFC chiral column to give (2R,3R,4R,5S)-3-(3-chloro-phenyl)-4-(4-chloro-phenyl)-4-cyano-5-(2,2-dimethyl-propyl)-pyrrolidine-2-carboxylic acid ((S)-3,4-dihydroxy-butyl)-amide (178.6 mg, 34.4%) and (2S,3S,4S,5R)-3-(3-chloro-phenyl)-4-(4-chloro-phenyl)-4-cyano-5-(2,2-dimethylpropyl)-pyrrolidine-2-carboxylic acid ((S)-3,4-dihydroxy-butyl)-amide (159.8 mg, 30.8%).

### Example 4

### Preparation of rac-(2S,3R,4R,5R)-4-(3-chloro-phenyl)-3-(4-chloro-phenyl)-2-(2,2-dimethylpropyl)-5-[4-(2-morpholin-4-yl-2-oxo-ethyl)-piperazine-1-carbonyl]-pyrrolidine-3-carbonitrite

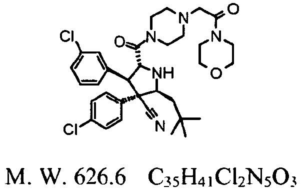

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (61.0 mg, 0.14 mmol) prepared in **Example 1d** was reacted with 1-morpholin-4-yl-2-piperazin-1-yl-ethanone (65.0 mg, 0.30 mmol), HATU (106.0 mg, 0.28 mmol) and iPr₂NEt (38.8 mg, 0.30 mmol) in CH₂Cl₂ (2 mL) at rt overnight to give rac-(2S,3R,4R,5R)-4-(3-chloro-phenyl)-3-(4-chloro-phenyl)-2-(2,2-dimethyl-propyl)-5-[4-(2-morpholin-4-yl-2-oxo-ethyl)-piperazine-1-carbonyl)-pyrrolidine-3-carbonitrile (45.5 mg, 51.9 %).

HRMS (ES⁺) m/z Calcd for C₃₅H₄₁Cl₂N₅O₃ + H [(M+H)⁺]: 626.2659, found: 626.2654.

### Example 5

### Preparation of rac-(2S,3R,4R,5R)-4-(3-chloro-phenyl)-3-(4-chloro-phenyl)-2-(2,2-dimethylpropyl)-5-[4-(2-oxo-2-pyrrolidin-1-yl-ethyl)-piperazine-1-carbonyl]-pyrrolidine-3-carbonitrile

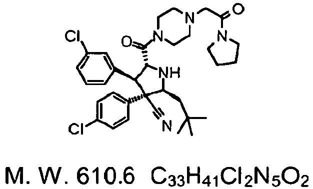

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (61.0 mg, 0.14 mmol) prepared in **Example 1d** was reacted with 2-piperazin-1-yl-1-pyrrolidin-1-yl-ethanone (65.0 mg, 0.33 mmol), HATU (106.0 mg, 0.28 mmol) and iPr₂NEt (38.8 mg, 0.30 mmol) in CH₂Cl₂ (2 mL) at rt overnight to give rac-(2S,3R,4R,5R)-4-(3-Chloro-phenyl)-3-(4-chloro-phenyl)-2-(2,2-dimethyl-propyl)-5-[4-(2-oxo-2-pyrrolidin-1-yl-ethyl)-piperazine-1-carbonyl]-pyrrolidine-3-carbonitrile (60.5 mg, 70.8 %).

HRMS (ES⁺) m/z Calcd for C₃₃H₄₁Cl₂N₅O₂ + H [(M+H)⁺]: 610.2710, found: 610.2708.

### Example 6

### Preparation of rac-(2S,3R,4R,5R)-4-(3-chloro-phenyl)-3-(4-chloro-phenyl)-2-(2,2-dimethylpropyl)-5-[4-(2-hydroxy-ethyl)-piperazine-1-carbonyl]-pyrrolidine-3-carbonitrile

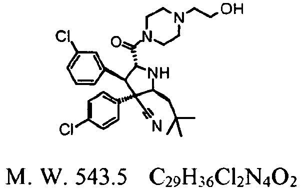

In a manner similar to the method described in **Example 1e**, rac-(2R,3R,4R,SS)-3-(3-chloro-phenyl)-4-(4-chloro-phenyl)-4-cyano-5-(2,2-dimethyl-propyl)-pyrrolidine-2-carboxylic acid (61.0 mg, 0.14 mmol) prepared in **Example 1d** was reacted with 2-piperazin-1-yl-ethanol (65.0 mg, 0.50 mmol), HATU (106.0 mg, 0.28 mmol) and iPr₂NEt (38.8 mg, 0.30 mmol) in CH₂Cl₂ (2 mL) at rt overnight to give rac-(2S,3R,4R,5R)-4-(3-chloro-phenyl)-3-(4-chloro-pheny))-2-(2,2-dimethyl-propyl)-5-[4-(2-hydroxy-ethyl)-piperazine-1-carbonyl]-pyrrolidine-3-carbonitrile (48.3 mg, 63.5 %).

HARMS (ES⁺) m/z Calcd for C₂₉H₁₆Cl₂N₄O₂ + H [(M+H)⁺]: 543.2288, found: 543.2284.

### Example 7

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid (4-hydroxy-butyl)-amide

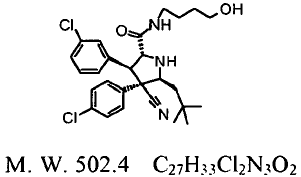

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (61.0 mg, 0.14 mmol) prepared in **Example 1d** was reached with 4-methylamino-butan-1-ol (44.5 mg, 0.50 mmol), HATU (106.0 mg, 0.28 mmol) and iPr₂NEt (38.8 mg, 0.30 mmol) in CH₂Cl₂ (2 mL) at rt overnight to give rac-(2R,3R,4R,5S)-3-(3-chloro-phenyl)-4-(4-chloro-phenyl)-4-cyano-5-(2,2-dimethyl-propyl)-pyrrolidine-2-carboxylic acid (4-hydroxy-butyl)-amide (30.5 mg, 43.4 %). HRMS (ES⁺) m/z Calcd for C₂₇H₃₃Cl₂N₃O₂+H [(M+H)⁺]:502.2023, found: 502.2020.

### Example 8

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid (2-pyrrolidin-1-yl-ethyl)-amide

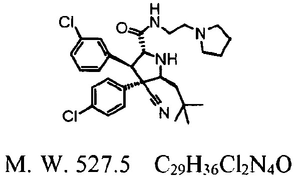

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (82.2 mg, 0.20 mmol) prepared in **Example 1d** was reacted with 2-pyrrolidin-1-yl-ethylamine (34.2 mg, 0.30 mmol), HATU (76.0 mg, 0.20 mmol) and iPr₂NEt (38.8 mg, 0.30 mmol) in CH₂Cl₂ (2 mL) at rt overnight to give rac-(2R,3F,4R,5S)-3-(3-chloro-phenyl)-4-(4-chloro-phenyl)-4-cyano-5-(2,2-dimethyl-propyl)-pyrrolidine-2-carboxylic acid (2-pyrrolidin-1-yl-ethyl)-amide (50.6 mg, 64.0 %). HRMS (ES⁺) m/z Calcd For C₂₉H₃₆Cl₂N₄O +H [(M+H)⁺]: 527.2339, found: 527.2338.

### Example 9

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid (2-piperazin-1-yl-ethyl)-amide

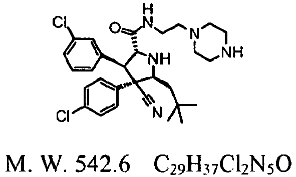

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (82.2 mg, 0.20 mmol) prepared in **Example 1d** was reacted with 2-piperazin-1-yl-ethylamine (38.7 mg, 0.30 mmol), HATU (76.0 mg, 0.20 mmol) and iPr₂NEt (38.8 mg, 0.30 mmol) in CH₂Cl₂ (2 mL) at rt overnight to give rac-(2R,3R,4R,5S)-3-(3-chloro-phenyl)-4-(4-chloro-phenyl)-4-cyano-5-(2,2-dimethyl-propyl)-pyrrolidine-2-carboxylic acid (2-piperazin-1-yl-ethyl)-amide (45.9 mg, 58.0 %). HRMS (ES⁺) m/z Calcd for C₂₉H₃₇Cl₂N₅O +H [(M+H)⁺] : 542.2448, found: 542.2445.

### Example 10a

### Preparation of (S)-2-{[(2R,3R,4R,5S)-3-(3-chloro-phenyl)-4-(4-chloro-phenyl)-4-cyano-5-(2,2-dimethyl-propyl)-pyrrolidine-2-carbonyl]-amino}-3-methyl-butyric acid tert-butyl ester

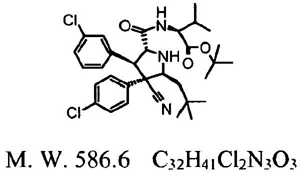

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (215.7 mg, 0.50 mmol) prepared in **Example 1d** was reacted with (S)-2-amino-3-methyl-butyric acid tert-butyl ester (125.4 mg, 0.60 mmol), HATU (210.1.0 mg, 0.60 mmol) and iPr₂NEt (129.3 mg, 1.00 mmol) in CH₂Cl₂ (5 mL) at rt overnight to give (S)-2-{[(2R,3R,4R,5S)-3-(3-chloro-phenyl)-4-(4-chloro-phenyl)-4-cyano-5-(2,2-dimethyl-propyl)-pyrrolidine-2-carbonyl]-amino}-3-methyl-butyric acid tert-butyl ester (95.0 mg, 32.4 %) after column separation. HRMS (ES⁺) m/z Calcd for C₃₂H₄₁Cl₂N₃O₃+H [(M+H)⁺]: 586.2602, found: 586.2598.

### Example 10b

### Preparation of (S)-2-{[(2S,3S,4S,5R)-3-(3-chloro-phenyl)-4-(4-chloro-phenyl)-4-cyano-5-(2,2-dimethyl-propyl)-pyrrolidine-2-carbonyl]-amino}-3-methyl-butyric acid tert-butyl ester

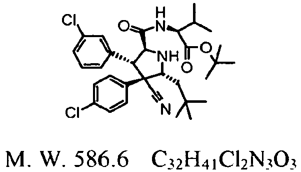

Column separation from the above example **(Example 10a)** gave (S)-2-{[(2S,3S,4S,5R)-3-(3-chloro-phenyl)-4-(4-chloro-phenyl)-4-cyano-5-(2,2-dimethyl-propyl)-pyrrolidine-2-carbonyl]-amino}-3-methyl-butyric acid tert-butyl ester (98.0 mg, 33.4 %).

HRMS (ES⁺) m/z Calcd for C₃₂H₄₁Cl₂N₃O₃+H [(M+H)⁺]: 5886.2601, found: 586.2598.

### Example 10c

### Preparation of (S)-2- {[(2R,3R,4R,5S)-3-(3-chloro-phenyl)-4-(4-chloro-phenyl)-4-cyano-5-(2,2-dimethyl-propyl)-pyrrolidine-2-carbonyl]-amino}-3-methyl-butyric acid methyl ester

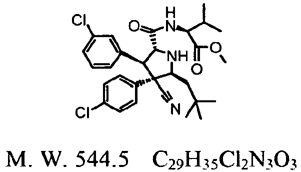

Column separation from the above example (**Example 10a**) gave a mixture of (S)-2-{[(2R,3R,4R,5S)-3-(3-chloro-phenyl)-4-(4-chloro-phenyl)-4-cyano-5-(2,2-dimethyl-propyl)-pyrrolidine-2-carbonyl]-amino}-3-methyl-butyric acid tert-butyl ester and (S)-2-{[(2S,3S,4S,5R)-3-(3-chloro-phenyl)-4-(4-chloro-phenyl)-4-cyano-5-(2,2-dimethyl-propyl)-pyrrolidine-2-carbonyl]-amino}-3-methyl-butyric acid tert-butyl ester (45.8 mg, 15.6 %). The mixture was treated with 2N H₂SO₄ (catalytic) in MeOH (1 mL) at 120°C for 10 min using CEM microwave reactor to give after purification byPR-HPLC: (S)-2-{[(2R,3R,4R,5S)-3-(3-chloro-phenyl)-4-(4-chloro-phenyl)-4-cyano-5-(2,2-dimethyl-propyl)-pyrrolidine-2-carbonyl]-amino}-3-methyl-butyric acid methyl ester (15.5 mg, 36.5%).

HRMS (ES⁺) m/z Calcd for C₂₉H₃₅Cl₂N₃O₃+H[(M+H)⁺]: 544.2128, found: 544.2127.

### Example 10d

### Preparation of (S)-2-{[(2S,3S,4S,5R)-3-(3-chloro-phenyl)-4-(4-chloro-phenyl)-4-cyano-5-(2,2-dimethyl-propyl)-pyrrolidine-2-carbonyl]-amino}-3-methyl-butyric acid methyl ester

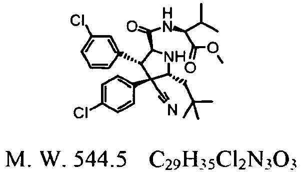

Column separation from the above example (**Example 10a**) gave a mixture of (S)-2-{[(2R,3R,4R,5S)-3-(3-chloro-phenyl)-4-(4-chloro-phenyl)-4-cyano-5-(2,2-dimethyl-propyl)-pyrrolidine-2-carbonyl]-amino}-3-methyl-butyric acid tert-butyl ester and (S)-2-([(2S,3S,4S,5R)-3-(3-chloro-phenyl)-4-(4-chloro-phenyl)-4-cyano-5-(2,2-dimethyl-propyl)-pyrrolidine-2-carbonyl]-amino}-3-methyl-butyric acid tert-butyl ester (45.8 mg, 15.6 %). The mixture was treated with 2N H₂SO₄ (catalytic) in MeOH (1 mL) at 120°C for 10 min using CEM microwave reactor to give after purification by reverse phase chromatography (20-95% of MeCN/water): (S)-2-{[(2S,3S,4S,5R)-3-(3-chloro-phenyl)-4-(4-chloro-phenyl)-4-cyano-5-(2,2-dimethyl-propyl)-pyrrolidine-2-carbonyl]-amino}-3-methyl-butyric acid methyl ester (13.5 mg, 31.8%).

HRMS (ES⁺) m/z Calcd for C₂₉H₃₅Cl₂N₃O₃+H [(M+H)⁺]: 544.2128. found: 544.2126.

### Example 11

### Preparation of (S)-2-{[(2R,3R,4R,5S)-3-(3-chloro-phenyl)-4-(4-chloro-phenyl)-4-cyano-5-(2,2-dimethyl-propyl)-pyrrolidine-2-carbonyl]-amino}-3-methyl-butyric acid

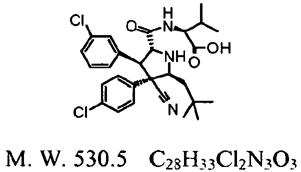

A mixture of (S)-2-{[(2R,3R,4R,5S)-3-(3-chloro-phenyl)-4-(4-chloro-phenyl)-4-cyano-5-(2,2-dimethyl-propyl)-pyrrolidine-2-carbonyl]-amino]-3-methyl-butyric acid tert-butyl ester (86.0 mg, 0.15 mmol) prepared in **Example 10a** and 2 N H₂SO₄ (0.5 mL) in MeCN (1 mL) was heated to 120 °C for 10 min with CEM microwave reactor. The mixture was then concentrated and the residue was purified by reverse phase chromatography (20-95% of MECN/water) to give: (S)-2-{[(2R,3R,4R,5S)-3-(3-chloro-phenyl)-4-(4-chloro-phenyl)-4-cyano-5-(2,2-dimethyl-propyl)-pyrrolidine-2-carbonyl]-amino}-3-methyl-butyric acid (45.1 mg, 58.0%).

HRMS (ES⁺) m/z Calcd for C₂₈H₃₃Cl₂N₃O₃+H [(M+H)⁺]:530.1972, found: 530.1971.

### Example 12

### Preparation of (S)-2-{[(2S,3S,4S,5R)-3-(3-chloro-phenyl)-4-(4-chloro-phenyl)-4-cyano-5-(2,2-dimethyl-propyl)-pyrrolidine-2-carbonyl]-amino}-3-methyl-butyric acid

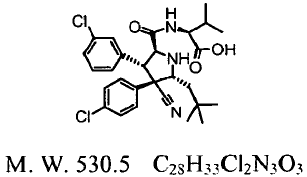

A mixture of(S)-2-{[(2S,3S,4S,5R)-3-(3-chloro-phenyl)-4-(4-chloro-phenyl)-4-cyano-5-(2,2-dimethyl-propyl)-pyrrolidine-2-carbonyl]-amino}-3-methyl-butyric acid tert-butyl ester (90 mg, 0.15 mmol) prepared in **Example 10b** and 2 N H₂SO₄ (0.5 mL) in MeCN (1 mL) was heated to 120 °C for 10 min with CEM microwave reactor. The mixture was then concentrated and the residue was purified by reverse phase chromatography (20-95% of MeCN/water) to give: (S)-2-([(2S,3S,4S,5R)-3-(3-chloro-phenyl)-4-(4-chloro-phenyl)-4-cyano-5-(2,2-dimethyl-propyl)-pyrrolidine-2-carbonyl]-amino}-3-methyl-butyric acid (45.8 mg, 56.3%).

HRMS (ES⁺)m/z Calcd for C₂₈H₃₃Cl₂N₃O₃+H[(M+H)⁺]: 530.1972, found: 530.1971.

### Example 13

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid (1-hydroxymethyl-cyclopropylmethyl)-amide

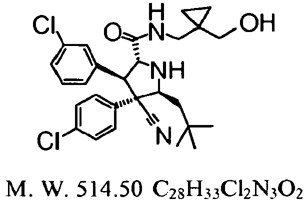

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (86.2 mg, 0.20 mmol) prepared in **Example 1d**, (1-aminomethyl-cyclopropyl)-methanol (30.3 mg, 0.3 mmol)), HATU (76.0 mg, 0.2 mmol) and iPr₂NEt (0.1 mL, 0.55 mmol) in CH₂Cl₂ (2 mL)) was stirred at rt overnight. The mixture was then diluted with CH₂Cl₂ and washed with water, brine. The organic phase was separated, filtered and dried over Na₂SO₄. The mixture was then concentrated and purified by reverse phase chromatography (20-95% of MeCN/water) to give rac-(2R,3R,4R,5S)-3-(3-chloro-phenyl)-4-(4-chloro-phenyl)-4-cyano-5-(2,2-dimethyl-propyl)-pyrrolidine-2-carboxylic acid (1-hydroxymethyl-cyclopropylmethyl)-amide (23.9 mg, 24.7 %) as a white powder.

### HRMS (ES⁺) m/z Calcd for C₂₈H₃₃Cl₂N₃O₂ + H [(M+H)⁺]: 514.2023, found: 514.2024.

**Example 14**

### Preparation of rac-(2R,3R,4R,5S)-3-(3-Chloro-phenyl)-4-(4-chloro-phenyl)-4-cyano-5-(2,2-dimethyl-propyl)-pyrrolidine-2-carboxylic acid (1-hydroxymethyl-cyclobutylmethyl)-amide

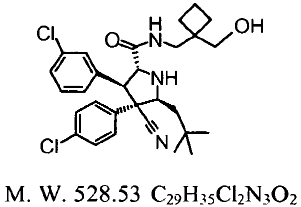

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (86.2 mg, 0.2 mmol) prepared in **Example 1d** was reacted with (1-aminomethyl-cyclobutyl)-methanol (34.5 mg, 0.3 mmol), HATU (76 mg, 0.2 mmol) and iPr₂NEt (0.1 mL, 0.55 mmol)) in CH₂Cl₂ (2 mL) at rt overnight to give rac-(2R,3R,4R,5S)-3-(3-chloro-phenyl)-4-(4-chloro-phenyl)-4-cyano-5-(2,2-dimethyl-propyl)-pyrrolidine-2-carboxylic acid (1-hydroxymethyl-cyclopropylmethyl)-amide (11.2 mg, 10.6 %).

HRMS (ES⁺) m/z Calcd for C₂₉H₃₅Cl₂N₃O₂ + H [(M+H)⁺]; 528.2179, found: 528.2179.

### Example 15

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid 4-tert-butyl benzylamide

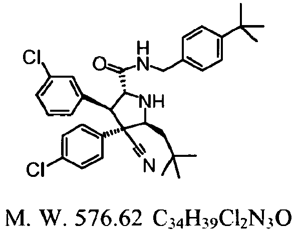

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (86.2 mg, 0.2 mmol) prepared in **Example 1d** was reacted with 4-tert-butylbenzylamine (48.98 mg, 0.3 mmol), HATU (76 mg, 0.2 mmol) and iPr₂NEt (0.1 mL, 0.55 mmol) in CH₂Cl₂ (2 mL) at rt overnight to give rac-(2R,3R,4R,5S)-3-(3-chloro-phenyl)-4-( 4-chloro-phenyl)-4-cyano-5-(2,2-dimethyl-propyl)-pyrrolidine-2-carboxylic acid 4-tert-butyl-benzylamide (41.8 mg, 36.25 %).

HRMS (ES') m/z Calcd for C₃₄H₃₉Cl₂N₃O+ H [(M+H)⁺]: 576.2543, found: 576.2541.

### Example 16

### Preparation of rac-(2R,3R,4R,5S)-3-(3-chloro-phenyl)-4-(4-chloro-phenyl)-4-cyano-5-(2,2-dimethyl-propyl) pyrrolidine-2-carboxylic acid (3,3-dimethyl-butyl)-amide

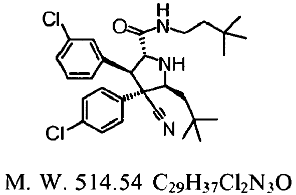

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (86.2 mg, 0.2 mmol) prepared in **Example 1d** was reacted with 3,3-dimethylbutylamine (30.36 mg, 0.3 mmol), HATU (76 mg, 0.2 mmol) and iPr₂NEt (0.1 mL, 0.55 mmol) in CH₂Cl₂ (2 mL) at rt overnight to give rac-(2R,3R,4R,5S)-3-(3-chloro-phenyl)-4-(4-chloro-phenyl)-4-cyano-5-(2,2-dimethyl-propyl) pyrrolidine-2-carboxylic acid (3,3-dimethyl-butyl)-amide (30.4 mg, 29.5 %).

HRMS (ES⁺) m/z Calcd for C₂₉H₃₇Cl₂N₃O+H [(M+H)⁺]: 514.2387, found: 514.2384.

### Example 17

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid (2,2-dimethyl-propyl)-amide

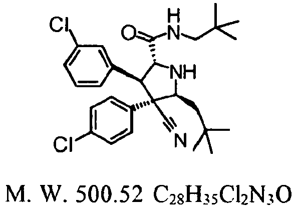

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (86.2 mg, 0.2 mmol) prepared in **Example 1d** was reacted with 2,2-dimethyl-propylamine (34.2 mg, 0.3 mmol), HATU (76 mg, 0.2 mmol) and iPr₂NEt (0.1 mL, 0.55 mmol) in CH₂Cl₂ (2 mL) at rt overnight to give rac-(2R,3R,4R,5S)-3-(3-chloro-phenyl)-4-(4-chloro-phenyl)-4-cyano-5-(2,2-dimethyl-propyl)-pyrrolidine-2-carboxylic acid (2,2-dimethyl-propyl)-amide (24.6 mg, 24.6%).

HRMS (ES⁺) m/z Calcd for C₂₈H₃₅Cl₂N₃O+ H [(M+H)⁺]: 500.2230, found: 500.2229.

### Example 18

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid (2,2,2-trifluoro-ethyl)-amide

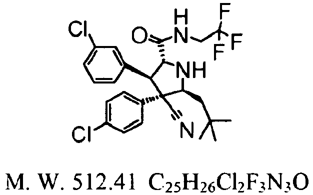

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (86.2 mg, 0.2 mmol) prepared in **Example 1d**, 2,2,2-trifluoroethylamine (29.7 mg, 0.3 mmol), HATU (76 mg, 0.2 mmol) and iPr₂NEt (0.1 mL, 0.55 mmol) in CH₂Cl₂ (2 mL) was stirred at rt overnight. The mixture was then diluted with CH₂Cl₂ and washed with water, brine. The organic phase was separated, filtered and dried over Na₂SO₄. The mixture was concentrated then purified by flash column (SiO₂, 1-20% of EtOAc in Heptane) to afford rac-(2R,3R,4R,5S)-3-(3-chloro-phenyl)-4-(4-chloro-phenyl)-4-cyano-5-(2,2-dimethyl-propyl)-pyrrolidine-2-carboxylic acid (2,2,2-trifluoro-ethyl)-amide (2,2-dimethyl-propyl)-amide (48.1 mg, 46.9 %).

HRMS (ES⁺) m/z Calcd for C₂₅H₂₆Cl₂F₃N₃O+ H [(M+H)⁺]: 512.1478, found: 512.14478.

### Example 19a

### Preparation of (2R,3S,4S,5S)-4-(3-chloro-phenyl)-3-(4-chloro-phenyl)-2-(2,2-dimethyl-propyl)-5-((S)-2-hydroxymethyl-pyrrolidine-1-carbonyl)-pyrrolidine-3-carbonitrile

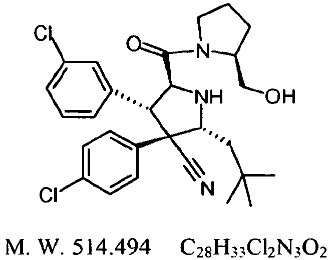

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (86.2 mg, 0.2 mmol) prepared in **Example 1d** was reacted with (S)-1-pyrrolidin-2-yl-methanol (30.3 mg, 0.3 mmol), HATU (76 mg, 0.2 mmol) and iPr₂NEt (0.1 mL, 0.55 mmol) in CH₂Cl₂ (2 mL) at rt overnight. The mixture was then diluted with CH₂Cl₂ and washed with water, brine. The organic phase was separated, filtered and dried over Na₂SO₄. The mixture was then concentrated and purified by reverse phase chromatography (30-95% of MeCN/water) to give (2R,3S,4S,5S)-4-(3-chloro-phenyl)-3-(4-chloro-phenyl)-2-(2,2-dimethyl-propyl)-5-((S)-2-hydroxymethyl-pyrrolidine-1-carbonyl)-pyrrolidine-3-carbonitrile (12.0 mg, 11.7 %).

HRMS (ES⁺) m/z Calcd for C₂₈H₃₃Cl₂N₃O₂ + H[(M+H)⁺]: 514.2023, found: 514.2023.

### Example 19b

### Preparation of (2S,3R,4R,5R)-4-(3-chloro-phenyl)-3-(4-chloro-phenyl)-2-(2,2-dimethyl-propyl)-5-((S)-2-hydroxymethyl-pyrrolidine-1-carbonyl) pyrrolidine-3-carbonitrile

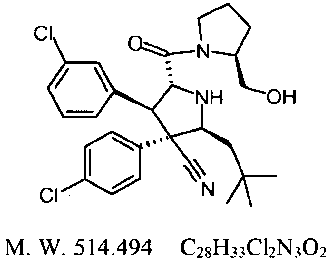

Reverse phase chromatography separation from the above example (**Example 19a**) gave (2S,3R,4R,5R)-4-(3-chloro-phenyl)-3-(4-chloro-phenyl)-2-(2,2-dimethyl-propyl)-5-((S)-2-hydroxymethyl-pyrrolidine-1-carbonyl) pyrrolidine-3-carbonitrile (18.1 mg, 17.6 %).

HRMS (ES⁺) m/z Calcd for C₂₈H₃₃Cl₂N₃O₂ + H [(M+H)⁺]: 514.2023, found: 514.2023.

### Example 20

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid [2-(3,4-dimethoxy-phenyl)-ethyl]-methyl-amide

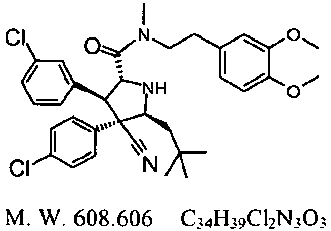

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (86.2 mg, 0.20 mmol) prepared in **Example 1d** was reacted with [2-(3,4-dimethoxy-phenyl)-ethyl]-methyl-amine (58.6 mg, 0.3 mmol), HATU (76.0 mg, 0.2 mmol) and iPr₂NEt (0.1 mL, 0.55 mmol) in CH₂Cl₂ (2 mL) to give rac-(2R,3R,4R,5S)-3-(3-chloro-phenyl)-4-(4-chloro-phenyl)-4-cyano-5-(2,2-dimethyl-propyl)-pyrrolidine-2-carboxylic acid [2-(3,4-dimethoxyphenyl)-ethyl]-methyl-amide (57.3 mg, 48.26 %) as a white powder.

HRMS (ES⁺) m/z Calcd for C₃₄H₃₉Cl₂N₃O₃ + H [(M+H)⁺]:608.2441, found: 608.2437.

### Example 21

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid 2-(3,4-dimethoxy-phenyl)ethyl amide

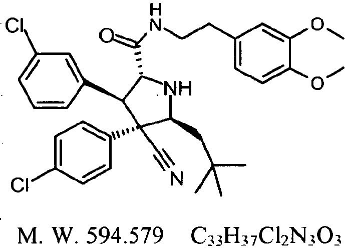

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (86.2 mg, 0.2 mmol) prepared in **Example 1d** was reached with 2-(3,4-dimethoxy-phenyl)ethyl amine (30.3 mg, 0.3 mmol), HATU (76 mg, 0.2 mmol) and iPr₂NEt (0.1 mL, 0.55 mmol) in CH₂Cl₂ (2 mL) to at rt overnight to give rac-(2R,3R,4R,5S)-3-(3-chloro-phenyl)-4-(4-chloro-phenyl)-4-cyano-5-(2,2-dimethyl-propyl)-pyrrolidine-2-carboxylic acid 2-(3,4-dimethoxyphenyl)ethyl amide (53.6 mg, 45.07 %).

HRMS (ES⁺)m/z Calcd for C₃₃H₃₇Cl₂N₃O₃ + H [(M+H)⁺]: 594.2285, found: 594.2283.

### Example 22

### Preparation of rac-(2R,3R,4R,55)-3-(3-chloro-phenyl)-4-(4-chloro-phenyl)-4-cyano-5-(2,2-dimethyl-propyl)-pyrrolidine-2-carboxylic acid 3-chloro-2-fluoro-benzylamide

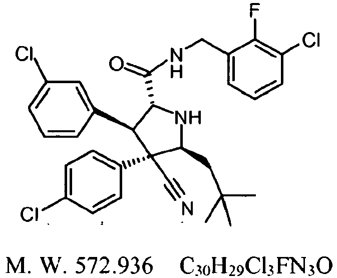

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (86.2 mg, 0.2 mmol) prepared in **Example 1d** was reacted with 3-chloro-2-fluoro-benzyl amine (47.9 mg, 0.3 mmol), HATU (76 mg, 0.2 mmol) and iPr₂NEt (0.1 mL, 0.55 mmol) in CH₂Cl₂ (2 mL) at rt overnight to rac-(2R,3R,4R,5S)-3-(3-chloro-phenyl)-4-(4-chloro-phenyl)-4-cyano-5-(2,2-dimethyl-propyl)-pyrrolidine-2-carboxylic acid 3-chloro-2-fluoro-benzylamide (24.5 mg, 21.4 %).

HRMS (ES⁺) m/z Calcd for C₃₀H₂₉Cl₃FN₃O + H [(M+H)⁺]: 572.1433, found: 572.1431.

### Example 23a

### Preparation of (2R,3R,4R,5S)-3-(3-chloro-phenyl)-4-(4-chloro-phenyl)-4-cyano-5-(2,2-dimethylpropyl)-pyrrolidine-2-carboxylic acid ((R)-1-hydroxymethyl-3-methyl-butyl)-amide

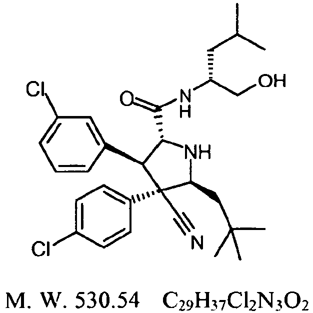

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (86.2 mg, 0.2 mmol) prepared in **Example 1d**, (R)-2-amino-4-methyl-pentan-1-ol (35.16 mg, 0.3 mmol), HATU (76 mg, 0.2 mmol) and iPr₂NEt (0.1 mL, 0.55 mmol) in CH₂Cl₂ (2 mL) was stirred at rt overnight. The mixture was then diluted with CH₂Cl₂ and washed with water, brine. The organic phase was separated, filtered and dried over Na₂SO₄. The mixture was then concentrated and purified by reverse phase chromatography (30-95% of MeCN/water) to give (2R,3R,4R,5S)-3-(3-chloro-phenyl)-4-(4-chloro-phenyl)-4-cyano-5-(2,2-dimethyl-propyl)-pyrrolidine-2-carboxylic acid ((R)-1-hydroxymethyl-3-methyl-butyl)-amide (26.2 mg, 24.7%). HRMS (ES⁺) m/z Calcd for C₂₉H₃₇Cl₂N₃O₂ + H [(M+H)⁺] 530.2336, found: 530.2333.

### Example 23b

### Preparation of (2S,3S,4S,5R)-3-(3-chloro-phenyl)-4-(4-chloro-phenyl)-4-cyano-5-(2,2-dimethylpropyl)-pyrrolidine-2-carboxylic acid ((R)-1-hydroxymethyl-3-methyl-butyl)-amide

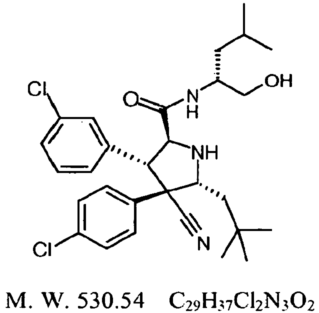

Reverse phase chromatography separation from the above example (**Example 23a**) gave (2S,3S,4S,5R)-3-(3-chloro-phenyl)-4-(4-chloro-phenyl)-4-cyano-5-(2,2-dimethyl-propyl)-pyrrolidine-2-carboxylic acid ((R)-1-hydroxymethyl-3-methyl-butyl)-amide (23.3 mg, 21.9 %). HRMS (ES⁺) m/z Calcd for C₂₉H₃₇Cl₂N₃O₂ + H [(M+H)⁺]: 530.2336, found: 530.2336.

### Example 24

### Preparation of rac-(2R,3R,4R,5S)-3-(3-chloro-phenyl)-4-(4-chloro-phenyl)-4-cyano-5-(2,2-dimethylpropyl)-pyrrolidine-2-carboxylic acid 3,4-difluoro-benzylamide

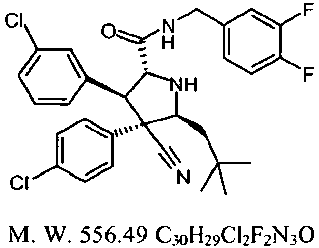

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (86.2 mg, 0.2 mmol) prepared in **Example 1d** was reacted with 3, 4-difluoro-benzyl amine (42.94 mg, 0.3 mmol), HATU (76 mg, 0.2 mmol) and iPr₂NEt (0.1 mL, 0.55 mmol) in CH₂Cl₂ (2 mL) at rt overnight to rac-(2R,3R,4R,5S)-3-(3-chloro-phenyl)-4-(4-chloro-phenyl)-4-cyano-5-(2,2-dimethyl-propyl)-pyrrolidine-2-carboxylic acid 3,4-difluoro-benzyl amide (53.5 mg, 48.1 %),

HRMS (ES⁺) m/z Calcd for C₃₀H₂₉Cl₂F₂N₃O+ H [(M+H)⁺]: 556.1729, found: 556.1728.

### Example 25a

### Preparation of intermediate rac-(2R,3R,4R,5S)-3-(3-chloro-phenyl)-4-(4-chloro-phenyl)-4-cyano-5-(2,2-dimethyl-propyl)-pyrrolidine-2-carboxylic acid trifluoroacetic acid

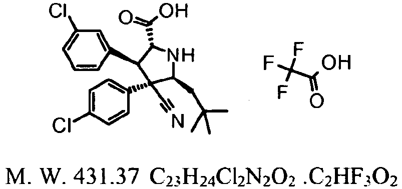

To a solution of rac-(2R,3R,4R,5S)-3-(3-chloro-phenyl)-4-(4-chloro-phenyl)-4-cyano-5-(2,2-dimethyl-propyl)-pyrrolidine-2-carboxylic acid tert-butyl ester prepared in **Example 1c** (2 g, 4.12 mmol) in dichloromethane (30 mL) was added trifluoroacetic acid (10 mL). The reaction mixture was stirred at room temperature for 18 h, and concentrated. The residue was then triturated with ethyl ether hexanes, concentrated, dried under reduced pressure to give rac-(2R,3R,4R,5S)-3-(3-chloro-phenyl)-4-(4-chloro-phenyl)-4-cyano-5-(2,2-dimethyl-propyl)-pyrrolidine-2-carboxylic acid trifluoroacetic acid as a white solid (2.1 g, 94%)

HRMS (ES⁺) m/z Calcd for C₂₃H₂₄Cl₂N₂O₂ + H [(M+H)⁺]: 431.1288, found: 431.1287.

### Example 25b

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid (3-hydroxy-propyl)-amide

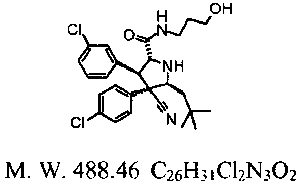

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid trifluoroacetic acid prepared in **Example 25a** (0.5 g, 1.1 mmol) was reacted with 3-amino-1-propanol (Aldrich) (0.4 g, 5.3 mmol), HATU (0.5 g, 1.31 mmol) and iPr₂NEt (1 g, 7.7 mmol) in CH₂Cl₂ (30 mL) at room temperature for 24 h to give rac-(2R,3R,4R,5S)-3-(3-chloro-phenyl)-4-(4-chloro-phenyl)-4-cyano-5-(2,2-dimethyl-propyl)-pyrrolidine-2-carboxylic acid (3-hydroxypropyl)-amide as a white solid (0.56 g, 93%).

HRMS (ES⁺) m/z Calcd for C₂₆H₃₁Cl₂N₃O₂ + H [(M+H)⁺]: 488.1866, found: 488.1864.

### Example 26a

### Preparation of intermediate (Z)-3-(3-chloro-2-fluoro-phenyl)-2-(4-chloro-phenyl)-acrylonitrile

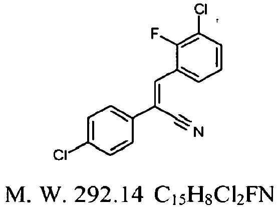

In a manner similar to the method described in **Example 1b**, 4-chlorobenzyl cyanide (8.9 g, 59 mmol) was reacted with 3-chloro-2-fluorobenzaldehyde (Oakwood) (10 g, 63 mmol), methanolic solution (25 wt%) of sodium methoxide (15 mL, 66 mmol) in methanol (300 mL) at 40 °C for 5h to give (Z)-3-(3-chloro-2-fluoro-phenyl)-2-(4-chloro-phenyl)-acrylonitrile as a white powder (16 g, 92%).

### Example 26b

### Preparation of intermediate rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid tert-butyl ester

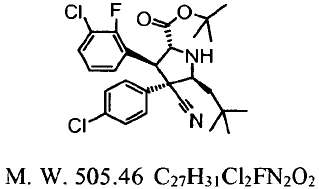

In a manner similar to the method described in **Example 1c**, [3,3-dimethyl-but-(E)-ylideneamino]-acetic acid tert-butyl ester prepared in **Example 1a** (2.1 g, 10 mmol) was reacted with (Z)-3-(3-chloro-2-fluoro-phenyl)-2-(4-chloro-phenyl)-acrylonitrile (2.3 g, 7.9 mmol) prepared in **Example 26a**, AgF (1.5 g, 12 mmol), and triethylamine (2 g, 20 mmol) in 1,2-dichloroethane (130 mL) at room temperature for 18 h to give rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid tert-butyl ester as a white foam (2.7 g, 68%).

### Example 26c

### Preparation of intermediate rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid trifluoroacetic acid

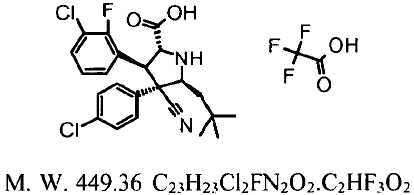

In a manner similar to the method described in **Example 25a**, rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid tert-butyl ester prepared in **Example 26b** (0.8 g, 1.6 mmol) was reacted with trifluoroacetic acid in dichloromethane at room temperature to give rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid trifluoroacetic acid as a white solid (0.9 g, 100%).

HRMS (ES⁺) m/z Calcd for C₂₃H₂₃Cl₂FN₂O₂+ H [(M+H)⁺]: 449.1194. found: 449.1194.

### Example 26d

### Preparation of rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid [2-(cis-2,6-dimethyl-morpholin-4-yl)-ethyl]-amide

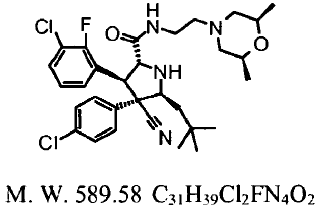

In a manner similar to the method described in **Example 1e**, rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid trifluoroacetic acid prepared in **Example 26c** (0.20 g, 0.36 mmol) was reacted with 4-(2-aminoethyl)-cis-2,6-dimethylmorpholine (Oakwood) (0.20 g, 1.2 mmol), HATU (0.3 g, 0.78 mmol) and iPr₂NEt (0.60 g, 4.6 mmol) in CH₂Cl₂ (20 mL) at room temperature for 20 hrs to give rac-(2R,3S,4R,5S)-3-(3-chloro-2-fluoro-phenyl)-4-(4-chloro-phenyl)-4-cyano-5-(2,2-dimethylpropyl)-pyrro lidine-2-carboxylic acid [2-(cis-2,6-dimethyl-morpholin-4-yl)-ethyl]-amide as a white solid (0.20 g, 94%). HRMS (ES⁺) m/z Calcd for C₃₁H₃₉Cl₂FN₄O₂ + H [(M+H)⁺]: 589.2507, found: 589.2507.

### Example 27

### Preparation of rac-(2R,3R,4R,55)-3-(3-chloro-phenyl)-4-(4-chloro-phenyl)-4-cyano-5-(2,2-dimethyl-propyl)-pyrrolidine-2-carboxylic acid (2-cyclopropyl-ethyl)-amide

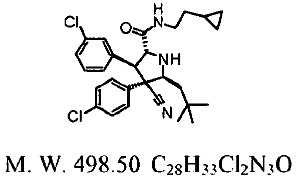

In a manner similar to the method described in **Example 1e**, rac-(2R,3R,4R,SS)-3-(3-chloro-phenyl)-4-(4-chloro-phenyl)-4-cyano-5-(2,2-dimethyl-propyl)-pyrrolidine-2-carboxylic acid trifluoroacetic acid prepared in **Example 25a** (0.16 g, 0.37 mmol) was reacted with 2-cyclopropylethylamine (Bridge Organics) (0.1 g, 1.1 mmol), HATU (0.2 g, 0.5 mmol) and iPr₂NEt (0.3 g, 2 mmol) in CH₂Cl₂ (20 mL) at room temperature for 20 h to give rac-(2R,3R,4R,5S)-3-(3-chloro-phenyl)-4-(4-chloro-phenyl)-4-cyano-5-(2,2-dimethyl-propyl)-pyrrolidine-2-carboxylic acid (2-cyclopropyl-ethyl)-amide as a white solid (0.11 g, 37%). HRMS (ES⁺) m/z Calcd for C₂₈H₃₃Cl₂N₃O + H [(M+H)⁺]: 498.2074, found: 498.2075.

### Example 28

### Preparation of rac-(3- {[(2R,3R,4R,5S)-3-(3-chloro-phenyl)-4-(4-chloro-phenyl)-4-cyano-5-(2,2-dimethyl-propyl)-pyrrolidine-2-carbonyl]-amino]-propyl)-carbamic acid tert-butyl ester

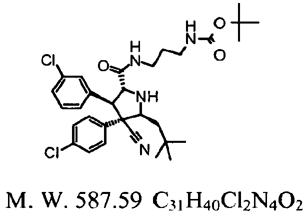

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid prepared in **Example 25a** (1 g, 1.8 mmol) was reacted with N-Boc-1,3-diaminopropane (Aldrich) (0.7 g, 4 mmol), HATU (1.4 g, 3.7 mmol) and iPr₂NEt (2.8 g, 21 mmol) in CH₂Cl₂ (100 mL) at room temperature for 60 h to give rac-(3-{[(2R,3R,4R,5S)-3-(3-chloro-phenyl)-4-(4-chloro-phenyl)-4-cyano-5-(2,2-dimethyl-propyl)-pyrrolidine-2-carbonyl]-amino}-propyl)-carbamic acid tert-butyl ester as a white solid (0.92 g, 87%).

HRMS (ES⁺) m/z Calcd for C₃₁H₄₀Cl₂N₄O₂ + H [(M+H)⁺]: 587.2550, found: 587.2551.

### Example 29

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid (3-amino-propyl)-amide

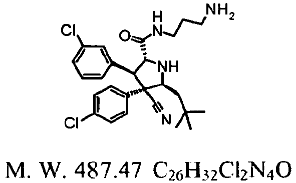

To a solution of rac-(3-1[(2R,3R,4R,5S)-3-(3-chloro-phenyl)-4-(4-chloro-phenyl)-4-cyano-5-(2,2-dimethyl-propyl)-pyrrolidine-2-carbonyl]-amino}-propyl)-carbamic acid tert-butyl ester prepared in **Example 28** (0.9 g, 1.5 mmol) in dichloromethane (30 mL) was added trifluoroacetic acid (5 mL). The reaction mixture was stirred at room temperature for 1 h, and concentrated. The residue was then neutralized with saturated aqueous NaHCO₃ solution, and then extracted with ethyl acetate. The organic layer was separated, dried over MgSO₄, concentrated, dried under reduced pressure to give rac-(2R,3R,4R,5S)-3-(3-chloro-phenyl)-4-(4-chloro-phenyl)-4-cyano-5-(2,2-dimethyl-propyl)-pyrrolidine-2-carboxylic acid (3)-amino-propyl)-amide as a white solid (0.8 g, 100%)

HRMS (ES⁺) m/z Calcd for C₂₆H₃₂Cl₂N₄O+H [(M+H)⁺]: 487.2026, found: 487.2027.

### Example 30

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid [3-(1-acetyl-piperidin-4-ylamino)-propyl]-amide

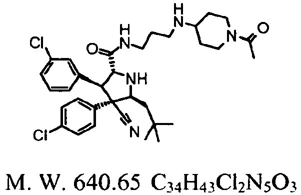

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (3-amino-propyl)-amide prepared in **Example 29** (0.18 g, 0.37 mmol) was reacted with 1-acetylpiperidine-4-carboxylic acid (Lancaster) (0.7 g, 0.58 mmol), HATU (0.3 g, 0.78 mmol) and iPr₂NEt (0.5 g, 3.9 mmol) in CH₂Cl₂ (20 mL) at room temperature for 20 h to give rac-(2R,3R,4R,5S)-3-(3-chloro-phenyl)-4-(4-chloro-phenyl)-4-cyano-5-(2,2-dimethyl-propyl)-pyrrolidine-2-carboxylic acid [3-(1-acetyl-piperidin-4-ylamino)-propyl]-amide as a white solid (0.16g, 67%).

HRMS (ES⁺) m/z Calcd for C₃₄H₄₃Cl₂N₅O₃ + H [(M+H)⁺]:640.2816, found: 640.2818.

### Example 31a

### Preparation of intermediate [3-Methyl-but-(E)-ylideneamino]-acetic acid tert-butyl ester

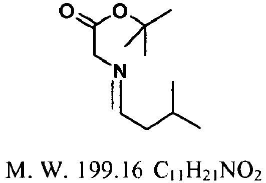

In a manner similar to the method described in **Example 1a,** glycine tert-butyl ester (0.65 g, 5 mmol) was reacted with isovaleraldehyde (Alfa) (0.43 g, 5 mmol) in CH₂Cl₂ at room temperature for 18 h to give [3-methyl-but-(E)-ylideneamino]-acetic acid tert-butyl ester as a colorless oil (0.98 g, 98%).

### Example 31b

### Preparation of intermediate rac-(2R,3R,4R,5S)-3-(3-chloro-phenyl)-4-(4-chloro-phenyl)-4-cyano-5-isobutyl-pyrrolidine-2-carboxylic acid tert-butyl ester

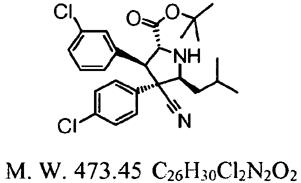

In a manner similar to the method described in **Example 1c**, [3-methyl-but-(E)-ylideneamino]-acetic acid tert-butyl ester prepared in **Example 31a** (2 g, 10 mmol) was reacted with (Z)-3-(3-chloro-phenyl)-2-(4-chloro-phenyl)-acrylonitrile (2 g, 7.3 mmol) prepared in **Example 1b**, AgF (1.3 g, 10 mmol), and triethylamine (2 g, 20 mmol) in dichloromethane (100 mL) at room temperature for 18 h to give rac-(2R,3R,4R,5S)-3-(3-chloro-phenyl)-4-(4-chloro-phenyl)-4-cyano-5-isobutyl-pyrrolidine-2-carboxylic acid tert-butyl ester as a white foam (0.7 g, 20%).

### Example 31c

### Preparation of intermediate rac-(2R,3R,4R,5S)-3-(3-chloro-phenyl)-4-(4-chloro-phenyl)-4-cyano-5-isobutyl-pyrrolidine-2-carboxylic acid trifluoroacetic acid

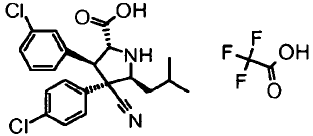

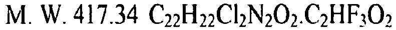

In a manner similar to the method described in **Example 25a**, rac-(2R,3R,4R,5S)-3-(3-chloro-phenyl)-4-(4-chloro-phenyl)-4-cyano-5-isobutyl-pyrrolidine-2-carboxylic acid tert-butyl ester prepared in **Example 31b** (0.4 g, 0.85 mmol) was reacted with trifluoroacetic acid in dichloromethane at room temperature to give rac-(2R,3R,4R,5S)-3-(3-chloro-phenyl)-4-(4-chloro-phenyl)-4-cyano-5-isobutyl-pyrrolidine-2-carboxylic acid trifluoroacetic acid as a off white solid (0.4 g, 89%).

HRMS (ES⁺) m/z Calcd for C₂₂H₂₂Cl₂N₂O₂+ H [(M+H)⁺]: 417.1131, found: 417.1131.

### Example 31d

### Preparation of rac-(2R,3R,4R,5S)-3-(3-chloro-phenyl)-4-(4-chloro-phenyl)-4-cyano-5-isobutyl-pyrrolidine-2-carboxylic acid (3-hydroxy-propyl)-amide

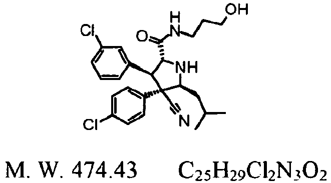

In a manner similar to the method described in **Example 1e**, rac-(2R,3R,4R,5S)-3-(3-chloro-phenyl)-4-(4-chloro-phenyl)-4-cyano-5-isobutyl-pyrrolidine-2-carboxylic acid trifluoroacetic acid prepared in **Example 31c** (0.6 g, 1.1 mmol) was reacted with 3-amino-1-propanol (Aldrich) (0.4 g, 5.3 mmol), HATU and iPr₂NEt in CH₂Cl₂ at room temperature to give rac-(2R,3R,4R,5S)-3-(3-chloro-phenyl)-4-(4-chloro-phenyl)-4-cyano-5-isobutyl-pyrrolidine-2-carboxylic acid (3-hydroxy-propyl)-amide as a white solid (0.21 g, 40%).

HRMS (ES⁺) m/z Calcd for C₂₅H₂₉Cl₂N₃O₂ + H [(M+H)⁺]: 474.1710, found: 474.1710.

### Example 32a

### Preparation of intermediate {[1-(3-Chloro-phenyl)-meth-(E)-ylidene]-amino}-acetic acid tert-butyl ester

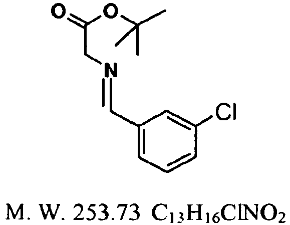

In a manner similar to the method described in **Example 1a,** glycine tert-butyl ester (1.31 g, 10 mmol) was reacted with 3-chlorobenzaldehyde (Aldrich) (1.4 g, 10) mmol) in CH₂Cl₂ at room temperature for 18 h to give {[1-(3-chloro-phenyl)-meth-(E)-ylidene]-amino}-acetic acid tert-butyl ester as a plae yellow oil (2.4 g, 95%).

### Example 32b

### Preparation of intermediate (Z)-2-(4-chloro-phenyl)-5,5-dimethyl-hex-2-enenitrile

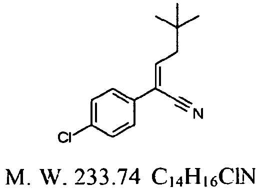

In a manner similar to the method described in **Example 1b**, 4-chlorobenzyl cyanide (4.5 g, 30 mmol) was reacted with 3,3-dimethyl-butyraldehyde (Aldrich) (3 g, 30 mmol), methanolic solution (25 wt%) of sodium methoxide (7 mL, 30 mmol) in methanol (130 mL) at room temperature for 3 h to give (Z)-2-(4-chloro-phenyl)-5,5-dimethyl-hex-2-enenitrile as a colorless oil (5 g, 71%).

### Example 32c

### Preparation of intermediate rac-(2R,3R,4R,5S)-5-(3-Chloro-phenyl)-4-(4-chloro-phenyl)-4-cyano-3-(2,2-dimethyl-propyl)-pyrrolidine-2-carboxylic acid tert-butyl ester

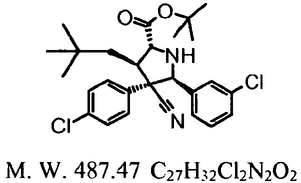

In a manner similar to the method described in **Example 1c**, {[1-(3-chloro-phenyl)-meth-(E)-ylidene]-amino}-acetic acid tert-butyl ester prepared in **Example 32a** (2.6 g, 11 mmol) was reacted with (Z)-2-(4-chloro-phenyl)-5,5-dimethyl-hex-2-enenitrile (2 g, 7.9 mmol) prepared in **Example 32b**, AgF (1.3 g, 10 mmol), and triethylamine (2.2 g, 22 mmol) in 1,2-dichloroethane (100 mL) at room temperature for 24 h to give rac-(2R,3R,4R,5S)-5-(3-Chloro-phenyl)-4-(4-chloro-phenyl)-4-cyano-3-(2,2-dimethyl-propyl)-pyrrolidine-2-carboxylic acid tert-butyl ester as a white foam (1.2 g, 31 %).

HRMS (ES⁺) m/z Calcd for C₂₇H₃₂Cl₂N₂O₂+ H [(M+H)⁺]: 487.1914, found: 487.1912.

### Example 32d

### Preparation of intermediate rac-(2R,3R,4R,5S)-5-(3-Chloro-phenyl)-4-(4-chloro-phenyl)-4-cyano-3-(2,2-dimethyl-propyl)-pyrrolidine-2-carboxylic acid trifluoroacetic acid

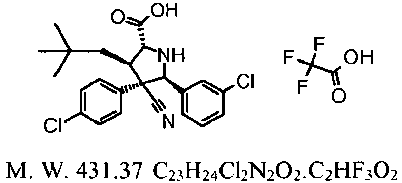

In a manner similar to the method described in **Example 25a**, rac-(2R,3R,4R,5S)-5-(3-Chloro-phenyl)-4-(4-chloro-phenyl)-4-cyano-3-(2,2-dimethyl-propyl)-pyrrolidine-2-carboxylic acid tert-butyl ester prepared in **Example 32c** (1.2 g, 2.5 mmol) was reacted with trifluoroacetic acid in dichloromethane at room temperature to give rac-(2R,3R,4R,5S)-5-(3-Chloro-phenyl)-4-(4-chloro-phenyl)-4-cyano-3-(2,2-dimethyl-propyl)-pyrrolidine-2-carboxylic acid trifluoroacetic acid as a yellow solid (1.0 g, 76%).

HRMS (ES⁺) m/z Calcd for C₂₃H₂₄Cl₂N₂O₂+ H [(M+H)⁺]: 431.1288, found: 431.1288.

### Example 32e

### Preparation of rac-(2R,3R,4R,5R)-5-(3-chloro-phenyl)-4-(4-chloro-phenyl)-4-cyano-3-(2,2-dimethyl-propyl)-pyrrolidine-2-carboxylic acid (3-hydroxy-propyl)-amide

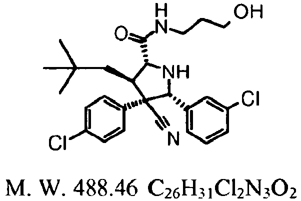

In a manner similar to the method described in **Example 1e**, rac-(2R,3R,4R,5R)-5-(3-chloro-phenyl)-4-(4-chloro-phenyl)-4-cyano-3-(2,2-dimethyl-propyl)-pyrrolidine-2-carboxylic acid trifluoroacetic acid prepared in **Example 32d** (0.6 g, 1.1 mmol) was reacted with 3-amino-1-propanol (Aldrich) (0.6 g, 8 mmol), HATU and iPr₂NEt in CH₂Cl₂ at room temperature to give rac-(2R,3R,4R,5R)-5-(3-chloro-phenyl)-4-(4-chloro-phenyl)-4-cyano-3-(2,2-dimethyl-propyl)-pyrrolidine-2-carboxylic acid (3-hydroxy-propyl)-amide as a yellow solid (0.12 g, 22%). HRMS (ES⁺) m/z Calcd for C₂₆H₃₁Cl₂N₃O₂+ H [(M+H)⁺]: 488.1866, found: 488.1864.

### Example 33a

### Preparation of intermediate (S)-2-[3,3-Dimethyl-but-(E)-ylideneamino]-propionic acid tert-butyl ester

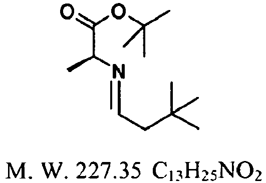

A mixture of L-alanine tert-butyl ester hydrochloride (Bachem) (1.8 g, 10 mmol) and MgSO₄ in CH₂Cl₂ (100 mL) was added triethylamine (1.5 g, 15 mmol). The mixture was stirred at room temperature for 1 h, and 3,3-dimethyl-butyraldehyde (1g, 10 mmol) was added. The reaction mixture was stirred at room temperature for 18 h. The mixture was filtered, and the filtrate was washed with water, brine, and concentrated. The residue was dried under reduced pressure to give (S)-2-[3,3-dimethyl-but-(E)-ylideneaminol-propionic acid tert-butyl ester as colorless oil (2,3 g, 100%) which was used without further purification.

### Example 33b

### Preparation of intermediate rac-(2R,3R,4R,5S)-3-(3-Chloro-phenyl)-4-(4-chloro-phenyl)-4-cyano-5-(2,2-dimethyl-propyl)-2-methyl-pyrrolidine-2-carboxylic acid tert-butyl ester

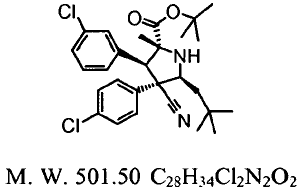

In a manner similar to the method described in **Example 1c**, (S)-2-[3,3-dimethyl-but-(E)-ylideneamino]-propionic acid tert-butyl ester prepared in **Example 33a** (2.4 g, 11 mmol) was reacted with (Z)-3-(3-chloro-phenyl)-2-(4-chloro-phenyl)-acrylonitrile (2.4 g, 8.8 mmol) prepared in **Example 1b**, AgF (1.6 g, 13 mmol), and triethylamine (2.4 g, 24 mmol) in 1,2-dichloroethane (150 mL) at room temperature for 20 h to give rac-(2R,3R,4R,5S)-3-(3-Chloro-phenyl)-4-(4-chloro-phenyl)-4-cyano-5-(2,2-dimethyl-propyl)-2-methyl-pyrrolidine-2-carboxylic acid tert-butyl ester as a white foam (2.4 g, 54%).

HRMS (ES⁺) m/z Calcd for C₂₈H₃₄Cl₂N₂O₂+ H [(M+H)⁺]: 511.2070, found: 501.2066.

### Example 33c

### Preparation of intermediate rac-(2R,3R,4R,5S)-3-(3-chloro-phenyl)-4-(4-chloro-phenyl)-4-cyano-5-(2,2-dimethyl-propyl)-2-methyl-pyrrolidine-2-carboxylic acid trifluoroacetic acid

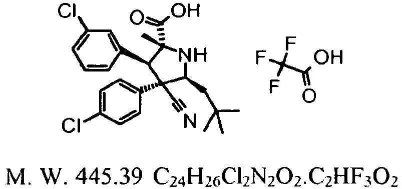

In a manner similar to the method described in **Example 25a**, rac-(2R,3R,4R,5S)-3-(3-chloro-phenyl)-4-(4-chloro-phenyl)-4-cyano-5-(2,2-dimethyl-propyl)-2-methyl-pyrrolidine-2-carboxylic acid tert-butyl ester prepared in **Example 33b** (1g, 2mmol) was reacted with trifluoroacetic acid in dichloromethane at room temperature to give rac-(2R,3R,4R,5S)-3-(3-chloro-phenyl)-4-(4-chloro-phenyl)-4-cyano-5-(2,2-dimethyl-propyl)-2-methyl-pyrrolidine-2-carboxylic acid trifluoroacetic acid as a white solid (1.1 g, 98%).

HRMS (ES⁺) m/z Calcd for C₂₄H₂₆Cl₂N₂O₂+ H [(M+H)⁺]: 445.1444, found: 445.1443.

### Example 33d

### Preparation of rac-(2R,3R,4R,5S)-3-(3-chloro-phenyl)-4-(4-chloro-phenyl)-4-cyano-5-(2,2-dimethyl-propyl)-2-methyl-pyrrolidine-2-carboxylic acid (3-hydroxy-propyl)-amide

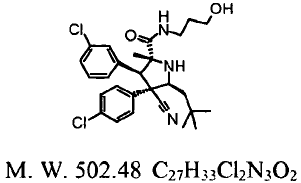

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-2-methyl-pyrrolidine-2-carboxylic acid trifluoroacetic acid prepared in **Example 33c** (0.4 g, 0.7 mmol) was reacted with 3-amino-1-propanol (Aldrich) (0.4 g, 5.3 mmol), HATU (0.5 g, 1.3 mmol) and iPr₂NEt (1 g, 7.7 mmol) in CH₂Cl₂ (30 mL) at room temperature for 24 h to give rac-(2R,3R,4R,5S)-3-(3-chloro-phenyl)-4-(4-chloro-phenyl)-4-cyano-5-(2,2-dimethyl-propyl)-2-methyl-pyrrolidine-2-carboxylic acid (3-hydroxy-propyl)-amide as a white solid (0.21 g, 60%).

HRMS (ES⁺) m/2 Calcd for C₂₇H₃₃Cl₂N₃O₂ + H [(M+H)⁺]: 502.2023, found: 502.2021.

### Example 34a

### Preparation of intermediate [2-Cyclopentyl-eth-(E)-ylideneamino]-acetic acid tert-butyl ester

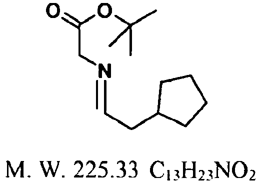

In a manner similar to the method described in **Example 1a**, glycine tert-butyl ester (0.7 g, 5 mmol) was reacted with 2-cyclopentylacetaldehyde (Betapharma) (0.9 g, 8 mmol,) in CH₂Cl₂ at room temperature for 20 h to give [2-cyclopentyl-eth-(E)-ylideneamino]-acetic acid tert-butyl ester as a colorless oil (1 g, 90%).

### Example 34b

### Preparation of intermediate rac-(2R,3S,4R,5S)-3-(3-chloro-2-fluoro-phenyl)-4-(4-chloro-phenyl)-4-cyano-5-cyclopentylmethyl-pyrrolidine-2-arboxylic acid tert-butyl ester

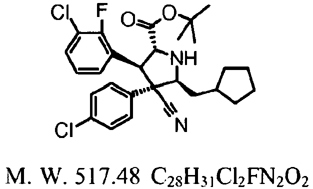

In a manner similar to the method described in **Example 1e**, [2-cyclopentyl-eth-(E)-ylideneamino]-acetic acid tert-butyl ester prepared in **Example 34a** (1 g, 4.4 mmol) was reacted with (Z)-3-(3-chloro-2-fluoro-phenyl)-2-(4-chloro-phenyl)-acrylonitrile (0.9 g, 3 mmol) prepared in **Example 26a**, AgF (1.3 g, 10 mmol), and triethylamine (2 g, 20 mmol) in dichloromethane (150 mL) at room temperature for 24 h to give rac-(2R,3S,4R,5S)-3-(3-chloro-2-fluoro-phenyl)-4-(4-chloro-phenyl)-4-cyano-5-cyclopentylmethyl-pyrrolidine-2-arboxylic acid tert-butyl ester as a white foam (0.4 g, 26%).

### Example 34c

### Preparation of intermediate rac-(2R,3S,4R,5S)-3-(3-chloro-2-fluoro-phenyl)-4-(4-chloro-phenyl)-4-cyano-5-cyclopentylmethyl-pyrrolidine-2-arboxylic acid trifluoroacetic acid

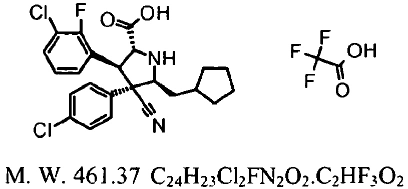

In a manner similar to the method described in **Example 25a**, rac-(2R,3S,4R,5S)-3-(3-chloro-2-fluoro-phenyl)-4-(4-chloro-phenyl)-4-cyano-5-cyclopentylmethyl-pyrrolidine-2-arboxylic acid tert-butyl ester prepared in **Example 34b** (0.4 g, 0.77 mmol) was reacted with trifluoroacetic acid in dichloromethane at room temperature to give rac-(2R,3S,4R,5S)-3-(3-chloro-2-fluoro-phenyl)-4-(4-chloro-phenyl)-4-cyano-5-cyclopentylmethyl-pyrrolidine-2-arboxylic acid trifluoroacetic acid as a off white solid (0.5 g, 100%).

### Example 34d

### Preparation of rac-(2R,35,4R,5S)-3-(3-chloro-2-fluoro-phenyl)-4-(4-chloro-phenyl)-4-cyano-5-cyclopentylmethyl-pyrrolidine-2-carboxylic acid ((S)-3,4-dihydroxy-butyl)-amide

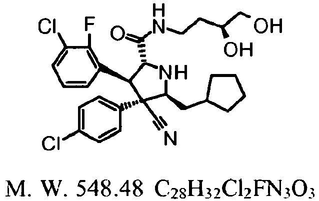

In a manner similar to the method described in **Example 3b**, rac-(2R,3S,4R,5S)-3-(3-chloro-2-fluoro-phenyl)-4-(4-chloro-phenyl)-4-cyano-5-cyclopentylmethyl-pyrrolidine-2-arboxylic acid trifluoroacetic acid prepared in **Example 34c** (0.4 g, 0.71 mmol) was reacted with 2-((S)-2,2-dimethyl-[1,3]dioxolan-4-yl)-ethylamine (0.2 g, 1.4 mmol), HATU (0.4 g, 1.1 mmol) and iPr₂NEt (0.6 g, 4.7 mmol) in CH₂Cl₂ at room temperature for 20 h, then reacted with aqueous HCl solution in tetrahydrofuran at room temperature for 2 h to give rac-(2R,3S,4R,5S)-3-(3-chloro-2-fluoro-phenyl)-4-(4-chloro-phenyl)-4-cyano-5-cyclopentylmethyl-pyrrolidine-2-carboxylic acid ((S)-3,4-dihydroxy-butyl)-amide as a white solid (0.14 g, 36%).

HRMS (ES⁺) m/z Calcd for C₂₈H₃₂Cl₂FN₃O₃+ H [(M+H)⁺]:548.1878, found: 548.1880.

### Example 35

### Preparation of rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid (2-hydroxy-1,1-dimethyl-ethyl)-amide

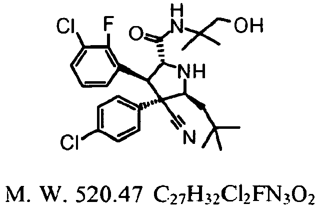

In a manner similar to the method described in **Example 1e**, rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid trifluoroacetic acid prepared in **Example 26c** (0.2 g, 0.36 mmol) was reacted with 2-amino-2-methyl-1-propanol (Fluka) (0.2 g, 2.2mmol), HATU (0.3 g, 0.78 mmol) and iPr₂NEt (0.5 g, 3.8 mmol) in CH₂Cl₂ (10 mL) at room temperature for 24 h to give rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid (2-hydroxy-1,1-dimethyl-ethyl)-amide as a white solid (0.17 g, 91%).

HRMS (ES⁺) m/z Calcd for C₂₇H₃₂Cl₂FN₃O₂+ H [(M+H)⁺]: 520.1929, found: 590.1929.

### Example 36

### Preparation of rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid (3-hydroxy-2,2-dimethyl-propyl)-amide

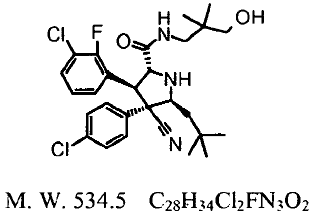

In a manner similar to the method described in **Example 1e**, rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid trifluoroacetic acid prepared in **Example 26c** (0.2 g, 0.36 mmol) was reacted with 3-amino-2,2-dimethyl-1-propanol (TCI-US) (0.2 g, 2 mmol), HATU (0.2 g, 0.5 mmol) and iPr₂NEt (0.2 g, 1.6 mmol) in CH₂Cl₂ (20 mL) at room temperature for 20 h to give rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid (3-hydroxy-2,2-dimethyl-propyl)-amide as a white solid (0.16 g, 83%).

HRMS (ES⁺) m/z Calcd for C₂₈H₃₄Cl₂FN₃O2+ H [M+H)⁺]: 534.2085, found: 534.2084.

### Example 37

### Preparation of rac-(2R,35,4R,5S)-3-(3-chloro-2-fluoro-phenyl)-4-(4-chloro-phenyl)-4-cyano-5-(2,2-dimethyl-propyl)-pyrrolidine-2-carboxylic acid [2-(2-hydroxy-ethoxy)-ethyl]-amide

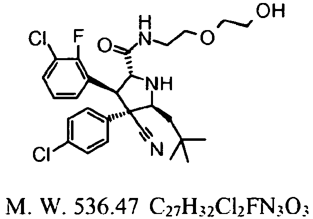

In a manner similar to the method described in **Example 1e**, rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid trifluoroacetic acid prepared in **Example 26c** (0.3 g, 0.54 mmol) was reacted with 2-(2-aminoethyl)ethanol (Aldrich) (0.15 g, 1.4 mmol), HATU (0.3 g, 0.75 mmol) and iPr₂NEt (0.6 g, 4.8 mmol) in CH₂Cl₂ (20 mL) at room temperature for 24 h to give rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid [2-(2-hydroxy-ethoxy)-ethyl]-amide as a white solid (0.18 g, 62%).

HRMS (ES⁺) m/z Calcd for C₂₇H₃₂Cl₂FN₃O₃+ H [(M+H)⁺]: 536.1878, found; 536.1877.

### Example 38

### Preparation of rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid (2-acetylamino-ethyl)-amide

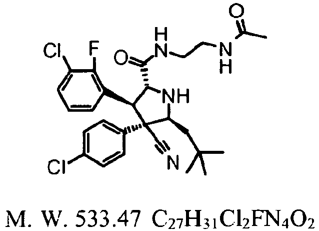

In a manner similar to the method described in **Example 1e**, rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid trifluoroacetic acid prepared in **Example 26c** (0.3 g, 0.54 mmol) was reacted with N-acetylethylenediamine (Aldrich) (0.15 g, 1.5 mmol), HATU (0.3 g, 0.75 mmol) and iPr₂NEt (0.6 g, 4.8 mmol) in CH₂Cl₂ (20 mL) at room temperature for 24 h to give rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid (2-acetylamino-ethyl)-amide as a white solid (0.24 g, 83%).

HRMS (ES⁺) m/z Calcd for C₂₇H₃₁Cl₂FN₄O₂+ H [(M+H)⁺]: 533.1881, found: 533.1882.

### Example 39

### Preparation of rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid (3-imidazol-1-yl-propyl)-amide

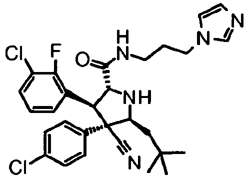

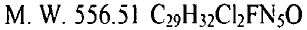

In a manner similar to the method described in **Example 1e**, rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid trifluoroacetic acid prepared in **Example 26c** (0.2 g, 0.36 mmol) was reacted with 1-(3-aminopropopyl)imidazole (Aldrich) (0.15 g, 1.2 mmol), HATU (0.3 g, 0.75 mmol) and iPr₂NEt (0.5 g, 3.6 mmol) in CH₂Cl₂ (20 mL) at room temperature for 24 h to give rac-(2R,3S,4R,5S)-3-(3-chloro-2-fluoro-phenyl)-4-(4-chloro-phenyl)-4-cyano-5-(2,2-dimethyl-propyl)-pyrro lid ine-2-carboxylic acid (3-imidazol-1-yl-propyl)-amide as a white solid (0.19 g, 94%).

HRMS (ES⁺) m/z Calcd for C₂₉H₃₂Cl₂FN₅O + H [(M+H)⁺]: 556.2041, found: 556.2040.

### Example 40

### Preparation of rac-(2R,35,4R,5S)-3-(3-chloro-2-fluoro-phenyl)-4-(4-chloro-phenyl)-4-cyano-5-(2,2-dimethyl-propyl)-pyrrolidine-2-carboxylic acid ((R)-4-hydroxy-3-methyl-butyl)-amide

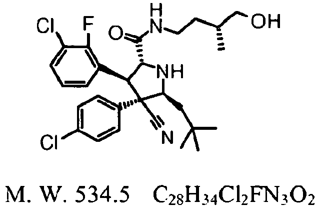

In a manner similar to the method described in **Example 1e**, rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid trifluoroacetic acid prepared in **Example 26c** (0.16 g, 0.29 mmol) was reacted with (R)-4-amino-2-methyl-I-butanol (TCI-US) (0.1 g, 1 mmol), HATU (0.2 g, 0.5 mmol) and iPr₂NEt (0.3 g, 2 mmol) in CH₂Cl₂ (20 mL) at room temperature for 20 h to give rac-(2R,3S,4R,5S)-3-(3-chloro-2-fluoro-phenyl)-4-(4-chloro-phenyl)-4-cyano-5-(2,2-dimethyl-propyl)-pyrro lidine-2-carboxylic acid ((R)-4-hydroxy-3-methyl-butyl)-amide as a white solid (0.1 g, 65%).

HRMS (ES⁺) m/z Calcd for C₂₈H₃₄Cl₂FN₃O₂+ H [(M+H)⁺]: 534.2085, found: 534.2084.

### Example 41

### Preparation of rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid cyclopropylmethoxy-amide

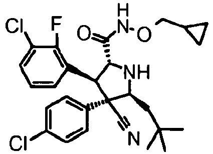

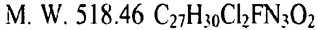

In a manner similar to the method described in **Example 1e**, rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid trifluoroacetic acid prepared in **Example 26c** (0.15 g, 0.27 mmol) was reacted with O-cyclopropylmethylhydroxyamine (HUHU Tech) (0.1 g, 1.1 mmol), HATU (0.2 g, 0.5 mmol) and iPr₂NEt (0.3 g, 2 mmol) in CH₂Cl₂ (20 mL) at room temperature for 20 h to give rac-(2R,3S,4R,5S)-3-(3-chloro-2-fluoro-phenyl)-4-(4-chloro-phenyl)-4-cyano-5-(2,2-dimethylpropyl)-pyrrolidine-2-carboxylic acid cyclopropylmethoxy-amide as a white solid (30 mg, 21%). HRMS (ES⁺) m/z Calcd for C₂₇H₃₀Cl₂FN₃O₂+H [(M+H)⁺]: 518.17 found: 518.1773.

### Example 42a

### Preparation of intermediate rac-(2R,3R,4R,5S)-3,5-bis-(3-chloro-phenyl)-4-(4-chloro-phenyl)-4-cyano-pyrrolidine-2-carboxylic acid tert-butyl ester

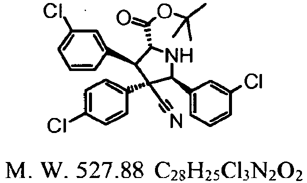

In a manner similar to the method described in **Example 1c**, {[1-(3-Chloro-phenyl)-meth-(E)-ylidene]-amino}-acetic acid tert-butyl ester prepared in **Example 32a** (2 g, 7.6 mmol) was reacted with (Z)-3-(3-chloro-phenyl)-2-(4-chloro-phenyl)-acrylonitrile (0.55 g, 2 mmol) prepared in **Example 1b**, AgF (1.3 g, 10 mmol), and triethylamine (1.9 g, 19 mmol)) in dichloromethane (30 mL) at 50 °C for 20 h to give rac-(2R,3R,4R,5S)-3,5-bis-(3-chloro-phenyl)-4-(4-chloro-phenyl)-4-cyano-pyrrolidine-2-carboxylic acid tert-butyl ester as a white foam (0.45 g, 44%). HRMS (ES⁺) m/z Calcd for C₂₈H₂₅Cl₃N₂O₂ +H [(M+H)⁺]: 527.1055, found: 527.1051.

### Example 42b

### Preparation of intermediate rac-(2R,3R,4R,5S)-3,5-bis-(3-chloro-phenyl)-4-(4-chloro-phenyl)-4-cyano-pyrrolidine-2-carboxylic acid trifluoroacetic acid

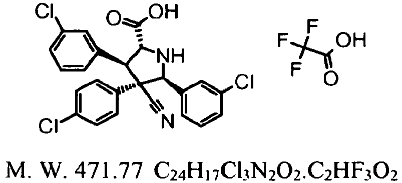

In a manner similar to the method described in **Example 25a**, rac-(2R,3R,4R,5S)-3,5-bis-(3-chloro-phenyl)-4-(4-chloro-phenyl)-4-cyano-pyrrolidine-2-carboxylic acid tert-butyl ester prepared in **Example 42a** (0.45 g, 0.85 mmol) was reacted with trifluoroacetic acid in dichloromethane at room temperature to give rac-(2R,3R,4R,5S)-3,5-bis-(3-chloro-phenyl)-4-(4-chloro-phenyl)-4-cyano-pyrrolidine-2-carboxylic acid trifluoroacetic acid as a off white solid (0.49 g, 98%).

HRMS (ES⁺) m/z. Calcd for C₂₄H₁₇Cl₃N₂O₂+H [(M + H)⁺]: 471.0429, found: 471.0429.

### Example 42c

### Preparation of rac-(2R,3R,4R,55)-3,5-bis-(3-chloro-phenyl)-4-(4-chloro-phenyl)-4-cyanopyrrolidine-2-carboxylic acid [2-((S)-2,2-dimethyl-[1,3]dioxolan-4-yl)-ethyl]-amide

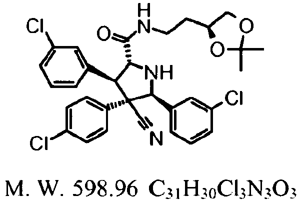

In a manner similar to the method described in **Example 1e**, rac-(2R,3R,4R,5S)-3,5-bis-(3-chloro-phenyl)-4-(4-chloro-phenyl)-4-cyano-pyrrolidine-2-carboxylic acid trifluoroacetic acid prepared in **Example 42b** (0.3 g, 0.5 mmol) was reacted with 2-((S)-2,2-dimethyl-[1,3]dioxolan-4-yl)-ethylamine (0.3 g, 2 mmol), HATU (0.3 g, 0.75 mmol) and iPr₂NEt (0.6 g, 4 mmol) in CH₂Cl₂ (30 mL) at room temperature for 20 h to give rac-(2R,3R,4R,5S)-3,5-bis-(3-chloro-phenyl)-4-(4-chloro-phenyl)-4-cyano-pyrrolidine-2-carboxylic acid [2-((S)-2,2-dimethyl-[1,3]dioxolan-4-yl)-ethyl]-amide as a offwhite solid (0.25 g, 83%).

HRMS (ES⁺) m/z Calcd for C₃₁H₃₀Cl₃N₃O+ H [(M+H)⁺]: 598.1426, found: 598.1424.

### Example 42d

### Preparation of rac-(²R,3R,4R,5S)-3,5-bis-(3-chloro-phenyl)-4-(4-chloro-phenyl)-4-cyanopyrrolidine-2-carboxylic acid ((S)-3,4-dihydroxy-butyl)-amide

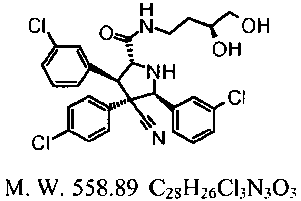

To a solution of rac-(2R,3R,4R,5S)-3,5-bis-(3-chloro-phenyl)-4-(4-chloro-phenyl)-4-cyanopyrrolidine-2-carboxylic acid [2-((S)-2,2-dimethyl-[1,3]dioxolan-4-yl)-ethyl]-amide prepared in **Example 42c** (0.4 g, 0.66 mol) in tetrahydrofuran (10 mL) was added aqueous HCl solution (1N, 10 mL). The reaction mixture was stirred at room temperature for 2 h, then concentrated. Then the residue was partitioned between ethyl acetate and water. The organic layer was separated, washed with water, aqueous saturated NaHCO₃, brine, dried over MgSO₄, concentrated, dried under reduced pressure to give rac-(2R,3R,4R,5S)-3,5-bis-(3-chloro-phenyl)-4-(4-chloro-phenyl)-4-cyano-pyrrolidine-2-carboxylic acid ((S)-3,4-dihydroxy-butyl)-amide as a white solid (0.2 g, 89%).

HRMS (ES⁺) m/z Calcd for C₂₈H₂₆Cl₃N₃O₃+ H [(M+H)⁺]: 538.1113, found= 558.1110.

### Example 43a

### Preparation of intermediate [2-ethyl-but-(E)-ylideneamino]-acetic acid tert-butyl ester

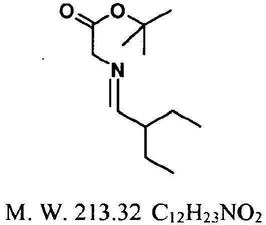

In a manner similar to the method described in **Example 1a**, glycine tert-butyl ester (0.65 g, 5 mmol) was reacted with 2-ethylbutyraldehyde (Aldrich) (0.55 g, 5 mmol) in CH₂Cl₂ at room temperature for 18 h to give [2-ethyl-but-(E)-ylideneamino]-acetic acid tert-butyl ester as a colorless oil (1 g, 94%).

### Example 43b

### Preparation of intermediate rac-(2R,3R,4R,5S)-3-(3-chloro-phenyl)-4-( 4-chloro-phenyl)-4-cyano-5-(1-ethyl-propyl)-pyrrolidine-2-carboxylic acid tert-butyl ester

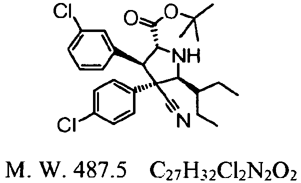

In a manner similar to the method described in **Example 1c,** [2-ethyl-but-(E)-ylideneamino]-acetic acid tert-butyl ester prepared in **Example 43a** (1 g. 4.7 mmol) was reacted with (Z)-3-(3-chloro-phenyl)-2-(4-chloro-phenyl)-acrylonitrile (0.91 g, 3.3 mmol) prepared in **Example 1b**, AgF (1.5 g, 12 mmol), and triethylamine (1.9 g, 19 mmol) in 1,2-dichloroethane (50 mL) at room temperature for 18 h to give rac-(2R,3R,4R,5S)-3-(3-chloro-phenyl)-4-(4-chloro-phenyl)-4-cyano-5-(1-ethyl-propyl)-pyrrolidine-2-carboxylic acid tert-butyl ester as a white foam (1.1 g, 68%).

### Example 43c

### Preparation of intermediate rac-(2R,3R,4R,5S)-3-(3-chloro-phenyl)-4-(4-chloro-phenyl)-4-cyano-5-(1-ethyl-propyl)-pyrrolidine-2-carboxylic acid trifluoroacetic acid

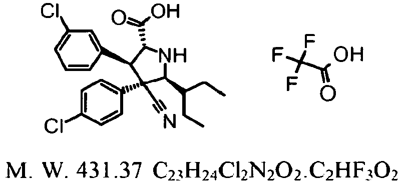

In a manner similar to the method described in **Example 25a**, rac-(2R,3R,4R,5S)-3-(3-chloro-phenyl)-4-(4-chloro-phenyl)-4-cyano-5-(1-ethyl-propyl)-pyrrolidine-2-carboxylic acid tert-butyl ester prepared in **Example 43b** (1.1 g, 2.3 mmol) was reacted with trifluoroacetic acid in dichloromethane at room temperature to give rac-(2R,3R,4R,5S)-3-(3-chloro-phenyl)-4-(4-chloro-phenyl)-4-cyano-5-(1-ethyl-propyl)-pyrrolidine-2-carboxylic acid trifluoroacetic acid as a white solid (1.2 g, 98%).

HRMS (ES⁺) m/z Calcd for C₂₃H₂₄Cl₂N₂O₂+H [(M+H)⁺]: 431.1288. found: 431.1286.

### Example 43d

### Preparation of rac-(2R,3R,4R,5S)-3-(3-chloro-phenyl)-4-(4-chloro-phenyl)-4-cyano-5-(1-ethylpropyl)-pyrrolidine-2-carboxylic acid ((S)-3,4-dihydroxy-butyl)-amide

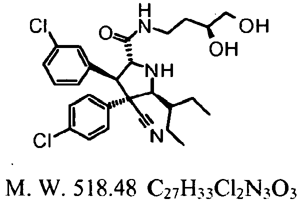

In a manner similar to the method described in **Examples 42c, 42d**, rac-(2R,3R,4R,5S)-3-(3-chloro-phenyl)-4-(4-chloro-phenyl)-4-cyano-5-(1-ethyl-propyl)-pyrrolidine-2-carboxylic acid trifluoroacetic acid prepared in **Example 43c** (0.55 g, 1 mmol) was reacted with 2-((S)-2.2-dimethyl-[1,3]dioxolan-4-yl)-ethylamine (0.2 g, 1.3 mmol), HATU (0.4 g, 1.1 mmol) and iPr₂NEt (0.2 g, 1.5 mmol) in CH₂Cl₂ at room temperature for 20 h, then reacted with aqueous HCl solution in tetrahydrofuran at room temperature for 2 h to give rac-(2R,3R,4R,5S)-3-(3-chloro-phenyl)-4-(4-chloro-phenyl)-4-cyano-5-(1-ethyl-propyl)-pyrrolidine-2-carboxylic acid ((S)-3,4-dihydroxy-butyl)-amide as a white solid (0.5 g, 96%).

HRMS (ES⁺) m/z Calcd for C₂₇H₃₃Cl₂N₃O₃+ H [(M+H)⁺]: 518.1972. found: 518.1970,

### Example 44a

### Preparation of rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid ((S)-3,4-dihydroxy-butyl)-amide

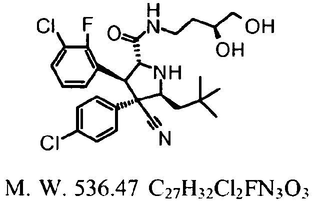

In a manner similar to the method described in **Examples 42c, 42d**, rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid trifluoroacetic acid prepared in **Example 26c** (0.25g, 0.44 mmol) was reacted with 2-((S)-2,2-dimethyl-[1,3]dioxolan-4-yl)-ethylamine (0.2 g, 1.3 mmol). HATU (0.3 g, 0.79 mmol) and iPr₂NEt (0.5 g, 3.9 mmol) in CH₂Cl₂ at room temperature for 20 h, then reacted with aqueous HCl solution in tetrahydrofuran at room temperature for 2 h to give rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid ((S)-3,4-dihydroxy-butyl)-amide as a white solid (0.21 g, 89%).

HRMS (ES⁺) m/z Calcd for C₂₇H₃₂Cl₂FN₃O₃+H [(M+H)⁺]: 536.1878, found: 536.1875.

### Example 44b

### Preparation of (2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid ((S)-3,4-dihydroxy-butyl)-amide

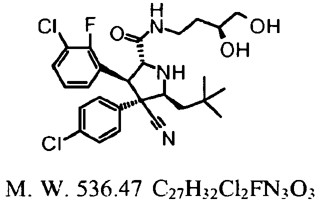

Rac-(2R,3S,4R,5S)-3-(3-chloro-2-fluoro-phenyl)-4-(4-chloro-phenyl)-4-cyano-5-(2,2-dimethylpropyl)-pyrrolidine-2-carboxylic acid ((S)-3,4-dihydroxy-butyl)-amide (0.19 g) was separated by chiral SFC chromatography to provide chiral-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid ((S)-3,4-dihydroxy-butyl)-amide as a white solid (85 mg, 45%) and chiral-(2S,3R,4SR,5R)-3-(3-chloro-2-fluoro-phenyl)-4-(4-chloro-phenyl)-4-cyano-5-(2,2-dimethyl-propyl)-pyrrolidine-2-carboxylic acid ((S)-3,4-dihydroxy-butyl)-amide as a white solid (81 mg, 43%).

### Example 45

### Preparation of rac-(2R,3R,4R,5S)-3-(3-chloro-phenyl)-4-(4-chloro-phenyl)-4-cyano-5-isobutyl-pyrrolidine-2-carboxylic acid ((S)-3,4-dihydroxy-butyl)-amide

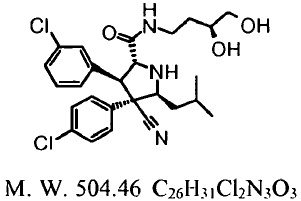

In a manner similar to the method described in **Examples 42c, 42d**, rac-(2R,3R,4R,5S)-3-(3-chloro-phenyl)-4-(4-chloro-phenyl)-4-cyano-5-isobutyl-pyrrolidine-2-carboxylic acid trifluoroacetic acid prepared in **Example 31c** (0.4 g, 0.75 mmol) was reacted with 2-((S)-2,2-dimethyl-[1,3]dioxolan-4-yl)-ethylamine (0.25 g, 1.6 mmol), HATU (0.4 g, 1.1 mmol) and iPr₂NEt (1 g, 7.8 mmol) in CH₂Cl₂ at room temperature for 20 h, then reacted with aqueous HCl solution in tetrahydrofuran at room temperature for 2 h to give rac-(2R,3R,4R,5S)-3-(3-chloro-phenyl)-4-(4-chloro-phenyl)-4-cyano-5-isobutyl-pyrrolidine-2-carboxylic acid ((S)-3,4-dihydroxy-butyl)-amide as a white solid (0.4 g, 95%).

HRMS (ES⁺) m/z Calcd for C₂₆H₃₁Cl₂N₃O₃- H [(M+H)⁺]: 504.1815, found: 504.1815.

### Example 46a

### Preparation of intermediate rac-(2R,3R,4R,5R)-3-(3-chloro-phenyl)-4-(4-chloro-phenyl)-4-cyano-5-isobutyl-pyrrolidine-2-carboxylic acid tert-butyl ester

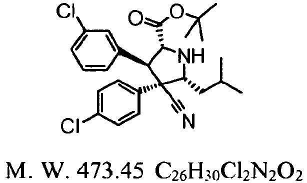

In the preparation of rac-(2R,3R,4R,5S)-3-(3-chloro-phenyl)-4-(4-chloro-phenyl)-4-cyano-5-isobutyl-pyrrolidine-2-carboxylic acid tert-butyl ester as described in **Example 31b**, rac-(2R,3R,4R,5R)-3-(3-chloro-phenyl)-4-(4-chloro-phenyl)-4-cyano-5-isobutyl-pyrrolidine-2-carboxylic acid tert-butyl ester was obtained as the second product: white powder, Yield: 0.82 g, 24%.

HRMS (ES⁺) m/z Calcd for C₂₆H₃₀Cl₂N₂O₂+H [(M+H)⁺]: 473.1757, found: 473.1756.

### Example 46b

### Preparation of intermediate rac-(2R,3R,4R,5R)-3-(3-chloro-phenyl)-4-(4-chloro-phenyl)-4-cyano-5-isobutyl-pyrrolidine-2-carboxylic acid trifluoroacetic acid

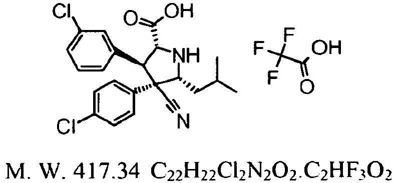

In a manner similar to the method described in **Example 25a**, rac-(2R,3R,4R,5R)-3-(3-chloro-phenyl)-4-(4-chloro-phenyl)-4-cyano-5-isobutyl-pyrrolidine-2-carboxylic acid tert-butyl ester prepared in **Example 46a** (0.6 g, 1.3 mmol) was reacted with trifluoroacetic acid in dichloromethane at room temperature to give rac-(2R,3R,4R,5R)-3-(3-chloro-phenyl)-4-(4-chloro-phenyl)-4-cyano-5-isobutyl-pyrrolidine-2-carboxylic acid trifluoroacetic acid as a off white solid (0.6 g, 89%).

### Example 46c

### Preparation of rac-(2R,3R,4R,5R)-3-(3-chloro-phenyl)-4-(4-chloro-phenyl)-4-cyano-5-isobutyl-pyrrolidine-2-carboxylic acid ((S)-3,4-dihydroxy-butyl)-amide

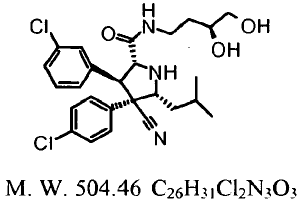

In a manner similar to the method described in **Examples 42c, 42d**, rac-(2R,3R,4R,5R)-3-(3-chloro-phenyl)-4-(4-chloro-phenyl)-4-cyano-5-isobutyl-pyrrolidine-2-carboxylic acid trifluoroacetic acid prepared in **Example 46b** (0.6 g, 1.1 mmol) was reacted with 2-((S)-2,2-dimethyl-[1,3]dioxolan-4-yl)-ethylamine (0.3 g, 2 mmol), HATU (0.5 g, 1.3 mmol) and iPr₂NEt (1.2 g, 9.3 mmol) in CH₂Cl₂ at room temperature for 20 h, then reacted with aqueous HCl solution in tetrahydrofuran at room temperature for 2 h to give rac-(2R,3R,4R,5R)-3-(3-chloro-phenyl)-4-(4-chloro-phenyl)-4-cyano-5-isobutyl-pyrrolidine-2-carboxylic acid ((S)-3,4-dihydroxy-butyl)-amide as a white solid (0.6 g, 95%).

HRMS (ES⁺) m/z Calcd for C₂₆H₃₁Cl₂N₃O₃+ H [(M+H)⁺]; 504.1815. found: 504.1816.

### Example 47a

### Preparation of intermediate rac-(2R,3R,4R,5R)-3-(3-chloro-phenyl)-4-(4-chloro-phenyl)-4-cyano-5-(1-ethyl-propyl)-pyrrolidine-2-carboxylic acid tert-butyl ester

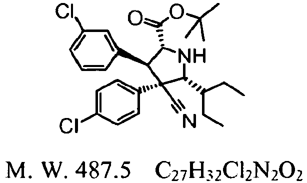

In the preparation of rac-(2R,3R,4R,5S)-3-(3-chloro-phenyl)-4-(4-chloro-phenyl)-4-cyano-5-(1-ethyl-propyl)-pyrrolidine-2-carboxylic acid tert-butyl ester as described in **Example 43b**, rac-(2R,3R,4R,5R)-3-(3-chloro-phenyl)-4-(4-chloro-phenyl)-4-cyano-5-(1-ethyl-propyl)-pyrro lidine-2-carboxylic acid tert-butyl ester was obtained as the second product: white foam, Yield, 0.26 g, 16%.

### Example 47b

### Preparation of intermediate rac-(2R,3R,4R,5R)-3-(3-chloro-phenyl)-4-(4-chloro-phenyl)-4-cyano-5-(1-ethyl-propyl)-pyrrolidine-2-carboxylic acid trifluoroacetic acid

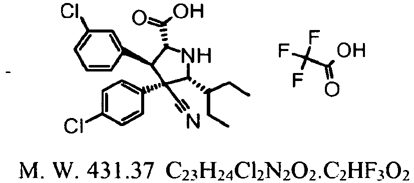

In a manner similar to the method described in **Example 25a**, rac-(2R,3R,4R,5R)-3-(3-chloro-phenyl)-4-(4-chloro-phenyl)-4-cyano-5-(1-ethyl-propyl)-pyrrolidine-2-carboxylic acid tert-butyl ester prepared in **Example 47a** (0.25 g, 0.5 mmol) was reacted with trifluoroacetic acid in dichloromethane at room temperature to give rac-(2R,3R,4R,5R)-3-(3-chloro-phenyl)-4-(4-chloro-phenyl)-4-cyano-5-(1-ethyl-propyl)-pyrrolidine-2-carboxylic acid trifluoroacetic acid as a off white solid (0.2 g, 73%).

HRMS (ES⁺) m/z Calcd for C₂₃H₂₄Cl₂N₂O₂+ H [(M+M)⁺]: 431.1288, found: 431.1285.

### Example 47c

### Preparation of rac-(2R,3R,4R,5R)-3-(3-chloro-phenyl)-4-(4-chloro-phenyl)-4-cyano-5-(1-ethylpropyl)-pyrrolidine-2-carboxylic acid ((S)-3,4-dihydroxy-butyl)-amide

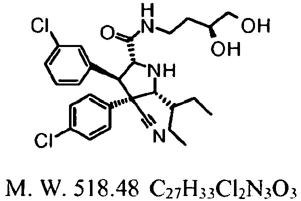

In a manner similar to the method described in **Example 42e**, rac-(2R,3R,4R,5S)-3-(3-chloro-phenyl)-4-(4-chloro-phenyl)-4-cyano-5-(1-ethyl-propyl)-pyrrolidine-2-carboxylic acid trifluoroacetic acid prepared in **Example 47b** (0.27 g, 0.5 mmol) was reacted with 2-((S)-2,2-dimethyl-[1,3]dioxolan-4-yl)-ethylamine (0.2 g, 1.3 mmol), HATU (0.4 g, 1.1 mmol) and iPr₂NEt (0.4 g, 3 mmol) in CH₂Cl₂ at room temperature for 20 h, then reacted with aqueous HCl solution in tetrahydrofuran at room temperature for 2 h to give rac-(2R,3R,4R,5R)-3-(3-chloro-phenyl)-4-(4-chloro-phenyl)-4-cyano-5-(1-ethyl-propyl)-pyrrolidine-2-carboxylic acid ((S)-3,4-dihydroxy-butyl)-amide as a white solid (0.23 g, 88%).

HRMS (ES⁺) m/z Calcd for C₂₇H₃₃Cl₂N₃O₃+H [(M+H)⁺]: 518.1972, found: 518.1971.

### Example 48a

### Preparation of intermediate (1-ethyl-propylideneamino)-acetic acid tert-butyl ester

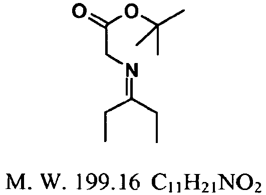

A mixture of glycine tert-butyl ester (Alfa) (0.66 g, 10 mmol) and 3-pentanone (6 g, 70 mmol) in ethanol (6 mL) was heated at 110 °C in a sealed tube for 48 h. The reaction mixture was concentrated and dried *in vacu* to give crude (1-ethyl-propylideneamino)-acetic acid tert-butyl ester as a colorless oil (1.0 g). The crude product contains unreacted glycine tert-butyl ester and was used without further purification.

### Example 48b

### Preparation of intermediate rac-(2R,3R,4R)-3-(3-chloro-phenyl)-4-(4-chloro-phenyl)-4-cyano-5,5-diethyl-pyrrolidine-2-carboxylic acid tert-butyl ester

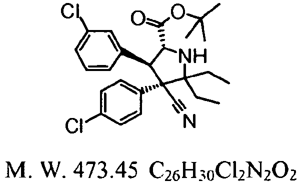

In a manner similar to the method described in **Example 1c**, crude (1-ethyl-propylideneamino)-acetic acid tert-butyl ester prepared in **Example 48a** (1.2 g, 6 mmol) was reacted with (Z)-3-(3-chloro-phenyl)-2-(4-chloro-phenyl)-acrylonitrile (0.7 g, 2.5 mmol) prepared in **Example 1b**, AgF (1.9 g, 15 mmol), and triethylamine (2.5 g, 25 mmol) in 1,2-dichloroethane (130 mL) at room temperature for 10 h to give rac-(2R,3R,4R)-3-(3-chloro-phenyl)-4-(4-chloro-phenyl)-4-cyano-5,5-diethyl-pyrrolidine-2-carboxylic acid tert-butyl ester as a yellow gum (0.33 g, 28%).

### Example 48c

### Preparation of intermediate rac-(2R,3R,4R)-3-(3-chloro-phenyl)-4-(4-chloro-phenyl)-4-cyano-5,5-diethyl-pyrrolidine-2-carboxylic acid trifluoroacetic acid

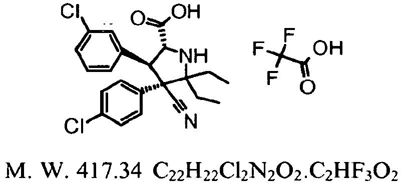

In a manner similar to the method described in **Example 25a,** rac-(2R,3R,4R)-3-(3-chloro-phenyl)-4-(4-chloro-phenyl)-4-cyano-5,5-diethyl-pyrrolidine-2-carboxylic acid tert-butyl ester prepared in **Example 48c** (0.33 g, 0.7 mmol) was reacted with trifluoroacetic acid in dichloromethane at room temperature to give rac-(2R,3R,4R)-3-(3-chloro-phenyl)-4-(4-chloro-phenyl)-4-cyano-5,5-diethyl-pyrrolidine-2-carboxylic acid trifluoroacetic acid as a off white foam (0.35 g. 96%).

HRMS (ES⁺) m/z Calcd tor C₂₂H₂₂Cl₂N₂O₂- H [(M+H)⁺]: 417.1131.0429, found: 417.1132.

### Example 48d

### Preparation of rac-(2R,3R,4R)-3-(3-chloro-phenyl)-4-(4-chloro-phenyl)-4-cyano-5,5-diethylpyrrolidine-2-carboxylic acid ((S)-3,4-dihydroxy-butyl)-amide

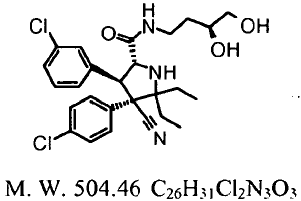

In a manner similar to the method described in **Examples 42c, 42d**, rac-(2R,3R,4R)-3-(3-chloro-phenyl)-4-(4-chloro-phenyl)-4-cyano-5,5-diethyl-pyrrolidine-2-carboxylic acid trifluoroacetic acid prepared in **Example 48c** (0.33 g, 0.62 mmol) was reacted with 2-((S)-2,2-dimethyl-[1,3]dioxolan-4-yl)-ethylamine (0.15 g, 1 mmol), HATU (0.34 g, 0.89 mmol) and iPr₂NEt (1 g, 7.8 mmol) in CH₂Cl₂ at room temperature for 20 h, then reacted with aqueous HCl solution in tetrahydrofuran at room temperature for 2 h to give rac-(2R,3R,4R)-3-(3-chloro-phenyl)-4-(4-chloro-phenyl)-4-cyano-5,5-diethyl-pyrrolidine-2-carboxylic acid ((S)-3,4-dihydroxy-butyl)-amide as a off white solid (0.4 g, 95%).

HRMS (ES⁺) m/z Calcd for C₂₆H₃₁Cl₂N₃O₃+H [(M+H)⁺] 504.1815, found: 504.18.15.

### Example 49a

### Preparation of intermediate [2-methyl-prop-(E)-ylideneamino]-acetic acid tert-butyl ester

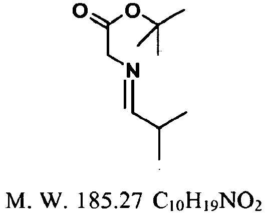

In a manner similar to the method described in **Example 1a,** glycine tert-butyl ester (0.65 g, 5 mmol) was reacted with isobutyraldehyde (Aldrich) (0.4 g, 5 mmol) in CH₂Cl₂ at room temperature for 20 h to give [2-methyl-prop-(E)-ylideneamino]-acetic acid tert-butyl ester as a colorless oil (0.9 g, 97%).

### Example 49b

### Preparation of intermediate rac-(2R,3R,4R,5S)-3-(3-chloro-phenyl)-4-(4-chloro-phenyl)-4-cyano-5-isopropyl-pyrrolidine-2-carboxylic acid tert-butyl ester

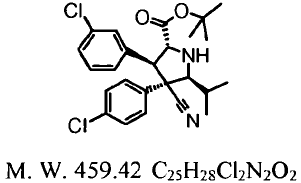

In a manner similar to the method described in **Example 1c,** [2-methyl-prop-(E)-ylideneamino]-acetic acid tert-butyl ester prepared in **Example 49a** (1g, 5.4 mmol) was reacted with (Z)-3-(3-chloro-phenyl)-2-(4-chloro-phenyl)-acrylonitrile (0.85 g, 3.1 mmol) prepared in **Example 1b**, AgF (1.5 g, 12 mmol), and triethylamine (2 g, 20 mmol) in dichloromethane (100 mL) at room temperature for 20 h to give rac-(2R,3R,4R,5S)-3-(3-chloro-phenyl)-4-(4-chloro-phenyl)-4-cyano-5-isopropyl-pyrrolidine-2-carboxylic acid tert-butyl ester as a white foam (0.64 g, 45%).

### Example 49c

### Preparation of intermediate rac-(2R,3R,4R,5S)-3-(3-chloro-phenyl)-4-(4-chloro-phenyl)-4-cyano-5-isopropyl-pyrrolidine-2-carboxylic acid trifluoroacetic acid

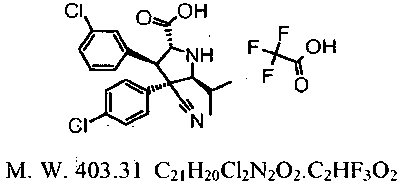

In a manner similar to the method described in **Example 25a**, rac-(2R,3R,4R,5S)-3-(3-chloro-phenyl)-4-(4-chloro-phenyl)-4-cyano-5-isopropyl-pyrrolidine-2-carboxylic acid tert-butyl ester prepared in **Example 49b** (0.64 g, 1.4 mmol) was reacted with trifluoroacetic acid in dichloromethane at room temperature to give rac-(2R,3R,4R,5S)-3-(3-chloro-phenyl)-4-(4-chloro-phenyl)-4-cyano-5-isopropyl-pyrrolidine-2-carboxylic acid trifluoroacetic acid as a off white solid (0.7 g, 100%).

HRMS (ES⁺) m/z Calcd for C₂₁H₂₀Cl₂N₂O₂+H [(M+H)⁺]: 403.0975. found; 403.0974.

### Example 49d

### Preparation of rac-(2R,3R,4R,5S)-3-(3-chloro-phenyl)-4-(4-chloro-phenyl)-4-cyano-5-isopropyl-pyrrolidine-2-carboxylic acid ((S)-3,4-dihydroxy-butyl)-amide

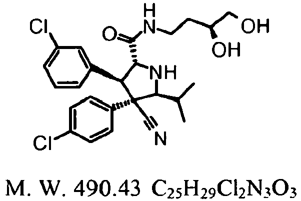

In a manner similar to the method described in **Examples 42c, 42d**, rac-(2R,3R,4R,5S)-3-(3-chloro-phenyl)-4-(4-chloro-phenyl)-4-cyano-5-isopropyl-pyrrolidine-2-carboxylic acid trifluoroacetic acid prepared in **Example 49c** (0.5 g, 0.97 mmol) was reacted with 2-((S)-2,2-dimethyl-[1,3]dioxolan-4-yl)-ethylamine (0.25 g, 1.7 mmol), HATU (0.5 g, 1.3 mmol) and iPr₂NEt (1 g, 7.8 mmol) in CH₂Cl₂ at room temperature for 20 h, then reacted with aqueous HCl solution in tetrahydrofuran at room temperature for 2 h to give rac-(2R,3R,4R,5S)-3-(3-chloro-phenyl)-4-(4-chloro-phenyl)-4-cyano-5-isopropyl-pyrrolidine-2-carboxylic acid ((S)-3,4-dihydroxy-butyl)-amide as a white solid (0.25 g, 52%).

HRMS (ES⁺) m/z Calcd for C₂₅H₂₉Cl₂N₃O₃+H[(M+H)⁺] : 490.1659, found: 490.1657.

### Example 50a

### Preparation of intermediate {[1-cyclohexyl-meth-(E)-ylidene]-amino}-acetic acid tert-butyl ester

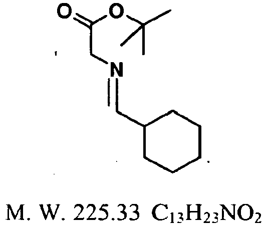

In a manner similar to the method described in **Example 1a,** glycine tert-butyl ester (0.65 g, 5 mmol) was reacted with cyclohexanecarbaldehyde (Aldrich) (0.6 g, 5 mmol) in CH₂Cl₂ at room temperature for 20 h to give {[1-cyclohexyl-meth-(E)-ylidene]-amino}-acetic acid tert-butyl ester as a colorless oil (1.2 g, 100%).

### Example 50b

### Preparation of intermediate rac-(2R,3R,4R,5S)-3-(3-chloro-phenyl)-4-(4-chloro-phenyl)-4-cyano-5-cyclohexyl-pyrrolidine-2-carboxylic acid tert-butyl ester

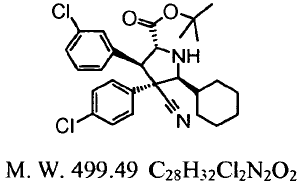

In a manner similar to the method described in **Example 1c**, {[1-cyclohexyl-meth-(E)-ylidene]-amino}-acetic acid tert-butyl ester prepared in **Example 50a** (1.2 g, 5.3 mmol) was reacted with (Z)-3-(3-chloro-phenyl)-2-(4-chloro-phenyl)-acrylonitrile (1 g, 3.7 mmol) prepared in **Example 1b**, AgF (1.5 g, 12 mmol), and triethylamine (2 g, 20 mmol) in dichloromethane (100 mL) at room temperature for 20 h to give rac-(2R,3R,4R,5S)-3-(3-chloro-phenyl)-4-(4-chloro-phenyl)-4-cyano-5-cyclohexyl-pyrrolidine-2-carboxylic acid tert-butyl ester as a white foam (0.69 g, 38%).

### Example 50c

### Preparation of intermediate rac-(2R,3R,4R,5S)-3-(3-chloro-phenyl)-4-(4-chloro-phenyl)-4-cyano-5-cyclohexyl-pyrrolidine-2-carboxylic acid trifluoroacetic acid

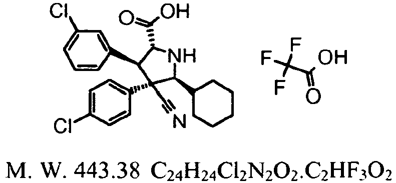

In a manner similar to the method described in **Example 25a**, rac-(2R,3R,4R,5S)-3-(3-chloro-phenyl)-4-(4-chloro-phenyl)-4-cyano-5-cyclohexyl-pyrrolidine-2-carboxylic acid tert-butyl ester prepared in **Example 50b** (0.69 g, 1.4 mmol) was reacted with trifluoroacetic acid in dichloromethane at room temperature to give rac-(2R,3R,4R,5S)-3-(3-chloro-phenyl)-4-(4-chloro-phenyl)-4-cyano-5-cyclohexyl-pyrrolidine-2-carboxylic acid trifluoroacetic acid as a off white solid (0.8 g, 100%).

HRMS (ES⁺) m/z Calcd for C₂₄H₂₄O₂N₂O₂+H [(M+H)⁺]:443.1288, found: 443.1286.

### Example 50d

### Preparation of rac-(2R,3R,4R,5S)-3-(3-chloro-phenyl)-4-(4-chloro-phenyl)-4-cyano-5-cyclohexyl-pyrrolidine-2-carboxylic acid ((S)-3,4-dihydroxy-butyl)-amide

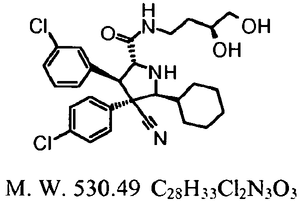

In a manner similar to the method described in **Examples 42c, 42d**, rac-(2R,3R,4R,5S)-3-(3-chloro-phenyl)-4-(4-chloro-phenyl)-4-cyano-5-cyclohexyl-pyrrolidine-2-carboxylic acid trifluoroacetic acid prepared in **Example 50c** (0.5 g, 0.76 mmol) was reacted with 2-((S)-2,2-dimethyl-[1,3]dioxolan-4-yl)-ethylamine (0.3 g, 2 mmol), HATU (0.4 g, 1.1 mmol) and iPr₂NEt (0.9 g, 7 mmol) in CH₂Cl₂ at room temperature for 20 h, then reacted with aqueous HCl solution in tetrahydrofuran at room temperature for 2 h to give rac-(2R,3R,4R,5S)-3-(3-chloro-phenyl)-4-(4-chloro-phenyl)-4-cyano-5-cyclohexyl-pyrrolidine-2-carboxylic acid ((S)-3,4-dihydroxybutyl)-amide as a white solid (0.25 g, 62%).

HRMS (ES⁺) m/z Calcd for C₂₈H₃₃Cl₂N₃O₃+H [(M+H)⁺]: 530.1972. found: 530.1971.

### Example 51a

### Preparation of intermediate [2,2-dimethyl-prop-(E)-ylideneamino]-acetic acid tert-butyl ester

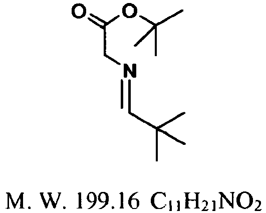

In a manner similar to the method described in **Example 1a**, glycine tert-butyl ester (0.65 g, 5 mmol) was reacted with trimethylacetaldehyde (Aldrich) (0.4:c g, 5 mmol) in CH₂Cl₂ at room temperature for 20 h to give [2,2-dimethyl-prop-(E)-ylideneamino]-acetic acid tert-butyl ester as a colorless oil (1.0 g, 100%).

### Example 51b

### Preparation of intermediate rac-(2R,3S,4R,5S)-5-tert-butyl-3-(3-chloro-2-fluoro-phenyl)-4-(4-chloro-phenyl)-4-cyano-pyrrolidine-2-carboxylic acid tert-butyl ester

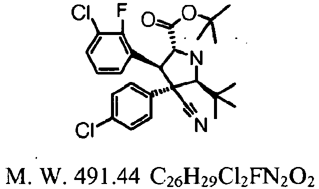

In a manner similar to the method described in **Example 1c**, [2,2-dimethyl-prop-(E)-ylideneamino]-acetic acid tert-butyl ester prepared in **Example 51a** (1 g, 5 mmol) was reacted with (Z)-3-(3-chloro-2-fluoro-phenyl)-2-(4-chloro-phenyl)-acrylonitrile (0.8 g, 2.7 mmol) prepared in **Example 26a**, AgF (1.5 g, 12 mmol), and triethylamine (2 g, 20 mmol) in dichloromethane (50 mL) at room temperature for 24 h to give (2R,3S,4R,5S)-5-tert-Butyl-3-(3-chloro-2-fluoro-phenyl)-4-(4-chloro-phenyl)-4-cyano-pyrrolidine-2-carboxylic acid tert-butyl ester as a white foam (0.4 g, 30%).

### Example 51c

### Preparation of intermediate rac-(2R,3S,4R,5S)-5-tert-butyl-3-(3-chloro-2-fluoro-phenyl)-4-(4-chloro-phenyl)-4-cyano-pyrrolidine-2-carboxylic acid trifluoroacetic acid

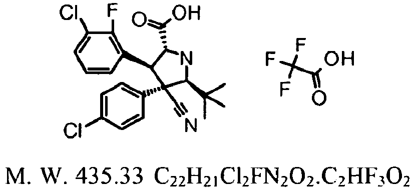

In a manner similar to the method described in **Example 25a**, rac-(2R,3S,4R,5S)-5-tert-Butyl-3-(3-chloro-2-fluoro-phenyl)-4-(4-chloro-phenyl)-4-cyano-pyrrolidine-2-carboxylic acid tert-butyl ester prepared in **Example 51b** (0.3 g, 0.6 mmol) was reacted with trifluoroacetic acid in dichloromethane at room temperature to give rac-(2R,3S,4R,5S)-5-tert-Butyl-3-(3-chloro-2-fluoro-phenyl)-4-(4-chloro-phenyl)-4-cyano-pyrrolidine-2-carboxylic acid trifluoroacetic acid as a off white solid (0.4 g, 100%).

HRMS (ES⁺) m/z Calcd for C₂₂H₂₁Cl₂FN₂O₂+H [(M+H)⁺]: 435.1037, found: 435.1036.

### Example 51d

### Preparation of rac-(2R,3S,4R,SS)-5-tert-butyl-3-(3-chloro-2-fluoro-phenyl)-4-(4-chloro-phenyl)-4-cyano-pyrrolidine-2-carboxylic acid ((S)-3,4-dihydroxy-butyl)-amide

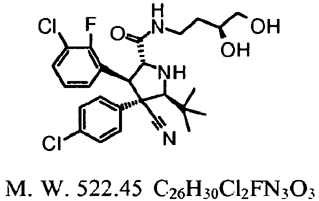

In a manner similar to the method described in **Examples 42c, 42d**, rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid trifluoroacetic acid prepared in **Example 51c** (0.4 g, 0.73 mmol) was reacted with 2-((S)-2,2-dimethyl-[1,3]dioxolan-4-yl)-ethylamine (0.2 g, 1.3 mmol), HATU (0.3 g, 0.79 mmol) and iPr₂NEt (0.6 g, 4.7 mmol) in CH₂Cl₂ at room temperature for 20 h, then reacted with aqueous HCl solution in tetrahydrofuran at room temperature for 2 h to give rac-(2R,3S,4R,5S)-5-tert-butyl-3-(3-chloro-2-fluoro-phenyl)-4-(4-chloro-phenyl)-4-cyano-pyrrolidine-2-carboxylic acid ((S)-3,4-dihydroxy-butyl)-amide as a white solid (0.22 g, 58%).

HRMS (ES⁺) m/z Calcd for C₂₆H₃₀Cl₂FN₃O₃ + H [(M+H)⁺]; 522.1721, found: 522.1719.

### Example 52a

### Preparation of intermediate (Z)-3-(3-chloro-2-fluoro-phenyl)-2-(4-chloro-2-fluoro-phenyl)-acrylonitrile

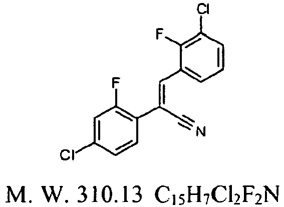

In a manner similar to the method described in **Example 1b,** 4-chloro-2-fluorophenylacetonitrile (5 g, 30 mmol) was reacted with 3-chloro-2-fluorobenzaldehyde (5 g, 32 mmol), methanolic solution (25 wt%) of sodium methoxide (21 mL, 92 mmol) in methanol (200 mL) at 45 °C for 5 h to give (Z)-3-(3-chloro-2-fluoro-phenyl)-2-(4-chloro-2-fluoro-phenyl)-acrylonitrile as a white powder (9 g, 97%).

### Example 52b

### Preparation of intermediate rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid tert-butyl ester

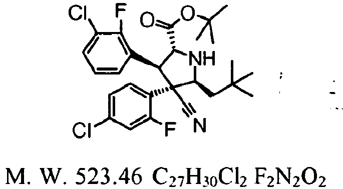

In a manner similar to the method described in **Example 1c**, [3-methyl-but-(E)-ylideneamino]-acetic acid tert-butyl ester prepared in **Example 1a** (2.3 g, 11 mmol) was reacted with (Z)-3-(3-chloro-2-fluoro-phenyl)-2-(4-chloro-2-fluoro-phenyl)-acrylonitrile (2.5g, 8 mmol) prepared in **Example 52a**, AgF (0.7 g, 5.5 mmol), and triethylamine (2.9 g, 29 mmol) in dichloromethane (200 mL) at room temperature for 18 h to give rac-(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid tert-butyl ester as a white foam (3 g, 64%).

### Example 52c

Preparation of intermediate rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid trifluoroacetic acid

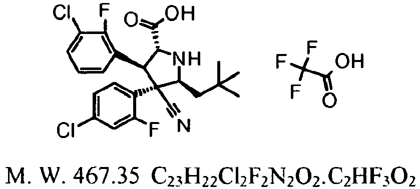

In a manner similar to the method described in **Example 25a**, rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid tert-butyl ester prepared in **Example 52b** (0.4 g, 0.8 mmol) was reacted with trifluoroacetic acid in dichloromethane at room temperature to give rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid trifluoroacetic acid as a white solid (0.5 g, 100%).

HRMS (ES⁺ ) m/z Calcd for C₂₃H₂₂Cl₂F₂N₂O₂+ H [(M+H)⁺]: 467.1099, found: 467.1098,

### Example 52d

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide

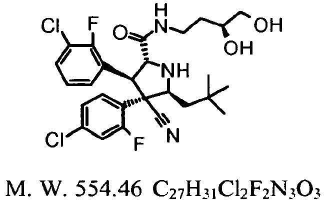

In a manner similar to the method described in **Examples 42c, 42d,** rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid trifluoroacetic acid prepared in **Example 52c** (0.4 g, 0.69 mmol) was reacted with 2-((S)-2,2-dimethyl-[1,3]dioxolan-4-yl)-ethylamine (0.25 g, 1.7 mmol), HATU (0.35 g, 0.92 mmol) and iPr₂NEt (0.75 g, 5.8 mmol) in CH₂Cl₂ at room temperature for 20 h, then reacted with aqueous HCl solution in tetrahydrofuran at room temperature for 2 h to give rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide as a white solid (0.26 g, 84%).

HRMS (ES⁺) m/z Calcd for C₂₇H₃₁Cl₂F₂N₃O₃+ H [(M+H)⁺]: 554.1784, found: 554.1783.

### Example 52e

### Preparation of (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide

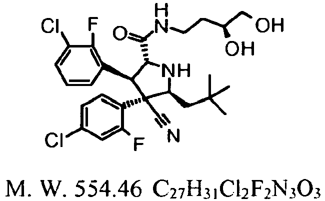

Rac-(2R,35,4R,55)-3-(3-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide (0.3g) was separated by chiral SFC chromatography to provide chiral-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide as a white solid (120 mg, 40%) and chiral-(2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide as a white solid (121 mg, 40%).

### Example 53a

### Preparation of intermediate rac-(2S,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid tert-butyl ester

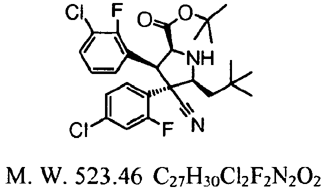

In preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid tert-butyl ester as described in **Example 52b**, rac-(2S,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid tert-butyl ester was obtained as the second product: a white foam (0.98 g, 21%).

### Example 53b

### Preparation of intermediate rac-(2S,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid trifluoroacetic acid

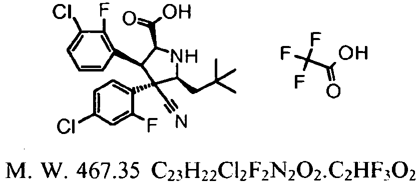

In a manner similar to the method described in **Example 25a**, rac-(2S,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid tert-butyl ester prepared in **Example 53a** (0.4 g, 0.8 mmol) was reacted with trifluoroacetic acid in dichloromethane at room temperature to give rac-(2S,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid trifluoroacetic acid as a white solid (0.5 g, 100%).

HRMS (ES⁺) m/z Calcd for C₂₃H₂₂O₂F₂N₂O₂ +H [(M+H)⁺]: 467.1099, found: 467.1099.

### Example 53c

### Preparation of rac-(2S,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide

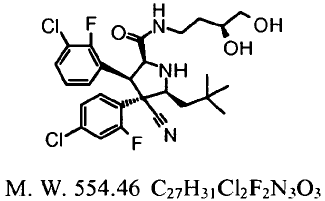

In a manner similar to the method described in **Examples 42c, 42d**, rac-(2S,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid trifluoroacetic acid prepared in **Example 53b** (0.3 g, 0.5 mmol) was reacted with 2-((S)-2,2-dimethyl-[1,3]dioxolan-4-yl)-ethylamine (0.15 g, 1 mmol), HATU (0.3 g, 0.79 mmol) and iPr₂NEt (0.4 g, 3.1 mmol) in CH₂Cl₂ at room temperature for 20 h, then reacted with aqueous HCl solution in tetrahydrofuran at room temperature for 2 h to give rac-(2S,3S,4R,SS)-3-(3-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide as a white solid (0.26 g, 94%).

HRMS (ES⁺) m/z Calcd tor C₂₇H₃₁Cl₂F₂N₃O₃+ H [(M+H)⁺]: 354.1784, found: 554.1782.

### Example 54a

### Preparation of intermediate (Z)-2-(4-bromo-phenyl)-3-(3-chloro-2-fluoro-phenyl)-acrylonitrile

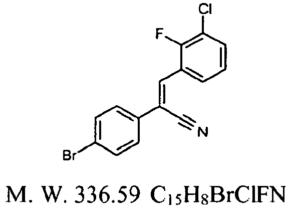

In a manner similar to the method described in **Example 1b**, 4-bromophenylacetonitrile (Aldrich) (4.5 g, 23 mmol) was reacted with 3-chloro-2-fluorobenzaldehyde (5.2 g, 33 mmol), methanolic solution (25 wt%) of sodium methoxide (15 mL, 66 mmol) in methanol (150 mL) at 50 °C for 3 h to give (Z)-2-(4-bromo-phenyl)-3-(3-chloro-2-fluoro-phenyl)-acrylonitrile as a white powder (7.8 g, 100%).

### Example 54b

### Preparation of intermediate rac-(2R,3S,4R,5S)-4-(4-bromo-phenyl)-3-(3-chloro-2-fluoro-phenyl)-4-cyano-5-(2,2-dimethyl-propyl)-pyrrolidine-2-carboxylic acid tert-butyl ester

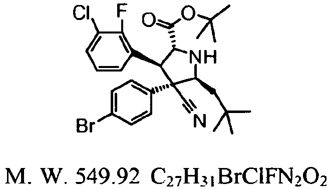

In a manner similar to the method described in **Example 1c,** [3,3-dimethyl-but-(E)-ylideneamino]-acetic acid tert-butyl ester prepared in **Example 1a** (1.1 g, 5 mmol) was reacted with (Z)-2-(4-bromo-phenyl)-3-(3-chloro-2-fluoro-phenyl)-acrylonitrile (1.2 g, 3.6 mmol) prepared in **Example 54a**, AgF (1.3 g, 10 mmol), and triethylamine (2 g, 20 mmol) in dichloromethane (100 mL) at room temperature for 3 h to give rac-(2R,3S,4R,5S)-4-(4-bromophenyl)-3-(3-chloro-2-fluoro-phenyl)-4-cyano-5-(2,2-dimethyl-propyl)-pyrrolidine-2-carboxylic acid tert-butyl ester as a white foam (1.1 g, 56%).

### Example 54c

### Preparation of intermediate rac-(2R,3S,4R,5S)-4-(4-bromo-phenyl)-3-(3-chloro-2-fluoro-phenyl)-4-cyano-5-(2,2-dimethyl-propyl)-pyrrolidine-2-carboxylic acid trifluoroacetic acid

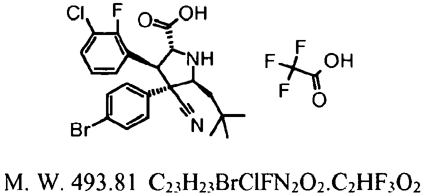

In a manner similar to the method described in **Example 25a**, rac-(2R,3S,4R,5S)-4-(4-bromophenyl)-3-(3-chloro-2-fluoro-phenyl)-4-cyano-5-(2,2-dimethyl-propyl)-pyrrolidine-2-carboxylic acid tert-butyl ester prepared in **Example 54b** (1.1 g, 2 mmol) was reacted with trifluoroacetic acid in dichloromethane at room temperature to give rac-(2R,3S,4R,5S)-4-(4-bromo-phenyl)-3-(3-chloro-2-fluoro-phenyl)-4-cyano-5-(2,2-dimethyl-propyl)-pyrrolidine-2-carboxylic acid trifluoroacetic acid as a off white solid (1.2 g, 99%).

HRMS (ES⁺) m/z Calcd for C₂₃H₂₃BrClFN₂O₂- H [(M-H)⁺]: 493.0688, found: 493.0689.

### Example 54d

### Preparation of rac-(2R,3S,4R,5S)-4-(4-bromo-phenyl)-3-(3-chloro-2-fluoro-phenyl)-4-cyano-5-(2,2-dimethyl-propyl)-pyrrolidine-2-carboxylic acid ((S)-3,4-dihydroxy-butyl)-amide

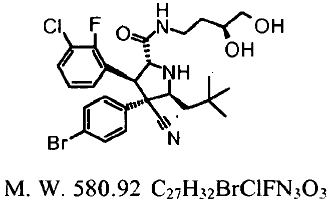

In a manner similar to the method described in **Examples 42c, 42d**, rac-(2R,3S,4R.SS)-4-(4-bromo-phenyl)-3-(3-chloro-2-fluoro-phenyl)-4-cyano-5-(2,2-dimethyl-propyl)-pyrrolidine-2-carboxylic acid trifluoroacetic acid prepared in **Example 54c** (0.3 g, 0.49 mmol) was reacted with 2-((S)-2,2-dimethyl-[1,3]dioxolan-4-yl)-ethylamine (0.15 g, 1 mmol), HATU (0.23 g, 0.6 mmol) and iPr₂NEt (0.4 g, 3.1 mmol) in CH₂Cl₂ at room temperature for 20 h, then reacted with aqueous HCl solution in tetrahydrofuran at room temperature for 2 h to give rac-(2R,3S,4R,5S)-4-(4-bromo-phenyl)-3-(3-chloro-2-fluoro-phenyl)-4-cyano-5-(2,2-dimethyl-propyl)-pyrrolidine-2-carboxylic acid ((S)-3,4-dihydroxy-butyl)-amide as a white solid (0.18 g, 63%).

HRMS (ES⁺) m/z Calcd for C₂₇H₃₂BrClFN₃O₃+H[(M+H)⁺]: 580.1373. found: 550.1372

### Example 55a

### Preparation of intermediate (Z)-3-(3-chloro-2-fluoro-phenyl)-2-(4-fluoro-phenyl)-acrylonitrile

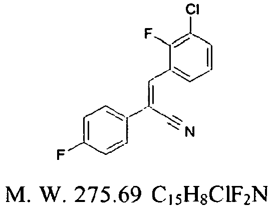

In a manner similar to the method described in **Example 1b**, 4-fluorophenylacetonitrile (Aldrich) (3.5 g, 26 mmol) was reacted with 3-chloro-2-fluorobenzaldehyde (5.3 g, 34 mmol), methanolic solution (25 wt%) of sodium methoxide (15 mL, 66 mmol) in methanol (200 mL) at 50 °C for 3 h to give (Z)-3-(3-chloro-2-fluoro-phenyl)-2-(4-fluoro-phenyl)-acrylonitrile as a white powder (5.7 g. 80%).

### Example 55b

### Preparation of intermediate rac-(2R,3S,4R,5S)-3-(3-chloro-2-fluoro-phenyl)-4-cyano-5-(2,2-dimethyl-propyl)-4-(4-fluoro-phenyl)-pyrrolidine-2-carboxylic acid tert-butyl ester

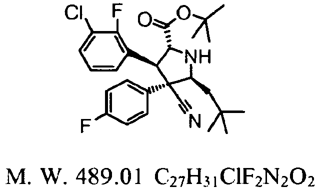

In a manner similar to the method described in **Example 1c**, [3,3-dimethyl-but-(E)-ylideneamino]-acetic acid tert-butyl ester prepared in **Example 1a** (1.1 g, 5 mmol) was reacted with (Z)-3-(3-chloro-2-fluoro-phenyl)-2-(4-fluoro-phenyl)-acrylonitrile (1.25 g, 4.5 mmol) prepared in **Example 55a**, AgF (1.6 g, 13 mmol), and triethylamine (1.6 g, 16 mmol) in dichloromethane (100 mL) at room temperature for 5 h to give rac-(2R,3S,4R,5S)-3-(3-chloro-2-fluoro-phenyl)-4-cyano-5-(2,2-dimethyl-propyl)-4-(4-fluoro-phenyl)-pyrro lidine-2-carboxylic acid tert-butyl ester as a white foam (1.6 g, 72%).

### Example 55c

### Preparation of intermediate rac-(2R,3S,4R,5S)-3-(3-chloro-2-fluoro-phenyl)-4-cyano-5-(2,2-dimethyl-propyl)-4-(4-fluoro-phenyl)-pyrrolidine-2-carboxylic acid trifluoroacetic acid

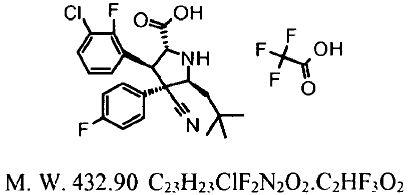

In a manner similar to the method described in **Example 25a**, rac-(2R,3S,4R,5S)-3-(3-chloro-2-fluoro-phenyl)-4-cyano-5-(2,2-dimethyl-propyl)-4-(4-fluoro-phenyl)-pyrrolidine-2-carboxylic acid tert-butyl ester prepared in **Example 55b** (1.6 g, 3.3 mmol) was reacted with trifluoroacetic acid in dichloromethane at room temperature to give rac-(2R,3S,4R,5S)-3-(3-chloro-2-fluoro-phenyl)-4-cyano-5-(2,2-dimethyl-propyl)-4-(4-fluoro-phenyl)-pyrrolidine-2-carboxylic acid trifluoroacetic acid as a white solid (1.7 g, 94%).

### Example 55d

### Preparation of rac-(2R,3S,4R,5S)-3-(3-chtoro-2-fluoro-phenyl)-4-cyano-5-(2,2-dimethylpropyl)-4-(4-fluoro-phenyl)-pyrrolidine-2-carboxylic acid ((S)-3,4-dihydroxy-butyl)-amide

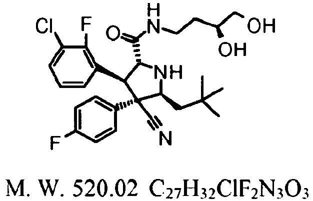

In a manner similar to the method described in **Example 42e**, rac-(2R,3S,4R,5S)-3-(3-chloro-2-fluoro-phenyl)-4-cyano-5-(2,2-dimethyl-propyl)-4-(4-fluoro-phenyl)-pyrrolidine-2-carboxylic acid trifluoroacetic acid prepared in **Example 55c** (0.4 g, 0.73 mmol) was reacted with 2-((S)-2,2-dimethyl-[1,3]dioxolan-4-yl)-ethylamine (0.2 g, 1.4 mmol), HATU (0.3 g, 0.8 mmol) and iPr₂NEt (0.6 g, 4.6 mmol) in CH₂Cl₂ at room temperature for 20 h, then reacted with aqueous HCl solution in tetrahydrofuran at room temperature for 2 h to give rac-(2R,3S,4R,5S)-3-(3-chloro-2-fluoro-phenyl)-4-cyano-5-(2,2-dimethyl-propyl)-4-(4-fluoro-phenyl)-pyrrolidine-2-carboxylic acid ((S)-3,4-dihydroxy-butyl)-amide as a white solid (0.21 g, 55%).

HRMS (ES⁺) m/z Calcd for C₂₇H₃₂ClF₂N₃O₃+ H [(M+H)⁺]: 520.2173, found: 520.2175.

### Example 56a

### Preparation of intermediate (Z)-3-(3-Chloro-2-fluoro-phenyl)-2-(2,4-dichloro-phenyl)-acrylonitrile

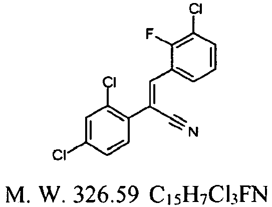

In a manner similar to the method described in **Example 1b**, 2, 4-dichlorobenzyl cyanide (6 g, 32 mmol) was reacted with 3-chloro-2-fluorobenzaldehyde (6 g, 38 mmol), methanolic solution (25 wt%) of sodium methoxide (30 mL, 131 mmol) in methanol (200 mL) at 50 °C for 3 h to give (Z)-3-(3-chloro-2-fluoro-phenyl)-2-(2,4-dichloro-phenyl)-acrylonitrile as a white powder (7 g, 67%).

### Example 56b

### Preparation of intermediate rac-(2R,3S,4R,5S)-3-(3-chloro-2-fluoro-phenyl)-4-cyano-4-(2,4-dichloro-phenyl)-5-(2,2-dimethyl-propyl)-pyrrolidine-2-carboxylic acid tert-butyl ester

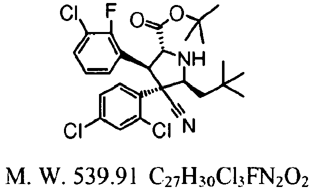

In a manner similar to the method described in **Example 1c**, [3-methyl-but-(E)-ylideneamino]-acetic acid tert-butyl ester prepared in **Example 1a** (2.1 g, 10 mmol) was reacted with (Z)-3-(3-chloro-2-fluoro-phenyl)-2-(2,4-dichloro-phenyl)-acrylonitrile (2.2 g, 6.7 mmol) prepared in **Example 56a**, AgF (2 g, 16 mmol), and triethylamine (5 g, 50 mmol) in dichloromethane (200 mL) at room temperature for 20 h to give rac-(2R,3S,4R,5S)-3-(3-chloro-2-fluoro-phenyl)-4-cyano-4-(2,4-dichloro-phenyl)-5-(2,2-dimethyl-propyl)-pyrrolidine-2-carboxylic acid tert-butyl ester as a white foam (2.4 g, 66%).

### Example 56c

### Preparation of intermediate rac-(2R,3S,4R,5S)-3-(3-chloro-2-fluoro-phenyl)-4-cyano-4-(2,4-dichloro-phenyl)-5-(2,2-dimethyl-propyl)-pyrrolidine-2-carboxylic acid trifluoroacetic acid

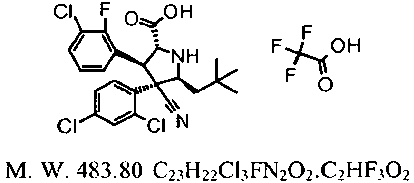

In a manner similar to the method described in **Example 25a**, rac-(2R,3S,4R,5S)-3-(3-chloro-2-fluoro-phenyl)-4-cyano-4-(2,4-dichloro-phenyl)-5-(2,2-dimethyl-propyl)-pyrrolidine-2-carboxylic acid tert-butyl ester prepared in **Example 56b** (2.4 g, 7.4 mmol) was reacted with trifluoroacetic acid in dichloromethane at room temperature to give rac-(2R,3S,4R,5S)-3-(3-chloro-2-fluoro-phenyl)-4-cyano-4-(2,4-dichloro-phenyl)-5-(2,2-dimethyl-propyl)-pyrrolidine-2-carboxylic acid trifluoroacetic acid as a white solid (2.7 g, 100%).

### Example 56d

### Preparation of rac-(2R,3S,4R,5S)-3-(3-chloro-2-fluoro-phenyl)-4-cyano-4-(2,4-dichloro-phenyl)-5-(2,2-dimethyl-propyl)-pyrrolidine-2-carboxylic acid ((S)-3,4-dihydroxy-butyl)-amide

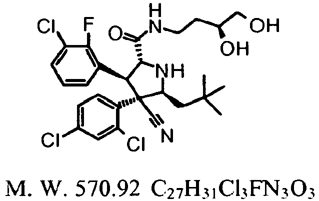

In a manner similar to the method described in **Examples 42c, 42d**, rac-(2R,3S,4R,5S)-3-(3-chloro-2-fluoro-phenyl)-4-cyano-4-(2,4-dichloro-phenyl)-5-(2,2-dimethyl-propyl)-pyrrolidine-2-carboxylic acid trifluoroacetic acid prepared in **Example 56c** (0.6 g, 1 mmol) was reacted with 2-((S)-2,2-dimethyl-[1,3]dioxolan-4-yl)-ethylamine (0.3 g, 2 mmol), HATU (0.5 g, 1.3 mmol) and iPr₂NEt (0.9 g, 7 mmol) in CH₂Cl₂ at room temperature for 20 h, then reacted with aqueous HCl solution in tetrahydrofuran at room temperature for 2 h to give rac-(2R,3S,4R,5S)-3-(3-chloro-2-fluoro-phenyl)-4-cyano-4-(2,4-dichloro-phenyl)-5-(2,2-dimethyl-propyl)-pyrrolidine-2-carboxylic acid ((S)-3,4-dihydroxy-butyl)-amide as a white solid (0.5 g, 88%).

HRMS (ES⁺) m/z Calcd for C₂₇H₃₁Cl₃FN₃O₃+H [(M+H)⁺]: 570.1488, found: 570.1487.

### Example 56e

### Preparation of (2R,3S,4R,5S)-3-(3-chloro-2-fluoro-phenyl)-4-cyano-4-(2,4-dichloro-phenyl)-5-(2,2-dimethyl-propyl)-pyrrolidine-2-carboxylic acid ((S)-3,4-dihydroxy-butyl)-amide

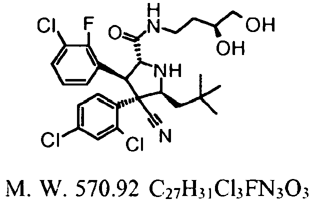

Rac-(2R,3S,4R,5S)-3-(3-chloro-2-fluoro-phenyl)-4-cyano-4-(2,4-dichloro-phenyl)-5-(2,2-dimethyl-propyl)-pyrrolidine-2-carboxylic acid ((S)-3,4-dihydroxy-butyl)-amide (0.5 g) was separated by chiral SFC chromatography to provide chiral-(2R,3S,4R,5S)-3-(3-chloro-2-fluoro-phenyl)-4-cyano-4-(2,4-dichloro-phenyl)-5-(2,2-dimethyl-propyl)-pyrrolidine-2-carboxylic acid ((S)-3,4-dihydroxy-butyl)-amide as a white solid (200 mg, 40%) and chiral-(2S,3R,4S,5R)-3-(3-chloro-2-fluoro-phenyl)-4-cyano-4-(2,4-dichloro-phenyl)-5-(2,2-dimethyl-propyl)-pyrrolidine-2-carboxylic acid ((S)-3,4-dihydroxy-butyl)-amide as a white solid (220 mg, 44%).

### Example 57a

### Preparation of intermediate (Z)-3-(3-chloro-2-fluoro-phenyl)-2-(4-chloro-2-methyl-phenyl)-acrylonitrile

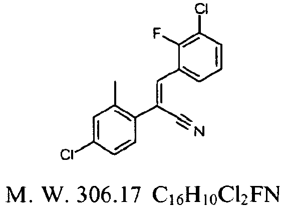

### Step A.

A mixture of4-chloro-2-methylbenzyl alcohol (Aldrich) (5 g, 32 mmol) in thionyl chloride (20 mL) was heated at refluxing (100 °C) for 30 min. The mixture was cooled to room temperature and concentrated. The residue was diluted with ethyl acetate, washed with saturated aqueous NaHCO₃ solution, water, brine, dried over MgSO₄, and concentrated to give 4-chloro-2-methylbenzyl chloride as a light yellow oil (5.2 g, 93%).

### Step B.

To a solution of4-chloro-2-methylbenzyl chloride (5.2 g, 30 mmol) in ethanol (40 mL) was added an aqueous solution (30 mL) of KCN (5 g, 77 mmol) at room temperature. The reaction mixture was then heated at 100 °C for 2 h. The mixture was cooled to room temperature and concentrated. The residue was partitioned between ethyl acetate and water. The organic layer was separated, concentrated. The residue was purifed by chromatography (EtOAc:hexanes=1;10, then 1:4) to give 4-chloro-2-methylbenzyl cyanide as a yellow oil (3.5 g, 66%).

### Step C

In a manner similar to the method described in **Example 1b**, 4-chloro-2-methylbenzyl cyanide (3.5 g, 21 mmol) was reacted with 3-chloro-2-fluorobenzaldehyde (5 g, 32 mmol), methanolic solution (25 wt%) of sodium methoxide (15 mL, 66 mmol) in methanol (100 mL) at 50 °C for 5 h to give (Z)-3-(3-chloro-2-fluoro-phenyl)-2-(4-chloro-2-methyl-phenyl)-acrylonitrile as a white powder (4 g, 62%).

### Example 57b

### Preparation of intermediate rac-(2R,3S,4R,5S)-3-(3-chloro-2-fluoro-phenyl)-4-(4-chloro-2-methyl-phenyl)-4-cyano-5-(2,2-dimethyl-propyl)-pyrrolidine-2-carboxylic acid tert-butyl ester

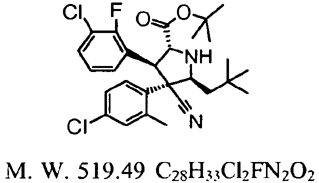

In a manner similar to the method described in **Example 1c**, [3-methyl-but-(E)-ylideneamino]-acetic acid tert-butyl ester prepared in **Example 1a** (2.1 g, 10 mmol) was reacted with (Z)-3-(3-chloro-2-fluoro-phenyl)-2-(4-chloro-2-methyl-phenyl)-acrylonitrile (2.3 g, 7.5 mmol) prepared in **Example 57a**, AgF (1.3 g, 10 mmol), and triethylamine (2.8 g, 28 mmol) in dichloromethane (100 mL) at room temperature for 20 h to give rac-(2R,3S,4R,5S)-3-(3-chloro-2-fluoro-phenyl)-4-(4-chloro-2-methyl-phenyl)-4-cyano-5-(2,2-dimethyl-propyl)-pyrrolidine-2-carboxylic acid tert-butyl ester as a white foam (1.9 g, 49%).

### Example 57c

### Preparation of intermediate rac-(2R,3S,4R,5S)-3-(3-chloro-2-fluoro-phenyl)-4-(4-chloro-2-mcthyl-phcnyl)-4-cyano-5-(2,2-dimethyl-propyl)-pyrrolidine-2-carboxylic acid trifluoroacetic acid

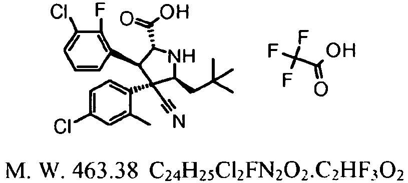

In a manner similar to the method described in **Example 25a**, rac-(2R,3S,4R,5S)-3-(3-chloro-2-fluoro-phenyl)-4-(4-chloro-2-methyl-phenyl)-4-cyano-5-(2,2-dimethyl-propyl)-pyrrolidine-2-carboxylic acid tert-butyl ester prepared in **Example 57b** (1.9 g, 3.7 mmol) was reacted with trifluoroacetic acid in dichloromethane at room temperature to give rac-(2R,3S,4R,5S)-3-(3-chloro-2-fluoro-phenyl)-4-(4-chloro-2-methyl-phenyl)-4-cyano-5-(2,2-dimethyl-propyl)-pyrrolidine-2-carboxylic acid trifluoroacetic acid as a white solid (2.1 g, 98%).

### Example 57d

### Preparation of rac-(2R,3S,4R,SS)-3-(3-chloro-2-fluoro-phenyl)-4-(4-chloro-2-methyl-phenyl)-4-cyano-5-(2,2-dimethyl-propyl)-pyrrolidine-2-carboxylic acid ((S)-3,4-dihydroxy-butyl)-amide

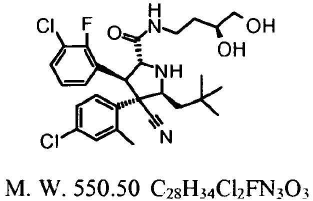

In a manner similar to the method described in **Examples 42c, 42d**, rac-(2R,3S,4R,5S)-3-(3-chloro-2-fluoro-phenyl)-4-(4-chloro-2-methyl-phenyl)-4-cyano-5-(2,2-dimethyl-propyl)-pyrrolidine-2-carboxylic acid trifluoroacetic acid prepared in **Example 57c** (0.4 g, 0.69 mmol) was reacted with 2-((S)-2,2-dimethyl-[1,3]dioxolan-4-yl)-ethylamine (0.2 g, 1.4 mmol), HATU (0.4 g, 1.1 mmol) and iPr₂NEt (0.8 g, 6.2 mmol) in CH₂Cl₂ at room temperature for 20 h, then reacted with aqueous HCl solution in tetrahydrofuran at room temperature for 2 h to give rac-(2R,35,4R,5S)-3-(3-chloro-2-fluoro-phenyl)-4-(4-chloro-2-methyl-phenyl)-4-cyano-5-(2,2-dimethyl-propyl)-pyrrolidine-2-carboxylic acid ((S)-3,4-dihydroxy-butyl)-amide as a white solid (0.29 g, 76%).

HRMS (ES⁺) m/z Calcd for C₂₈H₃₄Cl₂FN₃O₃+ H [(M+H)⁺]: 550.2034, found: 550.2036.

### Example 58a

### Preparation of intermediate (Z)-3-(3-chloro-2-fluoro-phenyl)-2-(4-chloro-2-methoxy-phenyl)-acrylonitrile

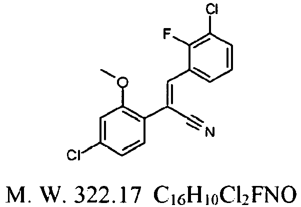

### Step A.

In a manner similar to the method described in **Example 57 Step A**, 4-chloro-2-methoxybenzyl alcohol (Aldrich) (4.9 g, 28 mmol) was reacted with thionyl chloride (20 mL) to give 4-chloro-2-methoxybenzyl chloride as a white solid (5.1 g, 95%).

### Step B

In a manner similar to the method described in **Example 57 Step B**, 4-chloro-2-methoxybenzyl chloride (5.1 g, 27 mmol) was reacted with NaCN (3 g, 61 mmol) in ethanol (40 mL) and water (20 mL) at 100 °C for 8 h to give 4-chloro-2-methoxybenzyl cyanide as a colorless oil (1.8 g, 36%)

### Step C

In a manner similar to the method described in **Example 1b**, 4-chloro-2-methoxybenzyl cyanide (1.8 g, 10 mmol) was reacted with 3-chloro-2-fluorobenzaldehyde (2 g, 13 mmol), methanolic solution (25 wt%) of sodium methoxide (15 mL, 66 mmol) in methanol (50 mL) at 50 °C for 2 h to give (Z)-3-(3-chloro-2-fluoro-phenyl)-2-(4-chloro-2-methoxy-phenyl)-acrylonitrile as a white powder (2.1 g, 65%).

### Example 58b

### Preparation of intermediate rac-(2S,3S,4R,5S)-3-(3-chloro-2-fluoro-phenyl)-4-(4-chloro-2-methoxy-phenyl)-4-cyano-5-(2,2-dimethyl-propyl)-pyrrolidine-2-carboxylic acid tert-butyl ester

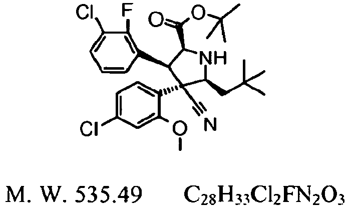

In a manner similar to the method described in **Example 1c**, [3-methyl-but-(E)-ylideneamino]-acetic acid tert-butyl ester prepared in **Example 1a** (2.1 g, 10 mmol) was reacted with (Z)-3-(3-chloro-2-fluoro-phenyl)-2-(4-chloro-2-methoxy-phenyl)-acrylonitrile (1.8 g, 5.6 mmol) prepared in **Example 58a**, AgF (1.7 g, 13 mmol), and triethylamine (2.8 g, 28 mmol) in dichloromethane (100 mL) at room temperature for 24 h to give rac-(2S,3S,4R,5S)-3-(3-chloro-2-fluoro-phenyl)-4-(4-chloro-2-methoxy-phenyl)-4-cyano-5-(2,2-dimethyl-propyl)-pyrrolidine-2-carboxylic acid tert-butyl ester as a white foam (1.8 g, 60%).

### Example 58c

### Preparation of intermediate rac-(2S,3S,4R,5S)-3-(3-chloro-2-fluoro-phenyl)-4-(4-chloro-2-methoxy-phenyl)-4-cyano-5-(2,2-dimethyl-propyl)-pyrrolidine-2-carboxylic acid trifluoroacetic acid

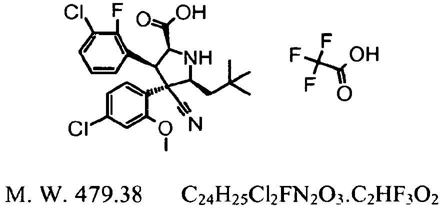

In a manner similar to the method described in **Example 25a**, rac-(2S,3S,4R,5S)-3-(3-chloro-2-fluoro-phenyl)-4-(4-chloro-2-methoxy-phenyl)-4-cyano-5-(2,2-dimethyl-propyl)-pyrro lidine-2-carboxylic acid tert-butyl ester prepared in **Example 58b** (1.3 g, 2.4 mmol) was reacted with trifluoroacetic acid in dichloromethane at room temperature to give rac-(2S,3S,4R,5S)-3-(3-chloro-2-fluoro-phenyl)-4-(4-chloro-2-methoxy-phenyl)-4-cyano-5-(2,2-dimethyl-propyl)-pyrrolidine-2-carboxylic acid trifluoroacetic acid as a white solid (1.5 g, 100%).

### Example 58d

### Preparation of rac-(2S,3S,4R,5S)-3-(3-chloro-2-fluoro-phenyl)-4-(4-chloro-2-methoxy-phenyl)-4-cyano-5-(2,2-dimethyl-propyl)-pyrrolidine-2-carboxylic acid ((S)-3,4-dihydroxy-butyl)-amide

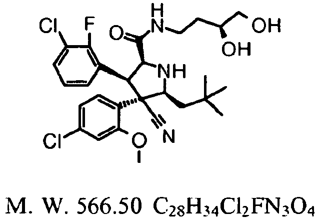

In a manner similar to the method described **in Examples 42c, 42d**, rac-(2S,3S,4R,5S)-3-(3-chloro-2-fluoro-phenyl)-4-(4-chloro-2-methoxy-phenyl)-4-cyano-5-(2,2-dimethyl-propyl)-pyrrolidine-2-carboxylic acid trifluoroacetic acid prepared in **Example 58c** (0.4 g, 0.67 mmol) was reacted with 2-((S)-2,2-dimethyl-[1,3]dioxolan-4-yl)-ethylamine (0.25 g, 1.7 mmol), HATU (0.4 g, 1.1 mmol) and iPr₂NEt (0.6 g, 4.7 mmol) in CH₂Cl₂ at room temperature for 20 h, then reacted with aqueous HCl solution in tetrahydrofuran at room temperature for 2 h to give rac-(2S,3S,4R,5S)-3-(3-chloro-2-fluoro-phenyl)-4-(4-chloro-2-methoxy-phenyl)-4-cyano-5-(2,2-dimethyl-propyl)-pyrrolidine-2-carboxylic acid ((S)-3,4-dihydroxy-butyl)-amide as a white solid (0.23 g, 61%).

HRMS (ES⁺) m/z Calcd for C₂₈H₃₄Cl₂FN₃O₄+ H [(M+H⁺]*:* 566.1983, found: 566. 1983.

### Example 59a

### Preparation of intermediate [2-cyclohexyl-eth-(E)-ylideneamino]-acetic acid tert-butyl ester

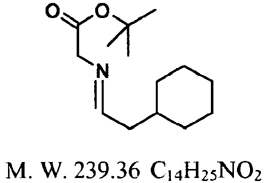

In a manner similar to the method described in **Example 1a**, glycine tert-butyl ester (1.3 g, 10 mmol) was reacted with 2-cyclohexylacetaldehyde (Betapharma) (1.3 g, 10 mmol) in CH₂Cl₂ at room temperature for 5 h to give [2-cyclohexyl-eth-(E)-ylideneamino]-acetic acid tert-butyl ester as a colorless oil (2.3 g, 96%).

### Example 59b

### Preparation of intermediate rac-(2R,3S,4R,5S)-3-(3-chloro-2-fluoro-phenyl)-4-(4-chloro-phenyl)-4-cyano-5-cyclohexylmethyl-pyrrolidine-2-carboxylic acid tert-butyl ester

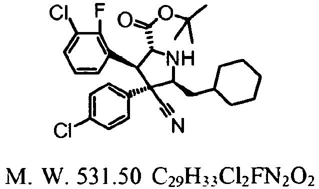

In a manner similar to the method described in **Example 1c**, [2-cyclohexyl-eth-(E)-ylideneamino]-acetic acid tert-butyl ester prepared in **Example 59a** (2.3 g, 10 mmol) was reacted with (Z)-3-(3-chloro-2-fluoro-phenyl)-2-(4-chloro-phenyl)-acrylonitrile(1.9 g, 6.5 mmol) prepared in **Example 26a**, AgF (1.7 g, 13 mmol), and triethylamine (2.6 g, 26 mmol) in dichloromethane (100 mL) at room temperature for 18 h to give rac-(2R,3S,4R,5S)-3-(3-chloro-2-fluoro-phenyl)-4-(4-chloro-phenyl)-4-cyano-5-cyclohexylmethyl-pyrrolidine-2-carboxylic acid tert-butyl ester as a white foam (1.9 g, 55%).

### Example 59c

### Preparation of intermediate rac-(2R,3S,4R,5S)-3-(3-chloro-2-fluoro-phenyl)-4-(4-chloro-phenyl)-4-cyano-5-cyclohexylmethyl-pyrrolidine-2-carboxylic acid trifluoroacetic acid

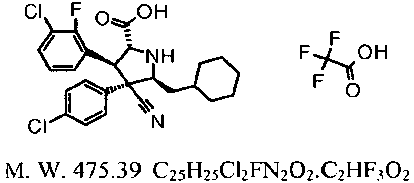

In a manner similar to the method described in **Example 25a**, rac-(2R,3S,4R.5S)-3-(3-chloro-2-fluoro-phenyl)-4-(4-chloro-phenyl)-4-cyano-5-cyclohexylmethyl-pyrrolidine-2-carboxylic acid tert-butyl ester prepared in **Example 59b** (1.9 g, 3.6 mmol) was reacted with trifluoroacetic acid in dichloromethane at room temperature to give rac-(2R,3S,4R,5S)-3-(3-chloro-2-fluoro-phenyl)-4-(4-chloro-phenyl)-4-cyano-5-cyclohexylmethyl-pyrrolidine-2-carboxylic acid trifluoroacetic acid as a white solid (2.1 g, 99%).

### Example 59d

### Preparation of rac-(2R,3S,4R,5S)-3-(3-chloro-2-fluoro-phenyl)-4-(4-chloro-phenyl)-4-cyano-5-cyclohexylmethyl-pyrrolidine-2-carboxylic acid ((S)-3,4-dihydroxy-butyl)-amide

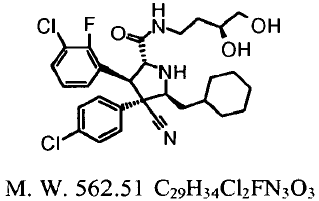

In a manner similar to the method described in **Examples 42c, 42d**, rac-(2R,3S,4R,5S)-3-(3-chloro-2-fluoro-phenyl)-4-(4-chloro-phenyl)-4-cyano-5-cyclohexylmethyl-pyrrolidine-2-carboxylic acid trifluoroacetic acid prepared in **Example 59c** (0.6 g, 1 mmol) was reacted with 2-((S)-2,2-dimethyl-[1,3]dioxolan-4-yl)-ethylamine (0.3 g, 2 mmol), HATU (0.6 g, 1.6 mmol) and iPr₂NEt (0.9 g, 7 mmol) in CH₂Cl₂ at room temperature for 20 h, then reacted with aqueous HCl solution in tetrahydrofuran at room temperature for 2 h to give rac-(2R,3S,4R,5S)-3-(3-chloro-2-fluoro-phenyl)-4-(4-chloro-phenyl)-4-cyano-5-cyclohexylmethyl-pyrrolidine-2-carboxylic acid ((S)-3,4-dihydroxy-butyl)-amide as a white solid (0.4 g, 71%).

HRMS (ES⁺) m/z Calcd for C₂₉H₃₄Cl₂FN₃O₃+ H [(M+H)⁺]: 562.2034, found: 562.2033.

### Example 60a

### Preparation of intermediate rac-(2S,3S,4R,5S)-3-(3-chloro-2-fluoro-phenyl)-4-(4-chloro-phenyl)-4-cyano-5-cyclohexylmethyl-pyrrolidine-2-carboxylic acid tert-butyl ester

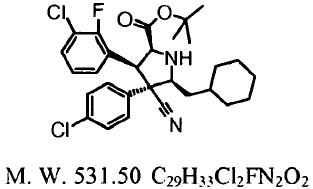

In preparation of rac-(2R,3S,4R,5S)-3-(3-chloro-2-fluoro-phenyl)-4-(4-chloro-phenyl)-4-cyano-5-cyclohexylmethyl-pyrrolidine-2-carboxylic acid tert-butyl ester as described in **Example 59b**, rac-(2S,3S,4R,5S)-3-(3-chloro-2-fluoro-phenyl)-4-(4-chloro-phenyl)-4-cyano-5-cyclohexylmethyl-pyrrolidine-2-carboxylic acid tert-butyl ester was obtained as the second product: a white foam, Yield, 1 g, 29%.

### Example 60b

### Preparation of intermediate rac-(2S,3S,4R,5S)-3-(3-chloro-2-fluoro-phenyl)-4-(4-chloro-phenyl)-4-cyano-5-cyclohexylmethyl-pyrrolidine-2-carboxylic acid trifluoroacetic acid

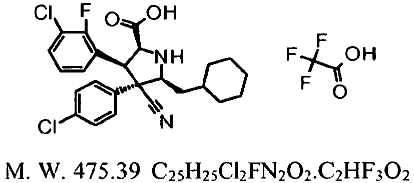

In a manner similar to the method described in **Example 25a**, rac-(2S,3S,4R,5S)-3-(3-chloro-2-fluoro-phenyl)-4-(4-chloro-phenyl)-4-cyano-5-cyclohexylmethyl-pyrrolidine-2-carboxylic acid tert-butyl ester prepared in **Example 60a** (0.4 g, 0.75 mmol) was reacted with trifluoroacetic acid in dichloromethane at room temperature to give rac-(2S,3S,4R,5S)-3-(3-chloro-2-fluoro-phenyl)-4-(4-chloro-phenyl)-4-cyano-5-cyclohexylmethyl-pyrrolidine-2-carboxylic acid trifluoroacetic acid as a white solid (0.46 g, 100%).

### Example 60c

### Preparation of rac-(2S,3S,4R,5S)-3-(3-chloro-2-fluoro-phenyl)-4-(4-chloro-phenyl)-4-cyano-5-cyclohexylmethyl-pyrrolidine-2-carboxylic acid ((S)-3,4-dihydroxy-butyl)-amide

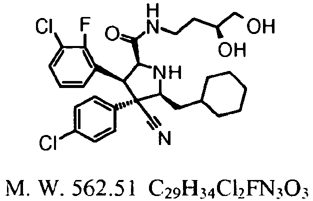

In a manner similar to the method described in **Example 42e**, rac-(2S,3S,4R,5S)-3-(3-chloro-2-fluoro-phenyl)-4-(4-chloro-phenyl)-4-cyano-5-cyclohexylmethyl-pyrrolidine-2-carboxylic acid trifluoroacetic acid prepared in **Example 60b** (0.45 g, 0.75 mmol) was reacted with 2-((S)-2,2-dhnethyl-[1,3]dioxolan-4-yl)-ethylamine (0.23 g, 1.6 mmol), HATU (0.45 g, 1.2 mmol) and iPr₂NEt (0.9 g, 7 mmol) in CH₂Cl₂ at room temperature for 20 h, then reacted with aqueous HCl solution in tetrahydrofuran at room temperature for 2 h to give rac-(2S,3S,4R,5S)-3-(3-chloro-2-fluoro-phenyl)-4-(4-chloro-phenyl)-4-cyano-5-cyclohexylmethyl-pyrrolidine-2-carboxylic acid ((S)-3,4-dihydroxy-butyl)-amide as a white solid (0.4 g, 95%).

HRMS (ES⁺) m/z Calcd for C₂₉H₃₄Cl₂N₃O₃+ H [(M+H)⁺]: 562.2034, found: 562.2033.

### Example 61a

### Preparation of intermediate (Z)-2-(4-chloro-2-methoxy-phenyl)-3-(3-chloro-phenyl)-acrylonitrile

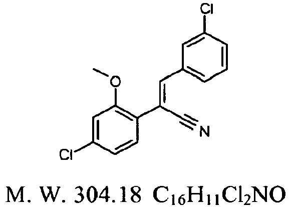

In a manner similar to the method described in **Example 1b**, 4-chloro-2-methoxybenzyl cyanide (2 g, 10 mmol) prepared in **Example 58a Step B** was reacted with 3-chlorobenzaldehyde (2 g, 14 mmol), methanolic solution (25 wt%) of sodium methoxide (15 mL, 66 mmol) in methanol (50 mL) at 50 °C for 5 h to give (Z)-2-(4-chloro-2-methoxy-phenyl)-3-(3-chloro-phenyl)-acrylonitrile as a white powder (1.9 g, 63%).

### Example 61b

### Preparation of intermediate rac-(2S,3R,4R,5S)-4-(4-chloro-2-methoxy-phenyl)-3-(3-chloro-phenyl)-4-cyano-5-(2,2-dimethyl-propyl)-pyrrolidine-2-carboxylic acid tert-butyl ester

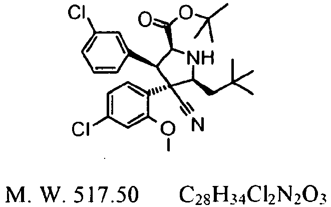

In a manner similar to the method described in **Example 1c**, [3-methyl-but-(E)-ylideneamino]-acetic acid tert-butyl ester prepared in **Example 1a** (2.1 g, 10 mmol) was reacted with (Z)-2-(4-chloro-2-methoxy-phenyl)-3-(3-chloro-phenyl)-acrylonitrile (1.8 g, 5.9 mmol) prepared in **Example 61a**, AgF (1.7 g, 13 mmol), and triethylamine (2.8 g, 28 mmol) in dichloromethane (100 mL) at room temperature for 24 h to give rac-(2S,3S,4R,5S)-3-(3-chloro-2-fluoro-phenyl)-4-(4-chloro-2-methoxy-phenyl)-4-cyano-5-(2,2-dimethyl-propyl)-pyrrolidine-2-carboxylic acid tert-butyl ester as a white foam (1.8 g, 60%).

### Example 61c

### Preparation of intermediate rac-(2S,3R,4R,5S)-4-(4-chloro-2-methoxy-phenyl)-3-(3-chloro-phenyl)-4-cyano-5-(2,2-dimethyl-propyl)-pyrrolidine-2-carboxylic acid trifluoroacetic acid

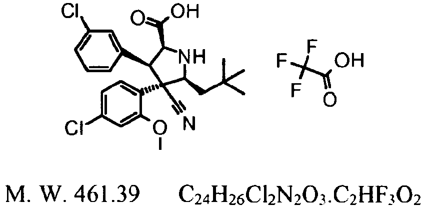

In a manner similar to the method described in **Example 25a**, rac-(2S,3R,4R,5S)-4-(4-chloro-2-methoxy-phenyl)-3-(3-chloro-phenyl)-4-cyano-5-(2,2-dimethyl-propyl)-pyrro lidine-2-carboxylic acid tert-butyl ester prepared in **Example 61b** (2 g, 3.9 mmol) was reacted with trifluoroacetic acid in dichloromethane at room temperature to give rac-(2S,3R,4R,5S)-4-(4-chloro-2-methoxyphenyl)-3-(3-chloro-phenyl)-4-cyano-5-(2,2-dimethyl-propyl)-pyrrolidine-2-carboxylic acid trifluoroacetic acid as a white solid (2.2 g, 98%).

### Example 61d

### Preparation of rac-(2S,3R,4R,5S)-4-(4-chloro-2-methoxy-phenyl)-3-(3-chloro-phenyl)-4-cyano-5-(2,2-dimethyl-propyl)-pyrrolidine-2-carboxylic acid ((S)-3,4-dihydroxy-butyl)-amide

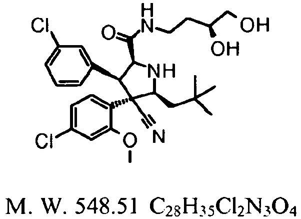

In a manner similar to the method described in **Examples 42c, 42d**, rac-(2S,3R,4R.5S)-4-(4-chloro-2-methoxy-phenyl)-3-(3-chloro-phenyl)-4-cyano-5-(2,2-dimethyl-propyl)-pyrrolidine-2-carboxylic acid trifluoroacetic acid prepared in **Example 61c** (0.2 g, 0.35 mmol) was reacted with 2-((S)-2,2-dimethyl-[1,3]dioxolan-4-yl)-ethylamine (0.15 g, 1.0 mmol), HATU (0.24 g, 0.63 mmol) and iPr₂NEt (0.3 mL, 1.7 mmol) in CH₂Cl₂ at room temperature for 20 h, then reacted with aqueous HCl solution in tetrahydrofuran at room temperature for 2 h to give rac-(2S,3R,4R,5S)-4-(4-chloro-2-methoxy-phenyl)-3-(3-chloro-phenyl)-4-cyano-5-(2,2-dimethylpropyl)-pyrrolidine-2-carboxylic acid ((S)-3,4-dihydroxy-butyl)-amide as a white solid (0.15 g, 84%).

HRMS (ES⁺) m/z Calcd for C₂₈H₃₅Cl₂N₃O₄ + H [(M+H)⁺]: 548,2078, found: 548.2077.

### Example 62a

### Preparation of intermediate (Z)-2-(4-chloro-2-fluoro-phenyl)-3-(2,3-difluoro-phenyl)-acrylonitrile

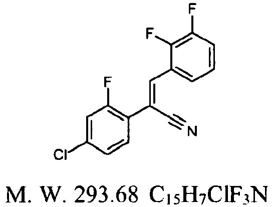

In a manner similar to the method described in **Example 1b**, 4-chloro-2-fluorophenylacetonitrile (4.5 g, 26 mmol) was reacted with 2,3-difluorobenzaldehyde (Aldrich) (4.5 g, 32 mmol), methanolic solution (25 wt%) of sodium methoxide (6.3 g, 29 mmol) in methanol (135 mL) at 50 °C for 3 h to give (Z)-2-(4-chloro-2-fluoro-phenyl)-3-(2,3-difluoro-phenyl)-acrylonitrile as a white powder (6.85 g, 88%).

### Example 62b

### Preparation of intermediate rac-(2R,3S,4R,5S)-4-(4-ch(oro-2-fluoro-phenyl)-4-cyano-3-(2,3-difluoro-phenyl)-5-(2,2-dimethyl-propyl)-pyrrolidine-2-carboxylic acid tert-butyl ester

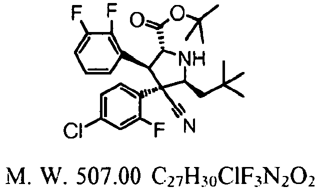

In a manner similar to the method described in **Example 1c**, [3-methyl-but-(E)-ylideneamino]-acetic acid tert-butyl ester prepared in **Example 1a** (2.1 g, 10 mmol) was reacted with (Z)-2-(4-chloro-2-fluoro-phenyl)-3-(2,3-difluoro-phenyl)-acrylonitrile (2.3 g, 8 mmol) prepared in **Example 62a**, AgF (1.5 g, 12 mmol), and triethylamine (2.7 mL, 20 mmol) in dichloromethane (120 mL) at room temperature for 18 h to give rac-(2R,3S,4R,5S)-4-(4-chloro-2-fluoro-phenyl)-4-cyano-3-(2,3-difluoro-phenyl)-5-(2,2-dimethyl-propyl)-pyrrolidine-2-carboxylic acid tert-butyl ester as a white foam (1.8 g, 44%).

### Example 62c

### Preparation of intermediate rac-(2R,3S,4R,5S)-4-(4-chloro-2-fluoro-phenyl)-4-cyano-3-(2,3-difluoro-phenyl)-5-(2,2-dimethyl-propyl)-pyrrolidine-2-carboxylic acid trifluoroacetic acid

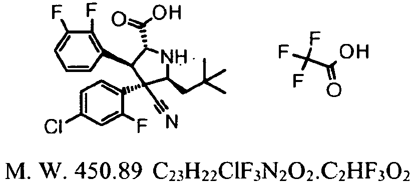

In a manner similar to the method described in **Example 25a**, rac-(2R,3S,4R,5S)-4-(4-chloro-2-fluoro-phenyl)-4-cyano-3-(2,3-difluoro-phenyl)-5-(2,2-dimethyl-propyl)-pyrrolidine-2-carboxylic acid tert-butyl ester prepared in **Example 62b** (1.8 g, 3.6 mmol) was reacted with trifluoroacetic acid in dichloromethane at room temperature to give rac-(2R,3S,4R,5S)-4-(4-chloro-2-fluoro-phenyl)-4-cyano-3-(2,3-difluoro-phenyl)-5-(2,2-dimethyl-propyl)-pyrrolidine-2-carboxylic acid trifluoroacetic acid as a white solid (2 g, 100%).

HRMS (ES⁺) m/z Calcd for C₂₃H₂₂ClF₃N₂0O₂+ H [(M+H)⁺]: 451.1395, found: 451.1394.

### Example 62d

### Preparation of rac-(2R,3S,4R,5S)-4-(4-chloro-2-fluoro-phenyl)-4-cyano-3-(2,3-difluoro-phenyl)-5-(2,2-dimethyl-propyl)-pyrrolidine-2-carboxylic acid ((S)-3,4-dihydroxy-butyl)-amide

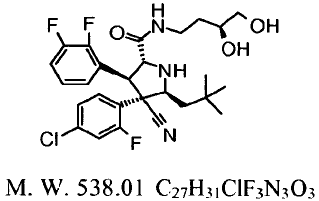

In a manner similar to the method described in **Examples 42c, 42d**, rac-(2R,3S,4R,5S)-4-(4-chloro-2-fluoro-phenyl)-4-cyano-3-(2,3-difluoro-phenyl)-5-(2,2-dimethyl-propyl)-pyrrolidine-2-carboxylic acid trifluoroacetic acid prepared in **Example 62c**(0.47 g, 0.83 mmol) was reacted with 2-((S)-2,2-dimethyl-[1,3]dioxolan-4-yl)-ethylamine (0.36 g, 2.5 mmol), HATU (0.57 g, 1.49 mmol) and iPr₂NEt (0.72 mL, 4.2 mmol) in CH₂Cl₂ at room temperature for 20 h, then reacted with aqueous HCl solution in tetrahydrofuran at room temperature for 2 h to give rac-(2R,3S,4R,5S)-4-(4-chloro-2-fluoro-phenyl)-4-cyano-3-(2,3-difluoro-phenyl)-5-(2,2-dimethylpropyl)-pyrrolidine-2-carboxylic acid ((S)-3,4-dihydroxy-butyl)-amide as a white solid (0.26 g, 58%).

HRMS (ES⁺) m/z Calcd for C₂₇H₃₁Cl₂F₂N₃O₃+ H [(M+H)⁺]: 538.2079, found: 538.2077.

### Example 62e

### Preparation of (2R,3S,4R,5S)-4-(4-chloro-2-fluoro-phenyl)-4-cyano-3-(2,3-difluoro-phenyl)-5-(2,2-dimethyl-propyl)-pyrrolidine-2-carboxylic acid ((S)-3,4-dihydroxy-butyl)-amide

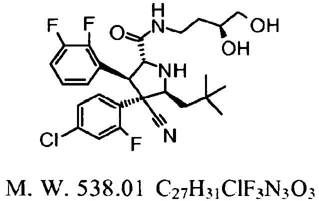

Rac-(2R,3S,4R,5S)-4-(4-chloro-2-fluoro-phenyl)-4-cyano-3-(2,3-difluoro-phenyl)-5-(2,2-dimethyl-propyl)-pyrrolidine-2-carboxylic acid ((S)-3,4-dihydroxy-butyl)-amide (0.22 g) was separated by chiral SFC chromatography to provide chiral-(2R,3S,4R,5S)-4-(4-chloro-2-fluoro-phenyl)-4-cyano-3-(2,3-difluoro-phenyl)-5-(2,2-dimethyl-propyl)-pyrrolidine-2-carboxylic acid ((S)-3,4-dihydroxy-butyl)-amide as a white solid (87 mg, 40%) and chiral-(2S,3R,4S,5R)-4-(4-chloro-2-fluoro-phenyl)-4-cyano-3-(2,3-difluoro-phenyl)-5-(2,2-dimethyl-propyl)-pyrrolidine-2-carboxylic acid ((S)-3,4-dihydroxy-butyl)-amide as a white solid (87 mg, 40%).

### Example 63a

### Preparation of intermediate (Z)-3-(3-bromo-2-fluoro-phenyl)-2-(4-chloro-2-fluoro-phenyl)-acrylonitrile

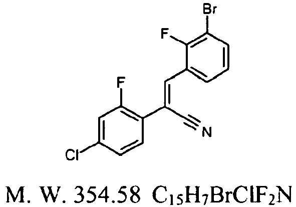

In a manner similar to the method described in **Example 1b**, 4-chloro-2-fluorophenylacetonitrile (1.39 g, 8.2 mmol) was reacted with 3-bromo-2-fluorobenzaldehyde (Apollo)(2 g, 9.9 mmol), methanolic solution (25 wt%) of sodium methoxide (2 g, 9 mmol) in methanol (40 mL) at 50 °C for 3 h to give (Z)-3-(3-bromo-2-fluoro-phenyl)-2-(4-chloro-2-fluoro-phenyl)-acrylonitrile as a white powder (2.3 g, 79%).

### Example 63b

### Preparation of intermediate rac-(2R,3S,4R,5S)-3-(3-bromo-2-fluoro-phenyl)-4-(4-chloro-2-fluoro-phenyl)-4-cyano-5-(2,2-dimethyl-propyl)-pyrrolidine-2-carboxylic acid tert-butyl ester

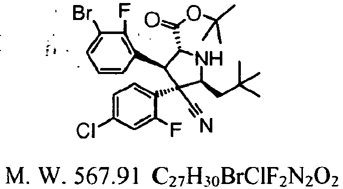

In a manner similar to the method described in **Example 1c**, [3-methyl-but-(E)-ylideneamino]-acetic acid tert-butyl ester prepared in **Example 1a** (2.1 g, 10 mmol) was reacted with (Z)-3-(3-bromo-2-fluoro-phenyl)-2-(4-chloro-2-fluoro-phenyl)-acrylonitrile (2.3 g, 6.5 mmol) prepared in **Example 63a**, AgF (1.5 g, 12 mmol), and triethylamine (2.7 mL, 20 mmol) in dichloromethane (120 mL) at room temperature for 18 h to give rac-(2R,3S,4R,5S)-3-(3-bromo-2-fluoro-phenyl)-4-(4-chloro-2-fluoro-phenyl)-4-cyano-5-(2,2-dimethyl-propyl)-pyrrolidine-2-carboxylic acid tert-butyl ester as a white foam (2 g, 54%).

### Example 63c

### Preparation of intermediate rac-(2R,3S,4R,5S)-3-(3-bromo-2-fluoro-phenyl)-4-(4-chloro-2-fluoro-phenyl)-4-cyano-5-(2,2-dimethyl-propyl)-pyrrolidine-2-carboxylic acid trifluoroacetic acid

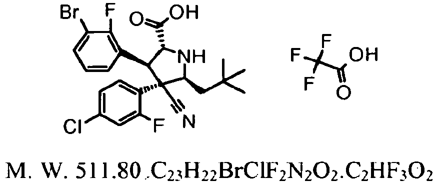

In a manner similar to the method described in **Example 1d**, rac-(2R,3S,4R,5S)-3-(3-bromo-2-fluoro-phenyl)-4-(4-chloro-2-fluoro-phenyl)-4-cyano-5-(2,2-dimethyl-propyl)-pyrrolidine-2-carboxylic acid tert-butyl ester prepared in **Example 63b** (2 g, 3.5 mmol) was reacted with trifluoroacetic acid in dichloromethane at room temperature to give rac-(2R,3S,4R,5S)-3-(3-bromo-2-fluoro-phenyl)-4-(4-chloro-2-fluoro-phenyl)-4-cyano-5-(2,2-dimethyl-propyl)-pyrrolidine-2-carboxylic acid trifluoroacetic acid as a white solid (2.1 g, 95%).

HRMS (ES⁺) m/z Calcd for C₂₃H₂₂BrClF₂N₂O₂+ H [(M+H)⁺]: 511.0594, found: 511.0595.

### Example 63d

### Preparation of rac-(2R,3S,4R,5S)-3-(3-bromo-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide

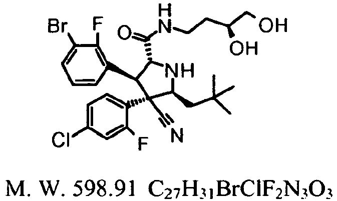

In a manner similar to the method described in **Examples 42c, 42d**, rac-(2R,3S,4R,5S)-3-(3-bromo-2-fluoro-phenyl)-4-(4-chloro-2-fluoro-phenyl)-4-cyano-5-(2,2-dimethyl-propyl)-pyrrolidine-2-carboxylic acid trifluoroacetic acid prepared in **Example 63c** (0.51 g, 0.83 mmol) was reacted with 2-((S)-2,2-dimethyl-[1,3]dioxolan-4-yl)-ethylamine (0.36 g, 2.5 mmol), HATU (0.57 g, 1.49 mmol) and iPr₂NEt (0.72 mL, 4.2 mmol) in CH₂Cl₂ at room temperature for 20 h, then reacted with aqueous HCl solution in tetrahydrofuran at room temperature for 2 h to give rac-(2R,3S,4R,5S)-3-(3-bromo-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide as a white solid (0.2 g, 40%).

HRMS (ES⁺) m/z Calcd for C₂₇H₃₁BrClF₂N₃O₃+ H [(M+H)⁺]: 598.1278, found: 398.1278.

### Example 63e

### Preparation of (2R,3S,4R,5S)-3-(3-bromo-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide

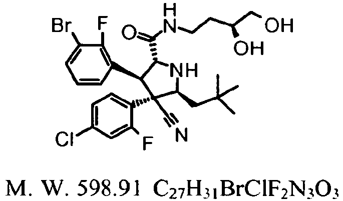

Rac-(2R,3S,4R,5S)-3-(3-bromo-2-fluoro-phenyl)-4-(4-chloro-2-fluoro-phenyl)-4-cyano-5-(2,2-dimethyl-propyl)-pyrrolidine-2-carboxylic acid ((S)-3.,4-dihydroxy-butyl)-amide (0.15 g) was separated by chiral SFC chromatography to provide chiral (2R,3S,4R,5S)-3-(3-bromo-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide as a white solid (66 mg, 44%) and chiral (2S,3R,4S,5R)-3-(3-bromo-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide as a white solid (70 mg, 47%).

### Example 64a

### Preparation of intermediate (Z)-2-(4-chloro-2-fluoro-phenyl)-3-(3-chloro-phenyl)-acrylonitrile

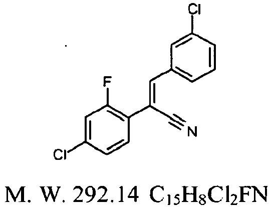

In a manner similar to the method described in **Example 1b**, 4-chloro-2-fluorophenylacetonitrile (4.5 g, 26 mmol) was reacted with 3-chlorobenzaldehyde (Aldrich) (4.4 g, 32 mmol), methanolic solution (25 wt%) of sodium methoxide (6.6 mL, 29 mmol) in methanol (150 mL) at 50 °C for 3 h to give (Z)-2-(4-Chloro-2-fluoro-phenyl)-3-(3-chloro-phenyl)-acrylonitrile as a white powder (6.5 g, 84%).

### Example 64b

### Preparation of intermediate rac-(2R,3R,4R,5S)-4-(4-chloro-2-fluoro-phenyl)-3-(3-chloro-phenyl)-4-cyano-5-(2,2-dimethyl-propyl)-pyrrolidine-2-carboxylic acid tert-butyl ester

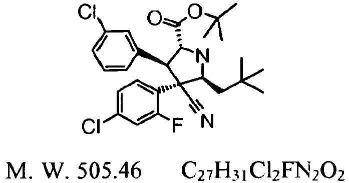

In a manner similar to the method described in **Example 1c**, [3-methyl-but-(E)-ylideneamino]-acetic acid tert-butyl ester prepared in **Example 1a** (2.1 g, 10 mmol) was reacted with (Z)-2-(4-chloro-2-fluoro-phenyl)-3-(3-chloro-phenyl)-acrylonitrile (2.3 g, 8 mmol) prepared in **Example 64a**, AgF (1.5 g, 12 mmol), and triethylamine (2.7 mL, 20 mmol) in dichloromethane (120 mL) at room temperature for 18 h to give rac-(2R,3R,4R,5S)-4-(4-chloro-2-fluoro-phenyl)-3-(3-chloro-phenyl)-4-cyano-5-(2,2-dimethyl-propyl)-pyrrolidine-2-carboxylic acid tert-butyl ester as a white foam (1.02 g, 25%).

### Example 64c

### Preparation of intermediate rac-(2R,3R,4R,5S)-4-(4-chloro-2-fluoro-phenyl)-3-(3-chloro-phenyl)-4-cyano-5-(2,2-dimethyl-propyl)-pyrrolidine-2-carboxylic acid trifluoroacetic acid

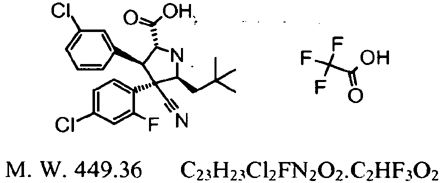

In a manner similar to the method described in **Example 1d**, rac-(2R,3R,4R,5S)-4-(4-chloro-2-fluoro-phenyl)-3-(3-chloro-phenyl)-4-cyano-5-(2,2-dimethyl-propyl)-pyrrolidine-2-carboxylic acid tert-butyl ester prepared in **Example 64b** (1 g, 2 mmol) was reacted with trifluoroacetic acid in dichloromethane at room temperature to give rac-(2R,3R,4R,5S)-4-(4-chloro-2-fluoro-phenyl)-3-(3-chloro-phenyl)-4-cyano-5-(2,2-dimethyl-propyl)-pyrrolidine-2-carboxylic acid trifluoroacetic acid as a white solid (0.88 g, 79%).

HRMS (ES⁺) m/z Calcd for C₂₃H₂₃Cl₂FN₂O₂+ H [(M+H)⁺]: 449.1194, found: 449.1194.

### Example 64d

### Preparation of rac-(2R,3R,4R,5S)-4-(4-chloro-2-fluoro-phenyl)-3-(3-chloro-phenyl)-4-cyano-5-(2,2-dimethyl-propyl)-pyrrolidine-2-carboxylic acid ((S)-3,4-dihydroxy-butyl)-amide

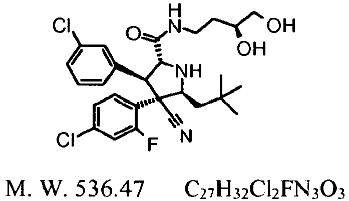

In a manner similar to the method described in **Examples 42c, 42d,** rac-(2R,3R,4R,5S)-4-(4-chloro-2-fluoro-phenyl)-3-(3-chloro-phenyl)-4-cyano-5-(2,2-dimethyl-propyl)-pyrrolidine-2-carboxylic acid trifluoroacetic acid prepared in **Example 64c** (0.46 g, 0.83 mmol) was reacted with 2-((S)-2,2-dimethyl-[1,3]dioxolan-4-yl)-ethylamine (0.36 g, 2.5 mmol), HATU (0.57 g, 1.5 mmol) and iPr₂NEt (0.72 mL, 4.2 mmol) in CH₂Cl₂ at room temperature for 20 h, then reacted with aqueous HCl solution in tetrahydrofuran at room temperature for 2 h to give rac-(2R,3R,4R,5S)-4-(4-chloro-2-fluoro-phenyl)-3-(3-chloro-phenyl)-4-cyano-5-(2,2-dimethylpropyl)-pyrrolidine-2-carboxylic acid ((S)-3,4-dihydroxy-butyl)-amide as a white solid (0.21 g, 48%).

HRMS (ES⁺) m/z Calcd for C₂₇H₃₂Cl₂FN₃O₃+ H [(M+H)⁺]: 536.1878, found: 5361877.

### Example 64e

### Preparation of (2R,3R,4R,5S)-4-(4-chloro-2-fluoro-phenyl)-3-(3-chloro-phenyl)-4-cyano-5-(2,2-dimethyl-propyl)-pyrrolidine-2-carboxylic acid ((S)-3,4-dihydroxy-butyl)-amide

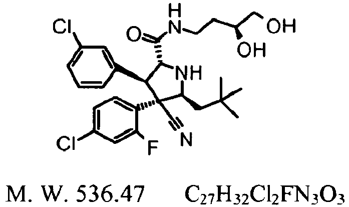

Rac-(2R,3R,4R,5S)-4-(4-chloro-2-fluoro-phenyl)-3-(3-chloro-phenyl)-4-cyano-5-(2,2-dimethylpropyl)-pyrrolidine-2-carboxylic acid ((S)-3,4-dihydroxy-butyl)-amide (0.15 g) was separated by chiral SFC chromatography to provide chiral (2R,3R,4R,5S)-4-(4-chloro-2-fluoro-phenyl)-3-(3-chloro-phenyl)-4-cyano-5-(2,2-dimethyl-propyl)-pyrrolidine-2-carboxylic acid ((S)-3,4-dihydroxy-butyl)-amide as a white solid (71 mg, 47%) and chiral-(2S,3S,4S,5R)-4-(4-chloro-2-fluoro-phenyl)-3-(3-chloro-phenyl)-4-cyano-5-(2,2-dimethyl-propyl)-pyrrolidine-2-carboxylic acid ((S)-3,4-dihydroxy-butyl)-amide as a white solid (70 mg, 47%).

### Example 65a

### Preparation of intermediate (Z)-2-(4-chloro-2-fluoro-phenyl)-3-(3-fluoro-phenyl)-acrylonitrile

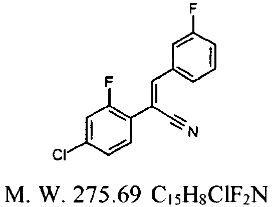

In a manner similar to the method described in **Example 1b,** 4-chloro-2-fluorophenylacetonitrile (3.27 g, 19 mmol) was reacted with 3-fluorobenzaldehyde (Aldrich) (2.87 g, 23 mmol), methanolic solution (25 wt%) of sodium methoxide (4.83 mL, 21 mmol) in methanol (90 mL) at 50 °C for 3 h to give (Z)-2-(4-chloro-2-fluoro-phenyl)-3-(3-fluoro-phenyl)-acrylonitrile as a white powder (5.2 g, 98%).

### Example 65b

### Preparation of intermediate rac-(2R,3R,4R,5S)-4-(4-chloro-2-fluoro-phenyl)-4-cyano-5-(2,2-dimethyl-propyl)-3-(3-fluoro-phenyl)-pyrrolidine-2-carboxylic acid tert-butyl ester

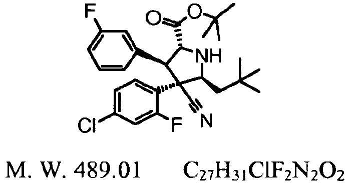

In a manner similar to the method described in **Example 1c,** [3-methyl-but-(E)-ylideneamino]-acetic acid tert-butyl ester prepared in **Example 1a** (2.1 g, 10 mmol) was reacted with (Z)-2-(4-chloro-2-fluoro-phenyl)-3-(3-fluoro-phenyl)-acrylonitrile (2.2 g, 8 mmol) prepared in **Example 65a,** AgF (1.5 g, 12 mmol), and triethylamine (2.7 mL, 20 mmol) in dichloromethane (120 mL) at room temperature for 18 h to give rac-(2R,3R,4R,5S)-4-(4-chloro-2-fluoro-phenyl)-4-cyano-5-(2,2-dimethyl-propyl)-3-(3-fluoro-phenyl)-pyrrolidine-2-carboxylic acid tert-butyl ester as a white foam (1.02 g, 26%).

### Example 65c

### Preparation of intermediate rac-(2R,3R,4R,5S)-4-(4-chloro-2-fluoro-phenyl)-4-cyano-5-(2,2-dimethyl-propyl)-3-(3-fluoro-phenyl)-pyrrolidine-2-carboxylic acid trifluoroacetic acid

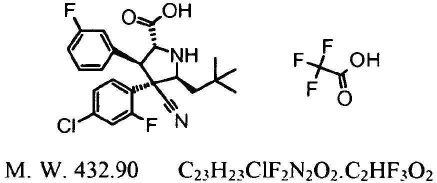

In a manner similar to the method described in **Example 1d,** rac-(2R,3R,4R,5S)-4-(4-chloro-2-fluoro-phenyl)-4-cyano-5-(2,2-dimethyl-propyl)-3-(3-fluoro-phenyl)-pyrrolidine-2-carboxylic acid tert-butyl ester prepared in **Example 65b** (1 g, 2.1 mmol) was reacted with trifluoroacetic acid in dichloromethane at room temperature to give rac-(2R,3R,4R,5S)-4-(4-chloro-2-fluoro-phenyl)-4-cyano-5-(2,2-dimethyl-propyl)-3-(3-fluoro-phenyl)-pyrrolidine-2-carboxylic acid trifluoroacetic acid as a white solid (1 g, 88%).

HRMS (ES⁺) m/z Calcd for C₂₃H₂₃ClF₂N₂O₂.+ H [(M+H)⁺]: 433.1489, found: 433.1487.

### Example 65d

### Preparation of rac-(2R,3R,4R,5S)-4-(4-chloro-2-fluoro-phenyl)-4-cyano-5-(2,2-dimethylpropyl)-3-(3-fluoro-phenyl)-pyrrolidine-2-carboxylic acid ((S)-3,4-dihydroxy-butyl)-amide

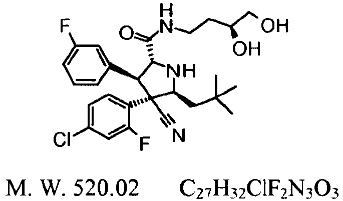

In a manner similar to the method described in **Examples 42c, 42d,** rac-(2R,3R,4R,5S)-4-(4-chloro-2-fluoro-phenyl)-4-cyano-5-(2,2-dimethyl-propyl)-3-(3-fluoro-phenyl)-pyrrolidine-2-carboxylic acid trifluoroacetic acid prepared in **Example 65c** (0.46 g, 0.83 mmol) was reacted with 2-((S)-2,2-dimethyl-[1,3]dioxolan-4-yl)-ethylamine (0.36 g, 2.5 mmol), HATU (0.57 g, 1.5 mmol) and iPr₂NEt (0.72 mL, 4.2 mmol) in CH₂Cl₂ at room temperature for 20 h, then reacted with aqueous HCl solution in tetrahydrofuran at room temperature for 2 h to give rac-(2R,3R,4R,5S)-4-(4-chloro-2-fluoro-phenyl)-4-cyano-5-(2,2-dimethyl-propyl)-3-(3-fluoro-phenyl)-pyrrolidine-2-carboxylic acid ((S)-3,4-dihydroxy-butyl)-amide as a white solid (0.21 g, 48%).

HRMS (ES⁺) m/z Calcd for C₂₇H₃₂ClF₂N₃O₃+ H [(M+H)⁺]: 520.2173, found: 520.2171.

### Example 66a

### Preparation of intermediate (Z)-3-(3-bromo-phenyl)-2-(4-chloro-2-fluoro-phenyl)-acrylonitrile

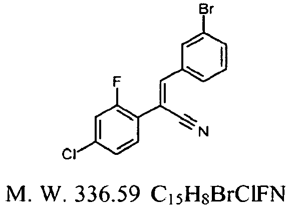

In a manner similar to the method described in **Example 1b,** 4-chloro-2-fluorophenylacetonitrile (1.9 g, 11 mmol) was reacted with 3-bromobenzaldehyde (Aldrich) (1.57 mL, 13.4 mmol), methanolic solution (25 wt%) of sodium methoxide (2.8 mL, 12 mmol) in methanol (50 mL) at 50 °C for 3 h to give (Z)-3-(3-bromo-phenyl)-2-(4-chloro-2-fluoro-phenyl)-acrylonitrite as a white powder (2.3 g, 60%).

### Example 66b

### Preparation of intermediate rac-(2R,3R,4R,5S)-3-(3-bromo-phenyl)-4-(4-chloro-2-fluoro-phenyl)-4-cyano-5-(2,2-dimethyl-propyl)-pyrrolidine-2-carboxylic acid tert-butyl ester

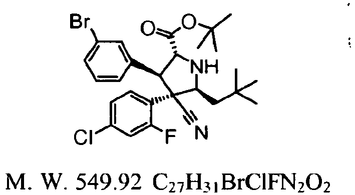

In a manner similar to the method described in **Example 1c,** [3-methyl-but-(E)-ylideneamino]-acetic acid tert-butyl ester prepared in **Example 1a** (2.1 g, 10 mmol) was reacted with (Z)-3-(3-bromo-phenyl)-2-(4-chloro-2-fluoro-phenyl)-acrylonitrile (2 g, 5.9 mmol) prepared in **Example 66a,** AgF (1.5 g, 12 mmol), and triethylamine (2.7 mL, 20 mmol) in dichloromethane (120 mL) at room temperature for 18 h to give rac-(2R,3R,4R,5S)-3-(3-bromo-phenyl)-4-(4-chloro-2-fluoro-phenyl)-4-cyano-5-(2,2-dimethyl-propyl)-pyrrolidine-2-carboxylic acid tert-butyl ester as a white foam (1.3 g, 40%).

### Example 66c

### Preparation of intermediate rac-(2R,3R,4R,5S)-3-(3-bromo-phenyl)-4-(4-chloro-2-fluoro-phenyl)-4-cyano-5-(2,2-dimethyl-propyl)-pyrrolidine-2-carboxylic acid trifluoroacetic acid

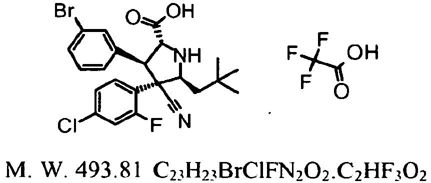

In a manner similar to the method described in **Example 25a,** rac-(2R,3R,4R,5S)-3-(3-bromophenyl)-4-(4-chloro-2-fluoro-phenyl)-4-cyano-5-(2,2-dimethyl-propyl)-pyrrolidine-2-carboxylic acid tert-butyl ester prepared in **Example 66b** (1.2 g, 2.2 mmol) was reacted with trifluoroacetic acid in dichloromethane at room temperature to give rac-(2R,3R,4R,5S)-3-(3-bromo-phenyl)-4-(4-chloro-2-fluoro-phenyl)-4-cyano-5-(2,2-dimethyl-propyl)-pyrrolidine-2-carboxylic acid trifluoroacetic acid as a white solid (1.1 g, 83%).

HRMS (ES⁺) m/z Calcd for C₂₃H₂₃BrClFN₂O₂+ H [(M+H)⁺]: 493.0688, found: 493.0688.

### Example 66d

### Preparation of rac-(2R,3R,4R,5S)-3-(3-bromo-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide

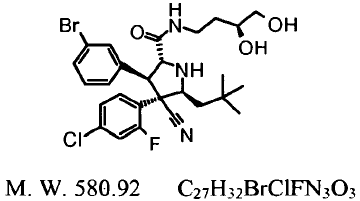

In a manner similar to the method described in **Examples 42c, 42d,** rac-(2R,3R,4R,5S)-3-(3-bromo-phenyl)-4-(4-chloro-2-fluoro-phenyl)-4-cyano-5-(2,2-dimethyl-propyl)-pyrrolidine-2-carboxylic acid trifluoroacetic acid prepared in **Example 66c** (0.5 g, 0.83 mmol) was reacted with 2-((S)-2,2-dimethyl-[1,3]dioxolan-4-yl)-ethylamine (0.36 g, 2.5 mmol), HATU (0.57 g, 1.49 mmol) and iPr₂NEt (0.72 mL, 4.2 mmol) in CH₂Cl₂ at room temperature for 20 h, then reacted with aqueous HCl solution in tetrahydrofuran at room temperature for 2 h to give rac-(2R,3R,4R,5S)-3-(3-bromo-phenyl)-4-(4-chloro-2-fluoro-phenyl)-4-cyano-5-(2,2-dimethylpropyl)-pyrrolidine-2-carboxylic acid ((S)-3,4-dihydroxy-butyl)-amide as a white solid (0.26 g, 55%).

HRMS (ES⁺) m/z Calcd for C₂₇H₃₂BrClFN₃O₃+ H [(M+H)⁺]: 580.1373, found: 580.1372.

### Example 67a

### Preparation of intermediate rac-(2S,3R,4R,5S)-3-(3-bromo-phenyl)-4-(4-chloro-2-fluoro-phenyl)-4-cyano-5-(2,2-dimethyl-propyl)-pyrrolidine-2-carboxylic acid tert-butyl ester

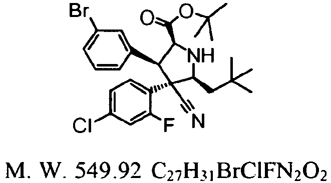

In preparation of rac-(2R,3R,4R,5S)-3-(3-bromo-phenyl)-4-(4-chloro-2-fluoro-phenyl)-4-cyano-5-(2,2-dimethyl-propyl)-pyrrolidine-2-carboxylic acid tert-butyl ester as described in **Example 66b**, rac-(2S,3R,4R,5S)-3-(3-bromo-phenyl)-4-(4-chloro-2-fluoro-phenyl)-4-cyano-5-(2,2-dimethyl-propyl)-pyrrolidine-2-carboxylic acid tert-butyl ester was obtained as the second product: a white foam (1.2 g, 37%).

### Example 67b

### Preparation of intermediate rac-(2S,3R,4R,5S)-3-(3-bromo-phenyl)-4-(4-chloro-2-fluoro-phenyl)-4-cyano-5-(2,2-dimethyl-propyl)-pyrrolidine-2-carboxylic acid trifluoroacetic acid

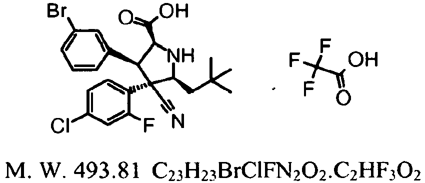

In a manner similar to the method described in **Example 25a,** rac-(2S,3R,4R,5S)-3-(3-bromophenyl)-4-(4-chloro-2-fluoro-phenyl)-4-cyano-5-(2,2-dimethyl-propyl)-pyrrolidine-2-carboxylic acid tert-butyl ester prepared in **Example 67a** (1.3 g, 2.4 mmol) was reacted with trifluoroacetic acid in dichloromethane at room temperature to give rac-(2S,3R,4R,5S)-3-(3-bromo-phenyl)-4-(4-chloro-2-fluoro-phenyl)-4-cyano-5-(2,2-dimethyl-propyl)-pyrrolidine-2-carboxylic acid trifluoroacetic acid as a white solid (1.2 g, 83%).

HRMS (ES⁺) m/z Calcd for C₂₃H₂₃BrClFN₂O₂+ H [(M+H)⁺]: 493.0688, found: 493.0689.

### Example 67c

### Preparation of rac-(2S,3R,4R,5S)-3-(3-bromo-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide

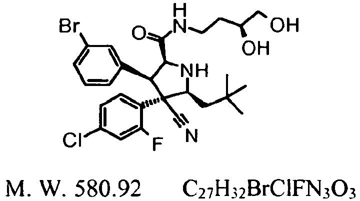

In a manner similar to the method described in **Examples 42c, 42d,** rac-(2S,3R,4R,5S)-3-(3-bromo-phenyl)-4-(4-chloro-2-fluoro-phenyl)-4-cyano-5-(2,2-dimethyl-propyl)-pyrrolidine-2-carboxylic acid trifluoroacetic acid prepared in **Example 67b** (0.5 g, 0.83 mmol) was reacted with 2-((S)-2,2-dimethyl-[1,3]dioxolan-4-yl)-ethylamine (0.36 g, 2.5 mmol), HATU (0.57 g, 1.49 mmol) and iPr₂NEt (0.72 mL, 4.2 mmol) in CH₂Cl₂ at room temperature for 20 h, then reacted with aqueous HCl solution in tetrahydrofuran at room temperature for 2 h to give rac-(2S,3R,4R,5S)-3-(3-bromo-phenyl)-4-(4-chloro-2-fluoro-phenyl)-4-cyano-5-(2,2-dimethylpropyl)-pyrrolidine-2-carboxylic acid ((S)-3,4-dihydroxy-butyl)-amide as a white solid (0.21 g, 44%).

HRMS (ES⁺) m/z Calcd for C₂₇H₃₂BrClFN₃O₃+ H [(M+H)⁺]: 580.1373, found: 580.1372,

### Example 68a

### Preparation of intermediate (Z)-2-(4-Chloro-2-fluoro-phenyl)-3-(3,4-dichloro-phenyl)-acrylonitrile

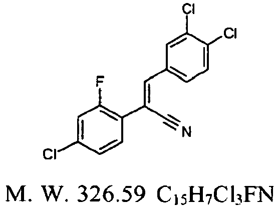

In a manner similar to the method described in **Example 1b,** 4-chloro-2-fluorophenyl acetonitrile (4.5 g, 26 mmol) was reacted with 3,4-dichlorobenzaldehyde (Aldrich) (5.5 g, 32 mmol), methanolic solution (25 wt%) of sodium methoxide (6.6 mL, 29 mmol) in methanol (150 mL) at 50 °C for 3 h to give (Z)-2-(4-chloro-2-fluoro-phenyl)-3-(3,4-dichloro-phenyl)-acrylonitrile as a white powder (6.5 g, 76%).

### Example 68b

### Preparation of intermediate rac-(2R,3R,4R,5S)-4-(4-chloro-2-fluoro-phenyl)-4-cyano-3-(3,4-dichloro-phenyl)-5-(2,2-dimethyl-propyl)-pyrrolidine-2-carboxylic acid tert-butyl ester

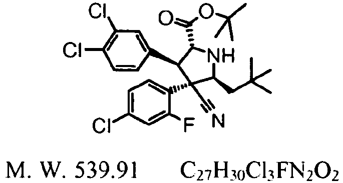

In a manner similar to the method described in **Example 1c**, [3-methyl-but-(E)-ylideneamino]-acetic acid tert-butyl ester prepared in **Example 1a** (2.1 g, 10 mmol) was reacted with (Z)-2-(4-chloro-2-fluoro-phenyl)-3-(3,4-dichloro-phenyl)-acrylonitrile (2.6 g, 8 mmol) prepared in **Example 68a**, AgF (1.5 g, 12 mmol), and triethylamine (2.7 mL, 20 mmol) in dichloromethane (120 mL) at room temperature for 18 h to give rac-(2R,3R,4R,5S)-4-(4-chloro-2-fluoro-phenyl)-4-cyano-3-(3,4-dichloro-phenyl)-5-(2,2-dimethyl-propyl)-pyrrolidine-2-carboxylic acid tert-butyl ester as a white foam (1.7 g, 39%).

### Example 68c

### Preparation of intermediate rac-(2R,3R,4R,5S)-4-(4-chloro-2-fluoro-phenyl)-4-cyano-3-(3,4-dichloro-phenyl)-5-(2,2-dimethyl-propyl)-pyrrolidine-2-carboxylic acid trifluoroacetic acid

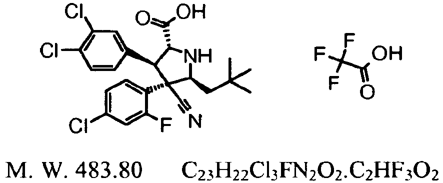

In a manner similar to the method described in **Example 25a,** rac-(2R,3R,4R,5S)-4-(4-chloro-2-fluoro-phenyl)-4-cyano-3-(3,4-dichloro-phenyl)-5-(2,2-dimethyl-propyl)-pyrrolidine-2-carboxylic acid tert-butyl ester prepared in **Example 68b** (1.7 g, 3.1 mmol) was reacted with trifluoroacetic acid in dichloromethane at room temperature to give rac-(2R,3R,4R,5S)-4-(4-chloro-2-fluoro-phenyl)-4-cyano-3-(3,4-dichloro-phenyl)-5-(2,2-dimethyl-propyl)-pyrrolidine-2-carboxylic acid trifluoroacetic acid as a white solid (1.8 g, 96%).

### Example 68d

### Preparation of rac-(2R,3R,4R,5S)-4-(4-chloro-2-fluoro-phenyl)-4-cyano-3-(3,4-dichlorophenyl)-5-(2,2-dimethyl-propyl)-pyrrolidine-2-carboxylic acid ((S)-3,4-dihydroxy-butyl)-amide

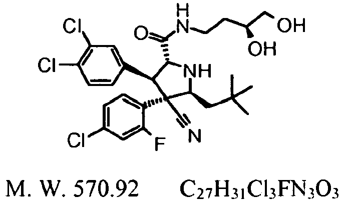

In a manner similar to the method described in **Examples 42c, 42d,** rac-(2R,3R,4R,5S)-4-(4-chloro-2-fluoro-phenyl)-4-cyano-3-(3,4-dichloro-phenyl)-5-(2,2-dimethyl-propyl)-pyrrolidine-2-carboxylic acid trifluoroacetic acid prepared in **Example 68c** (0.5 g, 0.84 mmol) was reacted with 2-((S)-2,2-dimethyl-[1,3]dioxolan-4-yl)-ethylamine (0.36 g, 2.5 mmol), HATU (0.57 g, 1.5 mmol) and iPr₂NEt (0.72 mL, 4.2 mmol) in CH₂Cl₂ at room temperature for 20 h, then reacted with aqueous HCl solution in tetrahydrofuran at room temperature for 2 h to give rac-(2R,3R,4R,5S)-4-(4-chloro-2-fluoro-phenyl)-4-cyano-3-(3,4-dichloro-phenyl)-5-(2,2-dimethylpropyl)-pyrrolidine-2-carboxylic acid ((S)-3,4-dihydroxy-butyl)-amide as a white solid (0.28 g, 59%).

HRMS (ES⁺) m/z Calcd for C₂₇H₃₁Cl₃FN₃O₃+ H [(M+H)⁺]: 570.1488, found: 570.1489.

### Example 69a

### Preparation of intermediate (Z)-3-(3-chloro-4-fluoro-phenyl)-2-(4-chloro-2-fluoro-phenyl)-acrylonitrile

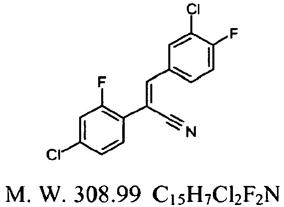

In a manner similar to the method described in **Example 1b**, 4-chloro-2-fluorophenylacetonitrile (3.3 g, 19 mmol) was reacted with 4-chloro-3-fluoro-benzaldehyde (Aldrich) (3.65 g, 23 mmol), methanolic solution (25 wt%) of sodium methoxide (4.8 mL, 21 mmol) in methanol (90 mL) at 50 °C for 3 h to give (Z)-3-(3-chloro-4-fluoro-phenyl)-2-(4-chloro-2-fluoro-phenyl)-acrylonitrile as a white powder (3 g, 50%).

### Example 69b

### Preparation of intermediate rac-(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carboxylic acid tert-butyl ester

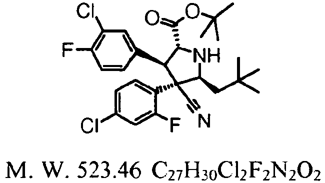

In a manner similar to the method described in **Example 1c**, [3-methyl-but-(E)-ylideneamino]-acetic acid tert-butyl ester prepared in **Example 1a** (2.1 g, 10 mmol) was reacted with (Z)-3-(3-chloro-4-fluoro-phenyl)-2-(4-chloro-2-fluoro-phenyl)-acrylonitrile (2.5 g, 8 mmol) prepared in **Example 69a**, AgF (1.5 g, 12 mmol), and triethylamine (2.7 mL, 20 mmol) in dichloromethane (120 mL) at room temperature for 18 h to give rac-(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carboxylic acid tert-butyl ester as a white foam (1 g, 24%).

### Example 69c

### Preparation of intermediate rac-(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carboxylic acid trifluoroacetic acid

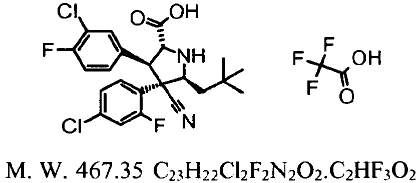

In a manner similar to the method described in **Example 25a**, rac-(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carboxylic acid tert-butyl ester prepared in **Example 69b** (1 g, 1.9 mmol) was reacted with trifluoroacetic acid in dichloromethane at room temperature to give rac-(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carboxylic acid trifluoroacetic acid as a white solid (1 g, 90%).

### Example 69d

### Preparation of rac-(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide

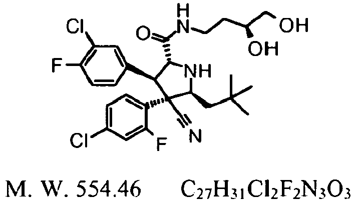

In a manner similar to the method described in **Examples 42c**, **42d**, rac-(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carboxylic acid trifluoroacetic acid prepared in **Example 69c** (0.28 g, 0.48 mmol) was reacted with 2-((S)-2,2-dimethyl-[1,3]dioxolan-4-yl)-ethylamine (0.21 g, 1.44 mmol), HATU (0.33 g, 0.87 mmol) and iPr₂NEt (0.42 mL, 2.4 mmol) in CH₂Cl₂ at room temperature for 20 h, then reacted with aqueous HCl solution in tetrahydrofuran at room temperature for 2 h to give rac-(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide as a white solid (0.18 g, 77%).

HRMS (ES⁺) m/z Calcd for C₂₇H₃₁Cl₃FN₃O₃+ H [(M+H)⁺]: 554.1784, found: 554.1785.

### Example 69e

### Preparation of (2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide

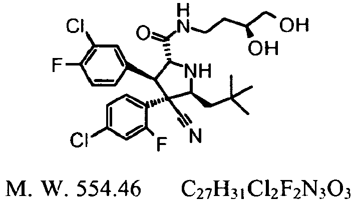

Rac-(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide (0.14 g) was separated by chiral SFC chromatography to provide chiral (2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide as a white solid (61 mg, 44%) and chiral-(2S,3S,4S,5R)-3-(3-chloro-4-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide as a white solid (61 mg, 44%).

### Example 70a

### Preparation of intermediate (Z)-3-(4-bromo-thiophen-2-yl)-2-(4-chloro-2-fluoro-phenyl)-acrylonitrile

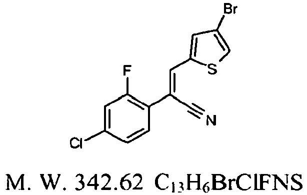

In a manner similar to the method described in **Example 1b**, 4-chloro-2-fluorophenylacetonitrile (4.05 g, 24 mmol) was reacted with 4-bromo-2-thiophenecarboxaldehyde (Aldrich) (6.08 g, 29 mmol), methanolic solution (25 wt%) of sodium methoxide (6 mL, 26 mmol) in methanol (90 mL) at 50 °C for 3 h to give (Z)-3-(4-bromo-thiophen-2-yi)-2-(4-chloro-2-fluoro-phenyl)-acrylonitrile as a light yellow solid (5.2 g, 64%).

### Example 70b

### Preparation of intermediate rac-(2R,3R,4R,5S)-3-(4-bromo-thiophen-2-yl)-4-(4-chloro-2-fluoro-phenyl)-4-cyano-5-(2,2-dimethyl-propyl)-pyrrolidine-2-carboxylic acid tert-butyl ester

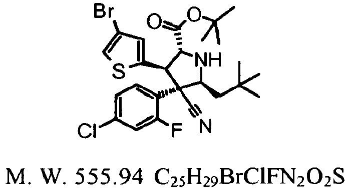

In a manner similar to the method described in **Example 1c**, [3-methyl-but-(E)-ylideneamino]-acetic acid tert-butyl ester prepared in **Example 1a** (2.1 g, 10 mmol) was reacted with (Z)-3-(4-bromo-thiophen-2-yl)-2-(4-chloro-2-fluoro-phenyl)-acrylonitrile (2.7 g, 8 mmol) prepared in **Example 70a**, AgF (1.5 g, 12 mmol), and triethylamine (2.7 mL, 20 mmol) in dichloromethane (120 mL) at room temperature for 18 h to give rac-(2R,3R,4R,5S)-3-(4-bromo-thiophen-2-yl)-4-(4-chloro-2-fluoro-phenyl)-4-cyano-5-(2,2-dimethyl-propyl)-pyrrolidine-2-carboxylic acid tert-butyl ester as a white foam (0.9 g, 20%).

### Example 70c

### Preparation of intermediate rac-(2R,3R,4R,5S)-3-(4-bromo-thiophen-2-yl)-4-(4-chloro-2-fluoro-phenyl)-4-cyano-5-(2,2-dimethyl-propyl)-pyrrolidine-2-carboxylic acid trifluoroacetic acid

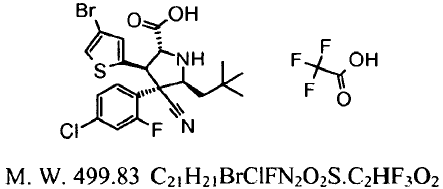

In a manner similar to the method described in **Example 25a**, rac-(2R,3R,4R,5S)-3-(4-bromothiophen-2-yl)-4-(4-chloro-2-fluoro-phenyl)-4-cyano-5-(2,2-dimethyl-propyl)-pyrrolidine-2-carboxylic acid tert-butyl ester prepared in **Example 70b** (0.9 g, 1.6 mmol) was reacted with trifluoroacetic acid in dichloromethane at room temperature to give rac-(2R,3R,4R,5S)-3-(4-bromo-thiophen-2-yl)-4-(4-chloro-2-fluoro-phenyl)-4-cyano-5-(2,2-dimethyl-propyl)-pyrrolidine-2-carboxylic acid trifluoroacetic acid as a light blue solid (0.9 g, 92%).

### Example 70d

### Preparation of rac-(2R,3R,4R,5S)-3-(4-bromo-thiophen-2-yl)-4-(4-chloro-2-fluoro-phenyl)-4-cyano-5-(2,2-dimethyl-propyl)-pyrrolidine-2-carboxylic acid ((S)-3,4-dihydroxy-butyl)-amide

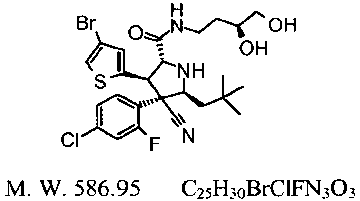

In a manner similar to the method described in **Examples 42c, 42d**, rac-(2R,3R,4R,5S)-3-(4-bromo-thiophen-2-yl)-4-(4-chloro-2-fluoro-phenyl)-4-cyano-5-(2,2-dimethyl-propyl)-pyrrolidine-2-carboxylic acid trifluoroacetic acid prepared in **Example 70c** (0.2 g, 0.33 mmol) was reacted with 2-((S)-2,2-dimethyl-[1,3]dioxolan-4-yl)-ethylamine (0.14 g, 1 mmol), HATU (0.22 g, 0.58 mmol) and iPr₂NEt (0.28 mL, 1.63 mmol) in CH₂Cl₂ at room temperature for 20 h, then reacted with aqueous HCl solution in tetrahydrofuran at room temperature for 2 h to give rac-(2R,3R,4R,5S)-3-(4-bromo-thiophen-2-yl)-4-(4-chloro-2-fluoro-phenyl)-4-cyano-5-(2,2-dimethyl-propyl)-pyrrolidine-2-carboxylic acid ((S)-3,4-dihydroxy-butyl)-amide as a white solid (0.15 g, 80%).

HRMS (ES⁺) m/z Calcd for C₂₅H₃₀BrClFN₃O₃+ H [(M+H)⁺]: 586.0937, found: 586.0935.

### Example 71a

### Preparation of intermediate (Z)-2-(4-Chloro-2-fluoro-phenyl)-3-(3-chloro-4-trifluoromethylphenyl)-acrylonitrile

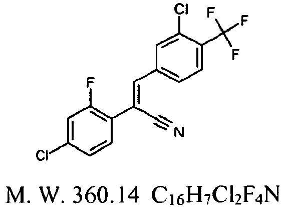

### Step A

A mixture of 3-chloro-4-(trifluoromethyl)benzyl alcohol (Synquest) (4.77 g, 23 mmol) and activated MnO₂ (19.5 g, 230 mmol) in 1,2-dichlorethane (80 mL) was heated and stirred at 80 °C for 3 h. The mixture was cooled to room temperature and filtered through a short pad of celite. The celite was washed with dichloromethane, and ethyl acetate. The filtrates were combined, concentrated, dried *under reduced pressure* to give 3-chloro-4-(trifluoromethyl)benzaldehyde as a light yellow oil (2.8 g, 60%).

### Step B

In a manner similar to the method described in **Example 1b**, 4-chloro-2-methylbenzyl cyanide (1.9 g, 11 mmol) was reacted with 3-chloro-4-(trifluoromethyl)benzaldehyde (2.8 g, 14 mmol), methanolic solution (25 wt%) of sodium methoxide (2.8 mL, 12 mmol) in methanol (50 mL) at 50 °C for 5 h to give (Z)-2-(4-chloro-2-fluoro-phenyl)-3-(3-chloro-4-trifluoromethyl-phenyl)-acrylonitrile as a yellow solid (2.45 g, 61%).

### Example 71b

### Preparation of intermediate rac-(2R,3R,4R,5S)-4-(4-chloro-2-fluoro-phenyl)-3-(3-chloro-4-trifluoromethyl-phenyl)-4-cyano-5-(2,2-dimethyl-propyl)-pyrrolidine-2-carboxylic acid tert-butyl ester

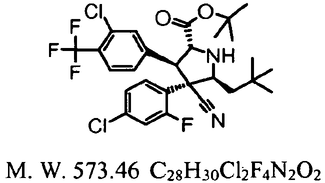

In a manner similar to the method described in **Example 1c**, [3-methyl-but-(E)-ylideneamino]-acetic acid tert-butyl ester prepared in **Example 1a** (2.1 g, 10 mmol) was reacted with (Z)-2-(4-chloro-2-fluoro-phenyl)-3-(3-chloro-4-trifluoromethyl-phenyl)-acrylonitrile (2.5 g, 6.9 mmol) prepared in **Example 71a**, AgF (1g, 8 mmol), and triethylamine (2.4 mL, 17 mmol) in dichloromethane (120 mL) at room temperature for 18 h to give rac-(2R,3R,4R,5S)-4-(4-chloro-2-fluoro-phenyl)-3-(3-chloro-4-trifluoromethyl-phenyl)-4-cyano-5-(2,2-dimethyl-propyl)-pyrrolidine-2-carboxylic acid tert-butyl ester as a white foam (1.2 g, 30%).

### Example 70c

### Preparation of intermediate rac-(2R,3R,4R,5S)-4-(4-chloro-2-fluoro-phenyl)-3-(3-chloro-4-trifluoromethyl-phenyl)-4-cyano-5-(2,2-dimethyl-propyl)-pyrrolidine-2-carboxylic acid trifluoroacetic acid

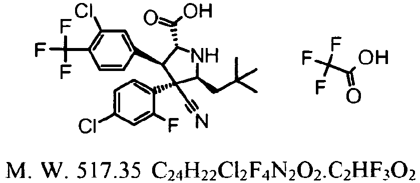

In a manner similar to the method described in **Example 25a**, rac-(2R,3R,4R,5S)-4-(4-chloro-2-fluoro-phenyl)-3-(3-chloro-4-trifluoromethyl-phenyl)-4-cyano-5-(2,2-dimethyl-propyl)-pyrrolidine-2-carboxylic acid tert-butyl ester prepared in **Example 70b** (1.2 g, 2.1 mmol) was reacted with trifluoroacetic acid in dichloromethane at room temperature to give rac-(2R,3R,4R,5S)-4-(4-chloro-2-fluoro-phenyl)-3-(3-chloro-4-trifluoromethyl-phenyl)-4-cyano-5-(2,2-dimethyl-propyl)-pyrrolidine-2-carboxylic acid trifluoroacetic acid as solid (1.1 g, 83%).

### Example 71d

### Preparation of (2R,3R,4R,5S)-4-(4-chloro-2-fluoro-phenyl)-3-(3-chloro-4-trifluoromethylphenyl)-4-cyano-5-(2,2-dimethyl-propyl)-pyrrolidine-2-carboxylic acid ((S)-3,4-dihydroxybutyl)-amide

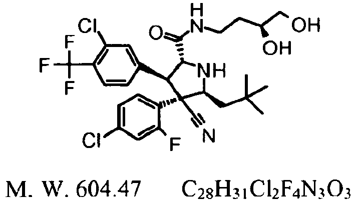

In a manner similar to the method described in **Examples 42c, 42d**, rac-(2R,3R,4R,5S)-4-(4-chloro-2-fluoro-phenyl)-3-(3-chloro-4-trifluoromethyl-phenyl)-4-cyano-5-(2,2-dimethyl-propyl)-pyrrolidine-2-carboxylic acid trifluoroacetic acid prepared in **Example 71c** (0.22 g, 0.35 mmol) was reacted with 2-((S)-2,2-dimethyl-[1,3]dioxolan-4-yl)-ethylamine (0.15 g, 1.05 mmol), HATU (0.24 g, 0.63 mmol) and iPr₂NEt (0.3 mL, 1.74 mmol) in CH₂Cl₂ at room temperature for 20 h, then reacted with aqueous HCl solution in tetrahydrofuran at room temperature for 2 h to give rac-(2R,3R,4R,5S)-3-(3-chloro-4-fluoro-phenyl)-4-(4-chloro-2-trifluoromethyl-phenyl)-4-cyano-5-(2,2-dimethyl-propyl)-pyrrolidine-2-carboxylic acid ((S)-3,4-dihydroxy-butyl)-amide as a white solid (0.14 g, 73%).

HRMS (ES⁺) m/z Calcd for C₂₈H₃₁Cl₂F₄N₃O₃⁺ H [(M+H)⁺]: 604.1752, found: 604.1748.

### Example 72a

### Preparation of rac-(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carboxylic acid ((R)-3,4-dihydroxy-butyl)-amide

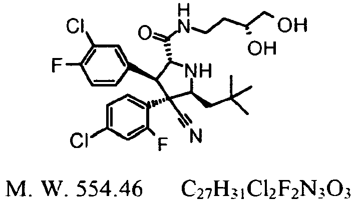

### Step A

In a manner similar to the method described in **Example 3a Step A to C**, (4R)-(-)-4-(2-hydroxyethyl)-2,2-dimethyl-1.3-dioxolane (Aldrich) (4.91 g, 33.6 mmol) was reacted with methanesulfonyl chloride (3.12 mL, 40.3 mmol) and triethylamine (9.34 mL, 67 mmol) in dichloromethane, then reacted with NaN₃ (10.7 g, 0.16 mol) in N,N-dimethylformamide, then treated with PtO₂ and H₂ (50 psi) in ethyl acetate to give 2-((R)-2,2-dimethyl-[1,3]dioxolan-4-yl)-ethylamine as a brown oil (4.4 g, 90% for three steps).

### Step B

In a manner similar to the method described in **Examples 42c, 42d**, rac-(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carboxylic acid trifluoroacetic acid prepared in **Example 69c** (0.2 g, 0.48 mmol) was reacted with 2-((R)-2,2-dimethyl-[1,3]dioxolan-4-yl)-ethylamine (0.15 g, 1.0 mmol), HATU (0.23 g, 0.62 mmol) and iPr₂NEt (0.3 mL, 1.72 mmol) in CH₂Cl₂ at room temperature for 20 h, then reacted with aqueous HCl solution in tetrahydrofuran at room temperature for 2 h to give rac-(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carboxylic acid ((R)-3,4-dihydroxy-butyl)-amide as a white solid (0.11 g, 74%).

HRMS (ES⁺) m/z Calcd for C₂₇H₃₁Cl₃FN₃O₃+ H [(M+H)⁺]: 554.1784, found: 554.1786.

### Example 72b

### Preparation of (2R,3R4R,55)-3-(3-chloro-4-fluoro-phenyl)-4-(4-chloro-2-fluoro-phenyl)-4-cyano-5-(2,2-dimethyl-propyl)-pyrrolidine-2-carboxylic acid ((R)-3,4-dihydroxy-butyl)-amide

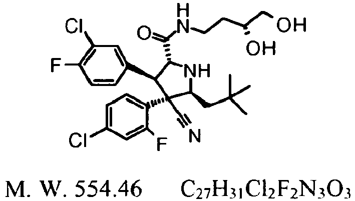

Rac-(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carboxylic acid ((R)-3,4-dihydroxy-butyl)-amide (80 mg) was separated by chiral SFC chromatography to provide chiral (2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carboxylic acid ((R)-3,4-dihydroxy-butyl)-amide as a white solid (35 mg, 44%) and chiral (2S,3S,4S,5R)-3-(3-chloro-4-fluoro-phenyl)-4-(4-chloro-2-fluoro-phenyl)-4-cyano-5-(2,2-dimethyl-propyl)-pyrrolidine-2-carboxylic acid ((R)-3,4-dihydroxy-butyl)-amide as a white solid (35 mg, 44%).

### Example 73a

### Preparation of rac-(2R,35,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((R)-3,4-dihydroxy-butyl)-amide

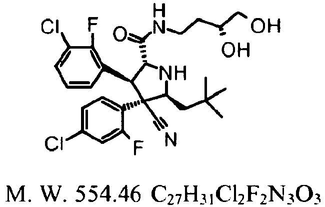

In a manner similar to the method described in **Example 72a**, rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid trifluoroacetic acid prepared i**n Example 53b** (0.44 g, 0.769 mmol) was reacted with 2-((R)-2,2-dimethyl-[1,3]dioxolan-4-yl)-ethylamine (0.33 g, 2.3 mmol), HATU (0.52 g, 1.36 mmol) and iPr₂NEt (0.66 mL, 3.8 mmol) in CH₂Cl₂ at room temperature for 20 h, then reacted with aqueous HCl solution in tetrahydrofuran at room temperature for 2 h to give rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((R)-3,4-dihydroxy-butyl)-amide as a white solid (0.29 g, 68%).

HRMS (ES⁺) m/z Calcd for C₂₇H₃₁Cl₂F₂N₃O₃+ H [(M+H)⁺]: 554.1784, found: 554.1783.

### Example 73b

### Preparation of (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((R)-3,4-dihydroxy-butyl)-amide

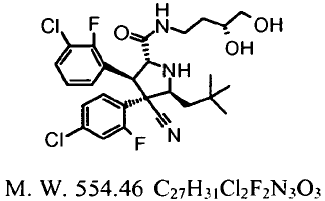

Rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((R)-3,4-dihydroxy-butyl)-amide (0.28 g) was separated by chiral SFC chromatography to provide chiral-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((R)-3,4-dihydroxy-butyl)-amide as a white solid (109 mg, 39%) and chiral-(2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid ((R)-3,4-dihydroxy-butyl)-amide as a white solid (109 mg, 39%).

### Example 74

### Preparation of rac-(2R,3R,4R,5S)-4-(4-chloro-2-fluoro-phenyl)-3-(3-chloro-phenyl)-4-cyano-5-(2,2-dimethyl-propyl)-pyrrolidine-2-carboxylic acid ((R)-3,4-dihydroxy-butyl)-amide

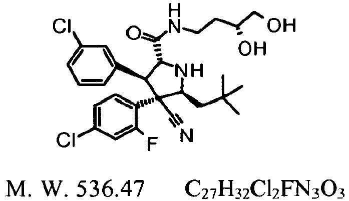

In a manner similar to the method described in **Example 72a**, rac-(2R,3R,4R,5S)-4-(4-chloro-2-fluoro-phenyl)-3-(3-chloro-phenyl)-4-cyano-5-(2,2-dimethyl-propyl)-pyrrolidine-2-carboxylic acid trifluoroacetic acid (0.2 g, 0.36 mmol) was reacted with 2-((R)-2,2-dimethyl-[1,3]dioxolan-4-yl)-ethylamine (0.15 g, 1.07 mmol), HATU (0.24 g, 0.64 mmol) and iPr₂NEt (0.31 mL, 1.78 mmol) in CH₂Cl₂ at room temperature for 20 h, then reacted with aqueous HCl solution in tetrahydrofuran at room temperature for 2 h to give rac-(2R,3R,4R,5S)-4-(4-chloro-2-fluoro-phenyl)-3-(3-chloro-phenyl)-4-cyano-5-(2,2-dimethyl-propyl)-pyrrolidine-2-carboxylic acid ((R)-3,4-dihydroxy-butyl)-amide as a white solid (0.1 g, 54%).

HRMS (ES⁺) m/z Calcd for C₂₇H₃₂Cl₂FN₃O₃+ H [(M+H)⁺]: 536.1878, found: 5361880.

### Example 75

### Preparation of rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid ((R)-3,4-dihydroxy-butyl)-amide

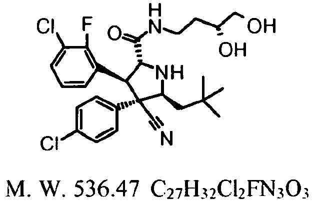

In a manner similar to the method described in **Example 72a**, rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid trifluoroacetic acid (0.2 g, 0.36mmol) was reacted with 2-((R)-2,2-dimethyl-[1,3]dioxolan-4-yl)-ethylamine (0.15 g, 1.07 mmol), HATU (0.24 g, 0.64 mmol) and iPr₂NEt (0.31 g, 1.78 mmol) in CH₂Cl₂ at room temperature for 20 h, then reacted with aqueous HCl solution in tetrahydrofuran at room temperature for 2 h to give rac-(2R,35,4R,5S)-3-(3-chloro-2-fluoro-phenyl)-4-(4-chloro-phenyl)-4-cyano-5-(2,2-dimethyl-propyl)-pyrrolidine-2-carboxylic acid ((R)-3,4-dihydroxy-butyl)-amide as a white solid (0.1 g, 54%).

HRMS (ES⁺) m/z Calcd for C₂₇H₃₂Cl₂FN₃O₃+ H [(M+H)⁺]: 536.1878, found: 536.1880.

### Example 76

### Preparation of rac-(2S,3R,4R,5S)-4-(4-chloro-2-methoxy-phenyl)-3-(3-chloro-phenyl)-4-cyano-5-(2,2-dimethyl-propyl)-pyrrolidine-2-carboxylic acid ((R)-3,4-dihydroxy-butyl)-amide

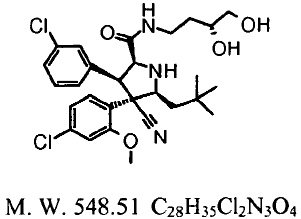

In a manner similar to the method described in **Example 72a**, rac-(2S,3R,4R,5S)-4-(4-chloro-2-methoxy-phenyl)-3-(3-chloro-phenyl)-4-cyano-5-(2,2-dimethyl-propyl)-pyrrolidine-2-carboxylic acid trifluoroacetic acid (0.2 g, 0.35 mmol) was reacted with 2-((R)-2,2-dimethyl-[1,3]dioxolan-4-yl)-ethylamine (0.15 g, 1.0 mmol), HATU (0.24 g, 0.63 mmol) and iPr₂NEt (0.3 mL, 1.7 mmol) in CH₂Cl₂ at room temperature for 20 h, then reacted with aqueous HCl solution in tetrahydrofuran at room temperature for 2 h to give rac-(2S,3R,4R,5S)-4-(4-chloro-2-methoxyphenyl)-3-(3-chloro-phenyl)-4-cyano-5-(2,2-dimethyl-propyl)-pyrrolidine-2-carboxylic acid ((R)-3,4-dihydroxy-butyl)-amide as a white solid (0.1 g, 57%).

HRMS (ES⁺) m/z Calcd for C₂₈H₃₅Cl₂N₃O₄+ H [(M+H)⁺]: 548.2078, found: 548.2074.

### Example 77

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid ((R)-3,4-dihydroxy-butyl)-amide

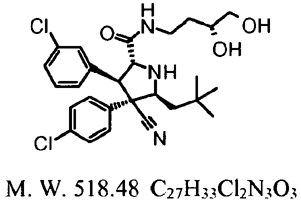

In a manner similar to the method described in **Example 72a**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid trifluoroacetic acid (0.2 g, 0.37 mmol) was reacted with 2-((R)-2,2-dimethyl-[1,3]dioxolan-4-yl)-ethylamine (0.159 g, 1.1 mmol), HATU (0.25 g, 0.66 mmol) and iPr₂NEt (0.32 mL, 1.8 mmol) in CH₂Cl₂ at room temperature for 20 h, then reacted with aqueous HCl solution in tetrahydrofuran at room temperature for 2 h to give rac-(2R,3R,4R,5S)-3-(3-chloro-phenyl)-4-(4-chloro-phenyl)-4-cyano-5-(2,2-dimethyl-propyl)-pyrrolidine-2-carboxylic acid ((R)-3,4-dihydroxy-butyl)-amide as a white solid (0.15 g, 81%).

HRMS (ES⁺) m/z Calcd for C₂₇H₃₃Cl₂N₃O₃+ H [(M+H)⁺]: 518.1972, found: 518.1972.

### Example 78a

### Preparation of intermediate [3,3,4-trimethyl-pent-(E)-ylideneamino]- acetic acid tert-butyl ester

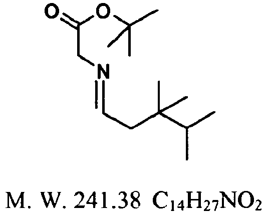

### Step A

To a solution of ethyl 3,3-dimethylacrylate (Aldrich) (6.98 g, 54 mmol) in anhydrous tetrahydrofuran (60 mL) was added chlorotrimethylsilane (12 mL, 70 mmol), CuI (1.5 g, 8 mmol) under nitrogen. The mixture was stirred and the temperature was cooled to -20 °C. To the stirring mixture was slowly added a tetrahydrofuran solution (2 N) if isopropylmagnesium chloride (40 mL, 80 mmol) during a period of 30 min while maitining the temperature below -10 °C. After the addition was finished, the reaction mixture was gradually warmed to 0 °C and stirred at 0 °C for 3 h. Aqueous saturated NH₄Cl solution was added to quench the reaction, and the mixture was extracted with ethyl acetate and ethyl ether. The organic layers were combined, concentrated. The residue was purifed by chromatography (EtOAc:hexanes=1;20, 1;10) to give 3,3,4-trimethyl-pentanoic acid ethyl ester as a colorless oil (7 g, 75%).

### Step B

To a solution of 3,3,4-trimethyl-pentanoic acid ethyl ester (7 g, 41 mmol) in anhydrous ethyl ether (100 mL) at 0 °C was added a ethyl ether solution (1M) of LiAlH₄ (67 mL, 67 mmol) under nitrogen. The reaction mixture was stirred at 0 °C for 1 h, then poured into a ice-water. The mixture was extracted with ethyl acetate. The organic layer were separated, washed with water, aqueous HCl solution, brine, dried over MgSO₄, and concentrated to give 3,3,4-trimethyl-pentan-1-ol as a colorless oil (5.4 g, 100%).

### Step C

To a solution of 3,3,4-trimethyl-pentan-1-ol (5.4 g, 41 mmol) in dichloromethane (100 mL) was added Dess-Martin periodinane (22 g,, 52 mmol) The reaction mixture was stirred at room temperature for 3 h. Aqueous Na₂SO₃ solution was added to quench the reaction. The organic layers were separated, washed with saturated NaHCO₃, brine, dried over MgSO₄, and concentrated. The residue was purified by chromatography (EtOAc:hexanes=1:30) to give 3,3,4-trimethyl-pentanal as a colorless oil (Yield: 1.1 g, 21%).

### Step D

In a manner similar to the method described in **Example 1a**, glycine tert-butyl ester (1g, 7.7 mmol) was reacted with 3,3,4-trimethyl-pentanal (1.1 g, 8 mmol) in CH₂Cl₂ at room temperature for 5 h to give [3,3,4-trimethyl-pent-(E)-ylideneamino]-acetic acid tert-butyl ester as a colorless oil (1.5 g, 80%).

### Example 78b

### Preparation of intermediate rac-(2R,3S,4R,5S)-3-(3-chloro-2-fluoro-phenyl)-4-(4-chloro-2-fluoro-phenyl)-4-cyano-5-(2,2,3-trimethyl-butyl)-pyrrolidine-2-carboxylic acid tert-butyl ester

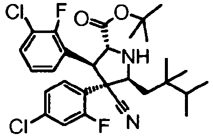

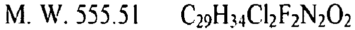

In a manner similar to the method described in **Example 1c,** [3,3,4-trimethyl-pent-(E)-ylideneamino]-acetic acid tert-butyl ester prepared in **Example 78a** (1.5 g, 6.2 mmol) was reacted with (Z)-3-(3-chloro-2-fluoro-phenyl)-2-(4-chloro-2-fluoro-phenyl)-acrylonitrile (1.1 g, 3.5 mmol) prepared in **Example 52a,** AgF (1.2 g, 9.5 mmol), and triethylamine (2 g, 20 mmol) in dichloromethane (150 mL) at room temperature for 18 h to give rac-(2R,3S,4R,5S)-3-(3-chloro-2-fluoro-phenyl)-4-(4-chloro-2-fluoro-phenyl)-4-cyano-5-(2,2,3-trimethyl-butyl)-pyrrolidine-2-carboxylic acid tert-butyl ester as a white foam (1.1 g, 56%).

### Example 78c

### Preparation of intermediate rac-(2R,3S,4R,5S)-3-(3-chloro-2-fluoro-phenyl)-4-(4-chloro-2-fluoro-phenyl)-4-cyano-5-(2,2,3-trimethyl-butyl)-pyrrolidine-2-carboxylic acid trifluoroacetic acid

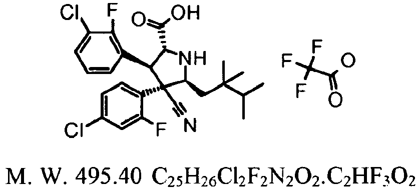

In a manner similar to the method described in **Example 25a,** rac-(2R,3S,4R,5S)-3-(3-chloro-2-fluoro-phenyl)-4-(4-chloro-2-fluoro-phenyl)-4-cyano-5-(2,2,3-trimethyl-butyl)-pyrrolidine-2-carboxylic acid tert-butyl ester prepared in **Example 78b** (1.1 g, 2 mmol) was reacted with trifluoroacetic acid in dichloromethane at room temperature to give rac-(2R,3S,4R,5S)-3-(3-chloro-2-fluoro-phenyl)-4-(4-chloro-2-fluoro-phenyl)-4-cyano-5-(2,2,3-trimethyl-butyl)-pyrrolidine-2-carboxylic acid trifluoroacetic acid as a off white solid (1.1 g, 91%).

### Example 78d

### Preparation of rac-(2R,3S,4R,5S)-3-(3-chloro-2-fluoro-phenyl)-4-(4-chloro-2-fluoro-phenyl)-4-cyano-5-(2,2,3-trimethyl-butyl)-pyrrolidine-2-carboxylic acid ((S)-3,4-dihydroxy-butyl)-amide

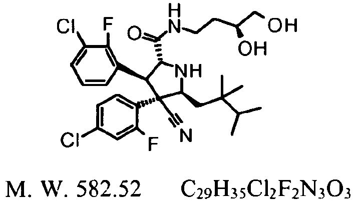

In a manner similar to the method described in **Examples 42c and 42d,** rac-(2R,3S,4R,5S)-3-(3-chloro-2-fluoro-phenyl)-4-(4-chloro-2-fluoro-phenyl)-4-cyano-5-(2,2,3-trimethyl-butyl)-pyrrolidine-2-carboxylic acid trifluoroacetic acid prepared in **Example 78c** (0.55 g, 0.9 mmol) was reacted with 2-((S)-2,2-dimethyl-[1,3]dioxolan-4-yl)-ethylamine (0.39 g, 2.7 mmol), HATU (0.62 g, 1.6 mmol) and iPr₂NEt (0.78 mL, 4.5 mmol) in CH₂Cl₂ at room temperature for 20 h, then reacted with aqueous HCl solution in tetrahydrofuran at room temperature for 2 h to give rac-(2R,3S,4R,4S)-3-(3-chloro-2-fluoro-phenyl)-4-(4-chloro-2-fluoro-phenyl)-4-cyano-5-(2,2,3-trimethyl-butyl)-pyrrolidine-2-carboxylic acid ((S)-3,4-dihydroxy-butyl)-amide as a white solid (0.32 g, 62%).

HRMS (ES⁺) m/z Calcd for C₂₉H₃₅Cl₂F₂N₃O₃+ H [(M+H)⁺]: 582.2097, found: 582.2095.

### Example 78e

### Preparation of (2R,3S,4R,5S)-3-(3-chloro-2-fluoro-phenyl)-4-(4-chloro-2-fluoro-phenyl)-4-cyano-5-(2,2,3-trimethyl-butyl)-pyrrolidine-2-carboxylic acid ((S)-3,4-dihydroxy-butyl)-amide

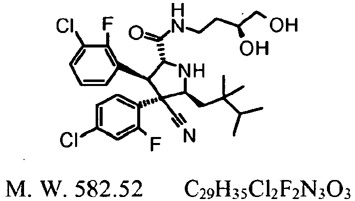

Rac-(2R,3S,4R,5S)-3-(3-chloro-2-fluoro-phenyl)-4-(4-chloro-2-fluoro-phenyl)-4-cyano-5-(2,2,3-trimethyl-butyl)-pyrrolidine-2-carboxylic acid ((S)-3,4-dihydroxy-butyl)-amide (0.25 g) was separated by chiral SFC chromatography to provide chiral (2R,3S,4R,5S)-3-(3-chloro-2-fluoro-phenyl)-4-(4-chloro-2-fluoro-phenyl)-4-cyano-5-(2,2,3-trimethyl-butyl)-pyrrolidine-2-carboxylic acid ((S)-3,4-dihydroxy-butyl)-amide as a white solid (0.1 g, 40%) and chiral (2S,3R,4S,5R)-3-(3-chloro-2-fluoro-phenyl)-4-(4-chloro-2-fluoro-phenyl)-4-cyano-5-(2,2,3-trimethyl-butyl)-pyrrolidine-2-carboxylic acid ((S)-3,4-dihydroxy-butyl)-amide as a white solid (0.1 g, 40%).

### Example 79a

### Preparation of rac-(2R,3S,4R,5S)-3-(3-chloro-2-fluoro-phenyl)-4-(4-chloro-2-fluoro-phenyl)-4-cyano-5-(2,2,3-trimethyl-butyl)-pyrrolidine-2-carboxylic acid ((R)-3,4-dihydroxy-butyl)-amide

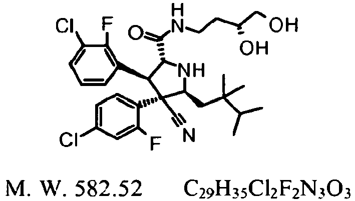

In a manner similar to the method described in **Example 72a** rac-(2R,3S,4R,5S)-3-(3-chloro-2-fluoro-phenyl)-4-(4-chloro-2-fluoro-phenyl)-4-cyano-5-(2,2,3-trimethyl-butyl)-pyrrolidine-2-carboxylic acid trifluoroacetic acid prepared in **Example 78c** (0.55 g, 0.9 mmol) was reacted with 2-((R)-2,2-dimethyl-[1,3]dioxolan-4-yl)-ethylamine (0.39 g, 2.7 mmol), HATU (0.62 g, 1.6 mmol) and iPr₂NEt (0.78 mL, 4.5 mmol) in CH₂Cl₂ at room temperature for 20 h, then reacted with aqueous HCl solution in tetrahydrofuran at room temperature for 2h to give rac-(2R,3S,4R,5S)-3-(3-chloro-2-fluoro-phenyl)-4-(4-chloro-2-fluoro-phenyl)-4-cyano-5-(2,2,3-trimethyl-butyl)-pyrrolidine-2-carboxylic acid ((R)-3,4-dihydroxy-butyl)-amide as a white solid (0.3 g, 58%).

HRMS (ES⁺) m/z Calcd for C₂₉H₃₅Cl₂F₂N₃O₃+ H [(M+H)⁺]: 582.2097, found: 582.2094.

### Example 79b

### Preparation of (2R,3S,4R,5S)-3-(3-chloro-2-fluoro-phenyl)-4-(4-chloro-2-fluoro-phenyl)-4-cyano-5-(2,2,3-trimethyl-butyl)-pyrrolidine-2-carboxylic acid ((R)-3,4-dihydroxy-butyl)-amide

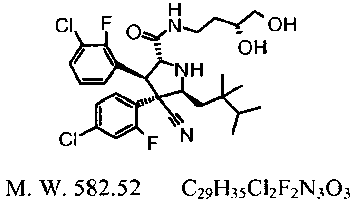

Rac-(2R,3S,4R,5S)-3-(3-chloro-2-fluoro-phenyl)-4-(4-chloro-2-fluoro-phenyl)-4-cyano-5-(2,2,3-trimethyl-butyl)-pyrrolidine-2-carboxylic acid ((R)-3,4-dihydroxy-butyl)-amide (0.29 g) was separated by chiral SFC chromatographyto provide chiral (2R,3S,4R,5S)-3-(3-chloro-2-fluoro-phenyl)-4-(4-chloro-2-fluoro-phenyl)-4-cyano-5-(2,2,3-trimethyl-butyl)-pyrrolidine-2-carboxylic acid ((R)-3,4-dihydroxy-butyl)-amide as a white solid (0.12 g, 41%) and chiral (2S,3R,4S,5R)-3-(3-chloro-2-fluoro-phenyl)-4-(4-chloro-2-fluoro-phenyl)-4-cyano-5-(2,2,3-trimethyl-butyl)-pyrrolidine-2-carboxylic acid ((R)-3,4-dihydroxy-butyl)-amide as a white solid (0.12 g, 41%).

### Example 80a

### Preparation of intermediate [3,3-dimethyl-pent-4-en-(E)-ylideneamino]-acetic acid tert-butyl ester

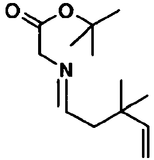

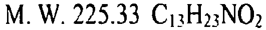

### Step A

To a solution of methyl 3,3-dimethyl-4-pentenoate (Aldrich) (6.1 g, 43 mmol) in anhydrous ethyl ether (100 mL) at 0 °C was added a tetrahydrofuran solution (2 M) of LiAlH₄ (32 mL, 64 mmol) under nitrogen. The reaction mixture was stirred at 0 °C for 1 h, then poured into a ice-water. The mixture was extracted with ethyl acetate. The organic layer were separated, washed with water, aqueous HCl solution, brine, dried over MgSO₄, and concentrated to give 3,3-dimethylpent-4-en-1-ol as a colorless oil (4.8 g, 98%).

### Step B

To a solution of oxalyl chloride (5.9 g, 46 mmol) (Aldrich) in dichloromethane (60 mL) at -78 °C was added the solution of dimethyl sulfoxide (6.6 mL, 92 mmol) in dichloromethane dropwise. After 5 mins, the solution of 3,3-dimethyl-pent-4-en-1-ol (4.8 g, 42 mmol) in dichloromethane (10 mL) was added dropwise. The reaction mixture was stirred at -78 °C for 15 min. Triethylamine (21 mL, 0.15 mol) was added and the reaction mixture was slowly warmed up to room temperature and stirred at room temperature for 45 min. The water was added. The organic layers were separated, and the aqueous layer was extracted with dichloromethane. The organic layers were combined, washed with 10% of HCl, saturated NaHCO₃, brine, dried over MgSO₄, and concentrated to give 3,3-dimethyl-pent-4-enal as a colorless oil (Yield: 3.2 g, 68%). **Step C**. In a manner similar to the method described in **Example 1a**, glycine tert-butyl ester (1.3 g, 10 mmol) was reacted with 3,3-dimethyl-pent-4-enal (1.2 g, 11 mmol) in CH₂Cl₂ at room temperature for 18 h to give [3,3-dimethyl-pent-4-en-(E)-ylideneamino]-acetic acid tert-butyl ester as a colorless oil (2.1 g, 93%).

### Example 80b

### Preparation of intermediate rac-(2R,3S,4R,5S)-3-(3-chloro-2-fluoro-phenyl)-4-(4-chloro-2-fluoro-phenyl)-4-cyano-5-(2,2-dimethyl-but-3-enyl)-pyrrolidine-2-carboxylic acid tert-butyl ester

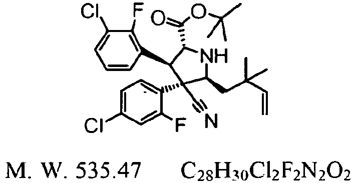

In a manner similar to the method described in **Example 1c**, [3,3-dimethyl-pent-4-en-(E)-ylideneamino]-acetic acid tert-butyl ester prepared in **Example 80a** (2.1 g, 9.3 mmol) was reacted with (Z)-3-(3-chloro-2-fluoro-phenyl)-2-(4-chloro-2-fluoro-phenyl)-acrylonitrile (2 g, 6.4 mmol) prepared in **Example 52a**, AgF (0.9 g, 7.1 mmol), and triethylamine (1.5 g, 15 mmol) in dichloromethane (150 mL) at room temperature for 18 h to give rac-(2R,3S,4R,5S)-3-(3-chloro-2-fluoro-phenyl)-4-(4-chloro-2-fluoro-phenyl)-4-cyano-5-(2,2-dimethyl-but-3-enyl)-pyrrolidine-2-carboxylic acid tert-butyl ester as a white foam (0.98 g, 56%).

### Example 80c

### Preparation of intermediate rac-(2R,3S,4R,5S)-3-(3-chloro-2-fluoro-phenyl)-4-(4-chloro-2-fluoro-phenyl)-4-cyano-5-(2,2-dimethyl-but-3-enyl)-pyrrolidine-2-carboxylic acid trifluoroacetic acid

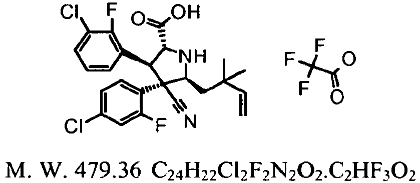

In a manner similar to the method described in **Example 25a**, rac-(2R,3S,4R,5S)-3-(3-chloro-2-fluoro-phenyl)-4-(4-chloro-2-fluoro-phenyl)-4-cyano-5-(2,2-dimethyl-but-3-enyl)-pyrrolidine-2-carboxylic acid tert-butyl ester prepared in **Example 80b** (1.0 g, 1.7 mmol) was reacted with trifluoroacetic acid in dichloromethane at room temperature to give rac-(2R,3S,4R,5S)-3-(3-chloro-2-fluoro-phenyl)-4-(4-chloro-2-fluoro-phenyl)-4-cyano-5-(2,2-dimethyl-but-3-enyl)-pyrrolidine-2-carboxylic acid trifluoroacetic acid as a white solid (1.0 g, 91%).

### Example 80d

### Preparation of intermediate rac-(2R,3S,4R,5S)-3-(3-chloro-2-fluoro-phenyl)-4-(4-chloro-2-fluoro-phenyl)-4-cyano-5-(2,2-dimethyl-but-3-enyl)-pyrrolidine-2-carboxylic acid [2-((S)-2,2-dimethyl-[1,3]dioxolan-4-yl)-ethyl]-amide

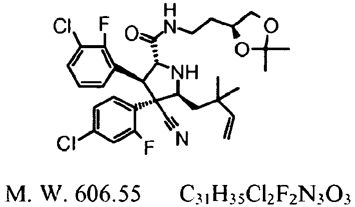

In a manner similar to the method described in **Example 42c**, rac-(2R,3S,4R,5S)-3-(3-chloro-2-fluoro-phenyl)-4-(4-chloro-2-fluoro-phenyl)-4-cyano-5-(2,2-dimethyl-but-3-enyl)-pyrrolidine-2-carboxylic acid trifluoroacetic acid prepared in **Example 80c** (1.0 g, 1.69 mmol) was reacted with 2-((S)-2,2-dimethyl-[1,3]dioxolan-4-yl)-ethylamine (0.73 g, 5.0 mmol), HATU (1.15 g, 3 mmol) and iPr₂NEt (1.46 mL, 8.4 mmol) in CH₂Cl₂ at room temperature for 20 h to give rac-(2R,3S,4R,5S)-3-(3-chloro-2-fluoro-phenyl)-4-(4-chloro-2-fluoro-phenyl)-4-cyano-5-(2,2-dimethyl-but-3-enyl)-pyrrolidine-2-carboxylic acid [2-((S)-2,2-dimethyl-[1,3]dioxolan-4-yl)-ethyl]-amide as a white solid (0.82 g, 80%).

### Example 80e

### Preparation of rac-(2R,35,4R,55)-3-(3-chloro-2-fluoro-phenyl)-4-(4-chloro-2-fluoro-phenyl)-4-cyano-5-(2,2-dimethyl-but-3-enyl)-pyrrolidine-2-carboxylic acid ((S)-3,4-dihydroxy-butyl)-amide

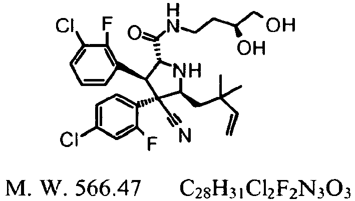

In a manner similar to the method described in **Example42d**, rac-(2R,3S,4R,5S)-3-(3-chloro-2-fluoro-phenyl)-4-(4-chloro-2-fluoro-phenyl)-4-cyano-5-(2,2-dimethyl-but-3-enyl)-pyrrolidine-2-carboxylic acid [2-((S)-2,2-dimethyl-[1,3]dioxolan-4-yl)-ethyl]-amide prepared in **Example 80d** (0.36 g, 0.59 mmol) was reacted with aqueous HCl solution (1 N, 1 mL) in tetrahydrofuran (10 mL) at room temperature for 2 h to give rac-(2R,3S,4R,5S)-3-(3-chloro-2-fluoro-phenyl)-4-(4-chloro-2-fluoro-phenyl)-4-cyano-5-(2,2-dimethyl-but-3-enyl)-pyrrolidine-2-carboxylic acid ((S)-3,4-dihydroxy-butyl)-amide as a white solid (0.32 g, 95%).

HRMS (ES⁺) m/z Calcd for C₂₈H₃₁Cl₂F₂N₃O₃+ H [(M+H)⁺]: 566.1784, found: 566.1786.

### Example 81a

### Preparation of intermediate rac-(2R,3S,4R,5S)-3-(3-chloro-2-fluoro-phenyl)-4-(4-chloro-2-fluoro-phenyl)-4-cyano-5-(2,2-dimethyl-butyl)-pyrrolidine-2-carboxylic acid [2-((S)-2,2-dimethyl-[1,3]dioxolan-4-yl)-ethyl]-amide

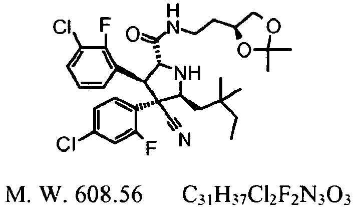

A suspension of rac-(2R,3S,4R,5S)-3-(3-chloro-2-fluoro-phenyl)-4-(4-chloro-2-fluoro-phenyl)-4-cyano-5-(2,2-dimethyl-but-3-enyl)-pyrrolidine-2-carboxylic acid [2-((S)-2,2-dimethyl-[1,3]dioxolan-4-yl)-ethyl]-amide prepared in **Example 80d** (0.4 g, 0.65 mmol) and PtO₂ (0.2 g) in ethyl acetate (15 mL) was vigorously shaken under H₂ atomosphere (30 psi) for I h. The mixture was filtered through a short pad of celite, and the filtrate was concentrated to give rac-(2R,3S,4R,5S)-3-(3-chloro-2-fluoro-phenyl)-4-(4-chloro-2-fluoro-phenyl)-4-cyano-5-(2,2-dimethyl-butyl)-pyrrolidine-2-carboxylic acid [2-((S)-2,2-dimethyl-[1,3]dioxolan-4-yl)-ethyl]-amide as a white gum (0.33 g, 83%).

### Example 81b

### Preparation of rac-(2R,35,4R,5S)-3-(3-chloro-2-fluoro-phenyl)-4-(4-chloro-2-fluoro-phenyl)-4-cyano-5-(2,2-dimethyl-butyl)-pyrrolidine-2-carboxylic acid ((S)-3,4-dihydroxy-butyl)-amide

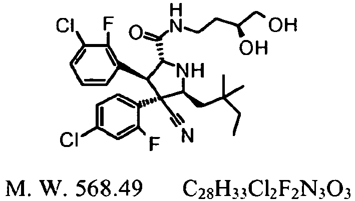

In a manner similar to the method described in **Example 42d**, rac-(2R,3S,4R,5S)-3-(3-chloro-2-fluoro-phenyl)-4-(4-chloro-2-fluoro-phenyl)-4-cyano-5-(2,2-dimethyl-butyl)-pyrrolidine-2-carboxylic acid [2-((S)-2,2-dimethyl-[1,3]dioxolan-4-yl)-ethyl]-amide prepared in **Example 81a** (0.41 g, 0.67 mmol) was reacted with aqueous HCl solution (1 N, 1 mL) in tetrahydrofuran (10 mL) at room temperature for 2 h to give rac-(2R,3S,4R,5S)-3-(3-chloro-2-fluoro-phenyl)-4-(4-chloro-2-fluoro-phenyl)-4-cyano-5-(2,2-dimethyl-butyl)-pyrrolidine-2-carboxylic acid ((S)-3,4-dihydroxy-butyl)-amide as a white solid (0.38 g, 99%).

HRMS (ES⁺) m/z Calcd for C₂₈H₃₃Cl₂F₂N₃O₃+ H [(M+H)⁺]: 568.1940, found: 568.1942.

### Example 82a

### Preparation of intermediate [3-methyl-3-phenyl-but-(E)-ylideneamino]-acetic acid tert-butyl ester

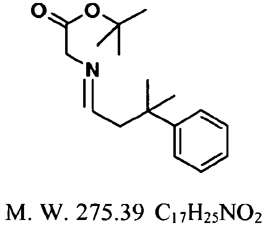

### Step A

To a solution of 3-methyl-3-phenylbutanoic acid (ChemBridge) (4.46 g, 25 mmol) in anhydrous tetrahydrofuran (150 mL) at 0 °C was added a tetrahydrofuran solution (1 M) of BH₃.THF (Aldrich, 50 mL, 50 mmol) under nitrogen. The reaction mixture was stirred at room temperature for 3 h, then concentrated. The residue was partitoned between ethyl acetate and water. The organic layer were separated, washed with water, aqueous HCl solution, brine, dried over MgSO₄, and concentrated to give 3-methyl-3-phenyl-butan-1-ol as a colorless oil (4.1 g, 100%).

### Step B

To a solution of oxalyl chloride (1.7 g, 13 mmol) (Aldrich) in dichloromethane (50 mL) at -78 °C was added the solution of dimethyl sulfoxide (1.9 mL, 27 mmol) in dichloromethane (10 mL) dropwise. After 5 mins, the solution of 3-methyl-3-phenyl-butan-1-ol (2 g, 12 mmol) in dichloromethane (10 mL) was added dropwise. The reaction mixture was stirred at -78 °C for 15 min. Triethylamine (6.1 mL, 44 mol) was added and the reaction mixture was slowly warmed up to room temperature and stirred at room temperature for 45 min. The water was added. The organic layers were separated, and the aqueous layer was extracted with dichloromethane. The organic layers were combined, washed with 10% of HCl, saturated NaHCO₃, brine, dried over MgSO₄, and concentrated to give 3-methyl-3-phenyl-butyraldehyde as a colorless oil (Yield: 1.8 g, 90%).

### Step C

In a manner similar to the method described in **Example 1a**, glycine tert-butyl ester (1.3 g, 10 mmol) was reacted with 3-methyl-3-phenyl-butyraldehyde (1.8 g, 11 mmol) in CH₂Cl₂ at room temperature for 18 h to give [[3-methyl-3-phenyl-but-(E)-ylideneamino]-acetic acid tert-butyl ester as a colorless oil (2.3 g, 93%).

### Example 82b

### Preparation of intermediate rac-(2R,3S,4R,5S)-3-(3-chloro-2-fluoro-phenyl)-4-(4-chloro-2-fluoro-phenyl)-4-cyano-5-(2-methyl-2-phenyl-propyl)-pyrrolidine-2-carboxylic acid tert-butyl ester

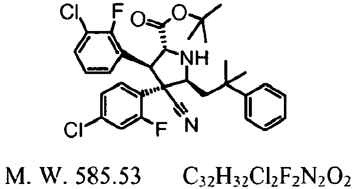

In a manner similar to the method described in **Example 1c**, [[3-methyl-3-phenyl-but-(E)-ylideneamino]-acetic acid tert-butyl ester prepared in **Example 82a** (2.3 g, 8.3 mmol) was reacted with (Z)-3-(3-chloro-2-fluoro-phenyl)-2-(4-chloro-2-fluoro-phenyl)-acrylonitrile (2.4 g, 7.7 mmol) prepared in **Example 52a**, AgF (0.7 g, 5.5mmol), and triethylamine (2.1 g, 21 mmol) in dichloromethane (150 mL) at room temperature for 18 h to give rac-(2R,3S,4R,5S)-3-(3-chloro-2-fluoro-phenyl)-4-(4-chloro-2-fluoro-phenyl)-4-cyano-5-(2-methyl-2-phenyl-propyl)-pyrrolidine-2-carboxylic acid tert-butyl ester as a white foam (1 g, 22%).

### Example 82c

### Preparation of intermediate rac-(2R,3S,4R,5S)-3-(3-chloro-2-fluoro-phenyl)-4-(4-chloro-2-fluoro-phenyl)-4-cyano-5-(2-methyl-2-phenyl-propyl)-pyrrolidine-2-carboxylic acid trifluoroacetic acid

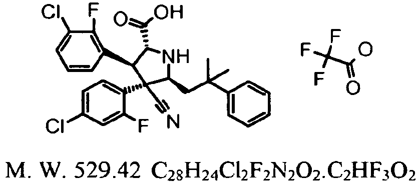

In a manner similar to the method described in **Example 25a,** rac-(2R,3S,4R,5S)-3-(3-chloro-2-fluoro-phenyl)-4-(4-chloro-2-fluoro-phenyl)-4-cyano-5-(2-methyl-2-phenyl-propyl)-pyrrolidine-2-carboxylic acid tert-butyl ester prepared in **Example 82b** (1.0 g, 1.7 mmol) was reacted with trifluoroacetic acid in dichloromethane at room temperature to give rac-(2R,3S,4R,5S)-3-(3-chloro-2-fluoro-phenyl)-4-(4-chloro-2-fluoro-phenyl)-4-cyano-5-(2-methyl-2-phenyl-propyl)-pyrrolidine-2-carboxylic acid trifluoroacetic acid as a white solid (1.0 g, 91%).

### Example 82d

### Preparation of rac-(2R,3S,4R,5S)-3-(3-chloro-2-fluoro-phenyl)-4-(4-chloro-2-fluoro-phenyl)-4-cyano-5-(2-methyl-2-phenyl-propyl)-pyrrolidine-2-carboxylic acid ((S)-3,4-dihydroxy-butyl)-amide

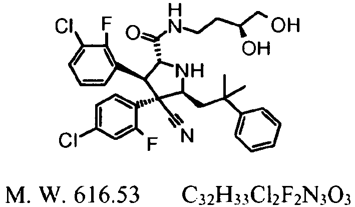

In a manner similar to the method described in **Examples 42c, 42d**, rac-(2R,3S,4R,5S)-3-(3-chloro-2-fluoro-phenyl)-4-(4-chloro-2-fluoro-phenyl)-4-cyano-5-(2-methyl-2-phenyl-propyl)-pyrrolidine-2-carboxylic acid trifluoroacetic acid prepared in **Example 82c** (0.35 g, 0.54 mmol) was reacted with 2-((S)-2,2-dimethyl-[1,3]dioxolan-4-yl)-ethylamine (0.24 g, 1.62 mmol), HATU (0.37 g, 0.98 mmol) and iPr₂NEt (0.47 mL, 2.7 mmol) in CH₂Cl₂ at room temperature for 20 h, then reacted with aqueous HCl solution in tetrahydrofuran at room temperature to give rac-(2R,3S,4R,5S)-3-(3-chloro-2-fluoro-phenyl)-4-(4-chloro-2-fluoro-phenyl)-4-cyano-5-(2-methyl-2-phenyl-propyl)-pyrrolidine-2-carboxylic acid ((S)-3,4-dihydroxy-butyl)-amide as a white solid (0.21 g, 66%).

HRMS (ES⁺) m/z Calcd for C₃₂H₃₃Cl₂F₂N₃O₃+ H [(M+H)⁺]: 616.1940, found: 616.1939.

### Example 83a

### Preparation of intermediate (Z)-3-(3-chloro-5-fluoro-phenyl)-2-(4-chloro-2-fluoro-phenyl)-acrylonitrile

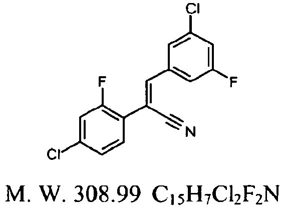

In a manner similar to the method described in **Example 1b**, 4-chloro-2-fluorophenylacetonitrite (3.4 g, 20 mmol) was reacted with 3-chloro-5-fluoro-benzaldehyde (Aldrich) (3.77 g, 24 mmol), methanolic solution (25 wt%) of sodium methoxide (4.99 mL, 22 mmol) in methanol (90 mL) at 50°C for 3 h to give (Z)-3-(3-chloro-5-fluoro-phenyl)-2-(4-chloro-2-fluoro-phenyl)-acrylonitrile as a white powder (5.5 g, 90%).

### Example 83b

### Preparation of intermediate rac-(2R,3R,4R,5S)-3-(3-chloro-5-fluoro-phenyl)-4-(4-chloro-2-fluoro-phenyl)-4-cyano-5-(2,2-dimethyl-propyl)-pyrrolidine-2-carboxylic acid tert-butyl ester

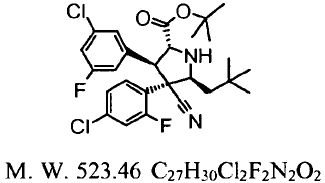

In a manner similar to the method described in **Example 1c**, [3-methyl-but-(E)-ylideneamino]-acetic acid tert-butyl ester prepared in **Example 1a** (2.1 g, 10 mmol) was reacted with (Z)-3-(3-chloro-5-fluoro-phenyl)-2-(4-chloro-2-fluoro-phenyl)-acrylonitrile (2.5 g, 8 mmol) prepared in **Example 83a**, AgF (1.0 g, 8 mmol), and triethylamine (2.8 mL, 20 mmol) in dichloromethane (120 mL) at room temperature for 18 h to give rac-(2R,3R,4R,5S)-3-(3-chloro-5-fluoro-phenyl)-4-(4-chloro-2-fluoro-phenyl)-4-cyano-5-(2,2-dimethyl-propyl)-pyrrolidine-2-carboxylic acid tert-butyl ester as a white foam (1.2 g, 29%).

### Example 83c

### Preparation of intermediate rac-(2R,3R,4R,5S)-3-(3-chloro-5-fluoro-phenyl)-4-(4-chloro-2-fluoro-phenyl)-4-cyano-5-(2,2-dimethyl-propyl)-pyrrolidine-2-carboxylic acid trifluoroacetic acid

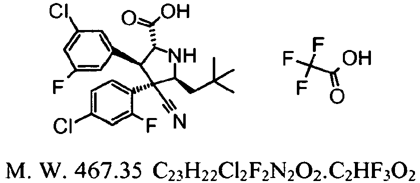

In a manner similar to the method described in **Example 25a**, rac-(2R,3R,4R,5S)-3-(3-chloro-5-fluoro-phenyl)-4-(4-chloro-2-fluoro-phenyl)-4-cyano-5-(2,2-dimethyl-propyl)-pyrrolidine-2-carboxylic acid tert-butyl ester prepared in **Example 83b** (1.2 g, 2.2 mmol) was reacted with trifluoroacetic acid in dichloromethane at room temperature to give rac-(2R,3R,4R,5S)-3-(3-chloro-5-fluoro-phenyl)-4-(4-chloro-2-fluoro-phenyl)-4-cyano-5-(2,2-dimethyl-propyl)-pyrrolidine-2-carboxylic acid trifluoroacetic acid as a white solid (1.3 g, 97%).

### Example 83d

### Preparation of rac-(2R,3R,4R,5S)-3-(3-chloro-5-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide

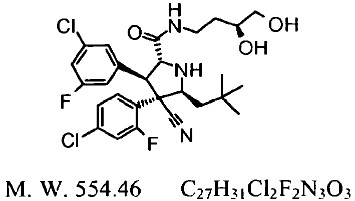

In a manner similar to the method described in **Examples 42c, 42d**, rac-(2R,3R,4R,5S)-3-(3-chloro-5-fluoro-phenyl)-4-(4-chloro-2-fluoro-phenyl)-4-cyano-5-(2,2-dimethyl-propyl)-pyrrolidine-2-carboxylic acid trifluoroacetic acid prepared in **Example 83c** (0.4 g, 0.69 mmol) was reacted with 2-((S)-2,2-dimethyl-[1,3]dioxolan-4-yl)-ethylamine (0.3 g, 2.06 mmol), HATU (0.47 g, 1.24 mmol) and iPr₂NEt (0.6 mL, 3.44 mmol) in CH₂Cl₂ at room temperature for 20 h, then reacted with aqueous HCl solution in tetrahydrofuran at room temperature to give rac-(2R,3R,4R,5S)-3-(3-chloro-5-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide as a white solid (0.13g, 35%).

HRMS (ES⁺) m/z Calcd for C₂₇H₃₁Cl₃FN₃O₃+ H [(M+H)⁺]: 554.1784, found: 554.1782.

### Example 84a

### Preparation of intermediate (Z)-3-{2-[2-(tert-butyl-dimethyl-silanyloxy)-ethoxy]-5-chloro-phenyl}-2-(4-chloro-2-fluoro-phenyl)-acrylonitrile

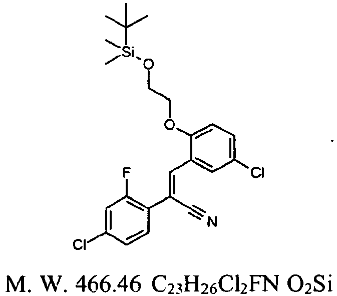

### Step A

To a solution of 5-chlorosalicylaldehyde (2 g, 12.8 mmol) (Aldrich) in N,N-dimethylformamide (40 mL) was added K₂CO₃ (5.3 g, 38 mmo), and (2-bromo-ethoxy)-tert-butyl-dimethyl-silane

(3.67 g, 15 mmol, Aldrich). The reaction mixture was heated at 60 °C for 18 h. The crude was cooled to room temperature, diluted with ethyl acetate, washed with water, brine. The organic layer was separated, concentrated, and the residue was purified by chromatography (EtOAc:Hexanes=1:8, then 1:4) to give 2-[2-(tert-butyl-dimethyl-silanyloxy)-ethoxy]-5-chlorobenzaldehyde as a brown oil (Yield 3.8 g, 91%).

### Step B

In a manner similar to the method described in **Example 1b**, 4-chloro-2-fluorophenylacetonitrile (1.7 g, 10 mmol) was reacted with 2-[2-(tert-butyl-dimethyl-silanyloxy)-ethoxy]-5-chlorobenzaldehyde (3.8 g, 12.5 mmol), methanolic solution (25 wt%) of sodium methoxide (2.5 mL, 11 mmol) in methanol (60 mL) at 50 °C for 3 h to give (Z)-3-{2-[2-(tert-butyl-dimethylsilanyloxy)-ethoxy]-5-chloro-phenyl}-2-(4-chloro-2-fluoro-phenyl)-acrylonitrile as a yellow oil (4.5 g, 80%).

### Example 84b

### Preparation of intermediate rac-(2R,3S,4R,5S)-3-{2-[2-(tert-butyl-dimethyl-silanyloxy)-ethoxy]-5-chloro-phenyl}-4-(4-chloro-2-fluoro-phenyl)-4-cyano-5-(2,2-dimethyl-propyl)-pyrrolidine-2-carboxylic acid tert-butyl ester

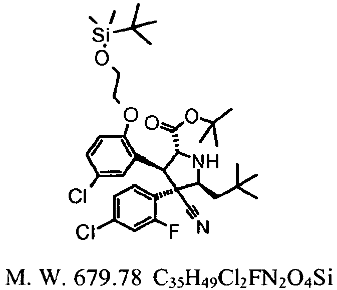

In a manner similar to the method described in **Example 1c**, [3-methyl-but-(E)-ylideneamino]-acetic acid tert-butyl ester prepared in **Example 1a** (2.1 g, 10 mmol) was reacted with (Z)-3-{2-[2-(tert-butyl-dimethyl-silanyloxy)-ethoxy]-5-chloro-phenyl}-2-(4-chloro-2-fluoro-phenyl)-acrylonitrile (3.7 g, 8 mmol) prepared in **Example 84a**, AgF (1.0 g, 8 mmol), and triethylamine (2.8 mL, 20 mmol) in dichloromethane (120 mL) at room temperature for 18 h to give rac-(2R,3S,4R,5S)-3-{2-[2-(tert-butyl-dimethyl-silanyloxy)-ethoxy]-5-chloro-phenyl}-4-(4-chloro-2-fluoro-phenyl)-4-cyano-5-(2,2-dimethyl-propyl)-pyrrolidine-2-carboxylic acid tert-butyl ester as a white foam (1.0 g, 18%).

### Example 84c

Preparation of intermediate rac-(2R,3S,4R,5S)-4-(4-chloro-2-fluoro-phenyl)-3-[5-chloro-2-(2-hydroxy-ethoxy)-phenyl]-4-cyano-5-(2,2-dimethyl-propyl)-pyrrolidine-2-carboxylic acid trifluoroacetic acid

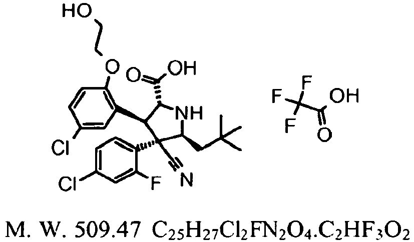

In a manner similar to the method described in **Example 25a**, rac-(2R,3S,4R,5S)-3-{2-[2-(tert-butyl-dimethyl-silanyloxy)-ethoxy]-5-chloro-phenyl}-4-(4-chloro-2-fluoro-phenyl)-4-cyano-5-(2,2-dimethyl-propyl)-pyrrolidine-2-carboxylic acid tert-butyl ester prepared in **Example 84b** (1.0 g, 1.5 mmol) was reacted with trifluoroacetic acid in dichloromethane at room temperature to give rac-(2R,3S,4R,5S)-4-(4-chloro-2-fluoro-phenyl)-3-[5-chloro-2-(2-hydroxy-ethoxy)-phenyl]-4-cyano-5-(2,2-dimethyl-propyl)-pyrrolidine-2-carboxylic acid trifluoroacetic acid as a white solid (0.9 g, 98%).

### Example 84d

### Preparation of rac-(2R,3S,4R,5S)-4-(4-chloro-2-fluoro-phenyl)-3-[5-chloro-2-(2-hydroxyethoxy)-phenyl]-4-cyano-5-(2,2-dimethyl-propyl)-pyrrolidine-2-carboxylic acid ((S)-3,4-dihydroxy-butyl)-amide

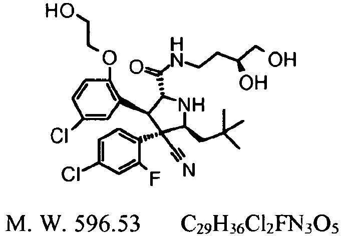

In a manner similar to the method described in **Examples 42c, 42d**, rac-(2R,3S,4R,5S)-4-(4-chloro-2-fluoro-phenyl)-3-[5-chloro-2-(2-hydroxy-ethoxy)-phenyl]-4-cyano-5-(2,2-dimethylpropyl)-pyrrolidine-2-carboxylic acid trifluoroacetic acid prepared in **Example 84c** (0.4 g, 0.64 mmol) was reacted with 2-((S)-2,2-dimethyl-[1,3]dioxolan-4-yl)-ethylamine (0.28 g, 1.93 mmol), HATU (0.44 g, 1.15 mmol) and iPr₂NEt (0.56 mL, 3.21 mmol) in CH₂Cl₂ at room temperature for 20 h, then reacted with aqueous HCl solution in tetrahydrofuran at room temperature to give rac-(2R,3S,4R,5S)-4-(4-chloro-2-fluoro-phenyl)-3-[5-chloro-2-(2-hydroxy-ethoxy)-phenyl]-4-cyano-5-(2,2-dimethyl-propyl)-pyrrolidine-2-carboxylic acid ((S)-3,4-dihydroxy-butyl)-amide as a white solid (50 mg, 13%).

HRMS (ES⁺) m/z Calcd for C₂₉H₃₆Cl₂FN₃O₅+ H [(M+H)⁺]: 596.2089, found: 596.2087.

### Example 85a

### Preparation of intermediate (Z)-2-(4-chloro-2-fluoro-phenyl)-3-(5-chloro-2-methoxy-phenyl)-acrylonitrile

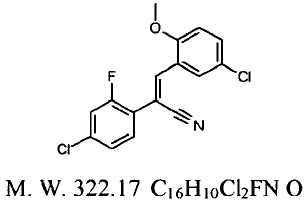

In a manner similar to the method described in **Example 1b**, 4-chloro-2-fluorophenylacetonitrile (2.8 g, 16.6 mmol) was reacted with 5-chloro-2-methoxybenzaldehyde (Matrix) (3.4 g, 19.9 mmol), methanolic solution (25 wt%) of sodium methoxide (4.17 mL, 18 mmol) in methanol (100 mL) at 50 °C for 3 h to give (Z)-2-(4-chloro-2-fluoro-phenyl)-3-(5-chloro-2-methoxyphenyl)-acrylonitrile as a white solid (2.0 g, 37%).

### Example 85b

### Preparation of intermediate rac-(2R,3S,4R,5S)-4-(4-chloro-2-fluoro-phenyl)-3-(5-chloro-2-methoxy-phenyl)-4-cyano-5-(2,2-dimethyl-propyl)-pyrrolidine-2-carboxylic acid tert-butyl ester

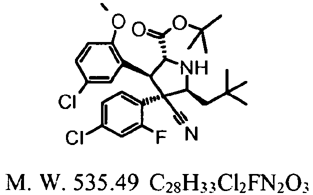

In a manner similar to the method described in **Example 1c**, [3-methyl-but-(E)-ylideneamino]-acetic acid tert-butyl ester prepared in **Example 1a** (1.1 g, 5 mmol) was reacted with (Z)-2-(4-chloro-2-fluoro-phenyl)-3-(5-chloro-2-methoxy-phenyl)-acrylonitrile (1.28 g, 4 mmol) prepared in **Example 85a**, AgF (0.76 g, 6 mmol), and triethylamine (1.38 mL, 10 mmol) in dichloromethane (100 mL) at room temperature for 18 h to give rac-(2R,3S,4R,5S)-4-(4-chloro-2-fluoro-phenyl)-3-(5-chloro-2-methoxy-phenyl)-4-cyano-5-(2,2-dimethyl-propyl)-pyrrolidine-2-carboxylic acid tert-butyl ester as a white foam (0.3 g, 14%).

### Example 85c

### Preparation of intermediate rac-(2R,3S,4R,5S)-4-(4-chloro-2-fluoro-phenyl)-3-(5-chloro-2-methoxy-phenyl)-4-cyano-5-(2,2-dimethyl-propyl)-pyrrolidine-2-carboxylic acid trifluoroacetic acid

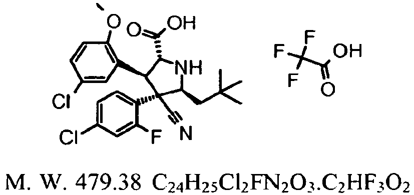

In a manner similar to the method described in **Example 25a**, rac-(2R,3S,4R,5S)-4-(4-chloro-2-fluoro-phenyl)-3-(5-chloro-2-methoxy-phenyl)-4-cyano-5-(2,2-dimethyl-propyl)-pyrrolidine-2-carboxylic acid tert-butyl ester prepared in **Example 85b** (0.3 g, 0.56 mmol) was reacted with trifluoroacetic acid in dichloromethane at room temperature to give rac-(2R,3S,4R,5S)-4-(4-chloro-2-fluoro-phenyl)-3-(5-chloro-2-methoxy-phenyl)-4-cyano-5-(2,2-dimethyl-propyl)-pyrrolidine-2-carboxylic acid trifluoroacetic acid as a white solid (0.32 g, 97%).

### Example 85d

### Preparation of rac-(2R,3S,4R,5S)-4-(4-chloro-2-fluoro-phenyl)-3-(5-chloro-2-methoxy-phenyl)-4-cyano-5-(2,2-dimethyl-propyl)-pyrrolidine-2-carboxylic acid ((S)-3,4-dihydroxy-butyl)-amide

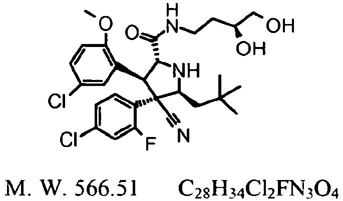

In a manner similar to the method described in **Examples 42c, 42d**, rac-(2R,3S,4R,5S)-4-(4-chloro-2-fluoro-phenyl)-3-(5-chloro-2-methoxy-phenyl)-4-cyano-5-(2,2-dimethyl-propyl)-pyrrolidine-2-carboxylic acid trifluoroacetic acid prepared in **Example 85c** (0.32 g, 0.54 mmol) was reacted with 2-((S)-2,2-dimethyl-[1,3]dioxolan-4-yl)-ethylamine (0.23 g, 1.62 mmol), HATU (0.37 g, 0.98 mmol) and iPr₂NEt (0.47 mL, 2.7 mmol) in CH₂Cl₂ at room temperature for 20 h, then reacted with aqueous HCl solution in tetrahydrofuran at room temperature to give rac-(2R,3S,4R,5S)-4-(4-chloro-2-fluoro-phenyl)-3-(5-chloro-2-methoxy-phenyl)-4-cyano-5-(2,2-dimethyl-propyl)-pyrrolidine-2-carboxylic acid ((S)-3,4-dihydroxy-butyl)-amide as a white solid (65 mg, 21%).

HRMS (ES⁺) m/z Calcd for C₂₈H₃₄Cl₂FN₃O₄+ H [(M+H⁺)]: 566.1983, found: 566.1984.

### Example 86

### Preparation of rac(2R,3R,4R,5S)-3-(3-chloro-phenyl)-4-(4-chloro-phenyl)-4-cyano-5-(2,2-dimethyl-propyl)-pyrrolidine-2-carboxylic acid (quinolin-3-ylmethyl)-amide

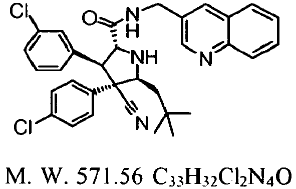

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (86.2 mg, 0.20 mmol) prepared in **Example 1d** was reacted with quinolin-3-yl-methylamine (47.5 mg, 0.3 mmol), HATU (76.0 mg, 0.2 mmol) and iPr₂NEt (0.1 mL, 0.55 mmol) in CH₂Cl₂ (2 mL) at rt overnight. to give rac-(2R,3R,4R,5S)-3-(3-chloro-phenyl)-4-(4-chloro-phenyl)-4-cyano-5-(2,2-dimethyl-propyl)-pyrrolidine-2-carboxylic acid (quinolin-3-ylmethyl)-amide (54.4 mg, 47.5 %) as a white powder.

HRMS (ES⁺) m/z Calcd for C₃₃H₃₂Cl₂N₄O+ H [(M+H)⁺]: 571.2026, found : 571.2027.

### Example 87

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid 4-trifluoromethyl-benzylamide

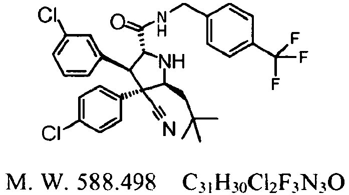

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (86.2 mg, 0.2 mmol)) prepared in **Example 1d** was reacted with 4-trifluoromethylbenzyl amine (52.5 mg, 0.3 mmol), HATU (76 mg, 0.2 mmol) and iPr₂NEt (0.1 mL, 0.55 mmol) in CH₂Cl₂ (2 mL) was stirred at rt overnight to rac-(2R,3R,4R,5S)-3-(3-chloro-phenyl)-4-(4-chloro-phenyl)-4-cyano-5-(2,2-dimethyl-propyl)-pyrrolidine-2-carboxylic acid 4-trifluoromethyl-benzylamide (58.1 mg, 58.04 %).

HRMS (ES⁺) m/z Calcd for C₃₁H₃₀Cl₂F₃N₃O+ H [(M+H)⁺]; 588.1791, found: 588.1788.

### Example 88

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid 3-trifluoromethyl-benzyl amide

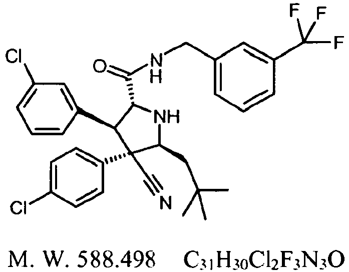

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (86.2 mg, 0.2 mmol) prepared in **Example 1d** was reacted with 3-trifluoromethylbenzyl amine (52.55 mg, 0.3 mmol), HATU (76 mg, 0.2 mmol) and iPr₂NEt (0.1 mL, 0.55 mmol) in CH₂Cl₂ (2 mL) was stirred at rt overnight to rac-(2R,3R,4R,5S)-3-(3-chloro-phenyl)-4-(4-chloro-phenyl)-4-cyano-5-(2,2-dimethyl-propyl)-pyrrolidine-2-carboxylic acid 3-trifluoromethyl-benzyl amide (26.2 mg, 26.2 %).

HRMS (ES⁺) m/z Calcd for C₃₁H₃₀Cl₂F₃N₃O+ H [(M+H)⁺]: 588.1791, found: 588.1788.

### Example 89

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid 4-hydroxy-benzylamide

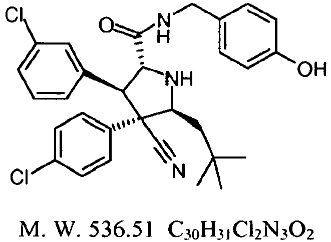

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (86.2 mg, 0.2 mmol) prepared in **Example 1d** was reacted with 4-hydroxybenzyl amine (36.9 mg, 0.3 mmol), HATU (76 mg, 0.2 mmol) and iPr₂NEt (0.1 mL, 0.55 mmol) in CH₂Cl₂ (2 mL) was stirred at rt overnight to rac-(2R,3R,4R,5S)-3-(3-chloro-phenyl)-4-(4-chloro-phenyl)-4-cyano-5-(2,2-dimethyl-propyl)-pyrrolidine-2-carboxylic acid 4-hydroxy-benzylamide (45.1 mg, 45.0 %).

HRMS (ES⁺) m/z Calcd for C₃₀H₃₁Cl₂N₃O₂⁺H [(M+H)⁺]: 536.1866, found; 536,1866.

### Example 90

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid 3-iodo-benzylamide

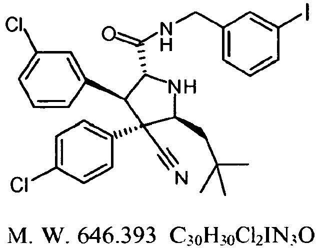

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (86.2 mg, 0.2 mmol) prepared in **Example 1d** was reacted with 3-iodobenzyl amine (68.92 mg, 0.3 mmol), HATU (76 mg, 0.2 mmol) and iPr₂NEt (0.1 mL, 0.55 mmol) in CH₂Cl₂ (2 mL) was stirred at rt overnight to rac-(2R,3R,4R,5S)-3-(3-chloro-phenyl)-4-(4-chloro-phenyl)-4-cyano-5-(2,2-dimethyl-propyl)-pyrrolidine-2-carboxylic acid 3-iodo-benzylamide (44.1 mg, 34.1 %). HRMS (ES⁺) m/z Calcd for C₃₀H₃₀Cl₂N₃O + H [(M+H)⁺]:646.0884. found; 646.0881,

### Example 91

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid (2-ethyl-butyl)-amide

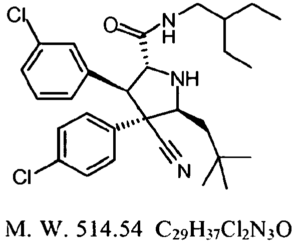

In a manner similar to the method described in **Example 1e**, rac-(2R,3R,4R,5S)-3-(3-chloro-phenyl)-4-(4-chloro-phenyl)-4-cyano-5-(2,2-dimethyl-propyl)-pyrrolidine-2-carboxylic acid (86.2 mg, 0.2 mmol) prepared in **Example 1d** was reacted with 2-ethyl butyl amine (30.3 mg, 0.3 mmol), HATU (76 mg, 0.2 mmol) and iPr₂NEt (0.1. mL, 0.55 mmol) in CH₂Cl₂ (2 mL) was stirred at rt overnight to rac-(2R,3R,4R,5S)-3-(3-chloro-phenyl)-4-(4-chloro-phenyl)-4-cyano-5-(2,2-dimethyl-propyl)-pyrrolidine-2-carboxylic acid (2-ethyl-butyl)-amide (34 mg, 33.04 %).

HRMS (ES⁺) m/z Calcd for C₂₉H₃₇Cl₂N₃O + H [(M+H)⁺]:514.2387, found: 514.2385.

### Example 92

### Preparation of rac-4-{[(2R,35,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-piperidine-1-carboxylic acid tert-butyl ester

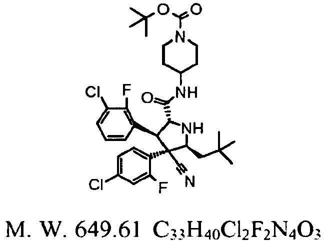

In a manner similar to the method described in **Examples 42c, 42d**, rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid trifluoroacetic acid prepared in **Example 52c** (1.8 g, 3.1 mmol) was reacted with 4-Amino-piperidine-1-carboxylic acid tert-butyl ester(Aldrich, 931 mg, 4.65 g, 4.65 mmol), HATU (2.12 g, 0.92 mmol) and iPr₂NEt (2.7 mL, 15.5 mmol) in CH₂Cl₂ at room temperature overnight, then reacted with aqueous HCl solution in tetrahydrofuran at room temperature for 2 h to give a white solid (0.758 g, 38%).

HRMS (ES⁺) m/z Caled for C₃₃H₄₀Cl₂F₂N₄O₃+H [(M+H)⁺]: 649.2519. found: 649.2518

### Example 93

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid piperidin-4-ylamide trifluoroacetic acid

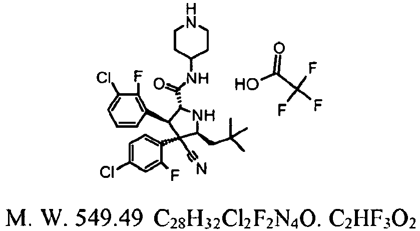

To a stirred solution of a mixture of TFA/CH₂Cl₂ (5 mL/10 mL), rac-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-piperidine-1-carboxylic acid tert-butyl ester (example 92, 510 mg, 0.79 mmol) was added and the mixture was stirred at rt for 15 min. The solvent was removed and the residue dried to give a white solid after precipation from methylene chloride and ethyl acetate. 492 mg.

HRMS (ES⁺) m/z Calcd for C₂₈H₃₂Cl₂F₂N₄O + H [(M+H)⁺]: 549.1994, found: 549.1994.

### Example 94

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-methanesulfonylpiperidin-4-yl)-amide

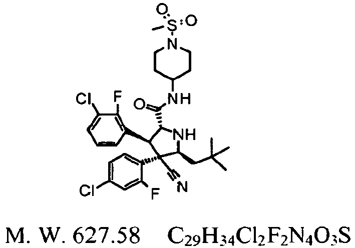

To a stirred solution of (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid piperidin-4-ylamide trifluoroacetic acid (80 mg, 0.10 mmol) in methylene chloride (10 mL), methanesulfonyl chloride (14 uL, 0.18 mmol) and triethylamine (84 uL, 0.61 mmol) were added and the misture was stirred at rt for 1 hr. The reaction was quenched with water and the organic layer was separated and dried with sodium sulfate. Removal of solvent gave the crued which was chromatographied on an ISCO machine (0-10% EtOAc/CH₂Cl₂) to give a white solid. 53 mg. HRMS (ES⁺) m/z Calcd for C₂₉H₁₄Cl₂F₂N₄O₃S+H [(M+H)⁺]: 627.1770, found: 627.1766.

### Example 95

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-methyl-carbonyl-piperidin-4-yl)-amide

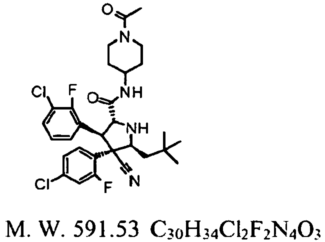

To a stirred solution of (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid piperidin-4-ylamide trifluoroacetic acid (80 mg, 0.10 mmol) in methylene chloride (10 mL), acetic anhydride (18 uL, 0.18 mmol) and triethylamine (84 uL, 0.61 mmol) were added and the misture was stirred at rt for 1.5 hr. The reaction was quenched with water and the organic layer was separated and dried with sodium sulfate. Removal of solvent gave the crued which was chromatographied on an ISCO machine (0-10% EtOAc/CH₂Cl₂) to give a white solid. 58 mg.

HRMS (ES⁺) m/z Calcd for C₃₀H₃₄Cl₂F₂N₄O₃+ H [(M+H)⁺]: 391.2100. found: 591.2099.

### Example 96

### Preparation of rac-(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (1-benzoyl-piperidin-4-yl)-amide

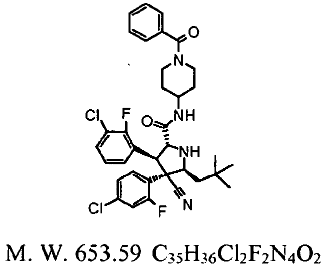

To a stirred solution of (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid piperidin-4-ylamide trifluoroacetic acid (80 mg, 0.10 mmol) in methylene chloride (10 mL), benzoyl chloride (21 uL, 0.18 mmol) and triethylamine (84 uL, 0.61 mmol) were added and the misture was stirred at rt for 1 hr. The reaction was quenched with water and the organic layer was separated and dried with sodium sulfate. Removal of solvent gave the crued which was chromatographied on an ISCO machine (0-10% EtOAc/CH₂Cl₂) to give a white solid. 36 mg. HRMS (ES⁺) m/z Calcd for C₃₅H₃₆Cl₂F₂N₄O₂ + H [(M+H)⁺] 653.2256, found: 653.2253.

### Example 97

### Preparation of rac-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}+-piperidine-1-carboxylic acid isopropylamide

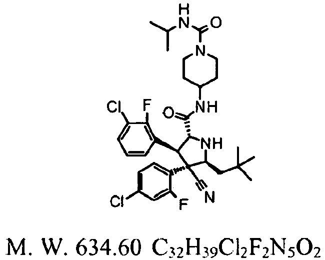

To a stirred solution of (2R,3S,4R,5S)-3-(3-Chloro-2-fluoro-phenyl)4-(4-chloro-2-fluoro-phenyl)-4-cyano-5-(2,2-dimethyl-propyl)-pyrrolidine-2-carboxylic acid piperidin-4-ylamide trifluoroacetic (80 mg, 0.10 mmol) in methylene chloride (10 mL), 2-propyl isocyante (18 uL, 0.18 mmol) and triethylamine (84 uL, 0.61 mmol) were added and the misture was stirred at rt for 1 h. The reaction was quenched with water and the organic layer was separated and dried with sodium sulfate. Removal of solvent gave the crued which was chromatographied on an ISCO machine (0-10% EtOAc/CH₂Cl₂) to give a white solid.

HRMS (ES⁺) m/z Calcd for C₃₂H₃₉Cl₂F₂N₅O₂ + H [(M+H)⁺]: 634.2520, found: 634.2.2522

### Example 98a

### Preparation of intermediate (Z)-3-(5-chloro-2-fluoro-phenyl)-2-(4-chloro-2-fluoro-phenyl)-acrylonitrile

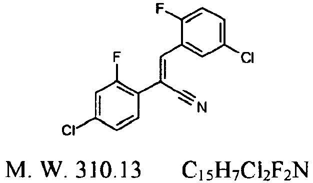

In a manner similar to the method described in **Example 1b**, 4-chloro-2-fluorophenylacetonitrile (2.8 g, 16.6 mmol) was reacted with 5-chloro-2-fluorobenzaldehyde (Alfa) (3.2 g, 19.9 mmol), methanolic solution (25 wt%) of sodium methoxide (4.17 mL, 18 mmol) in methanol (100 mL) at 50°C for 3 h to give (Z)-2-(4-chloro-2-fluoro-phenyl)-3-(5-chloro-2-methoxy-phenyl)-acrylonitrile as a off white solid (3.5 g, 68.6%).

### Example 98b

### Preparation of intermediate rac-(2R,3S,4R,5S)-3-(5-chloro-2-fluoro-phenyl)-4-(4-chloro-2-fluoro-phenyl)-4-cyano-5-(2,2-dimethyl-propyl)-pyrrolidine-2-carboxylic acid tert-butyl ester

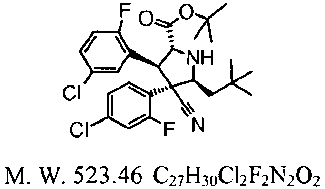

In a manner similar to the method described in **Example 1c**, [3-methyl-but-(E)-ylideneamino]-acetic acid tert-butyl ester prepared in **Example 1a** (2.2 g, 10 mmol) was reacted with (Z)-2-(4-chloro-2-fluoro-phenyl)-3-(5-chloro-2-methoxy-phenyl)-acrylonitrile (2.46 g, 8 mmol) prepared in **Example 98a**, AgF (1.52 g, 12 mmol), and triethylamine (2.78 mL, 20 mmol) in dichloromethane (1500 mL) at room temperature for 18 h to give rac-(2R,3S,4R,5S)-3-(5-chloro-2-fluoro-phenyl)-4-(4-chloro-2-fluoro-phenyl)-4-cyano-5-(2,2-dimethyl-propyl)-pyrrolidine-2-carboxylic acid tert-butyl ester as a white foam (1.1 g, 26%).

### Example 98c

### Preparation of intermediate rac-(2R,3S,4R,5S)-3-(5-chloro-2-fluoro-phenyl)-4-(4-chloro-2-fluoro-phenyl)-4-cyano-5-(2,2-dimethyl-propyl)-pyrrolidine-2-carboxylic acid trifluoroacetic acid

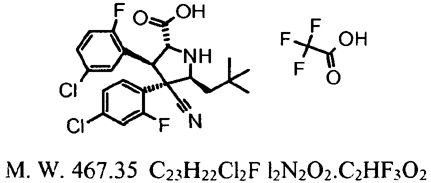

In a manner similar to the method described in **Example 25a**, rac-(2R,3S,4R,5S)-3-(5-chloro-2-fluoro-phenyl)-4-(4-chloro-2-fluoro-phenyl)-4-cyano-5-(2,2-dimethyl-propyl)-pyrrolidine-2-carboxylic acid tert-butyl ester prepared in **Example 98b** (1.1g, 2.1 mmol) was reacted with trifluoroacetic acid in dichloromethane at room temperature to give rac-(2R,3S,4R,5S)-3-(5-chloro-2-fluoro-phenyl)-4-(4-chloro-2-fluoro-phenyl)-4-cyano-5-(2,2-dimethyl-propyl)-pyrrolidine-2-carboxylic acid trifluoroacetic acid as a white solid (1.1g, 90%).

### Example 98d

### Preparation of rac-(2R,3S,4R,5S)-3-(5-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide

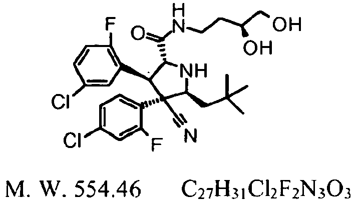

In a manner similar to the method described in **Examples 42c, 42d**, rac-(2R,3S,4R,5S)-3-(5-chloro-2-fluoro-phenyl)-4-(4-chloro-2-fluoro-phenyl)-4-cyano-5-(2,2-dimethyl-propyl)-pyrrolidine-2-carboxylic acid trifluoroacetic acid prepared in **Example 98c** (0.4 g, 0.68 mmol) was reacted with 2-((S)-2,2-dimethyl-[1,3]dioxolan-4-yl)-ethylamine (0.3 g, 2.06 mmol), HATU (0.47 g, 1.24 mmol) and iPr₂NEt (0.6 mL, 3.44 mmol) in CH₂Cl₂ at room temperature for 20 h, then reacted with aqueous HCl solution in tetrahydrofuran at room temperature to give rac-(2R,3S,4R,5S)-3-(5-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide as a white solid (0.36 g, 80%).

HRMS (ES⁺) m/z Calcd for C₂₇H₃₁Cl₂F₂H₃O₃+ H [(M+H)⁺]: 554.1784, found: 554.1782.

### Example 99a

### Preparation of intermediate [3-cyclopropyl-3-methyl-but-(E)-ylideneamino]-acetic acid tert-butyl ester

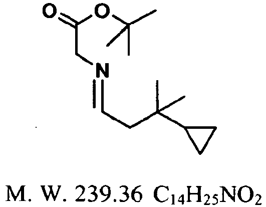

### Step A

To a suspension of CuI (7.61 g, 40 mmol) in anhydrous tetrahydrofuran (100 mL) at -50°C, was added cyclopropylmagnesium bromide (0.5 M, 160 mL, 80 mmol) during a period of 15 min. After the addition was finished, the reaction mixture was gradually warmed room temperature and stirred for 20 min. Then the temperature of the mixture was lowered to -50 °C, a tetrahydrofuran solution (50 mL) of diethyl isopropylidenemalonate (Aldrich) (4 g, 20 mmol) was added. The reaction mixture was allowed to slowly warmed to room temperature and stirred for 3 h. Aqueous saturated NH₄Cl solution was added to quench the reaction. The mixture was filtered, and the filtrate was concentrated to remove most of tetrahydrofuran. The residue was extracted with ethyl acetate twice. The organic layers were combined, concentrated. The residue was purifed by chromatography (EtOAc:hexanes=1;20, 1;10) to give 2-(1-cyclopropyl-1-methylethyl)-malonic acid diethyl ester as a colorless oil (4.3 g, 89%).

### Step B

To a solution of 2-(1-cyclopropyl-1-methyl-ethyl)-malonic acid diethyl ester (4.3 g, 17.8 mmol) in DMSO (30 mL) was added LiCl (1.5 g, 35.6 mmol) and H₂O (0.3 mL, 17.8 mmol). The reaction mixture was heated at 170 °C for 4 h, then poured into a ice-water, extracted with ethyl acetate. The organic layer were separated, washed with water, brine, dried over MgSO₄, and concentrated to give 3-cyclopropyl-3-methyl-butyric acid ethyl ester as a colorless oil (2 g, 66%).

### Step C

To a solution of 3-cyclopropyl-3-methyl-butyric acid ethyl ester (2 g, 11.75 mmol) in anhydrous tetrahydrofuran (40 mL) at 0°C was added a tetrhydrofuran solution (1M) of LiAlH₄ (23.5 mL, 23.5 mmol) under nitrogen. The reaction mixture was stirred at 0 °C for 1 h, then poured into a ice-water. The mixture was extracted with ethyl acetate. The organic layer were separated, washed with water, aqueous HCl solution, brine, dried over MgSO₄, and concentrated. The residue was purified by chromatography (EtOAc:hexanes = 1;10, 1:5) to give 3-cyclopropyl-3-methyl-butan-1-ol as a colorless oil (0.7 g, 46%).

### Step D

To a solution of oxalyl chloride (0.75 g, 5.9 mmol) (Aldrich) in dichloromethane (30 mL) at -78 °C was added the solution of dimethyl sulfoxide (0.84 mL, 11.8 mmol) in dichloromethane (5 mL) dropwise. After 5 mins, the solution of 3-cyclopropyl-3-methyl-butan-1-ol (0.69 g, 5.4 mmol) in dichloromethane (10 mL) was added dropwise. The reaction mixture was stirred at -78 °C for 15 min. Triethylamine (2.7 mL, 19.4 mmol) was added and the reaction mixture was slowly warmed up to room temperature and stirred at room temperature for 45 min. Then water was added. The organic layers were separated, and the aqueous layer was extracted with dichloromethane. The organic layers were combined, washed with 10% of HCl, saturated NaHCO₃, brine, dried over MgSO₄, and concentrated to give 3-cyclopropyl-3-methyl-butyraldehyde as a light yellow oil (Yield: 0.68 g, 98%).

### Step E

In a manner similar to the method described in **Example 1a**, glycine tert-butyl ester (0.65 g, 5 mmol) was reacted with 3-cyclopropyl-3-methyl-butyraldehyde (0.68 g, 5.3 mmol) in CH₂Cl₂ at room temperature for 5 h to give [3-cyclopropyl-3-methyl-but-(E)-ylideneamino]-acetic acid tert-butyl ester as a colorless oil (0.9 g, 75%).

### Example 99b

### Preparation of intermediate rac-(2R,3S,4R,5S)-3-(3-chloro-2-fluoro-phenyl)-4-(4-chloro-2-fluoro-phenyl)-4-cyano-5-(2-cyclopropyl-2-methyl-propyl)-pyrrolidine-2-carboxylic acid methyl ester

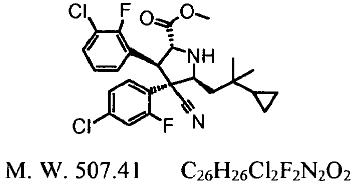

To a solution of [3-cyclopropyl-3-methyl-but-(E)-ylideneamino]-acetic acid tert-butyl ester prepared in **Example 99a** (0.9 g, 3.76 mmol) and (Z)-3-(3-chloro-2-fluoro-phenyl)-2-(4-chloro-2-fluoro-phenyl)-acrylonitrile (1.16 g, 3.76 mmol) prepared in **Example 52a** in dichloromethane (100 mL) were added triethylamine (0.76 g, 7.5 mmol) and AgF (0.47 g, 3.76 mmol), in one portion. The mixture was stirred at room temperature for overnight. The mixture was then quenched with sat. NH₄Cl and extracted with CH₂Cl₂. The organic phase was separated, filtered through celite and dried over Na₂SO₄, and concentrated. The residue was dissolved into methanol (40 mL), and DBU (3 mL) was added. The mixture was heated at 100 °C for 5 h, then cooled to room temperature, and concentrated. The residue was partitioned between ethyl acetate and water. The organic layer were separated, dried over MgSO₄, and concentrated. The residue was purified by chromatography (EtOAc:hexanes = 1;5, 1:3) to give rac-(2R,3S,4R,5S)-3-(3-chloro-2-fluoro-phenyl)-4-(4-chloro-2-fluoro-phenyl)-4-cyano-5-(2-cyclopropyl-2-methylpropyl)-pyrrolidine-2-carboxylic acid methyl ester as a white foam (0.95 g, 50%).

### Example 99c

### Preparation of intermediate rac-(2R,3S,4R,5S)-3-(3-chloro-2-fluoro-phenyl)-4-(4-chloro-2-fluoro-phenyl)-4-cyano-5-(2-cyclopropyl-2-methyl-propyl)-pyrrolidine-2-carboxylic acid

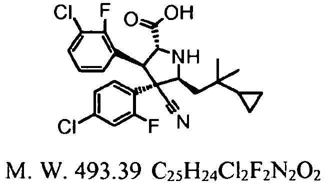

To rac-(2R,3S,4R,5S)-3-(3-chloro-2-fluoro-phenyl)-4-(4-chloro-2-fluoro-phenyl)-4-cyano-5-(2-cyclopropyl-2-methyl-propyl)-pyrrolidine-2-carboxylic acid methyl ester prepared in **Example 99b** (0.95 g, 1.87 mmol) in tetrahydrofuran (40 mL) was added an aqueous solution (20 mL) of NaOH (0.15 g, 3.7mmol) and methanol (20 mL). The reaction mixture was stirred at room temperature for 18 h. The "pH" of the mixture was adjusted to 5 by aqueous HCl solution. The mixture was concentrated. The residue was partitioned between ethyl acetate and water. The organic layer was separated, washed with water, brine, dried over MgSO₄, concentrated, and trituated with dichloromethane and hexanes to give rac-(2R,3S,4R,4S)-3-(3-chloro-2-fluoro-phenyl)-4-(4-chloro-2-fluoro-phenyl)-4-cyano-5-(2-cyclopropyl-2-methyl-propyl)-pyrrolidine-2-carboxylic acid as a white solid (0.78 g, 80%)

### Example 99d

### Preparation of rac-(2R,3S,4R,5S)-3-(3-chloro-2-fluoro-phenyl)-4-(4-chloro-2-fluoro-phenyl)-4-cyano-5-(2-cyclopropyl-2-methyl-propyl)-pyrrolidine-2-carboxylic acid ((S)-3,4-dihydroxybutyl)-amide

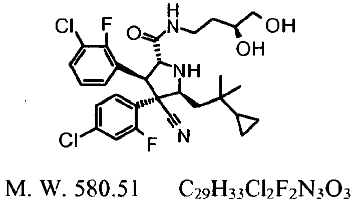

In a manner similar to the method described in **Examples 42c and 42d**, rac-(2R,3S,4R,5S)-3-(3-chloro-2-fluoro-phenyl)-4-(4-chloro-2-fluoro-phenyl)-4-cyano-5-(2-cyclopropyl-2-methylpropyl)-pyrrolidine-2-carboxylic acid prepared in **Example 99c** (0.41 g, 0.83 mmol) was reacted with 2-((S)-2,2-dimethyl-[1,3]dioxolan-4-yl)-ethylamine (0.36 g, 2.5 mmol), HATU (0.57 g, 1.5 mmol) and iPr₂NEt (0.43 mL, 2.5 mmol) in CH₂Cl₂ at room temperature for 20 h, then reacted with aqueous HCl solution in tetrahydrofuran at room temperature for 2 h to give rac-(2R,3S,4R,5S)-3-(3-chloro-2-fluoro-phenyl)-4-(4-chloro-2-fluoro-phenyl)-4-cyano-5-(2-cyclopropyl-2-methyl-propyl)-pyrrolidine-2-carboxylic acid ((S)-3,4-dihydroxy-butyl)-amide as a white solid (0.33 g, 70%).

HRMS (ES⁺) m/z Calcd for C₂₉H₃₃Cl₂F₂N₃O₃+ H [M+H)⁺] 580.1940, found: 580.1936.

### Example 99e

### Preparation of (2R,3S,4R,5S)-3-(3-chloro-2-fluoro-phenyl)-4-(4-chloro-2-fluoro-phenyl)-4-cyano-5-(2-cyclopropyl-2-methyl-propyl)-pyrrolidine-2-carboxylic acid ((S)-3,4-dihydroxybutyl)-amide

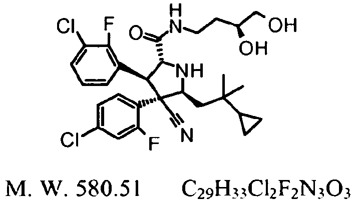

Rac-(2R,3S,4R,5S)-3-(3-chloro-2-fluoro-phenyl)-4-(4-chloro-2-fluoro-phenyl)-4-cyano-5-(2-cyclopropyl-2-methyl-propyl)-pyrrolidine-2-carboxylic acid ((S)-3,4-dihydroxy-butyl)-amide (0.3 g) was separated by chiral SFC chromatography to provide chiral (2R,3S,4R,5S)-3-(3-chloro-2-fluoro-phenyl)-4-(4-chloro-2-fluoro-phenyl)-4-cyano-5-(2-cyclopropyl-2-methylpropyl)-pyrrolidine-2-carboxylic acid ((S)-3,4-dihydroxy-butyl)-amide as a white solid (0.11 g, 37%) and chiral (2S,3R,4S,5R)-3-(3-chloro-2-fluoro-phenyl)-4-(4-chloro-2-fluoro-phenyl)-4-cyano-5-(2-cyclopropyl-2-methyl-propyl)-pyrrolidine-2-carboxylic acid ((S)-3,4-dihydroxybutyl)-amide as a white solid (0.11 g, 37%).

HRMS (ES⁺) m/z Calcd for C₂₉H₃₃Cl₂F₂N₃O₃+ H [(M+H)⁺]: 580.1940, found: 580.1941.

### Example 100a

### Preparation of intermediate (Z)-3-(3-chloro-2-fluoro-phenyl)-2-(4-chloro-3-methyl-phenyl)-acrylonitrile

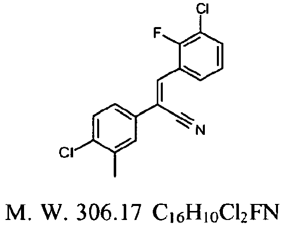

### Step A

In a manner similar to the method described in **Example 57 Step A**, 4-chloro-2-methylbenzyl alcohol (PLATTE) (1.69 g, 11 mmol) was reacted with thionyl chloride (20 mL) to give 4-chloro-2-methylbenzyl chloride as a colorless oil (1.83 g, 97%).

### Step B

### In a manner similar to the method described in Example 57 Step B, 4-chloro-2-methylbenzyl chloride (1.83 g, 10 mmol) was reacted with KCN (1.76 g, 27 mmol) in ethanol (13 mL) and water (10 mL) at 100 °C for 1h to give 4-chloro-2-methylbenzyl cyanide as a yellow oil (1.2 g, 69%)

### Step C

In a manner similar to the method described in **Example 1b**, 4-chloro-2-methylbenzyl cyanide (1.2 g, 7.2 mmol) was reacted with 3-chloro-2-fluorobenzaldehyde (1.38 g, 8.7 mmol), methanolic solution (25 wt%) of sodium methoxide (1.82 mL, 8 mmol) in methanol (50 mL) at 50 °C for 3 h to give (Z)-3-(3-chloro-2-fluoro-phenyl)-2-(4-chloro-3-methyl-phenyl)-acrylonitrile as a white solid (2.0 g, 91%).

### Example 100b

### Preparation of intermediate rac-(2R,3S,4R,5S)-3-(3-chloro-2-fluoro-phenyl)-4-(4-chloro-3-methyl-phenyl)-4-cyano-5-(2,2-dimethyl-propyl)-pyrrolidine-2-carboxylic acid tert-butyl ester

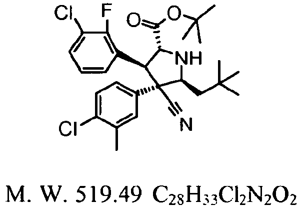

To a solution of [3-cyclopropyl-3-methyl-but-(E)-ylideneamino]-acetic acid tert-butyl ester prepared in **Example 1a** (0.93 g, 7.3 mmol) and To a solution of [3-cyclopropyl-3-methyl-but-(Epylideneamino]-acetic acid tert-butyl ester prepared in **Example 99a** (1.6 g, 7.3 mmol) and (Z)-3-(3-chloro-2-fluoro-phenyl)-2-(4-chloro-2-fluoro-phenyl)-acrylonitrile (1.5 g, 4.9 mmol) prepared in **Example 100a** in dichloromethane (100 mL) were added triethylamine (1.7 g, 12 mmol) and AgF (0.9 g, 7.3mmol), in one portion. The mixture was stirred at room temperature for 18 h. The mixture was then quenched with sat. NH₄Cl and extracted with CH₂Cl₂. The organic phase was separated, filtered through celite and dried over Na₂SO₄, and concentrated. The residue was dissolved into tert-butanol (30 mL), and DBU (5 mL) was added. The mixture was heated at 100°C for 18 h, then cooled to room temperature, and concentrated. The residue was partitioned between ethyl acetate and water. The organic layer were separated, dried over MgSO₄, and concentrated. The residue was purified by chromatography (EtOAc:hexanes = 1;10, 1:5) to give rac-(2R,3S,4R,5S)-3-(3-chloro-2-fluoro-phenyl)-4-(4-chloro-3-methyl-phenyl)-4-cyano-5-(2,2-dimethyl-propyl)-pyrrolidine-2-carboxylic acid tert-butyl ester as a white foam (2 g, 80%).

### Example 100c

### Preparation of intermediate rac-(2R,3S,4R,5S)-3-(3-chloro-2-fluoro-phenyl)-4-(4-chloro-3-methyl-phenyl)-4-cyano-5-(2,2-dimethyl-propyl)-pyrrolidine-2-carboxylic acid trifluoroacetic acid

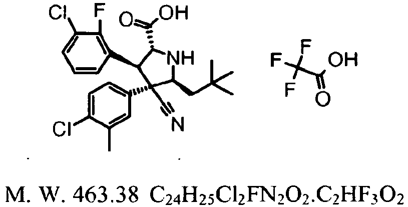

In a manner similar to the method described in **Example 25a**, rac-(2R,3S,4R,5S)-3-(3-chloro-2-fluoro-phenyl)-4-(4-chloro-3-methyl-phenyl)-4-cyano-5-(2,2-dimethyl-propyl)-pyrrolidine-2-carboxylic acid tert-butyl ester prepared in **Example 100b** (2.0 g, 3.8 mmol) was reacted with trifluoroacetic acid in dichloromethane at room temperature to give rac-(2R,3S,4R,5S)-3-(3-chloro-2-fluoro-phenyl)-4-(4-chloro-3-methyl-phenyl)-4-cyano-5-(2,2-dimethyl-propyl)-pyrrolidine-2-carboxylic acid trifluoroacetic acid as a white solid (1.88 g, 85%).

### Example 100d

### Preparation of rac-(2R,3S,4R,5S)-3-(3-chloro-2-fluoro-phenyl)-4-(4-chloro-3-methyl-phenyl)-4-cyano-5-(2,2-dimethyl-propyl)-pyrrolidine-2-carboxylic acid ((S)-3,4-dihydroxy-butyl)-amide

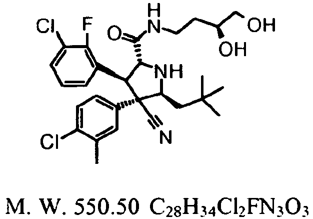

In a manner similar to the method described in **Examples 42c, 42d**, rac-(2R,3S,4R,5S)-3-(3-chloro-2-fluoro-phenyl)-4-(4-chloro-3-methyl-phenyl)-4-cyano-5-(2,2-dimethyl-propyl)-pyrrolidine-2-carboxylic acid trifluoroacetic acid prepared in **Example 100c** (0.44 g, 0.76 mmol) was reacted with 2-((S)-2,2-dimethyl-[1,3]dioxolan-4-yl)-ethylamine (0.33 g, 2.3 mmol), HATU (0.52 g, 1.37 mmol) and iPr₂NEt (0.66 mL, 3.8 mmol) in CH₂Cl₂ at room temperature for 20 h, then reacted with aqueous HCl solution in tetrahydrofuran at room temperature for 2 h to give rac-(2R,3S,4R,5S)-3-(3-chloro-2-fluoro-phenyl)-4-(4-chloro-3-methyl-phenyl)-4-cyano-5-(2,2-dimethyl-propyl)-pyrrolidine-2-carboxylic acid ((S)-3,4-dihydroxy-butyl)-amide as a white solid (0.26 g, 64%).

HRMS (ES⁺) m/z Calcd for C₂₈H₃₄Cl₂FN₃O₃+ H [(M+H)⁺]: 530.2034, found; 550.2034.

### Example 101a

### Preparation of intermediate [2-(tetrahydro-pyran-4-yl)-eth-(E)-ylideneamino]-acetic acid tert-butyl ester

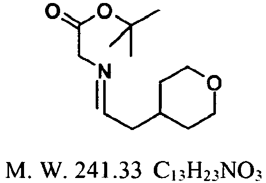

In a manner similar to the method described in **Example 1a**, glycine tert-butyl ester (0.65 g, 5 mmol) was reacted with (tetrahydro-pyran-4-yl)-acetaldehyde (Pharmacore) (0.64 g, 5 mmol) in CH₂Cl₂ at room temperature for 5 h to give [2-(tetrahydro-pyran-4-yl)-eth-(E)-ylideneamino]-acetic acid tert-butyl ester
as a colorless oil (0.43 g, 33%).

### Example 101b

### Preparation of intermediate rac-(2R,3S,4R,5S)-3-(3-chloro-2-fluoro-phenyl)-4-(4-chloro-2-fluoro-phenyl)-4-cyano-5-(tetrahydro-pyran-4-ylmethyl)-pyrrolidine-2-carboxylic acid tert-butyl ester

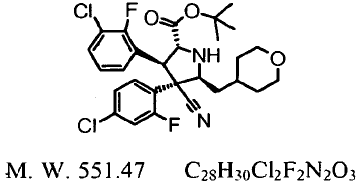

In a manner similar to the method described in **Example 100b**, [2-(tetrahydro-pyran-4-yl)-eth-(E)-ylideneamino]-acetic acid tert-butyl ester prepared in **Example 100a** (0.43 g, 1.8 mmol) was reacted with (Z)-3-(3-chloro-2-fluoro-phenyl)-2-(4-chloro-2-fluoro-phenyl)-acrylonitrile (0.31 g, 1 mmol) prepared in **Example 52a**, AgF (0.19 g, 1.5 mmol), and triethylamine (0.46 g, 4.5 mmol) in dichloromethane (50 mL) at room temperature for 18 h, followed by reaction with DBU in tert-butanol at 100°C for 8 h to give rac-(2R,3S,4R,5S)-3-(3-chloro-2-fluoro-phenyl)-4-(4-chloro-2-fluoro-phenyl)-4-cyano-5-(tetrahydro-pyran-4-ylmethyl)-pyrrolidine-2-carboxylic acid tert-butyl ester as a white solid (0.45 g, 82%).

### Example 101c

### Preparation of intermediate rac-(2R,3S,4R,5S)-3-(3-chloro-2-fluoro-phenyl)-4-(4-chloro-2-fluoro-phenyl)-4-cyano-5-(tetrahydro-pyran-4-ylmethyl)-pyrrolidine-2-carboxylic acid trifluoroacetic acid

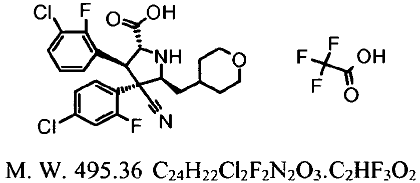

In a manner similar to the method described in **Example 25a**, rac-(2R,3S,4R,5S)-3-(3-chloro-2-fluoro-phenyl)-4-(4-chloro-2-fluoro-phenyl)-4-cyano-5-(tetrahydro-pyran-4-ylmethyl)-pyrrolidine-2-carboxylic acid tert-butyl ester prepared in **Example 101b** (0.45 g, 0.81 mmol) was reacted with trifluoroacetic acid in dichloromethane at room temperature to give rac-(2R,3S,4R,5S)-3-(3-chloro-2-fluoro-phenyl)-4-(4-chloro-2-fluoro-phenyl)-4-cyano-5-(tetrahydro-pyran-4-ylmethyl)-pyrrolidine-2-carboxylic acid trifluoroacetic acid as a white solid (0.4 g, 80%).

### Example 101d

### Preparation of rac-(2R,3S,4R,5S)-3-(3-chloro-2-fluoro-phenyl)-4-(4-chloro-2-fluoro-phenyl)-4-cyano-5-(tetrahydro-pyran-4-ylmethyl)-pyrrolidine-2-carboxylic acid ((S)-3,4-dihydroxy-butyl)-amide

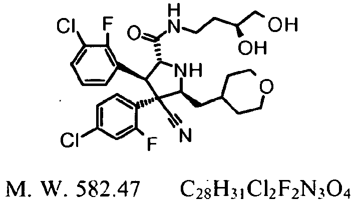

In a manner similar to the method described in **Examples 42c and 42d**, rac-(2R,3S,4R,5S)-3-(3-chloro-2-fluoro-phenyl)-4-(4-chloro-2-fluoro-phenyl)-4-cyano-5-(tetrahydro-pyran-4-ylmethyl)-pyrrolidine-2-carboxylic acid trifluoroacetic acid prepared in **Example 101c** (0.15 g, 0.25 mmol) was reacted with 2-((S)-2,2-dimethyl-[1,3]dioxolan-4-yl)-ethylamine (0.11 g, 0.74 mmol), HATU (0.17 g, 0.44 mmol) and iPr₂NEt (0.21 mL, 1.2 mmol) in CH₂Cl₂ at room temperature for 20 h, then reacted with aqueous HCl solution in tetrahydrofuran at room temperature for 2 h to give rac-(2R,3S,4R,5S)-3-(3-chloro-2-fluoro-phenyl)-4-(4-chloro-2-fluoro-phenyl)-4-cyano-5-(tetrahydro-pyran-4-ylmethyl)-pyrrolidine-2-carboxylic acid ((S)-3,4-dihydroxy-butyl)-amide as a white solid (0.25 g, 80%).

HRMS (ES⁺) m/z Calcd for C₂₈H₃₁Cl₃F₂N₃O₄+ H [(M+H)⁺]: 582. 1733, found: 582.1731.

### Example 102a

### Preparation of intermediate [2-(1-methyl-cyclohexyl)-eth-(E)-ylideneamino]-acetic acid tert-butyl ester

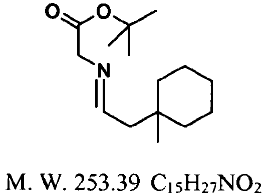

### Step A.

To a suspension ofNaH (60%, 3 g, 74 mmol) in DME (100 mL) was added methyl (dimethoxyphosphonyl)acetate (Aldrich) (11.3 g, 61.8 mmol). The mixture was stirred at room temperature for 40 min, then cyclohexanone (6.07 g, 61.8 mmol) was added. The reaction mixture was stirred at room temperature for 18 h. Aqueous saturated NH₄Cl solution was added and the mixture was extracted with ethyl acetate twice. The organic layers were combined, dried over MgSO₄, concentrated. The residue was purifed by chromatography (EtOAc:hexanes=1;10) to give cyclohexylidene-acetic acid methyl ester as a colorless oil (6.4 g, 67%).

Similar transformation was reported by Bruckner, R. et al in Eur. J. Org. Chem. 2006, 2119-2133 and the described procedures were used without modification.

### Step B

To a suspension of CuI (7.61 g, 40 mmol) in anhydrous ethyl ether (20 mL) at 0 °C was added an ethyl ether solution (1.6 M) ofMeLi (50 mL, 80 mmol) The reaction mixture was stirred at 0 °C for 10 min. The solvent was evaporated under reduced pressure, then dichloromethane (20 mL) was added under nitrogen at 0 °C. The mixture was stirred for 5 min. The solvent was evaporated again. To the residue was added dichlormethane (20 mL), and the temperature of the mixture was lowered to -78 °C. To the mixture was added chlorotrimethylsilane (4.3 g, 40 mmol) and a dichloromethane solution (20 mL) of cyclohexylidene-acetic acid methyl ester (3.1 g, 20 mmol). The reaction mixture was allowed to slowly warmed to 0 °C and stirred for 1h. Aqueous saturated NH₄Cl solution was added to quench the reaction. The mixture was extracted with ethyl ether twice. The organic layers were combined, dried over MgSO₄, concentrated. The residue was purifed by chromatography (EtOAc:hexanes=1;5) to give (1-methyl-cyclohexyl)-acetic acid methyl ester as a colorless oil (3.3 g, 97%).

Similar transformation was reported by Yamamoto, Y. et al in *Tetrahedron Letter* 44 (**2003**), 4265-4266 and the described procedures were used without modification.

### Step C

To a solution of (1-methyl-cyclohexyl)-acetic acid methyl ester (3.3 g, 19.4 mmol) in anhydrous tetrahydrofuran (50 mL) at 0 °C was added a tetrhydrofuran solution (1 M) of LiAlHa (29 mL, 29 mmol) under nitrogen. The reaction mixture was stirred at 0 °C for 1 h, then poured into a ice-water. The mixture was extracted with ethyl acetate. The organic layer were separated, washed with water, aqueous HCl solution, brine, dried over MgSO₄, and concentrated. The residue was purified by chromatography (EtOAc:hexanes = 1:4) to give 2-(1-methyl-cyclohexyl)-ethanol as a colorless oil (2.2 g, 80%).

### Step D

To a solution ofoxalyl chloride (2.18 g, 17.2 mmol) (Aldrich) in dichloromethane (12 mL) at -78 °C was added the solution of dimethyl sulfoxide (2.44 mL, 34.3 mmol) in dichloromethane (8 mL) dropwise. After 5 mins, the solution of 2-(1-methyl-cyclohexyl)-ethanol (2.2 g, 15.6 mmol) in dichloromethane (10 mL) was added dropwise. The reaction mixture was stirred at -78 °C for 15 min. Triethylamine (7.8 mL, 56mmol) was added and the reaction mixture was slowly warmed up to room temperature and stirred at room temperature for 45 min. Then water was added. The organic layers were separated, and the aqueous layer was extracted with dichloromethane. The organic layers were combined, washed with 10% of HCI, saturated NaHCO₃, brine, dried over MgSO₄, and concentrated to give (1-methyl-cyclohexyl)-acetaldehyde as a light yellow oil (Yield: 2 g, 91%).

### Step E

In a manner similar to the method described in **Example 1a**, glycine tert-butyl ester (1.87 g, 14.3 mmol) was reacted with (1-methyl-cyclohexyl)-acetaldehyde (2.9 g, 14.3 mmol) in CH₂Cl₂ at room temperature for 18 h to give [2-(1-methyl-cyclohexyl)-eth-(E)-ylideneamino]-acetic acid tert-butyl ester
as a colorless oil (3.4 g, 95%).

### Example 102b

### Preparation of intermediate rac-(2R,3S,4R,5S)-3-(3-chloro-2-fluoro-phenyl)-4-(4-chloro-2-fluoro-phenyl)-4-cyano-5-(1-methyl-cyclohexylmethyl)-pyrrolidine-2-carboxylic acid tert-butyl ester

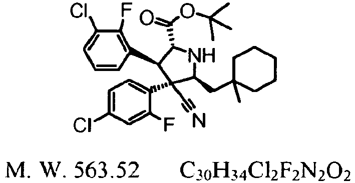

In a manner similar to the method described in **Example 100b**, [2-(1-methyl-cyclohexyl)-eth-(E)-ylideneamino]-acetic acid tert-butyl ester prepared in **Example 101a** (2.55 g, 10 mmol) was reacted with (Z)-3-(3-chloro-2-fluoro-phenyl)-2-(4-chloro-2-fluoro-phenyl)-acrylonitrile (2.5 g, 8 mmol) prepared in **Example 52a**, AgF (1.55 g, 12.3 mmol), and triethylamine (2.8 mL, 20 mmol) in dichloromethane (150 mL) at room temperature for 18 h, followed by the reaction with DBU (10 ml) in tert-butanol (50 mL) at 100 °C for 18 h to give rac-(2R,3S,4R,5S)-3-(3-chloro-2-fluoro-phenyl)-4-(4-chloro-2-fluoro-phenyl)-4-cyano-5-(1-methyl-cyclohexylmethyl)-pyrrolidine-2-carboxylic acid tert-butyl ester as a light yellow solid (3 g, 66%).

### Example 102c

### Preparation of intermediate rac-(2R,3S,4R,5S)-3-(3-chloro-2-fluoro-phenyl)-4-(4-chloro-2-fluoro-phenyl)-4-cyano-5-(1-methyl-cyclohexylmethyl)-pyrrolidine-2-carboxylic acid trifluoroacetic acid

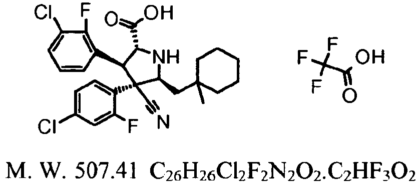

In a manner similar to the method described in **Example 25a**, rac-(2R,3S,4R,5S)-3-(3-chloro-2-fluoro-phenyl)-4-(4-chloro-2-fluoro-phenyl)-4-cyano-5-(1-methyl-cyclohexylmethyl)-pyrrolidine-2-carboxylic acid tert-butyl ester prepared in **Example 102b** (3 g, 5.3 mmol) was reached with trifluoroacetic acid in dichloromethane at room temperature to give rac-(2R,3S,4R,5S)-3-(3-chloro-2-fluoro-phenyl)-4-(4-chloro-2-fluoro-phenyl)-4-cyano-5-(1-methylcyclohexylmethyl)-pyrrolidine-2-carboxylic acid trifluoroacetic acid as a off white solid (3.3 g, 100%).

### Example 102d

### Preparation of rac-(2R,3S,4R,5S)-3-(3-chloro-2-fluoro-phenyl)-4-(4-chloro-2-fluoro-phenyl)-4-cyano-5-(1-methyl-cyclohexylmethyl)-pyrolidine-2-carboxylic acid ((S)-3,4-dihydroxy-butyl)-amide

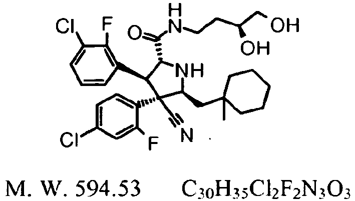

In a manner similar to the method described in **Examples 42c and 42d**, rac-(2R,3S,4R,SS)-3-(3-chloro-2-fluoro-phenyl)-4-(4-chloro-2-fluoro-phenyl)-4-cyano-5-(1-methyl-cyclohexylmethyl)-pyrrolidine-2-carboxylic acid trifluoroacetic acid prepared in **Example 102c** (0.5 g, 0.8 mmol) was reacted with 2-((S)-2,2-dimethyl-[1,3]dioxolan-4-yl)-ethylamine (0.35 g, 2.41 mmol), HATU (0.55 g, 1.45 mmol) and iPr₂NEt (0.70 mL, 4.0 mmol) in CH₂Cl₂ at room temperature for 20 h, then reacted with aqueous HCl solution in tetrahydrofuran at room temperature for 2 h to give rac-(2R,3S,4R,8S)-3-(3-chloro-2-fluoro-phenyl)-4-(4-chloro-2-fluoro-phenyl)-4-cyano-5-(1-methyl-cyclohexylmethyl)-pyrrolidine-2-carboxylic acid ((S)-3,4-dihydroxy-butyl)-amide as a white solid (0.22 g, 46%).

HRMS (ES⁺) m/z Called for C₃₀H₃₅Cl₂F₂N₃O₃+ H [(M+H)⁺]: 594.2097, found: 594.2094.

### Example 102e

### Preparation of (2R,3S,4R,5S)-3-(3-chloro-2-fluoro-phenyl)-4-(4-chloro-2-fluoro-phenyl)-4-cyano-5-(1-methyl-cyclohexylmethyl)-pyrrolidine-2-carboxylic acid ((S)-3,4-dihydroxy-butyl)-amide

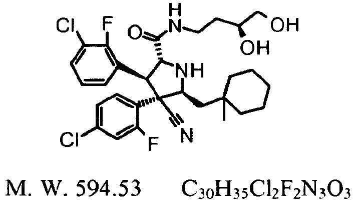

Rac-(2R,3S,4R,5S)- 3-(3-chloro-2-fluoro-phenyl)-4-(4-chloro-2-fluoro-phenyl)-4-cyano-5-(1-methyl-cyclohexylmethyl)-pyrrolidine-2-carboxylic acid ((S)-3,4-dihydroxy-butyl)-amide (0.18 g) was separated by chiral SFC chromatography to provide chiral (2R,3S,4R,5S)- 3-(3-chloro-2-fluoro-phenyl)-4-(4-chloro-2-fluoro-phenyl)-4-cyano-5-(1-methyl-cyclohexylmethyl)-pyrrolidine-2-carboxylic acid ((S)-3,4-dihydroxy-butyl)-amide as a white solid (78 mg, 71%) and chiral (2S,3R,4S,5R)-3-(3-chloro-2-fluoro-phenyl)-4-(4-chloro-2-fluoro-phenyl)-4-cyano-5-(1-methyl-cyclohexylmethyl)-pyrrolidine-2-carboxylic acid ((S)-3,4-dihydroxy-butyl)-amide as a white solid (80 mg, 73%).

HRMS (ES⁺) m/z Calcd for C₃₀H₃₅Cl₂F₂N₃O₃+ H [(M+H)⁺]: 594.2097, found: 594.2094.

### Example 103a

### Preparation of intermediate 4-[(E)-tert-butoxycarbonylmethylimino]-2,2-dimethyl-butyric acid benzyl ester

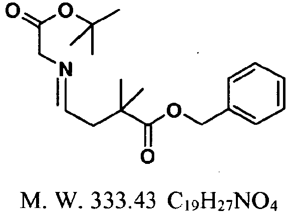

### Step A

A mixture of 2,2-dimethylbutyrolactone (6.84 g, 60 mmol) and KOH (3.36 mmol) in H₂O (60 mL) was heated at reflux for 2 h. The solution was cooled to room temperature and concentrated to dryness to give 4-hydroxy-2,2-dimethyl-butanoic acid monopotassium salt as a white solid (10.2 g, 100%).

### Step B

To the mixture of 4-hydroxy-2,2-dimethyl-butanoic acid monopotassium salt (60 mmol) and benzyl bromide (8.55 mL, 72 mmol) were added Nal (10.8 g, 72 mmol) and K₂CO₃ (8.29 g, 60 mmol). The reaction mixture was stirred at reflux for 18 h. The precipitate was filtered off and the filtrate was evaporated. The residue was purified by flash column chromatography (EtOAc: hexanes=1:2) to give 4-hydroxy-2,2-dimethyl-butyric acid benzyl ester as a colorless oil (9 g, 67%)

The same transformations were reported in EP246529 and the described procedures were used without modification.

### Step C

To a solution of oxalyl chloride (2.8 mL, 22 mmol) (Aldrich) in dichloromethane (40 mL) at -78 °C was added the solution of dimethyl sulfoxide (3.1 mL, 44 mmol) in dichloromethane (5 mL) dropwise. After 5 mins, the solution of 4-hydroxy-2,2-dimethyl-butyric acid benzyl ester (4.5 g, 20 mmol) in dichloromethane (5 mL) was added dropwise. The reaction mixture was stirred at - 78 °C for 15 min. Triethylamine (10 mL, 72 mmol) was added and the reaction mixture was slowly warmed up to room temperature and stirred at room temperature for 45 min. Then water was added. The organic layers were separated, and the aqueous layer was extracted with dichloromethane. The organic layers were combined, washed with 10% of HCl, saturated NaHCO₃, brine, dried over MgSO₄, and concentrated to give 2,2-dimethyl-4-oxo-butyric acid benzyl ester as a colorless oil (Yield: 2.9 g, 66%).

### Step D

In a manner similar to the method described in **Example 1a**, glycine tert-butyl ester (1.72 g, 13.2 mmol) was reacted with 2,2-dimethyl-4-oxo-butyric acid benzyl ester (4.1 g, 13.2 mmol) in CH₂Cl₂ at room temperature for 18 h to give 4-[(E)-tert-butoxyearbonylmethylimino]-2,2-dimethyl-butyric acid benzyl ester as a colorless oil (4.1 g, 93%).

### Example 103b

### Preparation of intermediate rac-(2R,3S,4R,5S)-5-(2-benzyloxycarbonyl-2-methyl-propyl)-3-(3-chloro-2-fluoro-phenyl)-4-(4-chloro-2-fluoro-phenyl)-4-cyano-pyrrolidine-2-carboxylic acid tert-butyl ester

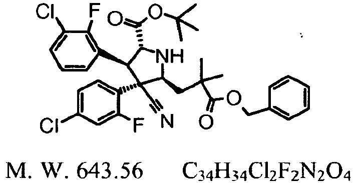

In a manner similar to the method described in **Example 100b**, 4-[(E)-tert-butoxycarbonylmethylimino]-2,2-dimethyl-butyric acid benzyl ester prepared in **Example 103a** (4.1 g, 12.3 mmol) was reacted with (Z)-3-(3-chloro-2-fluoro-phenyl)-2-(4-chloro-2-fluoro-phenyl)-acrylonitrile (2.5 g, 8 mmol) prepared in **Example 52a**, AgF (1.55 g, 12.3 mmol), and triethylamine (2.5 g, 24 mmol) in dichloromethane (150 mL) at room temperature for 18 h, followed by the reaction with DBU (4 ml) in tert-butanol (40 mL) at 100 °C for 4 h to give rac-(2R,3S,4R,5S)-5-(2-benzyloxycarbonyl-2-methyl-propyl)-3-(3-chloro-2-fluoro-phenyl)-4-(4-chloro-2-fluoro-phenyl)-4-cyano-pyrrolidine-2-carboxylic acid tert-butyl ester as a light yellow gum (0.98 g, 19%).

### Example 103c

### Preparation of intermediate rac-(2R,3S,4R,5S)-5-(2-benzyloxycarbonyl-2-methyl-propyl)-3-(3-chloro-2-fluoro-phenyl)-4-(4-chloro-2-fluoro-phenyl)-4-cyano-pyrrolidine-2-carboxylic acid trifluoroacetic acid

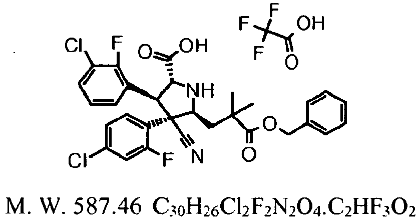

In a manner similar to the method described in **Example 25a**, rac-(2R,3S,4R,5S)-5-(2-benzyloxycarbonyl-2-methyl-propyl)-3-(3-chloro-2-fluoro-phenyl)-4-(4-chloro-2-fluoro-phenyl)-4-cyano-pyrrolidine-2-carboxylic acid tert-butyl ester prepared in **Example 103b** (0.98 g, 1.6 mmol) was reacted with trifluoroacetic acid in dichloromethane at room temperature to give rac-(2R,3S,4R,5S)-5-(2-benzyloxycarbonyl-2-methyl-propyl)-3-(3-chloro-2-fluoro-phenyl)-4-(4-chloro-2-fluoro-phenyl)-4-cyano-pyrrolidine-2-carboxylic acid trifluoroacetic acid as a white gum (0.94 g, 86%).

### Example 103d

### Preparation of rac-(2R,35,4R,5S)-5-(2-benzyloxycarbonyl-2-methyl-propyl)-3-(3-chloro-2-fluoro-phenyl)-4-(4-chloro-2-fluoro-phenyl)-4-cyano-pyrrolidine-2-carboxylic acid ((S)-3,4-dihydroxy-butyl)-amide

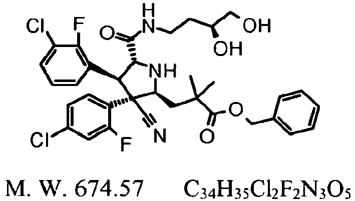

In a manner similar to the method described in **Examples 42c and 42d**, rac-(2R,3S,4R,5S)-5-(2-benzyloxycarbonyl-2-methyl-propyl)-3-(3-chloro-2-fluoro-phenyl)-4-(4-chloro-2-fluoro-phenyl)-4-cyano-pyrrolidine-2-carboxylic acid trifluoroacetic acid prepared in **Example 103c** (0.94 g, 1.34 mmol) was reacted with 2-((S)-2,2-dimethyl-[1,3]dioxolan-4-yl)-ethylamine (0.58 g, 4.0 mmol), HATU (0.92 g, 2.41 mmol) and iPr₂NEt (1.17 mL, 6.7 mmol) in CH₂Cl₂ at room temperature for 20 h, then reacted with aqueous HCl solution in tetrahydrofuran at room temperature for 2 h to give rac-(2R,3S,4R,5S)-5-(2-benzyloxycarbonyl-2-methyl-propyl)-3-(3-chloro-2-fluoro-phenyl)-4-(4-chloro-2-fluoro-phenyl)-4-cyano-pyrrolidine-2-carboxylic acid ((S)-3,4-dihydroxy-butyl)-amide as a white solid (0.2 g, 22%).

HRMS (ES⁺) m/z Calcd for C₃₄H₃₅Cl₂F₂N₃O₅+ H [(M+H⁺]: 674.1995, found: 674.1991.

### Example 104a

### Preparation of intermediate 4-(E)-tert-butoxycarbonylmethylimino]-2,2-dimethyl-butyric acid methyl ester

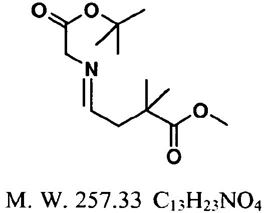

### Step A

A mixture of 2,2-dimethylbutyrolactone (6.84 g, 60 mmol) and KOH (3.36 g) in H₂O (60 mL) was heated at reflux for 2 h. The solution was cooled to room temperature, and acidified to "pH" 5 with aqueous HCl solution. The mixture was then extracted with ethyl acetate three times. The combined organic layers wer washed with brine, dried over MgSO₄, concentrated under reduced pressure to give 4-hydroxy-2,2-dimethyl-butanoic acid as a colorless oil (4 g, 51%).

### Step B

To the mixture of 4-hydroxy-2,2-dimethyl-butanoic acid (2.2 g, 16.6 mmol) in ethyl ether (16 mL) and methanol (24 mL) at 0°C was added a hexane solution (2.0 M) of trimethylsilyldiazomethane (Aldrich) (12.5 mL, 25mmol). The reaction mixture was stirred at 0 °C for 1h. The solvents were evaporated. The residue was taken up in ethyl acetate, washed with diluted aqueous HCl solution, saturated aqueous NaHCO₃ solution, brine, dried over MgSO₄, and concentrated to give 4-hydroxy-2,2-dimethyl-butyric acid methyl ester as a colorless oil (1.5g, 62%).

### Step C

To a solution of oxalyl chloride (1.09 mL, 12.5 mmol) (Aldrich) in dichloromethane (20 mL) at - 78 °C was added the solution of dimethyl sulfoxide (1.77 mL, 25 mmol) in dichloromethane (5 mL) dropwise. After 5 mins, the solution of 4-hydroxy-2,2-dimethyl-butyric acid methyl ester (1.5 g, 11.3 mmol) in dichloromethane (5 mL) was added dropwise. The reaction mixture was stirred at -78 °C for 15 min. Triethylamine (5.7 mL, 41 mmol) was added and the reaction mixture was slowly warmed up to room temperature and stirred at room temperature for 45 min. Then water was added. The organic layers were separated, and the aqueous layer was extracted with dichloromethane. The organic layers were combined, washed with 10% of HCl, saturated NaHCO₃, brine, dried over MgSO₄, and concentrated to give 2,2-dimethyl-4-oxo-butyric acid methyl ester as a light yellow oil (Yield: 1.2 g, 81%).

### Step D

In a manner similar to the method described in **Example 1a**, glycine tert-butyl ester (1.09 g, 8.32 mmol) was reacted with 2,2-dimethyl-4-oxo-butyric acid methyl ester (1.2 g, 8.32 mmol) in CH₂Cl₂ at room temperature for 18 h to give 4-[(E)-tert-butoxycarbonylmethylimino]-2,2-dimethyl-butyric acid methyl ester as a colorless oil (2.1 g, 100%).

### Example 104b

### Preparation of intermediate rac-(2R,3S,4R,5S)-3-(3-chloro-2-fluoro-phenyl)-4-(4-chloro-2-fluoro-phenyl)-4-cyano-5-(2-methoxycarbonyl-2-methyl-propyl)-pyrrolidine-2-carboxylic acid tert-butyl ester

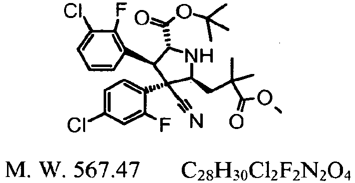

In a manner similar to the method described in **Example 100b**, 4-[(E)-tert-butoxycarbonylmethylimino]-2,2-dimethyl-butyric acid methyl ester prepared in **Example 104a** (2.1 g, 8.3 mmol) was reacted with (Z)-3-(3-chloro-2-fluoro-phenyl)-2-(4-chloro-2-fluoro-phenyl)-acrylonitrile (2.05 g, 6.7 mmol) prepared in **Example 52a**, AgF (1.27 g, 10 mmol), and triethylamine (2.3 mL, 17 mmol) in dichloromethane (150 mL) at room temperature for 18h, followed by the reaction with DBU (2 ml) in tert-butanol (10 mL) at 100°C for 2 h to give rac-(2R,3S,4R,5S)-3-(3-chloro-2-fluoro-phenyl)-4-(4-chloro-2-fluoro-phenyl)-4-cyano-5-(2-methoxycarbonyl-2-methyl-propyl)-pyrrolidine-2-carboxylic acid tert-butyl ester as a white solid (0.75 g, 20%).

### Example 104c

### Preparation of intermediate rac-(2R,3S,4R,5S)-3-(3-chloro-2-fluoro-phenyl)-4-(4-chloro-2-fluoro-phenyl)-4-cyano-5-(2-methoxycarbonyl-2-methyl-propyl)-pyrrolidine-2-carboxylic acid trifluoroacetic acid

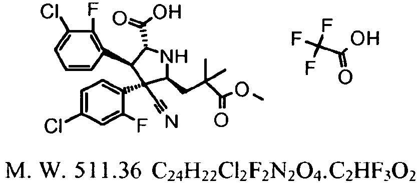

In a manner similar to the method described in **Example 25a,** rac-(2R,3S,4R,5S)-3-(3-chloro-2-fluoro-phenyl)-4-(4-chloro-2-fluoro-phenyl)-4-cyano-5-(2-methoxycarbonyl-2-methyl-propyl)-pyrrolidine-2-carboxylic acid tert-butyl ester prepared in **Example 104b** (0.7 g, 1.23 mmol) was reacted with trifluoroacetic acid in dichloromethane at room temperature to give rac-(2R,3S,4R,5S)-3-(3-chloro-2-fluoro-phenyl)-4-(4-chloro-2-fluoro-phenyl)-4-cyano-5-(2-methoxycarbonyl-2-methyl-propyl)-pyrrolidine-2-carboxylic acid trifluoroacetic acid as a white solid (0.75 g, 97%).

### Example 104d

### Preparation of rac-(2R,3S,4R,5S)-3-(3-chloro-2-fluoro-phenyl)-4-(4-chloro-2-fluoro-phenyl)-4-cyano-5-(2-methoxycarbonyl-2-methyl-propyl)-pyrrolidine-2-carboxylic acid ((S)-3,4-dihydroxy-butyl)-amide

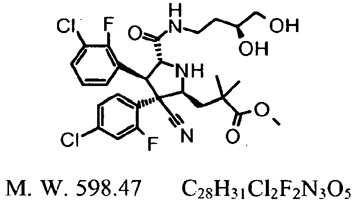

In a manner similar to the method described in **Examples 42c and 42d**, rac-(2R,3S,4R,5S)-3-(3-chloro-2-fluoro-phenyl)-4-(4-chloro-2-fluoro-phenyl)-4-cyano-5-(2-methyl-2-phenoxycarbonyl-propyl)-pyrrolidine-2-carboxylic acid trifluoroacetic acid prepared in **Example 104c** (0.75 g, 1.26 mmol) was reacted with 2-((S)-2,2-dimethyl-[1,3]dioxolan-4-yl)-ethylamine (0.52 g, 3.6 mmol), HATU (0.82 g, 2.16 mmol) and iPr₂NEt (1.04 mL, 6 mmol) in CH₂Cl₂ at room temperature for 20 h, then reacted with aqueous HC solution in tetrahydrofuran at room temperature for 2 h to give rac-(2R,3S,4R,5S)-3-(3-chloro-2-fluoro-phenyl)-4-(4-chloro-2-fluoro-phenyl)-4-cyano-5-(2-methyl-2-phenoxycarbonyl-propyl)-pyrrolidine-2-carboxylic acid ((S)-3,4-dihydroxy-butyl)-amide as a white solid (0.45 g, 56%).

HRMS (ES⁺) m/z Calcd for C₂₈H₃₁Cl₂F₂N₃O_{S}+H[(M+H)⁺]: 598.1682, found: 598.1679_{.}

### Example 105a

### Preparation of intermediate [4-(tert-butyl-dimethyl-silanyloxy)-3,3-dimethyl-but-(E)-ylideneamino]-acetic acid tert-butyl ester

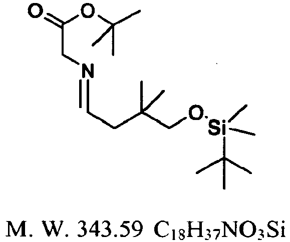

### Step A

A mixture of 2,2-dimethyl-propane-1,3-diol (Aldrich) (10 g, 96 mmol) and imidazole (9.8 g, 140 mmol) in dichloromethane (200 mL) was added tert-butyldimethylchlorosilane (15.9 g, 10.6 mmol). The reactiom mixture was stirred at room temperature for 0.5 h. Water was added. The organic layer was separated, the aqueous layer was then extracted with dichloromethane. The combined organic layers wer washed with brine, dried over MgSO₄, concentrated to give 3-(tert-butyl-dimethyl-silanyloxy)-2,2-dimethyl-propan-1-ol as a colorless oil (20.4 g, 97%).

### Step B

To the solution of 3-(tert-butyl-dimethyl-silanyloxy)-2,2-dimethyl-propan-1-ol (20.4 g, 93 mmol) and triethylamine (26 g, 186 mmol) in dichloromethane (200 mL) at 0 °C was added a dichlormethane solution (20 mL) of methanesulfonyl chloride (Aldrich) (8.69 mL, 112 mmol). The reaction mixture was stirred at 0 °C for 2 h. Water was added. Organic layer was separated, the aqueous layer was extracted with dichlormethane. The combined organic layers were washed with diluted aqueous HCl solution, saturated aqueous NaHCO₃ solution, brine, dried over MgSO₄, and concentrated to give methanesulfonic acid 3-(tert-butyl-dimethyl-silanyloxy)-2,2-dimethyl-propyl ester as a yellow oil (24 g, 87%).

### Step C

To the solution of methanesulfonic acid 3-(tert-butyl-dimethyl-silanyloxy)-2,2-dimethyl-propyl ester (5 g, 16.8 mmol) in anhydrous dimethyl sulfoxide (50 mL) was added KCN (2.85 g, 44 mmol). The reaction mixture was heated at 120 °C for 16 h. The mixture was cooled, and water was added. The mixture was extracted with ethyl acetate twice. The combined organic layers were washed with saturated aqueous NaHCO₃ solution, brine, dried over MgSO_{4,} and concentrated. The residue was purified by chromatography (EtOAc:hexanes = 1;4) to give 4-(tert-butyl-dimethyl-silanyloxy)-3,3-dimethyl-butyronitrile as a yellow oil (2.2 g, 57%).

### Step D

To a solution of 4-(tert-butyl-dimethyl-silanyloxy)-3,3-dimethyl-butyronitrile (2.2 g, 9.67 mmol) (Aldrich) in dichloromethane (20 mL) at -78 °C was added a toluene solution (1M) of DIBAL (10.6 mL, 10.6 mmol) dropwise. The reaction mixture was stirred at 0 °C for 3 h. The mixture was poured into aqueous saturated NH₄Cl solution. The organic layer was separated, and the aqueous layer was extracted with ethyl acetate. The organic layers were combined, washed with brine, dried over MgSO₄, and concentrated. The residue was purifed by chromatography (EtOAc:hexanes=1:4) to give 4-(tert-butyl-dimethyl-silanyloxy)-3,3-dimethyl-butyraldehyde as a colorless oil (Yield: 0.84 g, 38%).

### Step E

In a manner similar to the method described in **Example 1a**, glycine tert-butyl ester (0.52 g, 3.64 mmol) was reacted with 4-(tert-butyl-dimethyl-silanyloxy)-3,3-dimethyl-butyraldehyde (0.84 g, 3.64 mmol) in CH₂Cl₂ at room temperature for 18 h to give [4-(tert-butyl-dimethyl-silanytoxy)-3,3-dimethyl-but-(E)-ylideneamino]-acetic acid tert-butyl ester as a colorless oil (1.25 g, 100%).

### Example 105b

### Preparation of intermediate rac-(2R,3S,4R,5S)-5-[3-(tert-butyl-dimethyl-silanyloxy)-2,2-dimethyl-propyl]-3-(3-chloro-2-fluoro-phenyl)-4-(4-chloro-2-fluoro-phenyl)-4-cyanopyrrolidine-2-carboxylic acid tert-butyl ester

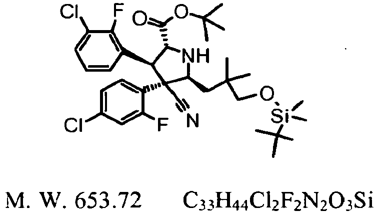

In a manner similar to the method described in **Example 100b**, [4-(tert-butyl-dimethyl-silanyloxy)-3,3-dimethyl-but-(E)-ylideneamino]-acetic acid tert-butyl ester prepared in **Example 105a** (1.25 g, 3.64 mmol) was reacted with (Z)-3-(3-chloro-2-fluoro-phenyl)-2-(4-chloro-2-fluoro-phenyl)-acrylonitrile (0.93 g, 3 mmol) prepared in **Example 52a**, AgF (0.57 g, 4.5 mmol), and triethylamine (1.05 mL, 7.5 mmol) in dichloromethane (100 mL) at room temperature for 18 h, followed by the reaction with DBU (3.6 ml) in tert-butanol (15 mL) at 100 °C for 2 h to give rac-(2R,3S,4R,5S)-5-[3-(tert-butyl-dimethyl-silanyloxy)-2,2-dimethyl-propyl]-3-(3-chloro-2-fluoro-phenyl)-4-(4-chloro-2-fluoro-phenyl)-4-cyano-pyrrolidine-2-carboxylic acid tert-butyl ester as a white solid (1.2 g, 61%).

### Example 105c

### Preparation of intermediate rac-(2R,3S,4R,5S)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid trifluoroacetic acid

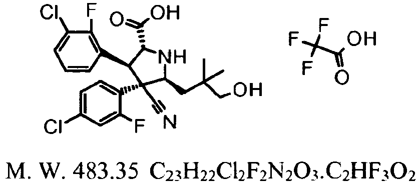

In a manner similar to the method described in **Example 25a**, rac-(2R,3S,4R,5S)-5-[3-(tert-butyl-dimethyl-silanyloxy)-2,2-dimethyl-propyl]-3-(3-chloro-2-fluoro-phenyl)-4-(4-chloro-2-fluoro-phenyl)-4-cyano-pyrrolidine-2-carboxylic acid tert-butyl ester prepared in **Example 105b** (1.1g, 1.68 mmol) was reacted with trifluoroacetic acid in dichloromethane at room temperature to give rac-(2R,3S,4R,5S)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid trifluoroacetic acid as a white solid (1g, 100%).

### Example 105d

### Preparation of rac-(2R,3S,4R,5S)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid ((S')-3,4-dihydroxybutyl)-amide

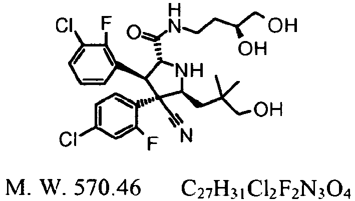

In a manner similar to the method described in **Examples 42c and 42d**, rac-(2R,3S,4R,5S)-3-(3-chloro-2-fluoro-phenyl)-4-(4-chloro-2-fluoro-phenyl)-4-cyano-5-(3-hydroxy-2,2-dimethylpropyl)-pyrrolidine-2-carboxylic acid trifluoroacetic acid prepared in **Example 105c** (1 g, 1.67 mmol) was reacted with 2-((S)-2,2-dimethyl-[1,3]dioxolan-4-yl)-ethylamine (0.73 g, 5 mmol), HATU (1.14 g, 3 mmol) and iPr₂NEt (1.46 mL, 8.4 mmol) in CH₂Cl₂ at room temperature for 20 h, then reacted with aqueous HCl solution in tetrahydrofuran at room temperature for 2 h to give rac-(2R,3S,4R,5S)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid ((S)-3,4-dihydroxy-butyl)-amide as a white solid (0.3 g, 64%).

HRMS (ES⁺) m/z Calcd for C₂₇H₃₁Cl₂F₂N₃O₄+H[(M+H)⁺]: 570.1733, found: 570.1731.

### Example 105e

### Preparation of (2R,3S,4R,5S)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid ((S)-3,4-dihydroxybutyl)-amide

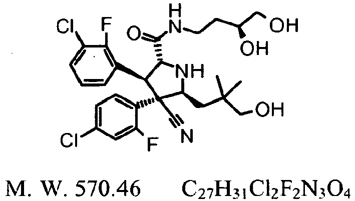

Rac-(2R,3S,4R,5S)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid ((S)-3,4-dihydroxy-butyl)-amide (0.24 g) was separated by chiral SFC chromatography to provide chiral (2R,3S,4R,5S)-3-(3-chloro-2-fluoro-phenyl)-4-(4-chloro-2-fluoro-phenyl)-4-cyano-5-(3-hydroxy-2,2-dimethylpropyl)-pyrrolidine-2-carboxylic acid ((S)-3,4-dihydroxy-butyl)-amide as a white solid (102 mg, 43%) and chiral (2S,3R,4S,5R)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid ((S)-3,4-dihydroxybutyl)-amide as a white solid (93 mg, 39%).

HRMS (ES⁺) m/x Calcd for C₂₇H₃₁Cl₂F₂N₃O₄+H[(M+H⁺] 570.1733, found: 570.1730.

### Example 106a

### Preparation of intermediate [3,3-diethyl-pent-(E)-ylideneamino]-acetic acid tert-butyl ester

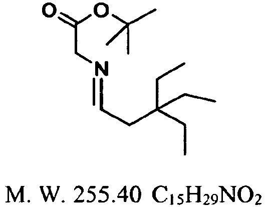

### Step A

To a solution of dimethyl malonate (10 g, 75 mmol), 3-pentanone (6.5 g, 75 mmol) and pyridine (7.9 g, 100 mmol) in anhydrous tetrahydrofuran (300 mL) at 0 °C was added a dichloromethane solution (1 M) of TiCl₄ (100 mL, 100 mmol) during a period of 1 h. After the addition was finished, the reaction mixture was gradually warmed room temperature and stirred for 18 h. Water was added to quench the reaction. The mixture was extracted with ethyl ether. The organic layer was separated, and the aqueous layer was extracted with ethyl acetate. The organic layers were combined, concentrated. The residue was purifed by chromatography (EtOAc:hexanes=1;20) to give 2-(1-ethyl-propylidene)-malonic acid dimethyl ester as a light yellow oil (7 g, 46%).

### Step B

To a suspension of Cul (7.61 g, 40 mmol) in anhydrous tetrahydrofuran (100 mL) at -50 °C was added ethylmagnesium chloride (2 M, 40 mL, 80 mmol) during a period of 15 min. After the addition was finished, the reaction mixture was gradually warmed room temperature and stirred for 20 min. Then the temperature of the mixture was lowered to -50 °C, a tetrahydrofuran solution (50 mL) of 2-(1-ethyl-propylidene)-malonic acid dimethyl ester (3.5 g, 17.5 mmol) was added. The reaction mixture was allowed to slowly warmed to room temperature and stirred for 3 h. Aqueous saturated NH₄Cl solution was added to quench the reaction. The mixture was filtered, and the filtrate was concentrated to remove most of tetrahydrofuran. The residue was extracted with ethyl acetate twice. The organic layers were combined, concentrated. The residue was purifed by chromatography (EtOAc:hexanes=1;30) to give 2-(1,1-diethyl-propyl)-malonic acid dimethyl ester as a colorless oil (2.6 g, 57%).

### Step C

To a solution of 2-(1,1-diethyl-propyl)-malonic acid dimethyl ester (2.5 g, 11 mmol) in DMSO (30 mL) was added LiCl (0.91 g, 21.6 mmol) and H₂O (0.19 ml, 11 mmol). The reaction mixture was heated at 170 °C for 4 h, then poured into a ice-water, extracted with ethyl acetate. The organic layer were separated, washed with water, brine, dried over MgSO₄, and concentrated to give 3,3-diethyl-pentanoic acid methyl ester as a yellow oil (1.9 g, 100%).

### Step D

To a solution of 3,3-diethyl-pentanoic acid methyl ester (1.9 g, 11 mmol) in anhydrous tetrahydrofuran (50 mL) at 0 °C was added a tetrhydrofuran solution (2 M) of LiAlH₄ (9 mL, 18 mmol) under nitrogen. The reaction mixture was stirred at 0 °C for 1 h, then poured into a ice-water. The mixture was extracted with ethyl acetate. The organic layer were separated, washed with water, aqueous HCl solution, brine, dried over MgSO₄, and concentrated to give 3,3-diethyl-pentan-l-ol as a yellow oil (1.4 g, 90%).

### Step E

To a solution of oxalyl chloride (0.86 mL, 9.9 mmol) (Aldrich) in dichloromethane (20 mL) at - 78 °C was added the solution of dimethyl sulfoxide (1.4 mL, 19.8 mmol) in dichloromethane (5 mL) dropwise. After 5 mins, the solution of 3,3-diethyl-pentan-1-ol (1.3 g, 9 mmol) in dichloromethane (5 mL) was added dropwise. The reaction mixture was stirred at -78 °C for 15 min. Triethylamine (4.5 mL, 32 mmol) was added and the reaction mixture was slowly warmed up to room temperature and stirred at room temperature for 45 min. Then water was added. The organic layers were separated, and the aqueous layer was extracted with dichloromethane. The organic layers were combined, washed with 10% of HCl, saturated NaHCO₃, brine, dried over MgSO₄, and concentrated to give 3,3-diethyl-pentanal as a yellow oil (Yield: 1 g, 78%).

### Step F

In a manner similar to the method described in **Example 1a**, glycine tert-butyl ester (0.92 g, 7 mmol) was reacted with 3,3-diethyl-pentanal (1g, 7 mmol) in CH₂Cl₂ at room temperature for 5 h to give [3,3-diethyl-pent-(E)-ylideneamino]-acetic acid tert-butyl ester as a colorless oil (1.8 g, 100%).

### Example 106b

### Preparation of intermediate rac-(2R,3S,4R,5S)-3-(3-chloro-2-fluoro-phenyl)-4-(4-chloro-2-fluoro-phenyl)-4-cyano-5-(2,2-diethyl-butyl)-pyrrolidine-2-carboxylic acid tert-butyl ester

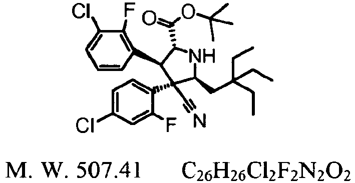

In a manner similar to the method described in **Example 100b**, [3,3-diethyl-pent-(E)-ylideneamino]-acetic acid tert-butyl ester prepared in **Example 106a** (1.8 g, 7 mmol) was reacted witch (Z)-3-(3-chloro-2-fluoro-phenyl)-2-(4-chloro-2-fluoro-phenyl)-acrylonitrile (1.74 g, 5.6 mmol) prepared in **Example 52a**, AgF (1.1 g, 8.6mmol), and triethylamine (1.95 mL, 14 mmol) in dichloromethane (150 mL) at room temperature for 18 h, followed by the reaction with DBU (7 ml) in tert-butanol (30 mL) at 100 °C for 2h to give rac-(2R,3S,4R,5S)-3-(3-chloro-2-fluoro-phenyl)-4-(4-chloro-2-fluoro-phenyl)-4-cyano-5-(2,2-diethyl-butyl)-pyrrolidine-2-carboxylic acid tert-butyl ester as a white gum (1.8 g, 57%).

### Example 106c

### Preparation of intermediate rac-(2R,3S,4R,5S)-3-(3-chloro-2-fluoro-phenyl)-4-(4-chloro-2-fluoro-phenyl)-4-cyano-5-(2,2-diethyl-butyl)-pyrrolidine-2-carboxylic acid trifluoroacetic acid

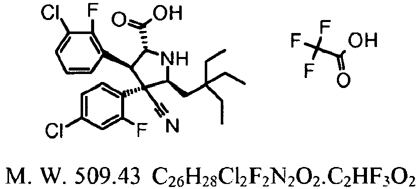

In a manner similar to the method described in **Example 25a**, rac-(2R,3S,4R,5S)-3-(3-chloro-2-fluoro-phenyl)-4-(4-chloro-2-fluoro-phenyl)-4-cyano-5-(2,2-diethyl-butyl)-pyrrolidine-2-carboxylic acid tert-butyl ester prepared in **Example 106b** (1.8 g, 3.2 mmol) was reacted with trifluoroacetic acid in dichloromethane at room temperature to give rac-(2R,3S,4R,5S)-3-(3-chloro-2-fluoro-phenyl)-4-(4-chloro-2-fluoro-phenyl)-4-cyano-5-(2,2-diethyl-butyl)-pyrrolidine-2-carboxylic acid trifluoroacetic acid as a light yellow solid (2 g, 100%).

### Example 106d

### Preparation of rac-(2R,35,4R,5S)-3-(3-chloro-2-fluoro-phenyl)-4-(4-chloro-2-fluoro-phenyl)-4-cyano-5-(2,2-diethyl-butyl)-pyrrolidine-2-carboxylic acid ((S)-3,4-dihydroxy-butyl)-amide

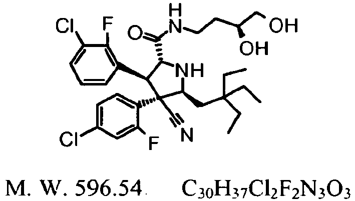

In a manner similar to the method described in **Examples 42c and 42d**, rac-(2R,3S,4R,5S)-3-(3-chloro-2-fluoro-phenyl)-4-(4-chloro-2-fluoro-phenyl)-4-cyano-5-(2,2-diethyl-butyl)-pyrrolidine-2-carboxylic acid trifluoroacetic acid prepared in **Example 106c** (0.5 g, 0.8 mmol) was reacted with 2-((S)-2,2-dimethyl-[1,3]dioxotan-4-yl)-ethylamine (0.35 g, 2.4 mmol), HATU (0.55 g, 1.4 mmol) and iPr₂NEt (0.7 mL, 4 mmol) in CH₂Cl₂ at room temperature for 20 h, then reacted with aqueous HCl solution in tetrahydrofuran at room temperature for 2 h to give rac-(2R,3S,4R,5S)-3-(3-chloro-2-fluoro-phenyl)-4-(4-chloro-2-fluoro-phenyl)-4-cyano-5-(2,2-diethyl-butyl)-pyrrolidine-2-carboxylic acid ((S)-3,4-dihydroxy-butyl)-amide as a white solid (0.33 g, 70%). HRMS (ES⁺) m/z Calcd for C₃₀H₃₇Cl₂F₂N₃O₃+H[(M+H)⁺]: 596.2253, found: 596.2254.

### Example 107a

### Preparation of intermediate [3-ethyl-3-methyl-pent-(E)-ylidenea mino]-acetic acid tert-butyl ester

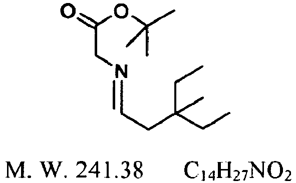

### Step A

To a suspension of CuI (5.7 g, 30 mmol) in anhydrous tetrahydrofuran (100 mL) at -50 °C was added methylmagnesium chloride (3 M, 20 mL, 60 mmol) during a period of 15 min. After the addition was finished, the reaction mixture was gradually warmed room temperature and stirred for 20 min. Then the temperature of the mixture was lowered to -50 °C, a tetrahydrofuran solution (20 mL) of 2-(1-ethyl-propylidene)-malonic acid dimethyl ester (3 g, 15 mmol) prepared in **Example 106a Step A** was added. The reaction mixture was allowed to slowly warmed to room temperature and stirred for 1 h. Aqueous saturated NH₄Cl solution was added to quench the reaction. The mixture was filtered, and the filtrate was concentrated to remove most of tetrahydrofuran. The residue was extracted with ethyl acetate twice. The organic layers were combined, concentrated. The residue was purifed by chromatography (EtOAc:hexanes=1;30) to give 2-(1-ethyl-1-methyl-propyl)-malonic acid dimethyl ester as a colorless oil (3 g, 93%).

### Step B

To a solution of 2-(1-ethyl-1-methyl-propyl)-malonic acid dimethyl ester (3 g, 14 mmol) in DMSO (30 mL) was added LiCl (1.2 g, 28 mmol) and H₂O (0.25 mL, 14 mmol). The reaction mixture was heated at 170 °C for 3 h, then poured into a ice-water, extracted with ethyl acetate. The organic layer were separated, washed with water, brine, dried over MgSO₄, and concentrated to give 3-ethyl-3-methyl-pentanoic acid methyl ester as a yellow oil (1.5 g, 68%).

### Step C

To a solution of 3-ethyl-3-methyl-pentanoic acid methyl ester (1.5 g, 9.4 mmol) in anhydrous tetrahydrofuran (30 mL) at 0 °C was added a tetrhydrofuran solution (2 M) of LiAlH₄ (5 mL, 10 mmol) under nitrogen. The reaction mixture was stirred at 0 °C for 1 h, then poured into a ice-water. The mixture was extracted with ethyl acetate. The organic layer were separated, washed with water, aqueous HCl solution, brine, dried over MgSO₄, and concentrated to give 3-ethyl-3-methyl-pentan-1-ol as a yellow oil (1.3 g, 100%).

### Step D

To a solution of oxalyl chloride (0.96 mL, 11 mmol) (Aldrich) in dichloromethane (20 m) at - 78 °C was added the solution of dimethyl sulfoxide (1.56 mL, 22 mmol) in dichloromethane (5 mL) dropwise. After 5 mins, the solution of 3-ethyl-3-methyl-pentan-1-ol (1.3 g, 10 mmol) in dichloromethane (5 mL) was added dropwise. The reaction mixture was stirred at -78 °C for 15 min. Triethylamine (5 mL, 36 mmol) was added and the reaction mixture was slowly warmed up to room temperature and stirred at room temperature for 45 min. Then water was added. The organic layers were separated, and the aqueous layer was extracted with dichloromethane. The organic layers were combined, washed with 10% of HCl, saturated NaHCO₃, brine, dried over MgSO₄, and concentrated to give 3-ethyl-3-methyl-pentanal as a light yellow oil (Yield: 1 g, 78%).

### Step F

In a manner similar to the method described in **Example 1a**, glycine tert-butyl ester (1.0 g, 7.8 mmol) was reacted with 3-ethyl-3-methyl-pentanal 4,1 g, 7.8 mmol) in CH₂Cl₂ at room temperature for 5 h to give [3-ethyl-3-methyl-pent-(E)-ylideneammo]-acetic acid tert-butyl ester as a colorless oil (1.9 g, 100%).

### Example 107b

### Preparation of intermediate rac-(2R,3S,4R,5S)-3-(3-chloro-2-fluoro-phenyl)-4-(4-chloro-2-fluoro-phenyl)-4-cyano-5-(2-ethyl-2-methyl-butyl)-pyrrolidine-2-carboxylic acid tert-butyl ester

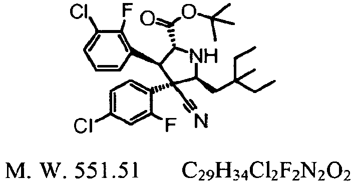

In a manner similar to the method described in **Example 100b**, [3-ethyl-3-methyl-pent-(E)-ylideneamino]-acetic acid tert-butyl ester prepared in **Example 107a** (1.9 g, 7.8 mmol) was reacted with (Z)-3-(3-chloro-2-fluoro-phenyl)-2-(4-chloro-2-fluoro-phenyl)-acrylonitrile (1.9 g, 6.2 mmol) prepared in **Example 52a**, AgF (1.2 g, 9.4 mmol), and triethylamine (2.1 mL, 15 mmol) in dichloromethane (150 mL) at room temperature for 18 h, followed by the reaction with DBU (7 ml) in tert-butanol (30 mL) at 100 °C for 2 h to give rac-(2R,3S,4R,5S)-3-(3-chloro-2-fluoro-phenyl)-4-(4-chloro-2-fluoro-phenyl)-4-cyano-5-(2-ethyl-2-methyl-butyl)-pyrrolidine-2-carboxylic acid tert-butyl ester as a light yellow gum (2.5 g, 74%).

### Example 107c

### Preparation of intermediate rac-(2R,3S,4R,5S)-3-(3-chloro-2-fluoro-phenyl)-4-(4-chloro-2-fluoro-phenyl)-4-cyano-5-(2-ethyl-2-methyl-butyl)-pyrrolidine-2-carboxylic acid trifluoroacetic acid

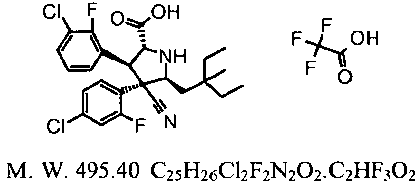

In a manner similar to the method described in **Example 25a**, rac-(2R,3S,4R,5S)-3-(3-chloro-2-fluoro-phenyl)-4-(4-chloro-2-fluoro-phenyl)-4-cyano-5-(2-ethyl-2-methyl-butyl)-pyrrolidine-2-carboxylic acid tert-butyl ester prepared in **Example 107b** (1.8 g, 3.2 mmol) was reacted with trifluoroacetic acid in dichloromethane at room temperature to give rac-(2R,3S,4R,5S)-3-(3-chloro-2-fluoro-phenyl)-4-(4-chloro-2-fluoro-phenyl)-4-cyano-5-(2-ethyl-2-methyl-butyl)-pyrrolidine-2-carboxylic acid trifluoroacetic acid as a light yellow solid (2.5 g, 91%).

### Example 107d

### Preparation of rac-(2R,3S,4R,5S)-3-(3-chloro-2-fluoro-phenyl)-4-(4-chloro-2-fluoro-phenyl)-4-cyano-5-(2-ethyl-2-methyl-butyl)-pyrrolidine-2-carboxylic acid ((S)-3,4-dihydroxy-butyl)-amide

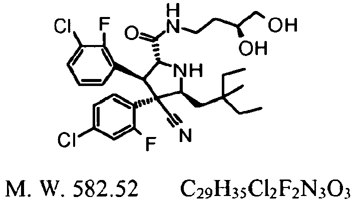

In a manner similar to the method described in **Examples 42c and 42d**, rac-(2R,3S,4R,5S)-3-(3-chloro-2-fluoro-phenyl)-4-(4-chloro-2-fluoro-phenyl)-4-cyano-5-(2-ethyl-2-methyl-butyl)-pyrrolidine-2-carboxylic acid trifluoroacetic acid prepared in **Example 106c** (0.6 g, 1 mmol) was reacted with 2-((S)-2,2-dimethyl-[1,3]dioxolan-4-yl)-ethylamine (0.43 g, 3 mmol), HATU (0.67 g, 1.8 mmol) and iPr₂NEt (0.86 mL, 4.9 mmol) in CH₂Cl₂ at room temperature for 20 h, then reacted with aqueous HCl solution in tetrahydrofuran at room temperature for 2 h to give rac-(2R,3S,4R,5S)-3-(3-chloro-2-fluoro-phenyl)-4-(4-chloro-2-fluoro-phenyl)-4-cyano-5-(2-ethyl-2-methyl-butyl)-pyrrolidine-2-carboxylic acid ((S)-3,4-dihydroxy-butyl)-amide as a white solid (0.3 g, 53%).

HRMS (ES⁺) m/z Calcd for C₂₉H₃₅Cl₂F₂N₃O₃+H[(M+H)⁺]: 582.2097, found: 582.2096.

### Example 108a

### Preparation of intermediate (Z)-2-(4-chloro-2,6-difluoro-phenyl)-3-(3-chloro-2-fluoro-phenyl)-acrylonitrile

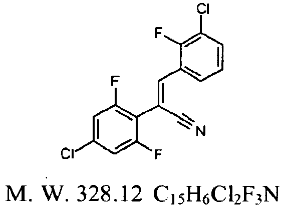

### Step A

To the solution of4-chloro-2,6-difluorobenzyl bromide (Alfa) (2.5 g, 10 mmol) in ethanol (13 mL) and H₂O (10 mL) was added KCN (1.75 g, 27 mmol). The reaction mixture was heated at 100 °C for 1h. The mixture was cooled, and extracted with ethyl acetate. The organic layer was separated, washed with saturated aqueous NaHCO₃ solution, brine, dried over MgSO₄, and concentrated. The residue was purified by chromatography (EtOAc:hexanes = 1;4) to give (4-chloro-2,6-difluoro-phenyl)-acetonitrile as a yellow oil (1.1 g, 57%).

### Step B

In a manner similar to the method described in **Example 1b**, (4-chloro-2,6-difluoro-phenyl)-acetonitrile (1.1 g, 6 mmol) was reacted with 2-fluoro-3-chlorobenzaldehyde (1.2 g, 7mmol), methanolic solution (25 wt%) of sodium methoxide (1.5 mL, 7 mmol) in methanol (40 mL) at 50 °C for 3 h to give (Z)-2-(4-chloro-2,6-difluoro-phenyl)-3-(3-chloro-2-fluoro-phenyl)-acrylonitrile as a white solid (1.5 g, 75%).

### Example 108b

### Preparation of intermediate rac-(2R,3R,4R,5S)-4-(4-chloro-2,6-difluoro-phenyl)-3-(3-chloro-phenyl)-4-cyano-5-(2,2-dimethyl-propyl)-pyrrolidine-2-carboxylic acid tert-butyl ester

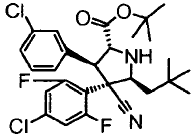

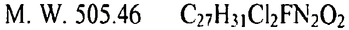

In a manner similar to the method described in **Example 1c**, [3-methyl-but-(E)-ylideneamino]-acetic acid tert-butyl ester prepared in **Example 1a** (1 g, mmol) was reacted with (Z)-2-(4-chloro-2,6-difluoro-phenyl)-3-(3-chloro-2-fluoro-phenyl)-acrylonitrile (1.3 g, 4mmol) prepared in **Example 109a**, AgF (0.77 g, 6 mmol), and triethylamine (1.4 mL, 10 mmol) in dichloromethane (120 mL) at room temperature for 18 h, followed by the reaction with DBU (4.8 ml) in tert-butanol (20 mL) at 100 °C for 2 h to give rac-(2R,3R,4R,5S)-4-(4-chloro-2,6-difluoro-phenyl)-3-(3-chloro-phenyl)-4-cyano-5-(2,2-dimethyl-propyl)-pyrrolidine-2-carboxylic acid tert-butyl ester as a white gum (0.9 g, 41%).

### Example 108c

### Preparation of intermediate rac-(2R,3R,4R,5S)-4-(4-chloro-2,6-difluoro-phenyl)-3-(3-chloro-phenyl)-4-cyano-5-(2,2-dimethyl-propyl)-pyrrolidine-2-carboxylic acid trifluoroacetic acid

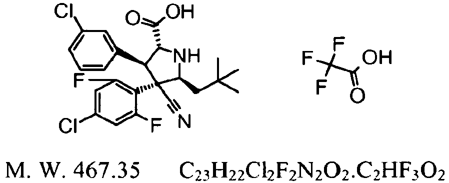

In a manner similar to the method described in Example 25a, rac-(2R,3R,4R,5S)-4-(4-chloro-2,6-difluoro-phenyl)-3-(3-chloro-phenyl)-4-cyano-5-(2,2-dimethyl-propyl)-pyrrolidine-2-carboxylic acid tert-butyl ester prepared in **Example 108b** (0.9 g, 1.7 mmol) was reacted with trifluoroacetic acid in dichloromethane at room temperature to give rac-(2R,3R,4R,5S)-4-(4-chloro-2,6-difluoro-phenyl)-3-(3-chloro-phenyl)-4-cyano-5-(2,2-dimethyl-propyl)-pyrrolidine-2-carboxylic acid trifluoroacetic acid as a white solid (0.9 g, 91%).

### Example 108d

### Preparation of rac-(2R,3R,4 R,5S)-4-(4-chloro-2,6-difluoro-phenyl)-3-(3-chloro-phenyl)-4-cyano-5-(2,2-dimethyl-propyl)-pyrrolidine-2-carboxylic acid ((S)-3,4-dihydroxy-butyl)-amide

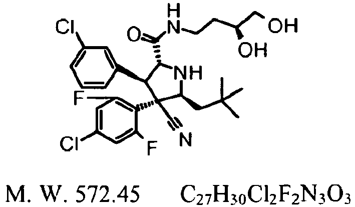

In a manner similar to the method described in **Examples 42c, 42d**, rac-(2R,3R,4R,5S)-4-(4-chloro-2,6-dilluoro-phenyl)-3-(3-chloro-phenyl)-4-cyano-5-(2,2-dimethyl-propyl)-pyrrolidine-2-carboxylic acid trifluoroacetic acid prepared in **Example 108c** (0.4 g, 0.67 mmol) was reacted with 2-((S)-2,2-dimethyl-[1,3]dioxolan-4-yl)-ethylamine (0.29 g, 2 mmol), HATU (0.46 g, 1.2 mmol) and iPr₂NEt (0.58 mL, 3.3 mmol) in CH₂Cl₂ at room temperature for 20 h, then reacted with aqueous HCl solution in tetrahydrofuran at room temperature for 2 h to give rac-(2R,3R,4R,5S)-4-(4-chloro-2,6-difluoro-phenyl)-3-(3-chloro-phenyl)-4-cyano-5-(2,2-dimethylpropyl)-pyrrolidine-2-carboxylic acid ((S)-3,4-dihydroxy-butyl)-amide as a white solid (0.3 g, 77%).

HRMS (ES⁺) m/z Calcd for C₂₇H₃₀Cl₂F₂N₃O₃+H[(M+H)⁺]: 572.1689, found: 572.1691.

### Example 109a

### Preparation of intermediate (Z)-2-(4-chloro-2,5-difluoro-phenyl)-3-(3-chloro-2-fluoro-phenyl)-acrylonitrile

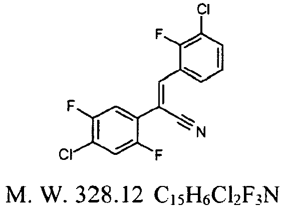

### Step A

To the solution of4-chloro-2,5-difluorobenzoic acid (Oakwood) (6.08 g, 31 mmol) in anhydrous tetrahydrofuran (75 mL) at 0 °C was added a solution of BH₃. THF (1M, 62 mL, 62 mmol). The reaction mixture was stirred at room temperature for 18 h. Aqueous HCl solution was added, and the mixture was extracted with ethyl acetate. The organic layer was separated, washed with saturated aqueous NaHCO₃ solution, brine, dried over MgSO₄, and concentrated to give (4-chloro-2,5-difluoro-phenyl)-methanol as a colorless oil (5.5 g, 98%).

### Step B

A mixture of (4-chloro-2,5-difluoro-phenyl)-methanol (5.5 g, 32 mmol) in thionyl chloride (25 mL) was heated at refluxing (100 °C) for 30 min. The mixture was cooled to room temperature and concentrated. The residue was diluted with ethyl acetate, washed with saturated aqueous NaHCO₃ solution, water, brine, dried over MgSO₄, and concentrated to give 1-chloro-4-chloromethyl-2,5-difluoro-benzene as a yellow oil (2.1 g, 34%).

### Step C

To the solution of 1-chloro-4-chloromethyl-2,5-difluoro-benzene (2.1 g, 11 mmol) in ethanol (13 mL) and H₂O (10 mL) was added KCN (1.8 g, 28 mmol). The reaction mixture was heated at 100 °C for 1 h. The mixture was cooled, and extracted with ethyl acetate. The organic layer was separated, washed with saturated aqueous NaHCO₃ solution, brine, dried over MgSO₄, and concentrated. The residue was purified by chromatography (EtOAc:hexanes = 1 ;4) to give 4-chloro-2,5-difluoro-phenyl)-acetonitrite as a light yellow oil (1.0 g, 50%).

### Step D

In a manner similar to the method described in **Example 1b**, 4-chloro-2,5-difluoro-phenyl)-acetonitrile (1.0 g, 5 mmol) was reacted with 2-fluoro-3-chlorobenzaldehyde (1.0 g, 6 mmol), methanolic solution (25 wt%) of sodium methoxide (1.3 mL, 5.9 mmol) in methanol (40 mL) at 50 °C for 3 h to give (Z)-2-(4-chloro-2,5-difluoro-phenyl)-3-(3-chloro-2-fluoro-phenyl)-acrylonitrile as a white solid (1.3 g, 75%).

### Example 109b

### Preparation of intermediate rac-(2R,3R,4R,5S)-4-(4-chloro-2,5-difluoro-phenyl)-3-(3-chloro-phenyl)-4-cyano-5-(2,2-dimethyl-propyl)-pyrrolidine-2-carboxylic acid tert-butyl ester

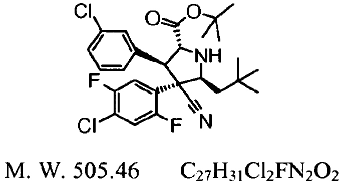

In a manner similar to the method described in **Example 1c**, [3-methyl-but-(E)-ylideneamino]-acetic acid tert-butyl ester prepared in **Example 1a** (5 mmol) was reacted with give (Z)-2-(4-chloro-2,5-difluoro-phenyl)-3-(3-chloro-2-fluoro-phenyl)-acrylonitrile (1.3 g, 3 mmol) prepared in **Example 109a**, AgF (0.77 g, 6 mmol), and triethylamine (1.4 mL, 10 mmol) in dichloromethane (120 mL) at room temperature for 18 h, followed by the reaction with DBU (4.8 ml) in tert-butanol (20 mL) at 100 °C for 2 h to give rac-(2R,3R,4R,5S)-4-(4-chloro-2,5-difluoro-phenyl)-3-(3-chloro-phenyl)-4-cyano-5-(2,2-dimethyl-propyl)-pyrrolidine-2-carboxylic acid tert-butyl ester as a light yellow gum (1.5 g, 69%).

### Example 109c

### Preparation of intermediate rac-(2R,3R,4R,5S)-4-(4-chloro-2,5-difluoro-phenyl)-3-(3-chloro-phenyl)-4-cyano-5-(2,2-dimethyl-propyl)-pyrrolidine-2-carboxylic acid trifluoroacetic acid

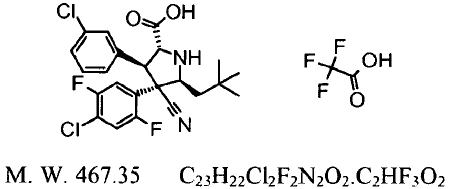

In a manner similar to the method described in **Example 25a**, rac-(2R,3R,4R,5S)-4-(4-chloro-2,5-difluoro-phenyl)-3-(3-chloro-phenyl)-4-cyano-5-(2,2-dimethyl-propyl)-pyrrolidine-2-carboxylic acid tert-butyl ester prepared in **Example 109b** (1.5 g, 2.8 mmol) was reacted with trifluoroacetic acid in dichloromethane at room temperature to give rac-(2R,3R,4R,5S)-4-(4-chloro-2,5-difluoro-phenyl)-3-(3-chloro-phenyl)-4-cyano-5-(2,2-dimethyl-propyl)-pyrrolidine-2-carboxylic acid trifluoroacetic acid as a off white solid (1.5 g, 91%).

### Example 109d

### Preparation of rac-(2R,3R,4R,5S)-4-(4-chloro-2,5-difluoro-phenyl)-3-(3-chloro-phenyl)-4-cyano-5-(2,2-dimethyl-propyl)-pyrrolidine-2-carboxylic acid ((S)-3,4-dihydroxy-butyl)-amide

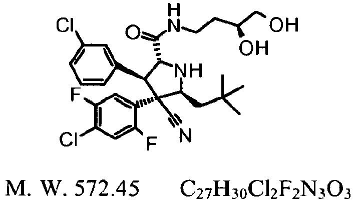

In a manner similar to the method described in **Examples 42c, 42d,** rac-(2R,3R,4R,5S)-4-(4-chloro-2,5-difluoro-phenyl)-3-(3-chloro-phenyl)-4-cyano-5-(2,2-dimethyl-propyl)-pyrrolidine-2-carboxylic acid trifluoroacetic acid prepared in **Example 108c** (0.5 g, 0.84 mmol) was reacted with 2-((S)-2,2-dimethyl-[1,3]dioxolan-4-yl)-ethylamine (0.36 g, 2.5 mmol), HATU (0.57 g, 1.5 mmol) and iPr₂NEt (0.73 mL, 4.2 mmol) in CH₂Cl₂ at room temperature for 20 h, then reacted with aqueous HCl solution in tetrahydrofuran at room temperature for 2 h to give rac-(2R,3R,4R,5S)-4-(4-chloro-2,5-difluoro-phenyl)-3-(3-chloro-phenyl)-4-cyano-5-(2,2-dimethylpropyl)-pyrrolidine-2-carboxylic acid ((S)-3,4-dihydroxy-butyl)-amide as a white solid (0.3 g, 50%).

HRMS (ES⁺) m/z Calcd for C₂₇H₃₀Cl₂F₂N₃O₃+Hl(M+H)⁺]: 572.1689, found: 572.1689.

### Example 110a

### Preparation of intermediate [4-methoxy-3,3-dimethyl-but-(E)-ylideneamino]-acetic acid tert-butyl ester

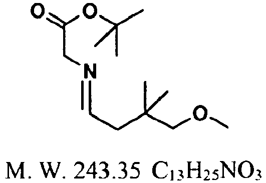

### Step A.

A mixture of 2,2-dimethyl-propane-1,3-diol (Aldrich) (5 g, 48 mmol) in anhydrous ethyl ether (100 mL) at 0 °C was added thionyl chloride (8.7 mL, 120 mmol). The reactiom mixture was stirred at 0 °C for 2 h. Water was added. The organic layer was separated, the aqueous layer was then extracted with ethyl ether. The combined organic layers wer washed with saturated aqueous NaHCO₃ solution, brine, dried over MgSO₄, concentrated to give 5,5-dimethyl-[1,3,2]dioxathiane 2-oxide as a light pink oil (4.8 g, 82%).

### Step B

To the solution of 5,5-dimethyl-[1,3,2]dioxathiane 2-oxide (4.8 g, 39 mmol) in anhydrous dimethyl sulfoxide (50 mL) was added NaCN (5.8 g, 118 mmol). The reaction mixture was heated at 120 °C for 5 h. The mixture was cooled, and water was added. The mixture was extracted with ethyl acetate twice. The combined organic layers were washed with saturated aqueous NaHCO₃ solution, brine, dried over MgSO₄, and concentrated. The residue was purified by chromatography (EtOAc:hexanes = 1;4) to give 4-hydroxy-3,3-dimethyl-butyronitrile as a yellow oil (1.6 g, 38%).

### Step C

To the solution of 4-hydroxy-3,3-dimethyl-butyronitrile (0.8 g, 7 mmol) in anhydrous dimethylformamide (5 mL) was added NaH (60%, 0.42 g, 11 mmol). The mixture was stirred at room temperature for 15 min, then iodomethane (0.88 mL, 14 mmol) was added. The mixture was stiired at room temperature for 1 h. Water was added. The mixture was extracted with ethyl acetate twice. The combined organic layers were washed with brine, dried over MgSO₄, and concentrated to give 4-methoxy-3,3-dimethyl-butyronitrile as a yellow oil (0.85 g, 94%).

### Step D

To a solution of 4-methoxy-3,3-dimethyl-butyronitrile (0.85 g, 6.7 mmol) in dichloromethane (20 mL) at -78 °C was added a toluene solution (1M) of DIBAL (7.4 mL, 7.4 mmol) dropwise. The reaction mixture was stirred at 0 °C for I h. The mixture was poured into aqueous saturated NH₄Cl solution. The organic layer was separated, and the aqueous layer was extracted with ethyl acetate. The organic layers were combined, washed with brine, dried over MgSO₄, and concentrated to give 4-methoxy-3,3-dimethyl-butyraldehyde as a colorless oil (Yield: 0.3 g, 34%).

### Step E

In a manner similar to the method described in **Example 1a**, glycine tert-butyl ester (0.3 g, 2.3 mmol) was reacted with 4-methoxy-3,3-dimethyl-butyraldehyde (0.3 g, 2.3 mmol) in CH₂Cl₂ at room temperature for 18 h to give [4-methoxy-3,3-dimethyl-but-(E)-ylideneamino]-acetic acid tert-butyl ester as a colorless oil (0.56 g, 100%).

### Example 110b

### Preparation of intermediate rac-(2R,3S,4R,5S)-3-(3-chloro-2-fluoro-phenyl)-4-(4-chloro-2-fluoro-phenyl)-4-cyano-5-(3-methoxy-2,2-dimethyl-propyl)-pyrrolidine-2-carboxylic acid tert-butyl ester

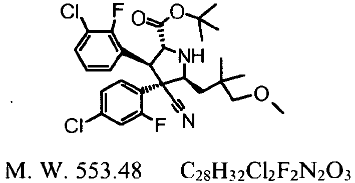

In a manner similar to the method described in **Example 100b**, [4-methoxy-3,3-dimethyl-but-(E)-ylideneamino]-acetic acid tert-butyl ester prepared in **Example 110a** (3.8 mmol) was reacted with (Z)-3-(3-chloro-2-fluoro-phenyl)-2-(4-chloro-2-fluoro-phenyl)-acrylonitrile (0.94 g, 3 mmol) prepared in **Example 52a**, AgF (0.58 g, 4.6 mmol), and triethylamine (1.06 mL, 7.6 mmol) in dichloromethane (100 mL) at room temperature for 18 h, followed by the reaction with DBU (3.6 ml) in tert-butanot (20 mL) at 100 °C for 2 h to give rac-(2R,3S,4R,5S)-3-(3-chloro-2-fluoro-phenyl)-4-(4-chloro-2-fluoro-phenyl)-4-cyano-5-(3-methoxy-2,2-dimethyl-propyl)-pyrrolidine-2-carboxylic acid tert-butyl ester as a light yellow solid (1 g, 59%).

### Example 110c

### Preparation of intermediate rac-(2R,3S,4R,5S)-3-(3-chloro-2-fluoro-phenyl)-4-(4-chloro-2-fluoro-phenyl)-4-cyano-5-(3-methoxy-2,2-dimethyl-propyl)-pyrrolidine-2-carboxylic acid trifluoroacetic acid

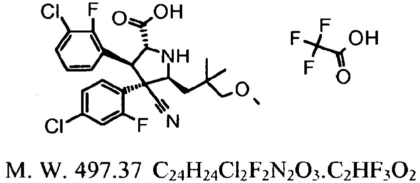

In a manner similar to the method described in **Example 25a,** rac-(2R,3S,4R,5S)-3-(3-chloro-2-fluoro-phenyl)-4-(4-chloro-2-fluoro-phenyl)-4-cyano-5-(3-methoxy-2,2-dimethyl-propyl)-pyrrolidine-2-carboxylic acid tert-butyl ester prepared in **Example 105b** (1.0 g, 1.8 mmol) was reacted with trifluoroacetic acid in dichloromethane at room temperature to give rac-(2R,3S,4R,SS)-3-(3-chloro-2-fluoro-phenyl)-4-(4-chloro-2-fluoro-phenyl)-4-cyano-5-(3-methoxy-2,2-dimethyl-propyl)-pyrrolidine-2-carboxylic acid trifluoroacetic acid as a yellow solid (0.9 g, 82%).

### Example 110d

### Preparation of rac-(2R,3S,4R,5S)-3-(3-chloro-2-fluoro-phenyl)-4-(4-chloro-2-fluoro-phenyl)-4-cyano-5-(3-methoxy-2,2-dimethyl-propyl)-pyrrolidine-2-carboxylic acid ((S)-3,4-dihydroxybutyl)-amide

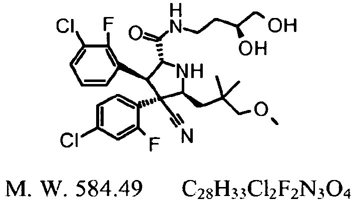

In a manner similar to the method described in **Examples 42c and 42d**, rac-(2R,3S,4R,5S)-3-(3-chloro-2-fluoro-phenyl)-4-(4-chloro-2-fluoro-phenyl)-4-cyano-5-(3-methoxy-2,2-dimethylpropyl)-pyrrolidine-2-carboxylic acid trifluoroacetic acid prepared in **Example 110c** (0.45 g, 0.74 mmol) was reacted with 2-((S)-2,2-dimethyl-[1,3]dioxolan-4-yl)-ethylamine (0.32 g, 2.2 mmol), HATU (0.5 g, 1.3 mmol) and iPr₂NEt (0.64 mL, 3.7 mmol) in CH₂Cl₂ at room temperature for 20 h, then reacted with aqueous HCl solution in tetrahydrofuran at room temperature for 2 h to give rac-(2R,3S,4R,5S)-3-(3-chloro-2-fluoro-phenyl)-4-(4-chloro-2-fluoro-phenyl)-4-cyano-5-(3-methoxy-2,2-dimethyl-propyl)-pyrrolidine-2-carboxylic acid ((S)-3,4-dihydroxy-butyl)-amide as a white solid (0.28 g, 65%).

HRMS (ES⁺) m/z Calcd for C₂₈H₃₃Cl₂F₂N₃O₄+ H [(M+H)⁺]: 584.1889, found: 584.1890.

### Example 110e

### Preparation of (2R,3S,4R,5S)-3-(3-chloro-2-fluoro-phenyl)-4-(4-chloro-2-fluoro-phenyl)-4-cyano-5-(3-methoxy-2,2-dimethyl-propyl)-pyrrolidine-2-carboxylic acid ((S)-3,4-dihydroxybutyl)-amide

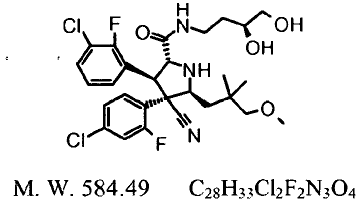

Rac-(2R,3S,4R,5S)-3-(3-chloro-2-fluoro-phenyl)-4-(4-chloro-2-fluoro-phenyl)-4-cyano-5-(3-methoxy-2,2-dimethyl-propyl)-pyrrolidine-2-carboxylic acid ((S)-3,4-dihydroxy-butyl)-amide (0.24 g) was separated by chiral SFC chromatography to provide chiral (2R,3S,4R,5S)-3-(3-chloro-2-fluoro-phenyl)-4-(4-chloro-2-fluoro-phenyl)-4-cyano-5-(3-methoxy-2,2-dimethylpropyl)-pyrrolidine-2-carboxylic acid ((S)-3,4-dihydroxy-butyl)-amide as a white solid (114 mg, 48%) and chiral (2S,3R,4S,5R)-3-(3-chloro-2-fluoro-phenyl)-4-(4-chloro-2-fluoro-phenyl)-4-cyano-5-(3-methoxy-2,2-dimethyl-propyl)-pyrrolidine-2-carboxylic acid ((S)-3,4-dihydroxybutyl)-amide as a white solid (114 mg, 48%).

HRMS (ES⁺) m/z Calcd for C₂₈H₃₃Cl₂F₂N₃O₄+ H [(M+H)⁺]: 584.1889, found: 584.1812.

### Example 111a

### Preparation of intermediate 3-(tert-butyl-dimethyl-silanyloxymethyl)-3-ethyl-pent-(E)-ylideneamino]-acetic acid tert-butyl ester

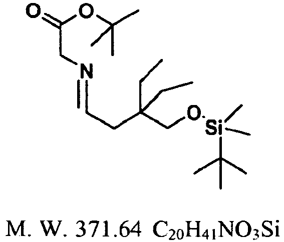

### Step A

A mixture of 2,2-diethyl-propane-1,3-diol (Aldrich) (5.5 g, 40 mmol) in anhydrous ethyl ether (100 mL) at 0 °C was added thionyl chloride (10.6 g, 90 mmol). The reactiom mixture was stirred at 0 °C for 2 h. Water was added. The organic layer was separated, the aqueous layer was then extracted with ethyl ether. The combined organic layers wer washed with saturated aqueous NaHCO₃ solution, brine, dried over MgSO₄, concentrated to give 5,5-dicthyl-[1,3,2]dioxathiane 2-oxide as a colorless oil (7 g, 98%).

### Step B

To the solution of 5,5-diethyl-[1,3,2]dioxathiane 2-oxide (7 g, 39 mmol) in anhydrous dimethyl sulfoxide (40 mL) was added NaCN (3.9 g, 80mmol). The reaction mixture was heated at 120 °C for 20 h. The mixture was cooled, and water was added. The mixture was extracted with ethyl acetate twice. The combined organic layers were washed with saturated aqueous NaHCO₃ solution, brine, dried over MgSO₄, and concentrated. The residue was purified by chromatography (EtOAc:hexanes = 1;2) to give 3-ethyl-3-hydroxymethyl-pentanenitrile as a yellow oil (1.7 g, 31%).

### Step C

To the solution of 3-ethyl-3-hydroxymethyl-pentanenitrile (1.7 g, 12 mmol) and imidazole (1.2 g, 18 mmol) in dichloromethane (80 mL) was added tert-butyldimethylchlorosilane (2 g, 13 mmol). The reactiom mixture was stirred at room temperature for 2 h. Water was added. The organic layer was separated, the aqueous layer was then extracted with dichloromethane. The combined organic layers wer washed with brine, dried over MgSO₄, concentrated to give 3-(tert-butyldimethyl-silanyloxymethyl)-3-ethyl-pentanenitrile as a colorless oil (2.28 g, 74%).

### Step D

To a solution of 3-(tert-butyl-dimethyl-silanyloxymethyl)-3-ethyl-pentanenitrile (2.28 g, 8.9 mmol) in dichloromethane (20 mL) at -78 °C was added a toluene solution (1 M) of DIBAL (9.8 mL, 9.8 mmol) dropwise. The reaction mixture was stirred at 0 °C for 1 h. The mixture was poured into aqueous saturated NH₄Cl solution. The organic layer was separated, and the aqueous layer was extracted with ethyl acetate. The organic layers were combined, washed with brine, dried over MgSO₄, and concentrated to give 3-(tert-butyl-dimethyl-silanyloxymethyl)-3-ethyl-pentanal as a colorless oil (Yield: 1.5 g, 65%).

### Step E

In a manner similar to the method described in **Example 1a**, glycine tert-butyl ester (0.78 g, 5.8 mmol) was reacted with 3-(tert-butyl-dimethyl-silanyloxymethyl)-3-ethyl-pentanal (1.5 g, 5.8 mmol) in CH₂Cl₂ at room temperature for 18 h to give [4-methoxy-3,3-dimethyl-but-(E)-ylideneamino]-acetic acid tert-butyl ester as a colorless oil (2.2 g, 100%).

### Example 111b

### Preparation of intermediate rac-(2R,3S,4R,5S)-5-[2-(tert-butyl-dimethyl-silanyloxymethyl)-2-ethyl-butyl]-3-(3-chloro-2-fluoro-phenyl)-4-(4-chloro-2-fluoro-phenyl)-4-cyano-pyrrolidine-2-carboxylic acid tert-butyl ester

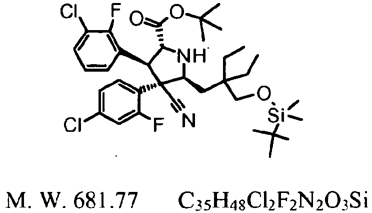

In a manner similar to the method described in **Example 100b**, give [4-(tert-butyl-dimethyl-silanyloxy)-3,3-dimethyl-but-(E)-ylideneamino]-acetic acid tert-butyl ester prepared in **Example 111a** (2.2 g, 5.8 mmol) was reacted with (Z)-3-(3-chloro-2-fluoro-phenyl)-2-(4-chloro-2-fluoro-phenyl)-acrylonitrile (1.43 g, 4.6 mmol) prepared in **Example 52a**, AgF (0.89 g, 7 mmol), and triethylamine (1.6 mL, 12 mmol) in dichloromethane (100 mL) at room temperature for 18 h, followed by the reaction with DBU (7 ml) in tert-butanol (20 mL) at 100 °C for 2 h to give rac-(2R,35,4R,5S)-5-[2-(tert-butyl-dimethl-silanyloxymethyl)-2-ethyl-butyl]-3-(3-chloro-2-fluoro-phenyl)-4-(4-chloro-2-fluoro-phenyl)-4-cyano-pyrrolidine-2-carboxylic acid tert-butyl ester as a light yellow solid (1.8 g, 58%).

### Example 111c

### Preparation of intermediate rac-(2R,3S,4R,5S)-3-(3-chloro-2-fluoro-phenyl)-4-(4-chloro-2-fluoro-phenyl)-4-cyano-5-(2-ethyl-2-hydroxymethyl-butyl)-pyrrolidine-2-carboxylic acid trifluoroacetic acid

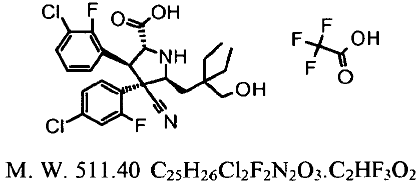

In a manner similar to the method described in **Example 25a**, rac-(2R,3S,4R,5S)-5-[2-(tert-butyl-dimethyl-silanyloxymethyl)-2-ethyl-butyl]-3-(3-chloro-2-fluoro-phenyl)-4-(4-chloro-2-fluoro-phenyl)-4-cyano-pyrrolidine-2-carboxylic acid tert-butyl ester prepared in **Example 111b** (1.8 g, 2.6 mmol) was reacted with trifluoroacetic acid in dichloromethane at room temperature to give rac-(2R,3S,4R,5S)-3-(3-chloro-2-fluoro-phenyl)-4-(4-chloro-2-fluoro-phenyl)-4-cyano-5-(2-ethyl-2-hydroxymethyl-butyl)-pyrrolidine-2-carboxylic acid trifluoroacetic acid as a light yellow solid (1.5 g, 94%).

### Example 111d

### Preparation of rac-(2R,35,4R,5S)-3-(3-chloro-2-fluoro-phenyl)-4-(4-chloro-2-fluoro-phenyl)-4-cyano-5-(2-ethyl-2-hydroxymethyl-butyl)-pyrrolidine-2-carboxylic acid ((S)-3,4-dihydroxybutyl)-amide

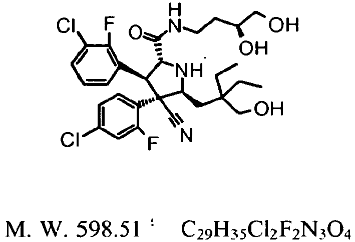

In a manner similar to the method described in **Examples 42c and 42d**, rac-(2R,3S,4R,5S)-3-(3-chloro-2-fluoro-phenyl)-4-(4-chloro-2-fluoro-phenyl)-4-cyano-5-(2-ethyl-2-hydroxymethyl-butyl)-pyrrolidine-2-carboxylic acid trifluoroacetic acid prepared in **Example 111c** (1.1 g, 1.8 mmol) was reacted with 2-((S)-2,2-dimethyl-[1,3]dioxolan-4-yl)-ethylamine (0.78 g, 5 mmol), HATU (1.2 g, 3 mmol) and iPr₂NEt (1.6 mL, 9 mmol) in CH₂Cl₂ at room temperature for 20 h, then reacted with aqueous HCl solution in tetrahydrofuran at room temperature for 2 h to give rac-(2R,3S,4R,5S)-3-(3-chloro-2-fluoro-phenyl)-4-(4-chloro-2-fluoro-phenyl)-4-cyano-5-(2-ethyl-2-hydroxymethyl-butyl)-pyrrolidine-2-carboxylic acid ((S)-3,4-dihydroxy-butyl)-amide as a white solid (0.11 g, 10%).

HRMS (ES⁺) m/z Calcd for C₂₉H₃₅Cl₂F₂N₃O₄+H[(M+H)⁺]: 598.2046, found: 598.2045.

### Example 112a

### Preparation of intermediate [2-(3-methyl-oxetan-3-yl)-eth-(E)-ylideneamino]-acetic acid tert-butyl ester

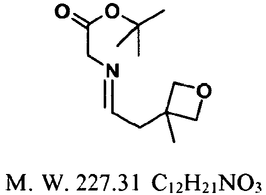

### Step A

To a solution of 3-methyl-3-oxetanemethanol (Aldrich) (3.5 g, 34 mmol) and triethylamine (10 g, 103 mmol) in dichloromethane (100 mL) at 0 °C was added a dichlormethane solution (20 mL) of methanesulfonyl chloride (Aldrich) (5.08 g, 45 mmol). The reaction mixture was stirred at 0 °C for 2 h. Water was added. Organic layer was separated, the aqueous layer was extracted with dichlormethane. The combined organic layers were washed with diluted aqueous HCl solution, saturated aqueous NaHCO₃ solution, brine, dried over MgSO₄, and concentrated to give methanesulfonic acid 3-methyl-oxetan-3-ylmethyl ester as a yellow oil (6 g, 97%).

### Step B

To the solution of methanesulfonic acid 3-methyl-oxetan-3-ylmethyl ester (6 g, 33 mmol)) in anhydrous dimethyl sulfoxide (30 mL) was added NaCN (3.2 g, 67 mmol). The reaction mixture was heated at 130 °C for 3 h. The mixture was cooled, and water was added. The mixture was extracted with ethyl acetate twice. The combined organic layers were washed with saturated aqueous NaHCO₃ solution, brine, dried over MgSO₄, and concentrated to give (3-methyl-oxetan-3-yl)-acetonitrile as a yellow oil (2.5 g, 68%).

### Step C

To a solution of(3-methyl-oxetan-3-yl)-acetonitrile (2.5 g, 22.5 mmol) in dichloromethane (30 mL) at -78 °C was added a toluene solution (1 M) of DIBAL (24.7 mL, 24.7 mmol) dropwise. The reaction mixture was stirred at 0 °C for 3 h. The mixture was poured into aqueous saturated NH₄Cl solution. The organic layer was separated, and the aqueous layer was extracted with ethyl acetate. The organic layers were combined, washed with brine, dried over MgSO₄, and concentrated to give (3-methyl-oxetan-3-yl)-acetaldehyde as a colorless oil (Yield: 0.8 g, 31%).

### Step D

In a manner similar to the method described in **Example 1a,** glycine tert-butyl ester (0.92 g, 7 mmol) was reacted with (3-methyl-oxetan-3-yl)-acetaldehyde (0.8 g, 7 mmol) in CH₂Cl₂ at room temperature for 18 h to give [2-(3-methyl-oxetan-3-yl)-eth-(E)-ylideneamino]-acetic acid tert-butyl ester
as a colorless oil (1.6 g, 100%).

### Example 112b

### Preparation of intermediate rac-(2R,3S,4R,5S)-3-(3-chloro-2-fluoro-phenyl)-4-(4-chloro-2-fluoro-phenyl)-4-cyano-5-(3-methyl-oxetan-3-ylmethyl)-pyrrolidine-2-carboxylic acid tert-butyl ester

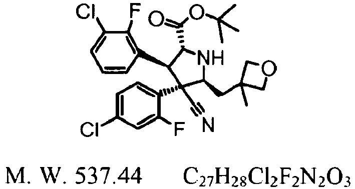

In a manner similar to the method described in **Example 100b**[2-(3-methyl-oxetan-3-yl)-eth-(E)-ylideneamino]-acetic acid tert-butyl ester prepared in **Example 112a** (1.6 g, 7 mmol) was reacted with (Z)-3-(3-chloro-2-fluoro-phenyl)-2-(4-chloro-2-fluoro-phenyl)-acrylonitrile (1.7 g, 5.6 mmol) prepared in **Example 52a**, AgF (1.1 g, 8.5 mmol), and triethylamine (1.9 mL, 14 mmol) in dichloromethane (100 mL) at room temperature for 18 h, followed by the reaction with DBU (6.7 ml) in tert-butanol (20 mL) at 100 °C for 2 h to rac-(2R,3S,4R,5S)-3-(3-chloro-2-fluoro-phenyl)-4-(4-chloro-2-fluoro-phenyl)-4-cyano-5-(3-methyl-oxetan-3-ylmethyl)-pyrrolidine-2-carboxylic acid tert-butyl ester as a light yellow gum (1.0 g, 33%).

### Example 112c

### Preparation of intermediate rac-(2R,3S,4R,5S)-3-(3-chloro-2-fluoro-phenyl)-4-(4-chloro-2-fluoro-phenyl)-4-cyano-5-(3-methyl-oxetan-3-ylmethyl)-pyrrolidine-2-carboxylic acid trifluoroacetic acid

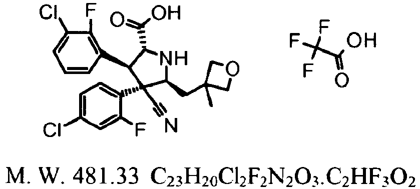

In a manner similar to the method described in **Example 25a**, rac-(2R,3S,4R,5S)-3-(3-chloro-2-fluoro-phenyl)-4-(4-chloro-2-fluoro-phenyl)-4-cyano-5-(3-methyl-oxetan-3-ylmethyl)-pyrrolidine-2-carboxylic acid tert-butyl ester prepared in **Example 112b** (1.0 g, 1.9 mmol) was reacted with trifluoroacetic acid in dichloromethane at room temperature to give rac-(2R,3S,4R,5S)-3-(3-chloro-2-fluoro-phenyl)-4-(4-chloro-2-fluoro-phenyl)-4-cyano-5-(3-methyl-oxetan-3-ylmethyl)-pyrrolidine-2-carboxylic acid trifluoroacetic acid as a white solid (1 g, 91%).

### Example 112d

### Preparation of rac-(2R,35,4R,5S)-3-(3-chloro-2-fluoro-phenyl)-4-(4-chloro-2-fluoro-phenyl)-4-cyano-5-(3-methyl-oxetan-3-ylmethyl)-pyrrolidine-2-carboxylic acid ((S)-3,4-dihydroxy-butyl)-amide

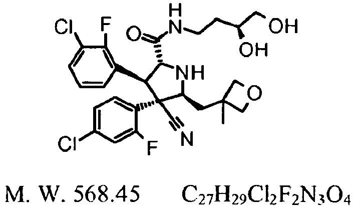

In a manner similar to the method described in **Examples 42c and 42d**, rac-(2R,3S,4R,5S)-3-(3-chloro-2-fluoro-phenyl)-4-(4-chloro-2-fluoro-phenyl)-4-cyano-5-(3-methyl-oxetan-3-ylmethyl)-pyrrolidine-2-carboxylic acid trifluoroacetic acid prepared in **Example 112c** (0.5 g, 0.84 mmol) was reacted with 2-((S)-2,2-dimethyl-[1,3]dioxolan-4-yl)-ethylamine (0.36 g, 2.5 mmol), HATU (0.57 g, 1.5 mmol) and iPr₂NEt (0.73 mL, 4.2 mmol) in CH₂Cl₂ at room temperature for 20 h, then reacted with aqueous HCl solution in tetrahydrofuran at room temperature for 2 h to give rac-(2R,3S,4R,5S)-3-(3-chloro-2-fluoro-phenyl)-4-(4-chloro-2-fluoro-phenyl)-4-cyano-5-(3-methyl-oxetan-3-ylmethyl)-pyrrolidine-2-carboxylic acid ((S)-3,4-dihydroxy-butyl)-amide as a white solid (0.23 g, 48%).

HRMS (ES⁺) m/z Calcd for C₂₇H₂₉Cl₂F₂N₃O₄+ H [(M+H)⁺]: 568.1576, found: 565.1579.

### Example 113a

### Preparation of intermediate [2-(3-ethyl-oxetan-3-yl)-eth-(E)-ylideneamino]-acetic acid tert-butyl ester

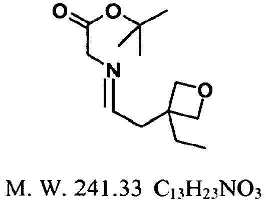

### Step A

In a manner similar to the methods described in **Example 112a Step A**. **Step B., and Step C**., 3-ethyl-3-oxetanemethanol (TCI-US) (3.5 g, 30 mmol) was reacted with triethylamine (6.6 g, 60 mmol) and treated with NaCN (2.2 g, 46mmol) in anhydrous dimethyl sulfoxide at 130 °C, followed by the reaction with DIBAL (1 M in heptane, 27 ml, 27 mmol) in dichloromethane at 0 °C to give (3-ethyl-oxetan-3-yl)-acetaldehyde as a light yellow oil (Yield: 2.5 g, 26% for three steps).

### Step B

In a manner similar to the method described in **Example 1a**, glycine tert-butyl ester (1 g, 7.8 mmol) was reacted with (3-ethyl-oxetan-3-yl)-acetaldehyde (1 g, 7.8 mmol) in CH₂Cl₂ at room temperature for 18 h to give [2-(3-ethyl-oxetan-3-yl)-eth-(E)-ylideneamino]-acetic acid tert-butyl ester as a yellow oil (1.9 g, 100%).

### Example 113b

### Preparation of intermediate rac-(2R,3S,4R,5S)-3-(3-chloro-2-fluoro-phenyl)-4-(4-chloro-2-fluoro-phenyl)-4-cyano-5-(3-ethyl-oxetan-3-ylmethyl)-pyrrolidine-2-carboxylic acid tert-butyl ester

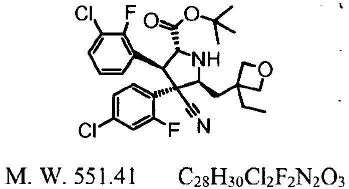

In a manner similar to the method described in **Example 100b**, [2-(3-ethyl-oxetan-3-yl)-eth-(E)-ylideneamino]-acetic acid tert-butyl ester prepared in **Example 113a** (1.9 g, 7.8 mmol) was reacted with (Z)-3-(3-chloro-2-fluoro-phenyl)-2-(4-chloro-2-fluoro-phenyl)-acrylonitrile (1.9 g, 6.2 mmol) prepared in **Example 52a**, AgF (1.2 g, 9.5 mmol), and triethylamine (2.2 mL, 16 mmol) in dichloromethane (100 mL) at room temperature for 18 h, followed by the reaction with DBU (7.5 ml) in tert-butanol (10 mL) at 100 °C for 2 h to give rac-(2R,3S,4R,5S)-3-(3-chloro-2-fluoro-phenyl)-4-(4-chloro-2-fluoro-phenyl)-4-cyano-5-(3-ethyl-oxetan-3-ylmethyl)-pyrrolidine-2-carboxylic acid tert-butyl ester
as a white solid (2 g, 58%).

### Example 113c

### Preparation of intermediate rac-(2R,3S,4R,5S)-3-(3-chloro-2-fluoro-phenyl)-4-(4-chloro-2-fluoro-phenyl)-4-cyano-5-(3-ethyl-oxetan-3-ylmethyl)-pyrrolidine-2-carboxylic acid trifluoroacetic acid

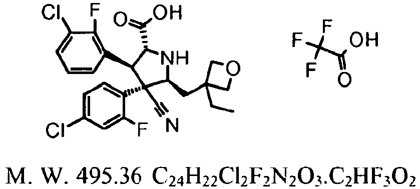

In a manner similar to the method described in **Example 25a**, rac-(2R,3S,4R,5S)-3-(3-chloro-2-fluoro-phenyl)-4-(4-chloro-2-fluoro-phenyl)-4-cyano-5-(3-ethyl-oxetan-3-ylmethyl)-pyrrolidine-2-carboxylic acid tert-butyl ester prepared in **Example 113b** (2 g, 3.6 mmol) was reacted with trifluoroacetic acid in dichloromethane at room temperature to give rac-(2R,3S,4R,5S)-3-(3-chloro-2-fluoro-phenyl)-4-(4-chloro-2-fluoro-phenyl)-4-cyano-5-(3-ethyl-oxetan-3-ylmethyl)-pyrrolidine-2-carboxylic acid trifluoroacetic acid as a white solid (2 g, 91%).

### Example 113d

### Preparation of rac-(2R,3S,4R,5S)-3-(3-chloro-2-fluoro-phenyl)-4-(4-chloro-2-fluoro-phenyl)-4-cyano-5-(3-ethyl-oxetan-3-ylmethyl)-pyrrolidine-2-carboxylic acid ((S),-3,4-dihydroxy-butyl)-amide

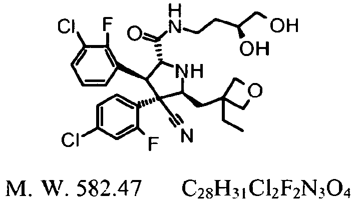

In a manner similar to the method described in **Examples 42c and 42d** rac-(2R,3S,4R,5S)-3-(3-chloro-2-fluoro-phenyl)-4-(4-chloro-2-fluoro-phenyl)-4-cyano-5-(3-ethyl-oxetan-3-ylmethyl)-pyrrolidine-2-carboxylic acid trifluoroacetic acid prepared in **Example 105c** (1 g, 1.6 mmol) was reacted with 2-((S)-2,2-dimethyl-[1,3]dioxolan-4-yl)-ethylamine (0.7 g, 5 mmol), HATU (1.1 g, 3 mmol) and iPr₂NEt (1.4 mL, 8 mmol) in CH₂Cl₂ at room temperature for 20 h, then reacted with aqueous HCl solution in tetrahydrofuran at room temperature for 2 h to give rac-(2R,3S,4R,5S)-3-(3-chloro-2-fluoro-phenyl)-4-(4-chloro-2-fluoro-phenyl)-4-cyano-5-(3-ethy)-oxetan-3-ylmethyl)-pyrrolidine-2-carboxylic acid ((S)-3,4-dihydroxy-butyl)-amide as a white solid (0.56 g, 58%).

HRMS (ES⁺) m/z Calcd for C₂₈H₃₁Cl₂F₂N₃O₄ + H [(M+H)⁺]: 582.1733, found: 582.1732.

### Example 114a

### Preparation of intermediate [2-[1-(tert-tutyl-dimethyl-silanyloxymethyl)-cyclopropyl]-eth-(E)-ylideneamino]-acetic acid tert-butyl ester

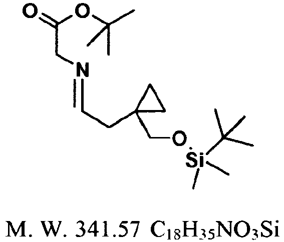

### Step A

In a manner similar to the methods described in **Example 111a Step A. Step B., Step C., and Step D.,** 1,1-bis(hydroxymethyl)-cyclopropane (Aldrich) (4 g, 39 mmol) was reacted with thionyl chloride (14 g, 126 mmol) in anhydrous ethyl ether at 0 °C, then reacted with NaCN (2.4 g, 49 mmol) in anhydrous dimethyl sulfoxide 120 °C for 18 h, then treated with tert-butyldimethylchlorosilane (1.4 g, 9 mmol) and imidazole (0.85 g, 13 mmol) in dichloromethane at room temperature, follone by the reaction with DIBAL (1 M in heptane, 8.3 mL, 8.3 mmol) at 0 °C to give [1-(tert-butyl-dimethyl-silanyloxymethyl)-cyclopropyl]-acetaldehyde as a colorless oil (Yield: 1.3 g, 15% for four steps).

### Step B

In a manner similar to the method described in **Example 1a**, glycine tert-butyl ester (0.75 g, 5.7 mmol) was reacted with [1-(tert-butyl-dimethyl-silanyloxym ethyl)-cyclopropyl]-acetaldehyde (1.3 g, 5.7 mmol) in CH₂Cl₂ at room temperature for 18 h to give [2-[1-(tert-tutyl-dimethyl-silanyloxymethyl)-cyclopropyl]-eth-(E)-ylideneamino]-acetic acid tert-butyl ester as a colorless oil (1.9 g, 100%).

### Example 114b

### Preparation of intermediate rac-(2R,3S,4R,5S)-3-(3-chloro-2-fluoro-phenyl)-4-(4-chloro-2-fluoro-phenyl)-4-cyano-5-(1-hydroxymethyl-cyclopropylmethyl)-pyrrolidine-2-carboxylic acid tert-butyl ester

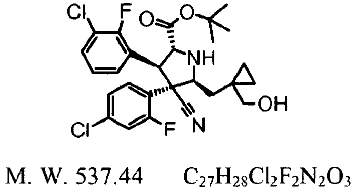

In a manner similar to the method described in **Example 100b**, [2-[1-(tert-tutyl-dimethyl-silanyloxymethyl)-cyclopropyl]-eth-(E)-ylideneamino]-acetic acid tert-butyl ester prepared in **Example 114a** (1.9 g, 5.7 mmol) was reached with (Z)-3-(3-chloro-2-fluoro-phenyl)-2-(4-chloro-2-fluoro-phenyl)-acrylonitrile (1.4 g, 4.6 mmol) prepared in **Example 52a**, AgF (0.89 g, 7.1 mmol), and triethylamine (1.6 mL, 12 mmol) in dichloromethane (100 mL) at room temperature for 18 h, followed by the reaction with DBU (5 ml) in tert-butanol (20 mL) at 100 °C for 2 h to give rac-(2R,3S,4R,5S)-3-(3-chloro-2-fluoro-phenyl)-4-(4-chloro-2-fluoro-phenyl)-4-cyano-5-(1-hydroxymethyl-cyclopropylmethyl)-pyrrolidine-2-carboxylic acid tert-butyl ester as a white solid (0.4 g, 30%).

### Example 114c

### Preparation of intermediate rac-(2R,3S,4R,5S)-3-(3-chloro-2-fluoro-phenyl)-4-(4-chloro-2-fluoro-phenyl)-4-cyano-5-(1-hydroxymethyl-cyclopropylmethyl)-pyrrolidine-2-carboxylic acid trifluoroacetic acid

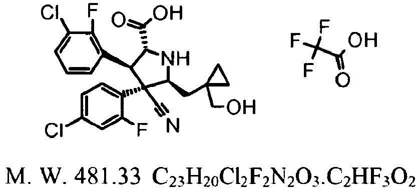

In a manner similar to the method described in **Example 25a**, rac-(2R,3S,4R,5S)-3-(3-chloro-2-fluoro-phenyl)-4-(4-chloro-2-fluoro-phenyl)-4-cyano-5-(1-hydroxymethyl-cyclopropylmethyl)-pyrrolidine-2-carboxylic acid tert-butyl ester prepared in **Example 114b** (0.4 g, 0.74 mmol) was reacted with trifluoroacetic acid in dichloromethane at room temperature to give rac-(2R,3S,4R,5S)-3-(3-chloro-2-fluoro-phenyl)-4-(4-chloro-2-fluoro-phenyl)-4-cyano-5-(1-hydroxymethyl-cyclopropylmethyl)-pyrrolidine-2-carboxylic acid trifluoroacetic acid as a white solid (0.4 g, 91 %).

### Example 114d

### Preparation of rac-(2R,3S,4R,5S)-3-(3-chloro-2-fluoro-phenyl)-4-(4-chloro-2-fluoro-phenyl)-4-cyan-5-(1-hydroxymethyl-cyclopropylmethyl)-pyrrolidine-2-carboxylic acid ((S)-3,4-dihydroxy-butyl)-amide

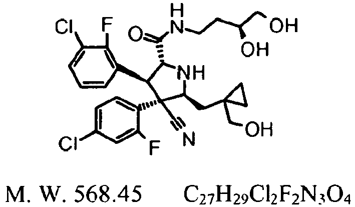

In a manner similar to the method described in **Examples 42c and 42d**, rac-(2R,3S,4R,5S)-3-(3-chloro-2-fluoro-phenyl)-4-(4-chloro-2-fluoro-phenyl)-4-cyano-5-(1-hydroxymethyl-cyclopropylmethyl)-pyrrolidine-2-carboxylic acid trifluoroacetic acid prepared in **Example 114c** (0.4 g, 0.67 mmol) was reacted with 2-((S)-2,2-dimethyl-[1,3]dioxolan-4-yl)-ethylamine (0.29 g, 2 mmol), HATU (0.46 g, 1.2 mmol) and iPr₂NEt (0.58 mL, 3.4 mmol) in CH₂Cl₂ at room temperature for 20 h, then reacted with aqueous HCl solution in tetrahydrofuran at room temperature for 2 h to give rac-(2R,3S,4R,5S)-3-(3-chloro-2-fluoro-phenyl)-4-(4-chloro-2-fluoro-phenyl)-4-cyano-5-(1-hydroxymethyl-cyclopropylmethyl)-pyrrolidine-2-carboxylic acid ((S)-3,4-dihydroxy-butyl)-amide as a white solid (0.15 g, 40%).

HRMS (ES⁺) m/z Calcd for C₂₇H₂₉Cl₂F₂N₃O₄+ H [(M+H⁺]: 568.1576, found: 568.1578,

### Example 114e

### Preparation of (2R,3S,4R,5S)-3-(3-chloro-2-fluoro-phenyl)-4-(4-chloro-2-fluoro-phenyl)-4-cyano-5-(1-hydroxymethyl-cyclopropylmethyl)-pyrrolidine-2-carboxylic acid ((S)-3,4-dihydroxy-butyl)-amide

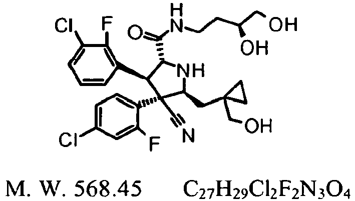

Rac-(2R,3S,4R,5S)-3-(3-chloro-2-fluoro-phenyl)-4-(4-chloro-2-fluoro-phenyl)-4-cyano-5-(1-hydroxymethyl-cyclopropylmethyl)-pyrrolidine-2-carboxylic acid ((S)-3,4-dihydroxy-butyl)-amide (0.12 g) was separated by chiral SFC chromatography to provide chiral (2R,3S,4R,5S)-3-(3-chloro-2-fluoro-phenyl)-4-(4-chloro-2-fluoro-phenyl)-4-cyano-5-(1-hydroxymethyl-cyclopropylmethyl)-pyrrolidine-2-carboxylic acid ((S)-3,4-dihydroxy-butyl)-amide as a white solid (46 mg, 38%) and chiral (2S,3R,4S,5R)-3-(3-chloro-2-fluoro-phenyl)-4-(4-chloro-2-fluoro-phenyl)-4-cyano-5-(1-hydroxymethyl-cyclopropylmethyl)-pyrrolidine-2-carboxylic acid ((S)-3,4-dihydroxy-butyl)-amide as a white solid (48 mg, 40%).

HRMS (ES⁺) m/z Calcd for C₂₇H₂₉Cl₂F₂N₃O₄+ H [(M+H)⁺]: 568.1576, found: 568.1578. 578.

### Example 115a

### Preparation of intermediate [2-[1-(tert-tutyl-dimethyl-silanyloxymethyl)-cyclobutyl]-eth-(E)-ylideneamino]-acetic acid tert-butyl ester

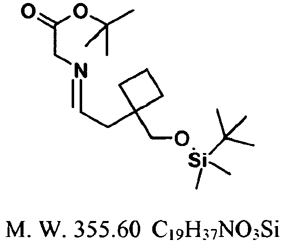

### Step A

In a manner similar to the methods described in **Example 111a Step A. Step B., Step C., and Step D.**, 1,1-bis(hydroxymethyl)-cyclobutane (Waterstone) (3.8 g, 33 mmol) was reacted with thionyl chloride (8 g, 72 mmol) in anhydrous ethyl ether at 0 °C, then reacted with NaCN (2 g, 41 mmol) in anhydrous dimethyl sulfoxide 120 °C for 18 h, then treated with tert-butyldimethylchlorosilane (1 g, 6 mmol) and imidazole (1 g, 15 mmol) in dichloromethane at room temperature, follone by the reaction with DIBAL (1 M in heptane, 6.4 mL, 6.4 mmol) at 0 °C to give [1-(tert-butyl-dimethyl-silanyloxym ethyl)-cyclobutyl]-acetaldehyde as a colorless oil (Yield: 0.48 g, 6% for four steps).

### Step B

In a manner similar to the method described in **Example 1a**, glycine tert-butyl ester (0.26 g, 2 mmol) was reacted with [1-(tert-butyl-dimethyl-silanyloxym ethyl)-cyclobutyl]-acetaldehyde (0.48 g,2 mmol) in CH₂Cl₂ at room temperature for 18 h to give [2-[1-(tert-tutyl-dimethyl-silanyloxymethyl)-cyclopropyl]-eth-(E)-ylideneamino]-acetic acid tert-butyl ester as a colorless oil (0.71 g, 100%).

### Example 115b

### Preparation of intermediate rac-(2R,3S,4R,5S)-5-[1-(tert-butyl-dimethyl-silanyloxymethyl)-cyclobutylmethyl]-3-(3-chloro-2-fluoro-phenyl)-4-(4-chloro-2-fluoro-phenyl)-4-cyanopyrrolidine-2-carboxylic acid tert-butyl ester

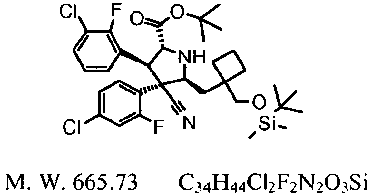

In a manner similar to the method described in **Example 100b**, [2-[1-(tert-tutyl-dimethyl-silanyloxymethyl)-cyclopropyl]-eth-(E)-ylideneamino]-acetic acid tert-butyl ester prepared in **Example 115a** (0.71 g, 2 mmol) was reacted with (Z)-3-(3-chloro-2-fluoro-phenyl)-2-(4-chloro-2-fluoro-phenyl)-acrylonitrile (0.49 g, 1.6 mmol) prepared in **Example 52a,** AgF (0.3 g, 2.4 mmol), and triethylamine (0.55 mL, 4 mmol) in dichloromethane (100 mL) at room temperature for 18 h, followed by the reaction with DBU (1 ml) in tert-butanol (15 mL) at 100 °C for 2 h to give rac-(2R,3S,4R,5S)-5-[1-(tert-butyl-dimethyl-silanyloxymethyl)-cyclobutylmethyl]-3-(3-chloro-2-fluoro-phenyl)-4-(4-chloro-2-fluoro-phenyl)-4-cyano-pyrrolidine-2-carboxylic acid tert-butyl ester as a yellow gum (0.7 g, 67%).

### Example 115c

### Preparation of intermediate rac-(2R,3S,4R,5S)-3-(3-chloro-2-fluoro-phenyl)-4-(4-chloro-2-fluoro-phenyl)-4-cyano-5-(1-hydroxymethyl-cyclobutylmethyl)-pyrrolidine-2-carboxylic acid trifluoroacetic acid

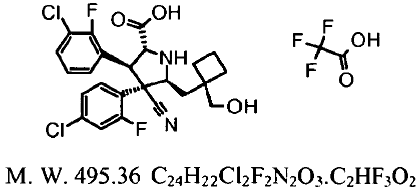

In a manner similar to the method described in **Example 25a**, rac-(2R,3S,4R,5S)-5-[1-(tert-butyl-dimethyl-silanyloxymethyl)-cyclobutylmethyl]-3-(3-chloro-2-fluoro-phenyl)-4-(4-chloro-2-fluoro-phenyl)-4-cyano-pyrrolidine-2-carboxylic acid tert-butyl ester prepared in **Example 115b** (0.7 g, 1 mmol) was reacted with trifluoroacetic acid in dichloromethane at room temperature to give rac-(2R,3S,4R,5S)-3-(3-chloro-2-fluoro-phenyl)-4-(4-chloro-2-fluoro-phenyl)-4-cyano-5-(1-hydroxymethyl-cyclobutylmethyl)-pyrolidine-2-carboxylic acid trifluoroacetic acid as a white solid (0.6 g, 100%).

### Example 115d

### Preparation of rac-(2R,3S,4R,5S)-3-(3-chloro-2-fluoro-phenyl)-4-(4-chloro-2-fluoro-phenyl)-4-cyano-5-(1-hydroxymethyl-cyclobutylmethyl)-pyrrolidine-2-carboxylic acid ((S)-3,4-dihydroxybutyl)-amide

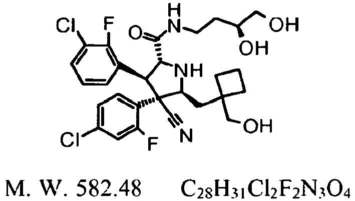

In a manner similar to the method described in **Examples 42c and 42d**, rac-(2R,3S,4R,5S)-3-(3-chloro-2-fluoro-phenyl)-4-(4-chloro-2-fluoro-phenyl)-4-cyano-5-(1-hydroxymethyl-cyclobutylmethyl)-pyrrolidine-2-carboxylic acid trifluoroacetic acid prepared in **Example 115c** (0.6 g, 1 mmol) was reacted with 2-((S)-2,2-dimethyl-[1,3]dioxolan-4-yl)-ethylamine (0.43 g, 3 mmol), HATU (0.67 g, 1.8 mmol) and iPr₂NEt (0.86 mL, 4.9 mmol) in CH₂Cl₂ at room temperature for 20 h, then reacted with aqueous HCl solution in tetrahydrofuran at room temperature for 2 h to give rac-(2R,3S,4R,5S)-3-(3-chloro-2-fluoro-phenyl)-4-(4-chloro-2-fluoro-phenyl)-4-cyano-5-(1-hydroxymethyl-cyclobutylmethyl)-pyrrolidinl-2-carboxylic acid ((S)-3,4-dihydroxy-butyl)-amide as a white solid (0.32 g, 56%).

HRMS (ES⁺) m/z Calcd for C₂₈H₃₁Cl₂F₂N₃O₄+ H [(M+H)⁺]: 582.1733, found: 582.1733.

### Example 115e

### Preparation of (2R,3S,4R,5S)-3-(3-chloro-2-fluoro-phenyl)-4-(4-chloro-2-fluoro-phenyl)-4-cyano-5-(1-hydroxymethyl-cyclobutylmethyl)-pyrrolidine-2-carboxylic acid ((S)-3,4-dihydroxybutyl)-amide

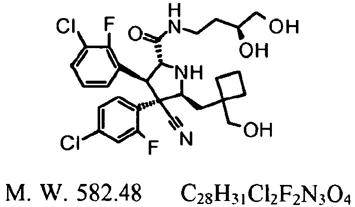

Rac-(2R,3S,4R,5S)-3-(3-chloro-2-fluoro-phenyl)-4-(4-chloro-2-fluoro-phenyl)-4-cyano-5-(1-hydroxymethyl-cyclobutylmethyl)-pyrrolidine-2-carboxylic acid ((S)-3,4-dihydroxy-butyl)-amide (0.25 g) was separated by chiral SFC chromatography to provide chiral (2R,3S,4R,5S)-3-(3-chloro-2-fluoro-phenyl)-4-(4-chloro-2-fluoro-phenyl)-4-cyano-5-(1-hydroxymethyl-cyclobutylmethyl)-pyrrolidine-2-carboxylic acid ((S)-3,4-dihydroxy-butyl)-amide as a white solid (104 mg, 41%) and chiral (2S,3R,4S,5R)-3-(3-chloro-2-fluoro-phenyl)-4-(4-chloro-2-fluoro-phenyl)-4-cyano-5-(1-hydroxymethyl-cyclobutylmethyl)-pyrrolidine-2-carboxylic acid ((S)-3,4-dihydroxy-butyl)-amide as a white solid (103 mg, 41%).

HRMS (ES⁺) m/z Calcd for C₂₈H₃₁Cl₂F₂N₃O₄+ H [(M+H)⁺]: 582.1733, found: 5821733.

### Example 116a

### Preparation of intermediate [5-(tert-butyl-dimethyl-silanyloxy)-3,3-dimethyl-pent-(E)-ylideneamino]-acetic acid methyl ester

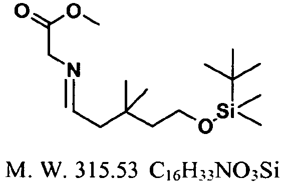

### Step A

To the solution of 3,3-dimethylglutaric acid (Aldrich) (5.1 g, 32 mmol) in anhydrous tetrahydrofuran (100 mL) at 0 °C was added a solution of BH₃.THF (1 M, 100 mL, 100 mmol). The reaction mixture was stirred at room temperature for 18 h. Aqueous HCl solution was added, and the mixture was extracted with ethyl acetate. The organic layer was separated, washed with saturated aqueous NaHCO₃ solution, brine, dried over MgSO₄, and concentrated. The residue was purified by chromatography (EtOAc) to give 3,3-dimethyl-pentane-1,5-diol as a colorless oil (1.5 g, 34%).

### Step B

A mixture of 3,3-dimethyl-pentane-1,5-diol (1.5 g, 11 mmol) and imidazole (1.4 g, 20 mmol) in dichloromethane (50 mL) was added tert-butyldimethylchlorosilane (1.7 g, 11 mmol). The reactiom mixture was stirred at room temperature for 2 h. Water was added. The organic layer was separated, the aqueous layer was then extracted with dichloromethane. The combined organic layers wer washed with brine, dried over MgSO₄, concentrated to give 5-(tert-butyldimethyl-silanyloxy)-3,3-dimethyl-pentan-1-ol as a colorless oil (2.7 g, 100%).

### Step C

To a solution ofoxalyl chloride (0.97 mL, 11 mmol) (Aldrich) in dichloromethane (20 mL) at - 78 °C was added the solution of dimethyl sulfoxide (1.6 mL, 22 mmol) in dichloromethane (5 mL) dropwise. After 5 mins, the solution of 5-(tert-butyl-dimethyl-silanyloxy)-3,3-dimethyl-pentan-1-ol (2.5 g, 10 mmol) in dichloromethane (10 mL) was added dropwise. The reaction mixture was stirred at -78 °C for 15 min. Triethylamine (5 mL, 36mmol) was added and the reaction mixture was slowly warmed up to room temperature and stirred at room temperature for 45 min. Then water was added. The organic layers were separated, and the aqueous layer was extracted with dichloromethane. The organic layers were combined, washed with 10% of HCl, saturated NaHCO₃, brine, dried over MgSO₄, and concentrated to give 5-(tert-butyl-dimethyl-silanyloxy)-3,3-dimethyl-pentanal as a light yellow oil (Yield: 1.75 g, 71%).

### Step D

In a manner similar to the method described in **Example 1a**, glycine methyl ester hydrochloride (0.9 g, 7.2 mmol) was reacted with 5-(tert-butyl-dimethyl-silanyloxy)-3,3-dimethyl-pentanal (1.75 g, 7.2 mmol) and triethylamine (1.49 mL, 11 mmol) in CH₂Cl₂ at room temperature for 18 h to give [4-(tert-butyl-dimethyl-silanyloxy)-3,3-dimethyl-but-(E)-ylideneamino]-acetic acid methyl ester as a colorless oil (2.3 g, 100%).

### Example 116b

### Preparation of intermediate rac-(2R,3S,4R,5S)-5-[4-(tert-tutyl-dimethyl-silanyloxy)-2,2-dimethyl-butyl]-3-(3-chloro-2-fluoro-phenyl)-4-(4-chloro-2-fluoro-phenyl)-4-cyano-pyrrolidine-2-carboxylic acid methyl ester

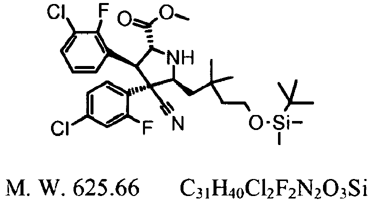

In a manner similar to the method described in **Example 1c**, [5-(tert-butyl-dimethyl-silanyloxy)-3,3-dimethyl-pent-(E)-ylideneamino]-acetic acid methyl ester prepared in **Example 116a** (6.4 mmol) was reached with (Z)-3-(3-chloro-2-fluoro-phenyl)-2-(4-chloro-2-fluoro-phenyl)-acrylonitrile (1.58 g, 5.1 mmol) prepared in **Example 52a**, AgF (1g, 7.8 mmol), and triethylamine (1.8 mL, 13 mmol) in dichloromethane (100 mL) at room temperature for 48 h to give rac-(2R,3S,4R,5S)-5-[4-(tert-tutyl-dimethyl-silanyloxy)-2,2-dimethyl-butyl]-3-(3-chloro-2-fluoro-phenyl)-4-(4-chloro-2-fluoro-phenyl)-4-cyano-pyrrolidine-2-carboxylic acid methyl ester as a yellow gum (1.6 g, 50%).

### Example 116c

### Preparation of intermediate rac-(2R,3S,4R,5S)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid

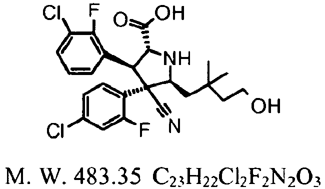

To rac-(2R,3S,4R,5S)-5-[4-(tert-tutyl-dimethyl-silanyloxy)-2,2-dimethyl-butyl]-3-(3-chloro-2-fluoro-phenyl)-4-(4-chloro-2-fluoro-phenyl)-4-cyano-pyrrolidine-2-carboxylic acid methyl ester prepared in **Example 116b** (0.7 g, 1.1 mmol) in tetrahydrofuran (10 mL) was added tetrahydrofuran solution (1 M, Aldrich) of TBAF (1.34 mL, 1.3 mmol). The reaction mixture was stirred at room temperature for 18 h. The mixture was concentrated, the residue was partitioned between ethyl acetate and water. The organic layer was separated, dried over MgSO₄, and concentrated. The residue was dissolved into tetrahydrofuran (10 mL), and an aqueous solution (1 M) of LiOH (10 mL, 10 mmol) was added. The reaction mixture was stirred at room temperature for 1 h. The "pH" of the mixture was adjusted to ∼4-5 by aqueous HCl solution. The mixture was concentrated. The residue was partitioned between ethyl acetate and water. The organic layer was separated, washed with water, brine, dried over MgSO₄, concentrated to give intermediate rac-(2R,3S,4R,5S)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid
as a light yellow solid (0.3 g, 54%)

### Example 116d

### Preparation of rac-(2R,3S,4R,5S)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid ((S)-3,4-dihydroxybutyl)-amide

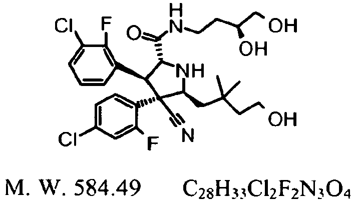

In a manner similar to the method described in **Examples 42c and 42d**, rac-(2R,3S,4R,5S)-3-(3-chloro-2-fluoro-phenyl)-4-(4-chloro-2-fluoro-phenyl)-4-cyano-5-(4-hydroxy-2,2-dimethylbutyl)-pyrrolidine-2-carboxylic acid
prepared in **Example 116c** (0.18 g, 0.36 mmol) was reacted with 2-((S)-2,2-dimethyl-[1,3]dioxolan-4-yl)-ethylamine (0.16 g, I mmol), HATU (0.25 g, 0.65 mmol) and iPr₂NEt (0.07 mL, 0.43 mmol) in CH₂Cl₂ at room temperature for 20 h, then reacted with aqueous HCl solution in tetrahydrofuran at room temperature for 2 h to give rac-(2R,3S,4R,5S)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid ((S)-3,4-dihydroxy-butyl)-amide as a white solid (0.1 g, 54%).

HRMS (ES⁺) m/z Calcd for C₂₈H₃₃Cl₂F₂N₃O₄+ H [(M+H)⁺] 584.1889, found: 584.1889.

### Example 116e

### Preparation of (2R,3S,4R,5S)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid ((S)-3,4-dihydroxybutyl)-amide

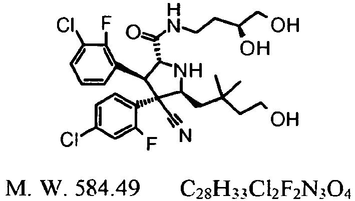

Rac-(2R,35,4R,5S)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid ((S)-3,4-dihydroxy-butyl)-amide(0.35 g) was separated by chiral SFC chromatography to provide chiral (2R,3S,4R,5S)-3-(3-chloro-2-fluoro-phenyl)-4-(4-chloro-2-fluoro-phenyl)-4-cyano-5-(4-hydroxy-2,2-dimethylbutyl)-pyrrolidine-2-carboxylic acid ((S)-3,4-dihydroxy-butyl)-amide as a white solid (157 mg, 45%) and chiral (2S,3R,4S,5R)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid ((S)-3,4-dihydroxybutyl)-amide as a white solid (155 mg, 44%).

HRMS (Es⁺) m/z Calcd for C₂₈H₃₃Cl₂F₂N₃O₄+ H [(M+H)⁺] 584.1889, found: 584.1891.

### Example 117a

### Preparation of intermediate [2-[1-(tert-butyl-dimethyl-silanyloxymethyl)-cyclohex-3-enyl]-eth-(E)-ylideneamino]-acetic acid tert-butyl ester

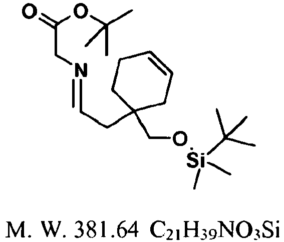

### Step A

In a manner similar to the methods described in **Example 111a Step A. Step B., Step C., and Step D.**, 3-cyclohexene-1,1-dimethanol (Aldrich) (5.3 g, 37 mmol) was reacted with thionyl chloride (15 g, 135 mmol) in anhydrous ethyl ether at 0 °C, then reacted with NaCN (3 g, 61 mmol) in anhydrous dimethyl sulfoxide 120 °C for 18 h, then treated with tert-butyldimethylchlorosilane (3.9 g, 26 mmol) and imidazole (2.4 g, 36 mmol) in dichloromethane at room temperature, follone by the reaction with DIBAL (1 M in heptane, 26 mL, 26 mmol) at 0 °C to give 1-(tert-butyl-dimethyl-silanyloxymethyl)-cyclohex-3-enecarbaldehyde as a light yellow oil (Yield: 6 g, 64% for four steps).

### Step B

In a manner similar to the method described in **Example 1a**, glycine tert-butyl ester (1.2 g, 9 mmol) was reacted with 1-(tert-butyl-dimethyl-silanyloxymethyl)-cyclohex-3-enecarbaldehyde (2.5 g, 9 mmol) in CH₂Cl₂ at room temperature for 18 h to give [2-[1-(tert-butyl-dimethyl-silanyloxymethyl)-cyclohex-3-enyl]-eth-(E)-ylideneamino]-acetic acid tert-butyl ester as a light yellow oil (3.5 g, 100%).

### Example 117b

### Preparation of intermediate rac-(2R,3S,4R,5S)-5-[1-(tert-butyl-dimethyl-silanyloxymethyl)-cyclohex-3-enylmethyl]-3-(3-chloro-2-fluoro-phenyl)-4-(4-chloro-2-fluoro-phenyl)-4-cyanopyrrolidine-2-carboxylic acid tert-butyl ester

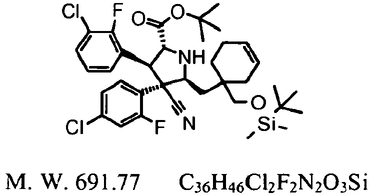

In a manner similar to the method described in **Example 100b**, [2-[1-(tert-butyl-dimethyl-silanyloxymethyl)-cyclohex-3-enyl]-eth-(E)-ylideneamino]-acetic acid tert-butyl ester prepared in **Example 117a** (3.5 g, 9 mmol) was reacted with (Z)-3-(3-chloro-2-fluoro-phenyl)-2-(4-chloro-2-fluoro-phenyl)-acrylonitrile (2.3 g, 7 mmol) prepared in **Example 52a**, AgF (1.4 g, 11 mmol), and triethylamine (2.6 mL, 19 mmol) in dichloromethane (100 mL) at room temperature for 18 h, followed by the reaction with DBU (9 ml) in tert-butanol (10 mL) at 100 °C for 2 h to give rac-(2R,3S,4R,5S)-5-[1-(tert-butyl-dimethyl-silanyloxymethyl)-cyclohex-3-enylmethyl]-3-(3-chloro-2-fluoro-phenyl)-4-(4-chloro-2-fluoro-phenyl)-4-cyano-pyrrolidine-2-carboxylic acid tert-butyl ester as a white solid (2.6 g, 51%).

### Example 117c

### Preparation of intermediate rac-(2R,3S,4R,5S)-3-(3-chloro-2-)-fluoro-phenyl)-4-(4-chloro-2-fluoro-phenyl)-4-cyano-5-(1-hydroxymethyl-cyclohex-3-enylmethyl)-pyrrolidine-2-carboxylic acid trifluoroacetic acid

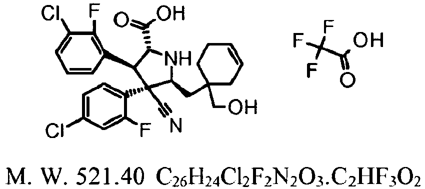

In a manner similar to the method described in **Example 25a**, rac-(2R,3S,4R,5S)-5-[1-(tert-butyl-dimethyl-silanyloxymethyl)-cyclohex-3-enylmethyl]-3-(3-chloro-2-fluoro-phenyl)-4-(4-chloro-2-fluoro-phenyl)-4-cyano-pyrrolidine-2-carboxylic acid tert-butyl ester prepared in **Example 117b** (2.6 g, 3.8 mmol) was reacted with trifluoroacetic acid in dichloromethane at room temperature to give rac-(2R,3S,4R,5S)-3-(3-chloro-2-fluoro-phenyl)-4-(4-chloro-2-fluoro-phenyl)-4-cyano-5-(1-hydroxymethyl-cyclohex-3-enylmethyl)-pyrrolidine-2-carboxylic acid trifluoroacetic acid as a white solid (2.2 g, 92%).

### Example 117d

### Preparation of intermediate rac-(2R,3S,4R,5S)-3-(3-chloro-2-fluoro-phenyl)-4-(4-chloro-2-fluoro-phenyl)-4-cyano-5-(1-hydroxymethyl-cyclohex-3-enylmethyl)-pyrrolidine-2-carboxylic acid [2-((S)-2,2-dimethyl-[1,3]dioxolan-4-yl)-ethyl]-amide

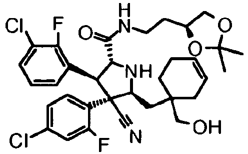

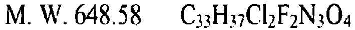

A mixture of rac-(2R,3S,4R,5S)-3-(3-chloro-2-fluoro-phenyl)-4-(4-chloro-2-fluoro-phenyl)-4-cyano-5-(1-hydroxymethyl-cyclohex-3-enylmethyl)-pyrrolidine-2-carboxylic acid trifluoroacetic acid prepared in **Example 117c** (1 g, 1.6 mmol), HATU (1.07 g, 2.8 mmol) and iPr₂NEt (1.37 mL, 7.8 mmol) in CH₂Cl₂ (10 mL) was stirred at room temperature for 18 h. The mixture was then diluted with CH₂Cl₂ and washed with water, brine. The organic layer was separated, the aqueous layer was extracted with CH₂Cl₂. The organic layers were combined, and concentrated. The residue was dissolved into tetrahydrofuran (5 mL), and aqueous saturated K₂CO₃ (5 mL) was added. The mixture was stirred at room temperature for 30 min, then partitioned between ethyl acetate and water. The organic layer was separated, washed with water, brine, dried over MgSO₄ and concentrated. The residue was purified by flash chromatography (EtOAc) to give rac-(2R,3S,4R,5S)-3-(3-chloro-2-fluoro-phenyl)-4-(4-chloro-2-fluoro-phenyl)-4-cyano-5-(1-hydroxymethyl-cyclohex-3-enylmethyl)-pyrrolidine-2-carboxylic acid [2-((S)-2,2-dimethyl-[1,3]dioxolan-4-yl)-ethyl]-amide as a white solid (0.48 g, 47%)

### Example 117e

### Preparation of rac-(2R,3S,4R,SS)-3-(3-chloro-2-fluoro-phenyl)-4-(4-chloro-2-fluoro-phenyl)-4-cyano-5-(1-hydroxymethyl-cyclohex-3-enylmethyl)-pyrrolidine-2-carboxylic acid ((S)-3,4-dihydroxy-butyl)-amide

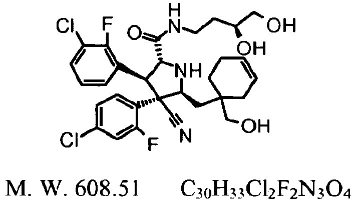

In a manner similar to the method described in **Examples 42d**, rac-(2R,3S,4R,5S)-3-(3-chloro-2-fluoro-phenyl)-4-(4-chloro-2-fluoro-phenyl)-4-cyano-5-(1-hydroxymethyl-cyclohex-3-enylmethyl)-pyrrolidine-2-carboxylic acid [2-((S)-2,2-dimethy)-[1,3]dioxolan-4-yl)-ethyl]-amide prepared in **Example 117d** (0.2 g, 0.3 mmol) was reacted with aqueous HCl solution (1 N, 1mL, 1 mol) in tetrahydrofuran (9 mL) at room temperature for 2 h to give rac-(2R,3S,4R,5S)-3-(3-chloro-2-fluoro-phenyl)-4-(4-chloro-2-fluoro-phenyl)-4-cyano-5-(1-hydroxymethyl-cyclohex-3-enylmethyl)-pyrrolidine-2-carboxylic acid ((S)-3,4-dihydroxy-butyl)-amide as a white solid (0.14 g, 75%).

HRMS (ES⁺) m/z Calcd for C₃₀H₃₃Cl₂F₂N₃O₄+ H [(M+H)⁺]: 608.1889, found: 608.1888.

### Example 118a

### Preparation of intermediate rac-(2R,3S,4R,5S)-3-(3-chloro-2-fluoro-phenyl)-4-(4-chloro-2-fluoro-phenyl)-4-cyano-5-(1-hydroxymethyl-cyclohexylmethyl)-pyrrolidine-2-carboxylic acid [2-((S)-2,2-dimethyl-[1,3]dioxolan-4-yl)-ethyl]-amide

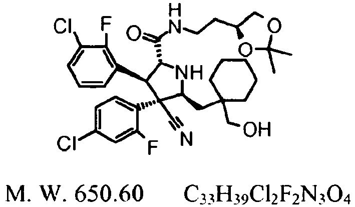

To a solution of rac-(2R,3S,4R,5S)-3-(3-chloro-2-fluoro-phenyl)-4-(4-chloro-2-fluoro-phenyl)-4-cyano-5-(1-hydroxymethyl-cyclohex-3-enylmethyl)-pyrrolidine-2-carboxylic acid [2-((S)-2,2-dimethyl-[1,3]dioxolan-4-yl)-ethyl]-amide (0.28 g, 0.43 mmol) prepared in **Example 117d** in ethyl acetate (10 mL) was added PtO₂ (0.1 g). The suspension was shaken vigorously under H₂ atmosphere (50 psi) for 1 h. The mixture was filtered through a short pad of celite. The filtrate was concentrated to give rac-(2R,3S,4R,5S)-3-(3-chloro-2-fluoro-phenyl)-4-(4-chloro-2-fluoro-phenyl)-4-cyano-3-(1-hydroxymethyl-cyclohexylmethyl)-pyrrolidine-2-carboxylic acid [2-((S)-2,2-dimethyl-[1,3]dioxolan-4-yl)-ethyl]-amide as a white solid (0.27 g, 96%)

### Example 118b

### Preparation of rac-(2R,35,4R,5S)-3-(3-chloro-2-fluoro-phenyl)-4-(4-chloro-2-fluoro-phenyl)-4-cyano-5-(1-hydroxymethyl-cyclohexylmethyl)-pyrrolidine-2-carboxylic acid ((S)-3,4-dihydroxybutyl)-amide

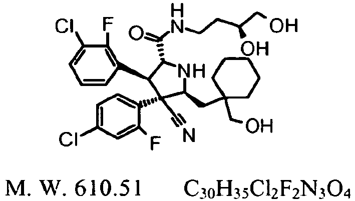

In a manner similar to the method described in **Examples 42d**, rac-(2R,3S,4R,5S)-3-(3-chloro-2-fluoro-phenyl)-4-(4-chloro-2-fluoro-phenyl)-4-cyano-5-(1-hydroxymethyl-cyclohexylmethyl)-pyrrolidine-2-carboxylic acid [2-((S)-2,2-dimethyl-[1,3]dioxolan-4-yl)-ethyl]-amide prepared in **Example 118a** (0.27 g, 0.4 mmol) was reacted with aqueous HCl solution (1 N, 1ML, 1 mol) in tetrahydrofuran (9 mL) at room temperature for 2 h to give rac-(2R,3S,4R,5S)-3-(3-chloro-2-fluoro-phenyl)-4-(4-chloro-2-fluoro-phenyl)-4-cyano-5-(1-hydroxymethyl-cyclohexylmethyl)-pyrrolidine-2-carboxylic acid ((S)-3,4-dihydroxy-butyl)-amide as a white solid (0.23 g, 91%).

HRMS (ES⁺) m/z Calcd for C₃₀H₃₅Cl₂F₂N₃O₄+ H [(M+H)⁺]: 6.10.2046, found: 610.2042.

### Example 119a

### Preparation of intermediate [5-[2-(tert-butyl-dimethyl-silanyloxy)-ethoxy]-3,3-dimethyl-pent-(E)-ylideneamino]-acetic acid tert-butyl ester

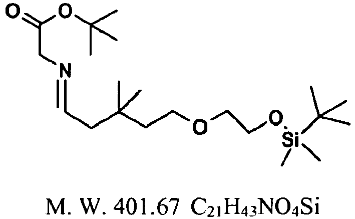

### Step A

To the solution of 3,3-dimethyl-pentane-1,5-diol (1.5 g, 11 mmol) prepared in **Example 116a Step A.** in anhydrous dimethylformamide (15 mL) was added NaH (60%, 0.68 g, 17 mmol). The mixture was stirred at room temperature for 15 min, then (2-bromoethoxy)-tert-butyldimethylsilane (3.3 g, 14 mmol) was added. The mixture was stiired at room temperature for 1 h. Water was added. The mixture was extracted with ethyl acetate twice. The combined organic layers were washed with brine, dried over MgSO₄, and concentrated. The residue was purified by chromatography (EtOAc:hexanes=1:3) to give 5-[2-(tert-butyl-dimethyl-silanyloxy)-ethoxy]-3,3-dimethyl-pentan-1-ol as a yellow oil (0.3 g, 9%).

### Step B

To a solution of oxalyl chloride (0.1 mL, 1 mmol) (Aldrich) in dichloromethane (5 mL) at -78 °C was added the solution of dimethyl sulfoxide (0.16 mL, 2.2 mmol) in dichloromethane (1 mL) dropwise. After 5 mins, the solution of 5-[2-(tert-butyl-dimethyl-silanyloxy)-ethoxy]-3,3-dimethyl-pentan-1-ol (0.3 g, 1 mmol) in dichloromethane (1 mL) was added dropwise. The reaction mixture was stirred at -78 °C for 15 min. Triethylamine (0.5 mL, 3.6 mmol) was added and the reaction mixture was slowly warmed up to room temperature and stirred at room temperature for 45 min. Then water was added. The organic layers were separated, and the aqueous layer was extracted with dichloromethane. The organic layers were combined, washed with 10% of HCl, saturated NaHCO₃, brine, dried over MgSO₄, and concentrated to give 5-[2-(tert-butyl-dimethyl-silanyloxy)-ethoxy]-3,3-dimethyl-pentanal as a yellow oil (Yield: 0.27 g, 94%).

### Step C

In a manner similar to the method described in **Example 1a**, glycine tert-butyl ester (0.13 g, 1 mmol) was reacted with 5-[2-(tert-butyl-dimethyl-silanyloxy)-ethoxy]-3,3-dimethyl-pentanal (0.27 g, 1 mmol) in CH₂Cl₂ at room temperature for 18 h to give [5-[2-(tert-butyl-dimethyl-silanyloxy)-ethoxy]-3,3-dimethyl-pent-(E)-ylideneamino]-acetic acid tert-butyl ester as a colorless oil (0.4 g, 100%).

### Example 119b

### Preparation of intermediate rac-(2R,35,4R,5S)-5-{4-[2-(tert-butyl-dimethyl-silanyloxy)-ethoxy]-2,2-dimethyl-butyl}-3-(3-chloro-2-fluoro-phenyl)-4-(4-chloro-2-fluoro-phenyl)-4-cyanopyrrolidine-2-carboxylic acid tert-butyl ester

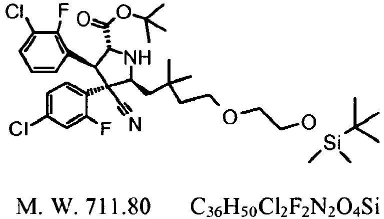

In a manner similar to the method described in **Example 100b**, [5-[2-(tert-butyl-dimethyl-silanyloxy)-ethoxy]-3,3-dimethyl-pent-(E)-ylideneamino]-acetic acid tert-butyl ester prepared in **Example 119a** (0.4 g, 1 mmol) was reacted with (Z)-3-(3-chloro-2-fluoro-phenyl)-2-(4-chloro-2-fluoro-phenyl)-acrylonitrile (0.3 g, I mmol) prepared in **Example 52a**, AgF (0.2 g, 1.5 mmol), and triethylamine (0.3 m, 2.4 mmol) in dichloromethane (50 mL) at room temperature for 18 h, followed by the reaction with DBU (1 ml) in tert-butanol (2 mL) at 100 °C for 2 h to give rac-(2R,3S,4R,5S)-5-{4-[2-(tert-butyl-dimethyl-silanyloxy)-ethoxy]-2,2-dimethyl-butyl}-3-(3-chloro-2-fluoro-phenyl)-4-(4-chloro-2-fluoro-phenyl)-4-cyano-pyrrolidine-2-carboxylic acid tert-butyl ester as a white gum (0.49 g, 60%).

### Example 119c

### Preparation of intermediate rac-(2R,3S,4R,5S)-3-(3-chloro-2-fluoro-phenyl)-4-(4-chloro-2-fluoro-phenyl)-4-cyano-5-{2,2-dimethyl-4-[2-(2,2,2-trifluoro-acetoxy)-ethoxy]-butyl}-pyrrolidine-2-carboxylic acid trifluoroacetic acid

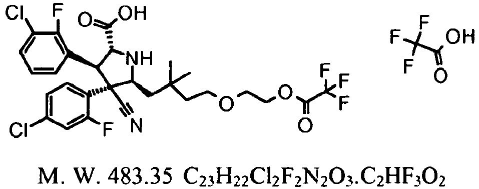

To a solution of rac-(2R,3S,4R,5S)-5-{4-[2-(tert-butyl-dimethyl-silanyloxy)-ethoxy]-2,2-dimethyl-butyl}-3-(3-chloro-2-fluoro-phenyl)-4-(4-chloro-2-fluoro-phenyl)-4-cyano-pyrrolidine-2-carboxylic acid tert-butyl ester prepared in **Example 119b** (0.49 g, 0.55 mmol) in dichloromethane (3 mL) at room temperature was added trifluoroacetic acid (3 mL). The reaction mixture was stirred at room temperature for 18 h. The mixture was concentrated under reduced pressure to give rac-(2R,3S,4R,5S)-3-(3-chloro-2-fluoro-phenyl)-4-(4-chloro-2-fluoro-phenyl)-4-cyano-5-{2,2-dimethyl-4-[2-(2,2,2-trifluoro-acetoxy)-ethoxy]-butyl}-pyrrolidine-2-carboxylic acid trifluoroacetic acid as a yellow oil (0.37 g, 97%).

### Example 119d

### Preparation of rac-(2R,3S,4R,5S)-3-(3-chloro-2-fluoro-phenyl)-4-(4-chloro-2-fluoro-phenyl)-4-cyano-5-[4-(2-hydroxy-ethoxy)-2,2-dimethyl-butyl]-pyrrolidine-2-carboxylic acid ((S)-3,4-dihydroxy-butyl)-amide

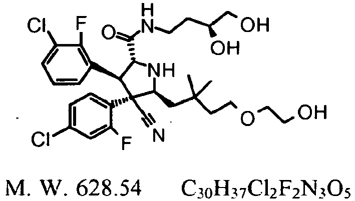

In a manner similar to the method described in **Examples 117d and 117e**, rac-(2R,3S,4R,5S)-3-(3-chloro-2-fluoro-phenyl)-4-(4-chloro-2-fluoro-phenyl)-4-cyano-5-{2,2-dimethyl-4-[2-(2,2,2-trifluoro-acetoxy)-ethoxy]-butyl}-pyrrolidine-2-carboxylic acid trifluoroacetic acid prepared in **Example 119**c (0.37 g, 0.58 mmol) was reacted with 2-((S)-2,2-dimethyl-[1,3]dioxolan-4-yl)-ethylamine (0.25 g, 1.7 mmol), HATU (0.4 g, 1 mmol) and iPr₂NEt (0.5 mL, 2.9 mmol) in CH₂Cl₂ at room temperature for 20 h, then treated with aqueous saturated K₂CO₃ solution in tetrahydrofuran, followed by reaction with aqueous HCl solution in tetrahydrofuran at room temperature to give rac-(2R,3S,4R,5S)-3-(3-chloro-2-fluoro-phenyl)-4-(4-chloro-2-fluoro-phenyl)-4-cyano-5-[4-(2-hydroxy-ethoxy)-2,2-dimethyl-butyl]-pyrrolidine-2-carboxylic acid ((S)-3,4-dihydroxy-butyl)-amide as a light yellow solid (90 mg, 25%).

HRMS (ES⁺) m/z Calcd for C₃₀H₃₇Cl₂F₂N₃O₅+ H [(M+H)⁺]: 628.2151, found: (128.2150.

### Example 120a

### Preparation of intermediate [5-azido-3,3-dimethyl-pent-(E)-ylideneamino]-acetic acid tert-butyl ester

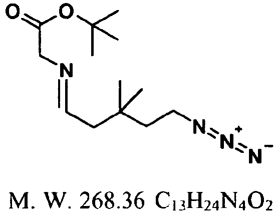

### Step A

To the solution of 5-(tert-butyl-dimethyl-silanyloxy)-3,3-dimethyl-pentan-1-ol (1.1 g, 5 mmol) prepared in **Example 116a Step B**. and triethylamine (1.39 mL, 10 mmol) in dichloromethane, (50 mL) at 0 °C was added a dichloromethane solution (10 mL) of methanesulfonyl chloride (Aldrich) (0.46 mL, 6mmol). The reaction mixture was stirred at 0 °C for 1 h. Water was added. Organic layer was separated, the aqueous layer was extracted with dichlormethane. The combined organic layers were washed with diluted aqueous HCl solution, saturated aqueous NaHCO₃ solution, brine, dried over MgSO₄, and concentrated to give methanesulfonic acid 5-(tert-butyl-dimethyl-silanyloxy)-3,3-dimethyl-pentyl ester as a yellow oil (1.48 g, 99%).

### Step B

To the solution of methanesulfonic acid 5-(tert-butyl-dimethyl-silanyloxy)-3,3-dimethyl-pentyl ester (1.48 g, 4.96 mmol) in anhydrous dimethylformamide (10 mL) was added NaN₃ (1.6 g, 25 mmol). The reaction mixture was heated at 60 °C for 18 h. The mixture was cooled, and water was added. The mixture was extracted with ethyl acetate twice. The combined organic layers were washed with saturated aqueous NaHCO₃ solution, brine, dried over MgSO₄ to give (5-azido-3,3-dimethyl-pentyloxy)-tert-butyl-dimethyl-silane as a yellow oil (0.8 g, 67%).

### Step C

To a solution of (5-azido-3,3-dimethyl-pentyloxy)-tert-butyl-dimethyl-silane (0.8 g, 3 mmol) in tetrahydrofuran (5 mL) was added tetrahydrofuran solution (1 M, Aldrich) of TBAF (4.9 mL, 4.9 mmol). The reaction mixture was stirred at room temperature for 1 h. The mixture was concentrated, the residue was partitioned between ethyl acetate and water. The organic layer was separated, dried over MgSO₄, and concentrated. The residue was purified by chromatography (EtOAc:hexanes=1:3) to give 5-azido-3,3-dimethyl-pentan-1-ol as a colorless oil (0g, 76%)

### Step D

To a solution of oxalyl chloride (0.24 mL, 2.7 mmol) (Aldrich) in dichloromethane (12 mL) at - 78 °C was added the solution of dimethyl sulfoxide (0.38 mL, 5.5 mmol) in dichloromethane (1 mL) dropwise. After 5 mins, the solution of 5-azido-3,3-dimethyl-pentan-1-ol (0.39 g, 2.5 mmol) in dichloromethane (1 mL) was added dropwise. The reaction mixture was stirred at -78 °C for 15 min. Triethylamine (1.2 mL, 9 mmol) was added and the reaction mixture was slowly warmed up to room temperature and stirred at room temperature for 45 min. Then water was added. The organic layers were separated, and the aqueous layer was extracted with dichloromethane. The organic layers were combined, washed with 10% of HCl, saturated NaHCO₃, brine, dried over MgSO₄, and concentrated to give 5-azido-3,3-dimethyl-pentanal as a yellow oil (Yield: 0.38 g, 99%).

### Step C

In a manner similar to the method described in **Example 1a**, glycine tert-butyl ester (0.32 g, 2.45 mmol) was reacted with 5-azido-3,3-dimethyl-pentanal (0.38 g, 2.45 mmol) in CH₂Cl₂ at room temperature for 18 h to give [5-azido-3,3-dimethyl-pent-(E)-ylideneamino]-acetic acid tert-butyl ester as a yellow oil (0.65 g, 100%).

### Example 120b

### Preparation of intermediate rac-(2R,3S,4R,5S)-5-(4-azido-2,2-dimethyl-butyl)-3-(3-chloro-2-fluoro-phenyl)-4-(4-chloro-2-fluoro-phenyl)-4-cyano-pyrrolidine-2-carboxylic acid tert-butyl ester

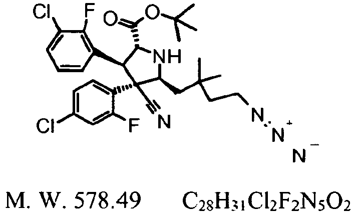

In a manner similar to the method described in **Example 100b**, [5-azido-3,3-dimethyl-pent-(E)-ylideneamino]-acetic acid tert-butyl ester prepared in **Example 120a** (0.65 g, 2.45 mmol) was reacted with (Z)-3-(3-chloro-2-fluoro-phenyl)-2-(4-chloro-2-fluoro-phenyl)-acrylonitrile (0.76 g, 2.45 mmol) prepared in **Example 52a**, AgF (0.47 g, 3.7 mmol), and triethylamine (0.55 mL, 6 mmol) in dichloromethane (80 mL) at room temperature for 18 h, followed by the reaction with DBU (3 ml) in tert-butanol (3 mL) at 100 °C for 2 h to give rac-(2R,3S,4R,5S)-5-(4-azido-2,2-dimethyl-butyl)-3-(3-chloro-2-fluoro-phenyl)-4-(4-chloro-2-fluoro-phenyl)-4-cyano-pyrrolidine-2-carboxylic acid tert-butyl ester as a yellow gum (0.5 g, 36%).

### Example 120c

### Preparation of intermediate rac-(2R,3S,4R,5S)-5-(4-azido-2,2-dimethyl-butyl)-3-(3-chloro-2-fluoro-phenyl)-4-(4-chloro-2-fluoro-phenyl)-4-cyano-pyrrolidine-2-carboxylic acid trifluoroacetic acid

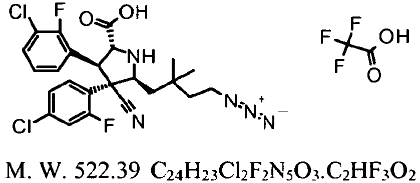

In a manner similar to the method described in **Example 25a**, rac-(2R,3S,4R,5S)-5-(4-azido-2,2-dimethyl-butyl)-3-(3-chloro-2-fluoro-phenyl)-4-(4-chloro-2-fluoro-phenyl)-4-cyano-pyrrolidine-2-carboxylic acid tert-butyl ester prepared in **Example 120b** (0.5 g, 0.86 mmol) was reached with trifluoroacetic acid in dichloromethane at room temperature to give rac-(2R,3S,4R,5S)-5-(4-azido-2,2-dimethyl-butyl)-3-(3-chloro-2-fluoro-phenyl)-4-(4-chloro-2-fluoro-phenyl)-4-cyanopyrrolidine-2-carboxylic acid trifluoroacetic acid as a yellow solid (0.54 g, 96%).

### Example 120d

### Preparation of intermediate rac-(2R,3S,4R,5S)-5-(4-azido-2,2-dimethyl-butyl)-3-(3-chloro-2-fluoro-phenyl)-4-(4-chloro-2-fluoro-phenyl)-4-cyano-pyrrolidine-2-carboxylic acid [2-((S)-2,2-dimethyl-[1,3]dioxolan-4-yl)-ethyl]-amide

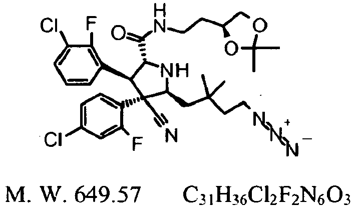

In a manner similar to the method described in **Examples 42c**, rac-(2R,3S,4R,5S)-5-(4-azido-2,2-dimethyl-butyl)-3-(3-chloro-2-fluoro-phenyl)-4-(4-chloro-2-fluoro-phenyl)-4-cyanopyrrolidine-2-carboxylic acid trifluoroacetic acid prepared in **Example 120c** (0.54 g, 0.85 mmol) was reacted with 2-((S)-2,2-dimethyl-[1,3]dioxolan-4-yl)-ethylamine (0.37 g, 2.54 mmol), HATU (0.58 g, 1.5 mmol) and iPr₂NEt (0.74 mL, 4.2 mmol) in CH₂Cl₂ at room temperature for 20 h, to give rac-(2R,3S,4R,5S)-5-(4-azido-2,2-dimethyl-butyl)-3-(3-chloro-2-fluoro-phenyl)-4-(4-chloro-2-fluoro-phenyl)-4-cyano-pyrrolidine-2-carboxylic acid (2-((S)-2,2-dimethyl-[1,3]dioxolan-4-yl)-ethyl]-amide as a light yellow solid (0.5 g, 91%).

### Example 120e

### Preparation of rac-(2R,35,4R,55)-5-(4-azido-2,2-dimethyl-butyl)-3-(3-chloro-2-fluoro-phenyl)-4-(4-chloro-2-fluoro-phenyl)-4-cyano-pyrrolidine-2-carboxylic acid ((S)-3,4-dihydroxy-butyl)-amide

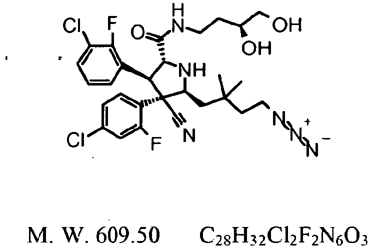

In a manner similar to the method described in **Examples 42d**, rac-(2R,3S,4R,5S)-5-(4-azido-2,2-dimethyl-butyl)-3-(3-chloro-2-fluoro-phenyl)-4-(4-chloro-2-fluoro-phenyl)-4-cyanopyrrolidine-2-carboxylic acid [2-((S)-2,2-dimethyl-[1,3]dioxolan-4-yl)-ethyl]-amide prepared in **Example 120d** (40 mg, 0.06 mmol) was reacted with aqueous HCl solution (1 N, 3 mL, 3 mol) in tetrahydrofuran (7 mL) at room temperature for 2 h to give rac-(2R,3S,4R,5S)-5-(4-azido-2,2-dimethyl-butyl)-3-(3-chloro-2-fluoro-phenyl)-4-(4-chloro-2-fluoro-phenyl)-4-cyano-pyrrolidine-2-carboxylic acid ((S)-3,4-dihydroxy-butyl)-amide as a off white solid (29 mg, 79%).

HRMS (ES⁺) m/z Calcd for C₂₈H₃₂Cl₂F₂N₆O₃+ H [(M+H)⁺]: 609.1954, found: 609.1954.

### Example 121a

### Preparation of intermediate rac-(2R,3S,4R,5S)-5-(4-amino-2,2-dimethyl-butyl)-3-(3-chloro-2-fluoro-phenyl)-4-(4-chloro-2-fluoro-phenyl)-4-cyano-pyrrolidine-2-carboxylic acid [2-((S)-2,2-dimethyl-[1,3]dioxolan-4-yl)-ethyl]-amide

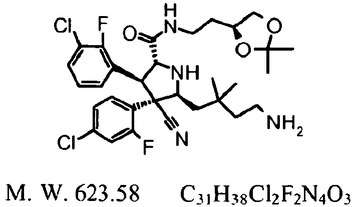

In a manner similar to the method described in **Examples 118a**, rac-(2R,3S,4R,5S)-5-(4-azido-2,2-dimethyl-butyl)-3-(3-chloro-2-fluoro-phenyl)-4-(4-chloro-2-fluoro-phenyl)-4-cyanopyrrolidine-2-carboxylic acid [2-((S)-2,2-dimethyl-[1,3]dioxolan-4-yl)-ethyl]-amide prepared in **Example 120d** (0.5 g, 0.77 mmol) was treated with PtO₂ and H₂ in ethyl acetate to give rac-(2R,3S,4R,5S)-5-(4-amino-2,2-dimethyl-butyl)-3-(3-chloro-2-fluoro-phenyl)-4-(4-chloro-2-fluoro-phenyl)-4-cyano-pyrrolidine-2-carboxylic acid [2-((S)-2,2-dimethyl-[1,3]dioxolan-4-yl)-ethyl]-amide as a black gum (0.47 g, 98%)

### Example 121b

### Preparation of rac-(2R,3S,4R,5S)-5-(4-amino-2,2-dimethyl-butyl)-3-(3-chloro-2-fluoro-phenyl)-4-(4-chloro-2-fluoro-phenyl)-4-cyano-pyrrolidine-2-carboxylic acid ((S)-3,4-dihydroxy-butyl)-amide

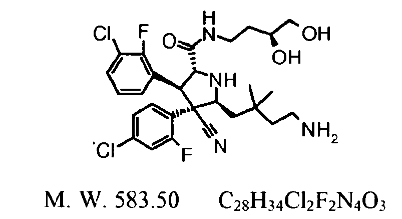 M. W. 583.50 C₂₈H₃₄Cl₂F₂N₄O₃

In a manner similar to the method described in **Examples 42d**, rac-(2R,3S,4R,5S)-5-(4-amino-2,2-dimethyl-butyl)-3-(3-chloro-2-fluoro-phenyl)-4-(4-chloro-2-fluoro-phenyl)-4-cyanopyrrolidine-2-carboxylic acid [2-((S)-2,2-dimethyl-[1,3]dioxolan-4-yl)-ethyl]-amide prepared in **Example 121a** (50 mg, 0.08 mmol) was reacted with aqueous HCl solution (1 N, 3 mL, 3 mol) in tetrahydrofuran (7 mL) at room temperature for 2 h to give rac-(2R,3S,4R,5S)-5-(4-amino-2,2-dimethyl-butyl)-3-(3-chloro-2-fluoro-phenyl)-4-(4-chloro-2-fluoro-phenyl)-4-cyanopyrrolidine-2-carboxylic acid ((S)-3,4-dihydroxy-butyl)-amide as a off white solid (29 mg, 62%). HRMS (ES⁺) m/z Calcd for C₂₈H₃₄Cl₂F₂N₄O₃+ H [(M+H)⁺]: 583.2049, found: 583.2047.

### Example 122a

### Preparation of intermediate rac-(2R,3S,4R,5S)-5-(4-acetylamino-2,2-dimethyl-butyl)-3-(3-chloro-2-fluoro-phenyl)-4-(4-chloro-2-fluoro-phenyl)-4-cyano-pyrrolidine-2-carboxylic acid [2-((S)-2,2-dimethyl-[1,3]dioxolan-4-yl)-ethyl]-amide

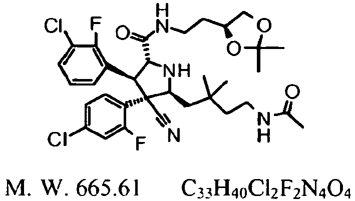

To a solution of rac-(2R,3S,4R,5S)-5-(4-amino-2,2-dimethyl-butyl)-3-(3-chloro-2-fluoro-phenyl)-4-(4-chloro-2-fluoro-phenyl)-4-cyano-pyrrolidine-2-carboxylic acid [2-((S)-2,2-dimethyl-[1,3]dioxolan-4-yl)-ethyl]-amide (60 mg, 0.096 mmol) prepared in **Example 121a** and triethylamine (0.033 mL, 0.24 mmol) in tetrahydrofuran (3 mL) was added acetyl chloride (0.08 mL, 0.1 mmol). The reaction mixture was stirred at room temperature for 30 min, then water was added. The mixture was partitioned between ethyl acetate and water. Organic layer was separated, washed with water, brine, dried over MgSO₄, and concentrated. The residue was purified by chromatography (2% MeOH in EtOAc) to give rac-(2R,3S,4R,5S)-5-(4-acetylamino-2,2-dimethyl-butyl)-3-(3-chloro-2-fluoro-phenyl)-4-(4-chloro-2-fluoro-phenyl)-4-cyanopyrrolidine-2-carboxylic acid [2-((S)-2,2-dimethyl-[1,3]dioxolan-4-yl)-ethyl]-amide as a off white gum (60 mg, 94%)

### Example 122b

### Preparation of rac-(2R,3S,4R,5S)-5-(4-acetylamino-2,2-dimethyl-butyl)-3-(3-chloro-2-fluoro-phenyl)-4-(4-chloro-2-fluoro-phenyl)-4-cyano-pyrrolidine-2-carboxylic acid ((S)-3,4-dihydroxybutyl)-amide

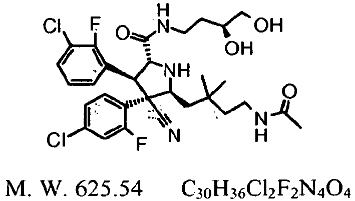

In a manner similar to the method described in **Examples 42d**, rac-(2R,3S,4R,5S)-5-(4-acetylamino-2,2-dimethyl-butyl)-3-(3-chloro-2-fluoro-phenyl)-4-(4-chloro-2-fluoro-phenyl)-4-cyano-pyrrolidine-2-carboxylic acid [2-((S)-2,2-dimethyl-[1,3]dioxolan-4-yl)-ethyl]-amide prepared in **Example 122a** (60 mg, 0.09 mmol) was reacted with aqueous HCl solution (1 N, 1 mL, 1 mol) in tetrahydrofuran (5 mL) at room temperature for 2 h to give rac-(2R,3S,4R,5S)-5-(4-acetylamino-2,2-dimethyl-butyl)-3-(3-chloro-2-fluoro-phenyl)-4-(4-chloro-2-fluoro-phenyl)-4-cyano-pyrrolidine-2-carboxylic acid ((S)-3,4-dihydroxy-butyl)-amide as a white solid (50 mg, 89%).

HRMS (IS⁺) m/z Calcd for C₃₀H₃₆Cl₂F₂N₄O₄+ H[(M+H)⁺]: 625.2155, found: 625.215.

### Example 123

### Preparation of rac-(2R,3S,4R,5S)-3-(3-chloro-2-fluoro-phenyl)-4-(4-chloro-2-fluoro-phenyl)-4-cyano-5-(4-methanesulfonylamino-2,2-dimethyl-butyl)-pyrrolidine-2-carboxylic acid ((S)-3,4-dihydroxy-butyl)-amide

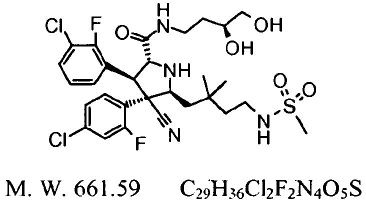

In a manner similar to the method described in **Examples 122a and 122b,** rac-(2R,3S,4R,5S)-5-(4-amino-2,2-dimethyl-butyl)-3-(3-chloro-2-fluoro-phenyl)-4-(4-chloro-2-fluoro-phenyl)-4-cyano-pyrrolidine-2-carboxylic acid [2-((S)-2,2-dimethyl-[1,3]dioxolan-4-yl)-ethyl]-amide (60 mg, 0.096 mmol) prepared in **Example 121a**
was reacted with triethylamine and methanesulfonyl chloride (13 mg, 0.11 mmol) in dichloromethane, followed by the reaction with aqueous HCl solution in tetrahydrofuran to give rac-(2R,3S,4R,5S)-3-(3-chloro-2-fluoro-phenyl)-4-(4-chloro-2-fluoro-phenyl)-4-cyano-5-(4-methanesulfonylamino-2,2-dimethyl-butyl)-pyrrolidine-2-carboxylic acid ((S)-3,4-dihydroxybutyl)-amide as a off white solid (57 mg, 90%)

HRMS (ES⁺) m/z Calcd for C₂₉H₃₆Cl₂F₂N₄O₅S+ H[(M+H)⁺]: 661.1825, found: 661.1821.

### Example 124

### Preparation of rac-(2R,3S,4R,5S)-5-(4-benzoylamino-2,2-dimethyl-butyl)-3-(3-chloro-2-fluoro-phenyl)-4-(4-chloro-2-fluoro-phenyl)-4-cyano-pyrrolidine-2-carboxylic acid ((S)-3,4-dihydroxybutyl)-amide

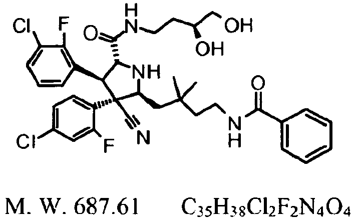

In a manner similar to the method described in **Examples 122a and 122b**, rac-(2R,3S,4R,5S)-5-(4-amino-2,2-dimethyl-butyl)-3-(3-chloro-2-fluoro-phenyl)-4-(4-chloro-2-fluoro-phenyl)-4-cyano-pyrrolidine-2-carboxylic acid [2-((S)-2,2-dimethyl-[1,3]dioxolan-4-yl)-ethyl]-amide (80 mg, 0.13 mmol) prepared in **Example 121a** was reacted with triethylamine and benzoyl chloride (22 mg, 0.16 mmol) in tetrahydrofuran, followed by the reaction with aqueous HCl solution in tetrahydrofuran to give rac-(2R,3S,4R,5S)-5-(4-benzoylamino-2,2-dimethyl-butyl)-3-(3-chloro-2-fluoro-phenyl)-4-(4-chloro-2-fluoro-phenyl)-4-cyano-pyrrolidine-2-carboxylic acid ((S)-3,4-dihydroxy-butyl)-amide as a off white solid (57 mg, 90%)

HRMS (ES⁺) m/z Calcd for C₃₅H₃₈Cl₂F₂N₄O₄+ H [(M+H)⁺] 687.2311, found: 687.2308

### Example 125a

### Preparation of intermediate [3-methyl-3-(5-methyl-furan-2-yl)-but-(E)-ylideneamino]-acetic acid tert-butyl ester

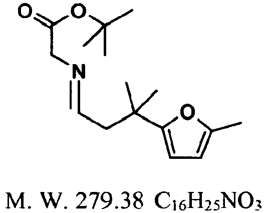

### Step A

To a solution of dimethyl malonate (6.5 g, 49 mmol), 2-acetyl-5-methylfuran (6.1 g, 49 mmol) and pyridine (16 g, 200 mmol) in anhydrous tetrahydrofuran (300 mL) at 0 °C was added a dichloromethane solution (1 M) of TiCl₄ (100 mL, 100 mmol) during a period of 1 h. After the addition was finished, the reaction mixture was gradually warmed room temperature and stirred for 18 h. Water was added to quench the reaction. The mixture was extracted with ethyl ether. The organic layer was separated, and the aqueous layer was extracted with ethyl acetate. The organic layers were combined, concentrated. The residue was purifed by chromatography (EtOAc:hexanes=1;10) to give 2-[1-(5-methyl-furan-2-yl)-ethylidene]-malonic acid dimethyl ester as a yellow oil (3.7 g, 32%).

### Step B

To a suspension of CuI (7.61 g, 40 mmol) in anhydrous tetrahydrofuran (100 mL) at -50 °C was added methylmagnesium chloride (3 M, 27 mL, 80 mmol) during a period of 15 min. After the addition was finished, the reaction mixture was gradually warmed room temperature and stirred for 20 min. Then the temperature of the mixture was lowered to -50 °C, a tetrahydrofuran solution (50 mL) of 2-[1-(5-methyl-furan-2-yl)-ethylidene]-malonic acid dimethyl ester (3.7 g, 15.5 mmol) was added. The reaction mixture was allowed to slowly warmed to room temperature and stirred for 3 h. Aqueous saturated NH₄Cl solution was added to quench the reaction. The mixture was filtered, and the filtrate was concentrated to remove most of tetrahydrofuran. The residue was extracted with ethyl acetate twice. The organic layers were combined, concentrated. The residue was purifed by chromatography (EtOAc:hexanes=1:20, 1:10) to give 2-[1-methyl-1-(5-methyl-furan-2-yl)-ethyl]-malonic acid dimethyl ester as a colorless oil (2.5 g, 63%).

### Step C

To a solution of 2-[1-methyl-1-(5-methyl-furan-2-yl)-ethyl]-malonic acid dimethyl ester (2.5 g, 9.8 mmol) in DMSO (30 mL) was added LiCl (1 g, 23.7 mmol) and H₂O (0.17 mL, 9.8 mmol). The reaction mixture was heated at 170 °C for 3 h, then poured into a ice-water, extracted with ethyl acetate. The organic layer were separated, washed with water, brine, dried over MgSO₄, and concentrated. The residue was purifed by chromatography (EtOAc:hexanes=1:20, 1:20) to give 3-methyl-3-(5-methyl-furan-2-yl)-butyric acid methyl ester as a colorless oil (1.5 g, 78%).

### Step D

To a solution of 3-methyl-3-(5-methyl-furan-2-yl)-butyric acid methyl ester (1.5 g, 7.8 mmol) in anhydrous tetrahydrofuran (50 mL) at 0 °C was added a tetrhydrofuran solution (1 M) of LiAlH₄ (10 m, 10 mmol) under nitrogen. The reaction mixture was stirred at 0.°C for 1 h, then poured into a ice-water. The mixture was extracted with ethyl acetate. The organic layer were separated, washed with water, aqueous HCl solution, brine, dried over MgSO₄, and concentrated to give 3-methyl-3-(5-methyl-furan-2-yl)-butan-1-ol as a yellow oil (1.2 g, 77%).

### Step E

To a solution ofoxalyl chloride (0.91 g, 7.1 mmol) (Aldrich) in dichloromethane (20 mL) at -78 °C was added the solution of dimethyl sulfoxide (1 mL, 14.3 mmol) in dichloromethane (5 mL) dropwise. After 5 mins, the solution of 3-methyl-3-(5-methyl-furan-2-yl)-butan-1-ol (1.2 g, 7.1 mmol) in dichloromethane (5 mL) was added dropwise. The reaction mixture was stirred at -78 °C for 15 min. Triethylamine (3.6 mL, 26 mmol) was added and the reaction mixture was slowly warmed up to room temperature and stirred at room temperature for 45 min. Then water was added. The organic layers were separated, and the aqueous layer was extracted with dichloromethane. The organic layers were combined, washed with 10% of HCl, saturated NaHCO₃, brine, dried over MgSO₄, and concentrated to give 3-methyl-3-(5-methyl-furan-2-yl)-butyraldehyde as a yellow oil (Yield: 1 g, 83%).

### Step F

In a manner similar to the method described in **Example 1a**, glycine tert-butyl ester (0.79 g, 6 mmol) was reacted with 3-methyl-3-(5-methyl-furan-2-yl)-butyraldehyde (1 g, 6 mmol) in CH₂Cl₂ at room temperature for 5 h to give [3-methyl-3-(5-methyl-furan-2-yl)-but-(E)-ylideneamino]-acetic acid tert-butyl ester as a colorless oil (1.7 g, 100%).

### Example 125b

### Preparation of intermediate rac-(2R,3S,4R,5S)-3-(3-chloro-2-fluoro-phenyl)-4-(4-chloro-2-fluoro-phenyl)-4-cyano-5-[2-methyl-2-(5-methyl-furan-2-yl)-propyl]-pyrrolidine-2-carboxylic acid tert-butyl ester

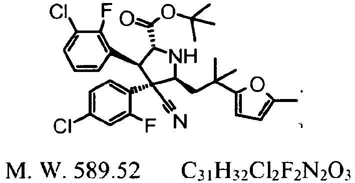

In a manner similar to the method described in **Example 100b**, [[3-methyl-3-(5-methyl-furan-2-yl)-but-(E)-ylideneamino]-acetic acid tert-butyl ester prepared in **Example 125a** (1.7 g, 6 mmol) was reacted with (Z)-3-(3-chloro-2-fluoro-phenyl)-2-(4-chloro-2-fluoro-phenyl)-acrylonitrile (1.48 g, 4.8 mmol) prepared in **Example 52a**, AgF (0.9 g, 7 mmol), and triethylamine (1.7 mL, 12 mmol) in dichloromethane (100 mL) at room temperature for 18 h, followed by the reaction with DBU (5.7 ml) in tert-butanol (10 mL) at 100 °C for 2 h to give rac-(2R,3S,4R,5S)-3-(3-chloro-2-fluoro-phenyl)-4-(4-chloro-2-fluoro-phenyl)-4-cyano-5-[2-methyl-2-(5-methyl-furan-2-yl)-propyl]-pyrrolidine-2-carboxylic acid tert-butyl ester as a yellow solid (1.3 g, 46%).

### Example 125c

### Preparation of intermediate rac-(2R,3S,4R,5S)-3-(3-chloro-2-fluoro-phenyl)-4-(4-chloro-2-fluoro-phenyl)-4-cyano-5-(2,2-diethyl-butyl)-pyrrolidine-2-carboxylic acid trifluoroacetic acid

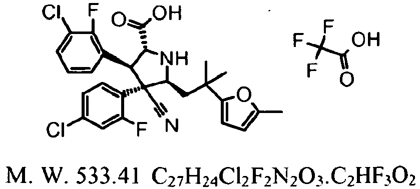

In a manner similar to the method described in **Example 25a,** rac-(2R,3S,4R,5S)-3-(3-chloro-2-fluoro-phenyl)-4-(4-chloro-2-fluoro-phenyl)-4-cyano-5-[2-methyl-2-(5-methyl-furan-2-yl)-propyl]-pyrrolidine-2-carboxylic acid tert-butyl ester prepared in **Example 125b** (1.3 g, 2.2 mmol) was reacted with trifluoroacetic acid in dichloromethane at room temperature to give rac-(2R,3S,4R,5S)-3-(3-chloro-2-fluoro-phenyl)-4-(4-chloro-2-fluoro-phenyl)-4-cyano-[2-methyl-2-(5-methyl-furan-2-yl)-propyl]-pyrrolidine-2-carboxylic acid trifluoroacetic acid as a brown solid (1.3 g, 92%).

### Example 125d

### Preparation of rac-(2R,3S,4R,5S)-3-(3-chloro-2-fluoro-phenyl)-4-(4-chloro-2-fluoro-phenyl)-4-cyano-5-[2-methyl-2-(5-methyl-furan-2-yl)-propyl]-pyrrolidine-2-carboxylic acid ((S)-3,4-dihydroxy-butyl)-amide

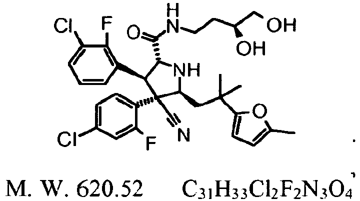

In a manner similar to the method described in **Examples 42c and 42d**, rac-(2R,3S,4R,5S)-3-(3-chloro-2-fluoro-phenyl)-4-(4-chloro-2-fluoro-phenyl)-4-cyano-5-[2-methyl-2-(5-methyl-furan-2-yl)-propyl]-pyrrolidine-2-carboxylic acid trifluoroacetic acid prepared in **Example 125c** (0.6 g, 0.93 mmol) was reacted with 2-((S)-2,2-dimethyl-[1,3]dioxolan-4-yl)-ethylamine (0.4 g, 2.8 mmol), HATU (0.6 g, 1.7 mmol) and iPr₂NEt (0.8 mL, 4.6 mmol) in CH₂Cl₂ at room temperature for 20 h, then reacted with aqueous HCl solution in tetrahydrofuran at room temperature for 2 h to give rac-(2R,3S,4R,5S)-3-(3-chloro-2-fluoro-phenyl)-4-(4-chloro-2-fluoro-phenyl)-4-cyano-5-[2-methyl-2-(5-methyl-furan-2-yl)-propyl]-pyrrolidine-2-carboxylic acid ((S)-3,4-dihydroxy-butyl)-amide as a white solid (0.16 g, 29%).

HRMS (ES⁺) m/z Calcd for C₃₁H₃₃Cl₂F₂N₃O₄+ H [(M+H)⁺]: 620.1889, found: 620.1889.

### Example 126a

### Preparation of intermediate [4-(4-methoxy-phenyl)-3,3-dimethyl-but-(E)-ylideneamino]-acetic acid methyl ester

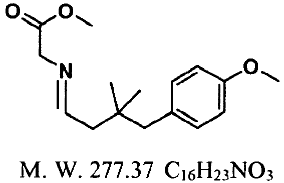

### Step A.

Under Argon, a mixture of NaOH (2.8 g, 70 mmol), tetrabutylammonium iodide (0.6 g, 1.6 mmol) in benzene (8 mL) and H₂O (2.8 mL) was heated at 70 °C to form a homogeneous mixture. A mixture of 4-methoxybenzyl chloride (Aldrich) (10 g, 64 mmol) and isobutyraldehyde (5.76 g, 80 mmol) in benzene (22 mL) was added dropwise. The resulting reaction mixture was heated at 70 °C for 3 h. The mixture was cooled, extracted with ethyl acetate. The organic layer was separated, and the aqueous layer was extracted with ethyl acetate. The organic layers were combined, concentrated. The residue was purifed by chromatography (EtOAc:hexanes=1;30) to give 2-(4-methoxy-phenyl)-2-methyl-propionaldehyde as a colorless oil (4.1 g, 33%).

### Step B

To a mixture of methoxymethyl triphenylphosphonium chloride (14.6 g, 42 mmol) in anhydrous tetrahydrofuran (60 mL) at 0°C was a tetrahydrofuran solution (Aldrich, 1 M) of LiHMDS (42 mL, 42 mmol) dropwise. After the addition was finished, the reaction mixture was stirred at 0 °C for 20 min. Then a tetrahydrofuran solution (40 mL) of 2-(4-methoxy-phenyl)-2-methyl-propionaldehyde (4.1g, 21 mmol) was added. The reaction mixture was allowed to slowly warmed to room temperature and stirred for 1 h. Water was added to quench the reaction. The mixture was extracted with ethyl acetate twice. The organic layers were combined, concentrated. The residue was purifed by chromatography (EtOAc:hexanes=1:30, 1:20) to give a yellow oil (3.5 g). The oil was dissolved into a solution of aqueous HCl solution (2 N, 50 mL, 100 mmol) and tetrahydrofuran (50 mL). The reaction mixture was heated at reflux for 1 h, then cooled to room temperature and concentrated. The residue partitioned between ethyl acetate and water. The organic layer was separated, concentrated. The residue was purified by chromatography (EtOAc:hexanes=1:10,1:5) to give 3-(4-methoxy-phenyl)-3-methyl-butyraldehyde as a colorless oil (2.1 g, 47%).

Similar transformations have been reported in US6531494 and the procedures described were used without modifications.

### Step C

In a manner similar to the method described in **Example 1a**, glycine methyl ester hydrochloride (1.25 g, 10 mmol) was reacted with 3-(4-methoxy-phenyl)-3-methyl-butyraldehyde (2.1 g, 10 mmol) and triethylamine (2.2 g, 20 mmol) in CH₂Cl₂ at room temperature for 5 h to give [4-(4-methoxy-phenyl)-3,3-dimethyl-but-(E)-ylideneamino]-acetic acid methyl ester as a colorless oil (2.7 g, 97%).

### Example 126b

Preparation of intermediate rac-(2R,3S,4R,5S)-3-(3-chloro-2-fluoro-phenyl)-4-(4-chloro-2-fluoro-phenyl)-4-cyano-5-[3-(4-methoxy-phenyl)-2,2-dimethyl-propyl]-pyrrolidine-2-carboxylic acid methyl ester

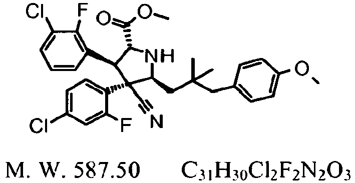

In a manner similar to the method described in **Example 1c,** [4-(4-methoxy-phenyl)-3,3-dimethyl-but-(E)-ylideneamino]-acetic acid methyl ester prepared in **Example 126a** (2.7 g, 9.7 mmol) was reacted with (Z)-3-(3-chloro-2-fluoro-phenyl)-2-(4-chloro-2-fluoro-phenyl)-acrylonitrile (3.1 g, 10 mmol) prepared in **Example 52a**, AgF (1.27 g, 10 mmol), and triethylamine (6 g, 60 mmol) in dichloromethane (100 mL) at room temperature for 20 h to give rac-(2R,3S,4R,5S)-3-(3-chloro-2-fluoro-phenyl)-4-(4-chloro-2-fluoro-phenyl)-4-cyano-5-[3-(4-methoxy-phenyl)-2,2-dimethyl-propyl]-pyrrolidine-2-carboxylic acid methyl ester as a yellow solid (4 g, 70%).

### Example 126c

### Preparation of intermediate rac-(2R,3S,4R,5S)-3-(3-chloro-2-fluoro-phenyl)-4-(4-chloro-2-fluoro-phenyl)-4-cyano-5-[3-(4-methoxy-phenyl)-2,2-dimethyl-propyl]-pyrrolidine-2-carboxylic acid

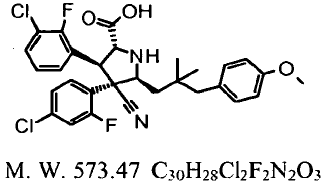

To rac-(2R,3S,4R.SS)-3-(3-chloro-2-fluoro-phenyl)-4-(4-chloro-2-fluoro-phenyl)-4-cyano-5-[3-(4-methoxy-phenyl)-2,2-dimethyl-propyl]-pyrrolidine-2-carboxylic acid methyl ester prepared in **Example 126b** (4 g, 6.8 mmol) in tetrahydrofuran (60 mL) was added an aqueous solution (1 N) of NaOH (20 mL, 20 mmol) and methanol (20 mL). The reaction mixture was stirred at room temperature for 3 h. The "pH" of the mixture was adjusted to ∼4-5 by aqueous HCl solution. The mixture was concentrated. The residue was partitioned between ethyl acetate and water. The organic layer was separated, washed with water, brine, dried over MgSO₄, concentrated to give rac-(2R,3S,4R,5S)-3-(3-chloro-2-fluoro-phenyl)-4-(4-chloro-2-fluoro-phenyl)-4-cyano-5-[3-(4-methoxy-phenyl)-2,2-dimethyl-propyl]-pyrrolidine-2-carboxylic acid as a light yellow solid (4 g, 100%)

### Example 126d

### Preparation of rac-(2R,3S,4R,SS)-3-(3-chloro-2-fluoro-phenyl)-4-(4-chloro-2-fluoro-phenyl)-4-cyano-5-[3-(4-methoxy-phenyl)-2,2-dimethyl-propyl]-pyrrolidine-2-carboxylic acid ((S)-3,4-dihydroxy-butyl)-amide

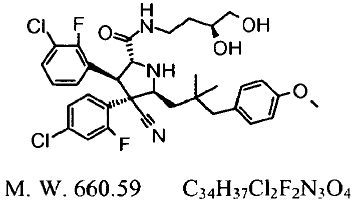

In a manner similar to the method described in **Examples 42c and 42d**, rac-(2R,3S,4R,5S)-3-(3-chloro-2-fluoro-phenyl)-4-(4-chloro-2-fluoro-phenyl)-4-cyano-5-[3-(4-methoxy-phenyl)-2,2-dimethyl-propyl]-pyrrolidine-2-carboxylic acid
prepared in **Example 126c** (0.5 g, 0.87 mmol) was reacted with 2-((S)-2,2-dimethyl-[1,3]dioxolan-4-yl)-ethylamine (0.25 g, 1.7 mmol), HATU (0.6 g, 1.7 mmol) and iPr₂NEt (0.45 mL, 2.6 mmol) in CH₂Cl₂ at room temperature for 20 h, then reacted with aqueous HCl solution in tetrahydrofuran at room temperature to give rac-(2R,3S,4R,5S)-3-(3-chloro-2-fluoro-phenyl)-4-(4-chloro-2-fluoro-phenyl)-4-cyano-5-[3-(4-methoxy-phenyl)-2,2-dimethyl-propyl]-pyrrolidine-2-carboxylic acid ((S)-3,4-dihydroxy-butyl)-amide as a white solid (0.16 g, 29%). HRMS (ES⁺) m/z Calcd for C₃₄H₃₇Cl₂F₂N₃O₄+ H [(M+H)⁺]: 660.2202, found: 660.2198.

### Example 127a

### Preparation of intermediate [3-(1-benzyl-1,2,3,6-tetrahydro-pyridin-4-yl)-3-methyl-but-(E)-ylideneamino]-acetic acid tert-butyl ester

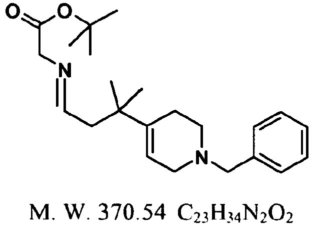

### Step A

To a tetrahydrofuran solution (Aldrich, 1.8 M) of LDA (60 mL, 109 mmol) at -50 °C was added isobutyric acid ethyl ester (Alfa) (12.2 mL, 91 mmol) dropwise. The reaction mixture was stirred -50 °C for 1 h, then a tretrahydrofuran solution (10 mL) of 1-benzyl-piperidin-4-one (12 mL, 68 mmol) was added dropwise. The reaction mixture was warmed up to room temperature and stirred for 18 h. Aqueous saturated NH₄Cl was added to quench the reaction. The mixture was extracted with ethyl ether. The organic layer was separated, and the aqueous layer was extracted with ethyl ether. The organic layers were combined, washed with brine, water, dried over MgSO₄, and concentrated to give 2-(1-benzyl-4-hydroxy-piperidin-4-yl)-2-methyl-propionic acid ethyl ester as an orange oil (18.5 g, 89%).

### Step B

To a solution of 2-(1-benzyl-4-hydroxy-piperidin-4-yl)-2-methyl-propionic acid ethyl ester ( 18.5 g, 61 mmol) in chloroform (75 mL) was added thionyl chloride (8.9 mL, 120 mmol) and dimethylformamide (0.17 mL). The reaction mixture was heated at 100 °C for 18 h, then cooled to room temperature and concentrated. To the resulting residue was added aqueous NaOH solution (10 N) to adjust the "pH" of the mixture to basic. The mixture was then extracted with ethyl ether twice. The combined organic extracts were washed with water, brine, dried over MgSO₄, and concentrated to give 2-(1-benzyl-1,2,3,6-tetrahydro-pyridin-4-yl)-2-methyl-propionic acid ethyl ester as a brown oil (13 g, 75%).

### Step C

To a solution of 2-(1-benzyl-1,2,3,6-tetrahydro-pyridin-4-yl)-2-methyl-propionic acid ethyl ester (6 g, 21 mmol) in anhydrous tetrahydrofuran 75 mL) at 0 °C was added a tetrhydrofuran solution (1 M) of LiAlH₄ (84 mL, 84 mmol) under nitrogen. The reaction mixture was heated at reflux for 3 h, then cooled to room temperature. Water and aqueous NaOH solution (2N) was added. The mixture was filtered to remove the precipitate, and the filtrate was concentrated. Water was added, and the mixture was extracted with ethyl acetate. The organic layer were separated, washed with water, aqueous HCl solution, brine, dried over MgSO₄, and concentrated to give 2-(1-benzyl-1,2,3,6-tetrahydro-pyridin-4-yl)-2-methyl-propan-1-ol as a brown oil (4.73 g, 77%).

### Step D

To a solution ofoxalyl chloride (2.46 mL, 28 mmol) (Aldrich) in dichloromethane (150 mL) at - 78 °C was added the solution of dimethyl sulfoxide (4 mL, 56 mmol) in dichloromethane (25 mL) dropwise. After 5 mins, the solution of 2-(1-benzyl-1,2,3,6-tetrahydro-pyridin-4-yl)-2-methyl-propan-1-ol (6.3 g, 25.6 mmol) in dichloromethane (25 mL) was added dropwise. The reaction mixture was stirred at -78 °C for 15 min. Triethylamine (12.8 mL, 92 mmol) was added and the reaction mixture was slowly warmed up to room temperature and stirred at room temperature for 45 min. Then water was added. The organic layers were separated, and the aqueous layer was extracted with dichloromethane. The organic layers were combined, washed with 10% of HCl, saturated NaHCO₃, brine, dried over MgSO₄, and concentrated to give 2-(1-benzyl-1,2,3,6-tetrahydro-pyridin-4-yl)-2-methyl-propionaldehyde as a brown oil (Yield: 5.6 g, 89%).

### Step E

To a mixture of methoxymethyl triphenylphosphonium chloride (12.6 g, 37 mmol) in anhydrous tetrahydrofuran (50 mL) at 0 °C was a tetrahydrofuran solution (Aldrich, I M) of LiHMDS (46 mL, 46 mmol) dropwise. After the addition was finished, the reaction mixture was stirred at 0 °C for 20 min. Then a tetrahydrofuran solution (40 mL) of 2-(1-benzyl-1,2,3,6-tetrahydro-pyridin-4-yl)-2-methyl-propionaldehyde (5.6 g, 23 mmol) was added. The reaction mixture was stirred at 0 °C for 2 h. Water was added to quench the reaction. The mixture was extracted with ethyl acetate twice. The organic layers were combined, concentrated. The residue was dissolved into a solution of aqueous HCl solution (2 N, 50 mL, 100 mmol) and tetrahydrofuran (50 mL). The reaction mixture was heated at reflux for 30 min, then cooled to room temperature and concentrated. The residue partitioned between ethyl acetate and water. The organic layer was separated, concentrated. The residue was purified by chromatography (EtOAc:hexanes=1:3) to give 3-(1-benzyl-1,2,3,6-tetrahydro-pyridin-4-yl)-3-methyl-butyraldehyde as a yellow oil (1.65 g, 28%).

### Step F

In a manner similar to the method described in **Example 1a**, glycine tert-butyl ester (0.84 g, 6.4 mmol) was reacted with 3-(1-benzyl-1,2,3,6-tetrahydro-pyridin-4-yl)-3-methyl-butyraldehyde (1.65 g, 6.4 mmol) in CH₂Cl₂ at room temperature for 5 h to give [3-(1-benzyl-1,2,3,6-tetrahydro-pyridin-4-yl)-3-methyl-but-(E)-ylideneamino]-acetic acid tert-butyl ester as a yellow oil (2.4 g, 100%).

### Example 127b

### Preparation of intermediate rac-(2R,3S,4R,5S)-5-[2-(1-benzyl-1,2,3,6-tetrahydro-pyridin-4-yl)-2-methyl-propyl]-3-(3-chloro-2-fluoro-phenyl)-4-(4-chloro-2-fluoro-phenyl)-4-cyano-pyrrolidine-2-carboxylic acid tert-butyl ester

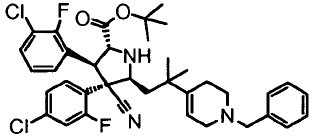

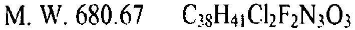

In a manner similar to the method described in **Example 100b**, [3-(1-benzyl-1,2,3,6-tetrahydropyridin-4-yF)-3-methyl-but-(E)-ylideneamino]-acetic acid tert-butyl ester prepared in **Example 127a** (2.4 g, 6.4 mmol) was reacted with (Z)-3-(3-chloro-2-fluoro-phenyl)-2-(4-chloro-2-fluoro-phenyl)-acrylonitrile (1.8 g, 6.4 mmol) prepared in **Example 52a**, AgF (1.3 g, 10 mmol), and triethylamine (2 mL, 14.5 mmol) in dichloromethane (100 mL) at room temperature for 18 h, followed by the reaction with DBU (7 ml) in tert-butanol (30 mL) at 100 °C for 2 h to give rac-(2R,3S,4R,5S)-5-[2-(1-benzyl-1,2,3,6-tetrahydro-pyridin-4-yl)-2-methyl-propyl]-3-(3-chloro-2-fluoro-phenyl)-4-(4-chloro-2-fluoro-phenyl)-4-cyano-pyrrolidine-2-carboxylic acid tert-butyl ester as a light yellow solid (3.2 g, 81%).

### Example 127c

### Preparation of intermediate rac-(2R,3S,4R,5S)-5-[2-(1-benzyl-1,2,3,6-tetrahydro-pyridin-4-yl)-2-methyl-propyl]-3-(3-chloro-2-fluoro-phenyl)-4-(4-chloro-2-fluoro-phenyl)-4-cyano-pyrrolidine-2-carboxylic acid trifluoroacetic acid

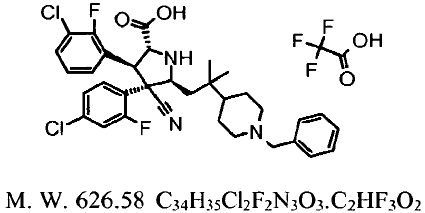

In a manner similar to the method described in **Example 25a**, rac-(2R,3S,4R,5S)-5-[2-(1-benzyl-1,2,3,6-tetrahydro-pyridin-4-yl)-2-methyl-propyl]-3-(3-chloro-2-fluoro-phenyl)-4-(4-chloro-2-fluoro-phenyl)-4-cyano-pyrrolidine-2-carboxylic acid tert-butyl ester prepared in **Example 127b** (1.5 g, 2.2 mmol) was reacted with trifluoroacetic acid in dichloromethane at room temperature to give rac-(2R,35,4R,5S)-5-[2-(1-benzyl-1,2,3,6-tetrahydro-pyridin-4-yl)-2-methyl-propyl]-3-(3-chloro-2-fluoro-phenyl)-4-(4-chloro-2-fluoro-phenyl)-4-cyano-pyrrolidine-2-carboxylic acid trifluoroacetic acid as a yellow solid (1.6 g, 98%).

### Example 127d

### Preparation of intermediate rac-(2R,3S,4R,5S)-5-[2-(1benzyl-1,2,3,6-tetrahydro-pyridin-4-yl)-2-methyl-propyl]-3-(3-chloro-2-fluoro-phenyl)-4-(4-chloro-2-fluoro-phenyl)-4-cyano-pyrrolidine-2-carboxylic acid [2-((S)-2,2-dimethyl-[1,3]dioxolan-4-yl)-ethyl]-amide

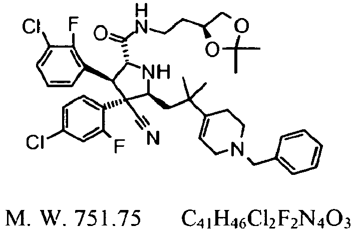

In a manner similar to the method described in **Examples 42c**, rac-(2R,3S,4R,5S)-5-[2-(1-benzyl-1,2,3,6-tetrahydro-pyridin-4-yl)-2-methyl-propyl]-3-(3-chloro-2-fluoro-phenyl)-4-(4-chloro-2-fluoro-phenyl)-4-cyano-pyrrolidine-2-carboxylic acid trifluoroacetic acid prepared in **Example 127c** (1.6 g, 2.2 mmol) was reacted with 2-((S)-2,2-dimethyl-[1,3]dioxolan-4-yl)-ethylamine (0.94 g, 6.5 mmol), HATU (2.5 g, 6.5 mmol) and iPr₂NEt (2.3 mL, 13 mmol) in CH₂Cl₂ at room temperature for 20 h to give rac-(2R,3S,4R,5S)-5-[2-(1-benzyl-1,2,3,6-tetrahydro-pyridin-4-yl)-2-methyl-propyl]-3-(3-chloro-2-fluoro-phenyl)-4-(4-chloro-2-fluoro-phenyl)-4-cyano-pyrrolidine-2-carboxylic acid [2-((S)-2,2-dimethyl-[1,3]dioxolan-4-yl)-ethyl]-amide as a light yellow gum (1 g, 83%).

### Example 127e

### Preparation of rac-(2R,3S,4R,5S)-5-[2-(1-benzyl-1,2,3,6-tetrahydro-pyridin-4-yl)-2-methylpropyl]-3-(3-chloro-2-fluoro-phenyl)-4-(4-chloro-2-fluoro-phenyl)-4-cyano-pyrro lid ine-2-carboxylic acid ((S)-3,4-dihydroxy-butyl)-amide

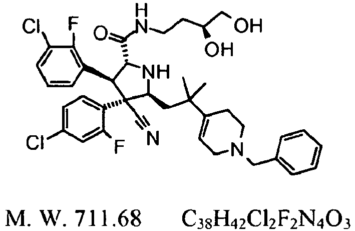

In a manner similar to the method described in **Examples 42d**, rac-(2R,3S,4R,5S)-5-[2-(1-benzyl-1,2,3,6-tetrahydro-pyridin-4-yl)-2-methyl-propyl]-3-(3-chloro-2-fluoro-phenyl)-4-(4-chloro-2-fluoro-phenyl)-4-cyano-pyrrolidine-2-carboxylic acid [2-((S)-2,2-dimethyl-[1,3]dioxolan-4-yl)-ethyl]-amide prepared in **Example 127d** (1 g, 1.3 mmol) was reacted with aqueous HCl solution (1 N, 5 mL, 5 mol) in tetrahydrofuran (5 mL) at room temperature for 2 h to give rac-(2R,3S,4R,5S)-5-[2-(1-benzyl- 1,2,3,6-tetrahydro-pyridin-4-yl)-2-methyl-propyl]-3-(3-chloro-2-fluoro-phenyl)-4-(4-chloro-2-fluoro-phenyl)-4-cyano-pyrrolidine-2-carboxylic acid ((S)-3,4-dihydroxy-butyl)-amide as a white solid (0.3 g, 32%).

HRMS (ES⁺) m/z Calcd for C₃₈H₄₂Cl₂F₂N₄O₃+ H [(M+H)⁺]: 711.2675, found: 711. 2675.

### Example 128

### Preparation of rac-(2R,3S,4R,5S)-5-[2-(1-benzyl-piperidin-4-yl)-2-methyl-propyl]-3-(3-chloro-2-fluoro-phenyl)-4-(4-chloro-2-fluoro-phenyl)-4-cyano-pyrrolidine-2-carboxylic acid ((S)-3,4-dihydroxy-butyl)-amide

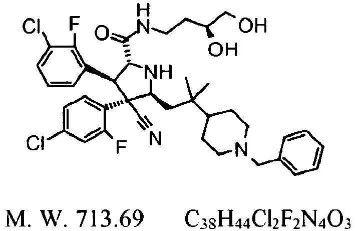

In a manner similar to the method described in **Examples 118a**, rac-(2R,3S,4R,5S)-5-[2-(1-benzyl-1,2,3,6-tetrahydro-pyrid in-4-yl)-2-methyl-propyl]-3-(3-chloro-2-fluoro-phenyl)-4-(4-chloro-2-fluoro-phenyl)-4-eyano-pyrrolidine-2-carboxylic acid ((S)-3,4-dihydroxy-butyl)-amide prepared in **Example 127e** (60 mg, 0.08 mmol) was treated with PtO₂ and H₂ in ethyl acetate to give rac-(2R,3S,4R,5S)-5-[2-(1-benzyl-piperidin-4-yl)-2-methyl-propyl]-3-(3-chloro-2-fluoro-phenyl)-4-(4-chloro-2-fluoro-phenyl)-4-cyano-pyrrolidine-2-carboxylic acid ((S)-3,4-dihydroxybutyl)-amide as a off white solid (15 mg, 25%)

HRMS (ES⁺) m/z Calcd for C₃₈H₄₄Cl₂F₂N₄O₃+H [(M+H)⁺]: 713.2832, found: 713.2837.

### Example 129a

### Preparation of intermediate [3-(3,6-dihydro-2H-pyran-4-yl)-3-methyl-but-(E)-ylideneamino]-acetic acid tert-butyl ester

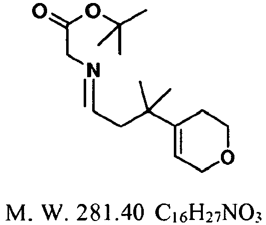

### Step A

To a hexane solution (Aldrich, 2 M) of LDA (78 mL, 160 mmol) in tetrahydrofuran (100 mL) at -50 °C was added a solution of isobutyric acid ethyl ester (Alfa) (17 mL, 127 mmol) in tetrahydrofuran (20 mL) dropwise. The reaction mixture was stirred -50 °C for 1 h, then a tretrahydrofuran solution (10 mL) of tetrahydro-pyran-4-one (Aldrich) (9.8 g, 98 mmol) was added dropwise. The reaction mixture was warmed up to room temperature and stirred for 18 h. Aqueous saturated NH₄Cl was added to quench the reaction. The mixture was extracted with ethyl ether. The organic layer was separated, and the aqueous layer was extracted with ethyl ether. The organic layers were combined, washed with brine, water, dried over MgSO₄, and concentrated to give 2-(4-hydroxy-tetrahydro-pyran-4-yl)-2-methyl-propionic acid ethyl ester as a yellow oil (19.5 g, 92%).

### Step B

To a solution of 2-(4-hydroxy-tetrahydro-pyran-4-yl)-2-methyl-propionic acid ethyl ester (19.5 g, 90 mmol) in chloroform (100 mL) was added thionyl chloride (13.3 mL, 180 mmol) and dimethylformamide (0.28 mL). The reaction mixture was heated at 100 °C for 18 h, then cooled to room temperature and concentrated. To the resulting residue was added aqueous NaOH solution (10 N) to adjust the "pH" of the mixture to basic. The mixture was then extracted with ethyl acetate twice. The combined organic extracts were washed with water, brine, dried over MgSO₄, and concentrated to give 2-(3,6-dihydro-2H-pyran-4-yl)-2-methyl-propionic acid ethyl ester as a brown oil (17.6 g, 99%).

### Step C

To a solution of 2-(3,6-dihydro-2H-pyran-4-yl)-2-methyl-propionic acid ethyl ester (6 g, 30 mmol) in anhydrous tetrahydrofuran (75 mL) at 0 °C was added a tetrhydrofuran solution (1 M) of LiAlH₄ (100 mL, 100 mmol) under nitrogen. The reaction mixture was heated at reflux for 3 h, then cooled to room temperature. Water and aqueous NaOH solution (2N) was added. The mixture was filtered to remove the precipitate, and the filtrate was concentrated. Water was added, and the mixture was extracted with ethyl acetate. The organic layer were separated, washed with water, aqueous HCl solution, brine, dried over MgSO₄, and concentrated to give 2-(3,6-dihydro-2H-pyran-4-yl)-2-methyl-propan-1-ol as a brown oil (4.63 g, 98%).

### Step D

To a solution of oxalyl chloride (2.84 mL, 33 mmol) (Aldrich) in dichloromethane (150 mL) at - 78 °C was added the solution of dimethyl sulfoxide (4.6 mL, 65 mmol) in dichloromethane (25 mL) dropwise. After 5 mins, the solution of 2-(3,6-dihydro-2H-pyran-4-yl)-2-methyl-propan-1-ol (4.6 g, 29 mmol) in dichloromethane (25 mL) was added dropwise. The reaction mixture was stirred at -78 °C for 15 min. Triethylamine (14.8 mL, 110 mmol) was added and the reaction mixture was slowly warmed up to room temperature and stirred at room temperature for 45 min. Then water was added. The organic layers were separated, and the aqueous layer was extracted with dichloromethane. The organic layers were combined, washed with 10% of HCl, saturated NaHCO₃, brine, dried over MgSO₄, and concentrated to give 2-(3,6-dihydro-2H-pyran-4-yl)-2-methyl-propionaldehyde as a brown oil (Yield: 5.6 g, 89%).

### Step E

To a mixture of methoxymethyl triphenylphosphonium chloride (31.3 g, 91 mmol) in anhydrous tetrahydrofuran (150 mL) at 0 °C was a tetrahydrofuran solution (Aldrich, 1 M) of LiHMDS (110 mL, 110 mmol) dropwise. After the addition was finished, the reaction mixture was stirred at 0 °C for 20 min. Then a tetrahydrofuran solution (40 mL) of 2-(3,6-dihydro-2H-pyran-4-yl)-2-methyl-propionaldehyde (4.4 g, 28.5 mmol) was added. The reaction mixture was stirred at 0 °C for 1 h. Water was added to quench the reaction. The mixture was extracted with ethyl acetate twice. The organic layers were combined, concentrated. The residue was dissolved into a solution of aqueous HCl solution (2 N, 50 mL, 100 mmol) and tetrahydrofuran (50 mL). The reaction mixture was heated at reflux for 30 min, then cooled to room temperature and concentrated. The residue partitioned between ethyl acetate and water. The organic layer was separated, concentrated. The residue was purified by chromatography (EtOAc:hexanes=1:3) to give 3-(3,6-dihydro-2H-pyran-4-yl)-3-methyl-butyraldehyde as a brown oil (2.61 g, 54%).

### Step F

In a manner similar to the method described in **Example 1a**, glycine tert-butyl ester (2 g, 15.5 mmol) was reacted with 3-(3,6-dihydro-2H-pyran-4-yl)-3-methyl-butyraldehyde (2.6 g, 15.5 mmol) in CH₂Cl₂ at room temperature for 5 h to give [3-(3,6-dihydro-2H-pyran-4-yl)-3-methyl-but-(E)-ylideneamino]-acetic acid tert-butyl ester as a yellow oil (4.3 g, 100%).

### Example 129b

### Preparation of intermediate rac-(2R,3S,4R,5S)-3-(3-chloro-2-fluoro-phenyl)-4-(4-chloro-2-fluoro-phenyl)-4-cyano-5-[2-(3,6-dihydro-2H-pyran-4-yl)-2-methyl-propyl]-pyrrolidine-2-carboxylic acid tert-butyl ester

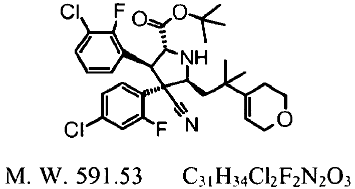

In a manner similar to the method described in **Example 100b**, [3-(3,6-dihydro-2H-pyran-4-yl)-3-methyl-but-(E)-ylideneamino]-acetic acid tert-butyl ester prepared in **Example 129a** (4.3 g, 15.5 mmol) was reacted with (Z)-3-(3-chloro-2-fluoro-phenyl)-2-(4-chloro-2-fluoro-phenyl)-acrylonitrile (3.8 g, 12.4 mmol) prepared in **Example 52a**, AgF (2.4 g, 19 mmol), and triethylamine (4.3 mL, 31mmol) in dichloromethane (100 mL) at room temperature for 18 h, followed by the reaction with DBU (19 ml) in tert-butanol (18 mL) at 100 °C for 2 h to give rac-(2R,3S,4R,5S)-3-(3-chloro-2-fluoro-phenyl)-4-(4-chloro-2-fluoro-phenyl)-4-cyano-5-[2-(3,6-dihydro-2H-pyran-4-yl)-2-methyl-propyl]-pyrrolidine-2-carboxylic acid tert-butyl ester as a yellow gum(5.5 g, 75%).

### Example 129c

### Preparation of intermediate rac-(2R,3S,4R,5S)-3-(3-chloro-2-fluoro-phenyl)-4-(4-chloro-2-fluoro-phenyl)-4-cyano-5-[2-(3,6-dihydro-2H-pyran-4-yl)-2-methyl-propyl]-pyrrolidine-2-carboxylic acid trifluoroacetic acid

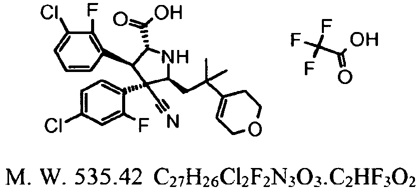

In a manner similar to the method described in **Example 25a**, rac-(2R,3S,4R,5S)-3-(3-chloro-2-fluoro-phenyl)-4-(4-chloro-2-fluoro-phenyl)-4-cyano-5-[2-(3,6-dihydro-2H-pyran-4-yl)-2-methyl-propyl]-pyrrolidine-2-carboxylic acid tert-butyl ester prepared in **Example 129b** (5.5 g, 9.29 mmol) was reacted with trifluoroacetic acid in dichloromethane at room temperature to give rac-(2R,3S,4R,5S)-3-(3-chloro-2-fluoro-phenyl)-4-(4-chloro-2-fluoro-phenyl)-4-cyano-5-[2-(3,6-dihydro-2H-pyran-4-yl)-2-methyl-propyl]-pyrrolidine-2-carboxylic acid trifluoroacetic acid as a dark solid (6 g, 99%).

### Example 129d

### Preparation of intermediate rac-(2R,3S,4R,5S)-3-(3-chloro-2-fluoro-phenyl)-4-(4-chloro-2-fluoro-phenyl)-4-cyano-5-[2-(3,6-dihydro-2H-pyran-4-yl)-2-methyl-propyl]-pyrro lid ine-2-carboxylic acid [2-((S)-2,2-dimethyl-[1,3]dioxolan-4-yl)-ethyl]-amide

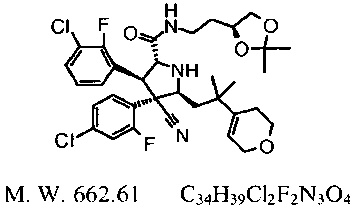

In a manner similar to the method described in **Examples 42c**, rac-(2R,3S,4R,5S)-3-(3-chloro-2-fluoro-phenyl)-4-(4-chloro-2-fluoro-phenyl)-4-cyano-5-[2-(3,6-dihydro-2H-pyran-4-yl)-2-methyl-propyl]-pyrrolidine-2-carboxylic acid trifluoroacetic acid prepared in **Example 129c** (0.8 g, 1.2 mmol) was reacted with 2-((S)-2,2-dimethyl-[1,3]dioxolan-4-yl)-ethylamine (0.36 g, 2.5 mmol), HATU (0.84 g, 2.2 mmol) and iPr₂NEt (0.64 mL, 3.7 mmol) in CH₂Cl₂ at room temperature for 20 h to give rac-(2R,3S,4R,5S)-5-[2-(1-benzyl-1,2,3,6-tctrahydro-pyridin-4-yl)-2-methyl-propyl]-3-(3-chloro-2-fluoro-phenyl)-4-(4-chloro-2-fluoro-phenyl)-4-cyanopyrrolidine-2-carboxylic acid [2-((S)-2,2-dimethyl-[1,3]dioxolan-4-yl)-ethyl]-amide as a off white gum (0.6 g, 74%).

### Example 129e

### Preparation of rac-(2R,3S,4R,SS)-3-(3-chloro-2-fluoro-phenyl)-4-(4-chloro-2-fluoro-phenyl)-4-cyano-5-[2-(3,6-dihydro-2H-pyran-4-yl)-2-methyl-propyl]-pyrrolidine-2-carboxylic acid ((S)-3,4-dihydroxy-butyl)-amide

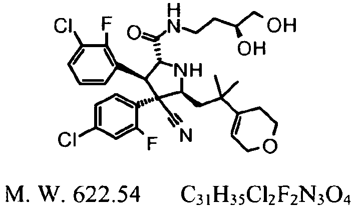

In a manner similar to the method described in **Examples 42d,** rac-(2R,3S,4R,5S)-3-(3-chloro-2-fluoro-phenyl)-4-(4-chloro-2-fluoro-phenyl)-4-cyano-5-[2-(3,6-dihydro-2H-pyran-4-yl)-2-methyl-propyl]-pyrrolidine-2-carboxylic acid [2-((S)-2,2-dimethyl-[1,3]dioxolan-4-yl)-ethyl]-amide prepared in **Example 129d** (0.6 g, 0.9 mmol) was reacted with aqueous HCl solution (1 N, 3 mL, 3 mol) in tetrahydrofuran (7 mL) at room temperature for 2 h to give rac-(2R,3S,4R,5S)-3-(3-chloro-2-fluoro-phenyl)-4-(4-chloro-2-fluoro-phenyl)-4-cyano-5-[2-(3,6-dihydro-2H-pyran-4-yl)-2-methyl-propyl]-pyrrolidine-2-carboxylic acid ((S)-3,4-dihydroxy-butyl)-amide as a white solid (0.52 g, 93%).

HRMS (ES⁺) m/z Calcd for C₃₁H₃₅Cl₂F₂N₃O₄+ H [(M+H)⁺]: 622.2046, found: 622.2046.

### Example 130

### Preparation of rac-(2R,3S,4R,5S)-3-(3-hloro-2-fluoro-phenyl)-4-(4-chloro-2-fluoro-phenyl)-4-cyano-5-[2-methyl-2-(tetrahydro-pyran-4-yl)-propyl]-pyrrolidine-2-carboxylic acid ((S)-3,4-dihydroxy-butyl)-amide

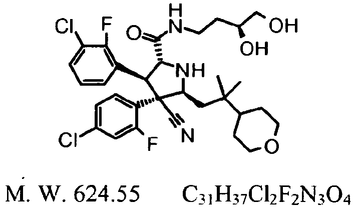

In a manner similar to the method described in **Examples 118a**, rac-(2R,3S,4R,5S)-3-(3-chloro-2-fluoro-phenyl)-4-(4-chloro-2-fluoro-phenyl)-4-cyano-5-[2-(3,6-dihydro-2H-pyran-4-yl)-2-methyl-propyl]-pyrrolidine-2-carboxylic acid ((S)-3,4-dihydroxy-butyl)-amide prepared in **Example 127e** (0.28 g, 0.45 mmol) was treated with PtO₂ and H₂ in ethyl acetate to give rac-(2R,3S,4R,5S)-3-(3-hloro-2-fluoro-phenyl)-4-(4-chloro-2-fluoro-phenyl)-4-cyano-5-(2-methyl-2-(tetrahydro-pyran-4-yl)-propyl]-pyrrolidine-2-carboxylic acid ((S)-3,4-dihydroxy-butyl)-amide as a off white solid (0.15 g, 54%)

HRMS (ES⁺) m/z Calcd for C₃₁H₃₇Cl₂F₂N₃O₄+ H [(M+H)⁺]: 624.2202, found: 624.2207.

### Example 131a

### Preparation of intermediate 2-((S)-2,2,5,5-ttramethyl-[1,3]dioxolan-4-yl)-ethylamine

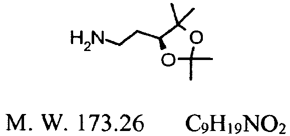

### Step A.

To a suspension of L-(-)-malic aicd (Aldrich) (10.3 g, 77 mmol) in 2,2-dimethoxypropane (20 mL) was added p-toluenesulfonic acid monohydrate (0.4 g). The reaction mixture was stirred at room temperature for 30 min. The mixture was partitioned between water and dichloromethane. The organic layer was separated, the aqueous layer was extracted with dichloromethane. The organic layers were combined, washed with water, brine, dried over MgSO₄, and concentrated to give ((S)-2,2-dimethyl-5-oxo-[1,3]dioxolan-4-yl)-acetic acid as a white solid (10.1 g, 75%).

### Step B

To the solution of ((S)-2,2-dimethyl-5-oxo-[1,3]dioxolan-4-yl)-acetic acid (10.1 g, 58 mmol) in anhydrous tetrahydrofuran (20 mL) at 0 °C was added a solution of BH₃.THF (1 M, 70 mL, 70 mmol). The reaction mixture was stirred at room temperature for 2 h. Aqueous HCl solution was added, and the mixture was extracted with ethyl acetate. The organic layer was separated, washed with saturated aqueous NaHCO₃ solution, brine, dried over MgSO₄, and concentrated. The residue was purified by chromatography (EtOAc) to give (S)-5-(2-hydroxy-ethyl)-2,2-dimethyl-[1,3]dioxolan-4-one as a colorless oil (6.8 g, 72%).

### Step C

A mixture of(S)-5-(2-hydroxy-ethyl)-2,2-dirnethyl-[1,3]dioxolan-4-one (6.8 g, 42 mmol) and imidazole (7.5 g, 107 mmol) in dimethylformamide (40 mL) was added tert-butyldimethylchlorosilane (7 g, 45 mmol). The reactiom mixture was stirred at room temperature for 18 h. Water was added. The organic layer was separated, the aqueous layer was then extracted with ethyl acetate twice. The combined organic layers wer washed with brine, dried over MgSO₄, concentrated to give (S)-5-[2-(tert-butyl-dimethyl-silanyloxy)-ethyl]-2,2-dimethyl-[1,3]dioxolan-4-one as a colorless oil (8.6 g, 74%).

### Step D

To a solution of (S)-5-[2-(tert-butyl-dimethyl-silanyloxy)-ethyl]-2,2-dimethyl-[1,3]dioxolan-4-one (8.5 g, 31 mmol) in diethyl ether (200 mL) at 0 °C was added a diethyl ether (1.6 M) solution of methyllithium (50 mL, 78 mmol) dropwise. The reaction mixture was stirred at 0 °C for 30 min. The mixture was poured into aqueous saturated NH₄Cl solution. The organic layer was separated, and the aqueous layer was extracted with ethyl acetate. The organic layers were combined, washed with brine, dried over MgSO₄, and concentrated to give (S)-5-(tert-butyldimethyl-silanyloxy)-2-methyl-pentane-2,3-diol as a yellow oil (Yield: 6.8 g, 88%).

### Step E

To a suspension of (S)-5-(tert-butyl-dimethyl-silanyloxy)-2-methyl-pentane-2,3-diol (6.8 g, 27 mmol) in 2,2-dimethoxypropane (35 mL) was added p-toluenesulfonic acid monohydrate (0.2g). The reaction mixture was stirred at room temperature for 30 min. The mixture was partitioned between water and dichloromethane. The organic layer was separated, the aqueous layer was extracted with dichloromethane. The organic layers were combined, washed with water, brine, dried over MgSO₄, and concentrated. The residue was purified by chromatography (EtOAc:hexanes=1:20) to give tert-butyl-dimethyl-[2-((S)-2,2,5,5-tetramethyl-[1,3]dioxolan-4-yl)-ethoxy]-silane as a yellow oil (4.56 g, 58%).

### Step F

To the solution of tert-butyl-dimethyl-[2-((S)-2,2,5,5-tetramethyl-[1,3]dioxolan-4-yl)-ethoxy]-silane (4.56 g, 15.8 mmol) in tetrahydrofuran (20 mL) at 0 °C was added a solution of tetrabutylammonium fluoride (1 M, 20 mL, 20 mmol). The reaction mixture was stirred at room temperature for 1 h. Water was added, and the mixture was extracted with ethyl acetate. The organic layer was separated, washed with saturated aqueous NaHCO₃ solution, brine, dried over MgSO₄, and concentrated to give 2-((S)-2,2,5,5-tetramethyl-[1,3]dioxolan-4-yl)-ethanol as a yellow oil (2.7 g, 100%).

### Step G

To a solution of 2-((S)-2,2,5,5-tetramethyl-[1,3]dioxolan-4-yl)-ethanol (2.7 g, 15.8 mol) and triethylamine (4.6 g, 45 mmol) in dichloromethane (100 mL) at 0 °C was added methanesulfonyl chloride (2.7 g, 24 mmol) dropwise. The reaction mixture was stirred at 0 °C for 1.5h, then water was added. The organic layer was separated, washed with water, brine, dried over MgSO₄, concentrated to give methanesulfonic acid 2-((S)-2,2,5,5-tetramethyl-[1,3]dioxolan-4-yl)-ethyl ester as a yellow oil (2.5 g, 62%).

### Step H

To a solution of methanesulfonic acid 2-((S)-2,2,5,5-tetramethyl-[1,3]dioxolan-4-yl)-ethyl ester (2.5 g, 9.9 mmol) in N,N-dimethylformamide (50 mL) was added NaN₃ (6 g, 70 mmol). The reaction mixture was heated at 95 °C for 4 h. Then the mixture was partitioned between ethyl acetate and water. The organic layer was separated, washed with water, brine several times, dried over MgSO₄, concentrated to give (S)-5-(2-azido-ethyl)-2,2,4,4-tetramethyl-[1,3]dioxolane as a yellow oil (1.6 g, 80%).

### Step I

A suspension of (S)-5-(2-azido-ethyl)-2,2,4,4-tetramethyl-[1,3]dioxolane (1.6 g, 8 mmol) and PtO₂ (0.32 g) in ethyl acetate (15 mL) was vigorously shaken in a Parr under atmosphere of H₂ (50 psi) for 18 h. The mixture was filtered through a short pad of celite. The filtrate was concentrated to give 2-((S)-2,2,5,5-tetramethyl-[1,3]dioxolan-4-yl)-ethylamine as a colorless oil (1.3 g, 94%).

### Example 131b

### Preparation of rac-(2R,3S,4R,5S)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid ((S)-3,4-dihydroxy-4-methyl-pentyl)-amide

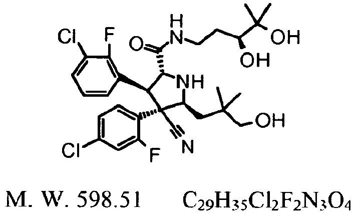

In a manner similar to the method described in **Examples 42c and 42d**, rac-(2R,3S,4R,5S)-3-(3-chloro-2-fluoro-phenyl)-4-(4-chloro-2-fluoro-phenyl)-4-cyano-5-(3-hydroxy-2,2-dimethylpropyl)-pyrrolidine-2-carboxylic acid trifluoroacetic acid prepared in **Example 105c** (0.82 g, 1.37 mmol) was reacted with 2-((S)-2,2,5,5-tetramethyl-[1,3]dioxolan-4-yl)-ethylamine prepared in **Example 131a** (0.5 g, 2.88 mmol), HATU (0.94 g, 2.5 mmol) and iPr₂NEt (1.2 mL, 6.9 mmol) in CH₂Cl₂ at room temperature for 20 h, then reacted with aqueous HCl solution in tetrahydrofuran at room temperature for 2 h to give rac-(2R,3S,4R,5S)-3-(3-chloro-2-fluoro-phenyl)-4-(4-chloro-2-fluoro-phenyl)-4-cyano-5-(3-hydroxy-2.2-dimethylpropyl)-pyrrolidine-2-carboxylic acid ((S)-3,4-dihydroxy-4-methyl-pentyl)-amide as a white solid (0.53 g, 70%).

HRMS (ES⁺) m/z Calcd for C₂₉H₃₅Cl₂F₂N₃O₄+ H [(M+H)⁺]: 598.2046, found: 598.2047.

### Example 131c

### Preparation of (2R,3S,4R,5S)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid ((S)-3,4-dihydroxy-4-methyl-pentyl)-amide

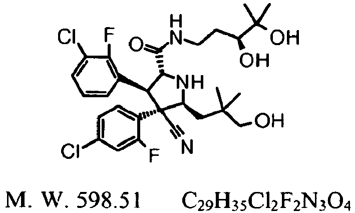

Rac-(2R,3S,4R,SS)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid ((S)-3,4-dihydroxy-4-methylpentyl)-amide (0.47 g) was separated by chiral SFC chromatography to provide chiral (2R,3S,4R,SS)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid ((S)-3,4-dihydroxy-4-methylpentyl)-amide as a white solid (0.18 g, 38%) and chiral (2S,3R,4S,5R)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid ((S)-3,4-dihydroxy-4-methyl-pentyl)-amide as a white solid (0.18 g, 38%). HRMS (ES⁺) m/z Calcd for C₂₉H₃₅Cl₂F₂N₃O₄+ H [(M+H)⁺]: 598.2046, found: 598.2045

### Example 132a

### Preparation of rac-(2R,35,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-4-methylpentyl)-amide -

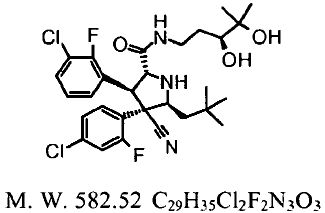

In a manner similar to the method described in **Examples 42c, 42d,** rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid trifluoroacetic acid prepared in **Example 52c** (0.67 g, 1.15 mmol) was reacted with 2-((S)-2,2,5,5-tetramethyl-[1,3]dioxolan-4-yl)-ethylamine prepared in **Example 131a** (0.4 g, 2.3 mmol), HATU (0.79 g, 2.1 mmol) and iPr₂NEt (1 mL, 5.8 mmol) in CH₂Cl₂ at room temperature for 20 h, then reacted with aqueous HCl solution in tetrahydrofuran at room temperature to give rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-4-methyl-pentyl)-amide as a white solid (0.29 g, 43%).

HRMS (ES⁺) m/z Calcd for C₂₉H₃₅Cl₂F₂N₃O₃+ H [(M+H)⁺]: 582.2097, found: 582.2098.

### Example 132b

### Preparation of (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-4-methylpentyl)-amide

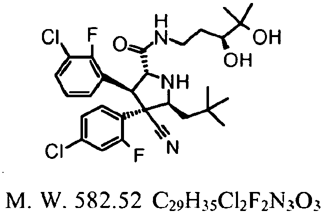

Rac-(2R,3S,4R,SS)-3-(3-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-4-methyl-pentyl)-amide (0.25 g) was separated by chiral SFC chromatography to provide chiral-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-4-methyl-pentyl)-amide as a white solid (103 mg, 41%) and chiral-(2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-4-methylpentyl)-amide as a white solid (114 mg, 45%).

HRMS (ES⁺) m/z Calcd for C₂₉H₃₅Cl₂F₂N₃O₃+ H [(M+H)⁺]: 582.2097, found: 582.2098.

### Example 133a

### Preparation of rac-(2R,3S,4R,5S)-3-(3-chloro-2-fluoro-phenyl)-4-(4-chloro-2-fluoro-phenyl)-4-cyano-5-(1-methyl-cyclohexylmethyl)-pyrrolidine-2-carboxylic acid ((S)-3,4-dihydroxy-4-methyl-pentyl)-amide

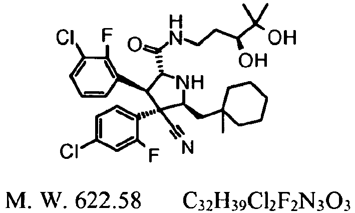

In a manner similar to the method described in **Examples 42c and 42d**, rac-(2R,3S,4R,5S)-3-(3-chloro-2-fluoro-phenyl)-4-(4-chloro-2-fluoro-phenyl)-4-cyano-5-(1-methyl-cyclohexylmethyl)-pyrrolidine-2-carboxylic acid trifluoroacetic acid prepared in **Example 102c** (0.72 g, 1.15 mmol) was reacted with. 2-((S)-2,2,5,5-tetramethyl-[1,3]dioxolan-4-yi)-ethylamine prepared in **Example 131a** (0.4 g, 2.3 mmol), HATU (0.79 g, 2.1 mmol) and iPr₂NEt (1 mL, 5.8 mmol) in CH₂Cl₂ at room temperature for 20 h, then reacted with aqueous HCl solution in tetrahydrofuran at room temperature to give rac-(2R,3S,4R,5S)-3-(3-chloro-2-fluoro-phenyl)-4-(4-chloro-2-fluoro-phenyl)-4-cyano-5-(1-methyl-cyclohexylmethyl)-pyrrolidine-2-carboxylic acid ((S)-3,4-dihydroxy-4-methyl-pentyl)-amide as a white solid (0.25 g, 42%).

HRMS (ES⁺) m/z Calcd for C₃₂H₃₉Cl₂F₂N₃O₃+ H [(M+H)⁺]: 622.2410, found: 622.2411.

### Example 133b

### Preparation of (2R,3S,4R,5S)-3-(3-chloro-2-fluoro-phenyl)-4-(4-chloro-2-fluoro-phenyl)-4-cyano-5-(1-methyl-cyclohexylmethyl)-pyrrolidine-2-carboxylic acid ((S)-3,4-dihydroxy-4-methyl-pentyl)-amide

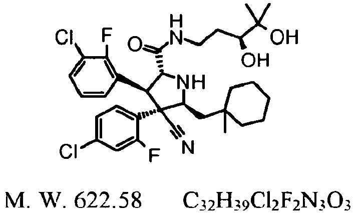

Rac-(2R,3S,4R,5S)- 3-(3-chloro-2-fluoco-phenyl)-4-(4-chloro-2-fluoro-phenyl)-4-cyano-5-(1-methyl-cyclohexylmethyl)-pyrrolidine-2-carboxylic acid ((S)-3,4-dihydroxy-4-methyl-pentyl)-amide (0.2 g) was separated by chiral SFC chromatography to provide chiral (2R,3S,4R,5S)- 3-(3-chloro-2-fluoro-phenyl)-4-(4-chloro-2-fluoro-phenyl)-4-cyano-5-(1-methylcyclohexylmethyl)-pyrrolidine-2-carboxylic acid ((S)-3,4-dihydroxy-4-methyl-pentyl)-amide as a white solid (61 mg, 32%) and chiral (2S,3R,4S,5R)-3-(3-chloro-2-fluoro-phenyl)-4-(4-chloro-2-fluoro-phenyl)-4-cyano-5-(1-methyl-cyclohexylmethyl)-pyrrolidine-2-carboxylic acid ((S)-3,4-dihydroxy-4-methyl-pentyl)-amide as a white solid (78 mg, 39%).

HRMS (ES⁺) m/z Calcd for C₃₂H₃₉Cl₂F₂N₃O₃+ H [(M+H)⁺]: 622.2410, found: 622.2412.

### Example 134

### Preparation of rac-(2S,3R,4S,5R)-4-(3-cloro-2-fluoro-phenyl)-3-(4-chloro-2-fluoro-phenyl)-2-(2,2-dimethyl-propyl)-5-(3-hydroxy-azetid ine-1-carbonyl)-pyrro lidine-3-carbonitrile

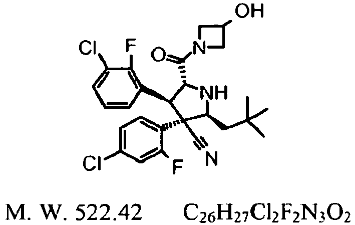

In a manner similar to the method described in **Examples 1e**, rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid trifluoroacetic acid prepared in **Example 52c** (0.25 g, 0.43 mmol) was reacted with azetidin-3-ol hydrochloride (Matrix) (0.25 g, 2.7 mmol), HATU (0.4 g, 1 mmol) and iPr₂NEt (0.6 g, 4.6 mmol) in CH₂Cl₂ at room temperature for 20 h to give rac-(2S,3R,4S,5R)-4-(3-cloro-2-fluoro-phenyl)-3-(4-chloro-2-fluoro-phenyl)-2-(2,2-dimethyl-propyl)-5-(3-hydroxy-azetidine-1-carbonyl)-pyrrolidine-3-carbonitrile as a white solid (0.2 g, 89%).

HRMS (ES⁺) m/z Calcd for C₂₆H₂₇Cl₂F₂N₃O₂+ H [(M+H)⁺]: 522. 1521, found: 522.1520.

### Example 135a

### Preparation of rac-(2R,3S,4R,5S)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid [1-(2-hydroxy-2-methyl-propyl)-1H-pyrazol-3-yl]-amide

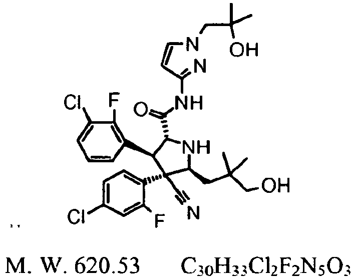

In a manner similar to the method described in **Examples 1e**, rac-(2R,3S,4R,5S)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid trifluoroacetic acid prepared in **Example 105c** (0.55 g, 0.92 mmol) was reacted with 1-(3-amino-pyrazol-1-yl)-2-methyl-propan-2-ol (0.17 g, 1.1 mmol), EDCI (0.26 g, 1.38 mmol), HOBT(0.19 g, 1.4 mmol) and NEt₃ (0.26 mL, 1.8 mmol) in CH₂Cl₂ at room temperature for 20 h to give rac-(2R,3S,4R,5S)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid [1-(2-hydroxy-2-methyl-propyl)-1H-pyrazol-3-yl]-amide as a white solid (0.11 g, 20%).

HRMS (ES⁺) m/z Calcd for C₃₀H₃₃Cl₂F₂N₃O₃+ H [(M+H)⁺]: 620.2002, found: 620.1997.

### Example 135b

### Preparation of (2R,3S,4R,5S)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid [1-(2-hydroxy-2-methyl-propyl)-1 H-pyrazo 1-3-yl]-amide

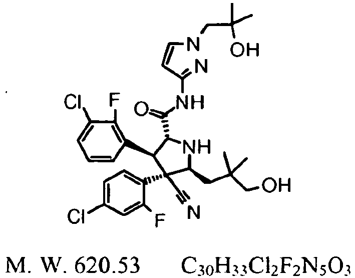

Rac-(2R,3S,4R,5S)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid [1-(2-hydroxy-2-methyl-propyl)-1H-pyrazol-3-yl]-amide (0.11 g) was separated by chiral SFC chromatography to provide chiral (2R,3S,4R,5S)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid (([1-(2-hydroxy-2-methyl-propyl)-1H-pyrazol-3-yl]-amide as a white solid (40 mg, 36%) and chiral (2S,3R,4S,5R)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid [1-(2-hydroxy-2-methyl-propyl)-1H-pyrazol-3-yl]-amide as a white solid (39 mg, 35%).

HRMS (ES⁺) m/z Calcd for C₃₀H₃₃Cl₂F₂N₅O₃+ H [(M+H)⁺]: 620.2002, found: 620.1999.

### Example 136a

### Preparation of rac-(2R,3S,4R,5S)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid [1-(2-hydroxy-2-methylpropyl)-1H-pyrazol-3-yl]-amide

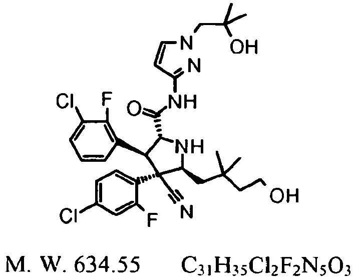

In a manner similar to the method described in **Examples 1e**, rac-(2R,3S,4R,5S)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid
prepared in **Example 116c** (0.48 g, 0.78 mmol) was reacted with 1-(3-amino-pyrazol-1-yl)-2-methyl-propan-2-ol (0.15 g, 0.9 mmol), HATU (0.6 g, 1.6 mmol) and iPr₂NEt (0.41 mL, 2.4 mmol) in CH₂Cl₂ at room temperature for 20 h to give rac-(2R,3S,4R,5S)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid [1-(2-hydroxy-2-methyl-propyl)-1H-pyrazol-3-yl]-amide as a white solid (0.27 g, 55%).

HRMS (ES⁺) m/z Calcd for C₃₁H₃₅Cl₂F₂N₅O₃+ H [(M+H)⁺]: 634.2158, found: 634.2153.

### Example 136b

### Preparation of (2R,3S,4R,5S)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid [1-(2-hydroxy-2-methylpropyl)-1H-pyrazol-3-yl]-amide

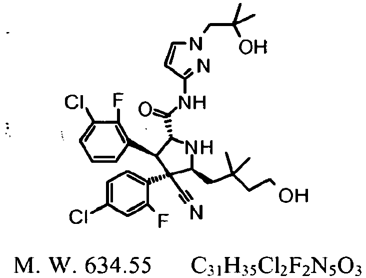

Rac-(2R,3S,4R,5S)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid [1-(2-hydroxy-2-methyl-propyl)-1H-pyrazol-3-yl]-amide (0.25 g) was separated by chiral SFC chromatography to provide chiral (2R,3S,4R,5S)-3-(3-chloro-2-fluoro-phenyl)-4-(4-chloro-2-fluoro-phenyl)-4-cyano-j-(4-hydroxy-2,2-dimethyl-butyl)-pyrrolidine-2-carboxylic acid [1-(2-hydroxy-2-methyl-propyl)-1H-pyrazol-3-yl]-amide as a white solid (105 mg, 42%) and chiral (2S,3R,4S,5R)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid [1-(2-hydroxy-2-methyl-propyl)-1H-pyrazol-3-yl]-amide as a white solid (105 mg, 42%).

HRMS (ES⁺) m/z Calcd for C₃₁H₃₅Cl₂F₂N₅O₃+ H [(M+H)⁺]: 634.2158, found: 634.2157.

### Example 137

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid amide

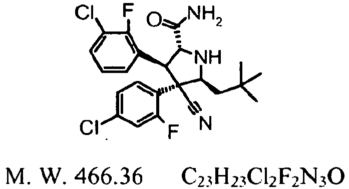

In a manner similar to the method described in **Examples 1e**, rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid trifluoroacetic acid prepared in **Example 52c** (0.5 g, 0.86 mmol) was reacted with a dioxane solution (0.5 M) of ammonia (2 mL, 1 mmol), HATU (0.38 g, 1 mmol) and iPr₂NEt (0.6 g, 4.6 mmol) in CH₂Cl₂ at room temperature for 20 h to give rac-(2R,3S,4R,5S)-3-(3-chloro-2-fluoro-phenyl)-4-(4-chtoro-2-fluoro-phenyl)-4-cyano-5-(2,2-dimethyl-propyl)-pyrrolidine-2-carboxylic acid amide as a white solid (0.3 g, 75%).

HRMS (ES⁺) m/z Calcd for C₂₃H₂₃Cl₂F₂N₃O+ H [(M+H)⁺]: 466.1259, found: 466.1259.

### Example 138

### Preparation of rac-6-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-nicotinic acid methyl ester

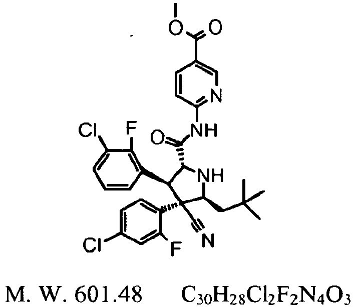

In a manner similar to the method described in **Examples 1e**, rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid trifluoroacetic acid prepared in **Example 52c** (0.5 g, 0.86 mmol) was reacted with 6-amino-nicotinic acid methyl ester (Aldrich) (0.3 g, 2 mmol), HATU (0.38 g, 1 mmol) and iPr₂NEt (0.6 g, 4.6 mmol) in CH₂Cl₂ at room temperature for 20 h to give rac-6-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-nicotinic acid methyl ester as a white solid (0.3 g, 58%).

HRMS (ES⁺) m/z Calcd for C₃₀H₂₈Cl₂F₂N₄O₃ + H [(M+H)⁺]: 601.1580, found: 601.1578.

### Example 139

### Preparation of rac-6-{[(2R,3S,4R,5S)-3-(3-chloro-2 -fluoro-phenyl)-4-(4-chloro-2-fluoro-phenyl)-4-cyano-5-(2,2-dimethyl-propyl)-pyrrolidine-2-carbonyl]-amino}-N-(2-hydroxy-1,1-dimethyl-ethyl)-nicotinamide

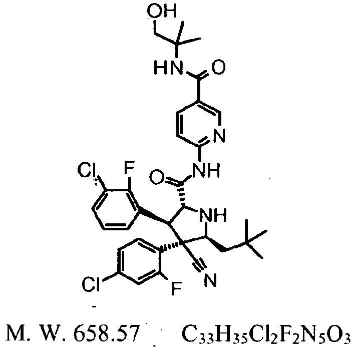

To a solution ofrac-6-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino} -nicotinic acid methyl ester prepared in **Example 138** (0.2 g, 0.33 mmol) in tetrahydrofuran (3 mL) was added an aqueous solution (1 N) of NaOH (1 mL, 1 mmol) and methanol (1 mL). The reaction mixture was stirred at room temperature fro 20 h, and the "pH" of the solution was adjusted to 5-6 by aqueous HCl solution. The mixture was extracted ethyl acetate twice. The combined organic extracts were washed with water, brine, dried over MgSO₄, and concentrated to give rac-6-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-nicotinic acid as a white foam (0.12 g).

In a manner similar to the method described in **Examples 1e**, rac-6-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-nicotinic acid (0.12 g, 0.2 mmol) was reacted with 2-amino-2-methyl-1-propanol (Aldrich) (0.1 g, 1.1 mmol), HATU (0.2 g, 0.5 mmol) and iPr₂NEt (0.3 g, 2.3 mmol) in CH₂Cl₂ at room temperature for 20 h to give rac-6-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-N-(2-hydroxy-1,1-dimethyl-ethyl)-nicotinamide as a white solid (30 mg, 22%). HRMS (ES⁺) m/z Calcd for C₃₃H₃₅Cl₂F₂N₅O₃ + H [(M+H)⁺]: 658.2158, found: 658.2155,

### Example 140

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (6-acetylamino-pyridin-3-yl)-amide

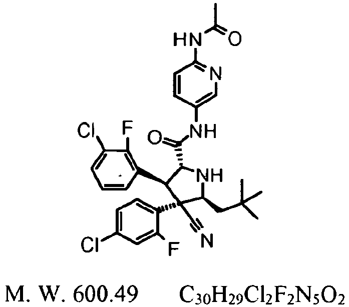

In a manner similar to the method described in **Examples 1e**, rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid trifluoroacetic acid prepared in **Example 52c** (0.16 g, 0.28 mmol) was reacted with N-(5-amino-pyridin-2-yl)-acetamide (Aldrich) (0.12 g, 0.79 mmol), HATU (0.1 g. 0.26 mmol) and iPr₂NEt (0.2 g, 1.5 mmol) in CH₂Cl₂ at room temperature for 20 h to give rac-(2R,3S,4R,SS)-3-(3-chloro-2-fluoro-phenyl)-4-(4-chloro-2-fluoro-phenyl)-4-cyano-5-(2,2-dimethyl-propyl)-pyrrolidine-2-carboxylic acid (6-acetylamino-pyridin-3-yl)-amide as a white solid (0.15 g, 89%).

HRMS (ES⁺) m/z Calcd for C₃₀H₂₉Cl₂F₂N₅O₂ +H [(M+H)⁺]: 600.1739, found: 600.1739.

### Example 141a

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-methyl-2-oxo-1,2-dihydropyridin-4-yl)-amide

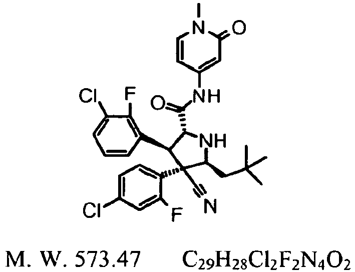

In a manner similar to the method described in **Examples 1e**, rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid trifluoroacetic acid prepared in **Example 52c** (0.2 g, 0.43 mmol) was reacted with 4-amino-1-methyl-1H-pyridin-2-one (Molbridge)(0.11 g, 0.86 mmol), HATU (0.29 g, 0.77 mmol) and iPr₂NEt (0.15 mL, 0.86 mmol) in CH₂Cl₂ at room temperature for 20 h to give rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-methyl-2-oxo-1,2-dihydro-pyridin-4-yl)-amide as a white solid (35 mg, 14%).

HRMS (ES⁺) m/z Calcd for C₂₉H₂₈Cl₂F₂N₄O₂+H [(M+H)⁺] : 573. 1630, found: 573.1633.

### Example 141b

### Preparation of (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-methyl-2-oxo-1,2-dihydropyridin-4-yl)-amide

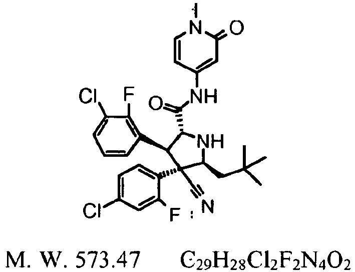

Rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-methyl-2-oxo-1,2-dihydro-pyridin-4-yl)-amide (68 mg) was separated by chiral SFC chromatography to provide chiral-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-methyl-2-oxo-1,2-dihydro-pyridin-4-yl)-amide as a white solid (16 mg, 24%) and chiral-(2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-methyl-2-oxo-1,2-dihydro-pyridin-4-yl)-amide as a white solid (16 mg, 24%).

HRMS (ES⁺)m/zCalcd for C₂₉H₂₈Cl₂F₂N₄O₂+ H [(M+H)⁺]: 573.1636, found: 573.1626.

### Example 142

### Preparation of rac-(2R,35,4m5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [5-((S)-1,2-dihydroxy-ethyl)-pyrazin-2-yl]-amide

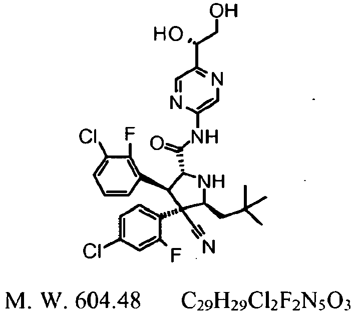

In a manner similar to the method described in **Examples 42c and 42d,** rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid trifluoroacetic acid prepared in **Example 52c** (0.1 g, 0.2 mmol) was reacted with 5-((S)-2,2-dimethyl-[1,3]dioxolan-4-yl)-pyrazin-2-ylamine (46 mg, 0.24 mmol), T3P (Aldrich) (0.32 mL, 0.53 mmol) and iPr₂NEt (0.11 mL, 0.64 mmol) in CH₂Cl₂ at room temperature for 20 h, then reacted with aqueous HCl solution in tetrahydrofuran at room temperature to give rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [5-((S)-1,2-dihydroxyethyl)-pyrazin-2-yl]-amide as a white solid (14 mg, 11%).

HRMS (ES⁺) m/z Calcd for C₂₉H₂₉Cl₂F₂N₅O₃+ H [(M+H)⁺]:604.1689, found: 604.1687.

### Example 143

### Preparation of rac-(2R,3S,4R,SS)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid (1-methyl-2-oxo-1,2-dihydro-pyridin-4-yl)-amide

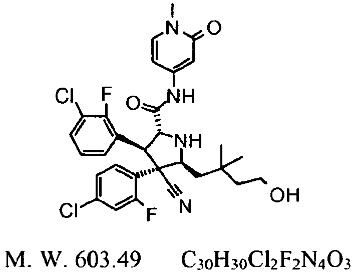

In a manner similar to the method described in **Examples 1e,** rac-(2R,3S,4R,5S)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid trifluoroacetic acid prepared in **Example 116c** (0.5 g, 0.8 mmol) was reacted with 4-amino-1-methyl-1H-pyridin-2-one (Molbridge)(0.18 g, 1.5 mmol), HATU (0.62 g, 1.6 mmol) and iPr₂NEt (0.71 mL, 4.1 mmol) in CH₂Cl₂ at room temperature for 20 h to give rac-(2R,3S,4R,5S)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid (1-methyl-2-oxo-1,2-dihydropyridin-4-yl)-amide as a white solid (25 mg, 5.1%).

HRMS (ES⁺) m/z Calcd for C₃₀H₃₀Cl₂F₂N₄O₃+ H [(M+H)⁺]: 6033.1736, found: 603.1739.

### Example 144

### Preparation of rac-5-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-furan-2-carboxylic acid methyl ester

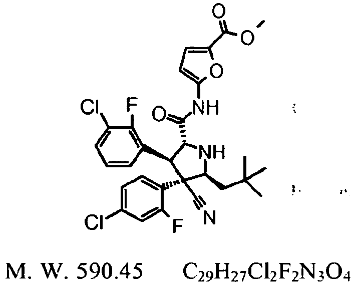

To a solution of rac-(2R,3S,4R,5S)-3-(3-chloro-2-fluoro-phenyl)-4-(4-chloro-2-fluoro-phenyl)4-cyano-5-(2,2-dimethyl-propyl)-pyrrolidine-2-carboxylic acid (0.5 g, 1.07 mmol) in dichloromethane (5 mL) at 0 °C was added oxalyl chloride (0.11 mL, 1.28 mmol) and DMF (0.03 mL). Thereaction mixture was stirred at 0 °C for 3 h, then concentrated. The residue was dissolved into dichloromethane (5 mL), triethylamine (0.45 mL, 3.2 mmol) and DMAP (20 mg, 0.14 mmol) were added, followed by the addtion of methyl 5-amino-2-furoate (Lancaster) (0.38 g, 2.7 mmol). The reaction mixture was stirred at room temperature for 4 h. Water was added, and the mixture was extracted with dichloromethane twice. The organic layers were combined, washed with water, brine, dried over MgSO₄, and concentrated. The residue was purified by chromatography (EtOAc:hexanes=1:3) to give rac-5-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-furan-2-carboxylic acid methyl ester as a off white solid (0.1 g, 16%).

HRMS (ES⁺) m/z Calcd for C₂₉H₂₇Cl₂F₂N₃O₄+ H [(M+H)⁺]; 390.1420, found: 590.1418.

### Example 145

### Preparation of rac-5-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-furan-2-carboxylic acid

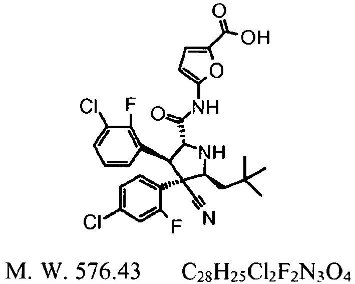

To a solution of rac-5-1[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-furan-2-carboxylic acid methyl ester prepared in **Example 144** (80 mg, 0.14 mmol) in tetrahydrofuran (2 mL) was added an aqueous solution (2 mL) ofLiOH (32 mg, 1.35 mmol). The reaction mixture was stirred at room temperature for 66 h, and the "pH" of the solution was adjusted to 5-6 by aqueous HCl solution. The mixture was extracted ethyl acetate twice. The combined organic extracts were washed with water, brine, dried over MgSO₄, and concentrated to give rac-5-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-furan-2-carboxylic acid as a yellow solid (60 mg, 74%).

HRMS (ES⁺) m/z Calcd for C₂₈H₂₅Cl₂F₂N₃O₄+ H [(M+H)⁺]: 576.1263, found: 576.1264.

### Example 146

### Preparation of rac-5-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-furan-2-carboxylic acid amide

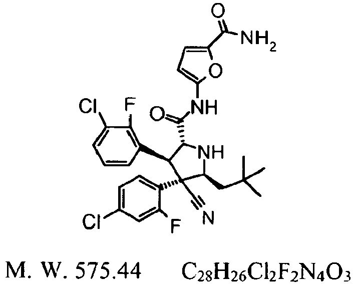

To a solution of rac-5-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-furan-2-carboxylic acid prepared in **Example 145** (60 mg, 0.1 mmol) in N,N-dimethylformamide (2 mL) was added NH₄Cl (28 mg, 0.5 mmol), EDCI (40 mg, 0.2 mmol), HOBT(28 mg, 0.2 mmol) and NEt₃ (0.029 mL, 0.2 mmol). The reaction mixture was heated at 75 °C for 20 h. The mixture was cooled to room temperature, and partitioned between ethyl acetate and water. The organic layer was separated, and the aqueous layer was extracted with ethyl acetate. The organic layers were combined, washed with water, brine, dried over MgSO₄, and concentrated. The residue was purified by chromatography (EtOAc) to give rac-5-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino }-furan-2-carboxylic acid amide as a white solid (30 mg, 50%).

HRMS (ES⁺) m/z Calcd for C₂₈H₂₆Cl₂F₂N₄O₃+ H [(M+H)⁺]: 575.1423, found: 575. 1425

### Example 147

### Preparation of rac-(2R,35,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (6-chloro-pyridazin-3-yl)-amide

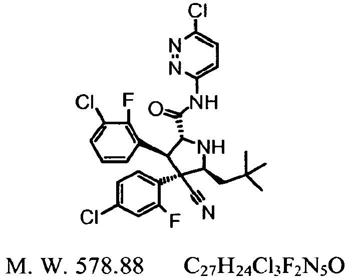

In a manner similar to the method described in **Examples 1e**, rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid trifluoroacetic acid prepared in **Example 52c** (0.3 g, 0.51 mmol) was reacted with 3-amino-6-chloro-pyridazine (Alfa) (0.15 g, 1.2 mmol), HATU (0.2 g, 0.5 mmol) and iPr₂NEt (0.6 g, 4.6 mmol) in CH₂Cl₂ at room temperature for 48 h to give rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (6-chloro-pyridazin-3-yl)-amide as a yellow solid (0.15 g, 51%).

### Example 148

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-methyl-pyridin-3-yl)-amide

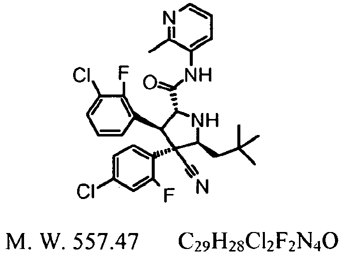

In a manner similar to the method described in **Examples 1e**, rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid trifluoroacetic acid prepared in **Example 52c** (0.3 g, 0.52 mmol) was reacted with 3-amino-2-methyl-pyridine (Aldrich) (0.11 g, 1.1 mmol), HATU (0.36 g, 0.94 mmol) and iPr₂NEt (0.27 g, 1.6 mmol) in CH₂Cl₂ at room temperature for 20 h to give rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-methyl-pyridin-3-yl)-amide as a yellow solid (0.16 g, 55%). HRMS (ES⁺) m/z Calcd for C₂₉H₂₈Cl₂F₂N₄O+ H [(M+H)⁺] 557.1681; found: 557.1677.

### Example 149a

### Preparation of intermediate 2-(4-amino-phenoxy)-ethanol

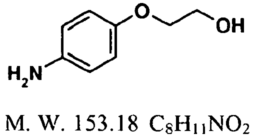

A suspension of 2-(4-nitrophenoxy)ethanol (Aldrich) (2 g, 10.9 mmol) and Pd/C (Aldrich, 10%, 0.2 g) in methanol (50 mL) was vigorously shaken in a Parr under atmosphere of H₂ (50 psi) for 1 h. The mixture was filtered through a short pad of celite. The filtrate was concentrated to give 2-(4-amino-phenoxy)-ethanol as a light yellow solid (1.6 g, 96%).

### Example 149b

### Preparation of rac-(2R,35,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(2-hydroxy-ethoxy)-phenyl]-amide

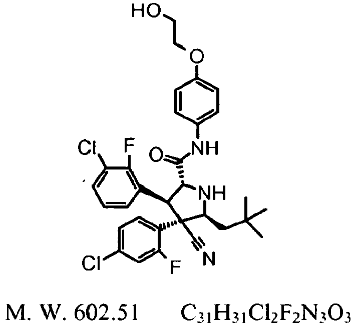

In a manner similar to the method described in **Examples 1e**, rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (0.4 g, 0.86 mmol) was reacted with 2-(4-amino-phenoxy)-ethanol (0.24 g, 1.5 mmol), HATU (0.58 g, 1.5 mmol) and iPr₂NEt (0.3 mL, 1.7 mmol) in CH₂Cl₂ (20 mL) at room temperature for 20 h to give rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(2-hydroxyethoxy)-phenyl]-amide as a white solid (0.4 g, 78%).

HRMS (ES⁺) m/z Calcd for C₃₁H₃₁Cl₂F₂N₃O₃+ H [(M+H)⁺]: 602.1784, found: 602.1783.

### Example 149c

### Preparation of (2R,35,4R,55)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(2-hydroxy-ethoxy)-phenyl]-amide

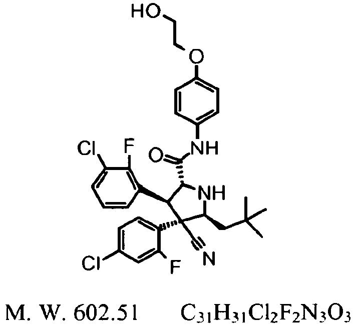

Rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(2-hydroxy-ethoxy)-phenyl]-amide (0.25 g) was separated by chiral SFC chromatography to provide chiral-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(2-hydroxy-ethoxy)-phenyl]-amide as a white solid (94 mg, 37%) and chiral-(2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(2-hydroxy-ethoxy)-phenyl]-amide as a white solid (100 mg, 40%).

HRMS (ES⁺) m/z Calcd for C₃₁H₃₁Cl₂F₂N₃O₃+ H [(M+H)⁺] 602.1784, found: 602.1784.

### Example 150

### Preparation of rac-5-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-thiophene-2-carboxylic acid methyl ester

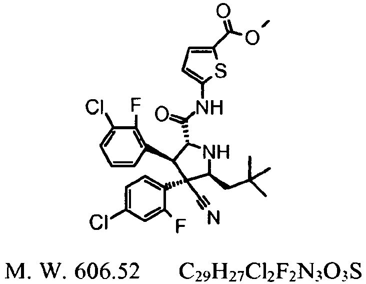

In a manner similar to the method described in **Examples 144**, rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (0.3 g, 0.64 mmol) was reacted with oxalyl chloride (0.12 mL, 1.4 mmol), triethylamine (0.22 mL, 1.6 mmol), DMAP (5 mg), and 5-amino-2-thiophene-carboxylate (Princeton) (0.14 g, 0.96 mmol) to give rac-5-{[(2R,3S,4R,5S)-3-(3-chloro-2-fluoro-phenyl)-4-(4-chloro-2-fluoro-phenyl)-4-cyano-5-(2,2-dimethyl-propyl)-pyrro lidine-2-carbonyl]-amino} - thiophene-2-carboxylic acid methyl ester as a yellow solid (0.11 g, 28%).

HRMS /(ES⁺) m/z Calcd for C₂₉H₂₇Cl₂F₂N₃O₃S₊ H [(M+H)⁺]: 606.1191, found: 606.1191.

### Example 151a

### Preparation of rac-5-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino -thiophene- 2-carboxylic acid

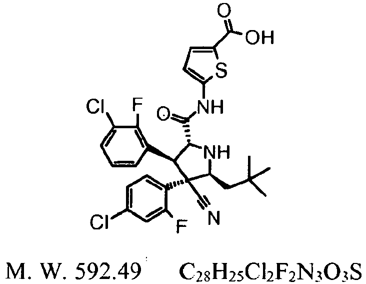

To a solution ofrac-5-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-thiophene-2-carboxylic acid methyl ester prepared in **Example 150** (90 mg, 0.15 mmol) in tetrahydrofuran (3 mL) was added an aqueous solution (3 mL) ofLiOH (36 mg, 1.5 mmol). The reaction mixture was stirred at room temperature for 66 h, and the "pH" of the solution was adjusted to 5 by aqueous HCl solution. The mixture was extracted ethyl acetate twice. The combined organic extracts were washed with water, brine, dried over MgSO₄, and concentrated to give rac-5-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-thiophene-2-carboxylic acid as a yellow solid (60 mg, 74%).

HRMS (ES⁺) m/z Calcd for C₂₈H₂₅Cl₂F₂N₃O₃S+ H [(M+H)⁺]: 592.1035, found: 592.1035.

### Example 151b

### Preparation of 5-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-thiophene-2-carboxylic acid

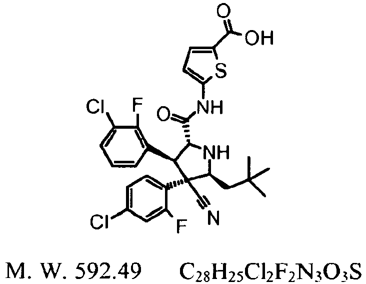

Rac-5- {[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-thiophene-2-carboxylic acid (50 mg) was separated by chiral SFC chromatography to provide chiral 5-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-thiophene-2-carboxylic acid as a white solid (12 mg, 24%) and chiral 5-{[(2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-thiophene-2-carboxylic acid as a white solid (12 mg, 24%).

HRMS (ES⁺) m/z Calcd for C₂₈H₂₅Cl₂F₂N₃O₃S+ H [(M+H)⁺]: 592.1035, found: 592.1035.

### Example 152

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-methoxy-pyridin-4-yl)-amide

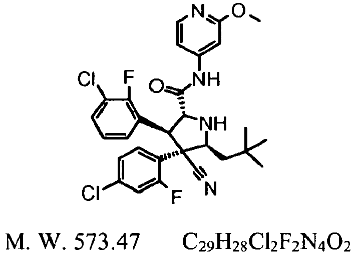

In a manner similar to the method described in **Examples 1e**, rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (0.4 g, 0.75 mmol) was reacted width 2-methoxy-pyridin-4-ylamine (Oakwood) (0.1g, 0.9 mmol), HATU (0.51 g, 1.35 mmol) and iPr₂NEt (0.33 mL, 1.9 mmol) in CH₂Cl₂ at room temperature to give rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-methoxy-pyridin-4-yl)-amide as a white solid (0.2 g, 47%).

HRMS (ES⁺) m/z Calcd for C₂₉H₂₈Cl₂F₂N₄O₂+ H [(M+H)⁺]: 573.1630, found: 573.1633.

### Example 153

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-hydroxy-pyridin-4-yl)-amide

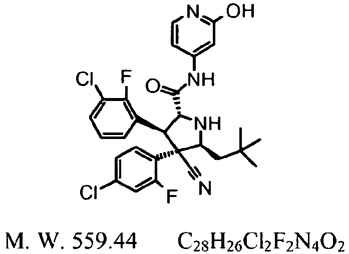

In a manner similar to the method described in **Examples 1e**, rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (0.49 g, 1. mmol) was reacted with 4-amino-pyridin-2-ol (Molbridge) (0.14 g, 1.3 mmol), HATU (0.72 g, 1.9 mmol) and iPr₂NEt (0.46 mL, 2.6 mmol) in CH₂Cl₂ at room temperature to give rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-hydroxy-pyridin-4-yl)-amide as a off white solid (20 mg, 3.4%).

HRMS (ES⁺) m/z Calcd for C₂₈H₂₆Cl₂F₂N₄O₂+ H [(M+H)⁺]: 559.1474, found: 559.1477.

### Example 154

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-acetyl-phenyl)-amide

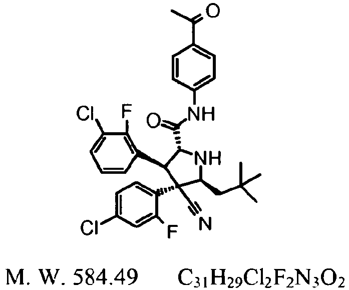

In a manner similar to the method described in **Examples 1e**, rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (0.35 g, 0.75 mmol) was reacted with 1-(4-amino-phenyl)-ethanone (Aldrich) (0.12 g, 0.9 mmol), HATU (0.5 g, 1.3 mmol) and iPr₂NEt (0.33 mL, 2.6 mmol) in CH₂Cl₂ at room temperature to give rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-acetyl-phenyl)-amide as a white solid (0.12 g, 27%).

HRMS (ES⁺) m/z Calcd for C₃₁H₂₉Cl₂F₂N₃O₂+ H [(M+H)⁺]: 584.1678, found: 584.1680.

### Example 155

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(2-bromo-acetyl)-phenyl]-amide

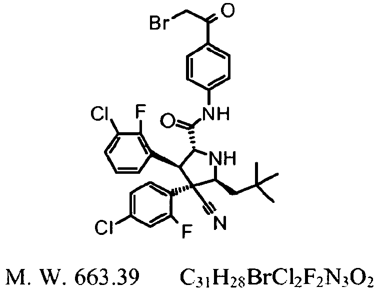

In a manner similar to the method described in **Examples 1e**, rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (0.35 g, 0.75 mmol) was reacted with 1-(4-amino-phenyl)-2-bromo-ethanone (Astatech) (0.17 g, 0.9 mmol), HATU (0.5 g, 1.3 mmol) and iPr₂NEt (0.33 mL, 2.6 mmol) in CH₂Cl₂ at room temperature to give rac-(2R,3S,45,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(2-bromo-acetyl)-phenyl]-amide as a white solid (36 mg, 7%).

HRMS (ES⁺)m/z Calcd for C₃₁H₂₈BrCl₂F₂N₃O₂+ H [(M+H)⁺]: 662.0783, found: 662.078..

### Example 156

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(2- dimethylamino-acetyl)-phenyl]-amide

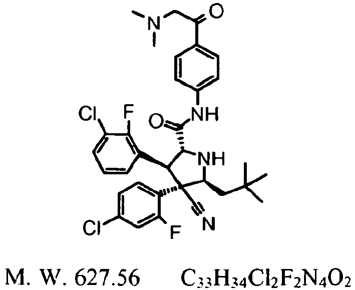

To the solution of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(2-bromo-acetyl)-phenyl]-amide (30 mg, 0.045 mmol) in tetrahydrofuran (1 mL) was added a tetrahydrofuran solution (2 M) of dimethylamine (0.057 mL, 0.11 mmol). The reaction mixture was stirred at room temperature for 30 min. The mixture was concentrated, and the residue was partitioned between ethyl acetate and water. The organic layer was separated, washed with water, brine, dried over MgSO₄, and concentrated to give rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(2-dimethylamino-acetyl)-phenyl]-amide as a yellow solid (10 mg, 35%)

HRMS (ES⁺) m/z Calcd for C₃₃H₃₄Cl₂F₂N₄O₂+ H [(M+H)⁺]: 627.2100, found: 627.2102.

### Example 157

### Preparation of 4-chloro-3-fluoro-phenyl)-acetonitrile

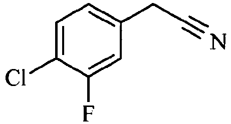

To a solution of4-bromo-1-chloro-2-fluoro-benzene in diisopropl ether (50 mL) at -78°C was added t-butyllithium maintaining the temperature below -70°C. A white precipitate formed. After 30 minutes, zinc chloride was added, maintaining the temperature below -50°C. The resulting mixture was added to a solution of bromoacetonitrile (0.8 mL, 12.1 mmol), nickel(II)acetylacetonate (0.1485 g, 0.578 mmol), and tri-o-tolylphosphine (0.1787 g, 0.578 mmol) in THF (100 mL) and the reaction heated under reflux for 2 hours with pentane removed by distillation. The reaction was concentrated under reduced pressure and partitioned between ethyl acetate and 2N aqueous sodium hydroxide solution. The organic layer was retained and the aqueous layer re-extracted with ethyl acetate. The organic portions were combined, washed with brine, dried over MgSO₄, filtered and concentrated under reduced pressure to give a brown oil. Purification by column chromatography, 40 g silica column, (1 to 100% EtOAc/heptane) to yield 4-chloro-3-fluoro-phenyl)-acetonitrile, 0.47 g, 33.6 %.

### Example 158

### Preparation of (Z)-3-(3-chloro-2-fluoro-phenyl)-2-(4-chloro-3-fluoro-phenyl)-acrylonitrile

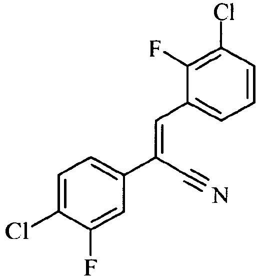

A mixture of4-chloro-3-fluorobenzylcyanide (1.0 g, 5.92 mmol), 3-chloro-2-fluorobenzaldehyde (0.938 g, 5.92 mmol), 2 N NaOH (4 mL) and isopropyl alcohol were stirred at rt. The mixture was stirred for 10 min. to afford a solid ppt that was collected by filtration with multiple water washes. The solid was dried ON under reduced pressure to afford a white solid (Z)-3-(3-chloro-2-fluoro-phenyl)-2-(4-chloro-3-fluoro-phenyl)-acrylonitrile, 1.62 g, 89.3%; HRMS (ES⁺) m/z Calcd for C₁₅H₇Cl₂F₂N+ H [(M+H)⁺]: 308.9924, found: 308.9926.

### Example 159

### Preparation of rac-(2S,3S,4R,5S)-3-(3-chloro-2-fluoro-phenyl)-4-(4-chloro-3-fluoro-phenyl)-4-cyano-5-(2,2-dimethyl-propyl)-pyrrolidine-2-carboxylic acid tert-butyl ester and rac-(2R,3S,4R,5S)-3-(3-chloro-2-fluoro-phenyl)-4-(4-chloro-3-fluoro-phenyl)-4-cyano-5-(2,2-dimethyl-propyl)-pyrrolidine-2-carboxylic acid tert-butyl ester

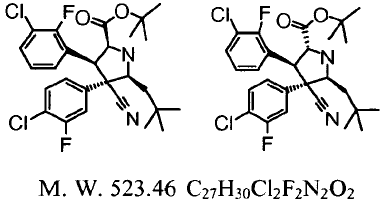

To a solution of (Z)-3-(3-chloro-2-fluoro-phenyl)-2-(4-chloro-3-fluoro-phenyl)-acrylonitrile and [3,3-dimethyl-but-(Z)-ylideneamino]-acetic acid tert-butyl ester in dichloroethane (20 mL) was added TEA (1.46 mL, 10.44 mmol), AgF (0.661 g, 5.22 mmol) and stirred at RT overnight. The mixture was then quenched with NH₄Cl (satd) solution and extracted with CH₂Cl₂. The organic phase was separated, filtered through celite and dried over Na₂SO₄. the solvent was removed by reduced pressure to yield crude oil that was purified with silica column chromatography (1-20 % EtOAc/heptane) to yield two products; rac-(2*S*,3*S*,4*R*,5S)-3-(3-chloro-2-fluoro-phenyl)-4-(4-chloro-3-fluoro-phenyl)-4-cyano-5-(2,2-dimethyl-propyl)-pyrrolidine-2-carboxylic acid tert-butyl ester (0.410 g, 15 %) as a yellow solid. HRMS (ES⁺) m/z Calcd for C₂₇H₃₀Cl₂F₂N₂O₂+ H [(M+H)⁺] 523.1725, found: 523.1725 and rac-(2*R*,3*S*,4*R*,5*S*)-3-(3-chloro-2-fluoro-phenyl)-4-(4-chloro-3-fluoro-phenyl)-4-cyano-5-(2,2-dimethyl-propyl)-pyrrolidine-2-carboxylic acid tert-butyl ester (0.92 g, 33.7 %) as an yellow solid. HRMS (ES⁺) m/z Calcd for C₂₇H₃₀Cl₂F₂N₂O₂ + H [(M+H)⁺]: 523.1725. found: 523.1722.

### Example 160

### Preparation of rac-(2R,3S,4R,5S)-3-(3-chloro-2-fluoro-phenyl)-4-(4-chloro-3-fluoro-phenyl)-4-cyano-5-(2,2-dimethyl-propyl)-pyrrolidine-2-carboxylic acid

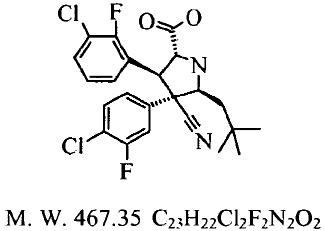

A mixture of rac-(2*R*,3*S*,4*R*,5*S*)-3-(3-chloro-2-fluoro-phenyl)-4-(4-chloro-3-fluoro-phenyl)-4-cyano-5-(2,2-dimethyl-propyl)-pyrrolidine-2-carboxylic acid tert-butyl ester (0.6 g, 1.15 mmol) was cooled to 0°C, then conc. H₂SO₄ (2 mL) was added slowly. The reaction was stirred at RT for 2 hours. The mixture was then poured into ice and extracted with EtOAc. The organic phase was separated, dried over Na₂SO₄, filtered and solvent was removed under reduced pressure to yield a residue that was triturated with ethyl acetate/ heptane and the precipitates were collected by filtration and washed with ether to yield the product rac-(2*R*,3*S*,4*R*,5*S*)-3-(3-chloro-2-fluoro-phenyl)-4-(4-chloro-3-fluoro-phenyl)-4-cyano-5-(2,2-dimethyl-propyl)-pyrrolidine-2-carboxylic acid (0.526 g, 98.1 %) as a white solid. HRMS (ES⁺) m/z Calcd for C₂₃H₂₂Cl₂F₂N₂O₂ + H [(M+H)⁺]: 467.1099, found: 467.1097.

### Example 161

### Preparation of rac-(2R,3S,4R,5S)-3-(3-chloro-2-fluoro-phenyl)-4-(4-chloro-3-fluoro-phenyl)-4-cyano-5-(2,2-dimethyl-propyl)-pyrrolidine-2-carboxylic acid [2-((S)-2,2-dimethyl-[1,3]dioxolan-4-yl)-ethyl]-amide

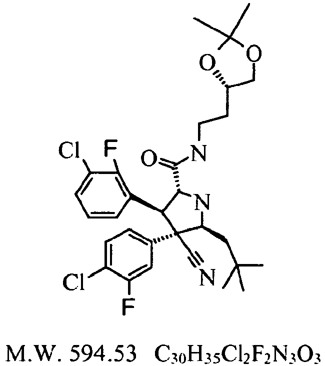

A mixture rac-(2*R*,3*S*,4*R*,5*S*)-3-(3-chloro-2-fluoro-phenyl)-4-(4-chloro-3-fluoro-phenyl)-4-cyano-5-(2,2-dimethyl-propyl)-pyrrolidine-2-carboxylic acid (125 mg, 0.267 mmol), 2-((S)-2,2-dimethyl-[1,3]dioxolan-4-yl)-ethylamine (0.2 mg, 1.3 mmol), 2-(7-azabenzotriazol-1-yl)-n,n,n',n'-tetramethyluronium hexafluorophosphate (HATU, 114.1 mg, 0.3 mmol) and iPr₂NEt (0.3 mL, 3.22 mmol) in CH₂Cl₂ (2 mL) was stirred at rt overnight. The mixture was then diluted with CH₂Cl₂ and washed with water, brine. The organic phase was separated, filtered and dried over Na₂SO₄. The mixture was then concentrated and purified by reverse phase chromatography (20-95% of ACN/water) to give rac-(2*R*,3*S*,4*R*,5*S*)-3-(3-chloro-2-fluoro-phenyl)-4-(4-chloro-3-fluoro-phenyl)-4-cyano-5-(2,2-dimethyl-propyl)-pyrrolidine-2-carboxylic acid [2-((*S*)-2,2-dimethyl-[1,3]dioxolan-4-yl)-ethyl]-amide (101 mg, 63.5 %) as an off-white powder. HRMS (ES⁺) m/z Calcd for C₃₀H₃₅Cl₂N₃O₃ + H [(M+H)⁺]: 594.2097, found: 594.2096,

### Example 162

### Preparation of rac-(2R,3S,4R,5S)-3-(3-chloro-2-fluoro-phenyl)-4-(4-chloro-3-fluoro-phenyl)-4-eyano-5-(2,2-dimethyl-propyl)-pyrrolidine-2-carboxylic acid ((S)-3,4-dihydroxy-butyl)-amide and rac-(2S,3R,4S,5R)-3-(3-chloro-2-fluoro-phenyl)-4-(4-chloro-3-fluoro-phenyl)-4-cyano-5-(2,2-dimethyl-propyl)-pyrrolidine-2-carboxylic acid ((S)-3,4-dihydroxy-butyl)-amide

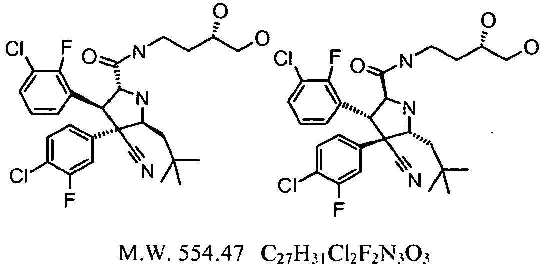

A mixture of rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid [1-((R)-2,2-dimethyl-[1,3]dioxolan-4-ylmethyl)-1H-pyrazol-3-yl]-amide (81 mg, 0.133 mmol), pyridinium p-toluene sulfonic acid (5 mg, 0.0198 mmol) and methanol (4 mL) was microwaved at 120°C for 5 min. Solvent evaporated and purified by reverse phase chromatography (20-95% of ACN/water) to rac-(2*R*,3*S*,4*R*,5*S*)-3-(3-Chloro-2-fluoro-phenyl)-4-(4-chloro-3-fluoro-phenyl)-4-cyano-5-(2,2-dimethyl-propyl)-pyrrolidine-2-carboxylic acid ((*S*)-3,4-dihydroxy-butyl)-amide (20.7 mg, 22 %) as a white powder. HRMS (ES⁺) m/z Calcd for C₂₇H₃₁Cl₂F₂N₃O₃ + H [(M+H)⁺]: 554.1784. found : 554.1780. rac-(2*S*,3*R*,4*S*,5*R*)-3-(3-chloro-2-fluoro-phenyl)-4-(4-chloro-3-fluoro-phenyl)-4-cyano-5-(2,2-dimethyl-propyl)-pyrrolidine-2-carboxylic acid ((S)-3,4-dihydroxy-butyl)-amide (18.8 mg, 20 %) as a white powder. HRMS (ES⁺) m/z Calcd for C₂₇H₃₁Cl₂F₂N₃O₃+ H [(M+H)⁺]: 554.1784, found: 554.1781

### Example 163

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [1-(2-hydroxy-2-methyl-propyl)-1H-pyrazol-3-yl]-amide

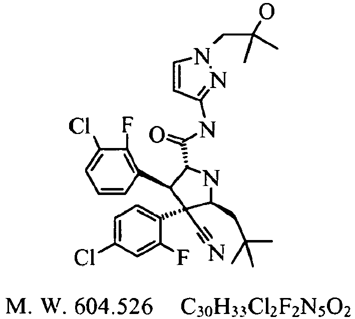

A mixture of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (86.2 mg, 0.20 mmol), 1-(3-amino-pyrazol-1-yl)-2-methyl-propan-2-ol (62 mg, 0.3 mmol), 2-(7-azabenzotriazol-1-yl)-n,n,n',n'-tetramethyluronium hexafluorophosphate (HATU, 114.06 mg, 0.3 mmol) and iPr₂NEt (0.1 mL, 0.6 mmol) in CH₂-Cl₂ (2 mL) was stirred at rt overnight. The mixture was then diluted with CH₂Cl₂ and washed with water, brine. The organic phase was separated, dried over Na₂SO₄ and filtered. The mixture was then concentrated and purified by reverse phase chromatography (20-95% of ACN/water) to give rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [1-(2-hydroxy-2-methyl-propyl)-1H-pyrazol-3-yl]-amide (23.1 mg, 20.8 %) as an off-white powder. HRMS (ES⁺) m/z Calcd for C₃₀H₃₃Cl₂F₂N₅O₂ + H [(M+H)⁺]: 604.2052, found: 604.2052.

### Example 164

### Preparation of chiral 2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [1-(2-hydroxy-2-methyl-propyl)-1H-pyrazol-3-yl]-amide and chiral (2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid [1-(2-hydroxy-2-methyl-propyl)-1H-pyrazol-3-yl]-amide

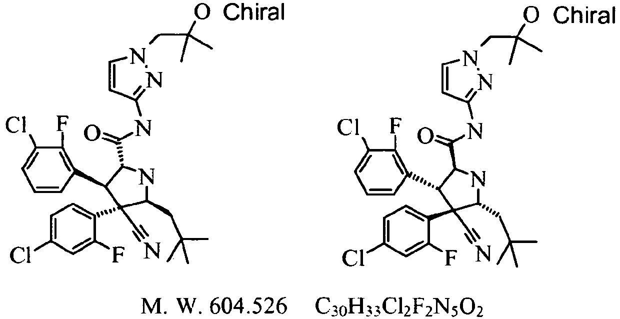

The racemate 2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [1-(2-hydroxy-2-methyl-propyl)-IH-pyrazol-3-yl]-amide (0.25 g) was submitted for SFC purification (30% methanol/water, 100psi) to afford of chiral 2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [1-(2-hydroxy-2-methyl-propyl)-1H-pyrazol-3-yl]-amide (117 mg, 21 %) as an off-white powder. HRMS (ES⁺) m/z Calcd for C₃₀H₃₃Cl₂F₂N₅O₂ + H [(M+H)⁺]: 604.2052; found: 604.205 and chiral (2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid [1-(2-hydroxy-2-methyl-propyl)-1H-pyrazol-3-yl]-amide (110 mg, 19.8 %) as an off-white powder. HRMS (ES⁺) m/z Calcd for C₃₀H₃₃Cl₂F₂N₅O₂ + H[(M+H)⁺]: 604.2052. found: 604.2052.

### Example 165

### Preparation of 1-(3-amino-pyrazol-1-yl)-2-methyl-propan-2-ol

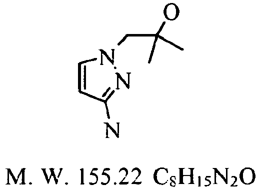

A mixture of 2-methyl-1-(3-nitro-pyrazol-1-yl)-propan-2-ol (0.3 g, 1.6 mmol), Zn dust( 0.42 g, 6.5 mmol), ammonium chloride (0.85 g, 16 mmol) and methanol (2 mL) was microwaved at 120°C for 10 min. The resulting suspension was filtered through celite with methanol and THF washes. Solvent was removed under reduced pressure to yield crude product.that was triterated with ethyl acetate and filtered again to get rid of ammonium chloride salt. Product 1-(3-amine-pyrazol-1-yl)-2-methyl-propan-2-ol was a yellow solid (233 mg, 94%).

### Example 166

### Preparation of 2-methyl-1-(3-nitro-pyrazol-1-yl)-propan-2-ol

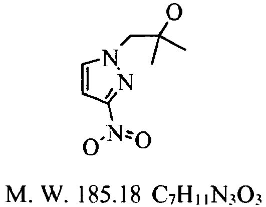

A mixture of3-nitro-1H-pyrazole (10.0 g, 88.43 mmol), 2,2-dimethyl-oxirane ( 15.7 mL, 176.9 mmol), potassium carbonate (18.2 g, 132 mmol) and DMF (100 mL) was stirred at 100°C for 1h, then stirred ON at rt. The mixture was then diluted with ethyl acetate and water, the organic layer was separated, dried over Na₂SO₄, and filtered. The resulting mixture was concentrated under reduced pressure to yield the crude product that was purified (50% EtOAc/heptane) to yield the product 2-methyl-1-(3-nitro-pyrazol-1-yl)-propan-2-ol as a waxy solid (4.88 g, 30%).

### Example 167

### Preparation of 1-[2-methyl-2-((R)-1-oxiranylmethoxy)-propyl]-3-nitro-1H-pyrazole

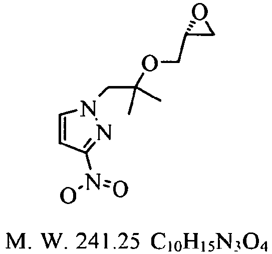

A mixture of 2-methyl-1-(3-nitro-pyrazol-1-yl)-propan-2-ol (1.31 g, 7.07 mmol), and DMF (60 mL) was stirred at 0°C for 5 min, then NaH (60% dispersion in oil, 0.85 g, 21.2 mmol) was added and stirred 20 min at 0°C. R-(-)-glycidyl-3-nitrobenzenesulfonate (2.75 g, 10.6 mmol) was added and stirred at 0°C for 1h then warmed to rt for 14h. The mixture was then diluted with NH₄Cl(s), ethyl acetate, the organic phase was separated, washed with NaHCO₃(satd) dried with Na₂SO₄, and filtered. The mixture was then concentrated and purified by column chromatography (40-240 g Analogix column, 70% EtoAc/heptane to yield the product 1-[2-methyl-2-((R)-1-oxiranylmethoxy)-propyl]-3-nitro-1H-pyrazole as a white solid (0.7 g, 41 %).

### Example 168

### Preparation of 1-[2-methyl-2-((R)-1-oxiranylmethoxy)-propyl]-1H-pyrazol-3yl amine

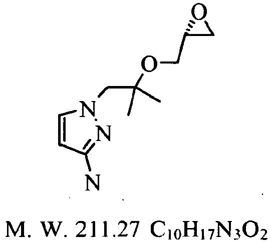

A mixture of 1-[2-methyl-2-((*R*)-]-oxiranylmethoxy)-propyl]-3-nitro-1H-pyrazole (0.3 g, 1.24 mmol), and ethyl acetate (15 mL), ethanol (15 mL) was subjected to the H-Cube (Thales Nano) at 1 mL/min at 10°C, 10 psi hydrogen. The first time through not completely reduced. Resubjected and complete reduction of nitro group by NMR. Solvent was removed under reduced pressure to afford 1-[2-methyl-2-((*R*)-1-oxiranylmethoxy)-propyl]-1H-pyrazol-3ylamine as an oil (0.27 g, 100%).

### Example 169

### Preparation of rac-(2R,3S,4R,5S)-3-(3-chloro-2-fluoro-phenyl)-4-(4-chloro-2-fluoro-phenyl)-4-cyano-5-(2,2-dimethylpropyl)-pyrrolidine-2-carboxylic acid {1-[2-methyl-2-((R)-1-oxiranylmethoxy)-propyl]-1H-pyrazol-3-yl}-amide

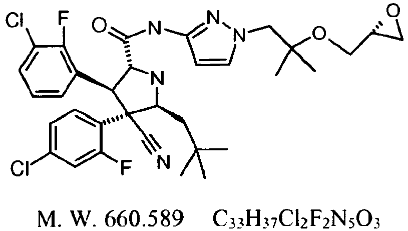

A mixture of rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (0.56 g, 1.2 mmol), 1-[2-methyl-2-((*R*)-1-oxiranylmethoxy)-propyl]-1H-pyrazol-3ylamine (0.27 g, 1.12 mmol), 2-(7-azabenzotriazol-1-yl)-n,n,n',n'-tetramethyluronium hexafluorophosphate (HATU, 0.912 g, 2.4 mmol) and iPr₂NEt (1.5 mL, 8.4 mmol) in CH₂Cl₂ (50 mL) was stirred at rt overnight. The mixture was then diluted with CH₂Cl₂ and washed with water, brine. The organic phase was separated, filtered and dried over Na₂SO₄. The mixture was then concentrated and purified by column chromatography (25-80 g Analgix column, 1-100% ethyl acetate/heptane) to give rac-(2*R*,3*S*,4*R*,5*S*)-3-(3-chloro-2-fluoro-phenyl)-4-(4-chloro-2-fluoro-phenyl)-4-cyano-5-(2,2-dimethylpropyl)-pyrrolidine-2-carboxylic acid {1-[2-methyl-2-((*R*)-1-oxiranylmethoxy)-propyl]-1H-pyrazol-3-yl}-amide (222 mg, 28 %) as an off-white solid. HRMS (ES⁺) m/z Calcd for C₃₃H₃₇Cl₂F₂N₅O₃ + H [(M+H)⁺] 660.2315. found: 660.2312.

### Example 170

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((R)-3-amino-2-hydroxy-propoxy)-2-methyl-propyl]-1H-pyrazo 1-3-yl}-amide

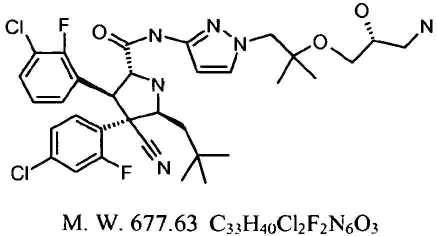

A mixture of rac-(2*R*,3*S*,4*R*,5*S*)-3-(3-chloro-2-fluoro-phenyl)-4-(4-chloro-2-fluoro-phenyl)-4-cyano-5-(2,2-dimethylpropyl)-pyrrolidine-2-carboxylic acid {1-[2-methyl-2-((*R*')-1-oxiranylmethoxy)-propyl]-1H-pyrazol-3-yl}-amide(43.6 mg, 0.066 mmol), isopropyl alcohol (2 mL), and ammonium hydroxide (1 mL) was microwaved at 130°C for 15 min. The mixture was then diluted with CH₂Cl₂ and washed with water, brine. The organic phase was separated, filtered and dried over Na₂SO₄. The mixture was then concentrated and purified by reverse phase chromatography (20-95% of ACN/water) to give rac-(2*R*,3*S*,4*R*,5*S*)-3-(3-chloro-2-fluorophenyl)-4-(4-chloro-2-fluoro-phenyl)-4-cyano-5-(2,2-dimethyl-propyl)-pyrro lidine-2-carboxylic acid {1-[2-((*R*)-3-amino-2-hydroxy-propoxy)-2-methyl-propyl]-1H-pyrazol-3-yl}-amide (11.4 mg, 26 %) as an off-white solid. HRMS (ES) m/z Calcd for C₃₃H₄₀Cl₂F₂N₆O₃ + H [(M+H)⁺]: 677.2580, found: 677.2576.

### Example 171

### Preparation of rac-(2R,3S,4R,5S)-3-(3-chloro-2-fluoro-phenyl)-4-(4-chloro-2-fluoro-phEnyl)-4-cyano-5-(2,2-dimethyl-propyl)-pyrrolidine-2-carboxylic acid (1-{2-[(R)-2-hydroxy-3-(3-hydroxy-propylamino)-propoxy]-2-methyl-propyl}-1H-pyrazol-3-yl)-amide

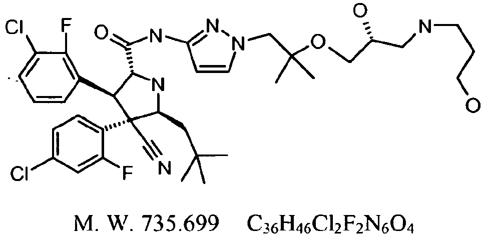

A mixture of rac-(2*R*,3*S*,4*R*,5*S*)-3-(3-chloro-2-fluoro-phenyl)-4-(4-chloro-2-fluoro-phenyl)-4-cyano-5-(2,2-dimethylpropyl)-pyrrolidine-2-carboxylic acid {1-[2-methyl-2-((*R*)-1-oxiranylmethoxy)-propyl]-1H-pyrazol-3-yl}-amide (43.6 mg, 0.066 mmol), isopropyl alcohol ( 2 mL), diisopropylethyl amine (0.1 mL, 0.56 mmol) and 3-amino-1-propanol (0.1 mL, 1.3 mmol) was microwaved at 130°C for 15 min. The mixture was then concentrated and purified by reverse phase chromatography (20-95% of ACN/water) to give rac-(2*R*,3*S*,4*R*,5*S*')-3-(3-chloro-2-fluoro-phenyl)-4-(4-chloro-2-fluoro-phenyl)-4-cyano-5-(2,2-dimethyl-propyl)-pyrrolidine-2-carboxylic acid (1-{2-[(*R*)-2-hydroxy-3-(3-hydroxy-propylamino)-propoxy]-2-methyl-propyl}-1H- pyrazol-3-yl)-amide (10.1 mg, 20.8 %) as an off-white powder. HRMS (ES⁺) m/z Calcd for C₃₆H₄₆Cl₂F₂N₆O₄ + H [(M+H)⁺]: 735.2999, found: 735.2998.

### Example 172

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-{2-[(R)-2-hydroxy-3-(2-hydroxy-1-hydroxymethyl-ethylamino)-propoxy]-2-methyl-propyl}-1H-pyrazol-3-yl)-amide

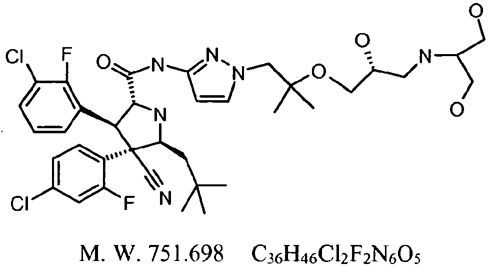

A mixture of rac-(2*R*,3*S*,4*R*,5*S*)-3-(3-chloro-2-fluoro-phenyl)-4-(4-chloro-2-fluoro-phenyl)-4-cyano-5-(2,2-dimethylpropyl)-pyrrolidine-2-carboxylic acid {1-[2-methyl-2-((*R*)-1-oxiranylmethoxy)-propyl]-1H-pyrazol-3-yl]-amide (43.6 mg, 0.066 mmol), isopropyl alcohol ( 2 mL), diisopropylethyl amine (0.1 mL, 0.56 mmol) and serinol (0.1 mL, 1.09 mmol) was microwaved at 130°C for 15 min. The mixture was then concentrated and purified by reverse phase chromatography (20-95% of ACN/water) to give rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-{2-[(*R*)-2-hydroxy-3-(2-hydroxy-1-hydroxymethyl-ethylamino)-propoxy]-2-methylpropyl}-1H-pyrazol-3-yl)-amide (14.2 mg, 28.6 %) as an off-white powder. HRMS (ES⁺) m/z Calcd for C₃₆H₄₆Cl₂F₂N₆O₅ + H [(M+H)⁺]: 751.2948, found: 751.2943.

### Example 173

### Preparation of rac-(2R,3S,4R,5S)-3-(3-chloro-2-fluoro-phenyl)-4-(4-chloro-2-fluoro-phenyl)-4-cyano-5-(2,2-dimethylpropyl)-pyrrolidine-2-carboxylic acid {1-[2-methyl-2-((S)-1-oxiranylmethoxy)-propyl]-1H-pyrazol-3-yl}-amide

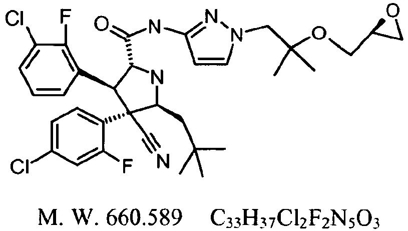

A mixture of rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (0.12 g, 0.28 mmol), 1-[2-methyl-2-((*S*)-1-oxiranylmethoxy)-propyl]-1H-pyrazol-3ylamine (52.4 mg, 0.248 mmol), 2-(7-azabenzotriazol-1-yl)-n,n,n',n'-tetramethyluronium hexafluorophosphate (HATU, 0.212 g, 0.56 mmol) and iPr₂NEt (0.25 mL, 1.4 mmol) in CH₂Cl₂ (3 mL) was stirred at rt overnight. The mixture was then diluted with CH₂Cl₂ and washed with water, brine. The organic phase was separated, filtered and dried over Na₂SO₄. The mixture was then concentrated and purified by column chromatography (4 g column, 1-100% ethyl acetate/heptane) to give rac-(2*R*,3*S*,4*R*,5*S*)-3-(3-chloro-2-fluoro-phenyl)-4-(4-chloro-2-fluoro-phenyl)-4-cyano-5-(2,2-dimethylpropyl)-pyrrolidine-2-carboxylic acid {1-[2-methyl-2-((*S*)-1-oxiranylmethoxy)-propyl]-1H-pyrazol-3-yl}-amide ( 58 mg, 35.4 %) as an off-white solid. HRMS (ES⁺) m/z Calcd for C₃₃H₃₇Cl₂F₂N₅O₃ + H (M+H)⁺]: 660.2315, found: 660.2316,

### Example 174

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((S)-3-amino-2-hydroxy-propoxy)-2-methyl-propyl]-1H-pyrazol-3-yl -amide

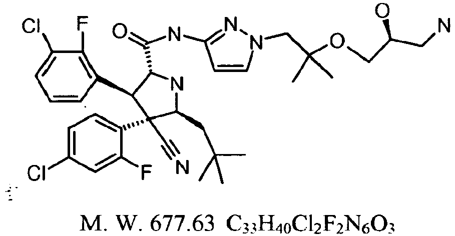

A mixture of rac-(2*R*,3*S*,4*R*,5*S*)-3-(3-chloro-2-fluoro-phenyl)-4-(4-chloro-2-fluoro-phenyl)-4-cyano-5-(2,2-dimethylpropyl)-pyrrolidine-2-carboxylic acid {1-[2-methyl-2-((*S*)-1-oxiranylmethoxy)-propyl]-1H-pyrazol-3-yl}-amide (203 mg, 0.307 mmol), isopropyl alcohol (2 mL), and ammonium hydroxide (2.5 mL) was microwaved at 130°C for 15 min. The mixture was then diluted with CH₂Cl₂ and washed with water, brine. The organic phase was separated, filtered and dried over Na₂SO₄. The mixture was then concentrated and purified by reverse phase chromatography (20-95% of ACN/water) to give rac- (2*R*,3*S*,4*R*,2*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((*S*)-3-amino-2-hydroxy-propoxy)-2-methyl-propyl]-1H-pyrazol-3-yl}-amide (50.2 mg, 24.1 %) as an white powder. HRMS (ES⁺) m/z Calcd for C₃₃H₄₀Cl₂F₂N₆O₃ + H [(M+H)⁺]: 677.2580, found: 677.2577.

### Example 175

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((S)-2,3-dihydroxy-propoxy)-2-methyl-propyl]-1H-pyrazol1-3-yl}-amide

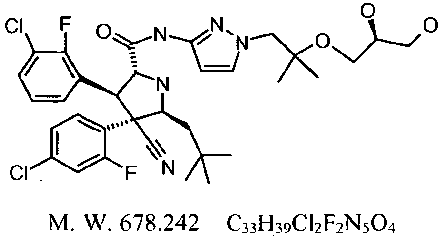

A mixture of rac-(2*R*,3*S*,4*R*,5*S*)-3-(3-chloro-2-fluoro-phenyl)-4-(4-chloro-2-fluoro-phenyl)-4-cyano-5-(2,2-dimethylpropyl)-pyrrolidine-2-carboxylic acid {1-[2-methyl-2-((*S*)-1-oxiranylmethoxy)-propyl]-1H-pyrazol-3-yl}-amide (51.2 mg, 0.078 mmol), water (2 mL), and acetone (10 mL) was cooled to 0°C, then a solution of 35% perchloric acid (0.1 mL) was added dropwise. The reaction was stirred an additional 0.5 h. Then an additional 1 mL of the 35% perchloric acid solution was added at 0°C, the reaction was allowed to warm to rt for 12 h. The mixture was then diluted with ethyl acetate and washed with sodium bicarbonate (sat'd). The organic phase was separated, dried over Na₂SO₄ and filtered. The mixture was then concentrated and purified by reverse phase chromatography (20-95% of ACN/water) to give rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((*S*)-2,3-dihydroxy-propoxy)-2-methylpropyl]-1H-pyrazol-3-yl}-amide (22.1 mg, 42 %) as an white powder. HRMS (ES⁺) m/z Calcd for C₃₃H₃₉Cl₂F₂N₅O₄ + H [(M+H)⁺]: 678.2420, found: 678.2416; C₃₃H₃₉Cl₂F₂N₅O₄ + [(Na+H)⁺]: 700.2239. found: 700.2235.

### Example 176

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((S)-3-dimethylamino-2-hydroxy-propoxy)-2-methyl-propyl]-1H-pyrazol-3-yl)-amide

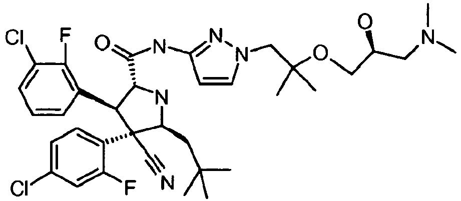

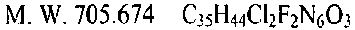

A mixture of rac-(2*R*,3*S*,4*R*,5*S*)-3-(3-chloro-2-fluoro-phenyl)-4-(4-chloro-2-fluoro-phenyl)-4-cyano-5-(2,2-dimethylpropyl)-pyrrolidine-2-carboxylic acid {1-[2-methyl-2-((S)-1-oxiranylmethoxy)-propyl]-1H-pyrazol-3-yl}-amide (87.5 mg, 0.133 mmol), isopropyl alcohol (2 mL), and dimethylamine (0.133 mL, 0.266 mmol, 2 M in dioxane) was microwaved at 130°C for 15 min. The mixture was extracted with dichloromethane and water. The organic layer was separated and the solvent evaporated under reduced pressure. The resulting oil was purified by reverse phase chromatography (20-95% of ACN/water) to give rac(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((*S*)-3-dimethylamino-2-hydroxy-propoxy)-2-methyl-propyl]-1H-pyrazol-3-yl)-amide (20.6 mg, 20.8 %) as a white powder. HRMS (ES⁺) m/z Calcd for C₃₅H₄₄Cl₂F₂N₆O₃ + H [(M+H)⁺]: 705.2890, found: 705.2893.

### Example 177

### Preparation of rac-{(S)-3-[2-(3-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-pyrazol-1-yl)-1,1-dimethyl-ethoxy]-2-hydroxy-propylamino}-acetic acid

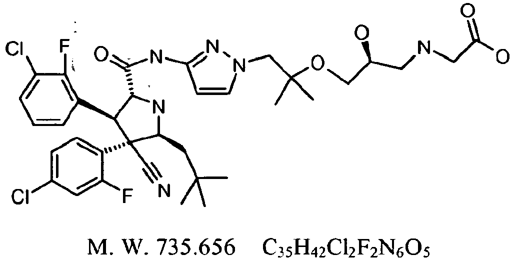

A mixture of rac-(2*R*,3*S*,4*R*,5*S*)-3-(3-chloro-2-fluoro-phenyl)-4-(4-chloro-2-fluoro-phenyl)-4-cyano-5-(2,2-dimethylpropyl)-pyrrolidine-2-carboxylic acid {1-[2-methyl-2-((*S*)-1-oxiranylmethoxy)-propyl]-1H-pyrazol-3-yl}-amide (85.8 mg, 0.133 mmol), isopropyl alcohol (2 mL), and t-butyl glycine (35 mg, 0.266 mmol) was microwaved at 130°C for 15 min. The solvent evaporated under reduced pressure. The resulting oil was dissolved in dichloromethane (2 mL) and cooled to 0°C; then TFA (1 mL) was slowly added and stirred for 4h. The mixture was concentrated under reduced pressure and purified by reverse phase chromatography (20-95% of ACN/water) to give rac-{(*S*)-3-[2-(3-{[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-pyrazol-1-yl)-1,1-dimethyl-ethoxy]-2-hydroxy-propylamino}-acetic acid (18.1 mg, 19 %) as an off-white powder. HRMS (ES⁺) m/z Calcd for C₃₅H₄₂Cl₂F₂N₆O₅ + H [(M+H)⁺]: 735.2635, found: 735.263

### Example 178

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)pyrrolidine-2-carboxylic acid {1-[2-((S)-2-hydroxy-3-methylamino-propoxy)-2-methyl-propyl]-1H-pyrazol-3-yl}-amide

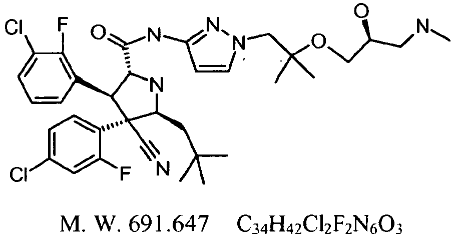

A mixture of rac-(2*R*,3*S*,4*R*,5*S*)-3-(3-chloro-2-fluoro-phenyl)-4-(4-chloro-2-fluoro-phenyl)-4-cyano-5-(2,2-dimethylpropyl)-pyrrolidine-2-carboxylic acid {1-[2-methyl-2-((*S*)-1-oxiranylmethoxy)-propyl]-1H-pyrazol-3-yl}-amide (87.5 mg, 0.133 mmol), isopropyl alcohol (2 mL), and methylamine (1.0 mL, 2 mmol, 2 M in methanol; old bottle) was microwaved at 130°C for 15 min. The solvent evaporated under reduced pressure. The resulting oil was purified by reverse phase chromatography (20-95% of ACN/water) to give rac-(2*R*,3*S*,4*R*,5*S*)-3-(3-chloro-2-fluoro-phenyl)-4-(4-chloro-2-fluoro-phenyl)-4-cyano-5-(2,2-dimethylpropyl)pyrrolidine-2-carboxylic acid {1-[2-((*S*)-2-hydroxy-3-methylamino-propoxy)-2-methylpropyl]-1H-pyrazol-3-yl}-amide (14.9 mg, 16.2 %) as an off-white powder. HRMS (ES⁺) m/z Calcd for C₃₄H₄₂Cl₂F₂N₆O₃ + H [(M+H)⁺]: 691.2737, found: 691.2731.

### Example 179

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((R)-3-dimethylamino-2-hydroxy-propoxy)-2-methyl-propyl]-1H-pyrazol-3-yl)-amide

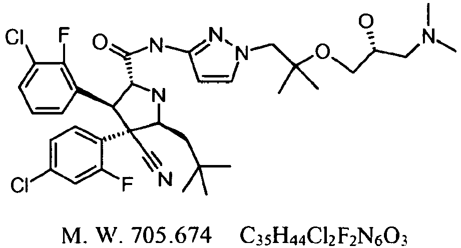

A mixture of rac-(2*R*,3*S*,4*R*,5*S*)-3-(3-chloro-2-fluoro-phenyl)-4-(4-chloro-2-fluoro-phenyl)-4-cyano-5-(2,2-dimethylpropyl)-pyrrolidine-2-carboxylic acid {1-[2-methyl-2-((*R*)-1-oxiranylmethoxy)-propyl]-1H-pyrazol-3-yl}-amide (0.69 g, 1.04 mmol), isopropyl alcohol (2 mL), and dimethylamine (1.04 mL, 2.08 mmol, 2 M in THF) was microwaved at 130°C for 15 min. The mixture was extracted with dichloromethane and water. The organic layer was separated and the solvent evaporated under reduced pressure. The resulting oil was purified by column chromatography (50% ethyl acetate/heptane) to give rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((*R*)-3-dimethylarnino-2-hydroxy-propoxy)-2-methyl-propyl]-1H-pyrazol-3-yl)-amide (147.2 mg, 20.05 %) as a white powder. HRMS (ES⁺) m/z Calcd for C₃₅H₄₄Cl₂F₂N₆O₃ + H [(M+H)⁺]: 705.2893, found:705.2893.

### Example 180

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid {1-[2-(tert-butyl-dimethyl-silanyloxy)-ethyl]-1H-pyrazol-3-yl}-amide

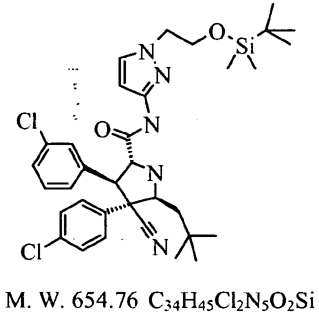

A mixture of rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (86.2 mg, 0.20 mmol), 1-[2-(tert-Butyl-dimethyl-silanyloxy)-ethyl]-1H-pyrazol-3-ylamine (72.42 mg, 0.3 mmol), 2-(7-azabenzotriazol-1-yl)-n,n,n',n'-tetramethyluronium hexafluorophosphate (HATU, 76 mg, 0.2 mmol) and iPr₂NEt (0.1 mL, 0.55 mmol) in CH₂Cl₂ (2 mL) was stirred at rt overnight. The mixture was then diluted with CH₂Cl₂ and washed with water, brine. The organic phase was separated, filtered and dried over Na₂SO₄. The mixture was then concentrated and purified by reverse phase chromatography (20-95% of ACN/water) to give rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid {1-[2-(tert-butyl-dimethyl-silanyloxy)-ethyl]-1H-pyrazol-3-yl}-amide (77.3mg, 59 %) as an off-white powder. HRMS (ES⁺) m/z Calcd for C₁₄H₄₅Cl₂N₅O₂ + H [(M+H)⁺]: 654.2793, found: 654.2790.

### Example 181

### Preparation of rac- (2R,3R,4R,5S)-3-(3-chloro-phenyl)-4-(4-chloro-phenyl)-4-cyano-5-(2,2-dimethyl-propyl)-pyrrolidine-2-carboxylic acid [1-((R)-2,2-dimethyl-[1,3]dioxolan-4-ylmethyl)-1H-pyrazol-3-yl]-amide

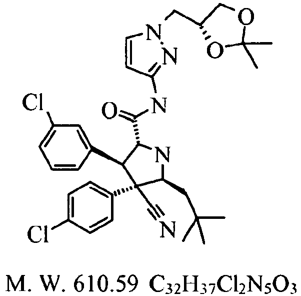

A mixture of rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (86.2 mg, 0.20 mmol), 1-(2,2-Dimethyl-[1,3]dioxolan-4-ylmethyl)-1H-pyrazol-3-ylamine (59.14 mg, 0.3 mmol), 2-(7-azabenzotriazol-1-yl)-n,n,n',n'-tetramethyluronium hexafluorophosphate (HATU, 76 mg, 0.2 mmol) and iPr₂NEt (0.1 mL, 0.55 mmol) in CH₂Cl₂ (2 mL) was stirred at rt overnight. The mixture was then diluted with CH₂Cl₂ and washed with water, brine. The organic phase was separated, filtered and dried over Na₂SO₄. The mixture was then concentrated and purified by reverse phase chromatography (20-95% of ACN/water) to give rac- (2*R*,3*R*,4*R*,5*S*)-3-(3-chloro-phenyl)-4-(4-chloro-phenyl)-4-cyano-S-(2,2-dimethyl-propyl)-pyrrolidine-2-carboxylic acid [1-((*R*)-2,2-dimethyl-[1,3]dioxolan-4-ylmethyl)-1H-pyrazol-3-yl]-amide (91.2mg, 74.68 %) as an off-white powder. HRMS (ES⁺) m/z Calcd for C₃₄H₄₅Cl₂N₅O₂ + H [(M+H)⁺]: 610.2346, found: 610.2345.

### Example 182

### Preparation of rac-(2R,3R,4R,5S)-3-(3-chloro-phenyl)-4-(4-chloro-phenyl)-4-cyano-5-(2,2-dimethyl-propyl)-pyrrolidine-2-carbo xylic acid ((S)-2,3-dihydroxy-propyl)-amide and rac- (2S,3S,4S,5R)-3-(3-chloro-phenyl)-4-(4-chloro-phenyl)-4-cyano-5-(2,2-dimethyl-propyl)-pyrrolidine-2-carboxylic acid ((S)-2,3-dihydroxy-propyl)-amide

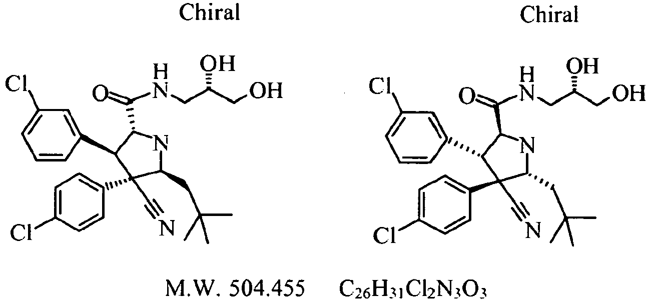

A mixture of rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (0.86, 2.0 mmol), (*S*)-3-amino-propane-1,2-diol (0.27 g, 3.0 mmol), 2-(7-azabenzotriazol-1-yl)-n,n,n',n'-tetramethyluronium hexafluorophosphate (HATU, 0.76 g, 2.0 mmol) and iPr₂NEt (1.0 mL, 5.5 mmol) in CH₂Cl₂ (40 mL) was stirred at rt overnight. The mixture was then diluted with CH₂Cl₂ and washed with water, brine. The organic phase was separated, filtered and dried over Na₂SO₄. The mixture was then concentrated to yield a product that was purified by silica column chromatography (1-100% EtOAc/heptane) to give 1.09g, 100% two diasteroisomers. The mixture was sent SFC to be separated (35 % CH₃OH, 100 barr, 30°C) to give rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid ((*S*)-2,3-dihydroxy-propyl)-amide (187.6 mg, 21.7 %) as an off-white powder. HRMS (ES⁺) m/z Calcd for C₂₆H₃₁Cl₂N₃O₃ + H [(M+H)⁺]: 504.455, found: 504.455 and rac- (2*S*,3*S*,4*S*,5*R*)-3-(3-chloro-phenyl)-4-(4-chloro-phenyl)-4-cyano-5-(2,2-dimethyl-propyl)-pyrrolidine-2-carboxylic acid ((*S*)-2,3-dihydroxy-propyl)-amide (188.1 mg, 74.68 %) as an off-white foam. HRMS (ES⁺) m/z Calcd for C₂₆H₃₁Cl₂N₃O₃ + H [(M+H)⁺]: 504.1815, found: 504.1815.

### Example 183

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid [1-(2-hydroxy-ethyl)-1H-pyrazol-3-yl]-amide

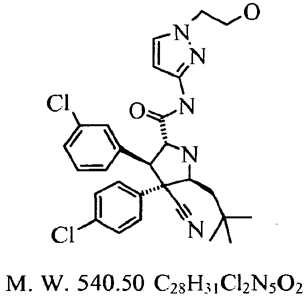

A mixture of rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid {1-[2-(tert-butyl-dimethyl-silanyloxy)-ethyl]-1H-pyrazol-3-yl}-amide (65.2 mg, 0.0995 mmol), acetic acid (2.0 mL, 31 mmol), water (1 mL) and stirred at RT over the weekend (48 h). Reaction complete by 50%. The mixture was then diluted with EtOAc and washed with NaHCO₃(s). The organic phase was separated, filtered and dried over Na₂SO₄. The mixture was then concentrated under reduced vacuum and purified by reverse phase chromatography (20-95% of ACN/water) to give rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid [1-(2-hydroxy-ethyl)-1H-pyrazol-3-yl]-amide (21.6 mg, 40.22 %) as a white powder. HRMS (ES⁺) m/z Calcd for C₂₈H₃₁Cl₂N₅O₂ + H[(M+H)⁺]: 540.1928, found: 540.1926.

### Example 184

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid [1-((R)-2,3-dihydroxy-propyl)-1H-pyrazol-3-yl]-amide

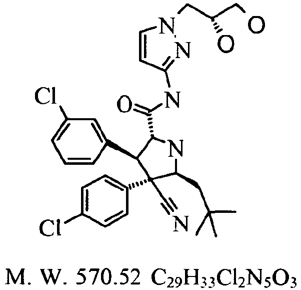

A mixture of rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid [1-((*R*)-2,2-dimethyl-[1,3]dioxolan-4-ylmethyl)-1H-pyrazol-3-yl]-amide (81 mg, 0.133 mmol), pyridinium p-toluene sulfonic acid (5 mg, 0.0198 mmol) and methanol (4 mL) was microwaved at 120°C for 5 min. Solvent evapoarated and purified by reverse phase chromatography (20-95% of ACN/water) to rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid [1-((*R*)-2,3-dihydroxy-propyl)-1H-pyrazol-3-yl]-amide (26.5 mg, 35 %) as an off-white powder. HRMS (ES⁺) m/z Calcd for C₂₉H₃₃Cl₂N₅O₃ + H [(M+H)⁺]: 570.2033, found: 570.2032.

### Example 185

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid 2-trifluoromethyl-benzylamide

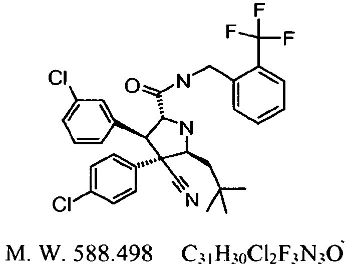

A mixture of rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (86.2 mg, 0.20 mmol), 2-trifluoromethyl-benzyl amine (52.55 mg, 0.3 mmol), 2-(7-azabenzotriazol-1-yl)-n,n,n',n'-tetramethyluronium hexafluorophosphate (HATU, 76 mg, 0.2 mmol) and iPr₂NEt (0.1 mL, 0.55 mmol) in CH₂Cl₂ (2 mL) was stirred at rt overnight. The mixture was then diluted with CH₂Cl₂ and washed with water, brine. The organic phase was separated, filtered and dried over Na₂SO₄. The mixture was then concentrated and purified by reverse phase chromatography (20-95% of ACN/water) to give rac rac-(2*R*,3*R*,4*R*,5*S*)-3-(3-chloro-phenyl)-4-(4-chloro-phenyl)-4-cyano-5-(2,2-dimethylpropyl)-pyrrolidine-2-carboxylic acid 2-trifluoromethyl-benzylamide (38 mg, 32.3 %) as an off-white powder. HRMS (ES⁺) m/z Calcd for C₃₁H₃₀Cl₂F₃N₃O + H [(M+H)⁺]: 588.1791, found: 588.1795.

### Example 186

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid 4-(2-oxo-pyrrolidin-1-yl)-benzylamide

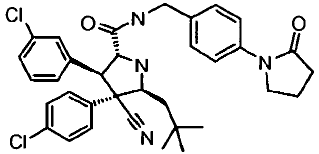

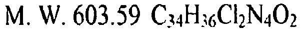

A mixture of rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (86.2 mg, 0.20 mmol), 4-aminomethyl phenyl pyrrolidin-2-one (42.94 mg, 0.3 mmol), 2-(7-azabenzotriazol-1-yl)-n,n,n',n'-tetramethyluronium hexafluorophosphate (HATU, 76 mg, 0.2 mmol) and iPr₂NEt (0.1 mL, 0.55 mmol) in CH₂Cl₂ (2 mL) was stirred at rt overnight. The mixture was then diluted with CH₂Cl₂ and washed with water, brine. The organic phase was separated, filtered and dried over Na₂SO₄). The mixture was then concentrated and purified by reverse phase chromatography (20-95% of ACN/water) to give rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid 4-(2-oxo-pyrrolidin-1-yl)-benzylamide (48.9 mg, 40.5 %) as an off-white powder. HRMS (ES⁺) m/z Calcd for C₃₄H₃₆Cl₂N₄O₂+ H [(M+H)⁺]: 603.2288, found: 603.2289.

### Example 187

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid (tetrahydro-pyran-4-ylmethyl)-amide

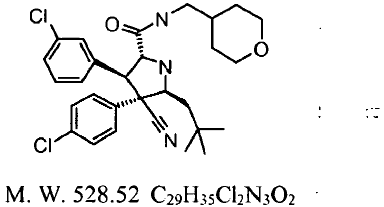

A mixture of rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (86.2 mg, 0.20 mmol), 4-aminomethyl tetrahydropyran (34.5 mg, 0.3 mmol), 2-(7-azabenzotriazol-1-yl)-n,n,n',n'-tetramethyluronium hexafluorophosphate (HATU, 76 mg, 0.2 mmol) and iPr₂NEt (0.1 mL, 0.55 mmol) in CH₂Cl₂ (2 mL) was stirred at rt overnight. The mixture was then diluted with CH₂Cl₂ and washed with water, brine. The organic phase was separated, filtered and dried over Na₂SO₄. The mixture was then concentrated and purified by reverse phase chromatography (20-95% of ACN/water) to give rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (tetrahydro-pyran-4-ylmethyl)-amide (10.8 mg, 10.2 %) as an off-white powder. HRMS (ES⁺) m/z Calcd for C₂₉H₃₅Cl₂N₃O₂ + H [(M+H)⁺]: 528.2179, found: 528.2180.

### Example 188

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid (3-hydroxy-2-hydroxymethyl-propyl)-amide

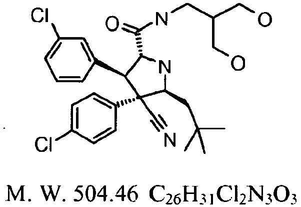

A mixture of rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (86.2 mg, 0.20 mmol), 2-aminomethyl-propane-1,3-diol (31.5 mg, 0.3 mmol), 2-(7-azabenzotriazol-1-yl)-n,n,n',n'-tetramethyluronium hexafluorophosphate (HATU, 76 mg, 0.2 mmol) and iPr₂NEt (0.1 mL, 0.55 mmol) in CH₂Cl₂ (2 mL) was stirred at rt overnight. The mixture was then diluted with CH₂Cl₂ and washed with water, brine. The organic phase was separated, filtered and dried over Na₂SO₄. The mixture was then concentrated and purified by reverse phase chromatography (20-95% of ACN/water) to give rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (3-hydroxy-2-hydroxymethyl-propyl)-amide (31.2 mg, 30.9%) as an off-white powder. HRMS (ES⁺) m/z Calcd for C₂₆H₃₁Cl₂N₃O₃ + [(M+H)⁺]: 504.455, found: 504.1815.

### Example 189

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid [2-(2-amino-ethoxy)-ethyl]-amide

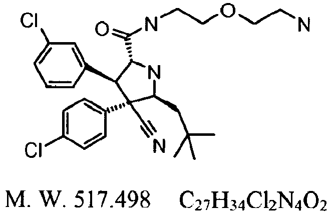

A mixture of rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (86.2 mg, 0.20 mmol), 2,2'-oxybis(ethylamine) (31.3 mg, 0.3 mmol), 2-(7-azabenzotriazol-1-yl)-n,n,n',n'-tetramethyluronium hexafluorophosphate (HATU, 76 mg, 0.2 mmol) and iPr₂NEt (0.1 mL, 0.55 mmol) in CH₂Cl₂ (2 mL) was stirred at rt overnight. The mixture was then diluted with CH₂Cl₂ and washed with water, brine. The organic phase was separated, filtered and dried over Na₂SO₄. The mixture was then concentrated and purified by reverse phase chromatography (20-95% of ACN/water) to give rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid [2-(2-amino-ethoxy)-ethyl]-amide (20.3 mg, 19.6%) as an off-white powder. HRMS (ES⁺) m/z Calcd for C₂₇H₃₄Cl₂N₄O₂ + H [(M+H)⁺]:517.2132, found: 517.2133

### Example 190

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid (3-methanesulfonyl-propyl)-amide

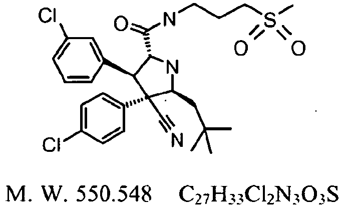

A mixture of rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (3-methylsulfanyl-propyl)-amide (119.2 mg, 0.23 mmol) and dichloromethane (2 mL) was added to a solution of refluxing acetic acid (5 mL, 79.4 mmol) and 30% hydrogen peroxide (2 mL) for 5 min. The reaction mixture was cooled to room temperature and stored in the freezer overnight. The mixture was diluted with water and dichloromethane, the organic layer was separated and solvent removed under reduced pressure. The resulting oil was carried directly to the next step by adding acetic acid (5 mL, 79.4 mmol) and zinc dust (0.85 g, 13 mmol). After stirring at room temperature for 3 h, an additional amount of zinc dust (0.85 g, 13 mmol) was added and stirred for an additional 3h at room temperature. Reaction worked up by filtration through celite, washing with dichloromethane (50 mL). The filtrate was washed with water (2x) and the organic layer was separated, dried with MgSO₄, filtered and the solvent evaporated under reduced pressure to yield crude oil. Purification by reverse phase chromatography (20-95% of ACN/water) to give rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (3-methanesulfonyl-propyl)-amide (36.5 mg, 28.8 %) as an off-white powder. HRMS (ES⁺) m/z Calcd for C₂₇H₃₃Cl₂N₃O₃S ÷ H [(M+H)⁺]: 550.1693, found: 550.1692.

### Example 191

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid (2-methanesulfonyl-ethyl)-arnide

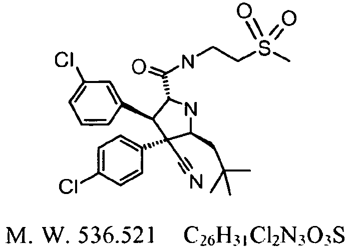

A mixture of rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (86.2 mg, 0.20 mmol), 2-methanesulfonyl-ethylamine (54.6 mg, 0.3 mmol), 2-(7-azabenzotriazol-1-yl)-n,n,n',n'-tetramethyluronium hexafluorophosphate (HATU, 76 mg, 0.2 mmol) and iPr₂NEt (0.1 mL, 0.55 mmol) in CH₂Cl₂ (2 mL) was stirred at rt overnight. The mixture was then diluted with CH₂Cl₂ and washed with water, brine. The organic phase was separated, filtered and dried over Na₂SO₄. The mixture was then concentrated and purified by reverse phase chromatography (20-95% of ACN/water) to give rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (3-hydroxy-2-hydroxymethyl-propyl)-amide (65.2 mg, 60.8 %) as a white powder. HRMS (ES⁺) m/z Calcd for C₂₆H₃₁Cl₂N₃O₃S + H [(M+H)⁺]: 536.1536, found: 536.1536.

### Example 192

### Preparation of rac-4-{[(2R,35,4R,5S)-3-(3-chloro-2-fluoro-phcnyl)-4-(4-chloro-2-fluoro-phenyl)-4-cyano-5-(2,2-dimethyl-propyl)-pyrrolidine-2-carbonyl]-amino}-cyclohexylamino-1-carboxylic acid tert-butyl ester

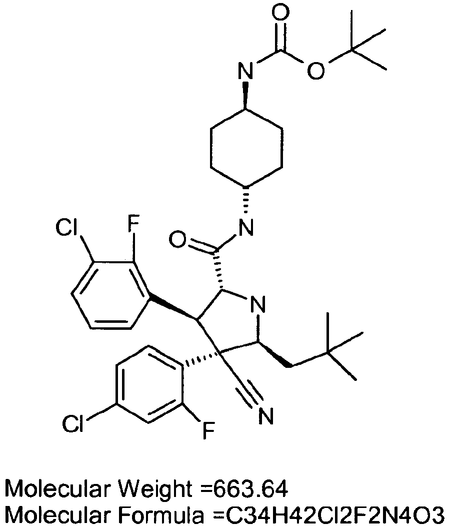

In a manner similar to the method described in **Examples 42c, 42d**, rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid trifluoroacetic acid prepared in **Example 52c** (582 mg, 1 mmol) was reacted with 4-Amino-cyclohexylamino-1-carboxylic acid tert-butyl ester(APAC, 429 mg, 2 mmol), HATU (Aldrich, 760.4 mg, 2 mmol) and iPr₂NEt (Aldrich, 350 uL, 2 mmol) in CH₂Cl₂ at room temperature for 1.5 hr. The solvent was reduced and the residue was loaded on 40 g silica gel column and eluted with MeOH/CH₂Cl₂ (2-5%) to give a white foam. 783 mg HRMS (ES⁺) m/z Calcd for C₃₄H₄₂Cl₂F₂N₄O₃ + H [(M+H)⁺]: 663.2675, found: 663.2675.

### Example 193

### Preparation of rac-4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-trans-cyclohexylamine trifluoroacetic acid salt

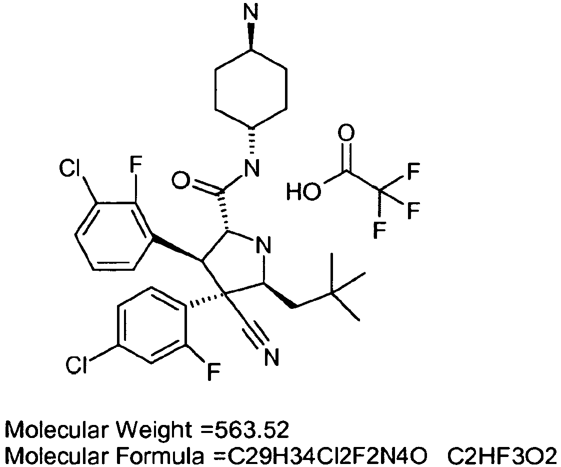

To a stirred solution oftrifluoroacetic acid in methylene chloride(3 mL/7 mL) at rt., rac-4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-cyclohexylamino-1-carboxylic acid tert-butyl ester (760 mg) was added and the mixture was stirred for 30 min.. The solvent was removed and the residue was treated with ether/hexane. The solid was filtered and washed with hexane to give a white solid. 860 mg. [(M+H)⁺]: 563.

### Example 194

### Preparation of rac-4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-trans-cyclohexyl-N-methanesulfonamide

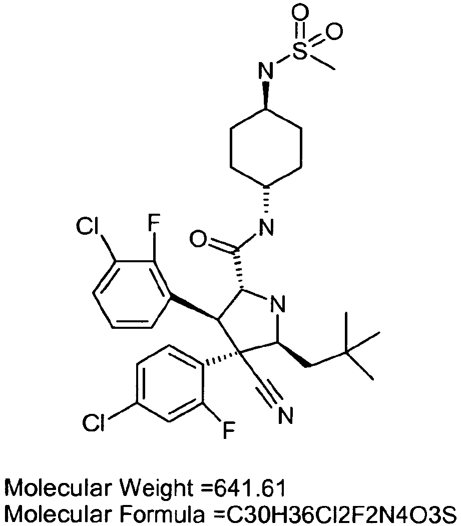

To a stirred solution of rac-4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-trans-cyclohexylamine trifluoroacetic acid salt (80 mg, 0.1 mmol) in methylene chloride (5 mL), Methane sulfonyl chloride (Aldrich, 11.6 uL, 0.15 mmol) was added followed by the addition of triethylamine(70 uL, 0.50 mmol). The mixture was stirred at rt for 1 hr and the reaction was quenched with addition of water. The mixture was extracted with methylene chloride (2X10 mL) and the extracts were dried with magnesium sulfate. The solvent was removed and the residue was chromatographied on ISCO machine (column, 12g, eluent 5% MeOH/CH2Cl2) to give a white solid. 44 mg.

HRMS (ES⁺) m/z Calcd: [(M+H)⁺]: 641.1926, found: 641.1926.

### Example 195

### Preparation of rac-4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-trans-cyclohexyl-N-methanesulfonamide

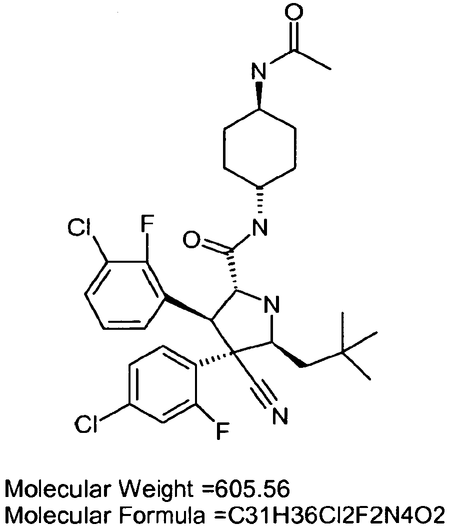

To a stirred solution of rac-4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-trans-cyclohexylamine trifluoroacetic acid salt (80 mg, 0.1 mmol) in methylene chloride (5 mL), Acetic anhydride (Aldrich, 16 uL, 0.15 mmol) was added followed by the addition of triethylamine(70 uL, 0.50 mmol). The mixture was stirred at rt for 30 min. and the reaction was quenched with addition of water. The mixture was extracted with methylene chloride (2X10 mL) and the extracts were dried with magnesium sulfate. The solvent was removed and the residue was chromatographied on ISCO machine (column, 12g, eluent 5% MeOH/CH2Cl2) to give a white solid. 30 mg.

HRMS (ES⁺) m/z Calcd: [(M+H)⁺]: 605.2256, found: 605.2259

### Example 196

### Preparation of rac-4-([(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-trans-cyclohexyl-(1,1-dioxo)-2-isothiazo lidine

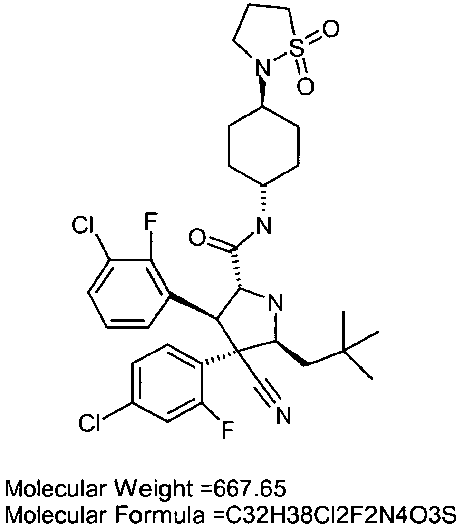

To a stirred solution of rac-4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-trans-cyclohexylamine trifluoroacetic acid salt (200 mg, 0.253 mmol) in methylene chloride (5 mL), 3-chloro-n-propyl sulfonyl chloride (Aldrich, 40 uL, 0.328 mmol) was added followed by the addition of triethylamine(176 uL, 1.265 mmol). The mixture was stirred at rt for 4 hrs and the reaction was quenched with addition of water. The mixture was extracted with methylene chloride (2X10 mL) and the extracts were dried with magnesium sulfate. The solvent was removed and the residue was chromatographied on ISCO machine (column, 12g, eluent 5% MeOH/CH2Cl2) to give a white solid. 122 mg.

The white solid (80 mg, 0.114 mmol) was dissolved in acetonitrile/water(3:1, 1 mL) and KI (10 mg) and KOAc (55 mg, 0.56 mmol) were added and the mixture was heated on a microwave for 30 min. at 150°C. The mixture was cooled and water was added. The resulting mixture was extracted with ethyl acetate (3 x 8 mL). The extracts were dried with magnesium sulfate and concentrated. The residue was chromatorgraphied (5% MeOH/CH₂Cl₂) to give a solid. 46 mg. HRMS (ES⁺) m/z Calcd: [(M+H)⁺]: 667.2053, found: 667.2084.

### Example 197

### Preparation of rac-4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-trans-cyclohexyl-urea

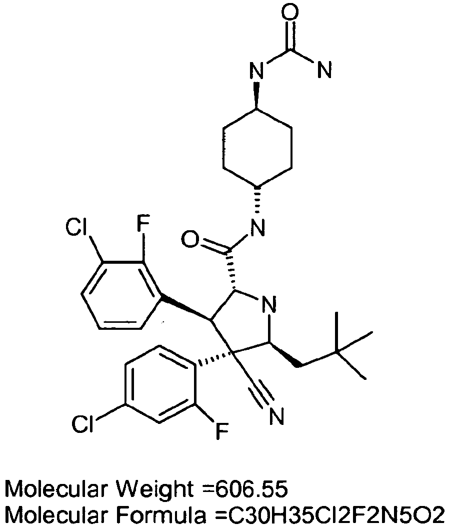

To a stirred solution of rac-4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-trans-cyclohexylamine trifluoroacetic acid salt (100 mg, 0.126 mmol) in 1,4-dioxane (5 mL), N-trimethylsilyl isocyanate (Aldrich, 42 uL, 0.316 mmol) was added followed by the addition of triethylamine(44 uL, 0.316 mmol). The mixture was stirred at rt overnight. The reaction was quenched with addition of water. The mixture was extracted with methylene chloride (2X10 mL) and the extracts were dried with magnesium sulfate. The solvent was removed and the residue was purified on a reverse phase HPLC to give a white solid. 39 mg.

HRMS (ES⁺) m/z Calcd: [(M+H)⁺]: 606.2209, found: 606.2209

### Example 198

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid N-[1-(2-hydroxy ethyl)-piperidin-4-yl]amide

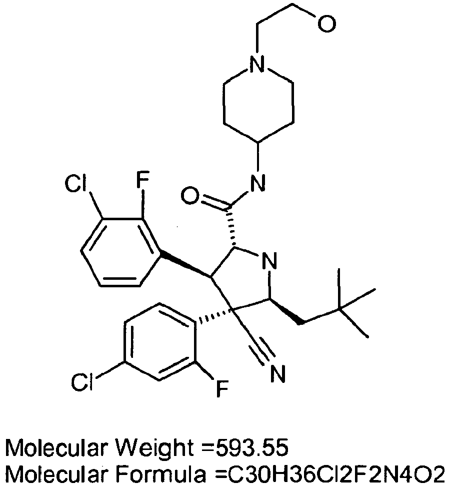

To a stirred solution of rac-4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-trans-cyclohexylamine trifluoroacetic acid salt (80 mg, 0.103 mmol) in ethanol (5 mL), 2-bromoethanol (Aldrich, 11 uL, 0.15 mmol) was added followed by the addition of sodium carbonate(106 mg, 1 mmol). The mixture was stirred at reflux overnight. The reaction was quenched with addition of water. The mixture was extracted with EtOAc (2X10 mL) and the extracts were dried with magnesium sulfate. The solvent was removed and the residue was purified on a ISCO machine (12g column, eluent, 5 MeOH/CH₂Cl₂) to give a white solid. 22 mg. HRMS (ES⁺) m/z Calcd: [(M+H)⁺]: found: 593.2256.

### Example 199

### Preparation of rac-4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-piperidine-1-sulfonic acid amide

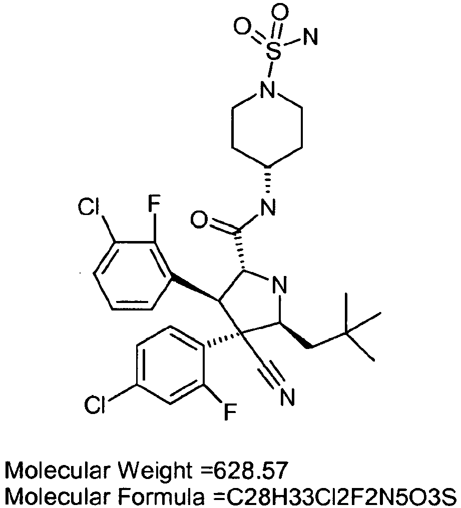

To a stirred solution of rac-4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-trans-cyclohexylamine trifluoroacetic acid salt (50 mg, 0.091 mmol) in THF (3 mL), sulfamide (Aldrich, 40 mg, 0.416 mmol) was added and the mixture was stirred at 110°C for 25 min. on microwave oven. The reaction was quenched with addition of water. The mixture was extracted with EtOAc (2X10 mL) and the extracts were dried with magnesium sulfate. The solvent was removed and the residue was purified on a reverse phase HPLC(25-90, acetonitrile/water) to give a white solid. 49 mg.

HRMS (ES⁺) m/z Calcd: [(M+H)⁺]: 678.1722, found: 678.1728.

### Example 200

### Preparation of rac 3-{4-[(2R,3R,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-piperazin-1-yl-acetic acid

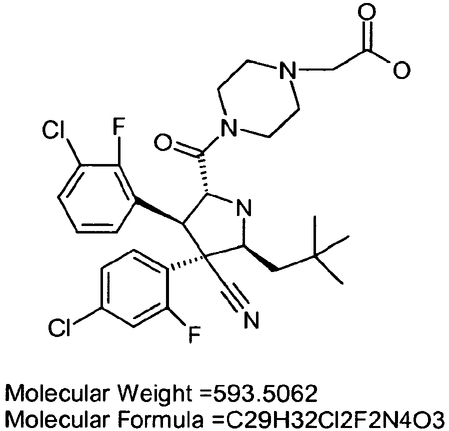

To a stirred solution of rac-(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carboxylic acid trifluoroacetic acid salt (475 mg, 0.871 mmol) in methylene chloride (5 mL), HATU (Aldrich, 490 mg, 1.29 mmol) was added followed by the addition of DIPEA( 0.9 mL, 5.2 mmol) and piperazin-1-yl-acetic acid tert-butyl ester (352 mg, 1.29 mmol). The mixture was stirred at rt for overnight. The reaction was quenched with addition of water. The mixture was extracted with methylene chloride (2X10 mL) and the extracts were dried with magnesium sulfate. The solvent was removed and the residue was purified on an ISCO machine (40g column, eluent, 50% EtOAc/hexanes) to give a white solid. 525 mg.

The solid (500 mg) was dissolved in 30% TFA/methylene chloride and the solution was stirred for 2hrs at rt. The solvent was removed at reduced pressure and the residue was triturated with ether and hexane to give a white solid. 560 mg

HRMS (ES⁺) m/z Calcd: [(M+H)⁺]: 592.1820, found: 592.1819

### Example 201

### Preparation of rac 3-{4-[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-piperazin-1-yl}-N,N-bis-(2-methoxy-ethyl)-acetamide

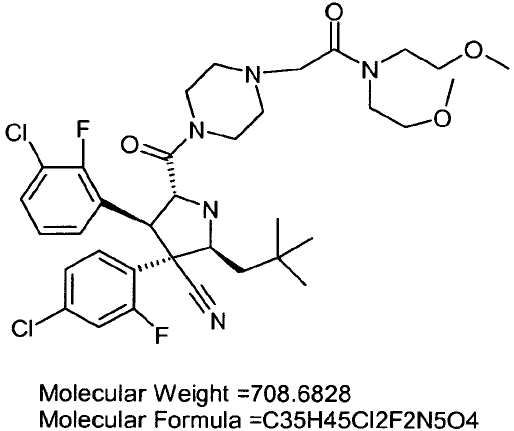

To a stirred solution of rac 3-{4-[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-piperazin-1-yl}-acetic acid (80 mg, 0.113 mmol) in methylene chloride (5 mL), HATU (Aldrich, 65 mg, 0.17 mmol) was added followed by the addition of DIPEA(0.06 mL, 0.34 mmol) di-(2-methoxy)-ethylamine (Aldrich, 35 uL, 0.17 mmol). The mixture was stirred at rt for overnight. The reaction was quenched with addition of water. The mixture was extracted with methylene chloride (2X10 mL) and the extracts were dried with magnesium sulfate. The solvent was removed and the residue was purified on an ISCO machine (12g column, eluent, 20% EtOAc/methylene chloride) to give a white solid. 70 mg.

HRMS (ES⁺) m/z Calcd: [(M+H)⁺]: 708.2890, found: 708.2887

### Example 202

### Preparation of rac 3-{4-[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-piperazin-1-yl}-N,N-bis-(2-hydroxy-ethyl)-acetamide

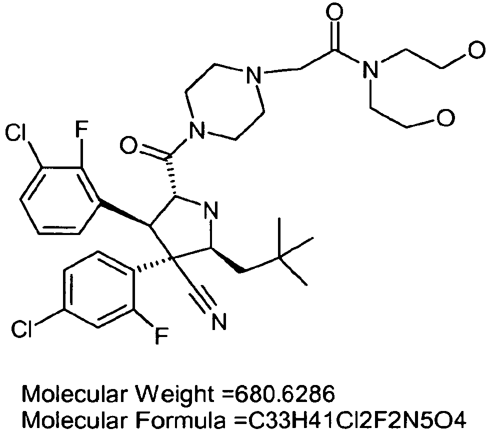

To a stirred solution of rac 3-{4-[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-piperazin-1-yl}-acetic acid (80 mg, 0.113 mmol) in methylene chloride (5 mL), HATU (Aldrich, 65 mg, 0.17 mmol) was added followed by the addition of DIPEA(0.06 mL, 0.34 mmol) di-(2-hydroxy)-ethylamine (Across, 35 uL, 0.17 mmol). The mixture was stirred at rt for overnight. The reaction was quenched with addition of water. The mixture was extracted with methylene chloride (2X10 mL) and the extracts were dried with magnesium sulfate. The solvent was removed and the residue was purified on a reversed HPLC (10-90%) to give a white solid. 30 mg.

HRMS (ES⁺) m/z Calcd: [(M+H)⁺]: 680.2577, found: 680.2575

### Example 203

### Preparation of rac 3-{4-[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-piperazin-1-yl}- N-(3-methoxy-propyl)-acetamide

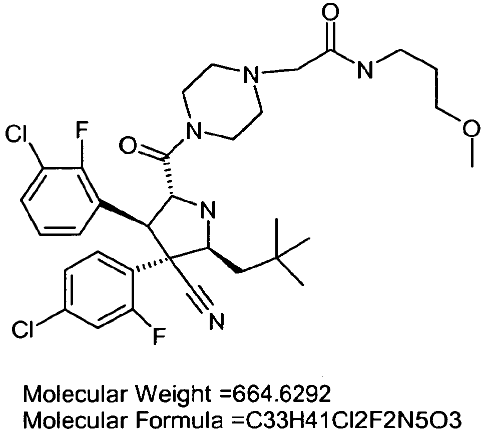

To a stirred solution of rac 3-{4-[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-piperazin-1-yl}-acetic acid (80 mg, 0.113 mmol) in methylene chloride (5 mL), HATU (Aldrich, 65 mg, 0.17 mmol) was added followed by the addition of DIPEA(0.06 mL, 0.34 mmol) and 3-methoxypropylamine (Aldrich, 0.17 mmol). The mixture was stirred at rt for overnight. The reaction was quenched with addition of water. The mixture was extracted with methylene chloride (2X10 mL) and the extracts were dried with magnesium sulfate. The solvent was removed and the residue was purified on a ISCO machine (12g column, eluent, 20% EtOAc/methylene chloride) to give a white solid. 80 mg.

HRMS (ES⁺) m/z Calcd: [(M+H)⁺]: 664.2628, found: 664.2625

### Example 204

### Preparation of rac 2-{4-[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-pipera zin-1-yl}-acetamide

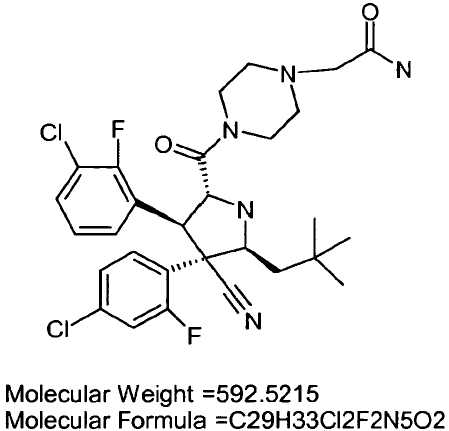

phenyl)-4-(4-chloro-2-fluoro-phenyl)-4-cyano-5-(2,2-dimethyl-propyl)-p yrrolidine-2-carboxylic acid (80 mg, 0.138 mmol) in methylene chloride (5 mL), HATU (Aldrich, 79 mg, 0.21 mmol) was added followed by the addition of DIPEA(0.145mL, 0.83 mmol) and 2-piperazin-1-yl-acetamide (Chembridge, 45 mg, 0.21 mmol). The mixture was stirred at rt for overnight. The reaction was quenched with addition of water. The mixture was extracted with methylene chloride (2X10 mL) and the extracts were dried with magnesium sulfate. The solvent was removed and the residue was purified on a ISCO machine (12g column, eluent, 20% EtOAc/methylene chloride) to give a white solid. 87 mg.

HRMS (ES⁺) m/z Calcd: [(M+H)⁺]: 592.2052, found: 592.2054

### Example 205

### Preparation of rac (2S,3R,4S,5R)-4-(3-chloro-2-fluoro-phenyl)-3-(4-chloro-2-fluoro-phenyl)-2-(2,2-dimethyl-propyl)-5-[4-(2-morpholin-4-yl-2-oxo-ethyl)-piperazi ne-1-carbonyl]-pyrrolidine-3-carbonitrile

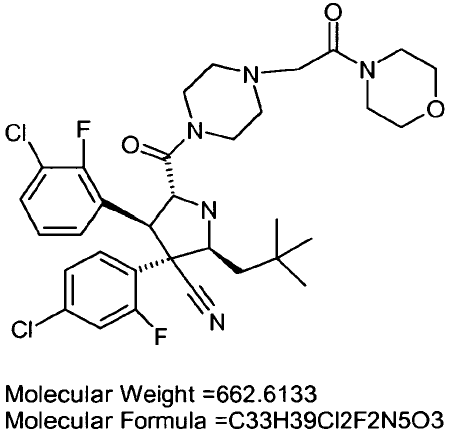

To a stirred solution of rac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (80 mg, 0.138 mmol) in methylene chloride (5 mL), HATU (Aldrich, 79 mg, 0.21 mmol) was added followed by the addition of DIPEA (0.145mL, 0.83 mmol) and 1-morpholin-4-yl-2-piperazin-1-yl-ethanone (Oakwood, 44 mg, 0.21 mmol). The mixture was stirred at rt for overnight. The reaction was quenched with addition of water. The mixture was extracted with methylene chloride (2X10 mL) and the extracts were dried with magnesium sulfate. The solvent was removed and the residue was purified on a ISCO machine (12g column, eluent, 20% EtOAc/methylene chloride) to give a white solid. 63 mg.

HRMS (ES⁺) m/z Calcd: [(M+H)⁺]: 662.2471, found: 662.2471

### Example 206

### Preparation of rac 2-{4-[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-piperazin-1-yl}-N-[2-((S)-2,2-dimethyl-[1,3]dioxolan-4-yl)-ethyl]-acetamide

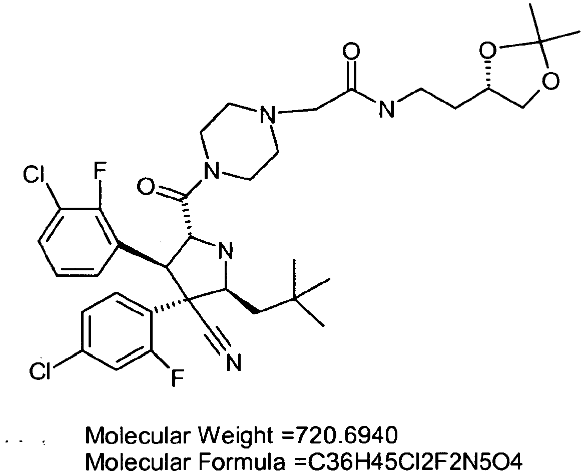

To a stirred solution ofrac 3-{4-[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-piperazin-1-yl}-acetic acid (80 mg, 0.113 mmol) in methylene chloride (5 mL), HATU (Aldrich, 86 mg, 0.23 mmol) was added followed by the addition of DIPEA (0.06 mL, 0.34 mmol) and 2-((S)-2,2-dimethyl-[1,3]dioxolan-4-yl)-ethylamine (Prepared before, 33 mg, 0.23 mmol). The mixture was stirred at rt for overnight. The reaction was quenched with addition of water. The mixture was extracted with methylene chloride (2X10 mL) and the extracts were dried with magnesium sulfate. The solvent was removed and the residue was purified on a reverse phase HPLC (eluent, 40-90% ACN/Water) to give a white solid. 27 mg.

MS (ES⁺) m/z Calcd: [(M+H)⁺]: 720, found: 720

### Example 207

### Preparation of rac 2-{4-[(2R,35,4R,5S)-3-(3-Chloro-2-fluoro-phenyl)-4-(4-chloro 2-fluoro-phenyl)-4-cyano-5-(2,2-dimethyl-propyl)-pyrrolidine-2-carbonyl]-pipera zin-1-yl}-N-((S)-3,4-dihydroxy-butyl)-acetamide

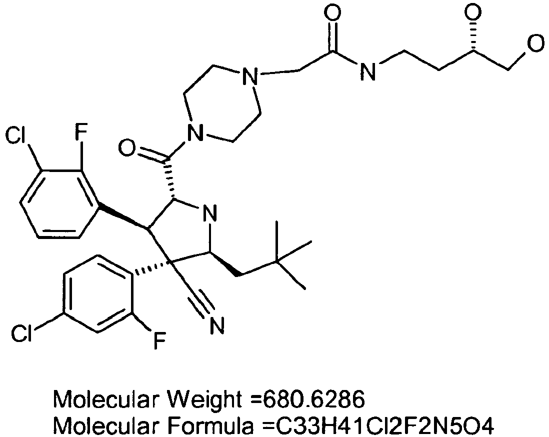

To a stirred solution ofrac 2-{4-[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-piperazin-1-yl}-N-[2-((S)-2,2-dimethyl-[1,3]dioxolan-4-yl)-ethyl]-acetamide (32 mg, 0.0445 mmol) in 1,4-dioxane (5 mL), 4 M HCl (2 mL) was added and the mixture was stirred at rt for 4 hrs.. The mixture was concentrated and the residue was dissolved in a mixture of 1,4-dioxane and freeze dried to give a white solid. 31 mg.

MS (ES⁺) m/z Calcd: [(M+H)⁺]: 680, found: 680.

### Example 208

### Preparation of rac {1-[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-piperidin-4-yl}-acetic acid methyl ester

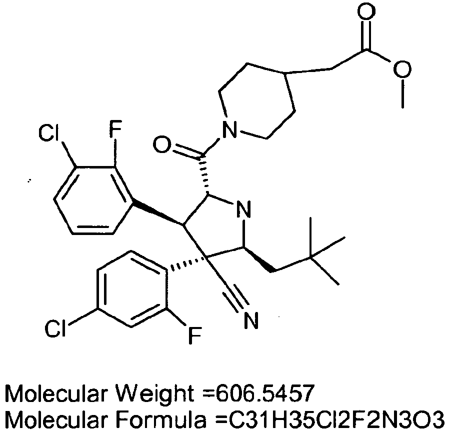

To a stirred solution of rac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (581mg, 1 mmol) in methylene chloride (10 mL), HATU (Aldrich, 500 mg, 1.3 mmol) was added followed by the addition of DIPEA (0.35mL, 2.2 mmol) and piperidin-4-yl-acetic acid methyl ester (Oakwood, 205 mg, 1.3 mmol). The mixture was stirred at rt for 3 hrs. The reaction was quenched with addition of water. The mixture was extracted with methylene chloride (2X10 mL) and the extracts were dried with magnesium sulfate. The solvent was removed and the residue was purified on a ISCO machine (12g column, eluent, 20% EtOAc/methylene chloride) to give a white solid. 586 mg.

HRMS (ES⁺) m/z Calcd: [(M+H)⁺]: 606.2097, found: 606.2096.

### Example 209

### Preparation of rac {1-[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-piperidin-4-yl}-acetic acid hydrochloride salt

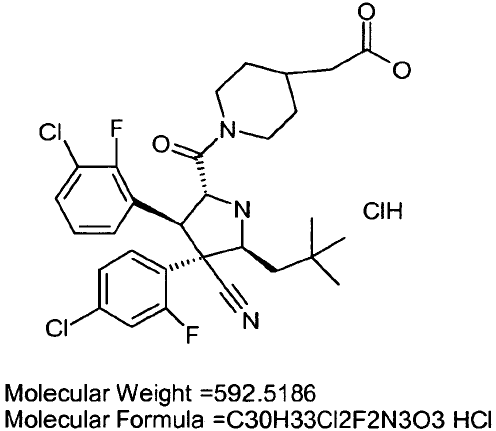

To a stirred solution ofrac {1-[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-piperidin-4-yl}-acetic acid methyl ester (556 mg, 0.92 mmol) in methanol (8 mL), LiOH.H₂O(Aldrich, 80 mg) in 1 mL of water was added. The mixture was stirred at 60°C overnight. The reaction was quenched with addition of water. The mixture was filtered and the filtrate was concentrated to removed methanol. The aqueous layer was washed with EtOAc (2x5 mL) and acidified with 3N HCl. The mixture was then extracted with EtOAc(3x6 mL). The extracts were combined and dried over sodium sulfate and concentrated to give a white solid. 649 mg.

HRMS (ES⁺) m/z Calcd: [(M+H)⁺]: 592.1940, found: 592.1939.

### Example 210

### Preparation of rac 2-{1-[(2R,3S,4R,5S)-3-(3-Chtoro-2-fluoro-phenyl)-4-(4-chloro-2-fluoro-phenyl)-4-cyano-5-(2,2-dimethyl-propyl)-pyrrolidine-2-carbonyl]-piperidin-4-yl}-acetamide

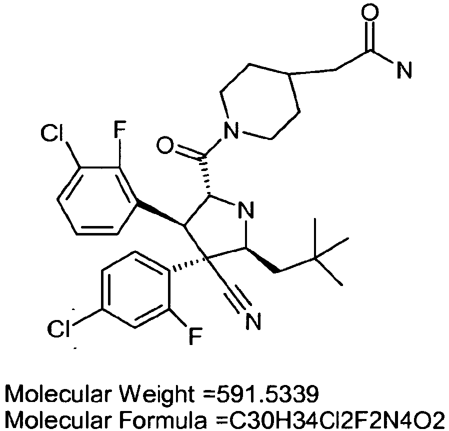

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (80 mg, 0.138 mmol) in methylene chloride (10 mL), HATU (Aldrich, 79 mg, 0.21 mmol) was added followed by the addition of DIPEA (0.145 mL, 0.83 mmol) and piperidin-4-yl-acetamide (Chembridge, 41 mg, 0.21 mmol). The mixture was stirred at rt for 3 hrs then overnight after the addition of 3 mL of DMF. The reaction was quenched with addition of water. The mixture was extracted with methylene chloride (2X10 mL) and the extracts were dried with magnesium sulfate. The solvent was removed and the residue was purified on an ISCO machine (12g column, eluent, 20% EtOAc/methylene chloride) to give a white solid. 21 mg.

HRMS (ES⁺) m/z Calcd: [(M+H)⁺]: 591.2100, found: 591.2100

### Example 211

### Preparation of rac 2-{1-[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-piperi din-4-yl}-N,N-bis-(2-hydroxy-ethyl)-acetamide

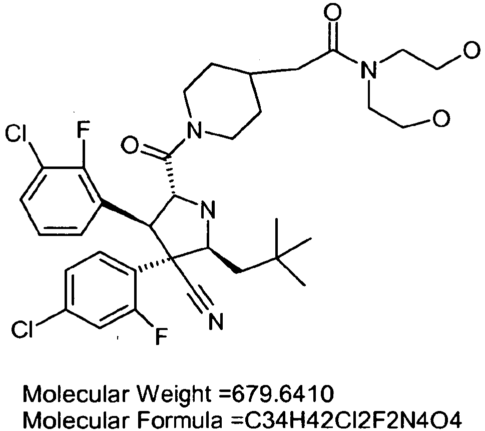

To a stirred solution of rac {1-[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-piperidin-4-yl} -acetic acid hydrochloride salt (80 mg, 0.127 mmol) in methylene chloride (5 mL), HATU (65 mg, 0.17 mmol) and DIPEA (80 uL, 0.339 mmol) were added followed by 2-(2-hydroxy-ethylamine)-ethanol (Across, 16 uL, 0.17 mmol) and the mixture was stirred at rt overnight. The reaction was quenched with water and the mixture was extracted with methylene chloride (3x10 mL) and the extracts were combined and dried over sodium sulfate. The solvent was removed and the residue was chromatographied on a reverse phase HPLC (acetonitrile/water, 25-85%) to give a white solid. 45 mg.

MS (ES⁺) m/z Calcd: [(M+H)⁺]: 679, found: 679.

### Example 212

### Preparation of rac 2-{1-[(2R,3S,4R,5S)-3-(3-Chloro-2-fluoro-phenyl)-4-(4-chloro 2-fluoro-phenyl)-4-cyano-5-(2,2-dimethyl-propyl)-pyrrolidine-2-carbonyl]-piperi din-4-yl}-N-(2-hydroxy-ethyl)-N-methyl-acetamide

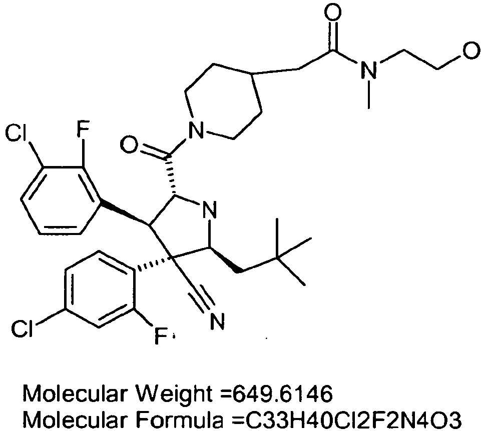

To a stirred solution ofrac {1-[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-piperidin-4-yl}-acetic acid hydrochloride salt (80 mg, 0.127 mmol) in methylene chloride (5 mL), HATU (77 mg, 0.28 mmol) and DIPEA (94 uL, 0.54mmol) were added followed by 2-hydroxy-ethyl-methyl amine (Oakwood, 20mg, 0.27 mmol) and the mixture was stirred at rt overnight. The reaction was quenched with water and the mixture was extracted with methylene chloride(3x10 mL) and the extracts were combined and dried over sodium sulfate. The solvent was removed and the residue was chromatographied on a reverse phase HPLC (acetonitrile/water, 25-85%) to give a white solid. 46 mg.

HRMS (ES⁺) m/z Calcd: [(M+H⁺)]: 649.2519, found: 645.2518

### Example 213

### Preparation of rac 2-{1-[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-piperi din-4-yl}-N-(2-hydroxy-propyl)-acetamide

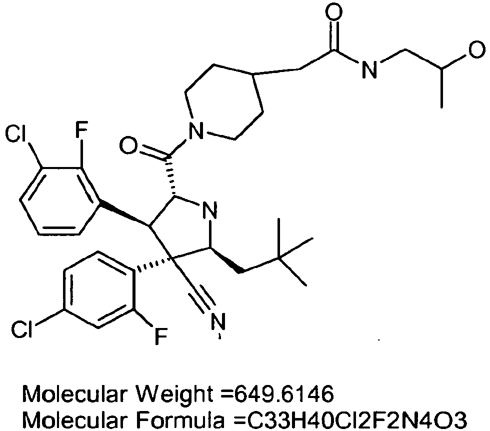

To a stirred solution ofrac {1-[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-piperidin-4-yl}-acetic acid hydrochloride salt (80 mg, 0.127 mmol) in methylene chloride (5 mL), HATU (77 mg, 0.28 mmol) and DIPEA (94 uL, 0.54mmol) were added followed by 2-hydroxy-2-methyl-ethylamine (Aldrich, 20mg, 0.27 mmol) and the mixture was stirred at rt for hers. The reaction was quenched with water and the mixture was extracted with methylene chloride(3x10 mL) and the extracts were combined and dried over sodium sulfate. The solvent was removed and the residue was chromatographied on a reverse phase HPLC (acetonitrile/water, 25-85%) to give a white solid. 48 mg.

HRMS (ES⁺) m/z Calcd: [(M+H)⁺]: 649.2519, found: 645.2519

### Example 214

### Preparation of rac {[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-acetic acid tert-butyl ester

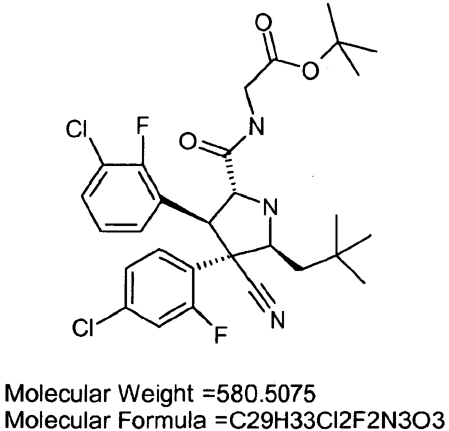

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (515 mg, 1.11 mmol) in methylene chloride (10 mL), HATU (Aldrich, 422 mg, 1.11 mmol) was added followed by the addition of DIPEA(0. 5 mL) and 2-amino-t-butyl-acetate (Aldrich, 145 mg, 1.11 mmol). The mixture was stirred at rt for 2 hrs. The reaction was quenched with addition of water. The mixture was extracted with methylene chloride (2X10 mL) and the extracts were dried with magnesium sulfate. The solvent was removed and the residue was purified on an ISCO machine (40g column, eluent, 30% EtOAc/hexanes chloride) to give a white solid. 510 mg.

HRMS (ES⁺) m/z Calcd: [(M+H)⁺]: 579.1867, found: 579.1866.

### Example 215

### Preparation of rac {[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-acetic aid trifluoro acetic acid salt.

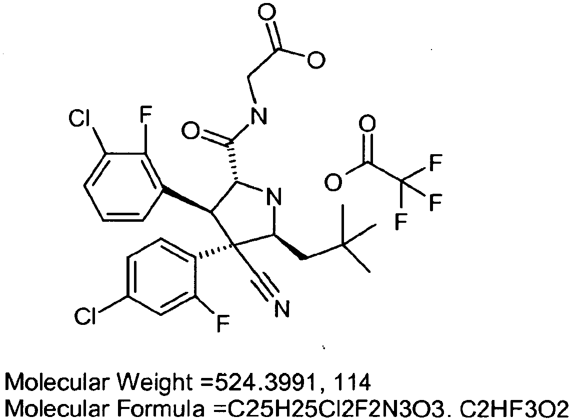

To a stirred solution of {[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino -acetic acid tert-butyl ester (500 mg) in methylene chloride (7 mL), TFA (3 mL) was added and the mixture was stirred at rt overnight. The solvent was removed under reduced pressure to give a white solid. 508 mg.

MS (ES⁺) m/z Calcd: [(M+H)⁺]: 524, found: 524.

### Example 216

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid carbamoylmethyl-amide

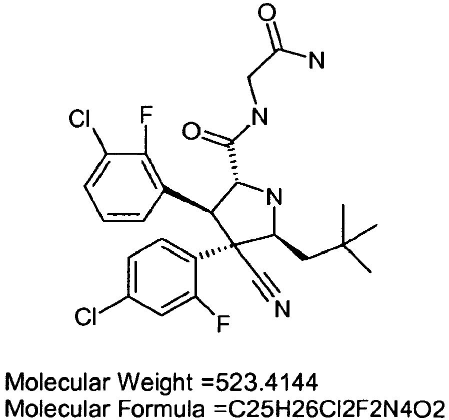

To a stirred solution ofrac {[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-acetic aid trifluoro acetic acid salt. (100 mg, 0.19 mmol) in methylene chloride (10 mL), HATU (Aldrich, 95 mg, 0.25 mmol) was added followed by the addition of DIPEA( 0. 5 mL) and 4N ammonia in methanol (0.2 mL). The mixture was stirred at rt for 2 hrs. The reaction was quenched with addition of water. The mixture was extracted with methylene chloride (3X7 mL) and the extracts were dried with magnesium sulfate. The solvent was removed and the residue was purified on an ISCO machine (40g column, eluent, 50% EtOAc/hexanes) to give a white solid. 34 mg.

MS (ES⁺) m/z Calcd: [(M+H)⁺]: 523, found: 523.

### Example 217

### Preparation of {[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-acetic aid trifluoro acetic acid salt.

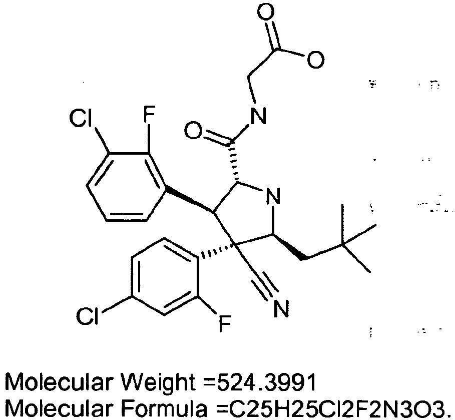

rac {[(2R,3S,4R,5S)-3-(3-Chloro-2-fluoro-phenyl)-4-(4-chloro-2-fluoro-phenyl)-4 cyano-5-(2,2-dimethyl-propyl)-pyrrolidine-2-carbonyl]-amino}-acetic aid trifluoro acetic acid salt was separate on a sfc machine (30% MeOH, 100 Bar, 30°C) give the desired enantiomer.

MS (ES⁺) m/z Calcd: [(M+H)⁺]: 524, found: 524.

### Example 218

### Preparation of rac 3-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid ethyl ester

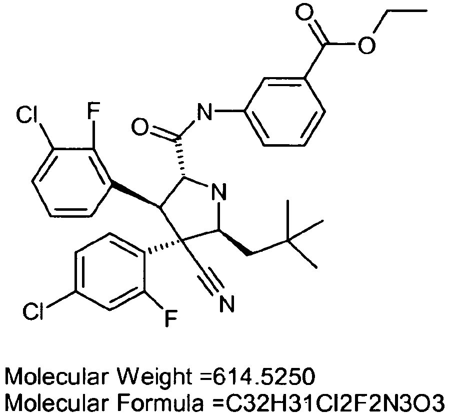

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (100 mg, 0.172 mmol) in methylene chloride (10 mL), HATU (Aldrich, 98 mg, 0.258 mmol) was added followed by the addition of DIPEA( 90 uL, 0.52 mmol) and 3-aminobenzoic acid ethyl ester (Aldrich, 43 mg, 0.26 mmol): The mixture was stirred at rt overnight. The reaction was quenched with addition of water. The mixture was extracted with methylene chloride (2X10 mL) and the extracts were dried with magnesium sulfate. The solvent was removed and the residue was purified on an ISCO machine (24g column, eluent, 10% EtOAc/methylene chloride) to give a white solid. 55 mg.

HRMS (ES⁺) m/z Calcd: [(M+H)⁺]: 614.1784, found: 614.1783

### Example 219

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (3-carbamoyl-phenyl)-amide

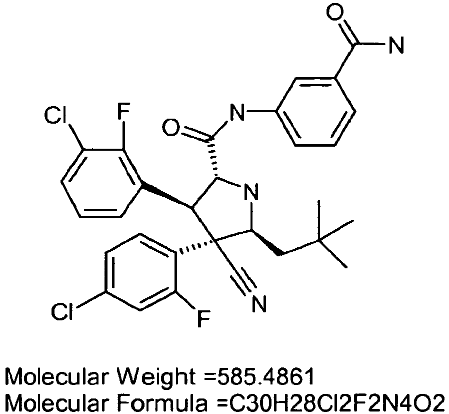

To a stirred solution of rac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (100 mg, 0.172 mmol) in methylene chloride (10 mL), HATU (Aldrich, 98 mg, 0.258 mmol) was added followed by the addition of DIPEA(90 uL, 0.52 mmol) and 3-amino benzamide (Oakwood, 35 mg, 0.26 mmol). The mixture was stirred at rt overnight. The reaction was quenched with addition of water. The mixture was extracted with methylene chloride (2X10 mL) and the extracts were dried with magnesium sulfate. The solvent was removed and the residue was purified on an ISCO machine (24g column, eluent, 30% EtOAc/methylene chloride) to give a white solid. 58 mg.

HRMS (ES⁺) m/z Calcd: [(M+H)⁺]: 585.1630, found: 585.1629

### Example 220

### Preparation of rac 3-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-b enzoic acid tert-butyl ester

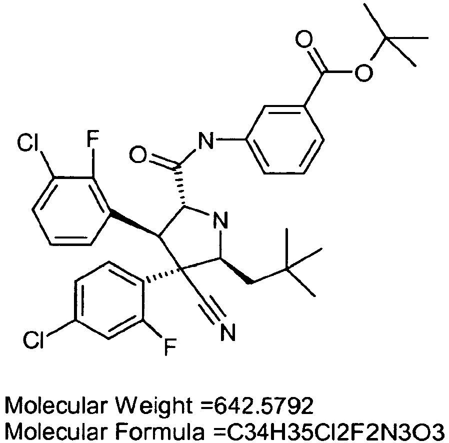

To a stirred solution of rac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (100 mg, 0.172 mmol) in methylene chloride (10 mL), HATU (Aldrich, 98 mg, 0.258 mmol) was added followed by the addition of D1PEA(90 uL, 0.52 mmol) and 3-amino benzoic acid t-butyl ester (Aldrich, 50 mg, 0.26 mmol). The mixture was stirred at rt overnight. The reaction was quenched with addition of water. The mixture was extracted with methylene chloride (2X10 mL) and the extracts were dried with magnesium sulfate. The solvent was removed and the residue was purified on an ISCO machine (24g column, eluent, 5% EtOAc/methylene chloride) to give a white solid. 80 mg.

HRMS (ES⁺) m/z Calcd: [(M+H)⁺]: 642.2097, found: 642.2101

### Example 221

### Preparation of rac 3-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid

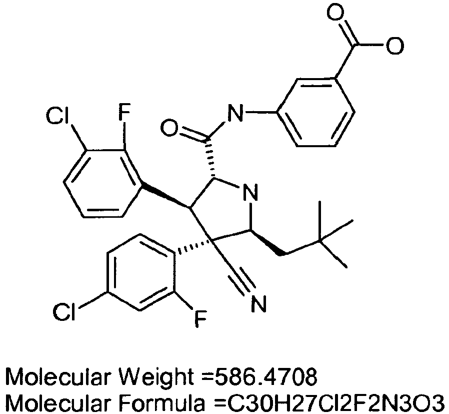

rac 3-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid t-butyl ester (20 mg, 0.031 mmol) was treated with 30 % TFA/methylene chloride (10 mL) overnight. Removal of solvent and freeze drying of the residue give a white powder. 12 mg.

HRMS (ES⁺) m/z Calcd: [(M+H)⁺]: 586.1471, found: 586.1467

### Example 222

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (3-hydroxymethyl-phenyl)-amide

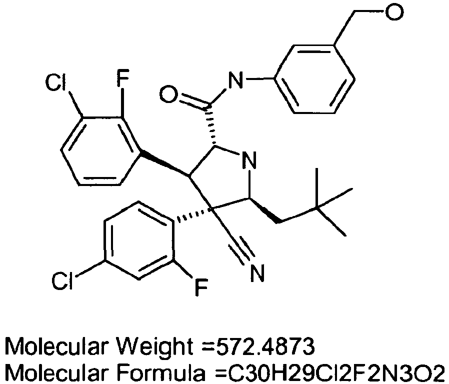

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (80 mg, 0.172 mmol) in methylene chloride (10 mL), HATU (Aldrich, 104 mg, 0.27 mmol) was added followed by the addition of DIPEA(80 uL,) and 3-amino benzyl alcohol (Aldrich, 80 uL). The mixture was stirred at rt overnight. The reaction was quenched with addition of water. The mixture was extracted with methylene chloride (2X10 mL) and the extracts were dried with magnesium sulfate. The solvent was removed and the residue was purified reverse phase HPLC (24g column, eluent, ACN/water, 20-90) to give a white solid. 40 mg.

HRMS (ES⁺) m/z Calcd: [(M+H)⁺]: 572.1678, found: 572.1679

### Example 223

### Preparation of rac (3-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino propyl)-carbamic acid tert-butyl ester

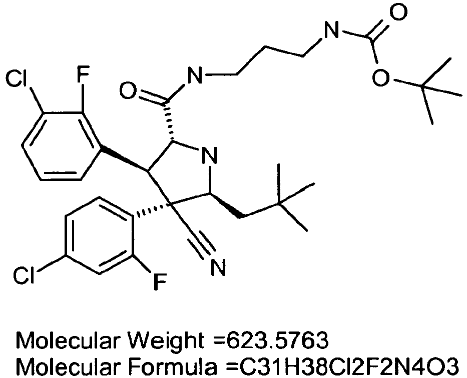

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (800 mg, 1.74 mmol) in methylene chloride (10 mL), HATU (Aldrich, 662 mg, 1.74 mmol) was added followed by the addition of DIPEA( 1.74 mmol) and (3-amino-propyl)-carbamic acid tert-butyl ester (Aldrich, 1.74 mmol). The mixture was stirred at rt for 1 hr. The reaction was quenched with addition of water. The mixture was extracted with methylene chloride (2X10 mL) and the extracts were dried with magnesium sulfate. The solvent was removed and the residue was purified on an ISCO machine (24g column, eluent, EtOAc/Hexanes, 20%) to give a white solid. 810 mg.

MS (ES⁺) m/z Calcd: [(M+H)⁺]: 623, found: 623

### Example 224

### Preparation of rac (2R,3S,4R,5S)-3-(3-Chloro-2-fluoro-phenyl)-4-(4-chloro-2-fluoro-phenl)-4-cyano-5-(2,2-dimethyl-propyl)-pyrrolidine-2-carboxylic acid (3-amino-propyl)-amide

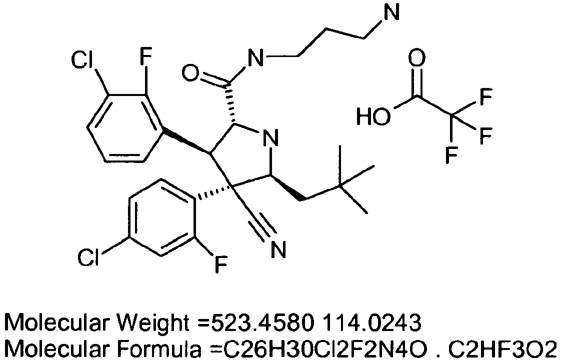

Rac (3-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-propyl)-carbamic acid tert-butyl ester (815 mg) was treated with 30 % TFA/methylene chloride (10 mL) overnight . Removal of solvent and freeze drying of the residue give a white powder. 800 mg.

MS (ES⁺) m/z Calcd: [(M+H)⁺]: 523, found: 523

### Example 225

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid [3-(aminosulfonyl-amino)-propyl)-amide

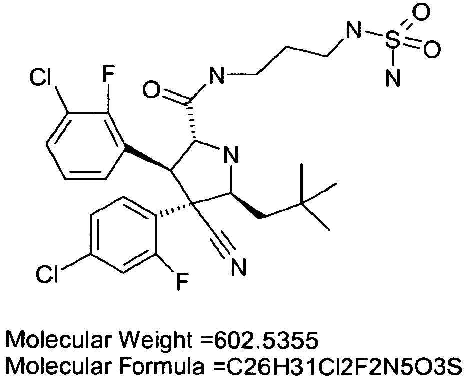

To a stirred solution of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (3-amino-propyl)-amide (100 mg) in DMF (3 mL), sulfamide (Aldrich, 62 mg) and potassium carbonate (50 mg) were added and the mixture was stirred at 100°C for 6 hrs. The solvent was removed under reduced pressure and the residue was portioned between EtOAc and water. The organic layer was separated and dried with sodium sulfate. The solvent was removed and the residue was chromatographied on an ISCO machine (70-100% EtOAc/ Hexanes) to give a white solid. 25 mg.

MS (ES⁺) m/z Calcd: [(M+H)⁺]: 602, found: 602

### Example 226

### Preparation of rac 2-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino -benzoic acid tert-butyl ester

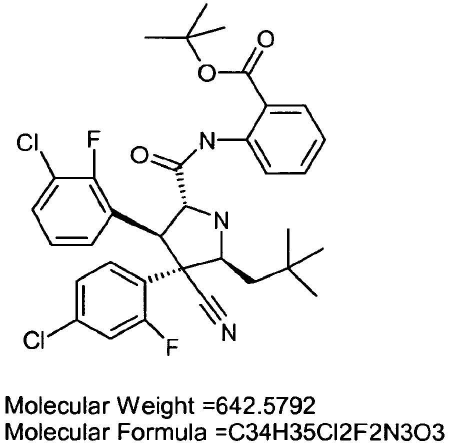

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (120 mg, 0.21 mmol) in methylene chloride (10 mL), HATU (Aldrich, 157 mg, 0.41 mmol) was added followed by the addition of DIPEA(110 uL, 0.62 mmol) and 2-amino benzoic acid t-butyl ester (Aldrich, 80 mg, 0.41 mmol). The mixture was stirred at rt overnight. The reaction was quenched with addition of water. The mixture was extracted with methylene chloride (2X10 mL) and the extracts were dried with magnesium sulfate. The solvent was removed and the residue was purified on an ISCO machine (40g column, eluent, 20% EtOAc/hexanes) to give a white solid. 71 mg.

HRMS (ES⁺) m/z Calcd: [(M+H)⁺]: 642.2100, found: 642.2009

### Example 227

### Preparation of rac4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid tert-butyl ester

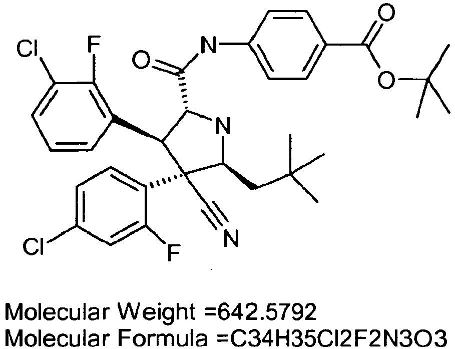

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (120 mg, 0.21 mmol) in methylene chloride (10 mL), HATU (Aldrich, 157 mg, 0.41 mmol) was added followed by the addition of DIPEA( 110 uL, 0.62 mmol) and 4-amino benzoic acid t-butyl ester (Aldrich, 80 mg, 0.41 mmol). The mixture was stirred at rt overnight. The reaction was quenched with addition of water. The mixture was extracted with methylene chloride (2X10 mL) and the extracts were dried with magnesium sulfate. The solvent was removed and the residue was purified on an ISCO machine (40g column, eluent, 20% EtOAc/hexanes) to give a white solid. 55 mg.

HRMS (ES⁺) m/z Calcd: [(M+H)⁺]: 642.2097, found: 642.2100

### Example 228

### Preparation of rac 4- {[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino)-benzoic acid ethyl ester

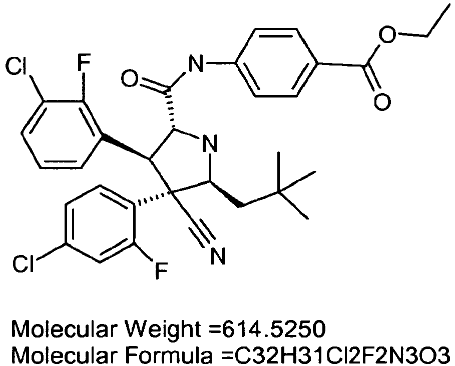

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (100 mg, 0.17 mmol) in methylene chloride (10 mL), HATU (Aldrich, 98 mg, 0.26 mmol) was added followed by the addition of DIPEA( 90 uL, 0.52 mmol) and 4-amino benzoic acid ethyl ester (43 mg, 0.26 mmol). The mixture was stirred at rt overnight. The reaction was quenched with addition of water. The mixture was extracted with methylene chloride (2X10 mL) and the extracts were dried with magnesium sulfate. The solvent was removed and the residue was purified on an ISCO machine (40g column, eluent, 20% EtOAc/hexanes) to give a white solid. 37 mg.

HRMS (ES⁺) m/x Calcd: [(M+H)⁺]: 614.784, found: 614.1786

### Examples 229

### Preparation of rac (2R,3S,4R,5S)3-(3-Chloro-2-fluoro-phenyl)-4-(4-chloro-2-fluoro-phenyl)-4-cyano-5-(2,2-dimethyl-propyl)-pyrrolidine-2-carboxylic acid (2-carbamoyl-phenyl)-amide

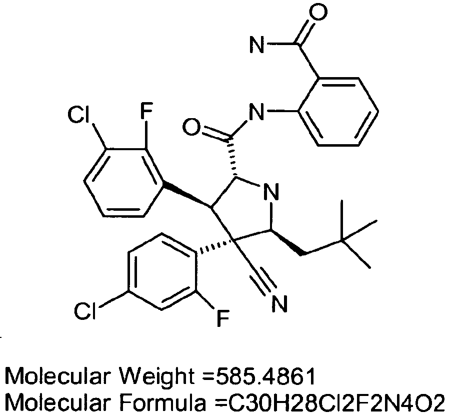

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (100 mg, 0.17 mmol) in methylene chloride (10 mL), HATU (Aldrich, 98 mg, 0.26 mmol) was added followed by the addition of DIPEA( 90 uL, 0.52 mmol) and 2-amino benzamide (Aldrich, 35 mg, 0.26 mmol). The mixture was stirred at rt overnight. The reaction was quenched with addition of water. The mixture was extracted with methylene chloride (2X10 mL) and the extracts were dried with magnesium sulfate. The solvent was removed and the residue was purified on an ISCO machine (40g column, eluent, 20% EtOAc/hexanes) to give a white solid. 30 mg.

HRMS (ES5⁺) m/z Calcd: [(M+H)⁺]: 585.1630, found: 585.1628

### Example 230

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (4-carbamoyl-phenyl)-amide

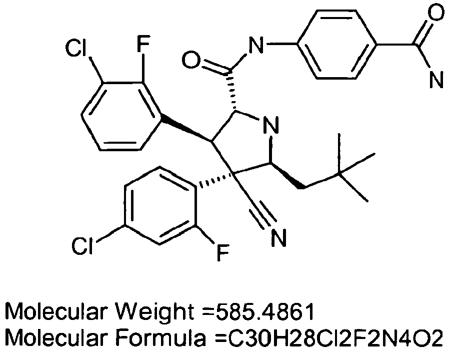

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (100 mg, 0.17 mmol) in methylene chloride (10 mL), HATU (Aldrich, 98 mg, 0.26 mmol) was added followed by the addition of DIPEA(90 uL, 0.52 mmol) and 4-amino benzamide (Aldrich, 35 mg, 0.26 mmol). The mixture was stirred at rt overnight. The reaction was quenched with addition of water. The mixture was extracted with methylene chloride (2X10 mL) and the extracts were dried with magnesium sulfate. The solvent was removed and the residue was purified on an ISCO machine (40g column, eluent, 20% EtOAc/hexanes) to give a white solid. 30 mg.

MS (ES⁺) m/z Calcd: [(M+H)⁺]: 585, found: 585

### Example 231

### Preparation of rac 2-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid ethyl ester

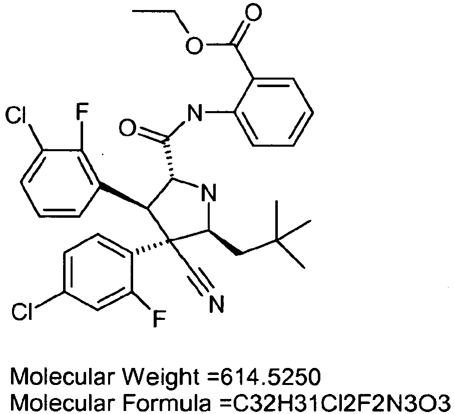

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (100 mg, 0.17 mmol) in methylene chloride (10 mL), HATU (Aldrich, 98 mg, 0.26 mmol) was added followed by the addition of DIPEA(90 uL, 0.52 mmol) and 2-amino benzoic acid ethyl ester (Aldrich, 43 mg, 0.26 mmol). The mixture was stirred at rt overnight. The reaction was quenched with addition of water. The mixture was extracted with methylene chloride (2X10 mL) and the extracts were dried with magnesium sulfate. The solvent was removed and the residue was purified on an ISCO machine (40g column, eluent, 20% EtOAc/hexanes) to give a white solid. 13 mg.

HRMS (ES⁺) m/z Calcd: [(M+H)⁺]: 614.1784, found: 614.1787

### Example 232

### Preparation of rac 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid

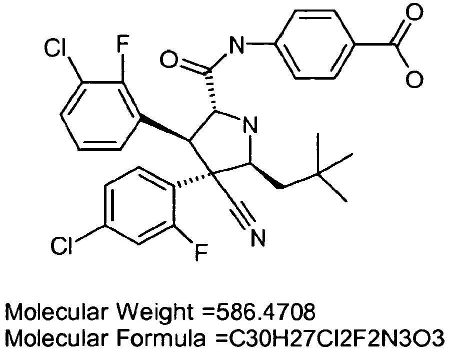

rac 4-([(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid t-butyl ester (35 mg, 0.031 mmol) was treated with 30 % TFA/methylene chloride (10 mL) overnight. Removal of solvent and freeze drying of the residue give a white powder. 31 mg.

HRMS (ES⁺) m/z Calcd: [(M+H)⁺]: 586.1471, found: 586.1470

### Example 233

### Preparation of rac 2-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid

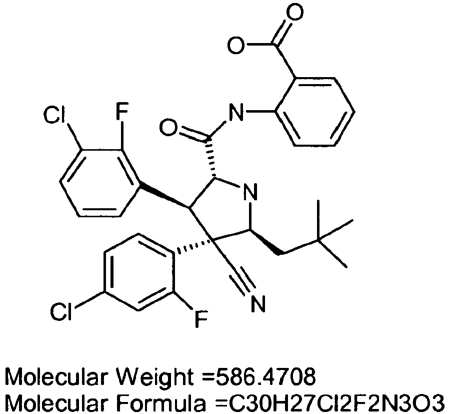

rac 2-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid t-butyl ester (50 mg, 0.031 mmol) was treated with 30 % TFA/methylene chloride (10 mL) overnight. Removal of so lvent and freeze drying of the residue give a white powder. 15 mg.

HRMS (ES⁺) m/z Calcd: [(M+H)⁺]: 516.1471, found: 586.1473

### Example 234

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (3-cyano-phenyl)-amide

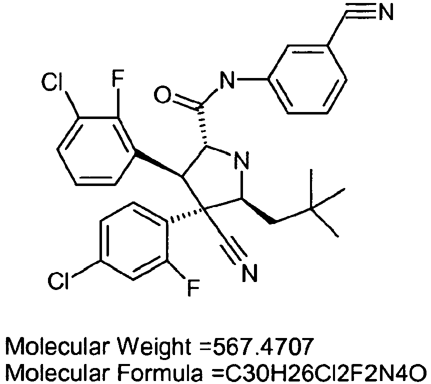

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (250 mg, 0.54 mmol) in methylene chloride (10 mL), HATU (Aldrich, 247 mg, 0.65 mmol) was added followed by the addition of DIPEA(0.65 mmol) and 3-amino benzonitrile(Aldrich, 77 mg, 0.65 mmol). The mixture was stirred at rt overnight. The reaction was quenched with addition of water. The mixture was extracted with methylene chloride (2X10 mL) and the extracts were dried with magnesium sulfate. The solvent was removed and the residue was purified on an ISCO machine (40g column, eluent, 35-50% EtOAc/hexanes) to give a white solid. 84 mg.

MS (ES⁺) m/z Calcd: [(M+H)⁺]: 567, found: 567

### Example 235

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (2-hydroxy-2-methyl-propyl)-amide

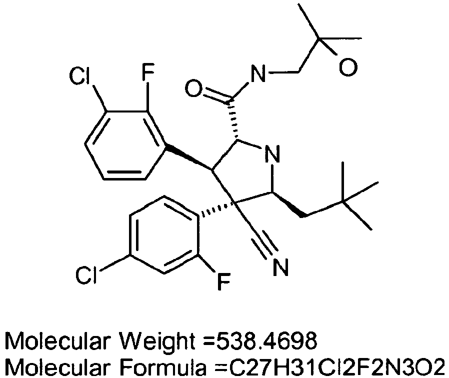

To a stirred solution of rac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidie-2-carboxylic acid (80 mg, 0.14 mmol) in methylene chloride (10 mL), HATU (Aldrich,105 mg, 0.28 mmol) was added followed by the addition of D1PEA(0.34 mmol) and 2-hydroxy-2-methyl-propylamine(Matrix, 25 mg, 028 mmol). The mixture was stirred at rt 1hr. The reaction was quenched with addition of water. The mixture was extracted with methylene chloride (2X10 mL) and the extracts were dried with magnesium sulfate. The solvent was removed and the residue was purified on an ISCO machine (40g column, eluent, 35-50% EtOAc/hexanes) to give a white solid. 50 mg.

MS (ES⁺) m/z Calcd: [(M+H)⁺]: 538.1834, found: 538.1834

### Example 236

### Preparation of rac 3- {[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid 2-hydroxy-2-methyl-propyl ester

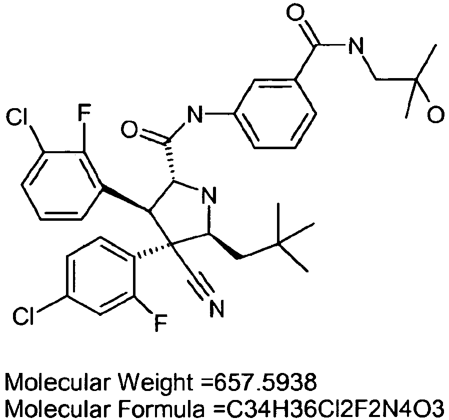

To a stirred solution rac 3-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid (80 mg, 0.14 mmol) in methylene chloride (10 mL), HATU (Aldrich,104 mg, 0.27 mmol) was added followed by the addition of DIPEA(0.41 mmol) and 2-hydroxy-2-methyl-propylamine(Matrix, 24 mg, 027 mmol). The mixture was stirred at rt 1hr. The reaction was quenched with addition of water. The mixture was extracted with methylene chloride (2X10 mL) and the extracts were dried with magnesium sulfate. The solvent was removed and the residue was purified on an ISCO machine (40g column, eluent, 35-50% EtOAc/hexanes) to give a white solid. 75 mg. MS (ES⁺) m/z Calcd: [(M+H)⁺]: 657.2206, found: 657.2208

### Example 237

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (3-methanesulfonylamino-phenyl)-amide

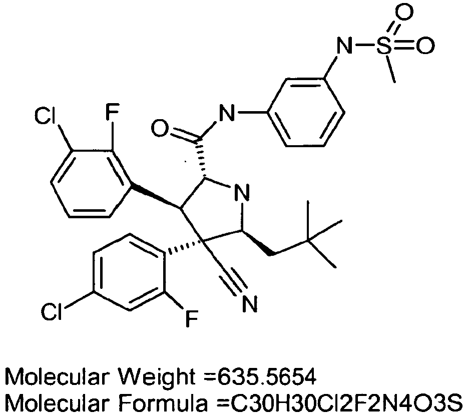

To a stirred solution of rac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (100 mg, 0.22 mmol) in methylene chloride (10 mL), HATU (Aldrich, 83 mg, 0.22 mmol) was added followed by the addition of DIPEA(0.20 mL) and 3-aminophenyl-methanamide(Aldrich, 0.25-mmol). The mixture was stirred at rt 1hr. The reaction was quenched with addition of water. The mixture was extracted with methylene chloride (2X10 mL) and the extracts were dried with magnesium sulfate. The solvent was removed and the residue was purified on an ISCO machine (40g column, eluent, 35-80% EtOAc/hexanes) to give a white solid. 64 mg.

MS (ES⁺) m/z Calcd: [(M+H)⁺]: 634, found: 634

### Example 238

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (1H-tetrazol-5-ylmethyl)-amide

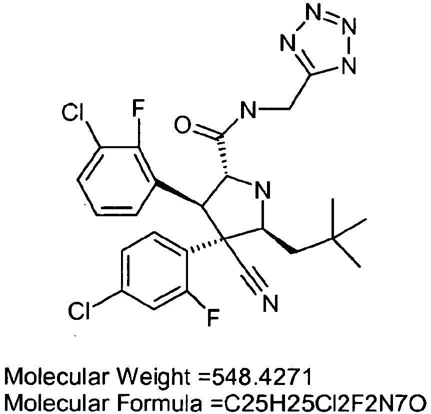

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (100 mg, 0.22 mmol) in methylene chloride (10 mL), HATU (Aldrich, 83 mg, 0.22 mmol) was added followed by the addition of DIPEA(0.20 mL) and 1H-tetrazol-5-yl-methylamine hydrobromide (Aldrich, 54 mg, 0.3 mmol). The mixture was stirred at rt 4.5hr. The reaction was quenched with addition of water. The mixture was extracted with methylene chloride (2X10 mL) and the extracts were dried with magnesium sulfate. The solvent was removed and the residue was purified on a reverse phase HPLC to give a white solid. 47 mg. MS (ES⁺) m/z Calcd: [(M+H)⁺]: 548, found: 548

### Example 239

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (3-ureado-propyl)-amide

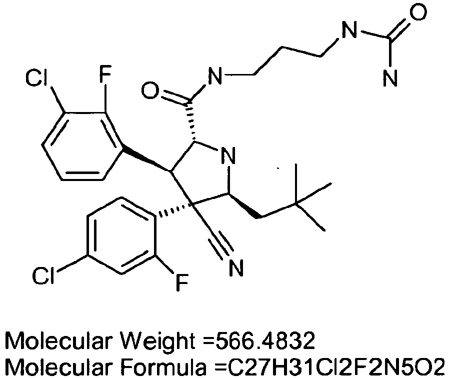

To a stirred solution of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (3-amino-propyl)-amide (100 mg) in DMF (3 mL), N-trimethylsilyl isocyanate (Aldrich, 0.13 mmol) and isopropylethylamine (Aldrich, 0.15 mL) were added and the mixture was stirred at rt for 2 hrs. The solvent was removed under reduced pressure and the residue was partioned between EtOAc and water. The organic layer was separated and dried with sodium sulfate. The solvent was removed and the residue was chromatographied on an ISCO machine (4-9% MeOH/EtOAc) to give a white solid. 52 mg.

MS (ES⁺) m/z Calcd: [(M+H)⁺]: 566, found: 566

### Example 240

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (3-methylsulfanyl-phenyl)-amide

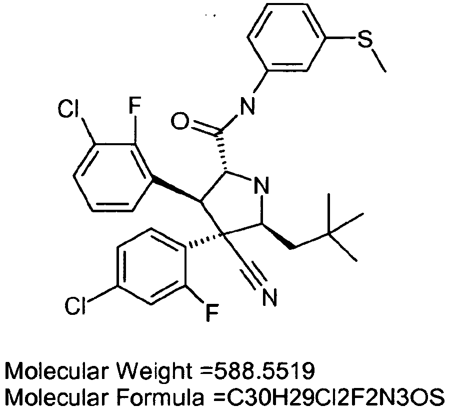

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (250 mg, 0.44 mmol) in methylene chloride (10 mL), HATU (Aldrich, 332 mg, 0.88 mmol) was added followed by the addition of DIPEA(0.230 mL, 1.31 mmol) and 3-amino-phenyl methyl sulfide (Aldrich, 122 mg, 0.88 mmol). The mixture was stirred at rt for 2 hr. The reaction was quenched with addition of water. The mixture was extracted with methylene chloride (2X10 mL) and the extracts were dried with magnesium sulfate. The solvent was removed and the residue was purified on an ISCO machine (50g column, eluent EtOAc/Hexanes, 5-10% )to give a white solid. 178 mg.

HRMS (ES⁺) m/z Calcd: [(M+H)⁺]_{:} 588.1449, found: 588.1450.

### Example 241

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (3-methanesulfonyl-phenyl)-amide

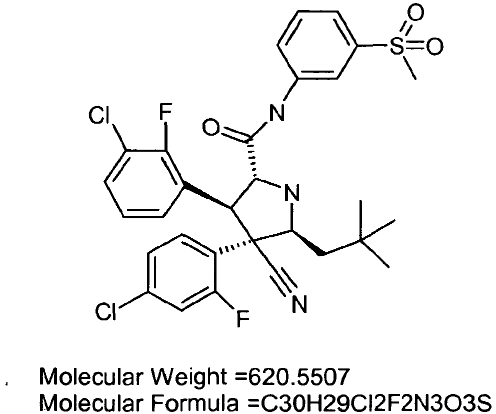

To a stirred solution of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (3-rnethylsulfanyl-phenyl)-amide(32 mg, 0.0544 mmol) in methylene chloride (5 mL) at rt, McPBA (Aldrich, 77%, 20.6 mg, 0.12 mmol) was added and the mixture was stirred for 2 hrs. The reaction was quenched with aqueous sodium thiosulfate and the layers were separated. The organic layer was washed with 10% sodium carbonate (10 mL) and dried with sodium sulfate. The solvent was removed and the residue was loaded onto a silica gel column (12g, 10% EtOAc/methylene chloride) to give a white solid. 28 mg.

HRMS (ES⁺) m/z Calcd: [(M+H)⁺]: 620.1348, found:620.1347

### Example 242

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (3-methanesulfinyl-phenyl)-amide

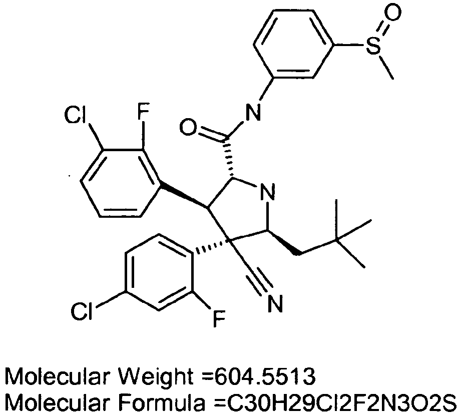

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (3-methylsulfanyl-phenyl)-amide(12 mg, 0.02mmol) in methylene chloride (5 mL) at rt, McPBA (Aldrich, 77%, 6 mg, 0.026 mmol) was added and the mixture was stirred for 1.5 hrs. The reaction was quenched with aqueous sodium thiosulfate and the layers were separated. The organic layer was washed with 10% sodium carbonate (10 mL) and dried with sodium sulfate. The solvent was removed and the residue was loaded onto a silica gel column (12g, 10% EtOAc/methylene chloride) to give a white solid. 6 mg.

HRMS (ES⁺) m/z (Calcd: [(M+H)], 604.1399, found: 604.13399 2 mg ofrac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (3-methanesulfonyl-phenyl)-amide was also obtained.

### Example 243

### Preparation of 3-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid

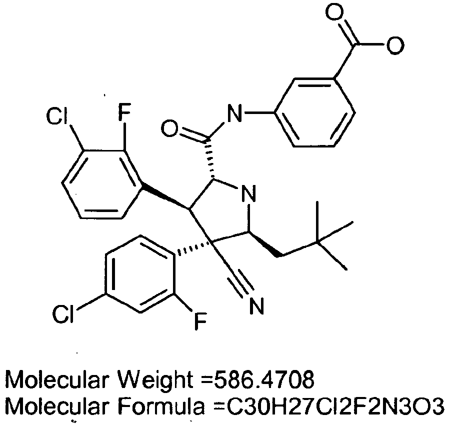

rac 3-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid t-butyl ester (26 mg, 0.411 mmol) was treated with 50 % TFA/methylene chloride (10 mL) overnight. Removal of solvent and freeze drying of the residue give a white powder. 250 mg. The solid was resolved on a Berger SFC system on a Whelk column under 100 bar, 30°C, and 45% of methanol to give two peaks: Peak 1, undesired enantiomer, 78 mg; Peak 2, 77 mg as white solid.

HRMS (ES⁺) m/z Calcd: [(M+H)⁺]: 586.1471, found: 586.1467

### Example 244

### Preparation of (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (3-carbamoyl-phenyl)-amide

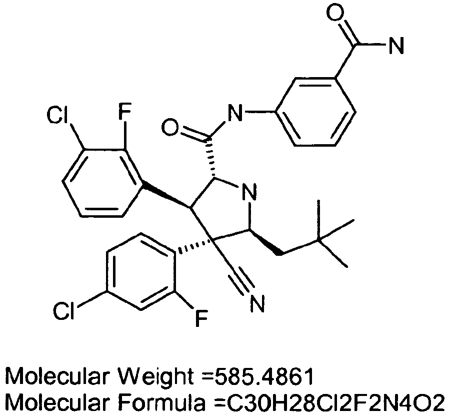

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (100 mg, 0.172 mmol) in methylene chloride (10 mL), HATU (Aldrich, 98 mg, 0.258 mmol) was added followed by the addition of DIPEA(90 uL, 0.52 mmol) and 3-amino benzamide (Oakwood, 35 mg, 0.26 mmol). The mixture was stirred at rt overnight. The reaction was quenched with addition of water. The mixture was extracted with methylene chloride (2X10 mL) and the extracts were dried with magnesium sulfate. The solvent was removed and the residue was purified on an ISCO machine (24g column, eluent, 30% EtOAc/methylene chloride) to give a white solid. 58 mg. The solid was resolved on a Berger SFC system on a Whelk column under 100 bar, 30°C, and 35% of methanol to give two peaks: Peak 1, undesired enantiomer, 19 mg; Peak 2, 21 mg as white solid.

HRMS (ES⁺) m/z (Calcd: [(M+H)⁺]: 585.1630. found: 583.1629

### Example 245

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid [3-(1H-tetrazol-5-yl)-phenyl]-amide

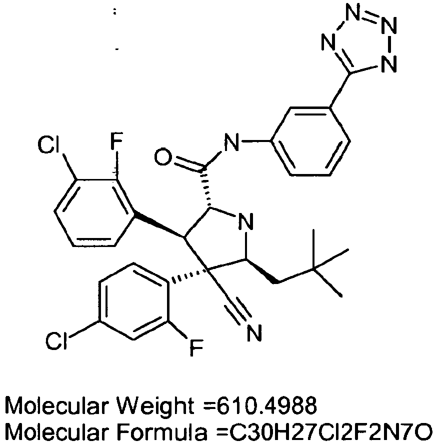

To a stirred solution of rac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (100 mg, 0.172 mmol) in methylene chloride (10 mL), HATU (Aldrich, 130 mg, 0.344 mmol) was added followed by the addition of DIPEA(90 uL, 0.52 mmol) and 3-(1H-tetrazol-5-yl)-phenylamine (Alfa, 55 mg, 0.344 mmol). The mixture was stirred at rt for 2 hrs. The reaction was quenched with addition of water. The mixture was extracted with methylene chloride (2X10 mL) and the extracts were dried with magnesium sulfate. The solvent was removed and the residue was purified on a reverse phase HPLC (24g column, eluent, 30% EtOAc/methylene chloride) to give a yellow solid. 21 mg.

HRMS (ES⁺) m/z Calcd: [(M+H)⁺]: 610.1695, found: 610.1698

### Example 246

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid [4-(1H-tetrazol-5-yl)-phenyl]-amide

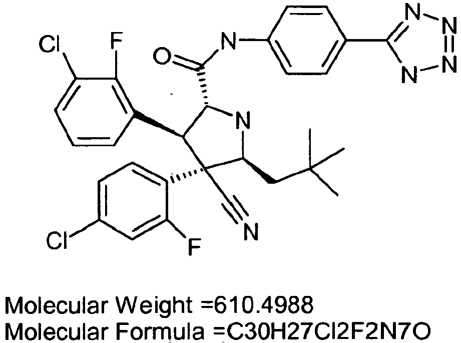

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (100 mg, 0.172 mmol) in methylene chloride (10 mL), HATU (Aldrich, 130 mg, 0.344 mmol) was added followed by the addition of DIPEA(90 uL, 0.52 mmol) and 4-(1H-tetrazol-5-yl)-phenylamine (Alfa, 55 mg, 0.344 mmol). The mixture was stirred at rt for 2 hrs. The reaction was quenched with addition of water. The mixture was extracted with methylene chloride (2X10 mL) and the extracts were dried with magnesium sulfate. The solvent was removed and the residue was purified on a reverse phase HPLC (24g column, eluent, 30% EtOAc/methylene chloride) to give a yellow solid. 21 mg.

HRMS (ES⁺) m/z Calcd: [(M+H)⁺]: 610.1695, found: 610.1696

### Example 247

### Preparation of rac (2R,3S,4R,5S)3-(3-Chloro-2-fluoro-phenyl)-4-(4-chloro-2-fluoro-phenyl)-4-cyano-5-(2,2-dimethyl-propyl)-pyrrolidine-2-carboxylic acid (4-amino-phenyl)-amide

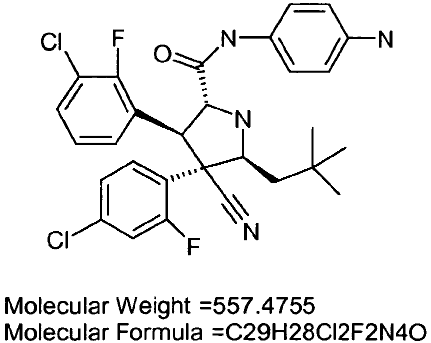

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (300 mg, 0.66 mmol) in methylene chloride (10 mL), HATU (Aldrich, 266 mg, 0.66 mmol) was added followed by the addition of DIPEA(0.30 mL) and 4-amino-aniline (Aldrich, 143 mg, 1.32 mmol). The mixture was stirred at rt for overnight. The reaction was quenched with addition of water. The mixture was extracted with methylene chloride (2X10 mL) and the extracts were dried with magnesium sulfate. The solvent was removed and the residue was purified on an ISCO machine (24g column, eluent, 40-85% EtOAc/hexanes) to give an off-white solid. 298 mg.

MS (ES⁺) m/z Calcd: [(M+H)⁺]: 557, found: 557

### Example 248

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (4-acetylamino-phenyl)-amide

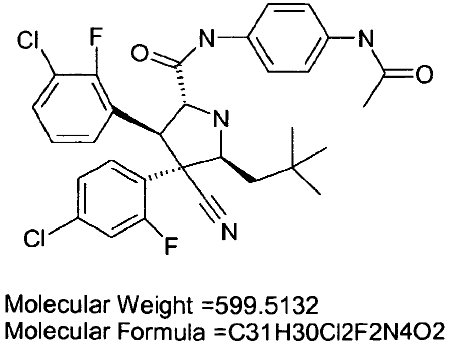

To a stirred solution of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (4-amino-phenyl)-amide (40 mg, 0.072 mmol) in methylene chloride (5 mL), acetic anhydride (0.072 mmol) was added at room temperature followed by triethylamine (0.10 mL). The mixture was stirred at for 1 hr. and then chromatographied on an ISCO machine (12g, 40-85% EtOAc/Hexanes) to give a white solid. 34 mg.

MS (ES⁺) m/z Calcd: [(M+H)⁺]: 599, found: 599

### Example 249

### Preparation of rac 2-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-thiazole-4-carboxylic acid ethyl ester

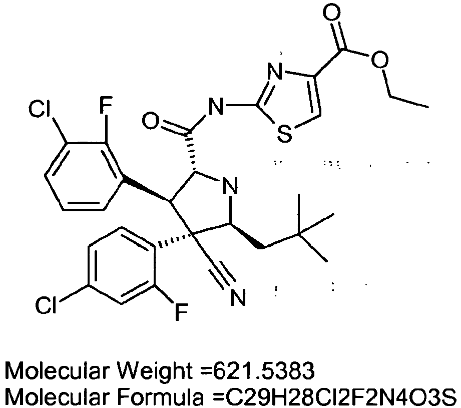

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (100 mg, 0.22 mmol) in methylene chloride (10 mL), HATU (Aldrich, 83 mg, 0.25 mmol) was added followed by the addition of DIPEA(100 uL) and 2-amino-thiazole-4-carboxylic acid ethyl ester (Oakwood, 43 mg, 0.25 mmol). The mixture was stirred at rt overnight. The reaction was quenched with addition of water. The mixture was extracted with methylene chloride (2X10 mL) and the extracts were dried with magnesium sulfate. The solvent was removed and the residue was purified on an ISCO machine (24g column, eluent, 40-80% EtOAc/hexanes) to give a solid. 32 mg.

MS (ES⁺) m/z Calcd: [(M+H)⁺]: 621, found: 621

### Example 250

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (1,3-dioxo-2,3-dihydro-]H-isoindol-5-yl)-amide

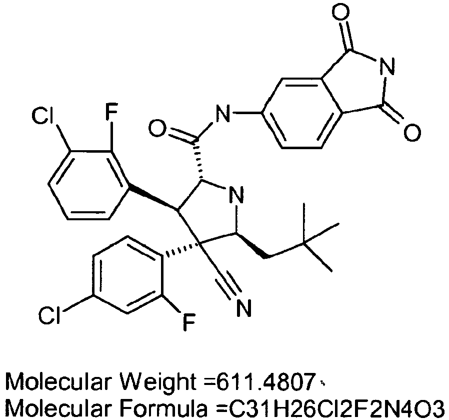

To a stirred solution ofthionyl chloride (2 mL), rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (110 mg, 0.24 mmol) was added and the mixture was stirred at rt for 2hrs. The excess thionyl chloride was removed and the residue was dissolved in methylene chloride (5 mL) and 5-amino-isoindole-1,3-dione( Aldrich, 0.30 mmol) was added followed by the addition of triethylamine (0.2 mL). The mixture was stirred overnight. The solvent was removed and the residue was purified on an ISCO machine (24g column, eluent, 40-80% EtOAc/hexanes) to give a pale yellow solid. 10 mg.

MS (ES⁺) m/z Calcd: [(M+H)⁺]: 611, found; 611

### Example 251

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (6-oxo-1,6-dihydro-pyridin-3-yl)-amide

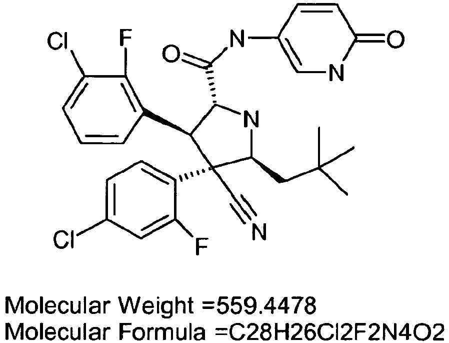

To a stirred solution of rac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (200 mg, 0.44 mmol) in methylene chloride (8 mL), HATU (Aldrich, 166 mg, 0.50 mmol) was added followed by the addition of DIPEA( 200 uL) and 5-amino-2-pyridinone (Alfa, 97 mg, 0.88 mmol). The mixture was stirred at rt overnight. The reaction was quenched with addition of water. The mixture was extracted with methylene chloride (2X10 mL) and the extracts were dried with magnesium sulfate. The solvent was removed and the residue was purified on an ISCO machine (24g column, eluent, 40-80% EtOAc/hexanes) to give a solid. 168 mg.

MS (ES⁺) m/z Calcd: [(M+H)⁺]: 559, found: 559

### Example 252

### Preparation of (2R,3S,4R,4S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (6-oxo-1,6-di^{h}ydro-pyridin-3-yl)-amide

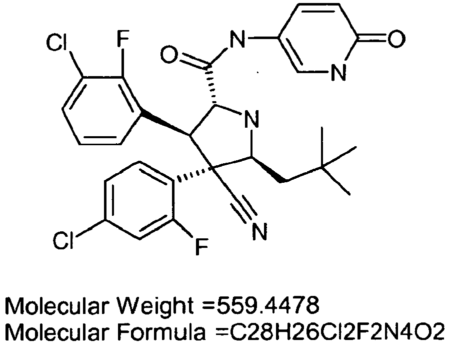

(?R,35,4R,55)-3-(3-Chloro-2-fluoro-phenyl)-4-(4-chloro-2-fluoro-phenyl)-4-cyano-5-(2,2-dimethyl-propyl)-pyrrolidine-2-carboxylic acid (6-oxo-1,6-dihydro-pyridin-3-yl)-amide(168 mg) was resolved on a Berger SFC machine under 100 bar, 30°C with 40 % of methanol at a rate of 2 mL/min give two separated peaks. Peak 1, 62 mg, peak 2, 64 mg (desired).

MS (ES+) m/z Calcd: [(M+H)⁺]: 559, found 559

### Example 253

### Preparation of rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-methylsulfanyl-phenyl)-amide

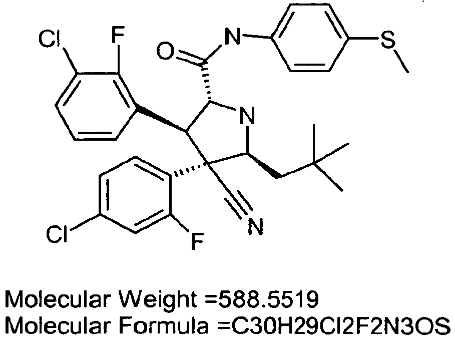

To a stirred solution ofrac (2R,3S,4R,SS)-3-(3-Chloro-2-fluoro-phenyl)-4-(4-chloro-2-fluoro-phenyl)-4-cyano-5-(2,2-dimethyl-propyl)-p yrrolidine-2-carboxylic acid (250 mg, 0.44 mmol) in methylene chloride (10 mL), HATU (Aldrich, 332 mg, 0.88 mmol) was added followed by the addition of DIPEA(0.230 mL, 1.31 mmol) and 4-amino-phenyl methyl sulfide (Aldrich, 122 mg, 0.88 mmol). The mixture was stirred at rt for 2 hr. The reaction was quenched with addition of water. The mixture was extracted with methylene chloride (2X10 mL) and the extracts were dried with magnesium sulfate. The solvent was removed and the residue was purified on an ISCO machine (50g column, eluent EtOAc/Hexanes, 5-10%) to give a white solid. 135 mg.

HRMS (ES⁺) m/z Calcd: [(M+H)⁺]: 588.1449, found: 588.1452.

### Example 254

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (4-methanesulfonyl-phenyl)-amide

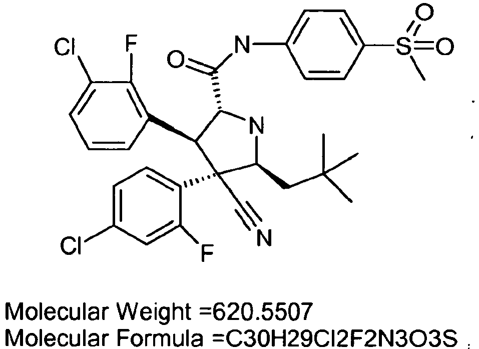

To a stirred solution ofrac (2R,3S.4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (3-methylsulfanyl-phenyl)-amide(41 mg, 0.085 mmol) in methylene chloride (5 mL) at rt, MCPBA (Aldrich, 77%, 32 mg, 0.187 mmol) was added and the mixture was stirred for 2 hrs. The reaction was quenched with aqueous sodium thiosulfate and the layers were separated. The organic layer was washed with 10% sodium carbonate (10 mL) and dried with sodium sulfate. The solvent was removed and the residue was loaded onto a silica gel column (12g, 10% EtOAc/methylene chloride) to give a white solid. 38 mg.

HRMS (ES⁺) m/z Calcd: 620.1348, found: 620.1348

### Example 255

### Preparation of 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid tert-butyl ester

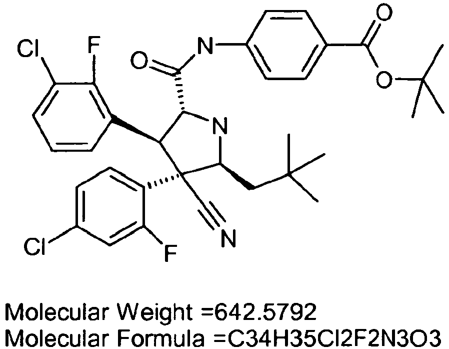

Racemic 4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid tert-butyl ester (670 mg) was resolved on a Berger SFC system under 30°C, 100 Bar, 20% MeOH on a O.D. column to give two peaks. Peak 1, desired, 267 mg, white solid; Peak 2, the other enantiomer, undesired, 267 mg, white solid;

HRMS (ES⁺) m/z Calcd: [(M+H)⁺]: 42, found: 642

### Example 256

### Preparation of 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid

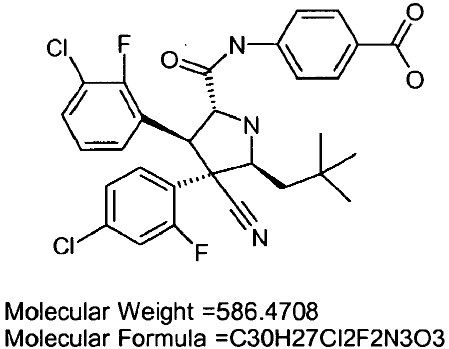

4- {[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid t-butyl ester (255 mg, 0.397 mmol) was treated with 50 % TFA/methylene chloride (10 mL) overnight. Removal of solvent and treating the residue with acetonitrile and water gave a white solid after filtration and drying. 236 mg.

HRMS (ES⁺) m/z CalCcd: [(M+H)⁺]: 586.1471, found: 586.1467

### Example 257

### Preparation of 4-{[(2S,3R,4S,5R)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino]-benzoic acid

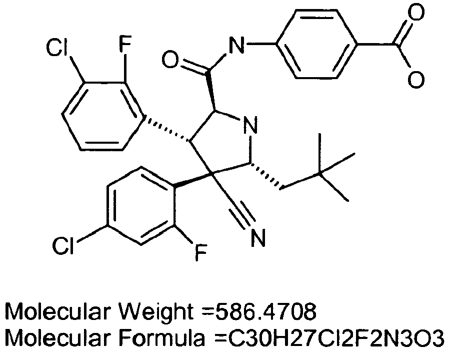

4-{[(2S,3R,4S,5R)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid t-butyl ester (made above, 255 mg, 0.397 mmol) was treated with 50 % TFA/methylene chloride (10 mL) overnight. Removal of solvent and treating the residue with acetonitrile and water gave a white solid after filtration and dying. 239 mg.

HRMS (ES⁺) m/z Calcd: [(M+H)⁺]: 586.1471, found: 586.1467

### Example 258

### Preparation of (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (4-carbamoyl-phenyl)-amide

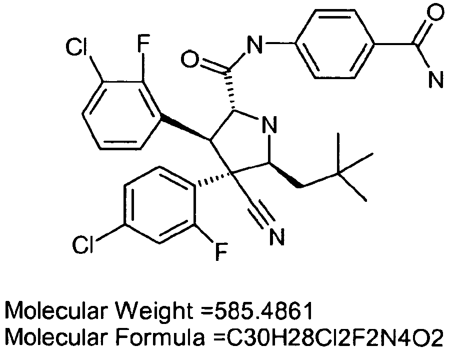

Rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (4-carbamoyl-phenyl)-amide(475 mg) was resolved on Berger SFC system under 30°C, 100 Bar, 35% MeOH on a O.D. Column to give two peaks. Peak 1, desired, 38 mg white solid; Peak 2, undesired, 38 mg white solid;

MS (ES⁺) m/z Calcd: [(M+H)⁺]: 585, found: 585

### Example 259

### Preparation of (25,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-carbamoyl-phenyl)-amide

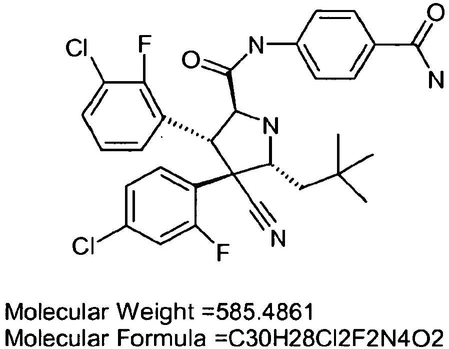

Rac (2S,3R,4S,5R)-3-(3-Chloro-2-fluoro-phenyl)-4-(4-chloro-2-fluoro-phenyl)-4-cyano-5-(2,2-dimethyl-propyl)-pyrrolidine-2-carboxylic acid (4-carbamoyl-phenyl)-amide(475 mg) was resolved on Berger SFC system under 30°C, 100 Bar, 35% MeOH on a O.D. Column to give two peaks. Peak 1, undesired, 38 mg white solid; Peak 2, desired, 38 mg white solid;

MS (ES⁺) m/z Calcd: [(M+H)⁺]: 585, found: 585

### Example 260

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (4-methanesulfonylamino-phenyl)-amide

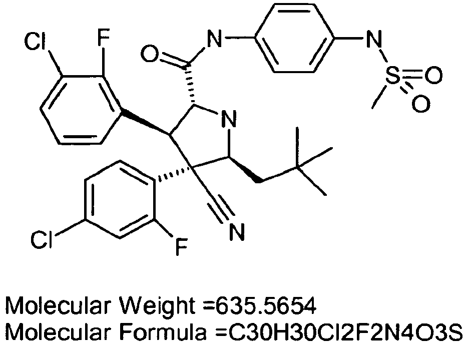

To a stirred solution of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (4-amino-phenyl)-amide (made above, 40 mg, 0.072 mmol) in methylene chloride (5 mL), methanesulfonic anhydride (Aldrich, 0.072 mmol) was added at room temperature followed by triethylamine (0.10 mL). The mixture was stirred at for 1 hr. and then chromatographied on an ISCO machine (12g, 40-85% EtOAc/Hexanes) to give a white solid. 31 mg.

MS (ES⁺) m/z Calcd: [(M+H)⁺]: 635, found: 635

### Example 261

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (4-trifluoro-methanesulfonylamino-phenyl)-amide

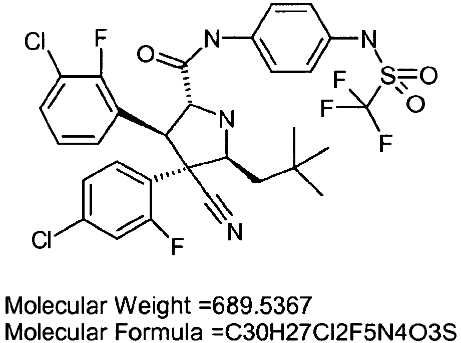

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (4-amino-phenyl)-amide (80 mg, 0.15 mmol) in methylene chloride (5 mL) at 0°C, trifluoromethanesulfonic anhydride (Aldrich, 0.15 mmol) was added at room temperature followed by triethylamine (0.10 mL). The mixture was stirred at for 1 hr. and then chromatographied on an ISCO machine (12g, 40-85% EtOAc/Hexanes) to give a white solid. 64 mg.

MS (ES⁺) m/z Calcd: [(M+H)⁺]: 689, found: 689

### Example 262

### Preparation of rac (2R,3R,4R,5S)-4-(4-Chloro-2-fluoro-phenyl)-3-(3-chloro-2-methyl-phenyl)-4-cyano-5-(2,2-dimethyl-propyl)-pyrrolidine-2-carboxylic acid [3-(1-methyl-1H-tetrazol-5-yl)-phenyl]-amide

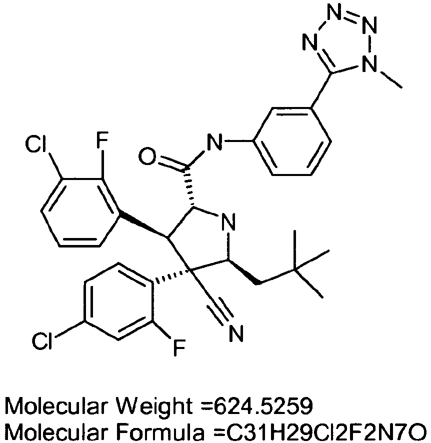

To a stirred solution of rac (2R,3R,4R,5S)-4-(4-Chloro-2-fluoro-phenyl)-3-(3-chloro-2-methylphenyl)-4-cyano-5-(2,2-dimethyl-propyl)-pyrrolidine-2-carboxylic acid [3-(1H-tetrazol-5-yl)-phenyl]-amide (44 mg, 0.172 mmol) in acetone(5 mL), sodium bicarbonate (84 mg, 1 mmol) and dimethyl sulfate (30 uL, 0.22 mmol) was added and the mixture was stirred at rt for 5 hrs. The solvent was removed and the residue was suspended in 3 mL of methylene chloride. The mixture was filtered and the filtrate was loaded on a silica gel column. Eluting with 35-70% EtOAc/Hexanes on an ISCO machine gave the desired product (12 mg) and the other regioisomer (26 mg).

MS (ES⁺) m/z Calcd: [(M+H)⁺]: 624, found: 624

### Example 263

### Preparation of rac (2R,3R,4R,5S)-4-(4-chloro-2-fluoro-phenyl)-3-(3-chloro-2-methyl-phenyl)-4-cyano-5-(2,2-dimethyl-propyl)-pyrrolidine-2-carboxylic acid [3-(2-methyl-1H-tetrazol-5-yl)-phenyl]-amide

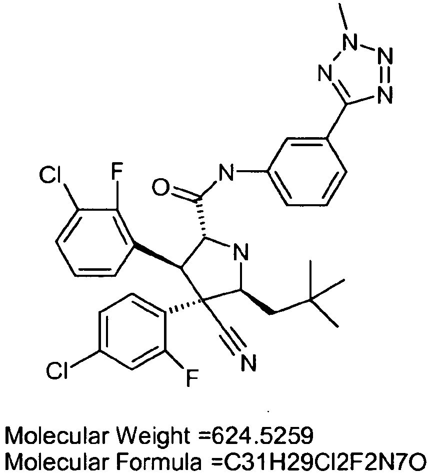

To a stirred solution of rac (2R,3R,4R,5S)-4-(4-Chloro-2-fluoro-phenyl)-3-(3-chloro-2-methylphenyl)-4-cyano-5-(2,2-dimethyl-propyl)-pyrrolidine-2-carboxylic acid [3-(1H-tetrazol-5-yl)-phenyl]-amide (44 mg, 0.172 mmol) in acetone(5 mL), sodium bicarbonate (84 mg, 1 mmol) and dimethyl sulfate (30 uL, 0.22 mmol) was added and the mixture was stirred at rt for 5 hrs. The solvent was removed and the residue was suspended in 3 mL of methylene chloride. The mixture was filtered and the filtrate was loaded on a silica gel column. Eluting with 35-70% EtOAc/Hexanes on an ISCO machine gave the desired product (26 mg) and the other regioisomer(12 mg).

MS (ES⁺) m/z Calcd: [(M+H)⁺]: 624, found: 624

### Example 264

### Preparation of (2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid (6-oxo-1,6-dihydro-pyridin-3-yl)-amide

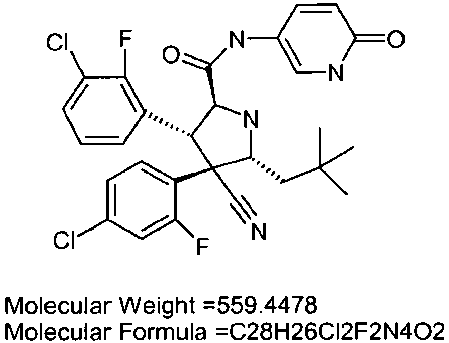

Rac (2R,35,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (6-oxo-1,6-dihydro-pyridin-3-yl)-amide(168 mg) was resolved on a Berger SFC machine under 100 bar, 30°C with 40 % of methanol at a rate of 2 mL/min give two separated peaks. Peak 1, 62 mg(desired), peak 2, 64 mg (undesired).

MS (ES⁺) m/z Calcd: [(M+H)⁺]: 559, found: 559

### Example 265

### Preparation of (2R,3S,4R,5S)-3-(3-Chloro-2-fluoro-phenyl)-4-(4-chloro-2 -fluoro-phenyl)-4-cyano-5-(2,2-dimethyl-propyl)-pyrrolidine-2-carboxylic acid [4-(1H-tetrazol-5-yl)-phenyl]-amide

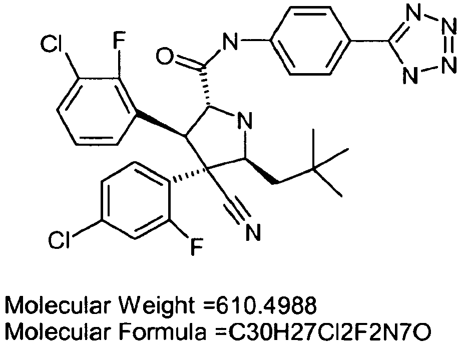

Rac (2R,35,4R,5S)-3-(3-Chloro-2-fluoro-phenyl)-4-(4-chloro-2 -fluoro-phenyl)-4-cyano-5-(2,2-dimethyl-propyl)-pyrrolidine-2-carboxylic acid [4-(1H-tetrazol-5-yl)-phenyl]-amide (522 mg)was resolved on a Berger SFC machine under 100 bar, 30°C with 35 % of methanol on an O.D. column gave two separated peaks. Peak 1, 186 mg (desired), peak 2, 185 mg (undesired). MS (ES⁺) m/z Calcd: [(M+H)⁺]: 610, found: 610

### Example 266

### Preparation of (25,3R,4S,5R)-3-(3-chloro-2-fluoro-phenyl)-4-(4-chloro-2 -fluoro-phenyl)-4-cyano-5-(2,2-dimethyl-propyl)-pyrrolidine-2-carboxylic acid [4-(1H-tetrazol-5-yl)-phenyl]-amide

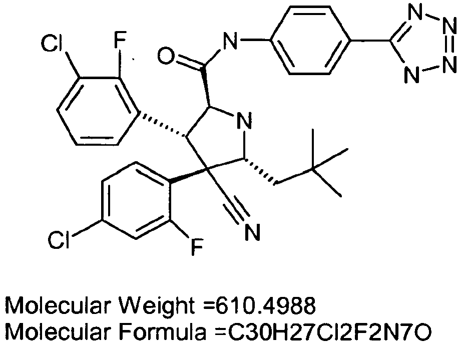

Rac (2R,3S,4R,5S)-3-(3-Chloro-2-fluoro-phenyl)-4-(4-chloro-2 -fluoro-phenyl)-4-cyano-5-(2,2-dimethyl-propyl)-pyrrolidine-2-carboxylic acid [4-(1H-tetrazol-5-yl)-phenyl]-amide (522 mg)was resolved on a Berger SFC machine under 1000 bar, 30°C with 35 % of methanol on an O.D. column gave two separated peaks. Peak 1, 186 mg(undesired), peak 2, 185 mg (desired). MS (ES⁺) m/z Calcd: [(M+H)⁺]: 610, found: 610

### Example 267

### Preparation of rac (2R,3R,4R,5S)-4-(4-Chloro-2-fluoro-phenyl)-3-(3-chloro-2-methyl-phenyl)-4-cyano-5-(2,2-dimethyl-propyl)-pyrrolidine-2-carboxylic acid [4-(2-methyl-1H-tetrazol-5-yl)-phenyl]-amide

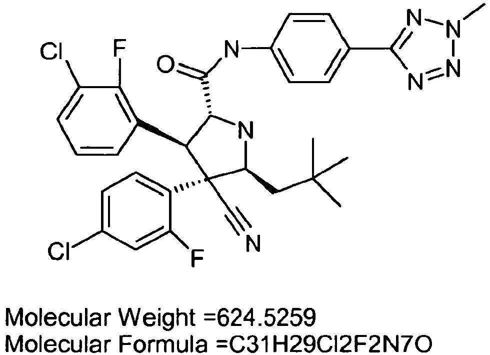

To a stirred solution ofrac (2R,3R,4R,5S)-4-(4-Chloro-2-fluoro-phenyl)-3-(3-chloro-2-methylphenyl)-4-cyano-5-(2,2-dimethyl-propyl)-pyrrolidine-2-carboxylic acid [4-(1H-tetrazol-5-yl)-phenyl]-amide (32 mg, 0.0524 mmol) in acetone(5 mL), sodium bicarbonate (45 mg, 0.6 mmol)) and dimethyl sulfate(0.11 mmol) was added and the mixture was stirred at rt for 5 hrs. The solvent was removed and the residue was suspended in 3 mL of methylene chloride. The mixture was filtered and the filtrate was loaded on a silica gel column. Eluting with 35-70% EtOAc/Hexanes on an ISCO machine gave the desired product (18 mg) and the other regioisomer (3 mg).

MS (ES⁺) m/z Calcd: [(M+H)⁺]: 624, found: 624

### Example 268

### Preparation of rac (2R,3R,4R,5S)-4-(4-chloro-2-fluoro-phenyl)-3-(3-chloro-2-methyl-phenyl)-4-cyano-5-(2,2-dimethyl-propyl)-pyrrolidine-2-carboxylic acid [4-(1-methyl-1H-tetrazol-5-yl)-phenyl]-amide

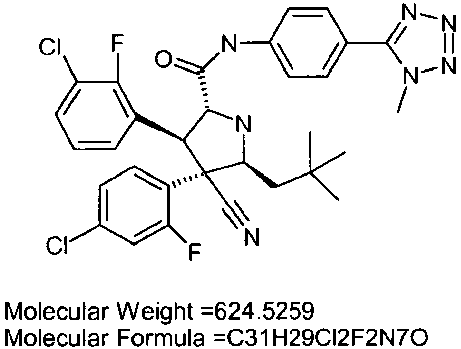

To a stirred solution of rac (2R,3R,4R,5S)-4-(4-Chloro-2-fluoro-phenyl)-3-(3-chloro-2-methylphenyl)-4-cyano-5-(2,2-dimethyl-propyl)-pyrrolidine-2-carboxylic acid [4-(1H-tetrazol-5-yl)-phenyl]-amide (32 mg, 0.0524 mmol) in acetone(5 mL), sodium bicarbonate (45 mg, 0.6 mmol) and dimethyl sulfate(0.11 mmol) was added and the mixture was stirred at rt for 5 hrs. The solvent was removed and the residue was suspended in 3 mL of methylene chloride. The mixture was filtered and the filtrate was loaded on a silica gel column. Eluting with 35-70% EtOAc/Hexanes on an ISCO machine gave the desired product (3 mg) and the other regioisomer (18 mg).

MS (ES⁺) m/z Calcd: [(M+H)⁺]: 624, found: 624

### Example 269

### Preparation of rac 5-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-2-fluoro-benzoic acid ethyl ester

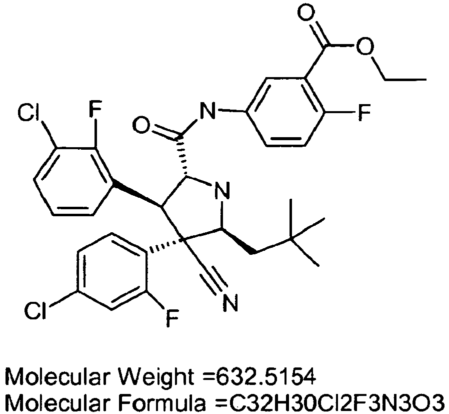

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (100 mg, 0.17 mmol) in methylene chloride (10 mL), HATU (Aldrich, 98 mg, 0.26 mmol) was added followed by the addition of DIPEA(0.150 mL, 0.855 mmol) and 5-amino-2-fluoro-benzoic acid ethyl ester (Oakwood, 63 mg, 0.34 mmol). The mixture was stirred at rt for 1.5 hr. The reaction was quenched with addition of water. The mixture was extracted with methylene chloride (2X10 mL) and the extracts were dried with magnesium sulfate. The solvent was removed and the residue was purified on an ISCO machine (50g column, eluent EtOAc/Hexanes, 5-20%) to give a white solid. 64 mg.

MS (ES⁺) m/z Calcd: [(M+H)⁺]: 632, found: 632

### Example 270

### Preparation of (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid [3-(1H-tetrazol-5-yl)-phenyl]-amide

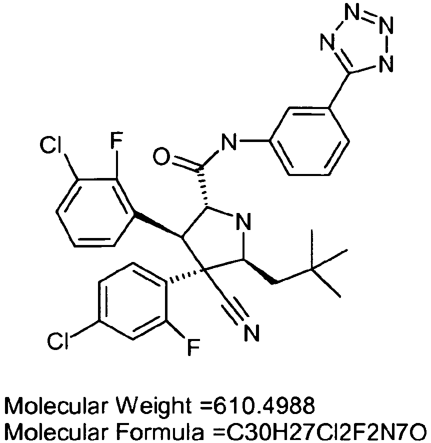

Rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid [3-(1H-tetrazol-5-yl)-phenyl]-amide (690 mg)was resolved on a Berger SFC machine under 100 bar, 30°C with 10 % of methanol on an O.D. column gave two separated peaks. Peak 1, 256 mg (desired), peak 2, 186 mg (undesired). MS (ES⁺) m/z Calcd: [(M+H)⁺]: 610, found: 610

### Example 271

### Preparation of (2S,3R,4S,5R)-3-(3-Chloro-2-fluoro-phenyl)-4-(4-chloro-2 -fluoro-phenyl)-4-cyano-5-(2,2-dimethyl-propyl)-pyrrolidine-2-carboxylic acid [3-(1H-tetrazol-5-yl)-phenyl]-amide

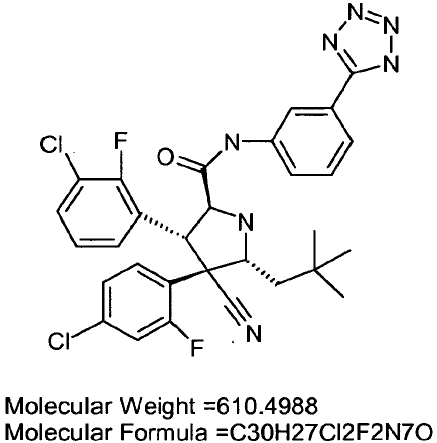

Rac (2R,3S,4R,5S)-3-(3-Chloro-2-fluoro-phenyl)-4-(4-chloro-2-fluoro-phenyl)-4-cyano-5-(2,2-dimethyl-propyl)-pynrolidine-2-carboxylic acid [3-(1H-tetrazol-5-yl)-phenyl]-amide (690 mg)was resolved on a Berger SFC machine under 100 bar, 30°C with 10 % of methanol on an O.D. column gave two separated peaks. Peak 1,256 mg(undesired), peak 2, 186 mg (desired). MS (ES⁺) m/z Calcd: [(M+H)⁺]: 610, found: 610

### Example 272

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (4-carbamoyl-3-chloro-phenyl)-amide

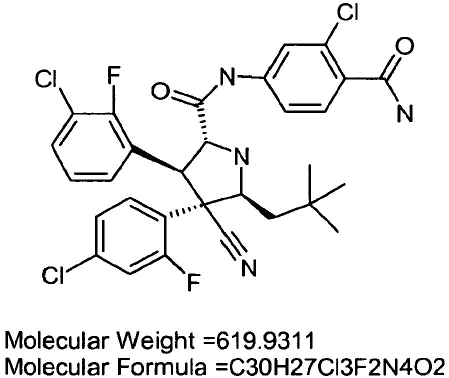

To a stirred solution of rac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (100 mg, 0.17 mmol) in methylene chloride (10 mL), HATU (Aldrich, 98 mg, 0.26 mmol) was added followed by the addition of DIPEA(0.150 mL, 0.855 mmol) and 4-amino-2-chloro -benzamide(Chembridge, 58 mg, 0.34 mmol). The mixture was stirred at rt for 1.5 hr. The reaction was quenched with addition of water. The mixture was extracted with methylene chloride (2X10 mL) and the extracts were dried with magnesium sulfate. The solvent was removed and the residue was purified on a reverse phase HPLC to give a white solid. 6 mg.

MS (ES⁺) m/z Calcd: [(M+H)⁺]: 619, found: 619

### Example 273

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid [3-chloro-4-(1H-tetrazol-5-yl)-phenyl]-amide

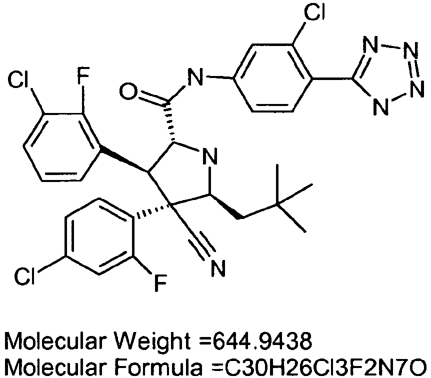

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (100 mg, 0.17 mmol) in methylene chloride (10 mL), HATU (Aldrich, 98 mg, 0.26 mmol) was added followed by the addition of DIPEA(0.150 mL, 0.855 mmol) and 3-chloro-4-(1H-tetrazol-5-yl) aniline(made below, 67 mg, 0.34 mmol). The mixture was stirred at rt for 1.5 hr. The reaction was quenched with addition of water. The mixture was extracted with methylene chloride (2X10 mL) and the extracts were dried with magnesium sulfate. The solvent was removed and the residue was purified on a reverse phase HPLC to give a white solid. 52 mg.

MS (ES⁺) m/z Calcd: [(M+H)⁺]: 644, found: 644

### Example 274

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (4-fluoro-phenyl)-arnide

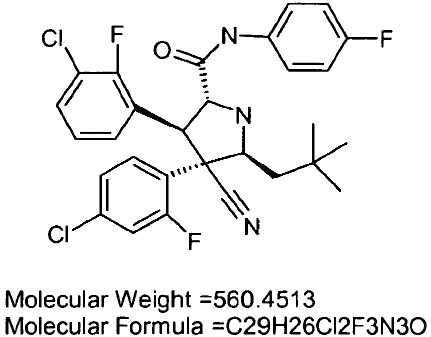

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (100 mg, 0.17 mmol) in methylene chloride (10 mL), HATU (Aldrich, 98 mg, 0.26 mmol) was added followed by the addition of DIPEA(0.150 mL, 0.855 mmol) and 4-fluoro-aniline(Aldrich, 38 mg, 0.32 mmol). The mixture was stirred at rt for 1.5 hr. The reaction was quenched with addition of water. The mixture was extracted with methylene chloride (2X10 mL) and the extracts were dried with magnesium sulfate. The solvent was removed and the residue was purified on an ISCO machine (20 g column, 0-10% EtOAc/methylene chloride) to give a white solid. 67 mg.

MS (ES⁺) m/z Calcd: [(M+H)⁺]: 560, found: 560

### Example 275

### Preparation of rac (2R,35,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (3-fluoro-phenyl)-amide

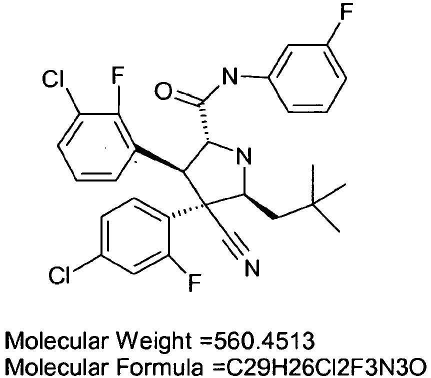

To a stirred solution of rac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (100 mg, 0.17 mmol) in methylene chloride (10 mL), HATU (Aldrich, 98 mg, 0.26 mmol) was added followed by the addition of DIPEA(0.1 150 mL, 0.855 mmol) and 3-fluoro-aniline(Aldrich, 38 mg, 0.32 mmol). The mixture was stirred at rt for 1.5 hr. The reaction was quenched with addition of water. The mixture was extracted with methylene chloride (2X10 mL) and the extracts were dried with magnesium sulfate. The solvent was removed and the residue was purified on an ISCO machine (20 g column, 0-10% EtOAc/methylene chloride) to give a white solid. 60 mg.

MS (ES⁺) m/z Calcd: [(M+H)⁺]: 560, found: 560

### Example 276

### Preparation of rac (2R,35,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (3-chloro-phenyl)-amide

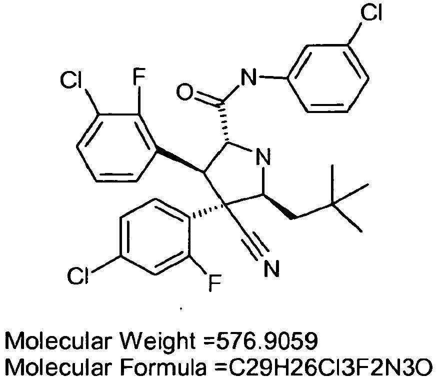

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (100 mg, 0.17 mmol) in methylene chloride (10 mL), HATU (Aldrich, 98 mg, 0.26 mmol) was added followed by the addition of DIPEA(0.150 mL, 0.855 mmol) and 3-chloro-aniline(Aldrich, 44 mg, 0.32 mmol). The mixture was stirred at rt for 1.5 hr. The reaction was quenched with addition of water. The mixture was extracted with methylene chloride (2X10 mL) and the extracts were dried with magnesium sulfate. The solvent was removed and the residue was purified on an ISCO machine (20 g column, 0-10% EtOAc/methylene chloride) to give a white solid. 48 mg.

MS (ES⁺) m/z Calcd: [(M+H)⁺]: 576, found: 576

### Example 277

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (4-chloro-phenyl)-amide

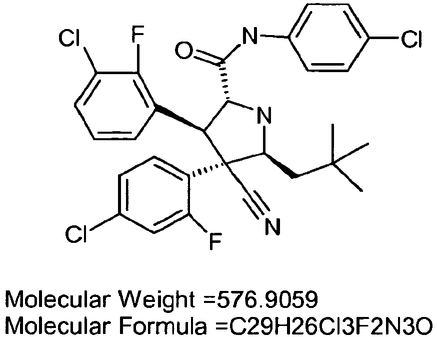

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (100 mg, 0.17 mmol) in methylene chloride (10 mL), HATU (Aldrich, 98 mg, 0.26 mmol) was added followed by the addition of DIPEA(0.150 mL, 0.855 mmol) and 4-chloro-aniline(Aldrich, 44 mg, 0.32 mmol). The mixture was stirred at rt for 1.5 hr. The reaction was quenched with addition of water. The mixture was extracted with methylene chloride (2X10 mL) and the extracts were dried with magnesium sulfate. The solvent was removed and the residue was purified on an ISCO machine (20 g column, 0-10% EtOAc/methylene chloride) to give a white solid. 46 mg.

MS (ES⁺) m/z Calcd: [(M+H)⁺]: 576, found: 576

### Example 278

### Preparation of rac 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-2-fluoro-benzoic acid tert-butyl ester

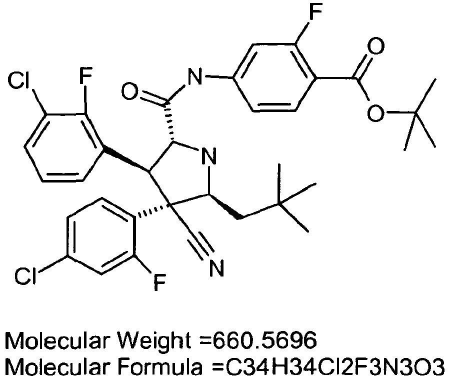

To a stirred solution of rac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (150 mg, 0.26 mmol) in methylene chloride (10 mL), HATU (Aldrich),147 mg, 0.39 mmol) was added followed by the addition of DIPEA(0.225 mL, 1.29 mmol) and 4-amino-2-flouro-benzoic acid t-butyl ester(Aldrich , 44 mg, 0.32 mmol). The mixture was stirred at rt for 5.5 hr. The reaction was quenched with addition of water. The mixture was extracted with methylene chloride (2X10 mL) and the extracts were dried with magnesium sulfate. The solvent was removed and the residue was purified on an ISCO machine (40 g column, 0-15% EtOAc/hexanes) to give a white solid. 19 mg.

MS (ES⁺) m/z Calcd: [M+H)⁺]: 660, found: 660

### Example 279

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (4-ethylcarbamoyl-3-fluoro-phenyl)-amide

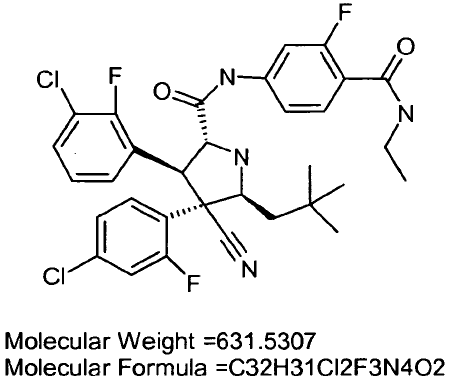

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (150 mg, 0.26 mmol) in methylene chloride (10 mL), HATU (Aldrich),147 mg, 0.39 mmol) was added followed by the addition of DIPEA(0.225 mL, 1.29 mmol) and 4-amino-2-flouro-.ethyl-benzamide(made by reducing the corresponding nitro precursor, 56mg, 0.34 mmol). The mixture was stirred at rt for overnight. The reaction was quenched with addition of water. The mixture was extracted with methylene chloride (2X10 mL) and the extracts were dried with magnesium sulfate. The solvent was removed and the residue was purified on an ISCO machine (40 g column, 0-15% EtOAc/hexanes) to give a white solid. 42 mg.

MS (ES⁺) m/z Calcd: [(M+H)⁺]: 631, found: 631

### Example 280

### Preparation of rac 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-2-fluoro-benzoic acid

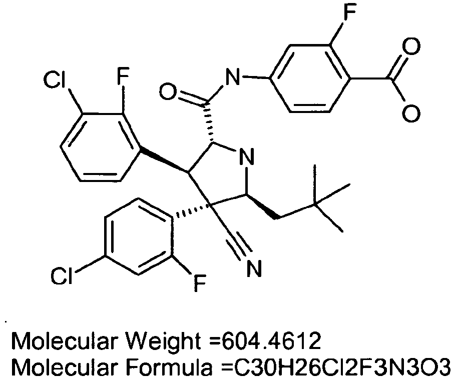

rac 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-2 -fluoro-benzoic acid tert-butyl ester(35 mg, 0.053 mmol) was treated with 50 % TFA/methylene chloride (10 mL) overnight. Removal of solvent and treating the residue with acetonitrile and water gave a white solid after filtration and dying. 29 mg.

HRMS (ES⁺) m/z Calcd: [(M+H)⁺]: 604.1376, found: 604.1376

### Example 281

### Preparation of rac (2R,35,4R,5S)-3-(3-Chloro-2-fluoro-phenyl)-4-(4-chloro-2-fluoro-phenyl)-4-cyano-5-(2,2-dimethyl-propyl)-pyrrotidine-2-carboxylic acid (6-methoxy-pyridin-3-yl)-amide

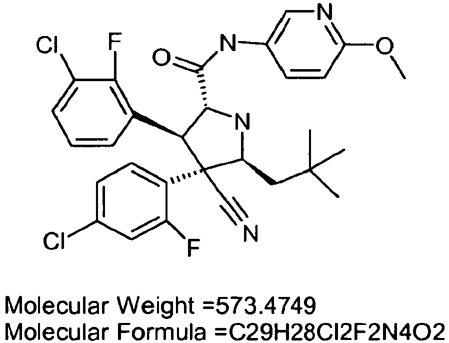

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (100 mg, 0.217 mmol) in methylene chloride (10 mL), HATU (Aldrich,118 mg, 0.31 mmol) was added followed by the addition of DIPEA(0.15 mL, 0.86 mmol) and 3-amino-6-methoxy-pyridine(Aldrich, 43 mg, 0.34 mmol). The mixture was stirred at rt for overnight. The reaction was quenched with addition of water. The mixture was extracted with methylene chloride (2X10 mL) and the extracts were dried with magnesium sulfate. The solvent was removed and the residue was purified on an ISCO machine (40 g column, 0-5% MeOH/methylene chloride) to give a white solid. 73 mg.

MS (ES⁺) m/z Calcd: [(M+H)⁺]: 573.1630, found: 573.1630

### Example 281

### Preparation of rac 3-({[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-methyl)-benzoic acid methyl ester.

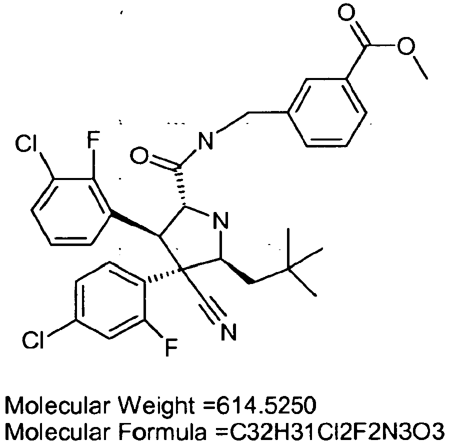

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (150 mg, 0.26 mmol) in methylene chloride (10 mL), HATU (Aldrich,177 mg, 0.46 mmol) was added followed by the addition of DIPEA(0.36 mL, 2.06 mmol) and 3-aminomethyl-benzoic acid methyl ester hydrochloride salt(Aldrich, 104 mg, 0.52 mmol). The mixture was stirred at rt for overnight. The reaction was quenched with addition of water. The mixture was extracted with methylene chloride (2X10 mL) and the extracts were dried with magnesium sulfate. The solvent was removed and the residue was purified on an ISCO machine (40 g column, 5-15% EtOAc/hexanes) to give a white solid. 118 mg.

MS (ES⁺) m/z Calcd: [(M+H)⁺]: 614, found: 614

### Example 282

### Preparation of rac 4-({[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-methyl)-benzoic acid methyl ester

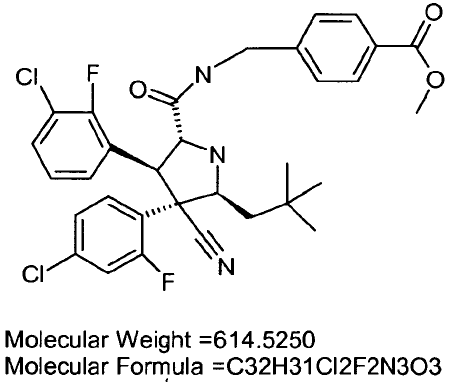

To a stirred solution of rac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (150 mg, 0.26 mmol) in methylene chloride (10 mL), HATU (Aldrich,177 mg, 0.46 mmol) was added followed by the addition of DIPEA(0.36 mL, 2.06 mmol) and 4-aminomethyl-benzoic acid methyl ester hydrochloride salt (Aldrich, 104 mg, 0.52 mmol). The mixture was stirred at rt for overnight. The reaction was quenched with addition of water. The mixture was extracted with methylene chloride (2X10 mL) and the extracts were dried with magnesium sulfate. The solvent was removed and the residue was purified on an ISCO machine (40 g column, 5-15% EtOAc/hexanes) to give a white solid. 119 mg.

MS (ES⁺) m/z Calcd: [(M+H)⁺]: 614, found: 614

### Example 283

### Preparation of rac (2R,35,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (4-chloro-phenyl)-amide

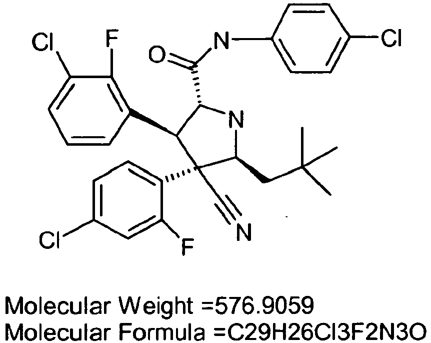

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (200 mg, 0.34 mmol) in methylene chloride (10 mL), HATU (Aldrich, 234 mg, 0.62 mmol) was added followed by the addition of DIPEA(0.30 mL, 1.71 mmol) and 4-chloro-aniline (Aldrich, 88 mg, 0.68 mmol). The mixture was stirred at rt for overnight. The reaction was quenched with addition of water. The mixture was extracted with methylene chloride (2X10 mL) and the extracts were dried with magnesium sulfate. The solvent was removed and the residue was purified on an ISCO machine (40 g column, 5-15% EtOAc/hexanes) to give a white solid. 105 mg.

MS (ES⁺) m/z Calcd: [(M+H)⁺]: 576, found : 576

### Example 284

### Preparation of (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (4-chloro-phenyl)-amide

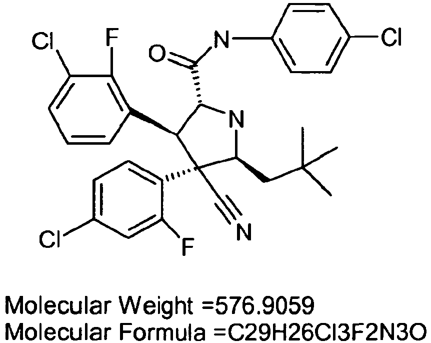

rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (4-chloro-phenyl)-amide (100mg) was separated on a Berger SFC machine under 100 bar, 30°C, and 45% of MEOH through a Whelk column to give a white solid (peak 2, 41 mg)

MS (ES⁺) m/z Calcd: [(M+H)⁺]: 576, found: 576

### Example 285

### Preparation of (2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-chloro-phenyl)-amide

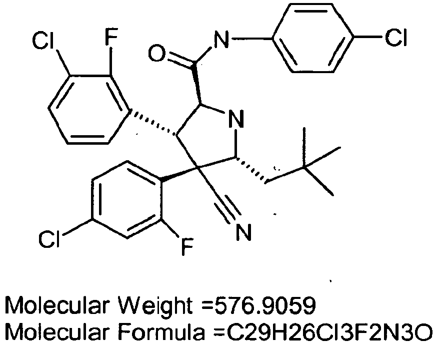

rac (2S,3R,4S,5R)-3-(3-Chloro-2-fluoro-phenyl)-4-(4-chloro-2-fluoro-phenyl)-4-cyano-5-(2,2-dimethyl-propyl)-pyrrolidine-2-carboxylic acid (4-chloro-phenyl)-amide (100mg) was separated on a Berger SFC machine under 100 bar, 30°C, and 45% of MeOH through a Whelk column to give a white solid (peak 1, 40 mg)

MS (ES⁺) m/z Calcd: [(M+H)⁺]: 576, found: 576

### Example 286

### Preparation of rac 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-2 -methoxy-benzoic acid methyl ester

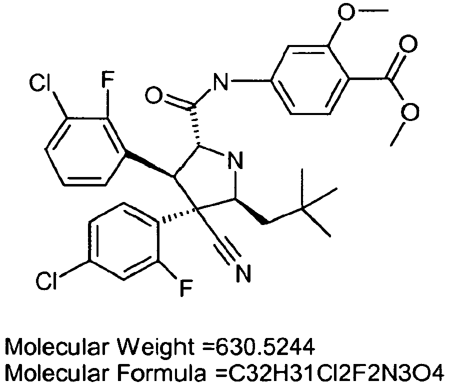

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (100 mg, 0.17 mmol) in ethylene chloride (10 mL), HATU (Aldrich,118 mg, 0.31 mmol) was added followed by the addition of DIPEA(0.15 mL, 0.86 mmol) and 4-amino-2-methoxy-benzoic acid (Avocado, 62 mg, 0.34 mmol). The mixture was stirred at rt for overnight. The reaction was quenched with addition of water. The mixture was extracted with methylene chloride (2X10 mL) and the extracts were dried with magnesium sulfate. The solvent was removed and the residue was purified on an ISCO machine (40 g column, 5-15% EtOAc/methylene chloride) to give a white solid. 29 mg.

MS (ES⁺) m/z Calcd: [(M+H)⁺]: 630.1733, found: 630.1732

### Example 287

### Preparation of rac 3-({[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-methyl)-benzoic acid

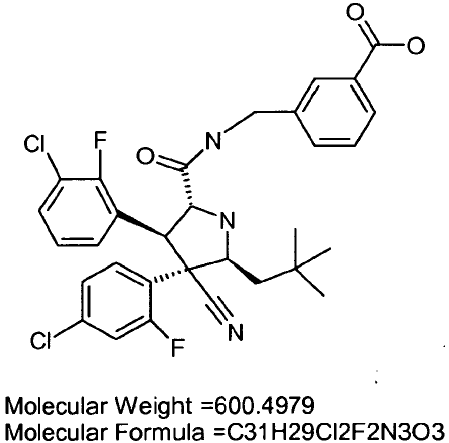

rac 3-({[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-methyl)-benzoic acid methyl ester (40 mg) was dissolved in MeOH (10 mL) with help of slight heating. To the stirred solution was added NaOH (1N, 2 mL) and the mixture was stirred for 1.5 hrs. The solvent was removed and the residue was treated with I N HCl to make the mixture acidic. The white suspension was extracted with EtOAc (3 x10 mL) and the extracts combined and dried with sodium sulfate. The solvent was removed and the residue was freeze dried to give a white powder. 38 mg.

MS (ES⁺) m/z Calcd: [(M+H)⁺]: 600, found: 600

### Example 288

### Preparation of rac 4-({[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-methyl)-benzoic acid

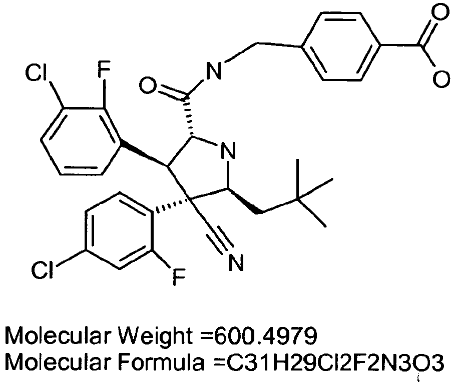

rac 4-({[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-methyl)-benzoic acid methyl ester (40 mg) was dissolved in MeOH (10 mL) with help of slight heating. To the stirred solution was added NaOH(1N, 2 mL) and the mixture was stiired for 1.5 hrs. The solvent was removed and the residue was treated with I N HCl to make the mixture acidic. The white suspension was extracted with EtOAc (3x10 mL) and the extracts combined and dried with sodium sulfate. The solvent was removed and the residue was freeze dried to give a white powder. 38 mg.

MS (ES⁺) m/z Calcd; [(M+H)⁺]: 600, found: 600

### Example 289

### Preparation of (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (4-acetylamino-phenyl)-amide

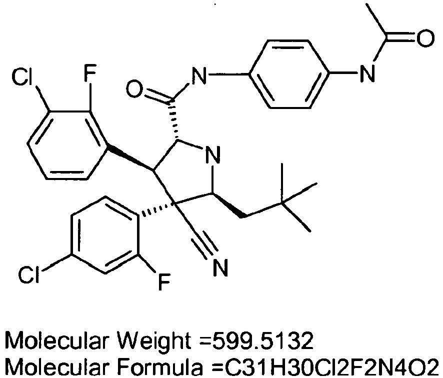

rac (2R,35,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (4-acetylamino-phenyl)-amide (360mg) was separated on a Berger SFC machine under 100 bar, 30°C, and 45% of MeOH through an OJ column to give a white solid (peak 1, 103 mg)

MS (ES⁺) m/z Calcd: [(M+H)⁺]: 599, found: 599

### Example 290

### Preparation of (2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-acetylamino-phenyl)-amide

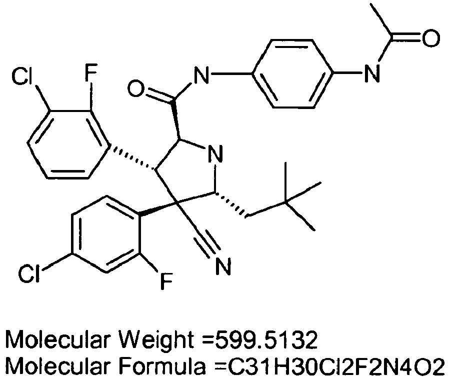

rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (4-acetylamino-phenyl)-amide (360mg) was separated on a Berger SFC machine under 100 bar, 30°C, and 45% of MeOH through a OJ column to give a white solid (peak 2, 100 mg)

MS (ES⁺) m/z Calcd: [(M+H)⁺]: 599, found: 599

### Example 291

### Preparation of (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-methanesulfonyl-phenyl)-amide

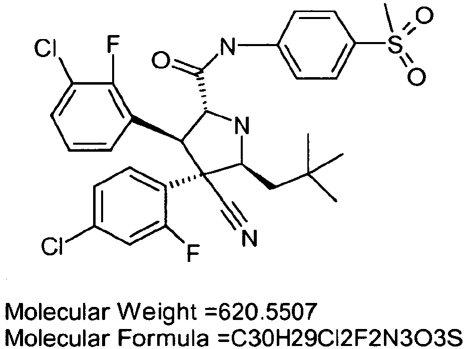

rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-methanesulfonyl-phenyl)-amide(165mg) was separated on a Berger SFC machine under 100 bar, 30°C, and 20% of MeOH to give a white solid (peak 1, 90 mg)

MS (ES⁺) m/z Calcd: [(M+H)⁺]: 620, found: 620

### Example 292

### Preparation of (2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-methanesulfonyl-phenyl)-amide

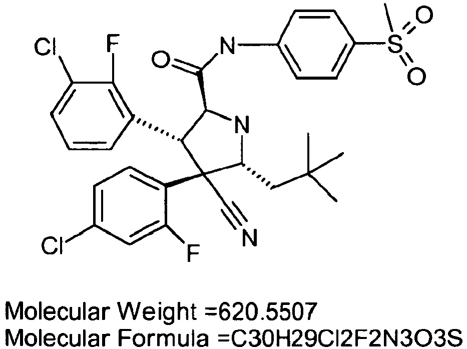

rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-methanesulfonyl-phenyl)-amide(165mg) was separated on a Berger SFC machine under 100 bar, 30°C, and 20% of MeOH to give a white solid (peak 2, 90 mg)

MS (ES⁺) m/z Calcd: [(M+H)⁺]: 620, found: 620

### Example 293

### Preparation of rac 4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-2-methoxy-benzoic acid

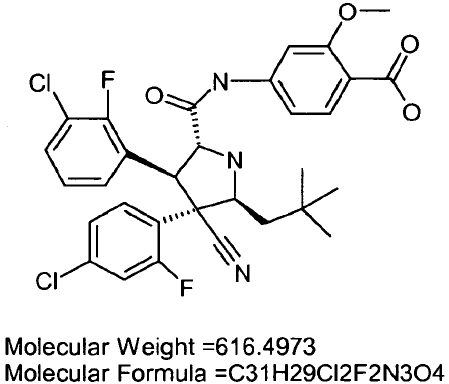

rac 4- {[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-2-methoxy-benzoic acid ester (40 mg) was dissolved in MeOH (10 mL) with help of slight heating. To the stirred solution was added NaOH (1N, 2 mL) and the mixture was stirred for 4hrs at 50°C. The solvent was removed and the residue was treated with I N HCl to make the mixture acidic. The white suspension was extracted with EtOAc (3 x10 mL) and the extracts combined and dried with sodium sulfate. The solvent was removed and the residue was freeze dried to give a white powder. 27 mg.

MS (ES⁺) m/z Calcd: [(M+H)⁺]: 616, found: 616

### Example 294

### Preparation of rac 5-bromo-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-2-methoxy-benzoic acid methyl ester

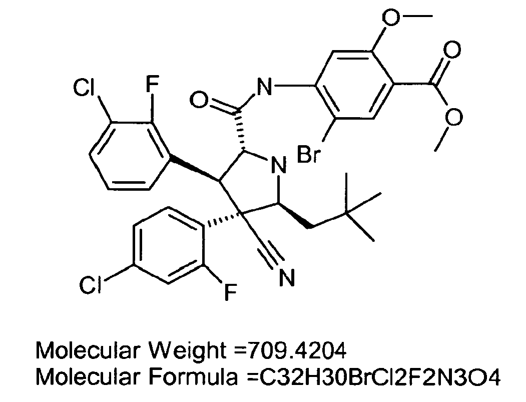

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (200 mg, 0.34 mmol) in methylene chloride (10 mL), HATU (Aldrich, 235 mg, 0.62 mmol) was added followed by the addition of DIPEA(0.30 mL, 1.72 mmol) and 4-amino-5-bromo-2-methoxy-benzoic acid (Aldrich, 179 mg, 0.69 mmol). The mixture was stirred at rt overnight. The reaction was quenched with addition of water. The mixture was extracted with methylene chloride (2X10 mL) and the extracts were dried with magnesium sulfate. The solvent was removed and the residue was purified on an ISCO machine (40 g column, 5-15% EtOAc/methylene chloride) to give a white solid. 7.3 mg.

MS (ES⁺) m/z Calcd: [(M+H)⁺]: 709, found: 709

### Example 295

### Preparation of rac 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-2-methyl-benzoic acid methyl ester

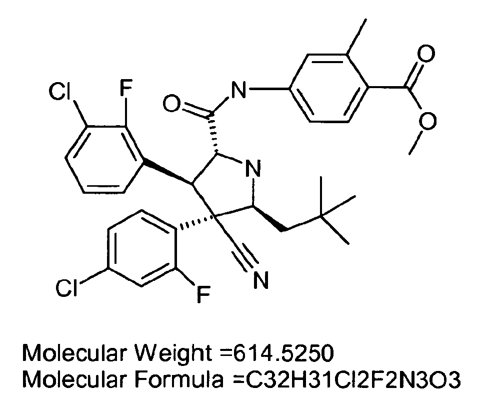

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (200 mg, 0.34 mmol) in methylene chloride (10 mL), HATU (Aldrich, 235 mg, 0.62 mmol) was added followed by the addition of DIPEA(0.30 mL, 1.72 mmol) and 4-amino-2 -methyl-benzoic acid (Aldrich, 114 mg, 0.69 mmol). The mixture was stirred at rt overnight. The reaction was quenched with addition of water. The mixture was extracted with methylene chloride (2X10 mL) and the extracts were dried with magnesium sulfate. The solvent was removed and the residue was purified on an ISCO machine (40 g column, 5-15% EtOAc/methylene chloride) to give a white solid. 102 mg.

MS (ES⁺) m/z Calcd: [(M+H)⁺]: 614, found: 614

### Example 296

### Preparation of 4-amino-2-chlorobenzoic acid methyl ester.

A solution of 4-nitro-2-chlorobenzoic acid methyl ester (Aldrich, 700 mg, 3.24 mmol) in ethyl acetate (50 mL) was treated with 10% Pd/C (50 mg) and hydrogenated at 1 atmosphere for 3 hrs. The mixture was filtered and concentrated to give a light yellow solid which was directly used for the next step.

### Example 297

### Preparation of rac 2-Chloro-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid methyl ester

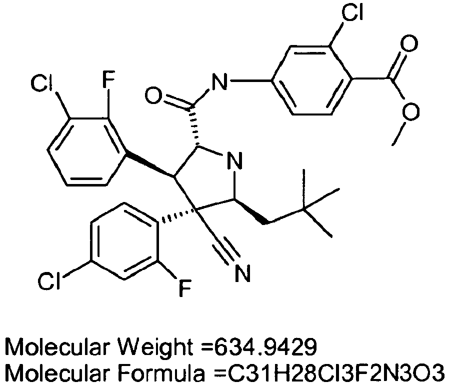

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (200 mg, 0.34 mmol) in methylene chloride (10 mL), HATU (Aldrich, 235 mg, 0.62 mmol) was added followed by the addition of DIPEA(0.30 mL, 1.72 mmol) and 4-amino-2-chloro-benzoic acid (made above, 128 mg, 0.69 mmol). The mixture was stirred at rt overnight. The reaction was quenched with addition of water. The mixture was extracted with methylene chloride (2X10 mL) and the extracts were dried with magnesium sulfate. The solvent was removed and the residue was purified on an ISCO machine (40 g column, 5-15% EtOAc/methylene chloride) to give a white solid. 74 mg.

MS (ES⁺) m/z Calcd: [(M+H)⁺]: 634, found: 634

### Example 298

### Preparation of 4-amino-2-trifluoromethyl benzoic acid methyl ester.

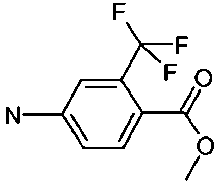

A solution of 4-nitro-2-trifluoromethyl benzoic acid methyl ester (Aldrich, 700 mg, 3.24 mmol) in ethyl acetate (50 mL) was treated with 10% Pd/C (50 mg) and hydrogenated at 1 atmosphere for 3 hrs. The mixture was filtered and concentrated to give a light yellow solid which was directly used for the next step.

### Example 299

### Preparation of rac 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-2-trifluoromethylbenzoic acid methyl ester

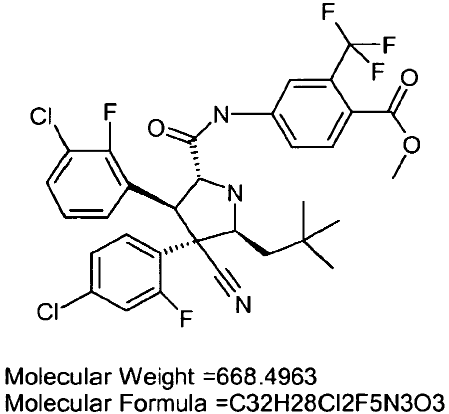

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (166 mg, 0.29 mmol) in methylene chloride (10 mL), HATU (Aldrich,195 mg, 0.54 mmol) was added followed by the addition of DIPEA(0.25 mL, 1.425 mmol) and 4-amino-2-trifluoromethyl-benzoic acid (made above, 125 mg, 0.57 mmol). The mixture was stirred at rt overnight. The reaction was quenched with addition of water. The mixture was extracted with methylene chloride (2X10 mL) and the extracts were dried with magnesium sulfate. The solvent was removed and the residue was purified on an ISCO machine (40 g column, 5-15% EtOAc/methylene chloride) to give a white solid. 40 mg.

MS (ES⁺) m/z Calcd: [(M+H)⁺] : 668, found: 668

### Example 300

### Preparation of rac 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-2-methyl-benzoic acid

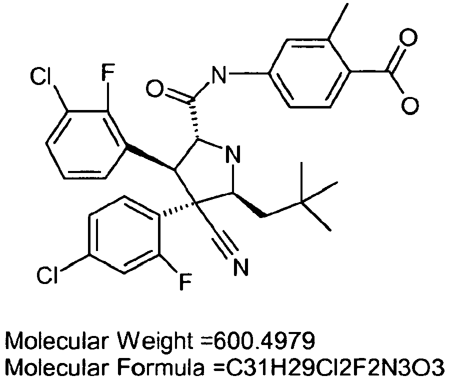

rac 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-2-methyl-benzoic acid methyl ester (82 mg) was dissolved in MeOH (10 mL) with help of slight heating. To the stirred solution was added NaOH (1N, 2 mL) and the mixture was stirred for 3hrs at 50°C. The solvent was removed and the residue was treated with 1N HCl to make the mixture acidic. The white suspension was extracted with EtOAc (3x10 mL) and the extracts combined and dried with sodium sulfate. The solvent was removed and the residue was freeze dried to give a white powder. 67 mg.

MS (ES⁺) m/z Calcd: [(M+H)⁺]: 600, found: 600

### Example 301

### Preparation of rac 2-Chloro-4- {[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid

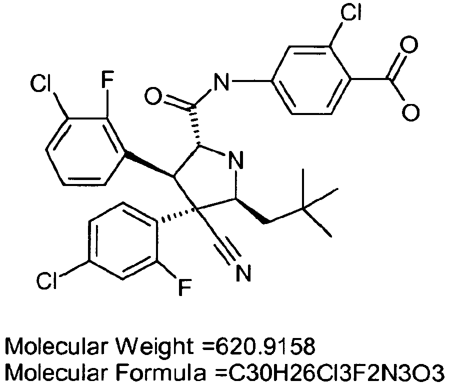

rac 4-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-2-chloro-benzoic acid methyl ester (54 mg) was dissolved in MeOH (10 mL) with help of slight heating. To the stirred solution was added NaOH (1N, 2 mL) and the mixture was stirred for 1hrs at 55°C. The solvent was removed and the residue was treated with 1 N HCl to make the mixture acidic. The white suspension was extracted with EtOAc (3 x10 mL) and the extracts combined and dried with sodium sulfate. The solvent was removed and the residue was freeze dried to give a white powder. 40 mg.

MS (ES⁺) m/z Calcd: [(M+H)⁺]: 620, found: 620

### Example 302

### Preparation of 4-nitro-benzimidic acid methyl ester hydrochloride

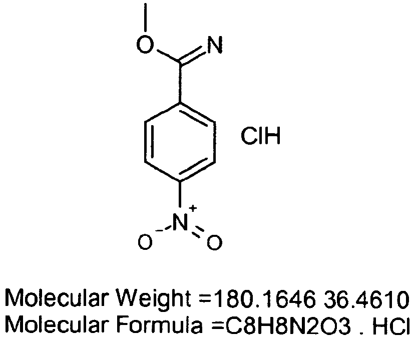

To a stirred solution of4-nitro-benzonitrile(Aldrich, 17 g) in methanol (200 mL) was added 0.53 g of sodium methoxide. The solution was stirred for 12 hrs and another 1.5 g of sodium methoxide was added and the mixture was stirred for 6 hrs. The solution was cooled to 0°C and HCl gas was bubbled in until a white solid forms. The solvent was reduced to about 100 mL and the solid was filtered and dried under vacuum to give a white solid. 12.1 g.

### Example 303

### Preparation of 5-(4-nitro-phenyl)- H-[1,2,4]triazole

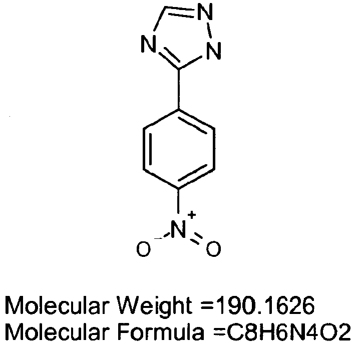

4-Nitro-benzimidic acid methyl ester hydrochloride (635 mg, 2.93 mmol) was suspended in pyridine (7 mL)and formic hydrazide (Aldrich, 178 mg, 2.95 mmol) was added and the mixture was stirred at rt for 1 hr. The solvent was removed and the residue was dissolved in toluene ( 10 mL) and the mixture was stirred at reflux for 1.5 hrs. the mixture was cooled and water was added. The organic layer was separated and dried over sodium sulfate. The solvent was removed and the residue was suspended in 25% EtOAc/Hexanes and the solid was filtered to give a so lid. 510 mg.

MS (ES⁺) m/z Calcd: [(M+H)⁺]: 191, found: 191

### Example 304

### Preparation of 5-(4-amino-phenyl)-1H-[1,2,4]triazole

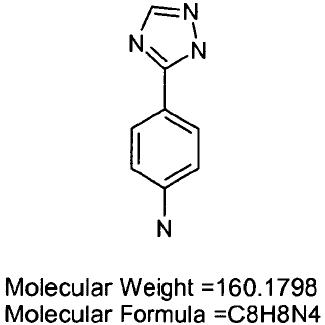

5-(4-Nitro-phenyl)-1H-[1,2,4]triazole (510 mg) was suspended in 50 mL of ethyl acetate and 56 mg of 10% Pd/C was added. The mixture was hydrogenated under 50 Psi for 100 min.. The mixture was filtered and the filtrate was concentrate to give a solid. 497 mg MS (ES⁺) m/z Calcd: [(M+H)⁺]: 161, found : 161

### Example 305

### Preparation of rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid [4-(2H-[1,2,4]triazol-3-yl)-phenyl]-amide

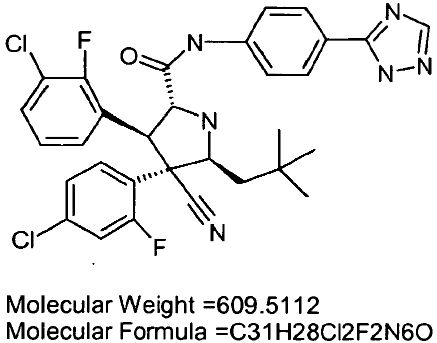

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (150 mg, 0.32 mmol) in methylene chloride (5 mL), HATU (Aldrich,152 mg, 0. 4 mmol) was added followed by the addition of DIPEA(0.1 mL) and 5-(4-amino-phenyl)-1H-[1,2,4]triazole (made above, 64 mg, 0.4 mmol). The mixture was stirred at rt for 2 hrs. The reaction was quenched with addition of water. The mixture was extracted with methylene chloride (2X10 mL) and the extracts were dried with magnesium sulfate. The solvent was removed and the residue was purified on an ISCO machine (40 g column, 70% EtOAc/hexanes) to give a white solid. 140 mg.

MS (ES⁺) m/z Calcd: [(M+H)⁺]: 609, found: 609

### Example 306

### Preparation of N'-[imino-(4-nitro-phenyl)-methyl]-hydrazinecarboxylic acid tert-butyl ester

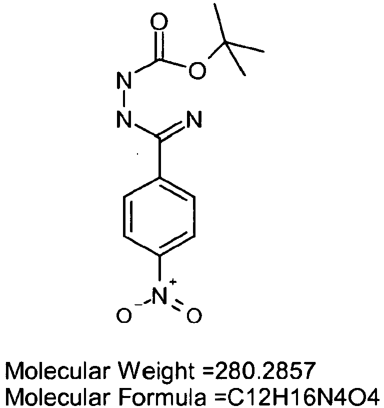

To a stirred solution of 4-nitro-benzimidic acid methyl ester hydrochloride (500 mg, 2.31 mmol) in ethanol (8 mL), hydrazinecarboxylic acid tert-butyl ester (305 mg, 2.31 mmol) was added followed by triethylamine. The mixture was stirred at rt over night. The solvent was removed and the residue was suspended in methylene chloride (10 mL). The mixture was filtered and the filtrate was concentrated to about 4 mL and loaded on a 20 g silica gel column. Eluting with 40-90% EtOAc/Hexanes on an ISCO machine gave a yellow solid. 410 mg. Which was directly used for the next step.

### Example 307

### Preparation of 5-(4-Nitro-phenyl)-1,2-dihydro-[1,2,4]triazol-3-one

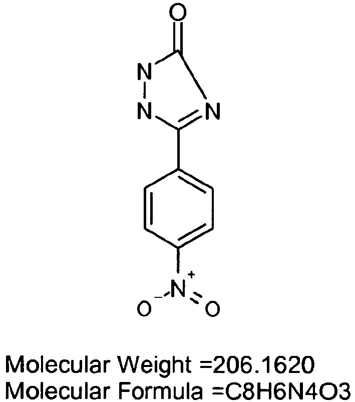

N'-[Imino-(4-nitro-phenyl)-methyl]-hydrazinecarboxylic acid tert-butyl ester (made above, 410 mg) was suspended in 3 mL of acetonitrile and the mixture was heated at 200°C for 5 min.. The mixture was cooled and the solid was filtered and dried. 341 mg.

MS (ES⁺) m/z Calcd: [(M+H)⁺]: 207, found: 207

### Example 308

### Preparation of 5-(4-amino-phenyl)-1,2-dihydro-[1,2,4]triazol-3-one

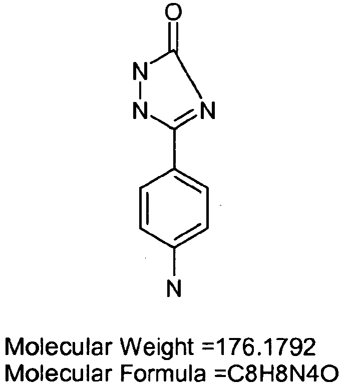

5-(4-amino-phenyl)-1,2-dihydro-[1,2,4]triazol-3-one (made above, 340 mg) was suspended in a mixture of THF and EtOAc( 10 mL each). 10% Pd/C (100 mg) was added and the mixture was hydrogenated under 50 psi for 4 hrs. Filtered and the filtrate was concentrated to give a white solid. 217 mg.

MS (ES⁺)m/z Calcd: [(M+H)⁺]; 177, found: 177.

### Example 309

### Preparation of rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(5-oxo-2,5-dihydro-1H-[1,2,4]triazol-3-yl)-phenyl]-amide

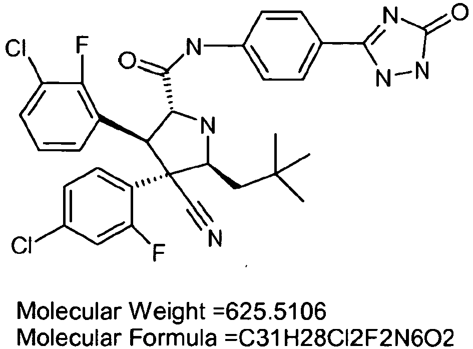

To a stirred solution ofrac (2R,3S,4R,5S)-3-(3-Chloro-2-fluoro-phenyl)-4-(4-chloro-2-fluoro-phenyl)-4-cyano-5-(2,2-dimethyl-propyl)-p yrrolidine-2-carboxylic acid (264 mg, 0.56 mmol) in DMF (5 mL), HATU (Aldrich, 213 mg, 0. 56 mmol) was added followed by the addition of DIPEA(0.1 mL) and 5-(4-amino-phenyl)-1,2-dihydro-[1,2,4]triazol-3-one (made above, 100 mg, 0.56 mmol). The mixture was stirred at rt for 4 hrs. The reaction was quenched with addition of water. The mixture was extracted with methylene chloride (2X10 mL) and the extracts were dried with magnesium sulfate. The solvent was removed and the residue was purified on an ISCO machine (40 g column, 5% MeOH/methylene chloride) to give a white solid. 47 mg.

MS (ES⁺) m/z Calcd: [(M+H)⁺]: 625, found: 625

### Example 310

### Preparation of 3-chloro-4-(1H-tetrazol-5-yl)-phenylamine

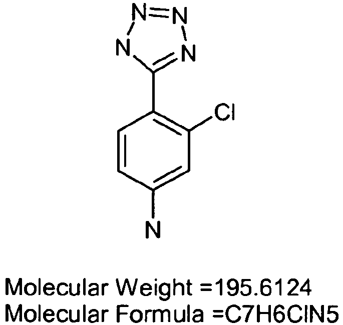

To a stirred solution of 4-amino-2-chloro-benzonitrile (Aldrich, 765 mg, 5 mmol) in toluene (10 mL), sodium azide (423 mg, 6.5 mmol) and triethylamine hydrochloride (895 mg, 6.5 mmol) was added and the mixture was stirred vigorously at 115°C overnight. The mixture was cooled and poured into water. The aqueous layer was adjusted to PH =5 by the addition of 6 N HCl and the solid formed was filtered and dried. 175 mg,

MS (ES⁺) m/z Calcd: [(M+H)⁺]: 196, found: 196

### Example 311

### Preparation of rac-(5-{[(2R,3S,4R,5S)-3-(3-chloro-2-fluoro-phenyl)-4-(4-chloro-2-fluoro-phenyl)-4-cyano-5-(2,2-dimethyl-propyl)-pyrro lidine-2-carbonyl]-amino} -4H-[1,2,4]triazol-3-yl)-acetic acid

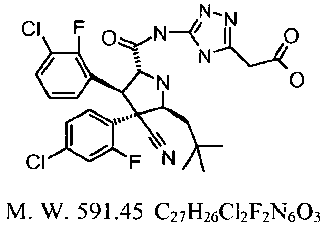

A mixture ofrac- (5-{[(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carbonyl]-amino}-4H-[1,2,4]triazol-3-yl)-acetic acid methyl ester (20 mg, 0.033 mmol) **was** dissolved in THF (0.6 mL) and methanol (0.2 mL), then 2N LiOH (0.2 mL) was added and stirred at room temperature for 3 hours. The mixture was concentrated and diluted with water and ethyl acetate. The organic phase was separated then concentrated to yield rac-(5-{[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-amino }-4H-[1,2,4]triazol-3-yl)-acetic acid (6.7 mg, 34 %) as an off-white powder. HRMS (ES⁺) m/z Calcd for C₂₇H₂₆Cl₂F₂N₆O₃+ H [(M+H)⁺]: 591.1485, found: 591.1483.

### Example 312

### Preparation of rac-(3-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino -pyrazol-1-yl)-acetic acid

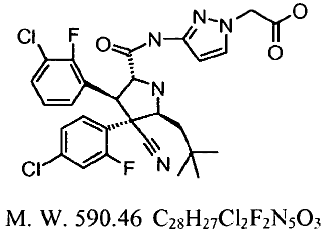

A mixture of rac-(3-{[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-pyrazol-1-yl)-acetic acid tert-butyl ester (25 mg, 0.039 mmol) **was** chilled to 0oC. Then concentrated sulfuric acid (1 mL) was added and the reaction was stirred for 2 hours. Ice and water added all at once, crystals filtered and washed with water. The crystals were azeotroped three times with toluene then treated to high vacuum overnight to yield rac-(3-{[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrro lidine-2-carbonyl]-amino}-pyrazol-1-yl)-acetic acid as an off white powder (12.1 mg, 53 %). HRMS (ES⁺) m/z Calcd for C₂₈H₂₇Cl₂F₂N₅O₃ + H [(M+H)⁺]: 590.1532, found: 590.1 532.

### Example 313

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (1H-imidazol-4-ylmethyl)-amide

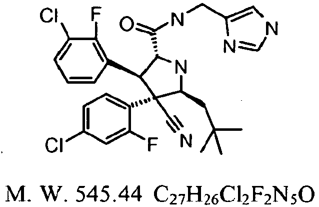

A mixture of rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (200 mg, 0.43 mmol), 1H-(imidazoyl-4-yl) methylamine (62 mg, 0.64 mmol), 2-(7-azabenzotriazol-1-yl)-n,n,n',n'-tetramethyluronium hexafluorophosphate (HATU, 243.3 mg, 0.64 mmol) and iPr₂NEt (0.22 mL, 1.2 mmol) in CH₂Cl₂ (10 mL) was stirred at rt overnight. The mixture was then diluted with CH₂Cl₂ and washed with water, brine. The organic phase was separated, dried over Na₂SO₄ and filtered. The mixture was then concentrated and purified by reverse phase chromatography (15-95% of ACN/water) to rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (1H-imidazol-4-ylmethyl)-amide ( 6.2 mg, 2.6 %) as an off-white powder. HRMS (ES⁺) m/z Calcd for C₂₇H₂₆Cl₂F₂N₅O+ H [(M+H)⁺]: 546.1634, found: 546. 1632.

### Example 314

### Preparation of rac-(2S,3R,4S,5R)-4-(3-chloro-2-fluoro-phenyl)-3-(4-chloro-2-fluoro-phenyl)-2-(2,2-dimethyl-propyl)-5-(2-oxa-6-aza-spiro[3.3]heptane-6-carbonyl)-pyrrolidine-3-carbonitrile

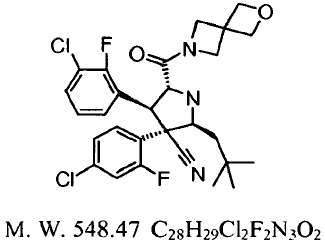

A mixture of rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (200 mg, 0.43 mmol), 2-oxa-6-aza-spiro[3.3] heptane oxylate salt (123 mg, 0.65 mmol), 2-(7-azabenzotriazol-1-yl)-n,n,n',n'-tetramethyluronium hexafluorophosphate (HATU, 243.3 mg, 0.64 mmol) and iPr₂NEt (0.22 mL, 1.2 mmol) in CH₂Cl₂ (10 mL) was stirred at rt overnight. The mixture was then diluted with CH₂Cl₂ and washed with water, brine. The organic phase was separated, dried over Na₂SO₄ and filtered. The mixture was then concentrated and purified by reverse phase chromatography (20-95% of ACN/water) to rac-(2*S*,3*R*,4*S*,5*R*)-4-(3-chloro-2-fluoro-phenyl)-3-(4-chloro-2-fluoro-phenyl)-2-(2,2-dimethyl-propyl)-5-(2-oxa-6-aza-spiro[3.3]heptane-6-carbonyl)-pyrrolidine-3-carbonitrile ( 78 mg, 33.3 %) as an off-white powder. HRMS (ES⁺) m/z Calcd for C₂₈H₂₉Cl₂F₂N₃O₂+ H [(M+H)⁺] 548.1678, found: 548.1678.

### Example 316

### Preparation of rac-1-[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-azetidine3-carboxylic acid

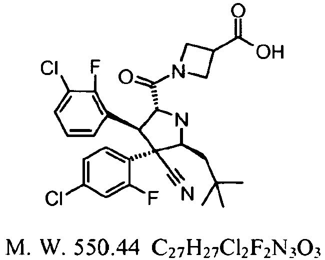

A mixture of rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (200 mg, 0.43 mmol), azetidine-3-carboxylate methyl ester hydrochloride (200 mg, 1.32 mmol), 2-(7-azabenzotriazol-1-yl)-n,n,n',n'-tetramethyluronium hexafluorophosphate (HATU, 243.3 mg, 0.64 mmol) and iPr₂NEt (0.22 mL, 1.2 mmol) in CH₂Cl₂ (10 mL) was stirred at rt overnight. The mixture was then diluted with CH₂Cl₂ and washed with water, brine. The organic phase was separated, dried over Na₂SO₄ and filtered. The mixture was then concentrated and purified by reverse phase chromatography (20-95% of ACN/water) to yield 1-[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-azetidine-3-carboxylic acid methyl ester (26 mg, 10.7 %). The ester was taken direcly to the hydrolysis step by dissolved in THF (0.6 mL) and methanol (0.2 mL), then 2N LiOH (0.2 mL) was added and stirred at room temperature for 3 hours. The mixture was concentrated and diluted with water and ethyl acetate. The organic phase was separated then concentrated to rac-1-[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-azetidine3-carboxylic acid (11.8 mg, 46.6 %) as an off-white powder. HRMS (ES⁺) m/z Calcd for C₂₇H₂₇Cl₂F₂NO₃+ H [(M+H)⁺]: 550.1471, found: 550.1 471.

### Example 317

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-[1,2,3]triazol-1-yl-ethyl)-amide

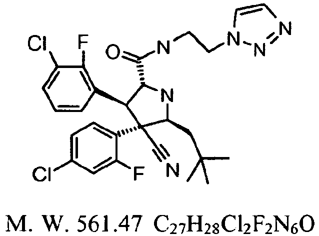

A mixture of rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (200 mg, 0.43 mmol), 2-[1,2,3]triazol-1-yl ethylamine (200 mg, 1.78 mmol), 2-(7-azabenzotriazol-1-yl)-n,n,n',n'-tetramethyluronium hexafluorophosphate (HATU, 243.3 mg, 0.64 mmol) and iPr₂NEt (0.22 mL, 1.2 mmol) in CH₂Cl₂ (10 mL) was stirred at rt overnight. The mixture was then diluted with CH₂Cl₂ and washed with water, brine. The organic phase was separated, dried over Na₂SO₄ and filtered. The mixture was then concentrated and purified by reverse phase chromatography (15-95% of ACN/water) rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-[1,2,3]triazol-1-yl-ethyl)-amide ( 83.7 mg, 34.7 %) as an off-white powder. HRMS (ES⁺) m/z Caled for C₂₇H₂₈Cl₂F₂N₆O + H [(M+H)⁺]: 561.1743, found: 561.1741.

### Example 318

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-carbamoylmethyl-1H-pyrazol-3-yl)-amide

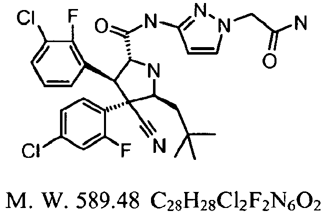

A mixture of rac-(3-{[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-pyrazol-1-yl)-acetic acid (85 mg, 0.144 mmol), ammonia (0.57 mL, 0.288 mmol, 0.5 M in dioxane), 2-(7-azabenzotriazol-1-yl)-n,n,n',n'-tetramethyluronium hexafluorophosphate (HATU, 109 mg, 0.288mmol) and iPr₂NEt (0.1 mL, 0.43 mmol) in CH₂Cl₂ (10 mL) was stirred at rt overnight. The mixture was then diluted with CH₂Cl₂ and washed with water, brine. The organic phase was separated, dried over Na₂SO₄ and filtered. The mixture was then concentrated and purified by reverse phase chromatography (20-95% of ACN/water) rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-[1,2,3]triazol-1-yl-ethyl)-amide (12.3 mg, 14.5 %) as an off-white powder. HRMS (ES⁺) m/z Calcd for C₂₈H₂₈Cl₂F₂N₆O₂ + H [(M+H)⁺]: 589.1692, found: 589.1693.

### Example 319

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid [1-(2-hydroxy-2-methyl-propyl)-1H-pyrazol-3-yl]-amide

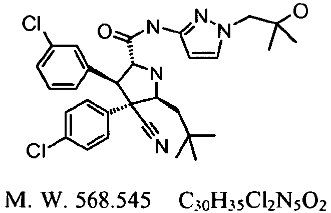

A mixture of rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (86.2 mg, 0.20 mmol), 1-(3-amino-pyrazol-1-yl)-2-methyl-propan-2-ol (93.12 mg, 0.3 mmol), 2-(7-azabenzotriazol-1-yl)-n,n,n',n'-tetramethyluronium hexafluorophosphate (HATU, 76 mg, 0.2 mmol) and iPr₂NEt (0.1 mL, 0.55 mmol) in CH₂Cl₂ (2 mL) was stirred at rt overnight. The mixture was then diluted with CH₂Cl₂ and washed with water, brine. The organic phase was separated, filtered and dried over Na₂SO₄. The mixture was then concentrated and purified by reverse phase chromatography (20-95% of ACN/water) to give rac-(2*R*,3*R*,4*R*,5*S*')-3-(3-chloro-phenyl)-4-(4-chloro-phenyl)-4-cyano-5-(2,2-dimethyl-propyl)-pyrrolidine-2-carboxylic acid [1-(2-hydroxy-2-methyl-propyl)-1H-pyrazol-3-yl]-amide (54.1 mg, 47.6 %) as an off-white powder. HRMS (ES⁺) m/z Calcd for C₃₀H₃₅Cl₂N₅O₂ + H [(M+H)⁺]: 568.2241, found: 568.2246.

### Example 320

### Preparation of rac-(2R,3R,4R,5S)-3-(3-chloro-phenyl)-4-(4-chloro-phenyl)-4-cyano-5-(2,2-dimethyl-propyl)-pyrrolidine-2-carboxylic acid (3-methanesulfonylamino-propyl)-amide

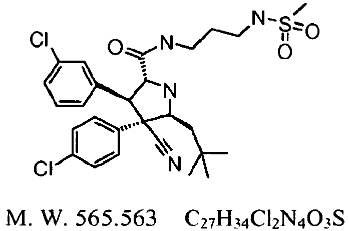

A mixture ofrac (2R,3R,4R,5S)-3-(3-chloro-phenyl)-4-(4-chloro-phenyl)-4-cyano-5-(2,2-dimethyl-propyl)-pyrrolidine-2-carboxylic acid (3-amino-propyl)-amide (120 mg, 0.24 mmol), methane sulfonyl chloride (49 L, 0.6 mmol) and dimethylaminopyridine (97.6 mg, 0.8 mmol) in CH₂Cl₂ (8 mL) was stirred for 5h at rt. The mixture was then extracted with NaHCO₃(s), water and the organic layer dried with MgSO₄, filtered and the solvent was evaporated under reduced pressure diluted with CH₂Cl₂ and washed with water, brine. The organic phase was separated, filtered and dried over Na₂SO₄. The mixture was then purified by reverse phase chromatography (20-95% of ACN/water) to give rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (3-methanesulfonylamino-propyl)-amide (112.1 mg, 80.5 %) as an off-white powder. HRMS (ES⁺) m/z Calcd for C₂₇H₃₄Cl₂N₄O₃S + H [(M+H)⁺]: 565.1802, found: 565.1800.

### Example 321

### Preparation of chiral (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((S)-3-dimethylamino-2-hydroxy-propoxy)-2-methyl-propyl]-1H-pyrazol-3- yl}-amide and chiral (2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((S)-3-dimethylamino-2-hydroxy-propoxy)-2-methylpropyl]-1H-pyrazol-3- yl}-amide

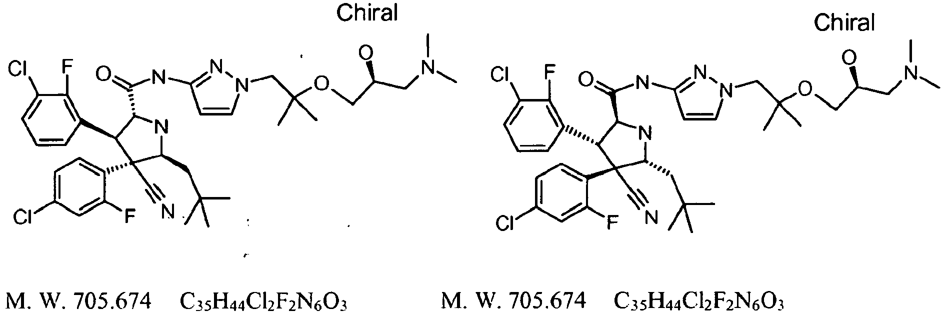

A mixture of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (86.2 mg, 0.20 mmol), (*S*)-1-[2-(3-amino-pyrazol-1-yl)-1,1-dimethyl-ethoxy]-3-dimethylamino-propan-2-ol (370 mg, 1.44 mmol), 2-(7-azabenzotriazol-1-yl)-n,n,n',n'-tetramethyluronium hexafluorophosphate (HATU, 950 mg, 2.5 mmol) and iPr₂NEt (1.4 mL, 8 mmol) in CH₂Cl₂ (50 mL) was stirred at rt overnight. The mixture was then diluted with CH₂Cl₂ and washed with water, brine. The organic phase was separated, dried over Na₂SO₄ and filtered. The mixture was then concentrated and purified by reverse phase chromatography (15-95% of ACN/water) to give rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((S)-3-dimethylamino-2-hydroxy-propoxy)-2-methyl-propyl]-1H-pyrazol-3- yl}-amide (255 mg, 18 %) as an off-white powder. The racemate mixture was submitted for SFC purification to of chiral (2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((*S*)-3-dimethylamino-2-hydroxy-propoxy)-2-methyl-propyl]-1H-pyrazol-3-yl}-amide (85.1 mg, 6.1 %) as an white powder. HRMS (ES⁺) m/z Calcd for C₃₅H₄₄Cl₂F₂N₆O₃ + H [(M+H)⁺] 705.2893, found: 705.2891 and chiral (2*S*,3*R*,4*S*,5*R*)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((S)-3-dimethylamino-2-hydroxy-propoxy)-2-methyl-propyl]-1H-pyrazol-3- yl}-amide(23.1 mg, 20.8 %) as an off-white powder. HRMS (ES⁺) m/z Calcd for C₃₅H₄₄Cl₂F₂N₆O₃+ H [(M+H)⁺]: 705.2893, found: 705.2889.

### Example 322

### Preparation of (S)-1-[2-(3-Amino-pyrazol-1-yl)-1,1-dimethyl-ethoxy]-3-dimethylamino-propan-2-ol

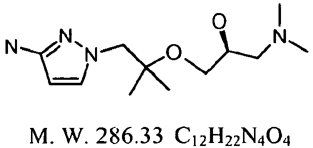

Compound (*S*)-1-dimethylamino-3-[1,1-dimethyl-2-(3-nitro-pyrazol-1-yl)-ethoxy]-propan-2-ol was prepared by reacting 1-[2-methyl-2-((*S*)-1-oxiranylmethoxy)-propyl]-3-nitro-1H-pyrazole (0.39 g, 1.62 mmol), isopropyl alcohol (6 mL), and dimethylamine (3 mL, 6 mmol, 2 M in dioxane) under microwaveconditions at 130°C for 15 min. The mixture was extracted with dichloromethane and water. The organic layer was separated and the solvent evaporated under reduced pressure to yield S)-1-dimethylamino-3-[1,1-dimethyl-2-(3-nitro-pyrazol-1-yl)-ethoxy]-propan-2-ol (0.4 g, 87 %). This compound was reduced under the following conditions-The mixture of (*S*)- 1-dimethylamino-3-[1,1-dimethyl-2-(3-nitro-pyrazol-1-yl)-ethoxy]-propan-2-ol (0.39 g, 1.66 mmol), ethyl acetate (30 mL), ethanol (30 mL) was added to a Parr bottle with 10% Pd/C (0.22 g) and subjected hydrogen gas (20 psi) for 2 h under the Parr Shaker Apparatus conditions. Work up by filtration via a glass membrane filter paper, solvent was removed under reduced pressure to afford (S)-1-[2-(3-Amino-pyrazol-1-yl)-1,1-dimethyl-ethoxy]-3-dimethylamino-propan-2-ol as an oil (0.37 g, 97.5 %).

### Example 323

### Preparation of 1-[2-methyl-2-((S)-1-oxiranylmethoxy)-propyl]-3-nitro-1H-pyrazole

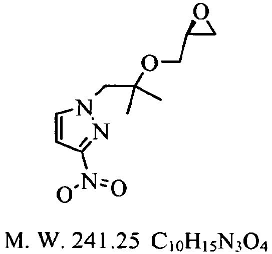

A mixture of 2-methyl-1-(3-nitro-pyrazol-1-yl)-propan-2-ol (0.64 g, 3.46 mmol), and DMF (30 mL) was stirred at 0°C for 5 min, then NaH (60% dispersion in oil, 0.415 g, 17.3 mmol) was added and stirred 20 min at 0°C. S-(+)-glycidyl-3-nitrobenzenesulfonate (1.79 g, 6.92 mmol) was added and stirred at 0°C for 1 h then warmed to 25°C for 3h. The mixture was then diluted with NH₄Cl(s), ethyl acetate, the organic phase was separated, washed with NaHCO₃(satd) dried with Na₂SO₄, and filtered. The mixture was then concentrated and purified by column chromatography (40-120 g Analogix column, 80% EtOAc/heptane to yield the product 1-[2-methyl-2-((*S*)-1-oxiranylmethoxy)-propyl]-3-nitro-1H-pyrazole as a white solid (0.36 g, 43.1 %).

### Example 324

### Preparation of chiral-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid and chiral (2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid

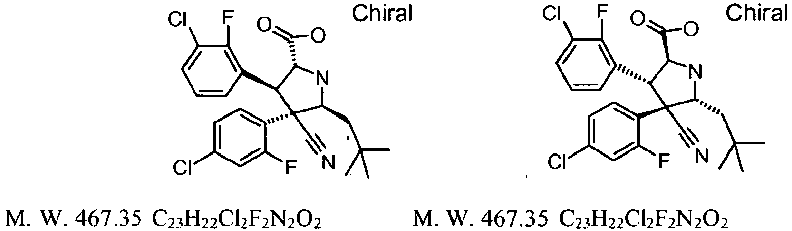

A mixture of rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid was synthesized by the Synthesis group (Lot# 40476-42-2, 84 g). A portion was submitted (20.5g, 40526-055-1) for SFC Separation CH#3978; AD column method #05200916, 2 mL/min; 10% methanol, 100 bar, 30°C to afford chiral-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid as a white solid (10.12 g) HRMS(ES⁺) *m*/*z* Calcd C₂₃H₂₂Cl₂F₂N₂O₂+H [(M+H):467.1099; Found:467.1099 and chiral (2*S*,3*R*,4*S*,5*R*)-3-(3-chloro-2-fluoro-phenyl)-4-(4-chloro-2-fluoro-phenyl)-4-cyano-5-(2,2-dimethyl-propyl)-pyrrolidine-2-carboxylic acid as a white solid (9.6 g) HRMS(ES⁺) *m*/*z* Calcd C₂₃H₂₂Cl₂F₂N₂O₂+H [(M+H):467.1099; Found:467.1099.

### Example 325

### Preparation of rac-1-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-cyclopropane carboxylic acid

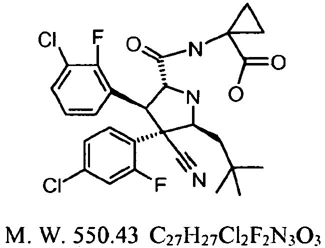

A mixture of rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (200 mg, 0.43 mmol), 1-amino-cyclopropanecarboxylic acid methyl ester hydrochloride (100 mg, 0.86 mmol), 2-(7-azabenzotriazol-1-yl)-n,n,n',n'-tetramethyluronium hexafluorophosphate (HATU, 243.3 mg, 0.64 mmol) and iPr₂NEt (0.22 mL, 1.2 mmol) in CH₂Cl₂ (20 mL) was stirred at rt overnight. The mixture was then diluted with CH₂Cl₂ and washed with water, brine. The organic phase was separated, dried over Na₂SO₄ and filtered. The mixture was then concentrated and purified flash column chromatography (1-100% ethyl acetate/heptane) to afford rac-1-{[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-amino -cyclopropanecarboxylic acid methyl ester as a white powder (120 mg, 49.6 %). HRMS (ES⁺) m/z Caled for C₂₈H₂₉Cl₂F₂N₃O₃+ H [(M+H)⁺]: 564.1627, found: 564.1627.

The ester was taken directly to the hydrolysis step by dissolved in THF (3 mL) and methanol (1 mL), then 2N LiOH (1 mL) was added and stirred at room temperature for 3 hours. The mixture was diluted with water and ethyl acetate, the organic phase was separated then concentrated under reduced pressure to rac-1-{[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-amino } - cyclopropanecarboxylic acid (65.2 mg, 67.2 %) as an off-white powder. HRMS (ES⁺) m/z Calcd for C₂₇H₂₇Cl₂F₂N₃O₃ + H [(M+H)⁺]: 550.1471, found: 550.1471.

### Example 326

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [1-(4-hydroxy-piperidin-4-ylmethyl)-1H-pyrazol-3-yl]-amide

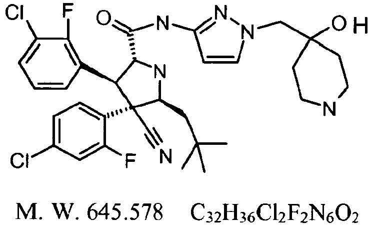

A mixture of rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (500 mg, 1.0mmol), 4-(3-amino-pyrazol-1-ylmethyl)-4-hydroxy-piperidine-1-carboxylic acid tert-butyl ester (592 mg, 2.0 mmol), 2-(7-azabenzotriazol-1-yl)-n,n,n',n'-tetramethyluronium hexafluorophosphate (HATU, 593 mg, 1.5 mmol) and iPr₂NEt (0.718 mL, 4 mmol) in CH₂Cl₂ (50 mL) was stirred at rt overnight. The mixture was then diluted with CH₂Cl₂ and washed with water, brine. The organic phase was separated, dried over Na₂SO₄ and filtered. The mixture was then concentrated and purified flash column chromatography (1-100% ethyl acetate/heptane) to afford rac- 4-(3-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-pyrazol-1-ylmethyl)-4-hydroxy-piperidine-1-carboxylic acid tert-butyl ester as a white powder (480 mg, 64.4 %).

The compound was taken directly to the deprotection step by dissolved in a solution of 30% TFA in dichloromethane (3 mL) and stirred at room temperature for 3 hours. The mixture was diluted with NaHCO₃(s) and dichloromethane and the organic phase was separated then concentrated under reduced pressure to afford an oil that was purified via triteration with ethyl acetate and heptane to afford a white foam rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid [1-(4-hydroxy-piperidin-4-ylmethyl)-1H-pyrazol-3-yl]-amide (370 mg, 64.3 %). HRMS (ES⁺) m/z Calcd for C₃₂H₃₆Cl₂F₂N₆O₂ + H [(M+H)⁺]: 645.2318, found: 645.2315.

### Example 327

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-acetyl-thiophen-3-yl)-amide

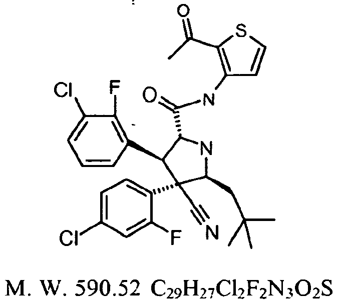

A mixture of rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (200 mg, 0.43 mmol), 1-(3-aminothiophen-2-yl) ethanone (140 mg, 0.98 mmol), 2-(7-azabenzotriazol-1-yl)-n,n,n',n'-tetramethyluronium hexafluorophosphate (HATU, 243.3 mg, 0.64 mmol) and iPr₂NEt (0.3 mL, 1.67 mmol) in CH₂Cl₂ (5 mL) was stirred at rt overnight. The mixture was then diluted with CH₂Cl₂ and washed with water, brine. The organic phase was separated, dried over Na₂SO₄ and filtered. The mixture was then concentrated and purified flash column chromatography (1-100% ethyl acetate/heptane) to afford rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-acetyl-thiophen-3-yl)-amide as a off white powder (40 mg, 15.8 %). HR.MS (ES⁺) m/z Calcd for C₂₉H₂₇Cl₂F₂N₃O₂S + H [(M+H)⁺]: 590.1242, found: 390.1244.

### Example 328

### Preparation of rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-carbamoyl-thiophen-3-yl)-amide

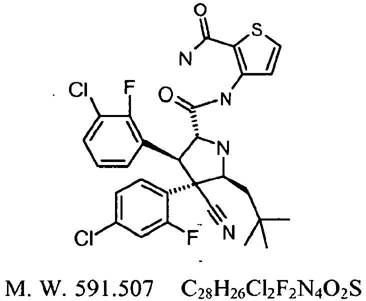

A mixture of rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (200 mg, 0.43 mmol), 3-aminothiophene-2-carboxylic acid amide (160 mg, 1.1 mmol), 2-(7-azabenzotriazol-1-yl)-n,n,n',n'-tetramethyluronium hexafluorophosphate (HATU, 243.3 mg, 0.64 mmol) and iPr₂NEt (0.3 mL, 1.67 mmol) in CH₂Cl₂ (5 mL) was stirred at rt overnight. The mixture was then diluted with CH₂Cl₂ and washed with water, brine. The organic phase was separated, dried over Na₂SO₄ and filtered. The mixture was then concentrated and purified flash column chromatography (1-100% ethyl acetate/heptane) to afford rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-carbamoyl-thiophen-3-yl)-amide as a off white powder (15.8 mg, 6.2 %). HRMS (ES⁺) m/z Calcd for C₂₈H₂₆Cl₂F₂N₄O₂S + H [(M+H)⁺]: 591.1195, found: 591.1191.

### Example 329

### Preparation of rac- (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [1-((S)-3-dimethylamino-2-hydroxy-propyl)-1H-pyrazol-3-yl]-amide

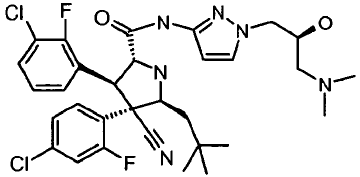

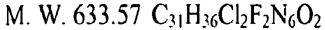

A mixture of rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (200 mg, 0.43 mmol), (*S*)-1-(3-amino-pyrazol-1-yl)-3-dimethylamino-propan-2-ol (158 mg, 0.86 mmol), 2-(7-azabenzotriazol-1-yl)-n,n,n',n'-tetramethyluronium hexafluorophosphate (HATU, 243.3 mg, 0.64 mmol) and iPr₂NEt (0.3 mL, 1.67 mmol) in CH₂Cl₂ (10 mL) was stirred at rt overnight. The mixture was then diluted with CH₂Cl₂ and washed with water, brine. The organic phase was separated, dried over Na₂SO₄ and filtered. The mixture was then concentrated and purified flash column chromatography (1-100% ethyl acetate/heptane) to afford rac- (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [1-((S)-3-dimethylamino-2-hydroxy-propyl)-1H-pyrazol-3-yl]-amide as an off white powder (63.2 mg, 43.6 %). HRMS (ES⁺) m/z Calcd for C₃₁H₃₆Cl₂F₂N₆O₂ + H [(M+H)⁺]: 633.2318, found: 633.2313.

### Example 330

### Preparation of (S)-1-(3-amino-pyrazol-1-yl)-3-dimethylamino-propan-2-ol

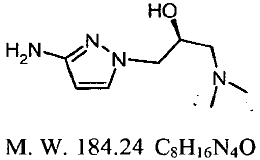

Compound ((*S*)-1-dimethylamino-3-(3-nitro-pyrazol-1-yl)-propan-2-o l was prepared by reacting 3-nitro-1-(R)-1-oxiranylmethyl-1H-pyrazole (0.8 g, 4.73 mmol), isopropyl alcohol (6 mL), and dimethylamine (4 mL, 8 mmol, 2 M in dioxane) under microwave conditions at 130°C for 15 min. The mixture was extracted with dichloromethane and water. The organic layer was separated and the solvent evaporated under reduced pressure to yield (*S*)-1-dimethylamino-3-[1,1-dimethyl-2-(3-nitro-pyrazol-1-yl)-ethoxy]-propan-2-ol (0.76 g, 75.2 %). This compound was reduced under the following conditions-

The mixture of (*S*)-1-dimethylamino-3-[1,1-dimethyl-2-(3-nitro-pyrazol-1-yl)-ethoxy]-propan-2-ol (0.76 g, 3.54 mmol), ethyl acetate (30 mL), ethanol (30 mL) was added to a Parr bottle with 10% Pd/C (0.14 g) and subjected hydrogen gas (20 psi) for 2 h under the Parr Shaker Apparatus conditions. Work up by filtration via a glass membrane filter paper, solvent was removed under reduced pressure to afford (*S*)-1-(3-amino-pyrazol-1-yl)-3-dimethylamino-propan-2-ol as an oil (0.62 g, 96.1 %).

### Example 331

### Preparation of 3-nitro-1-(R)-1-oxiranylmethyl-1H-pyrazole

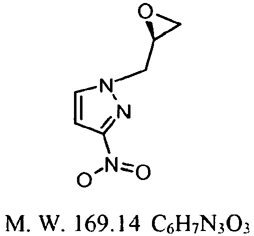

A mixture of 3-nitro-1H-pyrazole (0.76g, 6.72 mmol), (*S*)-(+)-glycidyl-3-nitrobenzenesulfonate (2 g, 7.72 mmol), cesium carbonate (5.6 g, 17.16 mmol) and DMF (30 mL) were stirred at 25°C for 16 hours. The mixture was then diluted with ethyl acetate and water(3x), the organic phase was separated, washed with NaHCO₃(satd) dried with Na₂SO₄, and filtered. The mixture was then concentrated and purified by column chromatography (40-120 g Analogix column, 1-100 % EtOAc/heptane to yield the product 3-nitro-1-(*R*)-1-oxiranylmethyl-1H-pyrazole as a waxy off-white solid (0.89 g, 78.8 %).

### Example 332

### Preparation of rac-(2R,35,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-hydroxycarbamoyl-phenyl)-amide

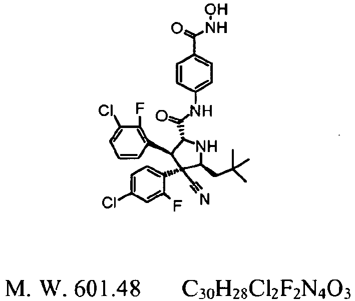

To a solution ofrac 4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}- -benzoic acid prepared in **Example 232** (50 mg, 0.09 mmol) in N,N-dimethylformamide (2 mL) was added NH₂OH.HCl (18 mg, 0.26 mmol), EDCI (33 mg, 0.17 mmol), HOBT(21 mg, 0.15 mmol) and NEt₃ (0.036 mL, 0.26 mmol). The reaction mixture was heated at 80°C for 48 h. The mixture was cooled to room temperature, and partitioned between ethyl acetate and water. The organic layer was separated, and the aqueous layer was extracted with ethyl acetate. The organic layers were combined, washed with water, brine, dried over MgSO₄, and concentrated. The residue was purified by prep-HPLC to give rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-hydroxycarbamoyl-phenyl)-amide as a white solid (24 mg, 47%).

HRMS (ES⁺) m/z Calcd for C₃₀H₂₈Cl₂F₂N₄O₃+ H [(M+H)⁺]: 601.1580, found: 601.1 1577.

### Example 333

### Preparation of rac-(2R,35,4R,5S)-3-(3-chloro-2-fluoro-phenyl)-4-(4-chloro-2-fluoro-phcnyl)-4-cyano-5-(2,2-dimethyl-propyl)-pyrrolidine-2-carboxylic acid (4-methanesulfonylaminocarbonyl-phenyl)-amide

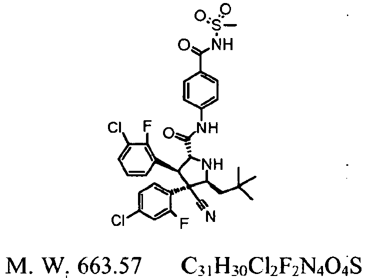

A solution of rac 4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid prepared in **Example 232** (0.14 g, 0.24 mmol) and CDI (97 mg, 0.6 mmol) in N,N-dimethylformamide (5 mL) was heated at 60 °C for 2 h, then to this solution was added a mixture of methanesulfonamide (0.14 g, 1.43 mmol) and NaH (60% in mineral oil, 63 mg, 1.58 mmol), which had been stirred at room temperature for 2 h. The resulting mixture was stirred at room temperature for 1 h, then poured into water. The mixture was acidifed to "pH" 1-2 by addition of aqueous HCl solution, then partitioned between ethyl acetate and water. The organic layer was separated, and the aqueous layer was extracted with ethyl acetate. The organic layers were combined, washed with water, brine, dried over MgSO₄, and concentrated. The residue was purified by chromatography (EtOAc) to give rac-(2R,3S,4R,5S)-3-(3-chloro-2-fluoro-phenyl)-4-(4-chloro-2-fluoro-phenyl)-4-cyano-5-(2,2-dimethyl-propyl)-pyrro lidine-2-carboxylic acid (4-methanesulfonylaminocarbonyl-phenyl)-amide as a white solid (11 mg, 7%). HRMS (ES⁺) m/z Calcd for C₃₁H₃₀Cl₂F₂N₄O₄S+H[(M+H)⁺]663.1406, found: 663.1407.

### Example 336

### Preparation of rac-4- {[(2R,3S,4R,5S)-3-(5-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid methyl ester

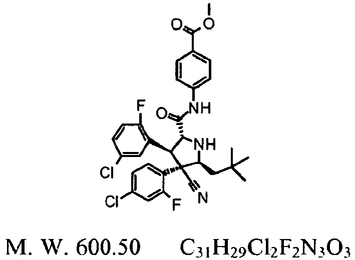

In a manner similar to the method described in **Examples 1e**, rae-(2R,3S,4R,5S)-3-(5-chloro-2-fluoro-phenyl)-4-(4-chloro-2-fluoro-phenyl)-4-cyano-5-(2,2-dimethyl-propyl)-pyrrolidine-2-carboxylic acid trifluoroacetic acid prepared in **Example 98c** (0.5 g, 0.89 mmol) was reacted with methyl 4-aminobenzoate (0.24 g, 1.6 mmol), HATU (0.61 g, 1.6 mmol) and iPr₂NEt (0.39 mL, 2.2 mmol) in CH₂Cl₂ at room temperature to give rac-4-{[(2R,3S,4R,5S)-3-(5-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid methyl ester as a white solid (0.14 g, 27%).

### Example 337

### Preparation of rac-4-{[(2R,3S,4R,5S)-3-(5-chloro-2-fluoro-phenyl)-4-(4-chloro-2-fluoro-phenyl)-4-cyano-5-(2,2-dimethyl-propyl)-pyrrolidine-2-carbonyl]-amino]-benzoic acid

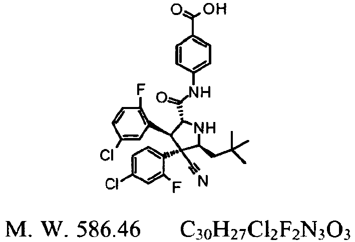

To a solution of rac-4-{[(2R,3S,4R,5S)-3-(5-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid methyl ester prepared in **Example 336** (125 mg, 0.21 mmol) in tetrahydrofuran (3 mL) was added an aqueous solution (1 N) of NaOH (3 mL, 3 mmol) and methanol (1 mL). The reaction mixture was heated at 80 °C for 2 h, and the "pH" of the solution was adjusted to 5 by aqueous HCl solution. The mixture was extracted ethyl acetate twice. The combined organic extracts were washed with water, brine, dried over MgSO₄, and concentrated to give rac-4-{[(2R,3S,4R,5S)-3-(5-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid as a white solid (90 mg, 73%).

### Example 338

### Preparation of rac-4-{[(2R,3R,4R,5S)-3-(3-chloro4-fluoro-phenyl)-4-(4-chloro-2-fluoro-phenyl)-4-cyano-5-(2,2-dimethyl-propyl)-pyrrolidine-2-carbonyl]-amino }-benzoic acid methyl ester

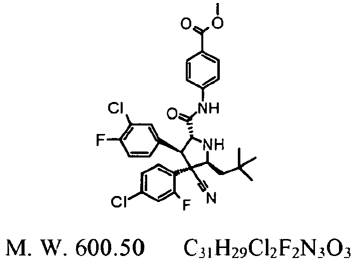

In a manner similar to the method described in **Examples 1e**, rac-(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carboxylic acid trifluoroacetic acid prepared in **Example 69c** (0.25 g, 0.43 mmol) was reacted with methyl 4-aminobenzoate (0.12 g, 0.8 mmol), HATU (0.29 g, 0.43 mmol) and iPr₂NEt (0.19 mL, 1.1 mmol) in CH₂Cl₂ at room temperature to give rac-4-{[(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carbonyl]-amino}-benzoic acid methyl ester as a white solid (0.125 g, 48%).

### Example 339

### Preparation of rac-4-{[(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carbonyl]-amino} -benzo ic acid

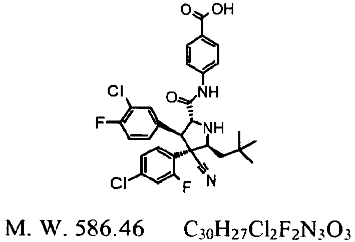

To a solution of rac-4-{[(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carbonyl]-amino}-benzoic acid methyl ester prepared in **Example 338** (0.11 g, 0.18 mmol) in tetrahydrofuran (9 mL) was added an aqueous solution (1 N) of NaOH (9 mL, 9 mmol) and methanol (3 mL). The reaction mixture was heated at 80°C for 2 h, and the "pH" of the solution was adjusted to 5 by aqueous HCl solution. The mixture was extracted ethyl acetate twice. The combined organic extracts were washed with water, brine, dried over MgSO₄, and concentrated to give rac-4-{[(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carbonyl]-amino }-benzoic acid as a white solid (0.1 g, 94%).

### Example 340

### Preparation of rac-4- {[(2R,3R,4R,5S)-3-(3-bromo-phenyl)-4-(4-chloro-2-fluoro-phenyl)-4-cyano-5-(2,2-dimethyl-propyl)-pyrrolidine-2-carbonyl]-amino}-benzoic acid methyl ester

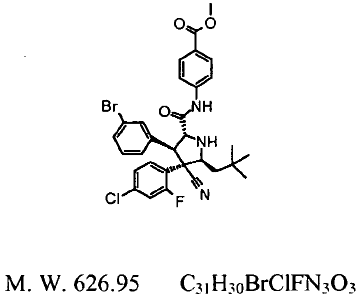

In a manner similar to the method described in **Examples 1e**, rac-(2R,3R,4R,5S)-3-(3-bromophenyl)-4-(4-chloro-2-fluoro-phenyl)-4-cyano-5-(2,2-dimethyl-propyl)-pyrrolidine-2-carboxylic acid trifluoroacetic acid prepared in **Example 66c** (0.44 g, 0.74 mmol) was reacted with methyl 4-aminobenzoate (0.1 g, 1.32 mmol), HATU (0.3 g, 0.4 mmol) and iPr₂NEt (0.32 mL, 1.8 mmol) in CH₂Cl₂ at room temperature to give rac-4-{[(2R,3R,4R,5S)-3-(3-bromo-phenyl)-4-(4-chloro-2-fluoro-phenyl)-4-cyano-5-(2,2-dimethyl-propyl)-pyrrolidine-2-carbonyl]-amino{-benzoic acid methyl ester as a white solid (0.17 g, 37%).

### Example 341

### Preparation of rac-4-{[(2R,3R,4R,5S)-3-(3-bromo-phenyl)-4-(4-chloro-2-fluoro-phenyl)-4-cyano-5-(2,2-dimethyl-propyl)-pyrrolidine-2-carbonyl]-amino}-benzoic acid

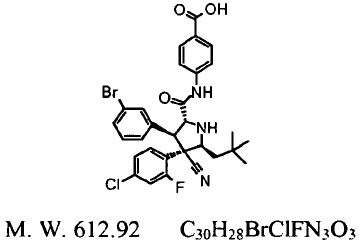

To a solution of rac-4-{[(2R,3R,4R,5S)-3-(3-bromo-phenyl)-4-(4-chloro-2-fluoro-phenyl)-4-cyano-5-(2,2-dimethyl-propyl)-pyrrolidine-2-carbonyl]-amino}-benzoic acid methyl ester prepared in **Example 340** (0.15 g, 0.25 mmol) in tetrahydrofuran (9 mL) was added an aqueous solution (1 N) of NaOH (9 mL, 9 mmol) and methanol (3 mL). The reaction mixture was heated at 80°C for 2 h, and the "pH" of the solution was adjusted to 5 by aqueous HCl solution. The mixture was extracted ethyl acetate twice. The combined organic extracts were washed with water, brine, dried over MgSO₄, and concentrated to give rac-4-{[(2R,3R,4R,5S)-3-(3-bromophenyl)-4-(4-chloro-2-fluoro-phenyl)-4-cyano-5-(2,2-dimethyl-propyl)-pyrrolidine-2-carbonyl]-amino}-benzoic acid as a white solid (90 mg, 60%).

### Example 342

### Preparation of rac-4-{[(2R,3S,4R,5S)-3-(3-chloro-2-fluoro-phenyl)-4-(4-chloro-phenyl)-4-cyano-5-(2,2-dimethyl-propyl)-pyrrolidine-2-carbonyl]-amino}-benzoic acid methyl ester

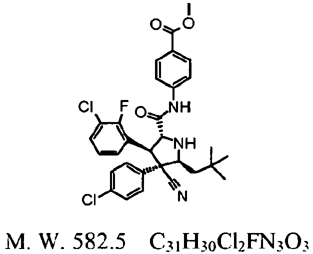

In a manner similar to the method described in **Examples 1e**, rac-(2R,3S,4R,5S)-3-(3-chloro-2-fluoro-phenyl)-4-(4-chloro-phenyl)-4-cyano-5-(2,2-dimethyl-propyl)-pyrrolidine-2-carboxylic acid trifluoroacetic acid prepared in **Example 26c** (0.38 g, 0.55 mmol)was reacted with methyl 4-aminobenzoate (0.33 g, 2.2 mmol), HATU (0.38 g, 1 mmol) and iPr₂NEt (0.29 mL. 1.7 mmol) in CH₂Cl₂ at room temperature to give rac-4-{[(2R,3S,4R,5S)-3-(3-chloro-2-fluoro-phenyl)-4-(4-chloro-phenyl)-4-cyano-5-(2,2-dimethyl-propyl)-pyrrolidine-2-carbonyl]-amino}-benzoic acid methyl ester as a white solid (0.11 g, 34%).

### Example 343

### Preparation of rac-4-{[(2R,3S,4R,5S)-3-(3-chloro-2-fluoro-phenyl)-4-(4-chloro-phenyl)-4-cyano-5-(2,2-dimethyl-propyl)-pyrrolidine-2-carbonyl]-amino]-benzoic acid

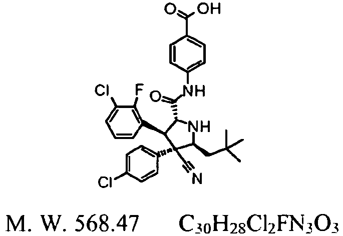

To a solution of rac-4-{[(2R,3S,4R,5S)-3-(3-chloro-2-fluoro-phenyl)-4-(4-chloro-phenyl)-4-cyano-5-(2,2-dimethyl-propyl)-pyrrolidine-2-carbonyl]-amino}-benzoic acid methyl ester prepared in **Example 342** (95 mg, 0.16 mmol) in tetrahydrofuran (6 mL) was added an aqueous solution (1 N) of NaOH (6 mL, 6 mmol) and methanol (2 mL). The reaction mixture was heated at 80°C for 2 h, and the "pH" of the solution was adjusted to 5 by aqueous HCl solution. The mixture was extracted ethyl acetate twice. The combined organic extracts were washed with water, brine, dried over MgSO₄, and concentrated to give rac-4-{[(2R,3S,4R,5S)-3-(3-chloro-2-fluoro-phenyl)-4-(4-chloro-phenyl)-4-cyano-5-(2,2-dimethyl-propyl)-pyrrolidine-2-carbonyl]-amino}-benzoic acid as a white solid (80 mg, 86%).

### Example 344

### Preparation of rac-4- {[(2R,3S,4R,5S)-3-(3-chloro-2-fluoro-phenyl)-4-(4-chloro-2-fluoro-phenyl)-4-cyano-5-(1-methyl-cyclohexylmethyl)-pyrrolidine-2-carbonyl]-amino}-benzoic acid methyl ester

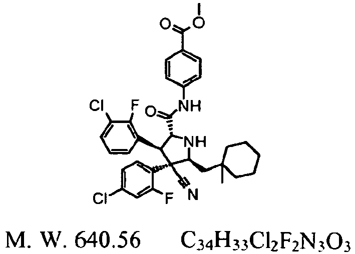

In a manner similar to the method described in **Examples 1e**, rac-(2R,3S,4R,5S)-3-(3-chloro-2-fluoro-phenyl)-4-(4-chloro-2-fluoro-phenyl)-4-cyano-5-(-methyl-cyclohexylmethyl)-pyrrolidine-2-carboxylic acid trifluoroacetic acid prepared in **Example 102c** (0.41 g, 0.66 mmol) was reacted with methyl 4-aminobenzoate (0.2 g, 1.3 mmol), HATU (0.45 g, 1.2 mmol) and iPr₂NEt (0.29 mL, 1.7 mmol) in CH₂Cl₂ at room temperature to give rac-4-{[(2R,3S,4R,5S)-3-(3-chloro-2-fluoro-phenyl)-4-(4-chloro-2-fluoro-phenyl)-4-cyano-5-(1-methylcyclohexylmethyl)-pyrrolidine-2-carbonyl]-amino}-benzoic acid methyl ester as a white solid (0.17 g, 41%).

### Example 345

### Preparation of rac-4-{[(2R,3S,4R,5S)-3-(3-chloro-2-fluoro-phenyl)-4-(4-chloro-2-fluoro-phenyl)-4-cyano-5-(1-methyl-cyclohexylmethyl)-pyrrolidine-2-carbonyl]-amino}-benzoic acid

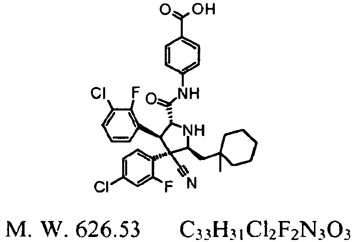

To a solution of rac-4-{[(2R,3S,4R,5S)-3-(3-chloro-2-fluoro-phenyl)-4-(4-chloro-2-fluoro-phenyl)-4-cyano-5-(1-methyl-cyclohexylmethyl)-pyrrolidine-2-carbonyl]-amino}-benzoic acid methyl ester prepared in **Example 344** (0.16 g, 0.24 mmol) in tetrahydrofuran (3 mL) was added an aqueous solution (1N) of NaOH (3 mL, 3 mmol) and methanol (1 mL). The reaction mixture was heated at 80 °C for 2 h, and the "pH" of the solution was adjusted to 5 by aqueous HCl solution. The mixture was extracted ethyl acetate twice. The combined organic extracts were washed with water, brine, dried over MgSO₄, and concentrated to give rac-4-{[(2R,3S,4R,5S)-3-(3-chloro-2-fluoro-phenyl)-4-(4-chloro-2-fluoro-phenyl)4-cyano-5-(1-methylcyclohexylmethyl)-pyrrolidine-2-carbonyl]-amino]-benzoic acid as a white solid (0.14 g, 90%).

### Example 346

### Preparation of rac-[4-(3-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino {-pyrazol-1-ylmethyl)-4-hydroxy-piperidin-1-yl]-acetic acid

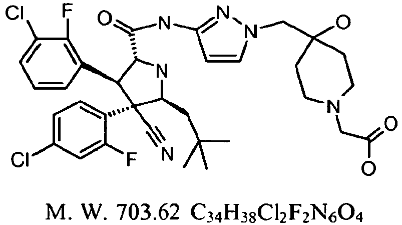

A mixture of rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid [1-(4-hydroxy-piperidin-4-ylmethyl)-1H-pyrazol-3-yl]-amide (80 mg, 0.12 mmol) was dissolved in DMF (3 mL) with cesium carbonate (100 mg, 0.31 mmol) then t-butyl acetate (0.1 mL, 0.677 mmol) was added stirred 16 hours at 25°C. The reaction mixture was diluted with ethyl acetate and separated with water (3x), the organic layer was dried with Na2SO4, filtered and concentrated under reduced pressure. The mixture was purified by reverse phase column chromatography (20-95% acetonitrile/ water) to yield comound [4-(3-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-pyrazol-1-ylmethyl)-4-hydroxy-piperidin-1-yl]-acetic acid tert-butyl ester (68 mg, 74.7%). The compound was taken directly to the deprotection step by dissolved in a solution of 30% TFA in dichloromethane (3 mL) and stirred at room temperature for 16 hours. The mixture was concentrated under reduced pressure then taken up in ethyl acette and NaHCO3(s), separated the organic layer, concentrated under reduced pressure to yield compound rac-[4-(3-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-pyrazol-1-ylmethyl)-4-hydroxy-piperidin-1-yl]-acetic acid (41.2 mg, 65.4 %). HRMS (ES⁺) m/z Calcd for C₃₄H₃₈Cl₂F₂N₆O₄ + H [(M+H)⁺]: 703.2373, found: 703.2376.

### Example 347

Reaction of the core group 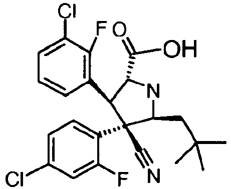 is carried out with fragments 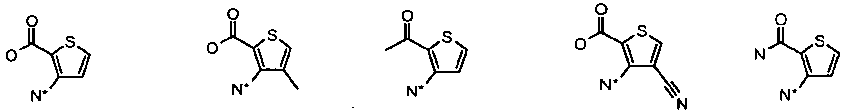 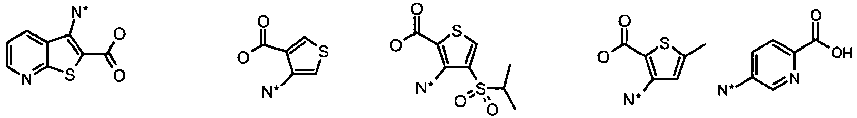 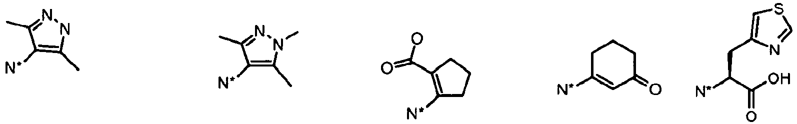 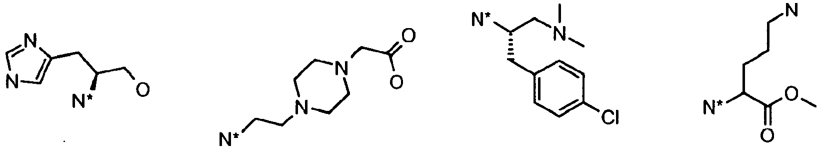 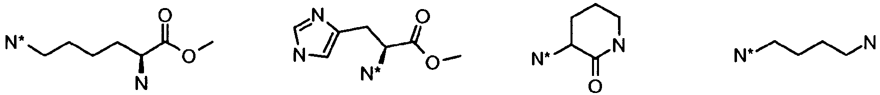 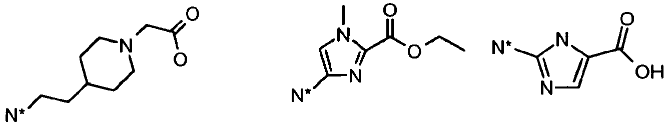 following the reactions set forth in Examples 325, 326 and 327
or via traditional coupling conditions using BOP, HBTU, CDI, DIC, EDCI, of other known methods, including protecting the N-pyrrolidine with BOC for the less reactive fragments.

### Example 348

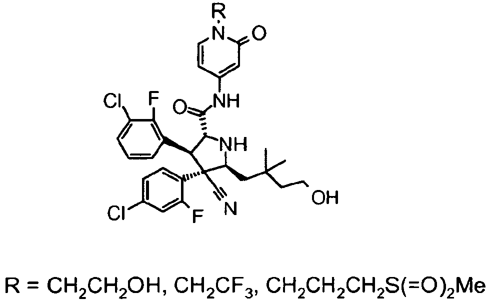

These compounds are prepared in a manner similar to the method described in Example 143 by reaction of 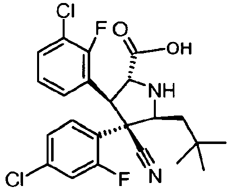 with the following amines 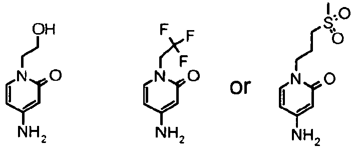 These three starting material can be prepared from 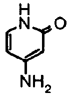 by N-alkylation with the corresponding substituted lower alkyl halide or methanesulfonic aid lower alkyl ester and a base like NaH in DMF.

### Example 349

### In Vitro Activity Assay

The ability of the compounds to inhibit the interaction between p53 and MDM2 proteins was measured by an HTRF (homogeneous time-resolved fluorescence) assay in which recombinant GST-tagged MDM2 binds to a peptide that resembles the MDM2-interacting region of p53 (Lane et al.). Binding of GST-MDM2 protein and p53-peptide (biotinylated on its N-terminal end) is registered by the FRET (fluorescence resonance energy transfer) between Europium (Eu)-labeled anti-GST antibody and streptavidin-conjugated Allophycocyanin (APC).

Test is performed in black flat-bottom 384-well places (Costar) in a total volume of 40 uL containing:90 nM biotinylate peptide, 160 ng/ml GST-MDM2, 20 nM streptavidin-APC (PerkinElmerWallac), 2 nM Eu-labded anti-GST-antibody (PerkinElmerWallac), 0.2% bovine serum albumin (BSA), 1 mM dithiothreitol (DTT) and 20 mM saline (TBS) buffer as follows: Add 10 uL of GST-MDM2 (640 ng/ml working solution) in reaction butter to each well. Add 10 uL diluted compounds (1:5 dilution in reaction buffer) to each well, mix by shaking. Add 20 uL biotinylated p53 peptide (180 nM working solution) in reaction buffer to each well and mix on shaker. Incubate at 37°C for 1 h. Add 20 uL streptavidin-APC and Eu-anti-GST antibody mixture (6 nM Eu-anti-GST and 60 nM streptavidin-APC working solution) in TBS buffer with 0.2% BSA, shake at room temperature for 30 minutes and read using a TRF-capable plate reader at 665 and 615 nm (Victor 5, Perkin ElmerWallac). If not specified, the reagents were purchased from Sigma Chemical Co.

Activity data for some of the Example compounds expressed as IC₅₀ :bsa:0.02% are as follows:

| Example Number | IC₅₀ :bsa:0.02% |
|---|---|
| 7 | 0.309 |
| 14 | 2.38 |
| 43d | 0.603 |
| 69d | 0.192 |
| 83d | 0.165 |

## Claims

1. A compound of the formula 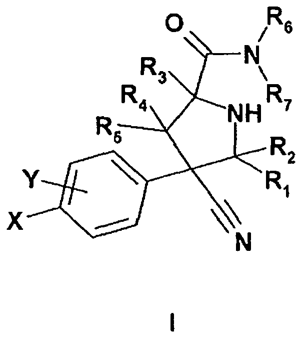 wherein
X is selected from the group consisting of H, F, Cl, Br, I, cyano, nitro, ethynyl, cyclopr-opyl, methyl, ethyl, isopropyl, vinyl and methoxy;
Y is one to four group(s) independently selected from the group consisting of H, F, Cl, Br, I, CN, OH, nitro, C1-6 alkyl, cycloalkyl, C1-6 alkoxy, C2-6 alkenyl, cycloalkenyl, C2-6 alkynyl, aryl, heteroaryl, heterocycle, COOR', OCOR', CONR'R", NR'COR", NR"SO₂R', SO₂NR'R" and NR'R", wherein
R' and R" is independently selected from H, substituted or unsubstituted C1-6 alkyl, substituted or unsubstituted C3-10 cycloalkyl, substituted or unsubstituted C2-6 alkenyl, substituted or unsubstituted C5-10 cycloalkenyl, substituted or unsubstituted aryl, substituted or unsubstituted heteroaryl or substituted or unsubstituted heterocycle; and
in the case of R' and R" may independently link to form a cyclic structure selected from substituted or unsubstituted cycloalkyl, substituted or unsubstituted cycloalkenyl, substituted
or unsubstituted heteroaryl or substituted or unsubstituted heterocycle;
one of R₁ and R₂ is selected from the group consisting of C 1-6 alkyl, substituted C 1-6 alkyl, C2-6 alkenyl, substituted C2-6 alkenyl, aryl, substituted aryl, heteroaryl, substituted heteroaryl, heterocycle, substituted heterocycle, cycloalkyl, substituted cycloalkyl, cycloalkenyl and substituted cycloalkenyl and the other is hydrogen or C1-6 alkyl;
R₃ is H or C1-6 alkyl;
one of R₄ and R₅ is selected from the group consisting of C1-6 alkyl, substituted C1-6 alkyl, C2-6 alkenyl, substituted C2-6 alkenyl, aryl, substituted aryl, heteroaryl, substituted heteroaryl, heterocycle, substituted heterocycle, cycloalkyl, substituted cycloalkyl, cycloalkenyl, and substituted cycloalkenyl and the other is hydrogen;
R₆ and R₇ are selected from the group consisting of (CH₂)ₙ-R', (CH₂)ₙ-NR'R", (CH₂)ₙ-NR'COR", (CH₂)ₙ-NR'SO₂R", (CH₂)ₙ-COOH, (CH₂)ₙ-COOR', (CH₂)ₙ-CONR'R", (CH₂)ₙ-OR', (CH₂)ₙ-SR', (CH₂)ₙ-SOR', (CH₂)ₙ-SO₂R', (CH₂)ₙ-COR', (CH₂)ₙ-SO₃H, (CH₂)ₙ-SON-R'R", (CH₂)ₙ-SO₂NR'R", (CH₂CH₂O)ₘ-(CH₂)ₙ-R', (CH₂CH₂O)ₘ-(CH₂)ₙ₋OH, (CH₂CH₂O)ₘ-(CH₂)ₙ-OR', (CH₂CH₂O)ₘ-(CH₂)ₙ-NR'R", (CH₂CH₂O)ₘ-(CH₂)ₙ-NR'COR", (CH₂CH₂O)ₘ-(CH₂)ₙ-NR'SO₂R", (CH₂CH₂O)ₘ-(CH₂)ₙ-COOH, (CH₂CH₂O)ₘ-(CHl₂)ₙ-COOR', (CH₂CH₂O)ₘ-(CH₂)ₙ-CONR'R", (CH₂CH₂O)ₘ-(CH₂)ₙ-SO₂R', (CH₂CH₂O)ₘ-(CH₂)ₙ-COR', (CH₂CH₂O)ₘ-(CH₂)ₙ-SONR'R", (CH₂CH₂O)ₘ-(CH₂)ₙ-SO₂NR'R", (CH₂)ₚ-(CH₂CH₂O)ₘ-(CH₂)ₙ-R', (CH₂)ₚ-(CH₂CH₂O)ₘ-(CH₂)ₙ-OH, (CH₂)ₚ-(CH₂CH₂O)ₘ-(CH₂)ₙ-OR', (CH₂)ₚ-(CH₂CH₂O)ₘ-(CH₂)ₙ-NR'R", (CH₂)ₚ-(CH₂CH₂O)ₘ-(CH₂)ₙ-NR'COR", (CH₂)ₚ-(CH₂CH₂O)ₙ-(CH₂)ₘNR'SO₂R", (CH₂)ₚ-(CH₂CH₂O)ₘ-(CH₂)ₙ-COOH, (CH₂)ₚ-(CH₂CH₂O)ₘ-(CH₂)ₙ-COOR', (CH₂)ₚ-(CH₂CH₂O)ₘ-(CH₂)ₙ-CONR'R", (CH₂)ₚ-(CH₂CH₂O)ₘ-(CH₂)ₙ-SO₂R', (CH₂)ₚ-(CH₂CH₂O)ₘ-CH₂)ₙ-COR', (CH₂)ₚ-(CH₂CH₂O)ₘ-(CH₂)ₙ-SONR'R", (CH₂)ₚ-(CH₂CH₂O)ₘ-(CH₂)ₙ-SO₂NR'R", -COR', -SOR' and SO₂R' wherein R' and R" are the same definitions as above;
m, n and p are independently 0 to 6;
and the pharmaceutically acceptable salts and esters thereof.

2. A compound of claim 1 of the formula 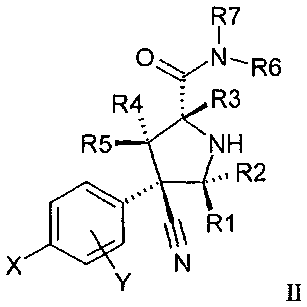 wherein
X is selected from the group consisting of H, F, Cl, Br, I, cyano, nitro, ethynyl, cyclopropyl, methyl, ethyl, isopropyl, vinyl and methoxy;
Y is one to four group(s) independently selected from the group consisting of H, F, Cl, Br, I, CN, OH, nitro, C1-6 alkyl, cycloalkyl, C1-6 alkoxy, C2-6 alkenyl, cycloalkenyl, C2-6 alkynyl, aryl, heteroaryl, heterocycle, COOR', OCOR', CONR'R", NR'COR", NR"SO₂R', SO₂NR'R" and NR'R" wherein
R' and R" are independently selected from H or substituted or unsubstituted C1-6 alkyl, substituted or unsubstituted C3-10 cycloalkyl, substituted or unsubstituted C2-6 alkenyl, substituted or unsubstituted C5-10 cycloalkenyl, substituted or unsubstituted aryl, substituted or unsubstituted heteroaryl or substituted or unsubstituted heterocycle, and wherein R' and R" may independently link to form a cyclic structure selected from substituted or unsubstituted cycloalkyl, substituted or unsubstituted cycloalkenyl, substituted or unsubstituted heteroaryl or substituted or unsubstituted heterocycle;
R₁ is selected from the group consisting of C1-6 alkyl, substituted C1-6 alkyl, C2-6 alkenyl, substituted C2-6 alkenyl, aryl, substituted aryl, heteroaryl, substituted heteroaryl, heterocycle, substituted heterocycle, cycloalkyl, substituted cycloalkyl, cycloalkenyl and substituted cycloalkenyl;
R₂ is hydrogen or C1-6 alkyl;
R₃ is H or C1-6 alkyl;
R₅ is selected from the group consisting of C1-6 alkyl, substituted C1-6 alkyl, C2-6 alkenyl, substituted C2-6 alkenyl, aryl, substituted aryl, heteroaryl, substituted heteroaryl, heterocycle, substituted heterocycle, cycloalkyl, substituted cycloalkyl, cycloalkenyl and substituted cycloalkenyl;
R₄ is hydrogen;
R₆ and R₇ are selected from the group consisting of (CH₂)ₙ-R', (CH₂)ₙ-NR'R", (CH₂)ₙ-NR'COR", (CH₂)ₙ-NR'SO₂R", (CH₂)ₙ-COOH, (CH₂)ₙ-COOR', (CH₂)ₙ-CONR'R", (CH₂)ₙ-OR', (CH₂)ₙ-SR', (CH₂)ₙ-SOR', (CH₂)ₙ-SO₂R', (CH₂)ₙ-COR', (CH₂)ₙ-SO₃H, (CH₂)ₙ-SONR'R", (CH₂)ₙ-SO₂NR'R", (CH₂CH₂O)ₘ-(CH₂)ₙ-R', (CH₂CH₂O)ₘ-(CH₂)ₙ-OH, (CH₂CH₂O)ₘ-(CH₂)ₙ-OR', (CH₂CH₂O)ₘ-(CH₂)ₙ-NR'R", (CH₂CH₂O)ₘ-(CH₂)ₙ-NR'COR", (CH₂CH₂O)ₘ-(CH₂)ₙ-NR'SO₂R", (CH₂CH₂O)ₘ-(CH₂)ₙ-COOH, (CH₂CH₂O)ₘ-(CH₂)ₙ-COOR', (CH₂CH₂O)ₘ-(CH₂)ₙ-CONR'R", (CH₂CH₂O)ₘ,-(CH₂)ₙ-SO₂R', (CH₂CH₂O)ₘ-(CH₂)ₙ-COR', (CH₂CH₂O)ₘ-(CH₂)ₙ-SONR'R", (CH₂CH₂O)ₘ-(CH₂)ₙ-SO₂NR'R", (CH₂)ₚ-(CH₂CH₂O)ₘ-(CH₂)ₙ-R', (CH₂)ₚ-(CH₂CH₂O)ₘ-(CH₂)ₙOH, (CH₂)ₚ-(CH₂CH₂O)ₘ-(CH₂)ₙ-OR', (CH₂)ₚ-(CH₂CH₂O)ₘ-(CH₂)ₙ-NR'R", (CH₂)ₚ-(CH₂CH₂O)ₘ-(CH₂)ₙ-NR'COR", (CH₂)ₚ-(CH₂CH₂O)ₘ-(CH₂)ₙ-NR'SO₂R", (CH₂)ₚ-(CH₂CH₂O)ₘ-(CH₂)ₙ-COOH, (CH₂)ₚ-(CH₂CH₂O)ₘ(CH₂)ₙ-COOR', (CH₂)ₚ-(CH₂CH₂O)ₘ-(CH₂)ₙ-CONR'R", (CH₂)ₚ-(CH₂CH₂O)ₘ-(CH₂)ₙ-SO₂R', (CH₂)ₚ-(CH₂CH₂O)ₘ-(CH₂)ₙ-COR', (CH₂)ₚ-(CH₂CH₂O)ₘ-(CH₂)ₙ-SONR'R", (CH₂)ₚ-(CH₂CH₂O)ₘ-(CH₂)ₙ-SO₂NR'R", -COR', -SOR' and SO₂R' wherein R' and R" are the same definitions as above;
m, n, and p are independently 0 to 6; and
the pharmaceutically acceptable salts and esters thereof.

3. The compound of claim 2 wherein
X is F,Cl or Br;
Y is one to two group(s) independently selected from the group consisting of H, F, Cl, Br, I, CN, OH, nitro, C1-6 alkyl, cycloalkyl, C1-6 alkoxy, C2-6 alkenyl, C5-1 0 cycloalkenyl and C2-6 alkynyl;
R₁ is selected from the group consisting of C1-6 alkyl, substituted C1-6 alkyl, C2-6 alkenyl, substituted C2-6 alkenyl, aryl, substituted aryl, heteroaryl, substituted heteroaryl, heterocycle, substituted heterocycle, cycloalkyl, substituted cycloalkyl, cycloalkenyl and substituted cycloalkenyl;
R₂ is hydrogen;
R₃ is H;
R₅ is selected from the group consisting of aryl, substituted aryl, heteroaryl and substituted heteroaryl;
R₄ is hydrogen;
R₆ and R₇ are selected from the group consisting of (CH₂)ₙ-R', (CH₂)ₙ-NR'R", (CH₂)"-NR'COR", (CH₂)ₙ-NR'SO₂R", (CH₂)ₙ-COOH, (CH₂)ₙ-COOR', (CH₂)ₙ-CONR'R", (CH₂)ₙ-OR', (CH₂)ₙ-SR', (CH₂)ₙ-SOR', (CH₂)ₙ-SO₂R', (CH₂)ₙ-COR', (CH₂)ₙ-SO₃H, (CH₂)ₙ-SONR'R", (CH₂)ₙ-SO₂NR'R", (CH₂CH₂O)ₘ-(CH₂)ₙ-R', (CH₂CH₂O)ₘ-(CH₂)ₙ-OH, (CH₂CH₂O)ₘ-(CH₂)ₙ-OR', (CH₂CH₂O)ₘ-(CH₂)ₙ-NR'R", (CH₂CH₂O)ₘ-(CH₂)ₙ-NR'COR", (CH₂CH₂O)ₘ-(CH₂)ₙ-NR'SO₂R", (CH₂CH₂O)ₘ-(CH₂)ₙ-COOH, (CH₂CH₂O)ₘ-(CH₂)ₙ-COOR', (CH₂CH₂O)ₘ-(CH₂)ₙ-CONR'-R", (CH₂CH₂O)ₘ-(CH₂)ₙ-SO₂R', (CH₂CH₂O)ₘ-(CH₂)ₙ-COR', (CH₂CH₂O)ₘ-(CH₂)ₙ-SONR'R", (CH₂CH₂O)ₘ-(CH₂)ₙ-SO₂NR'R", (CH₂CH₂O)ₘ-(CH₂)ₙ-R', (CH₂)ₚ₋ (CH₂CH₂O)ₘ-(CH₂)ₙ-OH, (CH₂)ₚ-(CH₂CH₂O)ₘ-(CH₂)ₙ-OR', (CH₂)ₚ₋(CH₂CH₂O)ₘ-(CH₂)ₙ-NR'R", (CH₂)ₚ₋(CH₂CH₂O)ₘ-(CH₂)ₙ-NR'COR", (CH₂)ₚ-(CH₂CH₂O)ₘ-(CH₂)ₙ-NR'SO₂R", (CH₂)ₚ-(CH₂CH₂O)ₘ-(CH₂)ₙ-COOH, (CH₂)ₚ-(CH₂CH₂O)ₘ-(CH₂)ₙ-COOR', (CH₂)ₚ-(CH₂CH₂O)ₘ-(CH₂)ₙ-CONR'R", (CH₂)ₚ-(CH₂CH₂O)ₘ-(CH₂)ₙ-SO₂R', (CH₂)ₚ-(CH₂CH₂O)ₘ-(CH₂)ₙ-COR', (CH₂)ₚ-(CH₂CH₂O)ₘ-(CH₂)ₙ-SONR'R", (CH₂)ₚ-(CH₂CH₂O)ₘ-(CH₂)ₙ-SO₂NR'R", -COR', -SOR' and SO₂R', wherein
R' and R" are independently selected from H or substituted or unsubstituted C1-6 alkyl, substituted or unsubstituted C3-10 cycloalkyl, substituted or unsubstituted C2-6 alkenyl, substituted or unsubstituted C5-10 cycloalkenyl, substituted or unsubstituted aryl, substituted or unsubstituted heteroaryl or substituted or unsubstituted heterocycle, and
wherein R' and R" may also independently link to form a cyclic structure selected from substituted or unsubstituted cycloalkyl, substituted or unsubstituted cycloalkenyl, substituted or unsubstituted heteroaryl or substituted or unsubstituted heterocycle;
m, n and p are independently 0 to 6; and
the pharmaceutically acceptable salts and esters thereof.

4. The compound of claim 2 wherein
X is F, Cl or Br;
Y is a mono-substituting group selected from H or F; and
R₁ is selected from the group consisting of C1-6 alkyl, substituted C1-6 alkyl, C2-6 alkenyl, substituted C2-6 alkenyl, heterocycle, substituted heterocycle, cycloalkyl, substituted cycloalkyl, cycloalkenyl and substituted cycloalkenyl .

5. The compound of claim 2 wherein
R₁ is a substituted C1-6 alkyl of the formula 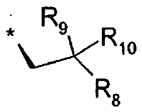 where R₈, R₉ are both methyl or linked to form a cyclopropyl, cyclobutyl, cyclopentyl or a cyclohexyl group;
R₁₀ is (CH₂)ₘ-R₁₁;
m is 0, 1 or 2,
R₁₁ is selected from hydrogen, hydroxyl, C1-6 alkyl, C1-6 alkoxy, aryl, substituted aryl heteroaryl, substituted heteroaryl, heterocycle or substituted heterocycle;
R₂ is H;
R₃ is H;
R₅ is a substituted phenyl selected from 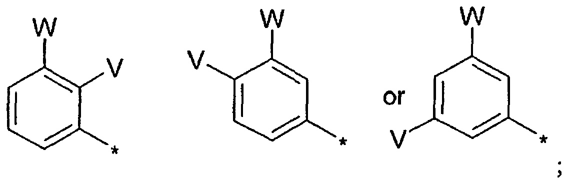 W is F, Cl or Br;
V is H or F;
R₄ is hydrogen;
one of R₆ and R₇ is hydrogen and the other is (CH₂)ₙ-R';
n is 0 or 1; and
R' is selected from aryl, substituted aryl, heteroaryl, substituted heteroaryl, heterocycle or substituted heterocycle.

6. A compound of claim 1 selected from the group consisting of
rac-(2R,3R,4R,5S)-3-(3-chloro-phenyl)-4-(4-chloro-phenyl)-4-cyano-5-(2,2-dimethyl-propyl)-pyrrolidine-2-carboxylic acid (2-morpholin-4-yl-ethyl)-amide,
(2R,3R,4R,5S)-3-(3-chloro-phenyl)-4-(4-chloro-phenyl)-4-cyano-5-(2,2-dimethyl-propyl)-pyrrolidine-2-carboxylic acid (2-morpholin-4-yl-ethyl)-amide,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid dimethylamide,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid ((s)-3,4-dihydroxy-butyl)-amide, (2R,3R,4R,5S)-3-(3-chloro-phenyl)-4-(4-chloro-phenyl)-4-cyano-5-(2,2-dimethyl-propyl)-pyrrolidine-2-carboxylic acid ((S)-3,4-dihydroxy-butyl)-amide,
rac-(2S,3R,4R,5R)-4-(3-chloro-phenyl)-3-(4-chloro-phenyl)-2-(2,2-dimethyl-propyl)-5-[4-(2-morpholin-4-yl-2-oxo-ethyl)-piperazine-1-carbonyl]-pyrrolidine-3-carbonitrile,
rac-(2S,3R,4R,5R)-4-(3-chloro-phenyl)-3-(4-chloro-phenyl)-2-(2,2-dimethyl-propyl)-5-[4-(2-oxo-2-pyrrolidin-1-yl-ethyl)-piperazine-1-carbonyl]-pyrrolidine-3-carbonitrile,
rac-(2S,3R,4R,5R)-4-(3-chloro-phenyl)-3-(4-chloro-phenyl)-2-(2,2-dimethyl-propyl)-5-[4-(2-hydroxy-ethyl)-piperazine-1-carbonyl]-pyrrolidine-3-carbontrile,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid (4-hydroxy-butyl)-amide,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid (2-pyrrolidin-1-yl-ethyl)-amide,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid (2-piperazin-1-yl-ethyl)-amide,
(S)-2-{[(2R,3R,4R,5S)-3-(3-chloro-phenyl)-4-(4-chloro-phenyl)-4-cyano-5-(2,2-dimethylpropyl)-pyrrolidine-2-carbonyl]-amino}-3-methyl-butyric acid methyl ester,
(S)-2-{[(2R,3R,4R,5S)-3-(3-chloro-phenyl)-4-(4-chloro-phenyl)-4-cyano-5-(2,2-dimethylpropyl)-pyrrolidine-2-carbonyl]-amino}-3-methyl-butyric acid,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid (1-hydroxymethyl-cyclopropylmethyl)-amide,
rac-(2R,3R,4R,5S)-3-(3-Chloro-phenyl)-4-(4-chloro-plenyl)-4-cyano-5-(2,2-dimethyl-propyl)-pyrrolidine-2-carboxylic acid (1-hydroxymethyl-cyclobutylmethyl)-amide,
rac-(2R,3R,4R,5S)-3-(3-chloro-phenyl)-4-(4-chloro-phenyl)-4-cyano-5-(2,2-dimethyl-propyl) pyrrolidine-2-carboxylic acid (3,3-dimethyl-butyl)-amide,
rac-(2R,3R,4R,5S)-3-(3-chloro-phenyl)-4-(4-chloro-phenyl)-4-cyano-5-(2,2-dimethyl-propyl)-pyirolidine-2-carboxylic acid (2,2-dimethyl-propyl)-amide,
(2S,3R,4R,5R)-4-(3-chloro-phenyl)-3-(4-chloro-phenyl)-2-(2,2-dimethyl-propyl)-5-((S)-2-hydroxymethyl-pyrrolidine-1-carbonyl) pyrrolidine-3-carbonitrile,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid 2-(3,4-dimethoxy-phenyl)ethyl amide,
(2S,3S,4S,5R)-3-(3-chloro-phenyl)-4-(4-chloro-phenyl)-4-cyano-5-(2,2-dimethyl-propyl)-pyrrolidine-2-carboxylic acid ((R)-1-hydroxymethyl-3-methyl-butyl)-amide,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid (3-hydroxy-propyl)-amide,
rac-(2R,3S,4R,5S)-3-(3-chloro-2-fluoro-phenyl)-4-(4-chloro-phenyl)-4-cyano-5-(2,2-dimethylpropyl)-pyrrolidine-2-carboxylic acid [2-(cis-2,6-dimethyl-morpholin-4-yl)-ethyl]-amide,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid (2-cyclopropyl-ethyl)-amide,
rac-(3-{[(2R,3R,4R,5S)-3-(3-chloro-phenyl)-4-(4-chloro-phenyl)-4-cyano-5-(2,2-dimethylpropyl)-pyrrolidine-2-carbonyl]-amino}-propyl)-carbamic acid tert-butyl ester,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid (3-amino-propyl)-amide,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid [3-(1-acetyl-piperidin-4-ylamino)-propyl]-amide,
rac-(2R,3R,4R,5S)-3-(3-chloro-phenyl)-4-(4-chloro-phenyl)-4-cyano-5-isobutyl-pyrrolidine-2-carboxylic acid (3-hydroxy-propyl)-amide,
rac-(2R,3R,4R,5R)-5-(3-chloro-phenyl)-4-(4-chloro-phenyl)-4-cyano-3-(2,2-dimethyl-propyl)-pyrrolidine-2-carboxylic acid (3-hydroxy-propyl)-amide,
rac-(2R,3R,4R,5S)-3-(3-chloro-phenyl)-4-(4-chloro-phenyl)-4-cyano-5-(2,2-dimethyl-propyl)-2-methyl-pyrrolidine-2-carboxylic acid (3-hydroxy-propyl)-amide,
rac-(2R,3S,4R,5S)-3-(3-chloro-2-fluoro-phenyl)-4-(4-chloro-phenyl)-4-cyano-5-cyclopentylmethyl-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-phenyl)-4-cyano-5-(2,2-dimethylpropyl)-pyrrolidine-2-carboxylic acid (2-hydroxy-1,1-dimethyl-ethyl)-amide,
rac-(2R,3S,4R,5S)-3-(3-chloro-2-fluoro-phenyl)-4-(4-chloro-phenyl)-4-cyano-5-(2,2-dimethylpropyl)-pyrrolidine-2-carboxylic acid (3-hydroxy-2,2-dimethyl-propyl)-amide,
rac-(2R,3S,4R,5S)-3-(3-chloro-2-fluoro-phenyl)-4-(4-chloro-phenyl)-4-cyano-5-(2,2-dimethylpropyl)-pyrrolidine-2-carboxylic acid [2-(2-hydroxy-ethoxy)-ethyl]-amide,
rac-(2R,3S,4R,5S)-3-(3-chloro-2-fluoro-phenyl)-4-(4-chloro-phenyl)-4-cyano-5-(2,2-dimethylpropyl)-pyrrolidine-2-carboxylic acid (2-acetylamino-ethyl)-amide,
rac-(2R,3S,4R,5S)-3-(3-chloro-2-fluoro-phenyl)-4-(4-chloro-phenyl)-4-cyano-5-(2,2-dimethylpropyl)-pyrrolidine-2-carboxylic acid (3-imidazol-1-yl-propyl)-amide,
rac-(2R,3S,4R,5S)-3-(3-chloro-2-fluoro-phenyl)-4-(4-chloro-phenyl)-4-cyano-5-(2,2-dimethylpropyl)-pyrrolidine-2-carboxylic acid ((R)-4-hydroxy-3-methyl-butyl)-amide,
rac-(2R,3S,4R,5S)-3-(3-chloro-2-fluoro-phenyl)-4-(4-chloro-phenyl)-4-cyano-5-(2,2-dimethylpropyl)-pyrrolidine-2-carboxylic acid cyclopropylmethoxy-amide,
rac-(2R,3R,4R,5S)-3,5-bis-(3-chloro-phenyl)-4-(4-chloro-phenyl)-4-cyano-pyrrolidine-2-carboxylic acid [2-((S)-2,2-dimethyl-[1,3]dioxolan-4-yl)-ethyl]-amide,
rac-(2R,3R,4R,5S)-3,5-bis-(3-chloro-phenyl)-4-(4-chloro-phenyl)-4-cyano-pyrrolidine-2-carboxylic acid ((S)-3,4-dihydroxy-butyl)-amide,
rac-(2R,3R,4R,5S)-3-(3-chloro-phenyl)-4-(4-chloro-phenyl)-4-cyano-5-(1-ethyl-propyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-phenyl)-4-cyano-5-(2,2-dimethy)-propyl)-pyrrolidine-2-carboxylic acid ((S)-3,4-dihydroxy-butyl)-amide,
(2R,3S,4R,5S)-3-(3-chloro-2-fluoro-phenyl)-4-(4-chloro-phenyl)-4-cyano-5-(2,2-dimethylpropyl)-pyrrolidine-2-carboxylic acid ((S)-3,4-dihydroxy-butyl)-amide,
rac-(2R,3R,4R,5S)-3-(3-chloro-phenyl)-4-(4-chloro-phenyl)-4-cyano-5-isobutyl-pyrrolidine-2-carboxylic acid ((S)-3,4-dihydroxy-butyl)-amide,
rac-(2R,3R,4R,5R)-3-(3-chloro-phenyl)-4-(4-chloro-phenyl)-4-cyano-5-isobutyl-pyrrolidine-2-carboxylic acid ((S)-3,4-dihydroxy-butyl)-amide,
rac-(2R,3R,4R,5R)-3-(3-chloro-phenyl)-4-(4-chloro-phenyl)-4-cyano-5-(1-ethyl-propyl)-pyrrolidine-2-carboxylic acid ((S)-3,4-dihydroxy-butyl)-amide,
rac-(2R,3R,4R)-3-(3-chloro-phenyl)-4-(4-chloro-phenyl)-4-cyano-5,5-diethyl-pyrrolidine-2-carboxylic acid ((S)-3,4-dihydroxy-butyl)-amide,
rac-(2R,3R,4R,5S)-3-(3-chloro-phenyl)-4-(4-chloro-phenyl)-4-cyano-5-isopropyl-pyrrolidine-2-carboxylic acid ((S)-3,4-dihydroxy-butyl)-amide,
rac-(2R,3R,4R,5S)-3-(3-chloro-phenyl)-4-(4-chloro-phenyl)-4-cyano-5-cyclohexyl-pyrrolidine-2-carboxylic acid ((S)-3,4-dihydroxy-butyl)-amide,
rac-(2R,3S,4R,5S)-5-tert-butyl-3-(3-chloro-2-fluoro-phenyl)-4-(4-chloro-phenyl)-4-cyanopyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide and
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide,
rac-(2S,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide,
rac-(2R,3S,4R,5S)-4-(4-bromo-phenyl)-3-(3-chloro-2-fluoro-phenyl)-4-cyano-5-(2,2-dimethylpropyl)-pyrrolidine-2-carboxylic acid ((S)-3,4-dihydroxy-butyl)-amide,
rac-(2R,3S,4R,5S)-3-(3-chloro-2-fluoro-phenyl)-4-cyano-5-(2,2-dimethyl-propyl)-4-(4-fluoro-phenyl)-pyrrolidine-2-carboxylic acid ((S)-3,4-dihydroxy-butyl)-amide,
rac-(2R,3S,4R,5S)-3-(3-chloro-2-fluoro-phenyl)-4-cyano-4-(2,4-dichloro-phenyl)-5-(2,2-dimethyl-propyl)-pyrrolidine-2-carboxylic acid ((S)-3,4-dihydroxy-butyl)-amide,
(2R,3S,4R,5S)-3-(3-chloro-2-fluoro-phenyl)-4-cyano-4-(2,4-dichloro-phenyl)-5-(2,2-dimethylpropyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-methyl-phenyl)-4-cyano-5-(2,2-dimethyl-propyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-phenyl)-4-cyano-5-cyclohexylmethyl-pyrrolidine-2-carboxylic acid ((S)-3,4-dihydroxy-butyl)-amide,
rac-(2S,3S,4R,5S)-3-(3-chloro-2-fluoro-phenyl)-4-(4-chloro-phenyl)-4-cyano-5-cyclohexylmethyl-pyrrolidine-2-carboxylic acid ((S)-3,4-dihydroxy-butyl)-amide,
rac-(2R,3S,4R,5S)-4-(4-chloro-2-fluoro-phenyl)-4-cyano-3-(2,3-difluoro-phenyl)-5-(2,2-dimethyl-propyl)-pyrrolidine-2-carboxylic acid ((S)-3,4-dihydroxy-butyl)-amide,
(2R,3S,4R,5S)-4-(4-chloro-2-fluoro-phenyl)-4-cyano-3-(2,3-difluoro-phenyl)-5-(2,2-dimethylpropyl)-pyrrolidine-2-carboxylic acid ((S)-3,4-dihydroxy-butyl)-amide,
rac-(2R,3S,4R,5S)-3-(3-bromo-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide and
(2R,3S,4R,5S)-3-(3-bromo-2-fluoro-phenyl)-4-(4-chloro-2-fluoro-phenyl)-4-cyano-5-(2,2-diinethyl-propyl)-pyrrolidine-2-carboxylic acid ((S)-3,4-dihydroxy-butyl)-amide,
rac-(2R,3R,4R,5S)-4-(4-chloro-2-fluoro-phenyl)-3-(3-chloro-phenyl)-4-cyano-5-(2,2-dimethylpropyl)-pyrrolidine-2-carboxylic acid ((S)-3,4-dihydroxy-butyl)-amide,
(2R,3R,4R,5S)-4-(4-chloro-2-fluoro-phenyl)-3-(3-chloro-phenyl)-4-cyano-5-(2,2-dimethylpropyl)-pyrrolidine-2-carboxylic acid ((S)-3,4-dihydroxy-butyl)-amide,
rac-(2R,3R,4R,5S)-4-(4-chloro-2-fluoro-phenyl)-4-cyano-5-(2,2-dimethyl-propyl)-3-(3-fluoro-phenyl)-pyrrolidine-2-carboxylic acid ((S)-3,4-dihydroxy-butyl)-amide,
rac-(2R,3R,4R,5S)-3-(3-bromo-phenyl)-4-(4-chloro-2-fluoro-phenyl)-4-cyano-5-(2,2-dimethylpropyl)-pyrrolidine-2-carboxylic acid ((S)-3,4-dihydroxy-butyl)-amide,
rac-(2S,3R,4R,5S)-3-(3-bromo-phenyl)-4-(4-chloro-2-fluoro-phenyl)-4-cyano-5-(2,2-dimethylpropyl)-pyrrolidine-2-carboxylic acid ((S)-3,4-dihydroxy-butyl)-amide,
rac-(2R,3R,4R,5S)-4-(4-chloro-2-fluoro-phenyl)-4-cyano-3-(3,4-dichloro-phenyl)-5-(2,2-dimethyl-propyl)-pyrrolidine-2-carboxylic acid ((S)-3,4-dihydroxy-butyl)-amide
rac-(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide,
(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide,
rac-(2R,3R,4R,5S)-3-(4-bromo-thiophen-2-yl)-4-(4-chloro-2-fluoro-phenyl)-4-cyano-5-(2,2-dimethyl-propyl)-pyrrolidine-2-carboxylic acid ((S)-3,4-dihydroxy-butyl)-amide,
rac-(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carboxylic acid ((R)-3,4-dihydroxy-butyl)-amide,
(2R,3S,4R,5S)-3-(3-chloro-2-fluoro-phenyl)-4-(4-chloro-2-fluoro-phenyl)-4-cyano-5-(2,2,3-trimethyl-butyl)-pyrrolidine-2-carboxylic acid ((R)-3,4-dihydroxy-butyl)-amide,
(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carboxylic acid ((R)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((R)-3,4-dihydroxy-butyl)-amide,
(2R,3S,4R,5S)-3-(3-chlor-o-2-fluoro-phenyl)-4-(4-chloro-2-fluoro-phenyl)-4-cyano-5-(2,2-dimethyl-propyl)-pyrrolidine-2-carboxylic acid ((R)-3,4-dihydroxy-butyl)-amide,
rac-(2R,3R,4R,55)-4-(4-chloro-2-fluoro-phenyl)-3-(3-chloro-phenyl)-4-cyano-5-(2,2-dimethylpropyl)-pyrrolidine-2-carboxylic acid ((R)-3,4-dihydroxy-butyl)-amide,
rac-(2R,3S,4R,5S)-3-(3-chloro-2-fluoro-phenyl)-4-(4-chloro-phenyl)-4-cyano-5-(2,2-dimethylpropyl)-pyrrolidine-2-carboxylic acid ((R)-3,4-dihydroxy-butyl)-amide,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid ((R)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(2,2,3-trimethyl-butyl)-pyrrolidine-2-carboxylic acid ((S)-3,4-dihydroxy-butyl)-amide,
(2R,3S,4R,5S)-3-(3-chloro-2-fluoro-phenyl)-4-(4-chloro-2-fluoro-phenyl)-4-cyano-5-(2,2,3-trimethyl-butyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(2,2,3-trimethyl-butyl)-pyrrolidine-2-carboxylic acid ((R)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(2,2-dimethyl-but-3-enyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(2,2-dimethyl-butyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(2-methyl-2-phenyl-propyl)-pyrrolidine-2-carboxylic acid ((S)-3,4-dihydroxy-butyl)-amide,
rac-(2R,3S,4R,5S)-4-(4-chloro-2-fluoro-phenyl)-3-(5-chloro-2-methoxy-phenyl)-4-cyano-5-(2,2-dimethyl-propyl)-pyrrolidine-2-carboxylic acid ((S)-3,4-dihydroxy-butyl)-amide,
rac(2R,3R,4R,5S)-3-(3-chloro-phenyl)-4-(4-chloro-phenyl)-4-cyano-5-(2,2-dimethyl-propyl)-pyrrolidine-2-carboxylic acid (quinolin-3-ylmethyl)-amide,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid 4-hydroxy-benzylamide,
rac-(2R,3R,4R,5S)-3-(3-chloro-phenyl)-4-(4-chloro-phenyl)-4-cyano-5-(2,2-dimethyl-propyl)-pyrrolidine-2-carboxylic acid (2-ethyl-butyl)-amide,
rac-(2R,3R,4R,5S)-3-(3-chloro-5-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide,
rac-(2R,3S,4R,5S)-4-(4-chloro-2-fluoro-phenyl)-3-[5-chloro-2-(2-hydroxy-ethoxy)-phenyl]-4-cyano-5-(2,2-dimethyl-propyl)-pyrrolidine-2-carboxylic acid ((S)-3,4-dihydroxy-butyl)-amide,
rac-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-piperidine-1-carboxylic acid tert-butyl ester,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid piperidin-4-ylamide trifluoroacetic acid,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-methanesulfonylpiperidin-4-yl)-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-methyl-carbonyl-piperidin-4-yl)-amide,
rac-(2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (1-benzoyl-piperidin-4-yl)-amide,
rac-4- {[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-piperidine-1-carboxylic acid isopropylamide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [1-(2-hydroxy-2-methyl-propyl)-1H-pyrazol-3-yl]-amide,
chiral 2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [1-(2-hydroxy-2-methyl-propyl)-1H-pyrazol-3-yl]-amide,
chiral (2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid [1-(2-hydroxy-2-methyl-propyl)-]1H-pyrazol-3-yl]-amide,
rac-(2R,3 S,4R, 5S)-3-(3-chloro-2-fluoro-phenyl)-4-(4-chloro-2-fluoro-phenyl)-4-cyano-5-(2,2-dimethylpropylpropyl)-pyrrolidine-2-carboxylic acid {1-[2-methyl-2-((R)-1-oxiranylmethoxy)-propyl]-1H-pyrazol-3-yl}-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((*R*)-3-amino-2-hydroxy-propoxy)-2-methyl-propyl]-1H-pyrazol-3-yl}-amide,
rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-{2-[(*R*)-2-hydroxy-3-(3-hydroxypropylamino)-propoxy]-2-methyl-propyl}-1H-pyrazol-3-yl)-amide,
rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-{2-[(*R*)-2-hydroxy-3-(2-hydroxy-1-hydroxymethyl-ethylamino)-propoxy]-2-methyl-propyl}-1H-pyrazol-3-yl)-amide,
rac-(2*R*,3*S*,4*R*,5*S*)-3-(3-chloro-2-fluoro-phenyl)-4-(4-chloro-2-fluoro-phenyl)-4-cyano-5-(2,2-dimethylpropyl)-pyrrolidine-2-carboxylic acid {1-[2-methyl-2-((*S*)-1-oxiranylmethoxy)-propyl]-1H-pyrazol-3-yl}-amide,
rac-(2*R*,3*S*,4*R*,5*S*)-3-(3-chloro-2-fluorophenyl)-4-(4-chloro-2-fluoro-phenyl)-4-cyano-5-(2,2-dimethyl-propyl)-pyrrolidine-2-carboxylic acid {1-[2-((*S*)-3-amino-2-hydroxy-propoxy)-2-methyl-propyl]-1H-pyrazol-3-yl}-amide,
rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((*S*)-2,3-dihydroxy-propoxy)-2-methylpropyl]-1H-pyrazol-3-yl}-amide,
of rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((*S*)-3-dimethylamino-2-hydroxypropoxy)-2-methyl-propyl]-1H-pyrazol-3-yl)-amide,
rac-{(*S*)-3-[2-(3-{[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-pyrazol-1-yl)-1,1-dimethylethoxy]-2-hydroxy-propylamino}-acetic acid,
rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)pyrrolidine-2-carboxylic acid {1-[2-((*S*)-2-hydroxy-3-methylamino-propoxy)-2-methyl-propyl]-1H-pyrazol-3-yl}-amide,
rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((*R*)-3-dimethylamino-2-hydroxy-propoxy)-2-methyl-propyl]-1H-pyrazol-3-yl)-amide,
rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid [1-((*R*)-2,3-dihydroxy-propyl)-1H-pyrazol-3-yl]-amide,
rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid [1-(2-hydroxy-ethyl-1H-pyrazol-3-yl]-amide,
rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid ((S)-2,3-dihydroxy-propyl)-amide,
rac- (2*S*,3*S*,4*S*,5*R*)-3-(3-chloro-phenyl)-4-(4-chloro-phenyl)-4-cyano-5-(2,2-dimethyl-propyl)-pyrrolidine-2-carboxylic acid ((*S*)-2,3-dihydroxy-propyl)-amide,
rac- (2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid [1-{(*R*)-2,2-dimethyl-[1,3]dioxolan-4-ylmethyl)-1H-pyrazol-3-yl]-amide,
rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid 2-trifluoromethyl-benzylamide,
rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid 4-(2-oxo-pyrrolidin-1-yl)-benzylamide,
rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (tetrahydro-pyran-4-ylmethyl)-amide,
rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (3-hydroxy-2-hydroxymethyl-propyl)-amide,
rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid [2-(2-amino-ethoxy)-ethyl]-amide,
rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (3-methanesulfonyl-propyl)-amide,
rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (2-methanesulfonyl-ethyl)-amide,
rac-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-cyclohexylamino-1-carboxylic acid tert-butyl ester,
rac-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-trans-cyclohexylamine trifluoroacetic acid salt,
rac-4-{([(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-trans-cyclohexyl-N-methanesulfonamide, rac-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-trans-cyclohexyl-N-methanesulfonamide, rac-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-trans-cyclohexyl-(1,1-dioxo)-2-isothiazolidine, rac-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-trans-cyclohexyl-urea,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid N-[1-(2-hydroxy ethyl)-piperidin-4-yl]amide, rac-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-piperidine-1-sulfonic acid amide, rac 3-{4-[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-piperazin-1-yl}-acetic acid,
rac 3-{4-[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-piperazin-1-yl}-N,N-bis-(2-methoxy-ethyl)-acetamide,
rac3-{4-[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-piperazin-1-yl}-N,N-bis-(2-hydroxy-ethyl)-acetamide,
rac 3-{4-[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-piperazin-1-yl}-N-(3-methoxy-propyl)-acetamide,
rac 2-{4-[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-piperazin-1-yl)-acetamide,
rac (2S,3R,4S,5R)-4-(3-chloro-2-fluoro-phenyl)-3-(4-chloro-2-fluoro-phenyl)-2-(2,2-dimethylpropyl)-5-[4-(2-morpholin-4-yl-2-oxo-ethyl)-piperazine-1-carbonyl]-pyrrolidine-3-carbontrile, rac 2-{4-[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-piperazin-1-yl}-N-[2-((S)-2,2-dimethyl-[1,3]dioxolan-4-yl)-ethyl]-acetamide,
rac 2-{4-[(2R,3S,4R,5S)-3-(3-chloro-2-fluoro-phenyl)-4-(4-chloro 2-fluoro-phenyl)-4-cyano-5-(2,2-dimethyl-propyl)-pyrrolidine-2-carbonyl]-piperazin-1-yl}-N-((S)-3,4-dihydroxy-butyl)-acetamide,
rac {1-[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-piperidin-4-yl}-acetic acid methyl ester, and
rac {1-[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-piperidin-4-yl}-acetic acid hydrochloride salt.

7. A compound of claim 1 selected from the group consisting of
rac 2- {1-[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-piperidin-4-yl}-acetamide,
rac 2-{1-[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-piperidin-4-yl}-N,N-bis-(2-hydroxy-ethyl)-acetamide,
rac 2-{1-[(2R,3S,4R,5S)-3-(3-chloro-2-fluoro-phenyl)-4-(4-chloro2-fluoro-phenyl)-4-cyano-5-(2,2-dimethyl-propyl)-pyrrolidine-2-carbonyl]-piperidin-4-yl}-N-(2-hydroxy-ethyl)-N-methylacetamide,
rac 2-{1-[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-piperidin-4-yl}-N-(2-hydroxy-propyl)-acetamide, rac {[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-acetic acid tert-butyl ester,
rac {[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-acetic aid trifluoro acetic acid salt,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid carbamoylmethyl-amide, {[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-acetic aid trifluoro acetic acid salt,
rac 3-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid ethyl ester,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (3-carbamoyl-phenyl)-amide,
rac 3-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid tert-butyl ester,
rac 3-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (3-hydroxytnethyl-phenyl)-amide,
rac (3-{[{2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-propyl)-carbamic acid tert-butyl ester,
rac (2R,35,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (3-amino-propyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrroiidine-2-carboxylic acid [3-(aminosulfonyl-amino)-propyl)-amide,
rac 2-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid tert-butyl ester,
rac 4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid tert-butyl ester,
rac 4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid ethyl ester,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-carbamoyl-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-carbamoyl-phenyl)-amide,
rac 2-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid ethyl ester,
rac 4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid,
rac 2-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid ,
rac (2R,3S,4R,5S)-3-(3-chloro-2-fluoro-phenyl)-4-(4-chloro-2-fluoro-phenyl)-4-cyano-5-(2,2-dimetliyl-propyl)-pyrrolidine-2-carboxylic acid (3-cyano-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-hydroxy-2-methyl-propyl)-amide,
rac 3-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid 2-hydroxy-2-methyl-propyl ester,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (3-methanesulfonylamino-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (1H-tetrazol-5-ylmethyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (3-ureado-propyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (3-methylsulfanyl-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (3-methanesulfonyl-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (3-methanesulfinyl-phenyl)-amide,
3-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid,
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (3-carbamoyl-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [3-(1H-tetrazol-5-yl)-phenyl]-amide,
rac (2R,3S,4R,5S)-3-(3-chloro-2-fluoro-phenyl)-4-(4-chloro-2fluoro-phenyl)-4-cyano-5-(2,2-dimethyl-propyl)-pyrrolidine-2-carboxylic acid [4-(1H-tetrazol-5-yl)-phenyl]-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-amino-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-acetylamino-phenyl)-amide,
rac 2-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl)-amino}-thiazole-4-carboxylic acid ethyl ester, rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (1,3-dioxo-2,3-dihydro-1H-isoindol-5-yl)-amide, rac (2R,3S,4R,5S)-3-(3-chloro-2-fluoro-phenyl)-4-(4-choro-2-fluoro-phenyl)-4-cyano-5-(2,2-dimethyl-propyl)-pyrrolidine-2-carboxylic acid (6-oxo-1,6-dihydro-pyridin-3-yl)-amide, (2R,3S,4R,5S)-3-(3-chloro-2-fluoro-phenyl)-4-(4-chloro-2-fluor-o-phenyl)-4-cyano-5-(2,2-dimethyl-propyl)-pyrrolidine-2-carboxylic acid (6-oxo-1,6-dihydro-pyridin-3-yl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-methylsulfanyl-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-methanesulfonyl-phenyl)-amide,
4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid teu-butyl ester,
4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid,
4- {[(2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid,
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-carbamoyl-phenyl)-amide,
(2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-carbamoyl-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-methanesulfonylamino-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-trifluoro-methanesulfonylamino-phenyl)-amide,
rac (2R,3R,4R,5S)-4-(4-chloro-2-fluoro-phenyl)-3-(3-chloro-2-methyl-phenyl)-4-cyano-5-(2,2-dimethyl-propyl)-pyrrolidine-2-carboxylic acid [3-(1-methyl-1H-tetrazol-5-yl)-phenyl]-amide,
rac (2R,3R,4R,4S)-4-(4-chloro-2-fluoro-phenyl)-3-(3-chloro-2-methyl-phenyl)-4-cyano-5-(2,2-dimethyl-propyl)-pyrrolidine-2-carboxylic acid [3-(2-methyl-1H-tetrazol-5-yl)-phenyl]-amide,
(2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid (6-oxo-1,6-dihydro-pyridin-yl)-amide,
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(1H-tetrazol-5-yl)-phenyl]-amide,
(2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(1H-tetrazol-5-yl)-phenyl]-amide,
rac (2R,3R,4R,5S)-4-(4-chloro-2-fluoro-phenyl)-3-(3-chloro-2-methyl-phenyl)-4-cyano-5-(2,2-dimethyl-propyl)-pyrrolidine-2-carboxylic acid [4-(2-methyl-1H-tetrazol-5-yl)-phenyl]-amide,
rac (2R,3R,4R,5S)-4-(4-chloro-2-fluoro-phenyl)-3-(3-chloro-2-methyl-phenyl)-4-cyano-5-(2,2-dimethyl-propyl)-pyrrolidine-2-carboxylic acid [4-(1-methyl-1H-tetrazol-5-yl)-phenyl]-amide,
rac 5-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-2-fluoro-benzoic acid ethyl ester,
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [3-(1H-tetrazol-5-yl)-phenyl]-amide,
(2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid [3-(1H-tetrazol-5-yl)-phenyl]-amide,
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-carbamoyl-3-chloro-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [3-chloro-4-(1H-tetrazol-5-yl)-phenyl]-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-fluoro-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (3-fluoro-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (3-chloro-phenyl)-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-chloro-phenyl)-amide,
rac 4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-2-fluoro-benzoic acid tert-butyl ester,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-ethylcarbamoyl-3-fluoro-phenyl)-amide,
rac 4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-2-fluoro-benzoic acid,
rac (2R,3S,4R,5S)-3-(3-Chloro-2-fluoro-phenyl)-4-(4-chloro-2-fluoro-phenyl)-4-cyano-5-(2,2-dimethyl-propyl)-pyrrolidine-2-carboxylic acid (6-methoxy-pyridin-3-yl)-amide,
rac 3-({[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-methyl)-benzoic acid methyl ester,
rac 4-({[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-methyl)-benzoic acid methyl ester,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-chloro-phenyl)-amide,
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-chloro-phenyl)-amide,
(2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-chloro-phenyl)-amide,
rac 4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-2-methoxy-benzoic acid methyl ester,
rac 3-({[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-methyl)-benzoic acid,
rac 4-({[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-methyl)-benzoic acid,
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-acetylamino-phenyl)-amide,
(2S,3R,4S,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-acetylamino-phenyl)-amide,
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-methanesulfonyl-phenyl)-amide,
(25,3R,4s,5R)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-methanesulfonyl-phenyl)-amide,
rac 4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-2-methoxy-benzoic acid,
rac 5-bromo-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-2-methoxy-benzoic acid methyl ester,
rac 4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-2-methyl-benzoic acid methyl ester,
rac 2-Chloro-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid methyl ester,
rac 4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-2-trifluoromethyl-benzoic acid methyl ester,
rac 4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-2-methyl-benzoic acid,
rac 2-Chloro-4-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(2H-[1,2,4]triazol-3-yl)-phenyl-amide,
rac (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(5-oxo-2,5-dihydro-1H-[1,2,4]tiazol-3-yl)-phenyl]-amide,
rac-(2R,3S,4R,5S)-3-(5-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(2-cyclopropyl-2-methyl-propyl)-pyrrolidine-2-carboxylic acid ((S)-3,4-dihydroxy-butyl)-amide,
(2R,3S,4R,5S)-3-(3-chloro-2-fluoro-phenyl)-4-(4-chloro-2-fluoro-phenyl)-4-cyano-5-(2-cyclopropyl-2-methyl-propyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-3-methyl-phenyl)-4-cyano-5-(2,2-dimethyl-propyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(tetrahydro-pyran-4-ylmethyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(1-methyl-cyclohexylmethyl)-pyrrolidine-2-carboxylic acid ((S)-3,4-dihydroxy-butyl)-amide,
(2R,3S,4R,5S)-3-(3-chloro-2-fluoro-phenyl)-4-(4-chloro-2-fluoro-phenyl)-4-cyano-5-(1-methylcyclohexylmethyl)-pyrrolidine-2-carboxylic acid ((S)-3,4-dihydroxy-butyl)-amide,
rac-(2R,35,4R,5S)-5-(2-benzyloxycarbonyl-2-methyl-propyl)-3-(3-chloro-2-fluoro-phenyl)-4-(4-chloro-2-fluoro-phenyl)-4-cyano-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(2-methoxycarbonyl-2-methyl-propyl)-pyrrolidine-2-carboxylic acid ((S)-3,4-dihydroxy-butyl)-amide,
rac-(2R,3S,4R,5S)-3-(3-choro-2-fluoro-phenyl)-4-(4-chloro-2-fluoro-phenyl)-4-cyano-5-(3-hydroxy-2,2-dimethyl-propyl)-pyrrolidine-2-carboxylic acid ((S)-3,4-dihydroxy-butyl)-amide,
(2R,3S,4R,5S)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(2,2-diethyl-butyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(2-ethyl-2-methyl-butyl)-pyrrolidine-2-carboxylic acid ((S)-3,4-dihydroxy-butyl)-amide,
rac-(2R,3R,4R,5S)-4-(4-chloro-2,6-difluoro-phenyl)-3-(3-chloro-phenyl)-4-cyano-5-(2,2-dimethyl-propyl)-pyrrolidine-2-carboxylic acid ((S)-3,4-dihydroxy-butyl)-amide,
rac-(2R,3R,4R,5S)-4-(4-chloro-2,5-difluoro-phenyl)-3-(3-chloro-phenyl)-4-cyano-5-(2,2-dimethyl-propyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(3-methoxy-2,2-dimethyl-propyl)-pyrrolidine-2-carboxylic acid ((S)-3,4-dihydroxy-butyl)-amide,
(2R,3S,4R,5S)-3-(3-chloro-2-fluoro-phenyl)-4-(4-chloro-2-fluoro-phenyl)-4-cyano-5-(3-methoxy-2,2-dimethyl-propyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(2-ethyl-2-hydroxymethyl-butyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(3-methyl-oxetan-3-ylmethyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(3-ethyl-oxetan-3-ylmethyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(1-hydroxymethyl-cyclopropylmethyl)-pyrrolidine-2-carboxylic acid ((S)-3,4-dihydroxy-butyl)-amide,
(2R,3S,4R,5S)-3-(3-chloro-2-fluoro-phenyl)-4-(4-chloro-2-fluoro-phenyl)-4-cyano-5-(1-hydroxymethyl-cyclopropylmethyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(1-hydroxymethyl-cyclobutylmethyl)-pyrrolidine-2-carboxylic acid ((S)-3,4-dihydroxy-butyl)-amide,
(2R,3S,4R,5S)-3-(3-chloro-2-fluoro-phenyl)-4-(4-chloro-2-fluoro-phenyl)-4-cyano-5-(1-hydroxymethyl-cyclobutylmethyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid ((S)-3,4-dihydroxy-butyl)-amide,
(2R,3S,4R,5S)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(1-hydroxymethyl-cyclohex-3-enylmethyl)-pyrrolidine-2-carboxylic acid ((S)-3,4-dihydroxybutyl)-amide,
rac-(2R,3S,4R,5S)-3-(3-chloro-2-fluoro-phenyl)-4-(4-chloro-2-fuoro-phenyl)-4-cyano-5-(1-hydroxymethyl-cyclohexylmethyl)-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-[4-(2-hydroxy-ethoxy)-2,2-dimethyl-butyl]-pyrrolidine-2-carboxylic acid ((S)-3,4-dihydroxy-butyl)-amide,
rac-(2R,3S,4R,5S)-5-(4-azido-2,2-dimethyl-butyl)-3-(3-chloro-2-fluoro-phenyl)-4-(4-chloro-2-fluoro-phenyl)-4-cyano-pyrrolidine-2-carboxylic acid ((S)-3,4-dihydroxy-butyl)-amide,
rac-(2R,3S,4R,5S)-5-(4-amino-2,2-dimethyl-butyl)-3-(3-chloro-2-fluoro-phenyl)-4-(4-chloro-2-fluoro-phenyl)-4-cyano-pyrrolidine-2-carboxylic acid ((S)-3,4-dihydroxy-butyl)-amide,
rac-(2R,3S,4R,5S)-5-(4-acetylamino-2,2-dimethyl-butyl)-3-(3-chloro-2-fluoro-phenyl)-4-(4-chloro-2-fluoro-phenyl)-4-cyano-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-(4-methanesulfonylamino-2,2-dimethyl-butyl)-pyrrolidine-2-carboxylic acid ((S)-3,4-dihydroxy-bulyl)-amide,
rac-(2R,3S,4R,5S)-5-(4-benzoylamino-2,2-dimethyl-butyl)-3-(3-chloro-2-fluoro-phenyl)-4-(4-chloro-2-fluoro-phenyl)-4-cyano-pyrrolidine-2-carboxylic acid {(S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-[2-methyl-2-(5-methyl-furan-2-yl)-propyl]-pyrrolidine-2-carboxylic acid ((S)-3,4-dihydroxy-butyl)-amide,
rac-(2R,3S,4R,5S)-3-(3-chloro-2-fluoro-phenyl)-4-(4-chloro-2-fluoro-phenyl)-4-cyano-5-[3-(4-methoxy-phenyl)-2,2-dimethyl-propyl]-pyrrolidine-2-carboxylic acid ((S)-3,4-dihydroxy-butyl)-amide,
rac-(2R,3S,4R,5S)-5-[2-(1-benzyl-1,2,3,6-tetrahydro-pyridin-4-yl)-2-methyl-propyl]-3-(3-chloro-2-fluoro-phenyl)-4-(4-chloro-2-fluoro-phenyl)-4-cyano-pyrrolidine-2-carboxylic acid ((S)-3,4-dihydroxy-butyl)-amide,
rac-(2R,3S,4R,5S)-5-[2-(1-benzyl-piperidin-4-yl)-2-methyl-propyl]-3-(3-chloro-2-fluoro-phenyl)-4-(4-chloro-2-fluoro-phenyl)-4-cyano-pyrrolidine-2-carboxylic acid ((S)-3,4-dihydroxybutyl)-amide,
rac-(2R,3S,4R,5S)-3-(3-chloro-2-fluoro-phenyl)-4-(4-chloro-2-fluoro-phenyl)-4-cyano-5-[2-(3,6-dihydro-2H-pyran-4-yl)-2-methyl-propyl]-pyrrolidine-2-carboxylic acid ((S)-3,4-dihydroxybutyl)-amide,
rac-(2R,3S,4R,5S)-3-(3-chloro-2-fluoro-phenyl)-4-(4-chloro-2-fluoro-phenyl)-4-cyano-5-[2-methyl-2-(tetrahydro-pyran-4-yl)-propyl]-pyrrolidine-2-carboxylic acid ((S)-3,4-dihydroxybutyl)-amide,
rac-(2R,3S,4R,5S)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid ((S)-3,4-dihydroxy-4-methylpentyl)-amide,
(2R,3S,4R,5S)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid ((S)-3,4-dihydroxy-4-methylpentyl)-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-4-methyl-pentyl)-amide,
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid ((S)-3,4-dihydroxy-4-methyl-pentyl)-amide,
rac-(2R,3S,4R,5S)-3-(3-chloro-2-fluoro-phenyl)-4-(4-chloro-2-fluoro-phenyl)-4-cyano-5-(1-methyl-cyclohexymethyl)-pyrrolidine-2-carboxylic acid ((S)-3,4-dihydroxy-4-methyl-pentyl)-amide,
(2R,3S,4R,5S)-3-(3-chloro-2-fluoro-phenyl)-4-(4-chloro-2-fluoro-phenyl)-4-cyano-5-(1-methylcyclohexylmethyl)-pyrrolidine-2-carboxylic acid ((S)-3,4-dihydroxy-4-methyl-pentyl)-amide,
rac-(2S,3R,4S,5R)-4-(3-chloro-2-fluoro-phenyl)-3-(4-chloro-2-fluoro-phenyl)-2-(2,2-dimethylpropyl)-5-(3-hydroxy-azetidine-1-carbonyl)-pyrrolidine-3-carbonitrile,
rac-(2R,3S,4R,5S)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid [1-(2-hydroxy-2-methyl-propyl)-I H-pyrazol-3-yl]-amide,
(2R,3S,4R,5S)-3-(3-chloro-2-fluoro-phenyl)-4-(4-chloro-2-fluoro-phenyl)-4-cyano-5-(3-hydroxy-2,2-dimethyl-propyl)-pyrrolidine-2-carboxylic acid [1-(2-hydroxy-2-methyl-propyl)-1H-pyrazol-3-yl]-amide,
rac-(2R,3S,4R,5S)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid [1-(2-hydroxy-2-methyl-propyl)-1H-pyrazol-3-yl]-amide,
(2R,3S,4R,5S)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid [1-(2-hydroxy-2-methylpropyl)-1H-pyrazol-3-yl]-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid amide,
rac-6-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-nicotinic acid methyl ester,
rac-6-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-N-(2-hydroxy-1,1-dimethyl-ethyl)-nicotinamide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (6-acetylamino-pyridin-3-yl)-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-methyl-2-oxo-1,2-dihydro-pyridin-4-yl)-amide,
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-methyl-2-oxo-1,2-dihydro-pyridin-4-yl)-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [5-((S)-1,2-dihydroxy-ethyl)-pyrazin-2-yl]-amide,
rac-(2R,3S,4R,5S)-3-(3-chloro-2-fluoro-phenyl)-4-(4-chloro-2-fluoro-phenyl)-4-cyano-5-(4-hydroxy-2,2-dimethyl-butyl)-pyrrolidine-2-carboxylic acid (1-methyl-2-oxo-1,2-dihydro-pyridin-4-yl)-amide,
rac-5-{[(2R,3S,4R,5S)-3-(3-chloro-2-fluoro-phenyl)-4-(4-chloro-2-fluoro-phenyl)-4-cyano-5-(2,2-dimethyl-propyl)-pyrolidine-2-carbonyl]-amino}-furan-2-carboxylic acid methyl ester,
rac-5-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-furan-2-carboxylic acid,
rac-5-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-furan-2-carboxylic acid amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (6-chloro-pyridazin-3-yl)-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-methyl-pyridin-yl)-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(2-hydroxy-ethoxy)-phenyl]-amide,
(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(2-hydroxy-ethoxy)-phenyl]-amide,
rac-5-{[(2R,3S,4R,5S}-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-thiophene-2-carboxylic acid methyl ester,
rac-5-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-thiophene-2-carboxylic acid,
5-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-thiophene-2-carboxylic acid,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-methoxy-pyridin-4-yl)-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-hydroxy-pyridin-4-yl)-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid (4-acetyl-phenyl)-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(2-bromo-acetyl)-phenyl]-amide,
rac-(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [4-(2- dimethylamino-acetyl)-phenyl]-amide,
rac-(5-{[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-4H-[1,2,4]triazol-3-yl)-acetic acid,
rac-(3-{[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-pyrazol-1-yl)-acetic acid,
rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (1H-imidazol-4-ylmethyl)-amide,
rac-(2*S*,3*R*,4*S*,5*R*)-4-(3-chloro-2-fluoro-phenyl)-3-(4-chloro-2-fluoro-phenyl)-2-(2,2-dimethylpropyl)-5-(2-oxa-6-aza-spiro[3.3]heptane-6-carbonyl)-pyrrol idine-3-car bonitri le,
rac-1-[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbony]-azetidine3-carboxylic acid,
rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-[1,2,3]triazol-1-yl-ethyl)-amide,
rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (1-carbamoylmethyl-1H-pyrazol-3-yl)-amide,
rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid [1-(2-hydroxy-2-methyl-propyl)-1H-pyrazol-3-yl]-amide,
rac-(2*R*,3*R*,4*R*,5*S*)-3-(3-chloro-phenyl)-4-(4-chloro-phenyl)-4-cyano-5-(2,2-dimethyl-propyl)-pyrrolidine-2-carboxylic acid (3-methanesulfonylamino-propyl)-amide,
chiral (2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((*S*)-3-dimethylamino-2-hydroxy-propoxy)-2-methyl-propyl]-1H-pyrazol-3-yl}-amide,
chiral (2*S*,3*R*,4*S*,5*R*)-3-(3-chloro-2-fluoro-phenyl)-4-(4-chloro-2-fluoro-phenyl)-4-cyano-5-(2,2-dimethyl-propyl)-pyrrolidine-2-carboxylic acid {1-[2-((*S*)-3-dimethylamino-2-hydroxy-propoxy)-2-methyl-propyl]-1H-pyrazol-3-yl}-amide,
rac-1-{[(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-cyclopropane carboxylic acid,
rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid [1-(4-hydroxy-piperidin-4-ylmethyl)-1H-pyrazol-3-yl]-amide,
rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-acetyl-thiophen-3-yl)-amide,
rac-(2*R*,3*S*,4*R*,5*S*)-3-(3-chloro-2-fluoro-phenyl)-4-(4-chloro-2-fluoro-phenyl)-4-cyano-5-(2,2-dimethyl-propyl)-pyrrolidine-2-carboxylic acid (2-carbamoyl-thiophen-3-yl)-amide,
rac- (2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carboxylic acid [1-((S)-3-dimethylamino-2-hydroxy-propyl)-1H-pyrazol-3-yl]-amide,
rac-4-{[(2R,3S,4R,5S)-3-(3-chloro-2-fluoro-phenyl)-4-(4-chloro-2-fluoro-phenyl)-4-cyano-5-(1-methyl-cyclohexylmethyl)-pyrrolidine-2-carbonyl]-amino}-benzoic acid,
rac-[4-(3-{[(2R,3S,4R,5S)-3-(3-chloro-2-fluoro-phenyl)-4-(4-chloro-2-fluoro-phenyl)-4-cyano-5-(2,2-dimethyl-propyl)-pyrrolidine-2-carbonyl]-amino}-pyrazol-1-ylmethyl)-4-hydroxy-piperidin-1-yl]-acetic acid,
rac-4- {[(2R,3S,4R,5S)-3-(3-chloro-2-fluoro-phenyl)-4-(4-chloro-2-fluoro-phenyl)-4-cyano-5-(1-methyl-cyclohexylmethyl)-pyrrolidine-2-carbonyl]-amino}-benzoic acid,
rac-4-{[(2R,3S,4R,5S)-3-(5-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid methyl ester,
rac-4-{([(2R,3S,4R,5S)-3-(5-chloro-2-fluoro-phenyl)-4-(4-chloro-2-fluoro-phenyl)-4-cyano-5-(2,2-dimethyl-propyl)-pyrrolidine-2-carbonyl]-amino}-benzoic acid,
rac-4-{[(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carbonyl]-amino}-benzoic acid methyl ester,
rac-4-{[(2R,3R,4R,5S)-3-(3-chloro-4-fluoro-phenyl)-4-(4-chloro-2-fluoro-phenyl)-4-cyano-5-(2,2-dimethyl-propyl)-pyrrolidine-2-carbonyl]-amino}-benzoic acid,
rac-4-{[(2R,3R,4R,5S)-3-(3-bromo-phenyl)-4-(4-chloro-2-fluoro-phenyl)-4-cyano-5-(2,2-dimethyl-propyl)-pyrrolidine-2-carbonyl]-amino}-benzoic acid methyl ester,
of rac-4-{[(2R,3R,4R,5S)-3-(3-bromo-phenyl)-4-(4-chloro-2-fluoro-phenyl)-4-cyano-5-(2,2-dimethyl-propyl)-pyrrolidine-2-carbonyl]-amino)-benzoic acid,
rac-4-{[(2R,3S,4R,5S)-3-(3-chloro-2-fluoro-phenyl)-4-(4-chloro-phenyl)-4-cyano-5-(2,2-dimethyl-propyl)-pyrrolidine-2-carbonyl]-amino}-benzoic acid methyl ester,
rac-4- {[(2R,3S,4R,5S)-3-(3-chloro-2-fluoro-phenyl)-4-(4-chloro-phenyl)-4-cyano-5-(2,2-dimethyl-propyl)-pyrrolidine-2-carbonyl]-amino} -benzoic acid, and
rac-4- [(2R,3S,4R,5S)-3-(3-chloro-2-fluoro-phenyl)-4-(4-chloro-2-fluoro-phenyl)-4-cyano-5-(1-methyl-cyclohexylmethyl)-pyrrolidine-2-carbonyl]-amino}-benzoic acid methyl ester.

8. A pharmaceutical composition comprising a compound of claims 1 to 7 together with pharmaceutically acceptable excipients or carriers.

9. A compound according to any one of claims 1 to 7 for the use as medicament.

10. A compound according to any one of claims 1 to 7 for the use as medicament for the treatment of cancer.

11. A compound according to any one of claims 1 to 7 for the use as medicament for the treatment of solid tumors.

12. A compound according to any one of claims I to 7 for the use as medicament for the treatment of breast, colon, lung and prostate tumors.

## Patentansprüche

1. Eine Verbindung der Formel 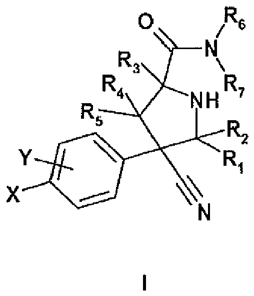 wobei
X ausgewählt ist aus der Gruppe bestehend aus H, F, Cl, Br, I, Cyano, Nitro, Ethinyl, Cyclopropyl, Methyl, Ethyl, Isopropyl, Vinyl und Methoxy;
Y ein bis vier Rest(e) ist, unabhängig ausgewählt aus der Gruppe bestehend aus H, F, Cl, Br, I, CN, OH, Nitro, C1-6-Alkyl, Cycloalkyl, C1-6-Alkoxy, C2-6-Alkenyl, Cycloalkenyl, C2-6-Alkinyl, Aryl, Heteroaryl, Heterocyclus, COOR', OCOR', CONR'R", NR'COR", NR"SO₂R', SO₂NR'R" und NR'R", wobei
R' und R" unabhängig ausgewählt sind aus H, substituiertem oder unsubstituiertem C1-6-Alkyl, substituiertem oder unsubstituiertem C3-10-Cycloalkyl, substituiertem oder unsubstituiertem C2-6-Alkenyl, substituiertem oder unsubstituiertem C5-C10-Cycloalkenyl, substituiertem oder unsubstituiertem Aryl, substituiertem oder unsubstituiertem Heteroaryl oder substituiertem oder unsubstituiertem Heterocyclus; und im Fall, dass sich R' und R" unabhängig verbinden können, um eine cyclische Struktur, ausgewählt aus substituiertem oder unsubstituiertem Cycloalkyl, substituiertem oder unsubstituiertem Cycloalkenyl, substituiertem oder unsubstituiertem Heteroaryl oder substituiertem oder unsubstituiertem Heterocyclus, zu bilden;
ein Rest R₁ und R₂ ausgewählt ist aus der Gruppe bestehend aus C1-6-Alkyl, substituiertem C1-C6-Alkyl, C2-6-Alkenyl, substituiertem C2-6-Alkenyl, Aryl, substituiertem Aryl, Heteroaryl, substituiertem Heteroaryl, Heterocyclus, substituiertem Heterocyclus, Cycloalkyl, substituierten Cycloalkyl, Cycloalkenyl und substituiertem Cycloalkenyl und der andere Wasserstoff oder C1-6-Alkyl ist;
R₃ gleich H oder C1-6-Alkyl ist;
einer der Reste R₄ und R₅ ausgewalzt ist aus der Gruppe bestehend aus C1-C1-Alkyl, substituiertem C1-6-Alkyl, C2-6-Alkenyl, substituiertem C2-6-Alkenyl, Aryl, substituiertem Aryl, Heteroaryl, substituiertem Heteroaryl, Heterocyclus, substituiertem Heterocyclus, Cycloalkyl, substituiertem Cycloalkyl, Cycloalkenyl und substituiertem Cycloalkenyl und der andere Wasserstoff ist;
R₆ und R₇ ausgewählt sind aus der Gruppe bestehend aus (CH₂)ₙ-R', (CH₂)ₙ-NR'R", (CH₂)ₙ-NR'COR", (CH₂)ₙ-NR'SO₂R", (CH₂)ₙ-COOH, (CH₂)ₙ-COOR', (CH₂)ₙ-CONR'R", (CH₂)ₙ-OR', (CH₂)ₙ-SR', (CH₂)ₙ-SOR', (CH₂)ₙ-SO₂R', (CH₂)ₙ-COR', (CH₂)ₙ-SO₃H, (CH₂)ₙ-SONR'R", (CH₂)ₙ-SO₂NR'R", (CH₂CH₂O)ₘ-(CH₂)ₙ-R', (CH₂CH₂O)ₘ-(CH₂)ₙ-OH, (CH₂CH₂O)ₘ-(CH₂)ₙ-OR', (CH₂CH₂O)ₘ-(CH₂)ₙ-NR'R", (CH₂CH₂O)ₘ₋(CH₂)ₙ-NR'COR", (CH₂CH₂O)ₘ-(CH₂)ₙ-NR'SO₂R", (CH₂CH₂O)ₘ-(CH₂)ₙ-COOH, (CH₂CH₂O)ₘ-(CH₂)ₙ-COOR', (CH₂CH₂O)ₘ-(CH₂)ₙ-CONR'R", (CH₂CH₂O)ₘ-(CH₂)ₙ-SO₂R', (CH₂CH₂O)ₘ-(CH₂)ₙ-COR', (CH₂CH₂O)ₘ-(CH₂)ₙ-SONR'R", (CH₂CH₂O)ₘ-(CH₂)ₙ-SO₂NR'R", (CH₂)ₚ-(CH₂CH₂O)ₘ-(CH₂)ₙ-R', (CH₂)ₚ-(CH₂CH₂O)ₘ-(CH₂)ₙ-OH, (CH₂)ₚ-(CH₂CH₂O)ₘ-(CH₂)ₙ-OR', (CH₂)ₚ-(CH₂CH₂O)ₘ-(CH₂)ₙ-NR'R", (CH₂)ₚ-(CH₂CH₂O)ₘ-(CH₂)ₙ-NR'COR", (CH₂)ₚ-(CH₂CH₂O)ₘ-(CH₂)ₙ-NR'SO₂R", (CH₂)ₚ-(CH₂CH₂O)ₘ-(CH₂)ₙ-COOH, (CH₂)ₚ-(CH₂CH₂O)ₘ-(CH₂)ₙ-COOR', (CH₂)ₚ-CH₂CH₂O)ₘ-(CH₂)ₙ-CONR'R", (CH₂)ₚ-(CH₂CH₂O)ₘ-(CH₂)ₚ-SO₂R', (CH₂)ₚ-(CH₂CH₂O)ₘ-(CH₂)ₙ-COR', (CH₂)ₚ-(CH₂CH₂O)ₘ-(CH₂)ₙ-SONR'R", (CH₂)ₚ-(CH₂CH₂O)ₘ-(CH₂)ₙ-SO₂NR'R", -COR', -SOR' und SO₂R', wobei R' und R" wie vorstehend definiert sind;
m, n und p unabhängig 0 bis 6 sind;
und die pharmazeutisch verträglichen Salze und Ester davon.

2. Eine Verbindung nach Anspruch 1 der Formel 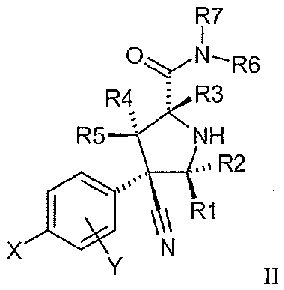 wobei
X ausgewählt ist aus der Gruppe bestehend aus H, F, Cl, Br, I, Cyano, Nitro, Ethinyl, Cyclopropyl, Methyl, Ethyl, Isopropyl, Vinyl und Methoxy;
Y ein bis vier Rest(e) ist, unabhängig ausgewählt aus der Gruppe bestehend aus H, F, Cl, Br, I, CN, OH, Nitro, C1-6-Alkyl, Cycloalkyl, C1-6-Alkoxy, C2-6-Alkenyl, Cycloalkenyl, C2-6-Alkinyl, Aryl, Heteroaryl, Heterocyclus, COOR', OCOR', CONR'R", NR'COR", NR"SO₂R', SO₂NR'R" und NR'R", wobei
R' und R" unabhängig ausgewählt sind aus H oder substituiertem oder unsubstituiertem C1-6-Alkyl, substituiertem oder unsubstituiertem C3-14-Cycloalkyl, substituiertem oder unsubstituiertem C2-6-Alkenyl, substituiertem oder unsubstituiertem C5-C10-Cycloalkenyl, substituiertem oder unsubstituiertem Aryl, substituiertem oder unsubstituiertem Heteroaryl oder substituiertem oder unsubstituiertem Heterocyclus; und wobei sich R' und R" unabhängig verbinden können, um eine cyclische Struktur, ausgewählt aus substituiertem oder unsubstituiertem Cycloalkyl, substituiertem oder unsubstituiertem Cycloalkenyl, substituiertem oder unsubstituiertem Heteroaryl oder substituiertem oder unsubstituiertem Heterocyclus, zu bilden;
R₁ ausgewählt ist aus der Gruppe bestehend aus C1-6-Alkyl, substituiertem C1-C6-Alkyl, C2-6-Alkenyl, substituiertem C2-6-Alkenyl, Aryl, substituiertem Aryl, Heteroaryl, substituiertem Heteroaryl, Heterocyclus, substituiertem Heterocyclus, Cycloalkyl, substituiertem Cycloalkyl, Cycloalkenyl und substituiertem Cycloalkenyl; R₂ Wasserstoff oder C1-6-Alkyl ist;
R₃ gleich H oder C1-6-Alkyl ist;
R₅ ausgewählt ist aus der Gruppe bestehend aus C1-6-Alkyl, substituiertem C1-6-Alkyl, C2-6-Alkenyl, substituiertem C2-6-Alkenyl, Aryl, substituiertem Aryl, Heteroaryl, substituiertem Heteroaryl, Heterocyclus, substituiertem Heterocyclus Cycloalkyl, substituiertem Cycloalkyl, Cycloalkenyl und substituiertem Cycloalkenyl;
R₄ Wasserstoff ist;
R₆ und R₇ ausgewählt sind aus der Gruppe bestehend aus (CH₂)ₙ-R', (CH₂)ₙ-NR'R", (CH₂)ₙ-NR'COR", (CH₂)ₙ-NR'SO₂R", (CH₂)"-COOH, (CH₂)"-COOR', (CH₂)ₙ-CONR'R", (CH₂)"-OR', (CH₂)ₙ-SR', (CH₂)ₙ-SOR', (CH₂)ₙ-SO₂R', (CH₂)ₙ-COR', (CH₂)ₙ-SO₃H, (CH₂)ₙ-SONR'R", (CH₂)ₙ-SO₂NR'R", (CH₂CH₂O)ₘ-(CH₂)ₙ-R', (CH₂CH₂O)ₘ-(CH₂)ₙ-OH, (CH₂CH₂O)ₘ-(CH₂)ₙ-OR', (CH₂CH₂O)ₘ-(CH₂)ₙ-NR'R", (CH₂CH₂O)ₘ-(CH₂)ₙ-NR'COR", (CH₂CH₂O)ₘ-(CH₂)ₙ-NR'SO₂R", (CH₂CH₂O)ₘ-(CH₂)ₙ-COOH, (CH₂CH₂O)ₘ-(CH₂)ₙ-COOR', (CH₂CH₂O)ₘ-(CH₂)ₙ-CONR'R", (CH₂CH₂O)ₘ-(CH₂)ₙ-SO₂R', (CH₂CH₂O)ₘ-(CH₂)ₙ-COR', (CH₂CH₂O)ₘ-CH₂)ₙ-SONR'R", (CH₂CH₂O)ₘ-(CH₂)ₙ-SO₂NR'R", (CH₂)ₚ-(CH₂CH₂O)ₘ-(CH₂)ₙ-R', (CH₂)ₚ-(CH₂CH₂O)ₘ-(CH₂)ₙ-OH, (CH₂)ₚ-(CH₂CH₂O)ₘ(CH₂)ₙ-OR', (CH₂)ₚ-(CH₂CH₂O)ₘ-(CH₂)ₙ-NR'R", (CH₂)ₚ-(CH₂CH₂O)ₘ-(CH₂)ₙ-NR'COR", (CH₂)ₚ-(CH₂CH₂O)ₘ-(CH₂)ₙ-NR'SO₂R", (CH₂)ₚ-(CH₂CH₂O)ₘ-(CH₂)ₙ-COOH, (CH₂)ₚ-(CH₂CH₂O)ₘ-(CH₂)ₙ-COOR', (CH₂)ₚ-(CH₂CH₂O)ₘ-(CH₂)ₙ-CONR'R", (CH₂)ₚ-(CH₂CH₂O)ₘ-(CH₂)ₙ-SO₂R', (CH₂)ₚ-(CH₂CH₂O)ₘ-(CH₂)ₙ-COR', (CH₂)ₚ-(CH₂CH₂O)ₘ-(CH₂)ₙ-SONR'R", (CH₂)ₚ-(CH₂CH₂O)ₘ-(CH₂)ₙ-SO₂NR'R", -COR', -SOR' und SO₂R', wobei R' und R" wie vorstehend definiert sind;
m, n und p unabhängig 0 bis 6 sind; und
die pharmazeutisch verträglichen Salze und Ester davon.

3. Die Verbindung nach Anspruch 2, wobei
X gleich F, Cl oder Br ist;
Y ein bis zwei Rest(e) ist, unabhängig ausgewählt aus der Gruppe bestehend aus H, F, Cl, Br, I, CN, OH, Nitro, C1-6-Alkyl, Cycloalkyl, C1-6-Alkoxy, C2-6-Alkenyl, C5-10-Cycloalkenyl und C2-6-Alkinyl ist;
R₁ ausgewählt ist aus der Gruppe bestehend aus C1-6-Alkyl, substituiertem C1-C6-Alkyl, C2-6-Alkenyl, substituiertem C2-6-Alkenyl, Aryl, substituiertem Aryl, Heteroaryl, substituiertem Heteroaryl, Heterocyclus, substituiertem Heterocyclus, Cycloalkyl, substituiertem Cycloalkyl, Cycloalkenyl und substituiertem Cycloalkenyl; R₂ Wasserstoff ist;
R₃ gleich H ist;
R₅ ausgewählt ist aus der Gruppe bestehend aus Aryl, substituiertem Aryl, Heteroaryl und substituiertem Heteroaryl;
R₄ Wasserstoff ist;
R₆ und R₇ ausgewählt sind aus der Gruppe bestehend aus (CH₂)ₙ-R', (CH₂)ₙ-NR'R", (CH₂)ₙ-NR'COR", (CH₂)ₙ-NR'SO₂R", (CH₂)ₙ-COOH, (CH₂)ₙ-COOR', (CH₂)ₙ-CONR'R", (CH₂)ₙ-OR', (CH₂)ₙ-SR', (CH₂)ₙ₋SOR" (CH₂)ₙ-SO₂R', (CH₂)ₙ-COR', (CH₂)ₙ-SO₃H, (CH₂)ₙ-SONR'R", (CH₂)ₙ-SO₂NR'R", (CH₂CH₂O)ₘ-(CH₂)ₙ-R', (CH₂CH₂O)ₘ-(CH₂)ₙ-OH, (CH₂CH₂O)ₘ-(CH₂)ₙ-OR', (CH₂CH₂O)ₘ-(CH₂)ₙ-NR'R", (CH₂CH₂O)ₘ-(CH₂)ₙ-NR'COR", (CH₂CH₂O)ₘ-(CH₂)ₙ-NR'SO₂R", (CH₂CH₂O)ₘ-(CH₂)ₙ-COOH, (CH₂CH₂O)ₘ-(CH₂)ₙ-COOR', (CH₂CH₂O)ₘ-(CH₂)ₙ-CONR'R", (CH₂CH₂O)ₘ-(CH₂)ₙ-SO₂R', (CH₂CH₂O)ₘ-(CH₂)ₙ-COR', (CH₂CH₂O)ₘ-(CH₂)ₙ-SONR'R", (CH₂CH₂O)ₘ-(CH₂)ₙ-SO₂NR'R", (CH₂)ₚ-(CH₂CH₂O)ₘ-(CH₂)ₙ-R', (CH₂)ₚ-(CH₂CH₂Oₘ-(CH₂)ₙ-OH, (CH₂)ₚ-(CH₂CH₂O)ₘ-(CH₂)ₙ-OR', (CH₂)ₚ-(CH₂CH₂O)ₘ-(CH₂)ₙ-NR'R", (CH₂)ₚ-(CH₂CH₂O)ₘ-(CH₂)ₙ-NR'COR", (CH₂)ₚ-(CH₂CH₂O)ₘ-(CH₂)ₙ-NR'SO₂R", (CH₂)ₚ-(CH₂CH₂O)ₘ-(CH₂)ₙ-COOH, (CH₂)ₚ-(CH₂CH₂O)ₘ-(CH₂)ₙ-COOR', (CH₂)ₚ-(CH₂CH₂O)ₘ-(CH₂)ₙ-CONR'R", (CH₂)ₚ-(CH₂CH₂O)ₘ-(CH₂)-SO₂R', (CH₂)ₚ-(CH₂CH₂O)ₘ-(CH₂)ₙ-COR', (CH₂)ₚ-(CH₂CH₂O)ₘ-CH₂)ₙ-SONR'R", (CH₂)ₚ-(CH₂CH₂O)ₘ-(CH₂)ₙ-SO₂NR'R", -COR', -SOR' und SO₂R', wobei
R' und R" unabhängig ausgewählt sind aus H, oder substituiertem oder unsubstituiertem C1-6-Alkyl, substituiertem oder unsubstituiertem C3-10-Cycloalkyl, substituiertem oder unsubstituiertem C2-6-Alkenyl, substituiertem oder unsubstituiertem C5-C10-Cycloalkenyl, substituiertem oder unsubstituiertem Aryl, substituiertem oder unsubstituiertem Heteroaryl oder substituiertem oder unsubstituiertem Heterocyclus; und wobei sich R' und R" auch unabhängig verbinden können, um eine cyclische Struktur, ausgewählt aus substituiertem oder unsubstituiertem Cycloalkyl, substituiertem oder unsubstituiertem Cycloalkenyl, substituiertem oder unsubstituiertem Heteroaryl oder substituiertem oder unsubstituiertem Heterocyclus, zu bilden;
m, n und p unabhängig 0 bis 6 sind; und
die pharmazeutisch verträglichen Salze und Ester davon.

4. Die Verbindung nach Anspruch 2, wobei
X gleich F, Cl oder Br ist;
Y eine einfach-Substitutionsgruppe, ausgewählt aus H oder F ist; und
R₁ ausgewählt ist aus der Gruppe bestehend aus C1-6-Alkyl, substituiertem C1-C6-Alkyl, C2-6-Alkenyl, substituiertem C2-6-Alkenyl, Heterocyclus, substituiertem Heterocyclus, Cycloalkyl, substituiertem Cycloalkyl, Cycloalkenyl und substituiertem Cycloalkenyl.

5. Die Verbindung nach Anspruch 2, wobei
R₁ ein substituiertes C1-6-Alkyl der Formel 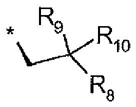 ist, wobei R₈, R₉ beide Methyl sind, oder verbunden sind, um eine Cyclopropyl-, Cyclobutyl-, Cyclopentyl- oder eine Cyclohexylgruppe zu bilden;
R₁₀ gleich (CH₂)ₘ-R₁₁ ist;
m gleich 0, 1 oder 2 ist,
R₁₁ ausgewählt ist aus Wasserstoff, Hydroxyl, C1-6-Alkyl, C1-6-Alkoxy, Aryl, substituiertem Aryl, Heteroaryl, substituiertem Heteroaryl, Heterocyclus oder substituiertem Heterocyclus;
R₂ gleich H ist;
R₃ gleich H ist;
R₅ ein substituiertes Phenyl ist, ausgewählt aus 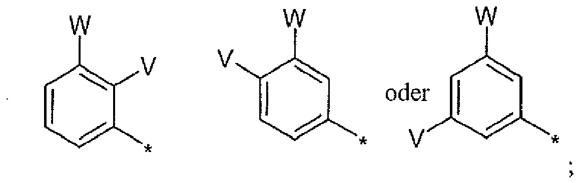 W gleich F, Cl oder Br ist;
V gleich H oder F ist;
R₄ Wasserstoff ist;
einer der Reste R₆ und R₇ Wasserstoff ist und der andere (CH₂)ₙ-R¹ ist;
n gleich 0 oder 1 ist; und
R' ausgewählt ist aus Aryl, substituiertem Aryl, Heteroaryl, substituiertem Heteroaryl, Heterocyclus oder substituiertem Heterocyclus.

6. Eine Verbindung nach Anspruch 1, ausgewählt aus der Gruppe bestehend aus
rac-(2R,3R,4R,5S)-3-(3-Chlor-phenyl)-4-(4-chlor-phenyl)-4-cyano-5-(2,2-dimethylpropyl)-pyrrolidin-2-carbonsäure-(2-morpholin-4-yl-ethyl)-amid,
(2R,3R,4R,5S)-3-(3-Chlor-phenyl)-4-(4-chlor-phenyl)-4-cyano-5-(2,2-dimethyl-propyl)-pyrrolidin-2-carbonsäure-(2-morpholin-4-yl-ethyl)-amid,
rac-(2R,3R,4R,5S)-3-(3-Chlor-phenyl)-4-(4-chlor-phenyl)-4-cyano-5-(2,2-dimethylpropyl)-pyrrolidin-2-carbonsäuredimethylamid,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-((S)-3,4-dihydroxy-butyl)-amid,
(2R,3R,4R,5S)-3-(3-Chlor-phenyl)-4-(4-chlor-phenyl)-4-cyano-5-(2,2-dimethyl-propyl)-pyrrolidin-2-carbonsäure-((S)-3,4-dihydroxy-butyl)-amid,
rac-(2S,3R,4R,5R)-4-(3-Chlor-phenyl)-3-(4-chlor-phenyl)-2-(2,2-dimethyl-propyl)-5-[4-(2-morpholin-4-yl-2-oxo-ethyl)-piperazin-1-carbonyl]-pyrrolidin-3-carbonitril,
rac-(2S,3R,4R,5R)-4-(3-Chlor-phenyl)-3-(4-chlor-phenyl)-2-(2,2-dimethyl-propyl)-5-[4-(2-oxo-2-pyrrolidin-1-yl-ethyl)-piperazin-1-carbonyl]-pyrrolidin-3-carbonitril,
rac-(2S,3R,4R,5R)-4-(3-Chlor-phenyl)-3-(4-chlor-phenyl)-2-(2,2-dimethyl-propyl)-5-[4-(2-hydroxy-ethyl)-piperazin-1-carbonyl]-pyrrolidin-3-carbonitril,
rac-(2R,3R,4R,5S)-3-(3-Chlor-phenyl)-4-(4-chlor-pheny)-4-cyano-5-(2,2-dimethylpropyl)-pyrrolidin-2-carbonsäure-(4-hydroxy-butyl)-amid,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-(2-pyrrolidin-1-yl-ethyl)-amid,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-(2-piperazin-1-yl-ethyl)-amid,
(S)-2-{[(2R,3R,4R,5S)-3-(3-Chlor-phenyl)-4-(4-chlor-phenyl)-4-cyano-5-(2,2-dimethylpropyl)-pyrrolidin-2-carbonyl]-amino}-3-methyl-butansäuremethylester;
(S)-2-{[(2R,3R,4R,5S)-3-(3-Chlor-phenyl)-4-(4-chlor-phenyl)-4-cyano-5-(2,2-dimethylpropyl)-pyrrolidin-2-carbonyl]-amino}-3-methyl-butansäure,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-(1-hydroxymethyl-cyclopropylmethyl)-amid,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-(1-hydroxymethyl-cyclobutylmethyl)-amid,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-(3,3-dimethyl-butyl)-amid,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-(2,2-dimethyl-propyl)-amid,
(2S,3R,4R,5R)-4-(3-Chlor-phenyl)-3-(4-chlor-phenyl)-2-(2,2-dimethyl-propyl)-5-((S)-2-hydroxymethyl-pyrrolidin-1-carbonyl)-pyrrolidin-3-carbonitril,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-2-(3,4-dimethoxy-phenyl)ethylamid,
(2S,3S,4S,5R)-3-(3-Chlor-phenyl)-4-(4-chlor-phenyl)-4-cyano-5-(2,2-dimethyl-propyl)-pyrrolidin-2-carbonsäure-((R)-1-hydroxymethyl-3-methyl-butyl)-amid,
rae-(2R,3R,4R,5S)-3-(3-Chlor-phenyl)-4-(4-chlor-phenyl)-4-cyano-5-(2,2-dimethylpropyl)-pyrrolidin-2-carbonsäure-(3-hydroxy-propyl)-amid,
rac-(2R,3S,4R,5S)-3-(3-Chlor-2-fluor-phenyl)-4-(4-chlor-phenyl)-4-cyano-5-(2,2-dimethyl-propyl)-pyrrolidin-2-carbonsäure-[2-(cis-2,6-dimethyl-morpholin-4-yl)-ethyl]-amid,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-(2-cyclopropyl-ethyl)-amid,
rac-(3-{[(2R,3R,4R,5S)-3-(3-Chlor-phenyl)-4-(4-chlor-phenyl)-4-cyano-5-(2,2-dimethyl-propyl)-pyrrolidin-2-carbonyl]-amino}-propyl)-carbamidsäure-tert-butylester,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-(3-amino-propyl)-amid,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-[3-(1-acetyl-piperidin-4-ylamino)-propyl]-amid,
rac-(2R,3R,4R,5S)-3-(3-Chlor-phenyl)-4-(4-chlor-phenyl)-4-cyano-5-isobutyl-pyrolidin-2-carbonsäure-(3-hydroxy-propyl)-amid,
rac-(2R,3R,4R,5R)-5-(3-Chlor-phenyl)-4-(4-chlor-phenyl)-4-cyano-3-(2,2-dimethylpropyl)-pyrrolidin-2-carbonsäure-(3-hydroxy-propyl)-amid,
rac-(2R,3R,4R,5S)-3-(3-Chlor-phenyl)-4-(4-chlor-phenyl)-4-cyano-5-(2,2-dimethylpropyl)-2-methyl-pyrrolidin-2-carbonsäure-(3-hydroxy-propyl)-amid,
rac-(2R,3S,4R,5S)-3-(3-Chlor-2-fluor-phenyl)-4-(4-chlor-phenyl)-4-cyano-5-cyclopentylmethyl-pyrrolidin-2-carbonsäure-((S)-3,4-dihydroxy-butyl)-amid,
rac-(2R,3S,4R,5S)-3-(3-Chlor-2-fluor-phenyl)-4-(4-chlor-phenyl)-4-cyano-5-(2,2-dimethyl-propyl)-pyrrolidin-2-carbonsäure-(2-hydroxy-1,1-dimethyl-ethyl)-amid,
rac-(2R,3S,4R,5S)-3-(3-Chlor-2-fluor-phenyl)-4-(4-chlor-phenyl)-4-cyano-5-(2,2-dimethyl-propyl)-pyrrolidin-2-carbonsäure-(3-hydroxy-2,2-dimethyl-propyl)-amid,
rac-(2R,3S,4R,5S)-3-(3-Chlor-2-fluor-phenyl)-4-(4-chlor-phenyl)-4-cyano-5-(2,2-dimethyl-propyl)-pyrrolidin-2-carbonsäure-[2-(2-hydroxy-ethoxy)-ethyl]-amid,
rac-(2R,3S,4R,5S)-3-(3-Chlor-2-fluor-phenyl)-4-(4-chlor-phenyl)-4-cyano-5-(2,2-dimethyl-propyl)-pyrrolidin-2-carbonsäure-(2-acetylamino-ethyl)-amid,
rac-(2R,3S,4R,5S)-3-(3-Chlor-2-fluor-phenyl)-4-(4-chlor-phenyl)-4-cyano-5-(2,2-dimethyl-propyl)-pyrrolidin-2-carbonsäure-(3-imidazol-1-yl-propyl)-amid,
rac-(2R,3S,4R,5S)-3-(3-Chlor-2-fluor-phenyl)-4-(4-chlor-phenyl)-4-cyano-5-(2,2-dimethyl-propyl)-pyrrolidin-2-carbonsäure-((R)-4-hydroxy-3-methyl-butyl)-amid,
rac-(2R,3S,4R,5S)-3-(3-Chlor-2-fluor-phenyl)-4-(4-chlor-phenyl)-4-cyano-5-(2,2-dimethyl-propyl)-pyrrolidin-2-carbonsäure-cyclopropylmethoxy-amid,
rac-(2R,3R,4R,5S)-3,5-bis-(3-Chlor-phenyl)-4-(4-chlor-phenyl)-4-cyano-pyrrolidin-2-carbonsäure-[2-((S)-2,2-dimethyl-[1,3]dioxolan-4-yl)-ethyl]-amid,
rac-(2R,3R,4R,5S)-3,5-bis-(3-Chlor-phenyl)-4-(4-chlor-phenyl)-4-cyano-pyrrolidin-2-carbonsäure-((S)-3,4-dihydroxy-butyl)-amid,
rac-(2R,3R,4R,5S)-3-(3-Chlor-phenyl)-4-(4-chlor-phenyl)-4-cyano-5-(1-ethyl-propyl)-pyrrolidin-2-carbonsäure-((S)-3,4-dihydroxy-butyl)-amid,
rac-(2R,3S,4R,5S)-3-(3-Chlor-2-fluor-phenyl)-4-(4-chlor-phenyl)-4-cyano-5-(2,2-dimethyl-propyl)-pyrrolidin-2-carbonsäure-((S)-3,4-dihydroxy-butyl)-amid,
(2R,3S,4R,5S)-3-(3-Chlor-2-fluor-phenyl)-4-(4-chlor-phenyl)-4-cyano-5-(2,2-dimethylpropyl)-pyrrolidin-2-carbonsäure-((S)-3,4-dihydroxy-butyl)-amid,
rac-(2R,3R,4R,5S)-3-(3-Chlor-phenyl)-4-(4-chlor-phenyl)-4-cyano-5-isobutyl-pyrrolidin-2-carbonsäure-((S)-3,4-dihydroxy-butyl)-amid,
rac-(2R,3R,4R,5R)-3-(3-Chlor-phenyl)-4-(4-chlor-phenyl)-4-cyano-5-isobutyl-pyrrolidin-2-carbonsäure-((S)-3,4-dihydroxy-butyl)-amid,
rac-(2R,3R,4R,5R)-3-(3-Chlor-phenyl)-4-(4-chlor-phenyl)-4-cyano-5-(1-ethyl-propyl)-pyrrolidin-2-carbonsäure-((S)-3,4-dihydroxy-butyl)-amid,
rac-(2R,3R,4R)-3-(3-chlor-phenyl)-4-(4-chlor-phenyl)-4-cyano-5,5-diethyl-pyrrolidin-2-carbonsäure-((S)-3,4-dihydroxy-butyl)-amid,
rac-(2R,3R,4R,5S)-3-(3-Chlor-phenyl)-4-(4-chlor-phenyl)-4-cyano-5-isopropyl-pyrrolidin-2-carbonsäure-((S)-3,4-dihydroxy-butyl)-amid,
rac-(2R,3R,4R,5S)-3-(3-Chlor-phenyl)-4-(4-chlor-phenyl)-4-cyano-5-cyclohexyl-pyrrolidin-2-carbonsäure-((S)-3,4-dihydroxy-butyl)-amid,
rac-(2R,3S,4R,5S)-5-tert-Butyl-3-(3-chlor-2-fluor-phenyl)-4-(4-chlor-phenyl)-4-cyano-pyrrolidin-2-carbonsäure-((S)-3,4-dihydroxy-butyl)-amid,
rac-(2R,3S,4R,5S)-3-(3-Chlor-2-fluor-phenyl)-4-(4-chloro-2-fluor-phenyl)-4-cyano-5-(2,2-dimethyl-propyl)-pyrrolidin-2-carbonsäure-((S)-3,4-dihydroxy-butyl)-amid und
(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-((S)-3,4-dihydroxy-butyl)-amid,
rac-(2S,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-((S)-3,4-dihydroxy-butyl)-amid,
rac-(2R,3S,4R,5S)-4-(4-Brom-phenyl)-3-(3-chlor-2-fluor-phenyl)-4-cyano-5-(2,2-dimethyl-propyl)-pyrrolidin-2-carbonsäure-((S)-3,4-dihydroxy-butyl)-amid,
rac-(2R,3S,4R,5S)-3-(3-Chlor-2-fluor-phenyl)-4-cyano-5-(2,2-dimethyl-propyl)-4-(4-fluor-phenyl)-pyrrolidin-2-carbonsäure-((S)-3,4-dihydroxy-butyl)-amid,
rac-(2R,3S,4R,5S)-3-(3-Chlor-2-fluor-phenyl)-4-cyano-4-(2,4-dichlor-phenyl)-5-(2,2-dimethyl-propyl)-pyrrolidin-2-carbonsäure-((S)-3,4-dihydroxy-butyl)-amid,
(2R,3S,4R,5S)-3-(3-Chlor-2-fluor-phenyl)-4-cyano-4-(2,4-dichlor-phenyl)-5-(2,2-dimethyl-propyl)-pyrrolidin-2-carbonsäure-((S)-3,4-dihydroxy-butyl)-amid,
rac-(2R,3S,4R,5S)-3-(3-Chlor-2-fluor-phenyl)-4-(4-chlor-2-methyl-phenyl)-4-cyano-5-(2,2-dimethyl-propyl)-pyrrolidin-2-carbonsäure-((S)-3,4-dihydroxy-butyl)-amid,
rac-(2R,3S,4R,5S)-3-(3-Chlor-2-fluor-phenyl)-4-(4-chlor-phenyl)-4-cyano-5-cyclohexylmethyl-pyrrolidin-2-carbonsäure-((S)-3,4-dihydroxy-butyl)-amid,
rac-(2S,3S,4R,5S)-3-(3-Chlor-2-fluor-phenyl)-4-(4-chlor-phenyl)-4-cyano-5-cyclohexylmethyl-pyrrolidin-2-carbonsäure-((S)-3,4-dihydroxy-butyl)-amid,
rac-(2R,3S,4R,5S)-4-(4-Chlor-2-fluor-phenyl)-4-cyano-3-(2,3-difluor-phenyl)-5-(2,2-dimethyl-propyl)-pyrrolidin-2-carbonsäure-((S)-3,4-dihydroxy-butyl)-amid,
(2R,3S,4R,5S)-4-(4-Chlor-2-fluor-phenyl)-4-cyano-3-(2,3-difluor-phenyl)-5-(2,2-dimethyl-propyl)-pyrrolidin-2-carbonsäure-((S)-3,4-dihydroxy-butyl)-amid,
rac-(2R,3S,4R,5S)-3-(3-Brom-2-fluor-phenyl)-4-(4-chlor-2-fluor-phenyl)-4-cyano-5-(2,2-dimethyl-propyl)-pyrrolidin-2-carbonsäure-((S)-3,4-dihydroxy-butyl)-amid und
(2R,3S,4R,5S)-3-(3-Brom-2-fluor-phenyl)-4-(4-chlor-2-fluor-phenyl)-4-cyano-5-(2,2-dimethyl-propyl)-pyrrolidin-2-carbonsäure-((S)-3,4-dihydroxy-butyl)-amid,
rac-(2R,3R,4R,5S)-4-(4-Chlor-2-fluor-phenyl)-3-(3-chlor-phenyl)-4-cyano-5-(2,2-dimethyl-propyl)-pyrrolidin-2-carbonsäure-((S)-3,4-dihydroxy-butyl)-amid,
(2R,3R,4R,5S)-4-(4-Chlor-2-fluor-phenyl)-3-(3-chlor-phenyl)-4-cyano-5-(2,2-dimethylpropyl)-pyrrolidin-2-carbonsäure-((S)-3,4-dihydroxy-butyl)-amid,
rac-(2R,3R,4R,5S)-4-(4-Chlor-2-fluor-phenyl)-4-cyano-5-(2,2-dimethyl-propyl)-3-(3-fluor-phenyl)-pyrrolidin-2-carbonsäure-((S)-3,4-dihydroxy-butyl)-amid,
rac-(2R,3R,4R,5S)-3-(3-Brom-phenyl)-4-(4-chlor-2-fluor-phenyl)-4-cyano-5-(2,2-dimethyl-propyl)-pyrrolidin-2-carbonsäure-((S)-3,4-dihydroxy-butyl)-amid,
rac-(2S,3R,4R,5S)-3-(3-Brom-phenyl)-4-(4-chlor-2-fluor-phenyl)-4-cyano-5-(2,2-dimethyl-propyl)-pyrrolidin-2-carbonsäure-((S)-3,4-dihydroxy-butyl)-amid,
rac-(2R,3R,4R,5S)-4-(4-Chlor-2-fluor-phenyl)-4-cyano-3-(3,4-dichlor-phenyl)-5-(2,2-dimethyl-propyl)-pyrrolidin-2-carbonsäure-((S)-3,4-dihydroxy-butyl)-amid,
rac-(2R,3R,4R,5S)-3-(3-Chlor-4-fluor-phenyl)-4-(4-chlor-2-fluor-phenyl)-4-cyano-5-(2,2-dimethyl-propyl)-pyrrolidin-2-carbonsäure-((S)-3,4-dihydroxy-butyl)-amid,
(2R,3R,4R,5S)-3-(3-Chlor-4-fluor-phenyl)-4-(4-chlor-2-fluor-phenyl)-4-cyano-5-(2,2-dimethyl-propyl)-pyrrolidin-2-carbonsäure-((S)-3,4-dihydroxy-butyl)-amid,
rac-(2R,3R,4R,5S)-3-(4-Brom-thiophen-2-yl)-4-(4-chlor-2-fluor-phenyl)-4-cyano-5-(2,2-dimethyl-propyl)-pyrrolidin-2-carbonsäure-((S)-3,4-dihydroxy-butyl)-amid,
rac-(2R,3R,4R,5S)-3-(3-Chlor-4-fluor-phenyl)-4-(4-chlor-2-fluor-phenyl)-4-cyano-5-(2,2-dimethyl-propyl)-pyrrolidin-2-carbonsäure-((R)-3,4-dihydroxy-butyl)-amid,
(2R,3S,4R,5S)-3-(3-Chlor-2-fluor-phenyl)-4-(4-chlor-2-fluor-phenyl)-4-cyano-5-(2,2,3-trimethyl-butyl)-pyrrolidin-2-carbonsäure-((R)-3,4-dihydroxy-butyl)-amid,
(2R,3R,4R,5S)-3-(3-Chlor-4-fluor-phenyl)-4-(4-chlor-2-fluor-phenyl)-4-cyano-5-(2,2-dimethyl-propyl)-pyrrolidin-2-carbonsäure-((R)-3,4-dihydroxy-butyl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-((R)-3,4-dihydroxy-butyl)-amid,
(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-((R)-3,4-dihydroxy-butyl)-amid,
rac-(2R,3R,4R,5S)-4-(4-Chlor-2-fluor-phenyl)-3-(3-chlor-phenyl)-4-cyano-5-(2,2-dimethyl-propyl)-pyrrolidin-2-carbonsäure-((R)-3,4-dihydroxy-butyl)-amid,
rac-(2R,3S,4R,5S)-3-(3-Chlor-2-fluor-phenyl)-4-(4-chlor-phenyl)-4-cyano-5-(2,2-dimethyl-propyl)-pyrrolidin-2-carbonsäure-((R)-3,4-dihydroxy-butyl)-amid,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-((R)-3,4-dihydroxy-butyl)-amid,
rac-(2R,3S,4R,5S)-3-(3-Chlor-2-fluor-phenyl)-4-(4-chlor-2-fluor-phenyl)-4-cyano-5-(2,2,3-trimethyl-butyl)-pyrrolidin-2-carbonsäure-((S)-3,4-dihydroxy-butyl)-amid,
(2R,3S,4R,5S)-3-(3-Chlor-2-fluor-phenyl)-4-(4-chlor-2-fluor-phenyl)-4-cyano-5-(2,2,3-trimethyl-butyl)-pyrrolidin-2-carbonsäure-((S)-3,4-dihydroxy-butyl)-amid,
rac-(2R,3S,4R,5S)-3-(3-Chlor-2-fluor-phenyl)-4-(4-chlor-2-fluor-phenyl)-4-cyano-5-(2,2,3-trimethyl-butyl)-pyrrolidin-2-carbonsäure-((R)-3,4-dihydroxy-butyl)-amid,
rac-(2R,3S,4R,5S)-3-(3-Chlor-2-fluor-phenyl)-4-(4-chlor-2-fluor-phenyl)-4-cyano-5-(2,2-dimethyl-but-3-enyl)-pyrrolidin-2-carbonsäure-((S)-3,4-dihydroxy-butyl)-amid,
rac-(2R,3S,4R,5S)-3-(3-Chlor-2-fluor-phenyl)-4-(4-chlor-2-fluor-phenyl)-4-cyano-5-(2,2-dimethyl-butyl)-pyrrolidin-2-carbonsäure-((S)-3,4-dihydroxy-butyl)-amid,
rac-(2R,3S,4R,5S)-3-(3-Chlor-2-fluor-phenyl)-4-(4-chlor-2-fluor-phenyl)-4-cyano-5-(2-methyl-2-phenyl-propyl)-pyrrolidin-2-carbonsäure-((S)-3,4-dihydroxy-butyl)-amid,
rac-(2R,3S,4R,5S)-4-(4-Chlor-2-fluor-phenyl)-3-(5-chloro-2-methoxy-phenyl)-4-cyano-5-(2,2-dimethyl-propyl)-pyrrolidin-2-carbonsäure-((S)-3,4-dihydroxy-butyl)-amid,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-(chinolin-3-ylmethyl)-amid,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-4-hydroxy-benzylamid,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-(2-ethyl-butyl)-amid,
rac-(2R,3R,4R,5S)-3-(3-Chlor-5-fluor-phenyl)-4-(4-chlor-2-fluor-phenyl)-4-cyano-5-(2,2-dimethyl-propyl)-pyrrolidin-2-carbonsäure-((S)-3,4-dihydroxy-butyl)-amid,
rac-(2R,3S,4R,5S)-4-(4-Chlor-2-fluor-phenyl)-3-[5-chlor-2-(2-hydroxy-ethoxy)-phenyl]-4-cyano-5-(2,2-dimethyl-propyl)-pyrrolidin-2-carbonsäure-((S)-3,4-dihydroxy-butyl)-amid,
rac-4-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-piperidin-1-carbonsäure-tert-butylester,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäurepiperidin-4-ylamid-trifluoressigsäure,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(1-methansulfonylpiperidin-4-yl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(1-methyl-carbonyl-piperidin-4-yl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(1-benzoyl-piperidin-4-yl)-amid,
rac-4-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-piperidin-1-carbonsäure-isopropylamid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-[1-(2-hydroxy-2-methyl-propyl)-1H-pyrazol-3-yl]-amid,
chiralem (2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-[1-(2-hydroxy-2-methyl-propyl)-1H-pyrazol-3-yl]-amid,
chiralem (2S,3R,4S,5R)-3-(3-Chlor-2-fluor-phenyl)-4-(4-chlor-2-fluor-phenyl)-4-cyano-5-(2,2-dimethyl-propyl)-pyrrolidin-2-carbonsäure-[1-(2-hydroxy-2-methyl-propyl)-1H-pyrazol-3-yl]-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-{1-[2-methyl-2-((R)-1-oxiranylmethoxy)-propyl]-1H-pyrazol-3-yl}-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-{1-[2-((R)-3-amino-2-hydroxy-propoxy)-2-methyl-propyl]-1H-pyrazol-3-yl}-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(1-{2-[(R)-2-hydroxy-3-(3-hydroxypropylamino)-propoxy]-2-methyl-propyl}-1H-pyrazol-3-yl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(1-{2-[(R)-2-hydroxy-3-(2-hydroxy-1-hydroxymethyl-ethylamino)-propoxy]-2-methyl-propyl}-1H-pyrazol-3-yl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure {1-[2-methyl-2-((S)-1-oxiranylmethoxy)-propyl]-1H-pyrazol-3-yl}-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-{1-[2-((S)-3-amino-2-hydroxy-propoxy)-2-methyl-propyl]-1H-pyrazol-3-yl}-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-{1-[2-((S)-2,3-dihydroxy-propoxy)-2-methyl-propyl]-1H-pyrazol-3-yl}-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-{1-[2-((S)-3-dimethylamino-2-hydroxy-propoxy)-2-methyl-propyl]-1H-pyrazol-3-yl)-amid,
rac-{(S)-3-[2-(3-{[(2R,3S,4R,5S)-3-(3-Chlor-2-fluor-phenyl)-4-(4-Chlor-2-fluorphenyl)-4-cyano-5-(2,2-dimethyl-propyl)-pyrrolidin-2-carbonyl]-amino}-pyrazol-1-yl)-1,1-dimethyl-ethoxy]-2-hydroxy-propylamino}-essigsäure,
rac-(2R,3S,4R,5S)-3-(3-Chlor-2-fluor-phenyl)-4-(4-fluor-2-fluor-phenyl)-4-cyano-5-(2,2-dimethyl-propyl)-pyrrolidin-2-carbonsäure-{1-[2-((S)-2-hydroxy-3-methylamino-propoxy)-2-methyl-propyl]-1H-pyrazol-3-yl}-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-{1-[2-((R)-3-dimethylamino-2-hydroxy-propoxy)-2-methyl-propyl]-1H-pyrazol-3-yl)-amid,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-[1-((R)-2,3-dihydroxy-propyl)-1H-pyrazol-3-yl]-amid,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-[1-(2-hydroxy-ethyl)-1H-pyrazol-3-yl]-amid,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-((S)-2,3-dihydroxy-propyl)-amid,
rac-(2S,3S,4S,5R)-3-(3-Chlor-phenyl)-4-(4-chlor-phenyl)-4-cyano-5-(2,2-dimethylpropyl)-pyrrolidin-2-carbonsäure-((S)-2,3-dihydroxy-propyl)-amid,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-[1-((R)-2,2-dimethyl-[[1,3]dioxolan-4-ylmethyl)-1H-pyrazol-3-yl]-amid,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-2-trifluomethyl-benzylamid,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-4-(2-oxo-pyrrolidin-1-yl)-benzylamid,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-(tetrahydro-pyran-4-ylmethyl)-amid,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-(3-hydroxy-2-hydroxymethyl-propyl)-amid,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-[2-(2-amino-ethoxy)-ethyl]-amid,
rac-(2R,3R,4R,5S)-3-(3-Chlor-phenyl)-4-(4-chlor-phenyl)-4-cyano- 5-(2,2-dimethylpropyl)-pyrolidin-2-carbonsäure-(3-methansulfonyl-propyl)-amid,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-(2-methansulfonyl-ethyl)-amid,
rac-4- {[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-cyclohexylamino-1-carbonsäure-tert-butylester,
rac-4- [{2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-trans-cyclohexylamin-trifluoressigsäuresalz,
rac-4-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-trans-cyclohexyl-N-methansulfonamid,
rac-4-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-trans-cyclohexyl-N-methansulfonamid,
rac-4- {[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-trans-cyclohexyl-(1,1-dioxo)-2-isothiazolidin,
rac-4-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-trans-cyclohexyl-harnstoff,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-N-[1-(2-hydroxyethyl)-piperidin-4-yl]amid,
rac-4- {[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-piperidin-1-sulfonsäureamid,
rac-3-{4-[(2R,3S,4R,5S)-3-(3-Chlor-2-fluor-phenyl)-4-(4-chlor-2-fluo-phenyl)-4-cyano-5-(2,2-dimethyl-propyl)-pyrrolidin-2-carbonyl]-piperazin-1-yl}-essigsäure,
rac-3-{4-[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-piperazin-1-y]}-N,N-bis-(2-methoxy-ethyl)-acetamid,
rac-3- {4-[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-piperazin-1-yl}-N,N-bis-(2-hydroxy-ethyl)-acetamid,
rac-3-{4-[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-piperazin-1-yl}-N-(3-methoxypropyl)-acetamid,
rac-2-{4-[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-piperazin-1-yl}-acetamid,
rac-(2S,3R,4S,5R)-4-(3-Chlor-2-fluor-phenyl)-3-(4-chlor-2-fluor-phenyl)-2-(2,2-dimethyl-propyl)-5-[4-(2-morpholin-4-yl-2-oxo-ethyl)-piperazin-1-carbonyl]-pyrrolidin-3-carbonitril,
rac-2- {4-[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-piperazin-1-yl}-N-[2-((S)-2,2-dimethyl-[1,3]dioxolan-4-yl)-ethyl]-acetamid,
rac-2-{4-[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-piperazin-1-yl}-N-((S)-3,4-dihydroxy-butyl)-acetamid,
rac-{1-[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-piperidin-4-yl}-essigsäure-methylester und
rac-{1-[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-piperidin-4-yl}-essigsäure-hydrochloridsalz.

7. Eine Verbindung nach Anspruch 1, ausgewählt aus der Gruppe bestehend aus
rac-2-{1-[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-piperidin-4-yl}-acetamid,
rac-2-{1-[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-piperidin-4-yl}-N,N-bis-(2-hydroxy-ethyl)-acetamid,
rac-2-{1-[{2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl)-piperidin-4-yl}-N-(2-hydroxy-ethyl)-N-methyl-acetamid,
rac-2-{1-[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-piperidin-4-yl}-N-(2-hydroxypropyl)-acetamid,
rac-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-essigsäure-tert-butylester,
rac-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-essigsäure-trifluoressigsäuresalz,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-carbamoylmethyl-amid,
{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-essigsäure-trifluoressigsäuresalz,
rac-3-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-benzoesäure-ethylester,
rac-(2R,3S,4R,5S)-3-(3-Chlor-fluor-phenyl)-4-(4-chlor-2-fluor-phenyl)-4-cyano-5-(2,2-dimethyl-propyl)-pyrrolidin-2-carbonsäure-(3-carbamoyl-phenyl)-amid,
rac-3-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-benzoesäure-tert-butylester,
rac-3-{[{2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-benzoesäure,
rac-(2R,35,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(3-hydroxymethyl-phenyl)-amid,
rac-(3-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-propyl)-carbamidsäure-tert-butylester,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(3-amino-propyl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-[3-(aminosulfonyl-amino)-propyl]-amid,
rac-2-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-benzoesäure-tert-butylester,
rac-4-{[(2R,3S,4R,5S)-3-(3-Chlor-2-fluor-phenyl)-4-(4-chlor-2-fluor-phenyl]-4-cyano-5-(2,2-dimethyl-propyl)-pyrrolidin-2-carbonyl]-amino}-benzoesäure-tert-butylester,
rac-4-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-benzoesäure-ethylester,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(2-carbamoyl-phenyl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(4-carbamoyl-phenyl)-amid,
rac-2-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-benzoesäure-ethylester,
rac-4-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl)-amino}-benzoesäure,
rac-2-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-benzoesäure,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(3-cyano-phenyl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(2-hydroxy-2-methyl-propyl)-amid,
rac-3-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-benzoesäure-2-hydroxy-2-methyl-propylester,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(3-methansulfonylamino-phenyl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(1H-tetrazol-5-ylmethyl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(3-ureado-propyl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(3-methylsulfanyl-phenyl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(3-methansulfonyl-phenyl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(3-methansulfinyl-phenyl)-amid,
3-[{2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-benzoesäure,
(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(3-carbamoyl-phenyl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-[3-(1H-tetrazol-5-yl)-phenyl]-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-[4-(1H-tetrazol-5-yl)-phenyl]-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(4-amino-phenyl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(4-acetylamino-phenyl)-amid,
rac-2- {[(2R,3S,4R,5S)-3-(3-Chlor-2-fluorphenyl)-4-(4-chlor-2-fluor-phenyl)-4-cyano-5-(2,2-dimethyl-propyl)-pyrrolidin-2-carbonyl]-amino}-thiazol-4-carbonsäure-ethylester,
rac-(2R,3S,4R,5S)-3-(3-Chlor-2-fluor-phenyl)-4-(4-chlor-2-fluor-phenyl)-4-cyano-5 - (2,2-dimethyl-propyl)-pyrrolidin-2-carbonsäure-(1,3-dioxo-2,3-dihydro-1H-isoindol-5-yl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(6-oxo-1,6-dihydro-pyridin-3-yl)-amid,
(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(6-oxo-1,6-dihydro-pyridin-3-yl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(4-methylsulfanyl-phenyl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(4-methansulfonyl-phenyl)-amid,
4- {[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-benzoesäure-tert-butylester,
4-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-benzoesäure,
4- {[(2S,3R,4S,5R)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-benzoesäure,
(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(4-carbamoyl-phenyl)-amid)
(25,3R,45,5R)-3-(3-Chlor-2-fluor-phenyl)-4-(4-chlor-2-fluor-phenyl)-4-cyano-5-(2,2-dimethyl-propyl)-pyrrolidin-2-carbonsäure-(4-carbamoyl-phenyl)-amid,
rac-(2R,35,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(4-methansulfanylamino-phenyl)-amid,
rac-(2R,3S,4R,5S)-3-(3-Chlor-2-fluor-phenyl)-4-(4-chlor-2-fluor-pbenyl)-4-cyano-5-(2,2-dimethyl-propyl)-pyrrolidin-2-carbonsäure-(4-trifluor-methansulfonylamino-phenyl)-amid,
rac-(2R,3R,4R,5S)-4-(4-Chlor-2-fluor-phenyl)-3-(3-chlor-2-methyl-phenyl)-4-cyano-5-(2,2-dimethyl-propyl)-pyrrolidin-2-carbonsäure-[3-(1-methyl-1H-tetrazol-5-yl)-phenyl]-amid,
rac-(2R,3R,4R,5S)-4-(4-Chlor-2-fluor-phenyl)-3-(3-chlor-2-methyl-phenyl)-4-cyano-5-(2,2-dimethyl-propyl)-pyrrolidin-2-carbonsäure-[3-(2-methyl-1H-tetrazo]-5-yl)-phenyl]-amid,
(2S,3R,4S,5R)-3-(3-Chlor-2-fluor-phenyl)-4-(4-chlor-2-fluor-phenyl)-4-cyano-5-(2,2-dimethyl-propyl)-pyrrolidin-2-carbonsäure-(6-oxo-1,6-dihydro-pyridin-3-yl)-amid,
(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-[4-(1H-tetrazol-5-yl)-phenyl]-amid,
(2S,3R,4S,5R)-3-(3-Chlor-2-fluor-phenyl)-4-(4-chlor-2-fluor-phenyl)-4-cyano-5-(2,2-dimethyl-propyl)-pyrrolidin-2-carbonsäure-[4-(1H-tetrazol-5-yl)-phenyl]-amid,
rac-(2R,3R,4R,5S)-4-(4-Chlor-2-fluor-phenyl)-3-(3-chlor-2-methyl-phenyl)-4-cyano-5-(2,2-dimethyl-propyl)-pyrrolidin-2-carbonsäure-[4-(2-methyl-1H-tetrazol-5-yl)-phenyl]-amid,
rac-(2R,3R,4R,5S)-4-(4-Chlor-2-fluor-phenyl)-3-(3-chlor-2-methyl-phenyl)-4-cyano-5-(2,2-dimethyl-propyl)-pyrrolidin-2-carbonsäure-[4-(1-methyl-1H-tetrazol-5-yl)-phenyl]-amid,
rac-5-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-2-fluor-benzoesäure-ethylester,
(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-[3-(1H-tetrazol-5-yl)-phenyl]-amid,
(2S,3R,4S,5R)-3-(3-Chlor-2-fluor-phenyl)-4-(4-chlor-2-fluor-phenyl)-4-cyano-5-(2,2-dimethyl-propyl)-pyrrolidin-2-carbonsäure-[3-(1H-tetrazol-5-yl)-phenyl]-amid,
(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(4-carbamoyl-3-chlor-phenyl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-[3-chlor-4-(1H-tetrazol-5-yl)-phenyl]-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(4-fluor-phenyl)-amid,
rac-(2R,3S,4R,5S)-3-(3-Chlor-2-fluor-phenyl)-4-(4-chlor-2-fluor-phenyl)-4-cyano-5-(2,2-dimnethyl-propyl)-pyrrolidin-2-carbonsäure-(3-fluor-phenyl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(3-chlor-phenyl)-amid,
rac-(2R,35,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(4-chlor-phenyl)-amid,
rac-4-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-2-fluor-benzoesäure-tert-butylester,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(4-ethylcarbamoyl-3-fluor-phenyl)-amid,
rac-4-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-2-fluor-benzoesäure,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(6-methoxy-pyridin-3-yl)-amid,
rac-3-({[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino]-methyl)-benzoesäure-methylester,
rac-4-({[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-methyl)-benzoesäure-methylester,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(4-chlor-phenyl)-amid,
(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(4-chlor-phenyl)-amid,
(2S,3R,4S,5R)-3-(3-Chlor-2-fluor-phenyl)-4-(4-chlor-2-fluor-phenyl)-4-cyano-5-(2,2-dimethyl-propyl)-pyrrolidin-2-carbonsäure-(4-chlor-phenyl)-amid,
rac-4- {[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-2-methoxy-benzoesäure-methylester,
rac-3-({[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-methyl)-benzoesäure,
rac-4-({(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-methyl)-benzoesäure,
(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(4-acetylamino-phenyl)-amid,
(2S,3R,4S,5R)-3-(3-Chlor-2-fluor-phenyl)-4-(4-chlor-2-fluor-phenyl)-4-cyano-5-(2,2-dimethyl-propyl)-pyrrolidin-2-carbonsäure-(4-acetylamino-phenyl)-amid,
(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(4-methansulfonyl-phenyl)-amid,
(2S,3R,4S,5R)-3-(3-Chlor-2-fluor-phenyl)-4-(4-chlor-2-fluor-phenyl)-4-cyano-5-(2 ,2-dimethyl-propyl)-pyrrolidin-2-carbonsäure-(4-methansulfonyl-phenyl)-amid,
rac-4- {[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-2-methoxy-benzoesäure,
rac-5-Brom-4-{[2R,3S,4R,5S)-3-(3-chlor-2-fluor-phenyl)-4-(4-chlor-2-fluor-phenyl)-4-cyano-5-(2,2-dimethyl-propyl)-pyrrolidin-2-carbonyl]-amino}-2-methoxy-benzoesäure-methylester,
rac-4-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-2-methyl-benzoesäure-methylester,
rac-2-Chlor-4- {[(2R,3S,4R,5S)-3-(3-chlor-2-fluor-phenyl)-4-(4-chlor-2-fluor-phenyl)-4-cyano-5-(2,2-dimethyl-propyl)-pyrrolidin-2-carbonyl]-amino}-benzoesäure-methylester,
rac-4- {[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-2-trifluormethyl-benzoesäure-methylester,
rac-4- [{(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-2-methyl-benzoesäure,
rac-2-Chlor-4-{[(2R,3S,4R,5S)-3-(3-chlor-2-fluor-phenyl)-4-(4-chlor-2-fluor-phenyl)-4-cyano-5-(2,2-dimethyl-propyl)-pyrrolidin-2-carbonyl]-amino}-benzoesäure, rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-[4-(2H-[1,2,4]triazol-3-yl)-phenyl]-amid, rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-[4-(5-oxo-2,5-dihydro-1H-[1,2,4] tiazol-3-yl)-phenyl]-amid, rac-(2R,3S,4R,5S)-3-(5-Chlor-2-fluor-phenyl)-4-(4-chlor-2-fluor-phenyl)-4-cyano-5-(2,2-dimethyl-propyl)-pyrrolidin-2-carbonsäure-((S)-3,4-dihydroxy-butyl)-amid, rac-(2R,3S,4R,5S)-3-(3-Chlor-2-fluor-phenyl)-4-(4-chlor-2-fluor-phenyl)-4-cyano-5-(2-cyclopropyl-2-methyl-propyl)-pyrrolidin-2-carbonsäure-((S)-3,4-dihydroxy-butyl)-amid, (2R,3S,4R,5S)-3-(3-Chlor-2-fluor-phenyl)-4-(4-chlor-2-fluor-phenyl)-4-cyano-5-(2-cyclopropyl-2-methyl-propyl)-pyrrolidin-2-carbonsäure-((S)-3,4-dihydroxy-butyl)-amid, rac-(2R,3S,4R,5S)-3-(3-Chlor-2-fluor-phenyl)-4-(4-chlor-3-methyl-phenyl)-4-cyano-5-(2,2-dimethyl-propyl)-pyrrolidin-2-carbonsäure-((S)-3,4-dihydroxy-butyl)-amid, rac-(2R,3S,4R,5S)-3-(3-Chlor-2-fluor-phenyl)-4-(4-chlor-2-fluor-phenyl)-4-cyano-5-(tetrahydro-pyran-4-ylmethyl)-pyrrolidin-2-carbonsäure-((S)-3,4-dihydroxy-butyl)-amid, rac-(2R,3S,4R,5S)-3-(3-Chlor-2-fluor-phenyl)-4-(4-chlor-2-fluor-phenyl)-4-cyano-5-(1 - methyl-cyclohexylmethyl)-pyrrolidin-2-carbonsäure-((S)-3,4-dihydroxy-butyl)-amid, (2R,3S,4R,5S)-3-(3-Chlor-2-fluor-phenyl)-4-(4-chlor-2-fluor-phenyl)-4-cyano-5-(1-methyl-cyclohexylmethyl)-pyrrolidin-2-carbonsäure-((S)-3,4-dihydroxy-butyl)-amid, rac-(2R,3S,4R,5S)-5-(2-Benzyloxycarbonyl-2-methyl-propyl)-3-(3-chlor-2-fluorphenyl)-4-(4-chlor-2-fluor-phenyl)-4-cyano-pyrrolidin-2-carbonsäure-((S)-3,4-dihydroxy-butyl)-amid, rac-(2R,3S,4R,5S)-3-(3-Chlor-2-fluor-phenyl)-4-(4-chlor-2-fluor-phenyl)-4-cyano-5-(2-methoxycarbonyl-2-methyl-propyl)-pyrrolidin-2-carbonsäure-((S)-3,4-dihydroxy-butyl)-amid, rac-(2R,3S,4R,5S)-3-(3-Chlor-2-fluor-phenyl)-4-(4-chlor-2-fluor-phenyl)-4-cyano-5-(3-hydroxy-2,2-dimethyl-propyl)-pyrrolidin-2-carbonsäure-((S)-3,4-dihydroxy-butyl)-amid, (2R,3S,4R,5S)-3-(3-Chlor-2-fluor-phenyl)-4-(4-chlor-2-fluor-phenyl)-4-cyano-5-(3-hydroxy-2,2-dimethyl-propyl)-pyrrolidin-2-carbonsäure-((S)-3,4-dihydroxy-butyl)-amid, rac-(2R,3S,4R,5S)-3-(3-Chlor-2-fluor-phenyl)-4-(4-chlor-2-fluor-phenyl)-4-cyano-5-(2,2-diethyl-butyl)-pyrrolidin-2-carbonsäure-((S)-3,4-dihydroxy-butyl)-amid, rac-(2R,3S,4R,5S)-3-(3-Chlor-2-fluor-phenyl)-4-(4-chlor-2-fluor-phenyl)-4-cyano-5-(2-ethyl-2-methyl-butyl)-pyrrolidin-2-carbonsäure-((S)-3,4-dihydroxy-butyl)-amid, rac-(2R,3R,4R,5S)-4-(4-Chlor-2,6-difluor-phenyl)-3-(3-chlor-phenyl)-4-cyano-5-(2,2-dimethyl-propyl)-pyrrolidin-2-carbansäure-((S)-3,4-dihydroxy-butyl)-amid, rac-(2R,3R,4R,5S)-4-(4-Chlor-2,5-difluor-phenyl)-3-(3-chlor-phenyl)-4-cyano-5-(2,2-dimethyl-propyl)-pyrrolidin-2-carbonsäure-((S)-3,4-dihydroxy-butyl)-amid, rac-(2R,3S,4R,5S)-3-(3-Chlor-2-fluor-phenyl)-4-(4-chlor-2-fluor-phenyl)-4-cyano-5-(3-methoxy-2,2-dimethyl-propyl)-pyrrolidin-2-carbonsäure-((S)-3,4-dihydroxy-butyl)-amid, (2R,3S,4R,5S)-3-(3-Chlor-2-fluor-phenyl)-4-(4-chlor-2-fluor-phenyl)-4-cyano-5-(3-methoxy-2,2-dimethyl-propyl)-pyrrolidin-2-carbonsäure-((S)-3,4-dihydroxy-butyl)-amid, rac-(2R,3S,4R,5S)-3-(3-Chlor-2-fluor-phenyl)-4-(4-chlor-2-fluor-phenyl)-4-cyano-5-(2-ethyl-2-hydroxymethyl-butyl)-pyrrolidin-2-carbonsäure-((S)-3,4-dihydroxy-butyl)-amid, rac-(2R,3S,4R,5S)-3-(3-Chlor-2-fluor-phenyl)-4-(4-chlor-2-fluor-phenyl)-4-cyano-5-(3-methyl-oxetan-3-ylmethyl)-pyrrolidin-2-carbonsäure-((S)-3,4-dihydroxy-butyl)-amid, rac-(2R,3S,4R,5S)-3-(3-Chlor-2-fluor-phenyl)-4-(4-chlor-2-fluor-phenyl)-4-cyano-5-(3-ethyl-oxetan-3-ylmethyl)-pyrrolidin-2-carbonsäure-((S)-3,4-dihydroxy-butyl)-amid, rac-(2R,3S,4R,5S)-3-(3-Chlor-2-fluor-phenyl)-4-(4-chlor-2-fluor-phenyl)-4-cyano-5-(1-hydroxymethyl-cyclopropylmethyl)-pyrrolidin-2-carbonsäure-((S)-3,4-dihydroxy-butyl)-amid, (2R,3S,4R,5S)-3-(3-Chlor-2-fluor-phenyl)-4-(4-chlor-2-fluor-phenyl)-4-cyano-5-(1-hydroxymethyl-cyclopropylmethyl)-pyrrolidin-2-carbonsäure-((S)-3,4-dihydroxy-butyl)-amid, rac-(2R,3S,4R,5S)-3-(3-Chlor-2-fluor-phenyl)-4-(4-chlor-2-fluor-phenyl)-4-cyano-5-(1-hydroxymethyl-cyclobutylmethyl)-pyrrolidin-2-carbonsäure-((S)-3,4-dihydroxy-butyl)-amid, (2R,3S,4R,5S)-3-(3-Chlor-2-fluor-phenyl)-4-(4-chlor-2-fluor-phenyl)-4-cyano-5-(1-hydroxymethyl-cyclobutylmethyl)-pyrrolidin-2-carbonsäure-((S)-3,4-dihydroxy-butyl)-amid, rac-(2R,3S,4R,5S)-3-(3-Chlor-2-fluor-phenyl)-4-(4-chlor-2-fluor-phenyl)-4-cyano-5-(4-hydroxy-2,2-dimethyl-butyl)-pyrrolidin-2-carbonsäure-((S)-3,4-dihydroxy-butyl)-amid, (2R,3S,4R,5S)-3-(3-Chlor-2-fluor-phenyl)-4-(4-chlor-2-fluor-phenyl)-4-cyano-5-(4-hydroxy-2,2-dimethyl-butyl)-pyrrolidin-2-carbansäure-((S)-3,4-dihydroxy-butyl)-amid,
rac-(2R,3S,4R,5S)-3-(3-Chlor-2-fluor-phenyl)-4-(4-chlor-2-fluor-phenyl)-4-cyano-5-(1-hydroxmethyl-cyclohex-3-enylmethyl)-pyrrolidin-2-carbonsäure-((S)-3,4-dihydroxy-butyl)-amid,
rac-(2R,3S,4R,5S)-3-(3-Chlor-2-fluor-phenyl)-4-(4-chlor-2-fluor-phenyl)-4-cyano-5-(1-hydroxymethyl-cyclohexylmethyl)-pyrrolidin-2-carbonsäure-((S)-3,4-dihydroxy-butyl)-amid,
rac-(2R,3S,4R,5S)-3-(3-Chlor-2-fluor-phenyl)-4-(4-chlor-2-fluor-phenyl)-4-cyano-5-[4-(2-hydroxy-ethoxy)-2,2-dimethyl-butyl)-pyrrolidin-2-carbonsäure-((S)-3,4-dihydroxy-butyl)-amid,
rac-(2R,3S,4R,5S)-5-(4-Azido-2,2-dimethyl-butyl)-3-(3-chlor-2-fluor-phenyl)-4-(4-chlor-2-fluor-phenyl)-4-cyano-pyrrolidin-2-carbonsäure-((S)-3,4-dihydroxy-butyl)-amid,
rac-(2R,3 S,4R,5S)-5-(4-Amino-2,2-dimethyl-butyl)-3-(3-chlor-2-fluor-phenyl)-4-(4-chlor-2-fluor-phenyl)-4-cyano-pyrrolidin-2-carbansäure-((S)-3,4-dihydroxy-butyl)-amid,
rac-(2R,3S,4R,5S)-5-(4-Acetylamino-2,2-dimethyl-butyl)-3-(3-chlor-2-fluor-phenyl)-4-(4-chlor-2-fluor-phenyl)-4-cyano-pyrrolidin-2-carbonsäure-((S)-3,4-dihydroxy-butyl)-amid,
rac-(2R,3S,4R,5S)-3-(3-Chlor-2-fluor-phenyl)-4-(4-chlor-2-fluor-phenyl)-4-cyano-5-(4-methansulfonylamino-2,2-dimethyl-butyl)-pyrrolidin-2-carbonsäure-((S)-3,4-dihydroxy-butyl)-amid,
rac-(2R,3S,4R,5S)-5-(4-Benzoylamino-2,2-dimethyl-butyl)-3-(3-chlor-2-fluor-phenyl)-4-(4-chlor-2-fluor-phenyl)-4-cyano-pyrrolidin-2-carbonsäure-((S)-3,4-dihydroxy-butyl)-amid,
rac-(2R,3S,4R,5S)-3-(3-Chlor-2-fluor-phzenyl)-4-(4-chlor-2-fluor-phenyl)-4-cyano-5-[2-methyl-2-(5-methyl-furan-2-yl)-propyl]-pyrrolidin-2-carbonsäure-((S)-3,4-dihydroxy-butyl)-amid,
rac-(2R,3S,4R,5S)-3-(3-Chlor-2-fluor-phenyl)-4-(4-chlor-2-fluor-phenyl)-4-cyano-5-[3-(4-methoxy-phenyl)-2,2-dimethyl-propyl]-pyrrolidin-2-carbonsäure-((S)-3,4-dihydroxy-butyl)-amid,
rac-(2R,3S,4R,5S)-5-[2-(1-Benzyl-1,2,3,6-tetrahydro-pyridin-4-yl)-2-methyl-propyl]-3-(3-chlor-2-fluor-phenyl)-4-(4-chlor-2-fluor-phenyl)-4-cyano-pyriolidin-2-carbonsäure-((S)-3,4-dihydroxy-butyl)-amid,
rac-(2R,3S,4R,5S)-5-[2-(1-Benzyl-piperidin-4-yl)-2-methyl-propyl]-3-(3-chlor-2-fluorphenyl)-4-(4-chlor-2-fluor-phenyl)-4-cyano-pyrrolidin-2-carbonsäure-((S)-3 ,4-dihydroxy-butyl)-amid,
rac-(2R,3S,4R,5S)-3-(3-Chlor-2-fluor-phenyl)-4-(4-chlor-2-fluor-phenyl)-4-cyano-5-[2-(3,6-dihydro-2H-pyran-4-yl)-2-methyl-propyl]-pyrrolidin-2-carbonsäure-((S)-3,4-dihydroxy-butyl)-amid,
rac-(2R,3S,4R,5S)-3-(3-Chlor-2-fluor-phenyl)-4-(4-chlor-2-fluor-phenyl)-4-cyano-5-[2-methyl-2-(tetrahydro-pyran-4-yl)-propyl]-pyrrolidin-2-carbonsäure-((S)-3,4-dihydroxy-butyl)-amid,
rac-(2R,3S,4R,5S)-3-(3-Chlor-2-fluor-phenyl)-4-(4-chlor-2-fluor-phenyl)-4-cyano-5-(3-hydroxy-2,2-dimethyl-propyl)-pyrrolidin-2-carbonsäure-((S)-3,4-dihydroxy-4-methylpentyl)-amid,
(2R,3S,4R,5S)-3-(3-Chlor-2-fluor-phenyl)-4-(4-chlor-2-fluor-phenyl)-4-cyano-5-(3-hydroxy-2,2-dimethyl-propyl)-pyrrolidin-2-carbonsäune-((S)-3,4-dihydroxy-4-methylpentyl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-((S)-3,4-dihydroxy-4-methyl-pentyl)-amid,
(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-((S)-3,4-dihydrox-4-methyl-pentyl)-amid,
rac-(2R,3S,4R,5S)-3-(3-Chlor-2-fluor-phenyl)-4-(4-chlor-2-fluor-phenyl)-4-cyano-5-(1-methyl-cyclohexylmethyl)-pyrrolidin-2-carbonsäure-((S)-3,4-dihydroxy-4-methylpentyl)-amid,
(2R,3S,4R,5S)-3-(3-Chlor-2-fluor-phenyl)-4-(4-chlor-2-fluor-phenyl)-4-cyano-5-(1-methyl-cyclohexylmethyl)-pyrrolidin-2-carbonsäure-((S)-3,4-dihydroxy-4-methylpentyl)-amid,
rae-(2S,3R,4S,5R)-4-(3-Chlor-2-fluor-phenyl)-3-(4-chlor-2-fluor-phenyl)-2-(2,2-dimethyl-propyl)-5-(3-hydroxy-azetidin-1-carbonyl)-pyrrolidin-3-carbonitril,
rac-(2R,3S,4R,5S)-3-(3-Chlor-2-fluor-phenyl)-4-(4-chlor-2-fluor-phenyl)-4-cyano-5-(3-hydroxy-2,2-dimethyl-propyl)-pyrrolidin-2-carbonsäure-[1-(2-hydroxy-2-methylpropyl)-1H-pyrazol-3-yl]-amid,
(2R,3S,4R,5S)-3-(3-Chlor-2-fluor-phenyl)-4-(4-chlor-2-fluor-phenyl)-4-cyano-5-(3-hydroxy-2,2-dimethyl-propyl)-pyrrolidin-2-carbonsäure-[1-(2-hydroxy-2-methylpropyl)-1H-pyrazol-3-yl]-amid,
rac-(2R,3S,4R,5S)-3-(3-Chlor-2-fluor-phenyl)-4-(4-chlor-2-fluor-phenyl)-4-cyano-5-(4-hydroxy-2,2-dimethyl-butyl)-pyrrolidin-2-carbonsäure-[1-(2-1)ydroxy-2-methyl-propyl)-1H-pyrazol-3-yl]-amid,
(2R,3S,4R,5S)-3-(3-Chlor-2-fluor-phenyl)-4-(4-chlor-2-fluor-phenyl)-4-cyano-5-(4-hydroxy-2,2-dimethyl-butyl)-pyrrolidin-2-carbonsäure-[1-(2-hydroxy-2-methyl-propyl)-1H-pyrazol-3-yl]-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäureamid,
rac-6-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-nikotinsäure-methylester,
rac-6-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-N-(2-hydroxy-1,1-dimethyl-ethyl)-nikotinamid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(6-acetylamino-pyridin-3-yl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure (1-methyl-2-oxo-1,2-dihydro-pyridin-4-yl)-amid,
(2R,3S,4R,5S)-3-(3-Chlor-2-fluor-phenyl)-4-(4-fluor-2-fluor-phenyl)-4-cyano-5-(2,2-dimethyl-propyl)-pyrrolidin-2-carbonsäure-(1-methyl-2-oxo-1,2-dihydro-pyridin-4-yl)-amid,
rac-(2R,3S,4R,5S)-3-(3-Clrlor-2-fluor-phenyl)-4-(4-chlor-2-fluor-phenyl)-4-cyano-5-(2,2-dimethyl-propyl)-pyrrolidin-2-carbonsäure-[5-((S)-1,2-dihydroxy-ethyl)-pyrazin-2-yl]-amid,
rac-(2R,3S,4R,5S)-3-(3-Chlor-2-fluor-phenyl)-4-(4-chlor-2-fluor-phenyl)-4-cyano-5-(4-hydroxy-2,2-dimethyl-butyl)-pyrrolidin-2-carbonsäure-(1-methyl-2-oxo-1,2-dihydro-pyridin-4-yl)-amid,
rac-5-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-furan-2-carbonsäuremethylester,
rac-5- {[(2R,3S,4R,5S)-3-(3-Chlor-2-fluor-phenyl)-4-(4-clrlor-2-fluor-phenyl)-4-cyano-5-(2,2-dimethyl-propyl)-pyrrolidin-2-carbonyl]-amino}-furan-2-carbonsäure,
rac-5-{[(2R,3S,4R,5S)-3-(3-Chlor-2-fluor-phenyl)-4-(4-chlor-2-fluor-phenyl)-4-cyano-5-(2,2-dimethyl-propyl)-prolidin-2-carbonyl]-amino}-furan-2-carbonsäureamid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(6-chlor-pyridazin-3-yl)-amid,
rac-(2R,3S,4R,5S)-3-(3-Chlor-2-fluor-phenyl)-4-(4-chlor-2-fluor-phenyl)-4-cyano-5-(2,2-dimthyl-propyl)-pyrrolidin-2-carbonsäure-(2-methyl-pyridin-3-yl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-[4-(2-hydroxy-ethoxy)-phenyl]-amid,
(2R,3S,4R,5S)-3-(3-Chlor-2-fluor-phenyl)-4-(4-chlor-2-fluor-phenyl)-4-cyano-5-(2,2-dimthyl-propyl)-pyrrolidin-2-carbonsäure-[4-(2-hydroxy-ethoxy)-phenyl]-amid,
rac-5-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-thiophen-2-carbonsäuremethylester,
rac-5- {[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-thiophen-2-carbonsäure,
5-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-thiophen-2-carbonsäure,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(2-methoxy-pyridin-4-yl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(2-hydroxy-pyridin-4-yl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(4-acetyl-phenyl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-[4-(2-brom-acetyl)-phenyl]-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-[4-(2-dimethylamino-acetyl)-phenyl]-amid,
rac-(5-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-4H-[1,2,4]triazol-3-yl)-essigsäure,
rac-(3- {[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-pyrazol-1-yl)-essigsäure,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure (1H-imidazol-4-ylmethyl)-amid,
rac-(2S,3R,4S,5R)-4-(3-Chlor-2-fluor-phenyl)-3-(4-chlor-2-fluor-phenyl)-2-(2,2-dimethyl-propyl)-5-(2-oxa-6-aza-spiro[3.3]heptan-6-carbonyl)-pyrrolidin-3-carbonitril,
rac-1-[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-azetidin-3-carbonsäure,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(2-[1,2,3]triazol-1-yl-ethyl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(1-carbamoylmethyl-1H-pyrazol-3-yl)-amid,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-[1-(2-hydroxy-2-methyl-propyl)-1H-pyrazol-3-yl]-amid,
rac-(2R,3R,4R,5S)-3-(3-Chlor-phenyl)-4-(4-chlor-phenyl)-4-cyano-5-(2,2-dimethylpropyl)-pyrrolidin-2-carbonsäure-(3-methansulfonylamino-propyl)-amid,
chiralem (2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-{1-[2-((S)-3-dimethylamino-2-hydroxy-propoxy)-2-methyl-propyl]-1H-pyrazol-3-yl}-amid,
chiralem (2S,3R,4S,5R)-3-(3-Chlor-2-fluor-phenyl)-4-(4-chlor-2-fluor-phenyl)-4-cyano-5-(2,2-dimethyl-propyl)-pyrrolidin-2-carbonsäure-{1-[2-((S)-3-dimethylamino-2-hydroxy-propoxy)-2-methyl-propyl]-1H-pyrazol-3-yl}-amid,
rac-1-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-cyclopropancarbonsäure,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-[1-(4-hydroxy-piperidin-4-ylmethyl)-1H-pyrazol-3-yl]-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-(2-acetyl-thiophen-3-yl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsaure-(2-carbamoyl-thiophen-3-yl)-amid,
rac-(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonsäure-[1-((S)-3-dimethylamino-2-hydroxypropyl)-1H-pyrazol-3-yl]-amid,
rac-4- {[(2R,3S,4R,5S)-3-(3-Chlor-2-fluor-phenyl)-4-(4-chlor-2-fluor-phenyl)-4-cyano-5-(1-methyl-cyclohexylmethyl)-pyrrolidin-2-carbonyl]-amino}-benzoesäure,
rac-[4-(3-{[(2R,3S,4R,5S)-3-(3-Chlor-2-fluor-phenyl)-4-(4-chlor-2-fluor-phenyl)-4-cyano-5-(2,2-dimethyl-propyl)-pyrrolidin-2-carbonyl]-amino}-pyrazol-1-ylmethyl)-4-hydroxy-piperidin-1-yl]-essigsäure,
rac-4-{[(2R,3S,4R,5S)-3-(3-Chlor-2-fluor-phenyl)-4-(4-chlor-2-fluor-phenyl)-4-cyano-5-(1-methyl-cyclohexylmethyl)-pyrrolidin-2-carbonyl]-amino}-benzoesäure,
rac-4-{[(2R,3S,4R,5S)-3-(5-Chlor-2-fluor-phenyl)-4-(4-chlor-2-fluor-phenyl)-4-cyano-5-(2,2-dimethyl-propyl)-pyrrolidin-2-carbonyl]-amino}-benzoesäure-methylester,
rac-4-{[(2R,3S,4R,5S)-3-(5-Chlor-2-fluor-phenyl)-4-(4-chlor-2-fluor-phenyl)-4-cyano-5-(2,2-dimethyl-propyl)-pyrrolidin-2-carbonyl]-amino}-benzoesäure,
rac-4-{[(2R,3R,4R,5S)-3-(3-Chlor-4-fluor-phenyl)-4-(4-chlor-2-fluor-phenyl)-4-cyano-5-(2,2-dimethyl-propyl)-pyrrolidin-2-carbonyl]-amino}-benzoesäure-methylester,
rac-4-{[(2R,3R,4R,5S)-3-(3-Chlor-4-fluor-phenyl)-4-(4-chlor-2-fluor-phenyl)-4-cyano-5-(2,2-dimethyl-propyl)-pyrrolidin-2-carbonyl]-amino}-benzoesäure,
rac-4-{[(2R,3R,4R,5S)-3-(3-Brom-phenyl)-4-(4-chlor-2-fluor-phenyl)-4-cyano-5-(2,2-dimethyl-propyl)-pyrrolidin-2-carbonyl]-amino}-benzoesäure-methylester,
rac-4-{[(2R,3R,4R,5S)-3-(3-Brom-phenyl)-4-(4-chlor-2-fluor-phenyl)-4-cyano-5-(2,2-dimethyl-propyl)-pyrrolidin-2-carbonyl]-amino}-benzoesäure,
rac-4- {[(2R,3S,4R,5S)-3-(3-Chlor-2-fluor-phenyl)-4-(4-chlor-phenyl)-4-cyano-5-(2,2-dimethyl-propyl)-pyrrolidin-2-carbonyl]-amino}-benzoesäure-methylester,
rae-4-{[(2R,3S,4R,5S)-3-(3-Chlor-2-fluor-phenyl)-4-(4-chlor-phenyl)-4-cyano-5-(2,2-dimethyl-propyl)-pyrrolidin-2-carbonyl]-amino}-benzoesäure und
rac-4-{[(2R,3S,4R,5S)-3-(3-Chlor-2-fluor-phenyl)-4-(4-chlor-2-fluor-phenyl)-4-cyano-5-(1-methyl-cyclohexylmethyl)-pyrrolidin-2-carbonyl]-amino}-benzoesäure-methylester.

8. Ein Arzneimittel, umfassend eine Verbindung der Ansprüche 1 bis 7 zusammen mit pharmazeutisch verträglichen Exzipienten oder Trägem.

9. Eine Verbindung gemäß einem der Ansprüche 1 bis 7 zur Verwendung als Medikament.

10. Eine Verbindung gemäß einem der Ansprüche 1 bis 7 zur Verwendung als Medikament zur Behandlung von Krebs.

11. Eine Verbindung gemäß einem der Ansprüche 1 bis 7 zur Verwendung als Medikament zur Behandlung von soliden Tumoren.

12. Eine Verbindung gemäß einem der Absprüche 1 bis 7 zur Verwendung als Medikament zur Behandlung von Brust-, Darm-, Lungen- und Prostatatumoreil.

## Revendications

1. Composé de formule: 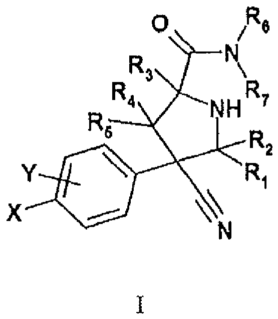 dans laquelle:
X est choisi dans le groupe constitué par H, F, Cl, Br, I, cyano, nitro, éthynyle, cyclopropyle, méthyle, éthyle, isopropyle, vinyle et méthoxy;
Y représente 1 à 4 groupes choisis indépendamment dans le groupe constitué par H, F, Cl, Br, I, CN, OH, nitro, alkyle en C₁-C₆, cycloalkyle, alcoxy en C₁-C₆, alcényle en C₂-C₆, cycloalcényle, alcynyle en C₂-C₆, aryle, hétéroaryle, hétérocycle, COOR', OCOR', CONR'R", NR'COR", NR"SO₂R', SO₂NR'R" et NR'R", où
R' et R" sont choisis indépendamment parmi H, alkyle en C₁-C₆ substitué ou non substitué, cycloalkyle en C₃-C₁₀ substitué ou non substitué, alcényle en C₂-C₆ substitué ou non substitué, cycloalcényle en C₅-C₁₀ substitué ou non substitué, aryle substitué ou non substitué, hétéroaryle substitué ou non substitué ou hétérocycle substitué ou non substitué; et R' et R" peuvent indépendamment se lier pour former une structure cyclique choisie parmi cycloalkyle substitué ou non substitué, cycloalcényle substitué ou non substitué, hétéroaryle substitué ou non substitué ou hétérocycle substitué ou non substitué;
l'un des R₁ et R₂ est choisi dans le groupe constitué par alkyle en C₁-C₆, alkyle en C₁-
C₆ substitué, alcényle en C₂-C₆. alcényle en C₂-C₆ substitué, aryle, aryle substitué, hétéroaryle, hétéroaryle substitué, hétérocycle, hétérocycle substitué, cycloalkyle, cycloalkyle substitué, cycloalcényle et cycloalcényle substitué, et l'autre est un hydrogène ou alkyle en C₁-C₆;
R₃ est H ou alkyle en C₁-C₆;
l'un des R₄ et R₅ est choisi dans le groupe constitué par alkyle en C₁-C₆, alkyle en C₁-C₆ substitué, alcényle en C₂-C₆, alcényle en C₂-C₆ substitué, aryle, aryle substitué, hétéroaryle, hétéroaryle substitué, hétérocycle, hétérocycle substitué, cycloalkyle, cycloalkyle substitué, cycloalcényle et cycloalcényle substitué, et l'autre est un hydrogène;
R₆ et R₇ sont choisis dans le groupe constitué par (CH₂)ₙ-R¹, (CH₂)ₙ-NR'R", (CH₂)ₙ-NR¹COR", (CH₂)ₙ-NR'SO₂R", (CH₂)ₙ-COOH, (CH₂)ₙCOOR', (CH₂)ₙ-CONR'R", (CH₂)ₙOR', (CH₂)ₙ-SR', (CH₂)ₙ-SOR', (CH₂)ₙ-SO₂R', (CH₂)ₙ-COR', (CH₂)ₙ-SO₃H, (CH₂)ₙ-SONR'R", (CH₂)ₙ-SO₂NR'R", (CH₂CH₂O)ₘ-(CH₂)ₙ-R', (CH₂CH₂O)ₘ-(CH₂)ₙ-OH, (CH₂CH₂O)ₘ-(CH₂)ₙ-OR', (CH₂CH₂O)ₘ-(CH₂)ₘ-NR'R", (CH₂CH₂O)ₘ-(CH₂)ₙ-NR'COR", (CH₂CH₂O)ₘ-(CH₂)"-NR'SO₂R", (CH₂CH₂O)ₘ-(CH₂)ₙ-COOH, (CH₂CH₂O)ₘ-(CH₂)ₙ-COOR', (CH₂CH₂O)ₘ-(CH₂)ₙ-CONR'R", (CH₂CH₂O)ₘ-(CH₂)ₙ-SO₂R', (CH₂CH₂O)ₘ-(CH₂)ₙ-COR', (CH₂CH₂O)ₘ-(CH₂)ₙ-SONR'R", (CH₂CH₂O)ₘ-(CH₂)ₙ-SO₂NR'R", (CH₂)ₚ-(CH₂CH₂O)ₘ-(CH₂)ₙ-R', (CH₂)ₚ-(CH₂CH₂O)ₘ-(CH₂)ₙ-OH, (CH₂)ₚ-(CH₂CH₂O)ₘ-(CH₂)ₙ-OR_{'}, (CH₂)ₚ-(CH₂CH₂O)ₘ(CH₂)"-NR`R", (CH₂)ₚ-(CH₂CH₂O)ₘ-(CH₂)ₙ-NR'COR", (CH₂)ₚ-(CH₂CH₂O)ₘ-(CH₂)ₙ-NR'SO₂R", (CH₂)ₚ-(CH₂CH₂O)ₘ-(CH₂)ₙ-COOH, (CH₂)ₚ-(CH₂CH₂O)ₘ-(CH₂)ₙ-COOR', (CH₂)ₚ-(CH₂CH₂O)ₘ-(CH₂)ₙ-CONR'R", (CH₂)ₚ-(CH₂CH₂O)ₘ-(CH₂)ₙ-SO₂R', (CH₂)ₚ-(CH₂CH₂O)ₘ-(CH₂)ₙ-COR', (CH₂)ₚ-(CH₂CH₂O)ₘ-(CH₂)ₙ-SONR'R", (CH₂)ₚ-(CH₂CH₂O)ₘ-(CH₂)ₙ-SO₂NR'R", -COR', -SOR' et SO₂R', où R' et R" ont les mêmes définitions que ci-dessus;
m, n et p sont indépendamment 0 à 6;
et ses sels et esters pharmaceutiquement acceptables.

2. Composé selon la revendication 1, de formule 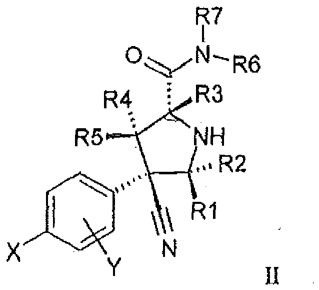 dans laquelle:
X est choisi dans le groupe constitué par H, F, Cl, Br, I, cyano, nitro, éthynyle, cyclopropyle, méthyle, éthyle, isopropyle, vinyle et méthoxy;
Y représente 1 à 4 groupes choisis indépendamment dans le groupe constitué par H, F, Cl, Br, I, CN, OH, nitro, alkyle en C₁-C₆, cycloalkyle, alcoxy en C₁-C₆, alcényle en C₂-C₆, cycloalcényle, alcynyle en C₂-C₆, aryle, hétéroaryle, hétérocycle, COOR', OCOR', CONR'R", NR'COR", NR"SO₂R', SO₂NR'R" et NR'R", où
R' et R" sont choisis indépendamment parmi H ou alkyle en C₁-C₆ substitué ou non substitué, cycloalkyle en C₃-C₁₀ substitué ou non substitué, alcényle en C₂-C₆ substitué ou non substitué, cycloalcényle en C₅-C₁₀ substitué ou non substitué, aryle substitué ou non substitué, hétéroaryle substitué ou non substitué ou hétérocycle substitué ou non substitué; et R' et R" peuvent indépendamment se lier pour former une structure cyclique choisie parmi cycloalkyle substitué ou non substitué, cycloalcényle substitué ou non substitué, hétéroaryle substitué ou non substitué ou hétérocycle substitué ou non substitué;
R₁ est choisi dans le groupe constitué par alkyle en C₁-C₆, alkyle en C₁-C₆ substitué, alcényle en C₂-C₆, alcényle en C₂-C₆ substitué, aryle, aryle substitué, hétéroaryle, hétéroaryle substitué, hétérocycle, hétérocycle substitué, cycloalkyle, cycloalkyle substitué, cycloalcényle et cycloalcényle substitué;
R₂ est un hydrogène ou alkyle en C₁-C₆;
R₃ est H ou alkyle en C₁-C₆;
R₅ est choisi dans le groupe constitué par alkyle en C₁-C₆, alkyle en C₁-C₆ substitué, alcényle en C₂-C₆, alcényle en C₂-C₆ substitué, aryle, aryle substitué, hétéroaryle, hétéroaryle substitué, hétérocycle, hétérocycle substitué, cycloalkyle, cycloalkyle substitué, cycloalcényle et cycloalcényle substitué;
R₄ est un hydrogène;
R₆ et R₇ sont choisis dans le groupe constitué par (CH₂)ₙ-R', (CH₂)ₙ-NR'R", (CH₂)ₙ-NR`COR", (CH₂)ₙ-NR'SO₂R", (CH₂)ₙ-COOH, (CH2)ₙCOOR', (CH₂)ₙ-CONR'R", (CH₂)ₙOR', (CH₂)ₙ-SR', (CH₂)ₙ-SOR', (CH₂)ₙ-SO₂R', (CH₂)ₙ-COR¹, (CH₂)ₙ-SO₃H, (CH₂)ₙ-SONR'R", (CH₂)ₙ-SO₂NR'R", (CH₂CH₂O)ₘ-(CH₂)ₙ-R', (CH₂CH₂O)ₘ-(CH₂)ₙ-OH, (CH₂CH₂O)ₘ-(CH₂)ₙ-OR', (CH₂CH₂O)ₘ-(CH₂)ₙ-NR'R", (CH₂CH₂O)ₘ, (CH₂)ₙ NR'COR", (CH₂CH₂O)ₘ-(CH₂)ₙ-NR'SO₂R", (CH₂CH₂O)ₘ-(CH₂)ₙ COOH, (CH₂CH₂O)ₘ-(CH₂)ₘ-COOR', (CH₂CH₂O)ₘ,-(CH₂)ₙ-CONR'R", (CH₂CH₂O)ₘ-(CH₂)ₙ-SO₂R', (CH₂CH₂O)ₘ-(CH₂)ₙ-COR', (CH₂CH₂O)ₘ-(CH₂)ₙ-SONR'R", (CH₂CH₂O)ₘ-(CH₂)ₙ-SO₂NR'R", (CH₂)ₚ-(CH₂CH₂O)ₘ-(CH₂)ₙ-R`, (CH₂)ₚ-(CH₂CH₂O)ₘ-(CH₂)ₙ-OH, (CH₂)ₚ-(CH₂CH₂O)ₘ-(CH₂)ₙ-OR', (CH₂)ₚ-(CH₂CH₂O)ₘ-(CH₂)ₙ-NR'R", (CH₂)ₚ-(CH₂CH₂O)ₘ-(CH₂)ₙ-NR'COR", (CH₂)ₚ-(CH₂CH₂O)ₘ-(CH₂)ₙ-NR'SO₂R", (CH₂)ₚ-(CH₂CH₂O)ₘ-(CH₂)ₙ-COOH, (CH₂)ₚ-(CH₂CH₂O)ₙ-(CH₂)ₙ-COOR', (CH₂)ₚ-(CH₂CH₂O)ₘ-(CH₂)ₙ-CONR'R", (CH₂)ₚ-(CH₂CH₂O)ₘ-(CH₂)ₙ-SO₂R', (CH₂)ₚ-(CH₂CH₂O)ₘ-(CH₂)ₙ-COR`, (CH₂)ₚ-(CH₂CH₂O)ₘ-(CH₂)ₙ-SONR'R", (CH₂)ₚ-(CH₂CH₂O)ₘ-(CH₂)ₙ-SO₂NR'R" -COR', -SOR' et SO₂R', où R' et R" ont les mêmes définitions que ci-dessus;
m, n et p sont indépendamment 0 à 6;
et ses sels et esters pharmaceutiquement acceptables.

3. Composé selon la revendication 2, dans lequel
X est F, Cl ou Br;
Y représente 1 ou 2 groupes choisis indépendamment dans le groupe constitué par H, F, Cl, Br, I, CN, OH, nitro, alkyle en C₁-C₆, cycloalkyle, alcoxy en C₁-C₆, alcényle en C₂-C₆, cycloalcényle en C₅-C₁₀ et alcynyle en C₂-C₆;
R₁ est choisi dans le groupe constitué par alkyle en C₁-C₆, alkyle en C₁-C₆ substitué, alcényle en C₂-C₆, alcényle en C₂-C₆ substitué, aryle, aryle substitué, hétéroaryle, hétéroaryle substitué, hétérocycle, hétérocycle substitué, cycloalkyle, cycloalkyle substitué, cycloalcényle et cycloalcényle substitué;
R₂ est un hydrogène;
R₃ est H;
R₅ est choisi dans le groupe constitué par aryle, aryle substitué, hétéroaryle et hétéroaryle substitué;
R₄ est un hydrogène;
R₆ et R₇ sont choisis dans le groupe constitué par (CH₂)ₙ-R', (CH₂)ₙ-NR'R", (CH₂)ₙ-NR'COR", (CH₂)ₙ-NR'SO₂R", (CH₂)ₙ-COOH, (CH₂)ₙCOOR', (CH₂)ₙ-CONR'R", (CH₂)ₙOR', (CH₂)ₙ-SR', (CH₂)ₙ-SOR', (CH₂)"-SO₂R', (CH₂)ₙ-COR', (CH₂)ₙ-SO₃H, (CH₂)ₙ-SONR'R", (CH₂)ₙ-SO₂NR'R", (CH₂CH₂O)ₘ-(CH₂)ₙ-R', (CH₂CH₂O)ₘ-(CH₂)ₙ-OH, (CH₂CH₂O)ₘ-(CH₂)ₙ-OR', (CH₂CH₂O)ₘ-(CH₂)ₙ-NR'R", (CH₂CH₂O)ₘ-(CH₂)ₙ-NR'COR", (CH₂CH₂O)ₘ-(CH₂)ₙ-NR'SO₂R", (CH₂CH₂O)ₘ-(CH₂)ₙ-COOH, (CH₂CH₂O)ₘ-(CH₂)ₙ-COOR', (CH₂CH₂O)ₘ-(CH₂)ₙ-CONR'R", (CH₂CH₂O)ₘ-(CH₂)ₙ-SO₂R', (CH₂CH₂O)ₘ-(CH₂)ₙ-COR', (CH₂CH₂O)ₘ-(CH₂)ₙ-SONR'R", (CH₂CH₂O)ₘ-(CH₂)ₙ-SH₂)ₙ-SO₂NR'R", (CH₂)ₚ-(CH₂CH₂O)ₘ-(CH₂)ₙ-R', (CH₂)ₚ-(CH₂CH₂O)ₘ-(CH₂)ₙ-OH, (CH₂)ₚ-(CH₂CH₂O)ₘ-(CH₂)ₙ-OR', (CH₂)ₚ-(CH₂CH₂O)ₘ-(CH₂)ₙ-NR'R", (CH₂)ₚ-(CH₂CH₂O)ₘ-(CH₂)ₙ-NR'COR", (CH₂)ₚ-(CH₂CH₂O)ₘ-(CH₂)ₙ-NR'SO₂R", (CH₂)ₚ-(CH₂CH₂O)ₘ-(CH₂)ₙ-COOH, (CH₂)ₚ-(CH₂CH₂O)ₘ-(CH₂)ₙ-COOR', (CH₂)ₚ-(CH₂CH₂O)ₘ-(CH₂)ₙ-CONR'R", (CH₂)ₚ-(CH₂CH₂O)ₙ-(CH₂)ₙ-SO₂R¹, (CH₂)ₚ-(CH₂CH₂O)ₘ-(CH₂)ₙ-COR', (CH₂)ₚ-(CH₂CH₂O)ₘ-(CH₂)ₙ-SONR'R", (CH₂)ₚ-(CH₂CH₂O)ₘ-(CH₂)ₙ-SO₂NR'R", -COR', -SOR' et SO₂R', où
R' et R" sont choisis indépendamment parmi H, alkyle en C₁-C₆ substitué ou non substitué, cycloalkyle en C₃-C₁₀ substitué ou non substitué, alcényle en C₂-C₆ substitué ou non substitué, cycloalcényle en C₅-C₁₀ substitué ou non substitué, aryle substitué ou non substitué, hétéroaryle substitué ou non substitué ou hétérocycle substitué ou non substitué; et R' et R" peuvent aussi indépendamment se lier pour former une structure cyclique choisie parmi cycloalkyle substitué ou non substitué, cycloalcényle substitué ou non substitué, hétéroaryle substitué ou non substitué ou hétérocycle substitué ou non substitué;
m, n et p sont indépendamment 0 à 6;
et ses sels et esters pharmaceutiquement acceptables.

4. Composé selon la revendication 2, dans lequel
X est F, Cl ou Br;
Y est un groupe mono-substituant choisi parmi H et F; et
R₁ est choisi dans le groupe constitué par alkyle en C₁-C₆, alkyle en C₁-C₆ substitué, alcényle en C₂-C₆, alcényle en C₂-C₆ substitué, hétérocycle, hétérocycle substitué, cycloalkyle, cycloalkyle substitué, cycloalcényle et cycloalcényle substitué.

5. Composé selon la revendication 2, dans lequel
R₁ est un alkyle en C₁-C₆ substitué de formule 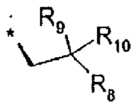 où
R₈, R₉ sont tous les deux un méthyle ou sont liés pour former un groupe cyclopropyle, cyclobutyle, cyclopentyle ou cyclohexyle;
R₁₀ est (CH₂)ₘ-R₁₁;
m est 0, 1 ou 2;
R₁₁ est choisi parmi hydrogène, hydroxyle, alkyle en C₁-C₆, alcoxy en C₁-C₆, aryle, aryle substitué, hétéroaryle, hétéroaryle substitué, hétérocycle ou hétérocycle substitué;
R₂ est H;
R₃ est H;
R₅ est un phényle substitué choisi parmi 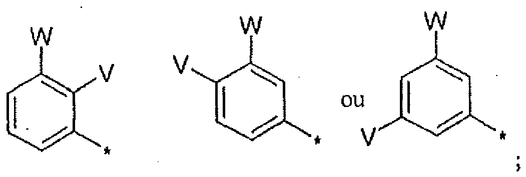 W est F, Cl ou Br;
V est H ou F;
R₄ est un hydrogène;
l'un des R₆ et R₇ est un hydrogène et l'autre est (CH₂)ₙ-R';
n est 0 ou 1; et
R' est choisi parmi aryle, aryle substitué, hétéroaryle, hétéroaryle substitué, hétérocycle ou hétérocycle substitué.

6. Composé selon la revendication 1, choisi dans le groupe constitué par:
le (2-morpholin-4-yléthyl)amide de l'acide rac-(2R,3R,4R,5S)-3-(3-chlorophényl)-4-(4-chlorophényl)-4-cyano-5-(2,2-diméthylpropyl)pyrrolidine-2-carboxylique,
le (2-morpholin-4-yléthyl)amide de l'acide (2R,3R,4R,5S)-3-(3-chlorophényl)-4-(4-chlorophényl)-4-cyano-5-(2,2-diméthylpropyl)pyrrolidine-2-carboxylique,
le diméthylamide de l'acide rac-(2R,3R,4R,5S)-3-(3-chlorophényl)-4-(4-chlorophényl)-4-cyano-5-(2,2-diméthylpropyl)pyrrolidine-2-carboxylique,
le ((S)-3,4-dihydroxybutyl)amide de l'acide rac-(2R,3R,4R,5S)-3-(3-chlorophényl)-4-(4-chlorophényl)-4-cyano-5-(2,2-diméthylpropyl)pyrrolidine-2-carboxylique,
le ((S)-3,4-dihydroxybutyl)amide de l'acide (2R,3R,4R,5S)-3-(3-chlorophényl)-4-(4-chlorophényl)-4-cyano-5-(2,2-diméthylpropyl)pyrrolidine-2-carboxylique,
le rac-(2S,3R,4R,5R)-4-(3-chlorophényl)-3-(4-chlorophényl)-2-(2,2-diméthylpropyl)-5-[4-(2-morpholin-4-yl-2-oxoéthyl)pipérazine-1-carbonyl]pyrrolidine-3-carbonitrile,
le rac-(2S,3R,4R,5R)-4-(3-chlorophényl)-3-(4-chlorophényl)-2-(2,2-diméthylpropyl)-5-[4-(2-oxo-2-pyrrolidin-1-yléthyl)pipérazine-1-carbonyl]pyrrolidine-3-carbonitrile,
le rac-(2S,3R,4R,5R)-4-(3-chlorophényl)-3-(4-chlorophényl)-2-(2,2-diméthylpropyl)-5-[4-(2-hydroxyéthyl)pipérazine-1-carbonyl]pyrrolidine-3-carbonitrile,
le (4-hydroxybutyl)amide de l'acide rac-(2R,3R,4R,5S)-3-(3-chlorophényl)-4-(4-chlorophényl)-4-cyano-5-(2,2-diméthylpropyl)pyrrolidine-2-carboxylique,
le (2-pyrrolidin-1-yléthyl)amide de l'acide rac-(2R,3R,4R,5S)-3-(3-chlorophényl)-4-(4-chlorophényl)-4-cyano-5-(2,2-diméthylpropyl)pyrrolidine-2-carboxylique,
le (2-pipérazin-1-yléthyl)amide de l'acide rac-(2R,3R,4R,5S)-3-(3-chlorophényl)-4-(4-chlorophényl)-4-cyano-5-(2,2-diméthylpropyl)pyrolidine-2-carboxylique,
le (S)-2-([(2R,3R,4R,5S)-3-(3-chlorophényl)-4-(4-chlorophényl)-4-cyano-5-(2,2-diméthylpropyl)pyrrolidine-2-carbonylamino}-3-méthylbutyrate de méthyle,
l'acide (S)-2-{[(2R,3R,4R,5S)-3-(3-chlorophényl}-4-(4-chlorophényl)-4-cyano-5-(2,2-diméthylpropyl)pyrrolidine-2-carbonyl]amino)-3-méthylbutyrique,
le (1-hydroxyméthylcyclopropylméthyl)amide de l'acide rac-(2R,3R,4R,5S)-3-(3-chlorophényl)-4-(4-chlorophényl)-4-cyano-5-(2,2-diméthylpropyl)pyrrolidine-2-carboxylique,
le (1-hydroxyméthylcyclobutylméthyl)amide de l'acide rac-(2R,3R,4R,5S)-3-(3-chlorophényl)-4-(4-chlorophényl)-4-cyano-5-(2,2-diméthylpropyl)pyrrolidine-2-carboxylique,
le (3,3-diméthylbutyl)amide de l'acide rac-(2R,3R,4R,5S)-3-(3-chlorophényl)-4-(4-chlorophényl)-4-cyano-5-(2,2-diméthylpropyl)pyrrolidine-2-carboxylique,
le (2,2-diméthylpropyl)amide de l'acide rac-(2R,3R,4R,5S)-3-(3-chlorophényl)-4-(4-chlorophényl)-4-cyano-5-(2,2-diméthylpropyl)pyrrolidine-2-carboxylique,
le (2S,3R,4R,5R)-4-(3-chlorophényl)-3-(4-chlorophényl)-2-(2,2-diméthylpropyl)-5-((S)-2-hydroxyméthylpyrrolidine-1-carbonyl)pyrrolidine-3-carbonitrile,
le 2-(3,4-diméthoxyphényl)éthylamide de l'acide rac-(2R,3R,4R,5S)-3-(3-chlorophényl)-4-(4-chlorophényl)-4-cyano-5-(2,2-diméthylpropyl)pyrrolidine-2-carboxylique,
le ((R)-1-hydroxyméthyl-3-méthylbutyl)amide de l'acide (2S,3S,4S,5R)-3-(3-chlorophényl)-4-(4-chlorophényl)-4-cyano-5-(2,2-diméthylpropyl)pyrrolidine-2-carboxylique,
le (3-hydroxypropyl)amide de l'acide rac-(2R,3R,4R,5S)-3-(3-chlorophényl)-4-(4-chlorophényl)-4-cyano-5-(2,2-diméthylpropyl)pyrrolidine-2-carboxylique,
le [2-(cis-2,6-diméthylmorpholin-4-yl)éthyl]amide de l'acide rac-(2R,3S,4R,5S)-3-(3-chloro-2-fluorophényl)-4-(4-chlorophén)-4-4-cyano-5-(2,2-diméthylpropyl)-pyrrolidine-2-carboxylique,
le (2-cyclopropyléthyl)amide de l'acide rac-(2R,3R,4R,5S)-3-(3-chlorophényl)-4-(4-chlorophényl)-4-cyano-5-(2,2-diméthylpropyl)pyrrolidine-2-carboxylique,
le rac-(3-{[(2R,3R,4R,5S)-3-(3-chlorophényl)-4-(4-chlorophényl)-4-cyano-5-(2,2-diméthylpropyl)pyrrolidine-2-carbonyl]amino}propyl)carbamate de tert-butyle,
le (3-aminopropyl)amide de l'acide rac-(2R,3R,4R,5S)-3-(3-chlorophényl)-4-(4-chlorophényl)-4-cyano-5-(2,2-dimethylpropyl)pyrrolidine-2-carboxylique,
le [3-(1-acétylpipéridin-4-ylamino)propyl]amide de l'acide rac-(2R,3R,4R,5S)-3-(3-chlorophényl)-4-(4-chlorophényl)-4-cyano-5-(2,2-diméthylpropyl)pyrrolidine-2-carboxylique,
le (3-hydroxypropyl)amide de l'acide rac-(2R,3R,4R,5S)-3-(3-chlorophényl)-4-(4-chlorophényl)-4-cyano-5-isobutylpyrrolidine-2-carboxylique,
le (3-hydroxypropyl)amide de l'acide rac-(2R,3R,4R,5R)-5-(3-chlorophényl)-4-(4-chlorophényl)-4-cyano-3-(2,2-diméthylpropyl)pyrrolidine-2-carboxylique,
le (3-hydroxypropyl)amide de l'acide rac-(2R,3R,4R,5S)-3-(3-chlorophényl)-4-(4-chlorophényl)-4-cyano-5-(2,2-diméthylpropyl)-2-méthylpyrrolidine-2-carboxylique,
le ((S)-3,4-dihydroxybutyl)amide de l'acide rac-(2R,3S,4R,5S)-3-(3-chloro-2-fluorophényl)-4-(4-chlorophényl)-4-cyano-5-cyclopentylméthylpyrrolidine-2-carboxylique,
le (2-hydroxy-1,1-diméthyléthyl)amide de l'acide rac-(2R,3S,4R,5S)-3-(3-chloro-2-fluorophényl)-4-(4-chlorophényl)-4-cyano-5-(2,2-diméthylpropyl)pyrrolidine-2-carboxylique,
le (3-hydroxy-2,2-diméthylpropyl)amide de l'acide rac-(2R,3S,4R,5S)-3-(3-chloro-2-fluorophényl)-4-(4-chlorophényl)-4-cyano-5-(2,2-diméthylpropyl)pyrrolidine-2-carboxylique,
le 2-(2-hydroxyéthoxy)éthyl]amide de l'acide rac-(2R,3S,4R,5S)-3-(3-chloro-2-fluorophényl)-4-(4-chlorophényl)-4-cyano-5-(2,2-diméthylpropyl)pyrrolidine-2-carboxylique,
le (2-acétylaminoéthyl)amide de l'acide rac-(2R,3S,4R,5S)-3-(3-chloro-2-fluorophényl)-4-(4-chlorophényl)-4-cyano-5-(2,2-diméthylpropyl)pyrrolidine-2-carboxylique,
le (3-imidazol-1-ylpropyl)amide de l'acide rac-(2R,3S,4R,5S)-3-(3-chloro-2-fluorophényl)-4-(4-chlorophényl)-4-cyano-5-(2,2-diméthylpropyl)pyrrolidine-2-carboxylique,
le ((R)-4-hydroxy-3-méthylbutyl)amide de l'acide rac-(2R,3S,4R,5S)-3-(3-chloro-2-fluorophényl)-4-(4-chlorophényl)-4-cyano-5-(2,2-diméthylpropyl)pyrrolidine-2-carboxylique,
le cyclopropylméthoxyamide de l'acide rac-(2R,3S,4R,5S)-3-(3-chloro-2-fluorophényl)-4-(4-chlorophényl)-4-cyano-5-(2,2-diméthylpropyl)pyrrolidine-2-carboxylique,
le [2-((S)-2,2-diméthyl-[1,3]dioxolan-4-yl)éthyl]amide de l'acide rac-(2R,3R,4R,5S)-3,5-bis-(3-chlorophényl)-4-(4-chlorophényl)-4-cyanopyrrolidine-2-carboxylique,
le ((S)-3,4-dihydroxybutyl)amide de l'acide rac-(2R,3R,4R,5S)-3,5-bis-(3-chlorophényl)-4-(4-chlorophényl)-4-cyanopyrrolidine-2-carboxylique,
le ((S)-3,4-dihydroxybutyl)amide de l'acide rac-(2R,3R,4R,5S)-3-(3-chlorophényl)-4-(4-chlorophényl)-4-cyano-5-(1-éthylpropyl)pyrrolidine-2-carboxylique,
le ((S)-3,4-dihydroxybutyl)amide de l'acide rac-(2R,3S,4R,5S)-3-(3-chloro-2-fluorophényl)-4-(4-chlorophényl)-4-cyano-5-(2,2-diméthylpropyl)pyrrolidine-2-carboxylique,
le ((S)-3,4-dihydroxybutyl)amide de l'acide (2R,3S,4R,5S)-3-(3-chloro-2-fluorophényl)-4-(4-chlorophényl)-4-cyano-5-(2,2diméthylpropyl)pyrrolidine-2-carboxylique,
le ((S)-3,4-dihydroxybutyl)amide de l'acide rac-(2R,3R,4R,5S)-3-(3-chlorophényl)-4-(4-chlorophényl)-4-cyano-5-isobutylpyrrolidine-2-carboxylique,
le ((S)-3,4-dihydroxybutyl)amide de l'acide rac-(2R,3R,4R,5R)-3-(3-chlorophényl)-4-(4-chlorophényl)-4-cyano-5-isobutylpyrrolidine-2-carboxylique,
le ((S)-3,4-dihydroxybutyl)amide de l'acide rac-(2R,3R,4R,5R)-3-(3-chlorophényl)-4-(4-chlorophényl)-4-cyano-5-(1-éthylpropyl)pyrrolidine-2-carboxylique,
le ((S)-3,4-dihydroxybutyl)amide de l'acide rac-(2R,3R,4R)-3-(3-chlorophényl)-4-(4-chlorophényl)-4-cyano-5,5-diéthylpyrrolidine-2-carboxylique,
le ((S)-3,4-dihydroxybutyl)amide de l'acide rac-(2R,3R,4R,5S)-3-(3-chlorophényl)-4-(4-chlorophényl)-4-cyano-5-isopropylpyrrolidine-2-carboxylique,
le ((S)-3,4-dihydroxybutyl)amide de l'acide rac-(2R,3R,4R,5S)-3-(3-chlorophényl)-4-(4-chlorophényl)-4-cyano-5-cyclohexylpyrrolidine-2-carboxylique,
le ((S)-3,4-dihydroxybutyl)amide de l'acide rac-(2R,3S,4R,5S)-5-tert-butyl-3-(3-chloro-2-fluorophényl)-4-(4-chlorophényl)-4-cyanopyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5-(2,2-diméthylpropyl)-pyrrolidine-2-carboxylique, et
le ((S)-3,4-dihydroxybutyl)amide de l'acide (2R,3S,4R,5S)-3-(3-chloro-2-fluorophényl)-4-(4-chloro-2-fluorophényl)-4-cyano-5-(2,2-diméthylpropyl)pyrrolidine-2-carboxylique,
le ((S)-3,4-dihydroxybutyl)amide de l'acide rac-(2S,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le ((S)-3,4-dihydroxybutyl)amide de l'acide rac-(2R,3S,4R,5S)-4-(4-bromophényl)-3-(3-chloro-2-fluorophényl)-4-cyano-5-(2,2-diméthylpropyl)pyrrolidine-2-carboxylique,
le ((S)-3,4-dihydroxybutyl)amide de l'acide rac-(2R,3S,4R,5S)-3-(3-chloro-2-fluorophényl)-4-cyano-5-(2,2-diméthylpropyl)-4-(4-fluorophényl)pyrrolidine-2-carboxylique,
le ((S)-3,4-dihydroxybutyl)amide de l'acide rac-(2R,3S,4R,5S)-3-(3-chloro-2-fluorophényl)-4-cyano-4-(2,4-dichlorophényl)diméthylpropyl)pyrrolidine-2-carboxylique,
le ((S)-3,4-dihydroxybutyl)amide de l'acide (2R,3S,4R,5S)-3-(3-chloro-2-fluorophényl)-4-cyano-4-(2,4-dichlorophényl)-5-(2,2-diméthylpropyl)pyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-méthylphényl)-4-cyano-5-(2,2-diméthylpropyl)-pyrrolidine-2-carboxylique,
le ((S)-3,4-dihydroxybutyl)amide de l'acide rac-(2R,3S,4R,5S)-3-(3-chloro-2-fluorophényl)-4-(4-chlorophényl)-4-cyano-5-cyclohexylméthylpyrrolidine-2-carboxylique,
le ((S)-3,4-dihydroxybutyl)amide de l'acide rac-(2S,3 S,4R,5S)-3-(3-chloro-2-fluorophényl)-4-(4-chlorophényl)-4-cyano-5-cyclohexylmethylpyrrolidine-2-carboxylique,
le ((S)-3,4-dihydroxybutyl)amide de l'acide rac-(2R,3S,4R,5S)-4-(4-chloro-2-fluorophényl)-4-cyano-3-(2,3-difluorophényl)-5-(2,2-diméthylpropyl)pyrrolidine-2-carboxylique,
le ((S)-3,4-dihydroxybutyl)amide de l'acide (2R,3S,4R,5S)-4-(4-chloro-2-fluorophényl)-4-cyano-3-(2,3-difluorophényl)-5-(2,2-diméthylpropyl)pyrrolidine-2-carboxylique,
le ((S)-3,4-dihydroxybutyl)amide de l'acide rac-(2R,3S,4R,5S)-3-(3-bromo-2-fluorophényl)-4-(4-chloro-2-fluorophényl)-4-cyano-5-(2,2-diméthylpropyl)-pyrrolidine-2-carboxylique, et
le ((S)-3,4-dihydroxybutyl)amide de l'acide (2R,3S,4R,5S)-3-(3-bromo-2-fluorophényl)-4-(4-chloro-2-fluorophényl)-4-cyano-5-(2,2-diméthylpropyl)-pyrrolidine-2-carboxylique,
le ((S)-3,4-dihydroxybutyl)amide de l'acide rac-(2R,3R,4R,5S)-4-(4-chloro-2-fluorophényl)-3-(3-chlorophényl)-4-cyano-5-(2,2-diméthylpropyl)pyrrolidine-2-carboxylique,
le ((S)-3,4-dihydroxybutyl)amide de l'acide (2R,3R,4R,5S)-4-(4-chloro-2-fluorophényl)-3-(3-chlorophényl)-4-cyano-5-(2,2-diméthylpropyl)pyrrolidine-2-carboxylique,
le ((S)-3,4-dihydroxybutyl)amide de l'acide rac-(2R,3R,4R,5S)-4-(4-chloro-2-fluorophényl)-4-cyano-5-(2,2-diméthylpropyl)-3-(3-fluorophényl)pyrrolidine-2-carboxylique,
le ((S)-3,4-dihydroxybutyl)amide de l'acide rac-(2R,3R,4R,5S)-3-(3-bromophényl)-4-(4-chloro-2-fluorophényl)-4-cyano-5-(2,2-diméthylpropyl)pyrrolidine-2-carboxylique,
le ((S)-3,4-dihydroxybutyl)amide de l'acide rac-(2S,3R,4R,5S)-3-(3-bromophényl)-4-(4-chloro-2-fluorophényl)-4-cyano-5-(2,2-diméthylpropyl)pyrrolidine-2-carboxylique,
le ((S)-3,4-dihydroxybutyl)amide de l'acide rac-(2R,3R,4R,5S)-4-(4-chloro-2-fluorophényl)-4-cyano-3-(3,4-dichlorophényl)-5-(2,2-diméthylpropyl)pyrrolidine-2-carboxylique,
le ((S)-3,4-dihydroxybutyl)amide de l'acide rac-(2R,3R,4R,5S)-3-(3-chloro-4-fluorophényl)-4-(4-chloro-2-fluorophényl)-4-cyano-5-(2,2-diméthylpropyl)-pyrrolidine-2-carboxylique,
le ((S)-3,4-dihydroxybutyl)amide de l'acide (2R,3R,4R,5S)-3-(3-chloro-4-fluorophényl)-4-(4-chloro-2-fluorophényl)-4-cyano-5-(2,2-diméthylpropyl)-pyrrolidine-1-carboxylique,
le ((S)-3,4-dihydroxybutyl)amide de l'acide rac-(2R,3R,4R,5S)-3-(4-bromo-thiophén-2-yl)-4-(4-chloro-2-fluorophényl)-4-cyano-5-(2,2-diméthylpropyl)pyrrolidine-2-carboxylique,
le ((R)-3,4-dihydroxybutyl)amide de l'acide rac-(2R,3R,4R,5S)-3-(3-chloro-4-fluorophényl)-4-(4-chloro-2-fluorophényl)-4-cyano-5-(2,2-diméthylpropyl)-pyrrolidine-2-carboxylique,
le ((R)-3,4-dihydroxybutyl)amide de l'acide (2R,3S,4R,5S)-3-(3-chloro-2-fluorophényl)-4-(4-chloro-2-fluorophenyl)-4-cyano-5-(2,2,3-triméthylbutyl)-pyrrolidine-2-carboxylique,
le ((R)-3,4-dihydroxybutyl)amide de l'acide (2R,3R,4R,5S)-3-(3-chloro-4-fluorophényl)-4-(4-chloro-2-fluorophényl)-4-cyano-5-(2,2-diméthylpropyl)-pyrrolidine-2-carboxylique,
le ((R)-3,4-dihydroxybutyl)amide de l'acide rac-(2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le ((R)-3,4-dihydroxybutyl)amide de l'acide (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le ((R)-3,4-dihydroxybutyl)amide de l'acide rac-(2R,3R,4R,5S)-4-(4-chloro-2-fluorophényl)-3-(3-chlorophényl)-4-cyano-5-(2,2-diméthylpropyl)pyrrolidine-2-carboxylique,
le ((R)-3,4-dihydroxybutyl)amide de l'acide rac-(2R,3S,4R,5S)-3-(3-chloro-2-fluorophényl)-4-(4-chlorophényl)-4-cyano-5-(2,2-diméthylpropyl)pyrrolidine-2-carboxylique,
le ((R)-3,4-dihydroxybutyl)amide de l'acide rac-(2R,3R,4R,5S)-3-(3-chlorophényl)-4-(4-chlorophényl)-4-cyano-5-(2,2-diméthylpropylpyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5-(2,2,3-triméthylbutyl)-pyrrolidine-2-carboxylique,
le ((S)-3,4-dihydroxybutyl)amide de l'acide (2R,3S,4R,5S)-3-(3-chloro-2-fluorophényl)-4-(4-chloro-2-fluorophényl)-4-cyano-5-(2,2,3-triméthylbutyl)-pyrrolidine-2-carboxylique,
le ((R)-3,4-dihydroxybutyl)amide de l'acide rac-(2R,3S,4R,5S)-3-(3-chloro-2-fluorophényl)-4-(4-chloro-2-fluorophényl)-4-cyano-5-(2,2,3-triméthylbutyl)-pyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5-(2,2-diméthylbut-3-ényl)pyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5-(2,2-diméthylbutyl)pyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5-(2-méthyl-2-phényl)propyl)pyrrolidine-2-carboxylique,
le ((S)-3,4-dihydroxybutyl)amide de l'acide rac-(2R,3S,4R,5S)-4-(4-chloro-2-fluorophényl)-3-(5-chloro-2-méthoxyphényl)-4-cyano-5-(2,2-diméthylpropyl)-pyrrolidine-2-carboxylique,
le (quinoléin-3-ylméthyl)amide de l'acide rac(2R,3R,4R,5S)-3-(3-chlorophényl)-4-(4-chlorophényl)-4-cyano-5-(2,2-diméthylpropyl)pyrrolidine-2-carboxylique,
le 4-hydroxybenzylamide de l'acide rac-(2R,3R,4R,5S)-3-(3-chlorophényl)-4-(4-chlorophényl)-4-cyano-5-(2,2-diméthyl)propyl)pyrrolidine-2-carboxylique,
le (2-éthylbutyl)amide de l'acide rac-(2R,3R,4R,5S)-3-(3-chlorophényl)-4-(4-chlorophényl)-4-cyano-5-(2,2-diméthylpropyl)pyrrolidine-2-carboxylique,
le ((S)-3,4-dihydroxybutyl)amide de l'acide rac-(2R,3R,4R,5S)-3-(3-chloro-5-fluorophényl)-4-(4-chloro-2-fluorophényl)-4-cyano-5-(2,2-diméthylpropyl)-pyrrolidine-2-carboxylique,
le ((S)-3,4-dihydroxybutyl)amide de l'acide rac-(2R,3S,4R,5S)-4-(4-chloro-2-fluorophényl)-3-[5-chloro-2-(2-hydroxyéthoxy)phényl]-4-cyano-5-(2,2-diméthylpropyl)pyrrolidine-2-carboxylique,
le rac-4-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}pipéridine-1-carboxylate de tert-butyle,
le pipérldin-4-ylamide de l'acide rac-(2R,3S,4R,5S)-3-(3-chloro-2-fluorophényl)-4-(4-chloro-2-fluorophényl)-4-cyano-5-(2,2-diméthylpropyl)pyrrolidine-2-carboxylique acide trifluoroacétique,
le (1-méthanesulfonylpipéridin-4-yl)amide de l'acide rac-(2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le (1-méthylcarbonylpipéridin-4-yl)amide de l'acide rac-(2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le (1-benzoylpipéridin-4-yl)amide de l'acide rac-(2R,3S,4R,5S)-3-(3-chloro-2-fluorophényl)-4-(4-chloro-2-fluorophényl)- 4-cyano-5-(2,2-diméthylpropyl); pyrrolidine-2-carboxylique,
l'isopropylamide de l'acide rac-4-{[(2R,35,4R,5S)-3-(3-chloro-2-fluorophényl)-4-(4-chloro-2-fluorophényl)-4-cyano-5-(2,2-diméthylpropyl)pyrrolidine-2-carbonyl]amino]pipéridine-1-carboxylique,
le [1-(2-hydroxy-2-méthylpropyl)-1H-pyrazol-3-yl]amide de l'acide rac-(2R,3S,4R,5S)-3-(3-chloro-2-fluorophényl)-4-(4-chloro-2-fluorophényl)-4-cyano-5-(2,2-diméthylpropyl)pyrrolidine-2-carboxylique,
le [1-(2-hydroxy-2-méthylpropyl)-1H-pyrazol-3-yl]amide de l'acide (2R,3S,4R,5S)-3-(3-chloro-2-fluorophényl)-4-(4-chloro-2-fluorophényl)-4-cyano-5-(2,2-diméthylpropyl)pyrrolidine-2-carboxylique chiral,
le [1-(2-hydroxy-2-méthylpropyl)-1H-pyrazol-3-yl]amide de l'acide (2S,3R,4S,5R)-3-(3-chloro-2-fluorophényl)-4-(4-chloro-2-fluorophényl)-4-cyano-5-(2,2-diméthylpropyl)pyrrolidine-2-carboxylique chiral,
le {1-[2--méthyl-2-((R)-1-oxiranylméthoxy)propyl]-1H-pyrazol-3-yl}amide de l'acide
rac-(2R,3S,4R,5S)-3-(3-chloro-2-fluorophényl)-4-(4-chloro-2-fluorophényl)-4-cyano-5-(2,2-diméthylpropyl)pyrrolidine-2-carboxylique,
le {1-[2-((R)-3-amino-2-hydroxypropoxy)-2-méthylpropyl]-1H-pyrazol-3-yl}amide de l'acide rac-(2R,3S,4R,5S)-3-(3-chloro-2-fluorophényl)-4-(4-chloro-2-fluorophényl)-4-cyano-5-(2,2-diméthylpropyl)pyrrolidine-2-carboxylique,
le (1-(2-[(R)-2-hydroxy-3-(3-hydroxypropylamino)propoxy]-2-méthylpropyl}-1H-pyrazol-3-yl)amide de l'acide rac-(2R,3S,4R,SS)-3-(3-chloro-2-fluorophényl)-4-(4-chloro-2-fluorophényl)-4-cyano-5-(2,2-diméthylpropyl)pyrrolidine-2-carboxylique,
le (1-(2-[(R)-2-hydroxy-3-(2-hydroxy-1-hydroxyméthyléthylamino)propoxy]-2-méthylpropyl}-1H-pyrazol-3-yl)amide de l'acide rac-(2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le {1-[2-méthyl-2-((S)-1-oxiranylméthoxy)propyl]-1H-pyrazol-3-yl}amide de l'acide rac-(2R,3S,4R,5S)-3-(3-chloro-2-fluorophényl)-4-(4-chloro-2-fluorophényl)-4-cyano-5-(2,2-diméthylpropyl)pyrrolidine-2-carboxylique,
le {1-[2-((S)-3-amino-2-hydroxypropoxy)-2-méthylpropyl]-1H-pyrozol-3-yl}amide de l'acide rac-(2R,3S,4R,5S)-3-(3-chloro-2-fluorophenyl)-4-(4-chloro-2-fluorophényl)-4-cyano-5-(2,2-diméthylpropyl)pyrrolidine-2-carboxylique,
le {1-[2-((S)-2,3-dihydroxypropoxy)-2-méthylpropyl]" -1H-pyrazol-3-yl} amide de l'acide rac-(2R,3S,4R,5S)-3-(3-chloro-2-fluorophényl)-4-(4-chloro-2-fluorophényl)-4-cyano-5-(2,2-diméthylpropyl)pyrrolidine-2-carboxylique,
le {1-[2-((S)-3-diméthylamino-2-hydroxypropoxy)-2-méthylpropyl]-1H-pyrazol-3-yl)amide de l'acide rac-(2R,3S,4R,5S)-3-(3-chloro-2-fluorophényl)-4-(4-chloro-2-fluorophényl)-4-cyano-5-(2,2-diméthylpropyl)pyrrolidine-2-carboxylique,
l'acide rac-{(S)-3-[2-(3-{[(2R,3S,4R,5S)-3-(3-chloro-2-fluorophényl)-4-cyano-5-(2,2-diméthylpropyl)pyrrolidine-2-carbonyl]amino)pyrazol-1-yl)-1,1-diméthyl-éthoxy] 2-hydroxypropylamino} acétique,
le {1-[2-((S)-2-hydroxy-3-méthylaminopropoxy)-2-méthylpropyl]-1H-pyrazol-3-yl}amide de l'acide rac-(2R,3S,4R,5S)-3-(3-chloro-2-fluorophény)-4-(4-chloro-2-fluorophényl)-4-cyano-5-(2,2-diméthylpropyl)pyrrolidine-2-carboxylique,
le {1-[2-((R)-3-diméthylamino-2-hydroxypropoxy)-2-méthylpropyl]-1H-pyrazol-3-yl)amide de l'acide rac-(2R,3S,4R,5S)-3-(3-chloro-2-fluorophényl)-4-(4-chloro-2-fluorophényl)-4-cyano-5-(2,2-diméthylpropyl)pyrrolidine-2-carboxylique,
le [1-((R)-2,3-dihydroxypropyl)-1H-pyrazol-3-yl]amide de l'acide rac-(2R,3R,4R,5S)-3-(3-chlorophényl)-4-(4-chlorophényl)-4-cyano-5-(2,2-diméthylpropyl)pyrrolidine-2-carboxylique,
le [1-(2-hydroxyéthyl)-1H-pyrazol-3-yl]amide de l'acide rac-(2R,3R,4R,5S)-3-(3-chlorophényl)-4-(4-chlorophényl)-4-cyano-5-(2,2-diméthylpropyl)pyrrolidine-2-carboxylique,
le ((S)-2,3-dihydroxypropyl)amide de l'acide rac-(2R,3R,4R,5S)-3-(3-chlorophényl)-4-(4-chlorophényl)-4-cyano-5-(2,2-diméthylpropyl)pyrrolidine-2-carboxylique,
le ((S)-2,3-dihydroxypropyl)amide de l'acide rac-(2S,3S,4S,5R)-3-(3-chlorophényl)-4-(4-chlorophényl)-4-cyano-5-(2,2-diméthylpropyl)pyrrolidine-2-carboxylique,
le [1-((R)-2,2-diméthyl-[1,3]dioxolan-4-ylméthyl)-1H-pyrazol-3 -yl]amide de l'acide rac-(2R,3R,4R,5S)-3-(3-chlorophényl)-4-(4-chlorophényl)-4-cyano-5-(2,2-diméthylpropyl)pyrrolidine-2-carboxylique,
le 2-trifluorométhylbenzylamide de l'acide rac-(2R,3R,4R,5S)-3-(3-chlorophényl)-4-(4-chlorophényl)-4-cyano-5-(2,2-diméthylpropyl)pyrrolidine-2-carboxylique,
le 4-(2-oxopyrrolidin-1-yl)-benzylamide de l'acide rac-(2R,3R,4R,5S)-3-(3-chlorophényl)-4-(4-chlorophényl)-4-cyano-5-(2,2-diméthylpropyl)pyrrolidine-2-carboxylique,
le (tétrahydropyran-4-ylméthyl)amide de l'acide rac-(2R,3R,4R,5S)-3-(3-chlorophényl)-4-(4-chlorophényl)-4-cyano-5-(2,2-diméthylpropyl)pyrrolidine-2-carboxylique,
le (3-hydroxy-2-hydroxyméthylpropyl)amide de l'acide rac-(2R,3R,4R,5S)-3-(3-chlorophényl)-4-(4-chlorophényl)-4-cyano-5-(2,2-diméthylpropyl)pyrrolidine-2-carboxylique,
le [2-(2-aminoéthoxy)éthyl]amide de l'acide rac-(2R,3R,4R,5S)-3-(3-chlorophényl)-4-(4-chlorophényl)-4-cyano-5-(2,2-diméthylpropyl)pyrrolidine-2-carboxylique,
le (3-méthanesulfonylpropyl)amide de l'acide rac-(2R,3R,4R,5S)-3-(3-chlorophényl)-4-(4-chlorophényl)-4-cyano-5-(2,2-diméthylpropyl)pyrrolidine-2-carboxylique,
le (2-méthanesulfonyléthyl)amide de l'acide rac-(2R,3R,4R,5S)-3-(3-chlorophényl)-4-(4-chlorophényl)-4-cyano-5-(2,2-diméthylpropyl)pyrrolidine-2-carboxylique,
le rac-4-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino)cyclohexylamino-1-carboxylate de tert-butyle,
le sel d'acide trifluoroacétique de la rac-4-{[{2R,3S,4R,5S)-3-(3-chloro-2-fluorophényl)-4-(4-chloro-2-fluorophényl)-4-cyano-5-(2,2-diméthylpropyl)-pyrrolidine-2-carbonyl]amino}-trans-cyclohexylamine,
le rac-4-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}-trans-cyclohexyl-N-méthanesulfonamide,
le rac-4-{[(2R,3S,4R,5S)-3-(3-chloro-2-fluorophényl)-4-(4-chloro-2-fluorophényl)-4-cyano-5-(2,2-diméthylpropyl)prolidine-2-carbonyl]amino}-trans-cyclohexyl-N-méthanesullfonamide,
la rac-4-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino)-trans-cyclohexyl-(1,1-dioxo)-2-isothiazolidine,
la rac-4-{[(2R,3S,4R,5S)-3-{3-chloro-2-fluorophényl)-4-(4-chloro-2-fluorophényl)-4-cyano-5-(2,2-diméthylpropyl)pyrrolidine-2-carbonyl]amino}-trans-cyclohexylurée,
le N-[1-(2-hydroxy éthyl)pipéridin-4-yl]amide de l'acide rac (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le rac-4-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl] amino} -pipéridine-1-sulfonamide,
l'acide rac 3-{4-[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]pipérazin-1-yl}acétique,
le rac 3-(4-[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]pipérazin-1-yl)-N,N-bis-(2-méthoxyéthyl)acétamide,
le rac 3-(4-[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]pipérazin-1-yl}-N,N-bis-(2-hydroxyéthyl)acétamide,
le rac 3-{4-[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]pipérazin-1-yl}-N-(3-méthoxypropyl)acétamide,
le rac 2-{4-[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]pipérazin-1-yl}acétamide, le rac (2S,3R,4S,5R)-4-(3-chloro-2-fluorophényl)-3-(4-chloro-2-fluorophényl)-2-(2,2-diméthylpropyl)-5-[4-(2-morpholin-4-yl-2-oxoéthyl)pipérazine-1-carbonyl]-pyrrolidine-3-carbonitrile,
le rac 2-{4-[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]pipérazin-1-yl)-N-[2-((S)-2,2-diméthyl-[1,3]dioxolan-4-yl)éthyl]acétamide,
le rac 2-{4-[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]pipérazin-1-yl)-N-((S)-3,4-dihydroxybutyl)acétamide,
le rac {1-[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]pipéridin-4-yl}acétate de méthyle, et le chlorhydrate de l'acide rac {1-[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]pipéridin-4-yl} acétique.

7. Composé selon la revendication 1, choisi dans le groupe constitué par:
le rac 2-{1-[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]pipéridin-4-yl}acétamide,
le rac 2-{1-[(2R,3S,4R,5S)-3-(3-chloro-2-fluorophényl)-4-(4-chloro-2-fluorophényl)-4cyano-5-(2,2-diméthylpropyl)pyrrolidine-2-carbonyl]pipéridin-4-yl}-N,N-bis-(2-hydroxyéthyl)acétamide,
le rac 2-{1-[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]pipéridin-4-yl}-N-(2-hydroxyéthyl)-N-méthylacétamide,
le rac 2-{(1-[(2R,35,4R,5S)-3-(3-chloro-2-fluorophényl)-4-(4-chloro-2-fluorophényl)-4-cyano-5-(2,2-diméthylpropyl)pyrrolidine-2-carbonyl]pipéridin-4-yl}-N-(2-hydroxypropyl)acétamide,
le rac {[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}acétate de tert-butyle,
le sel d'acide trifluoroacétique de l'acide rac{[(2R,3S,4R,5S)-3-(3-chloro-2-fluoro-phényl)-4-(4-chloro-2-fluorophényl)-4-cyano-5)2,2-diméthylpropyl)pyrrolidine-2-carbonyl]amino acétique,
le carbamoylméthylamide de l'acide rac (2R,3S,4R,5S)-3-(3-chloro-2-fluorophényl)-4-(4-chloro-2-fluorophényl)-4-cyano-5-(2,2-diméthylpropyl)pyrrolidine-2-carboxylique,
le sel d'acide trifluoroacétique de l'acide {[(2R,3S,4R,5S)-3-(3-chloro-2-fluorophényl)-4-(4-chloro-2-fluorophényl)-4-cyano-5-(2,2-diméthylpropyl)-pyrrolidine-2-carbonyl]amino acétique,
le rac 3-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}benzoate d'éthyle,
le (3-carbamoylphényl)amide de l'acide rac (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le rac 3-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}benzoate de tert-butyle,
l'acide rac 3-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}-benzoïque,
le (3-hydroxyméthylphényl)amide de l'acide rac (2R,3S,4R,5S)-3-(3-ehloro-2-fluorophényl)-4-(4-chloro-2-fluorophényl)-4-cyano-5-(2,2-diméthylpropyl)-pyrrolidine-2-carboxylique,
le rac (3-{[(2R,35,4R,5S)-3-(3-chloro-2-fluorophényl)-4-(4-chloro-2-fluorophényl)-4-cyano-5-(2,2-diméthylpropyl)pyrrolidine-2-carbonyl]amino}propyl)carbamate de tert-butyle,
le (3-aminopropyl)amide de l'acide rac (2R,3S,4R,5S)-3-(3-chloro-2-fluorophényl)-4-(4-chloro-2-fluorophényl)-4-cyano-5-(2,2-diméthylpropyl)pyrrolidine-2-carboxylique,
le [3-(aminosulfonylamino)propyl)amide de l'acide rac (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le rac 2-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}benzoate de tert-butyle,
le rac 4-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}benzoate de tert-butyle,
le rac 4-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}benzoate d'éthyle,
le (2-carbamoylphényl)amide de l'acide rac (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le (4-carbamoylphényl)amide de l'acide rac (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le rac 2-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}benzoate d'éthyle,
l'acide rac 4-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}-benzoïque,
l'acide rac 2-{[(2R,35,4R,5S)-3-(3-chloro-2-fluorophényl)-4-(4-chloro-2-fluorophényl)-4-cyano-5-(2,2-diméthylpropyl)pyrrolidine-2-carbonyl]amino}-benzoïque,
le (3-cyanophényl)amide de l'acide rac (2R,3S,4R,5S)-3-(3-chloro-2-fluorophényl)-4-(4-chloro-2-fluorophényl)-4-cyano-5-(2,2-diméthylpropyl)pyrrolidine-2-carboxylique,
le (2-hydroxy-2-méthylpropyl)amide de l'acide rac (2R,3S,4R,5S)-3-(3-chloro-2-fluorophényl)-4-(4-chloro-2-fluorophényl)-4-cyano-5-(2,2-dimethylpropyl)-pyrrolidine-2-carboxylique,
le rac 3-{[{2R,3S,4R,5S)-3-{3-chioro-2-fluorophényl)-4-(4-chloro-2-fluorophényl}-4-cyano-5-(2,2-diméthylpropyl)pyrrolidine-2-carbonyl]amino}benzoate de 2-hydroxy-2-méthylpropyle,
le (3-méthanesulfonylaminophényl)amide de l'acide rac (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le (1H-tétrazol-5-ylméthyl)amide de l'acide rac (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le (3-uréidopropyl)amide de l'acide rac (2R,3S,4R,5S)-3-(3-chloro-2-fluorophényl)-4-(4-chloro-2-fluorophényl)-4-cyano-5-(2,2-diméthylpropyl)pyrrolidine-2-carboxylique,
le (3-méthylsulfanylphényl)amide de l'acide rac (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le (3-méthanesulfonylphényl)amide de l'acide rac (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le (3-méthanesulfinylphényl)amide de l'acide rac (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
l'acide 3-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}benzoïque,
le (3-carbamoylphényl)amide de l'acide (2R,3S,4R,5S)-3-(3-chloro-2-fluorophényl)-4-(4-chloro-2-fluorophényl)-4-cyano-5-(2,2-diméthylpropyl)pyrrolidine-2-carboxylique,
le [3-(1H-tétrazol-5-yl)phényl]amide de l'acide rac (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le [4-(1H-tétrazol-5-yl)phényl]amide de l'acide rac (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le (4-aminophényl) amide de l'acide rac (2R,3S,4R,5S)-3-(3-chloro-2-fluorophényl)-4-(4-chloro-2-fluorophényl)-4-cyano-5-(2,2-diméthylpropyl)pyrrolidine-2-carboxylique,
le (4-acétylaminophényl)amide de l'acide rac (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le rac 2-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}thiazole-4-carboxylate d'éthyle,
le (1,3-dioxo-2,3-dihydro-1H-isoindol-5-yl)amide de l'acide rac (2R,3S,4R,5S)-3-(3-chloro-2-fluorophényl)-4-(4-chloro-2-fluorophényl)-4-cyano-5-(2,2-diméthylpropyl)-pyrrolidlne-2-carboxylique,
le (6-oxo-1,6-dihydropyridin-3-yl)amide de l'acide rac (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le (6-oxo-1,6-dihydropyridin-3-yl)amide de l'acide (2R,3S,4R,5S)-3-(3-chloro-2-fluorophényl)-4-(4-chloro-2-fluorophényl)-4-cyano-5-(2,2-diméthylpropyl)-pyirolidine-2-carboxylique,
le (4-méthylsulfanylphényl)amide de l'acide rac (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le (4-méthanesulfonylphényl)amide de l'acide rac (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le 4-{[(2R,3S,4R,5S)-3-(3-chloro-2-fluorophényl)-4-(4-chloro-2-fluorophényl)-4-Cyano-5-(2,2-diméthylpropyl)pyrrolidine-2-carbonyl]amino}benzoate de tert-butyle,
l'acide 4-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}benzoïque,
l'acide 4-{[(2S,3R,4S,5R)-3-(3-chloro-2-fluorophényl)-4-(4-chloro-2-fluorophényl)-4-cyano-5-(2,2-diméthylpropyl)pyrrolidine-2-carbonyl]amino}benzoïque,
le (4-carbamoylphényl)amide de l'acide. (2R,3S,4R,5S)-3-(3-chloro-2-fluorophényl)-4-(4-chloro-2-fluorophényl)-4-cyano-5-(2,2-diméthylpropyl)pyrrolidine-2-carboxylique,
le (4-carbamoylphényl)amide de l'acide (2S,3R,4S,5R)-3-(3-chloro-2-fluorophényl)-4-(4-chloro-2-fluorophényl)-4-cyano-5-(2,2-diméthylpropyl)pyrrolidine-2-carboxylique,
le (4-méthanesulfonylaminophényl)amide de l'acide rac (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le (4-trifluorométhanesulfonylaminophényl)amide de l'acide rac (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le [3-(1-méthyl-1H-tétrazol-5-yl)phényl]amide de l'acide rac (2R,3R,4R,5S)-4-(4-chloro-2-fluorophényl)-3-(3-chloro-2-méthylphényl)-4-cyano-5-(2>2-diméthyl-propyl)pyirolidine-2-carboxylique,
le [3-(2-méthyl-1H-tétrazol-5-yl)phényl]amide de l'acide rac (2R,3R,4R,5S)-4-(4-chloro-2-fluorophényl)-3-(3-chloro-2-méthylphényl)-4-cyano-5-(2,2-diméthyl-propyl)pyrralidine-2-carboxylique,
le (6-oxo-1,6-dihydropyridin-3-yl)amide de l'acide (2S,3R,4S,5R)-3-(3-chloro-2-fluorophényl)-4-(4-ehloro-2-fluorophényl)-4-cyano-5-(2,2-diméthylpropyl)-pyrrolidine-2-carboxylique,
le [4-(1H-tétrazol-5-yl)phényl]amide de l'acide (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le [4-(1H-téirazol-5-yl)phényl]amide de l'acide (2S,3R,4S,5R)-3-(3-chloro-2-fluorophényl)-4-(4-ehloro-2-fluorophényl)-4-cyano-5-(2,2-diméthylpropyl)-pyrrolidine-2-carboxylique,
le [4-(2-méthyl-1H-tétrazol-5-yl)phényl]amide de l'acide rac (2R,3R,4R,5S)-4-(4-chloro-2-fluorophényl)-3-(3-chloro-2-méthylphényl)-4-cyano-5-(2,2-diméthylpropyl)pyrrolidine-2-carboxylique,
le [4-(1-méthyl-1H-tétrazol-5-yl)phényl]amide de l'acide rac (2R,3R,4R,5S)-4-(4-chloro-2-fluorophényl)-3-(3-chloro-2-méthylphényl)-4-cyano-5-(2,2-diméthyl-propyl)pyrrolidine-2-carboxylique,
le rac 5-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}-2-fluorobenzoate d'éthyle,
le [3-(1H-tétrazol-5-yl)phényl]amide de l'acide (2R,3S,4R,5S)-3-(3-chloro-2-fluorophényl)-4-(4-ehloro-2-fluorophényl)-4-cyano-5-(2,2-diméthylpropyl)-pyrrolidine-2-carboxylique,
le [3-(1H-tétrazol-5-yl)phényl]amide de l'acide (2S,3R,4S,5R)-3-(3-chloro-2-fluorophényl)-4-(4-ehloro-2-fluorophényl)-4-cyano-5-(2,2-diméthylpropyl)-pyrrolidine-2-caxboxylique,
le (4-carbamoyl-3-chlorophényl)amide de l'acide (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le [3-chloro-4-(1H-tétrazol-5-yl)phényl]amide de l'acide rac (2R,3S,4R,SS)-3-(3-chloro-2-fluorophényl)-4-(4-chloro-2-fluorophényl)-4-cyano-5-(2,2-diméthylpropyl)pyrrolidine-2-carboxyliclue,
le (4-fluorophényl)amide de l'acide rac (2R,3S,4R,5S)-3-(3-ehloro-2-fluorophényl)-4-(4-chloro-2-fluorophényl)-4-cyano-5-(2,2-diméthylpropyl)pyrrolidine-2-carboxylique,
le (3-fluorophényl)amide de l'acide rac (2R,3S,4R,5S)-3-(3-chloro-2-fluorophényl)-4-(4-chloro-2-fluorophényl)-4-cyano-5-(2,2-diméthylpropyl)pyrrolidine-2-carboxylique,
le (3-chlorophényl)amide de l'acide rac (2R,3S,4R,5S)-3-(3-chloro-2-fluorophényl)-4-(4-ehloro-2-fluorophényl)-4-cyano-5-(2,2-diméthylpropyl)pyrrolidine-2-carboxylique,
le (4-chlorophényl)amide de l'acide rac (2R,3S,4R,5S)-3-(3-chloro-2-fluorophényl)-4-(4-chloro-2-fluorophényl)-4-cyano-5-(2,2-diméthylpropyl)pyrrolidine-2-carboxylique,
le rac 4-{[(2R,35,4R,5S)-3-(3-chloro-2-fluorophényl)-4-(4-chloro-2-fluorophényl)-4-cyano-5-(2,2-diméthylpropyl)pyrrolidine-2-carbonyl]amino}-2-fluorobenzoate de tert-butyle,
le (4-éthylcarbamoyl-3-fluorophényl)amide de l'acide rac (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
l'acide rac 4-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}-2-fluorobenzoïque,
le (6-méthoxypyridin-3-yl)amide de l'acide rac (2R,3S,4R,5S)-3-(3-chloro-2-fluorophényl)-4-(4-chloro-2-fluorophényl)-4-cyano-5-(2,2-dimétliylpropyl)-pyrrolidine-2-carboxylique,
le rac 3-({[(2R,3S,4R,5S)-3-(3-chloro-2-fluorophényl-4-(4-chloro-2-fluorophényl)-4-cyano-5-(2,2-diméthylpropyl)pyrrolidine-2-carbonyl]amino}méthyl)benzoate de méthyle,
le rac 4-({[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}méthyl)benzoate de méthyle,
le (4-chlorophényl)amide de l'acide rac (2R,3S,4R,5S)-3-(3-chloro-2-fluorophényl)-4-(4-chloro-2-fluorophényl)-4-cyano-5-(2,2-diméthylpropyl)pyrrolidine-2-carboxylique,
le (4-chlorophényl)amide de l'acide (2R,3S,4R,5S)-3-(3-chloro-2-fluorophényl)-4-(4-chloro-2-fluorophényl)-4-cyano-5-(2,2-diméthylpropyl)pyrrolidine-2-carboxylique,
le (4-chlorophényl)amide de l'acide (2S,3R,4S,5R)-3-(3-chloro-2-fluorophényl)-4-(4-chloro-2-fluorophényl)-4-cyano-5-(2,2-diméthylpropyl)pyrrolidine-2-carboxylique,
le rac 4-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}-2-méthoxybenzoate de méthyle,
l'acide rac 3-({[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}-méthyl)benzoïque,
l'acide rac 4-({[(2R,3S,4R,5S)-3-(3-chloro-2-fluorophényl)-4-(4-chloro-2-fluorophényl)-4-cyano-5-(2,2-diméthylpropyl)pymolidine-2-carbonyl]amino}-méthyl)benzoïque,
le (4-acétylaminophényl)amide de l'acide (2R,3S,4R,5S)-3-(3-chloro-2-fluorophényl)-4-(4-chloro-2-fluorophényl)-4-cyano-5-(2,2-diméthylpropyl)-pymolidine-2-carboxylique,
le (4-acétylaminophényl)amide de l'acide (2S,3R,4S,5R)-3-(3-chloro-2-fluorophényl)-4-(4-chloro-2-fluorophényl)-4-cyano-5-(2,2-diméthylpropyl)-pyrrolidine-2-carboxylique,
le (4-méthanesulfonylphényl)amide de l'acide (2R,3S,4R,5S)-3-(3-chloro-2-fluorophényl)-4-(4-chloro-2-fluorophényl)-4-cyano-5-(2,2-diméthylpropyl)-pymolidine-2-carboxylique,
le (4-méthanesulfonylphényl)amide de l'acide (2S,3R,4S,5R)-3-(3-chloro-2-fluorophényl)-4-(4-chloro-2-fluoropliényl)-4-cyano-5-(2,2-diméthylpropyl)-pyrrolidine-2-carboxylique,
l'acide rac 4-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}-2-méthoxybenzoïque,
le rac 5-bromo-4-{[(2R,3S,4R,SS)-3-(3-chloro-2-fluorophényl)-4-(4-chloro-2-fluorophényl)-4-cyano-5-(2,2-diméthylpropyl)pyrrolidine-2-carbonyl]amino}-2-méthoxybenzoate de méthyle,
le rac 4-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}-2-méthylbenzoate de méthyle,
le rac 2-chloro-4-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}-benzoate de méthyle,
le rac 4-{[(2R,3S,4R,5S)-3-(3-chloro-2-fluorophényl)-4-(4-chloxo-2-fluorophényl)-4-cyano-5-(2,2-diméthylpopyl)pyrrolidine-2-carbonyl]amino}-2-trifluorométhyl-benzoate de méthyle,
l'acide rac 4-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]aminol-2-méthylbenzoïque,
l'acide rac 2-chioro-4-{[(2R,3S,4R,5S)-3-(3-chloro-2-fluorophënyl)-4-(4-chloro-2-fluorophényl)-4-cyano-5-(2,2-diméthylpropyl)pyrrolidine-2-carbonyl]amino}-benzoïque,
le [4-(2H-[1,2,4]triazol-3-y1)phényl]amide de l'acide rac (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le [4-(5-oxo-2,5-dihydro-1H-[1,2,4]thiazol-3-yl)phényl]amide de l'acide rac (2R,3S,4R,5S)-3-(3-chloro-2-fluorophényl)-4-(4-chloro-2-fluorophényl)-4-cyano-5-(2,2-diméthylpropyl)pymolidine-2-carboxylique,
le ((S)-3,4-dihydroxybutyl)amide de l'acide rac-(2R,3S,4R,5S)-3-(5-chloro-2-fluorophényl)-4-(4-chloro-2-fluorophényl)-4-cyano-5-(2,2-diméthylpropyl)-pyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5-(2-cyclopropyl-2-méthylpropyl)pyrrolidine-2-carboxylique,
le ((S)-3,4-dihydroxybutyl)amide de l'acide (2R,3S,4R,5S)-3-(3-chloro-2-fluorophényl)-4-(4-chloro-2-fluorophényl)-4-cyano-5-(2-cyclopropyl-2-méthylpropyl)pyrrolidine-2-carboxylique,
le ((S)-3,4-dihydroxybutyl)amide de l'acide rac-(2R,3S,4R,5S)-3-(3-chloro-2-fluorophényl)-4-(4-chloro-3-méthylphényl)-4-cyano-5-(2,2-diméthylpropyl)-pyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5-(tétrahydropyran-4-ylméthyl)pyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5-(1-méthylcyclohexylméthyl)-pyrrolidine-2-carboxylique,
le ((S)-3,4-dihydroxybutyl)amide de l'acide (2R,3S,4R,5S)-3-(3-chloro-2-fluorophényl)-4-(4-chloro-2-fluorophényl)-4-cyano-5-(1-méthylcyclohexylméthyl)-pyrrolidine-2-carboxylique,
le ((S)-3,4-dihydroxybutyl)amide de l'acide rac-(2R,3S,4R,SS)-S-(2-benzyloxycarbonyl-2-méthylpropyl)-3-(3-chloro-2-fluorophényl)-4-(4-chloro-2-fluorophényl)-4-cyanopyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5-(2-méthoxycarbonyl-2-méthylpropyl)pyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5-(3-hydroxy-2,2-diméthylpropyl)pyrrolidine-2-carboxylique,
le ((S)-3,4-dihydroxybutyl)amide de l'acide (2R,3S,4R,SS)-3-(3-chloro-2-fluorophényl)-4-(4-chloro-2-fluorophényl)-4-cyano-5-(3-hydroxy-2,2-diméthylpropyl)pyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5-(2,2-diéthylbutyl)pyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5-(2-éthyl-2-méthylbutyl)pyrrolidine-2-carboxylique,
le ((S)-3,4-dihydroxybutyl)amide de l'acide rac-(2R,3R,4R,5S)-4-(4-chloro-2,6-difluorophényl)-3-(3-chlorophényl)-4-cyano-5-(2,2-diméthylpropyl)pyrrolidine-2-carboxylique,
le ((S)-3,4-dihydroxybutyl)amide de l'acide rac-(2R,3R,4R,5S)-4-(4-chloro-2,5-difluorophényl)-3-(3-chlorophényl)-4-cyano-5-(2,2-diméthylpropyl)pyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5-(3-méthoxy-2,2-diméthylpropyl)pyrrolidine-2-carboxylique,
le ((S)-3,4-dihydroxybutyl)amide de l'acide (2R,35,4R,5S)-3-(3-chloro-2-fluorophényl)-4-(4-chloro-2-fluorophényl)-4-cyano-5-(3-méthoxy-2,2-diméthylpropyl)pyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5-(2-éthyl-2-hydroxyméthylbutyl)pyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5-(3-méthyloxétan-3-ylméthyl)pyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5-(3-éthyloxétan-3-ylméthyl)pyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5-(1-hydroxyméthyl-cyclopropylméthyl)pyrrolidine-2-carboxylique,
le ((S)-3,4-dihydroxybutyl)amide de l'acide (2R,3S,4R,5S)-3-(3-chloro-2-fluorophényl)-4-(4-chloro-2-fluorophényl)-4-cyano-5-(1-hydroxyméthyl-cyclopropylméthyl)pyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5-(1-hydroxyméthyl-cyclobutylméthyl)pyrrolidine-2-carboxylique,
le ((S)-3,4-dihydroxybutyl)amide de l'acide (2R,3S,4R,SS)-3-(3-chloro-2-fluorophényl)-4-(4-chloro-2-fluorophényl)-4-cyano-5-(1-hydroxyméthyl-cyclobutylméthyl)pyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5-(4-hydroxy-2,2-diméthylbutyl)pyrrolidine-2-carboxylique,
le ((S)-3,4-dihydroxybutyl)amide de l'acide (2R,3S,4R,5S)-3-(3-chloro-2-fluorophényl)-4-(4-chloro-2-fluorophényl)-4-cyano-5-(4-hydroxy-2,2-diméthylbutyl)pyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5-(1-hydroxyméthylcyclohex-3-énylméthyl)pyrrolidine-2-carboxylique,
le ((S)-3,4-dihydroxybutyl)amide de l'acide rae-(2R,3S,4R,5S)-3-(3-chloro-2-fluorophényl)-4-(4-chloro-2-fluorophényl)-4-cyano-5-(1-hydroxyméthyl-cyclohexylméthyl)pyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5-[4-(2-hydroxyéthoxy)-2,2-diméthylbutyl]pyrrolidine-2-carboxylique,
le ((S)-3,4-dihydroxybutyl)amide de l'acide rac-(2R,3S,4R,5S)-5-(4-azido-2,2-diméthylbutyl)-3-(3-chloro-2-fluorophényl)-4-(4-chloro-2-fluorophényl)-4-cyanopyrrolidine-2-carboxylique,
le ((S)-3,4-dihydroxybutyl)amide de l'acide rac-(2R,3S,4R,5S)-5-(4-amino-2,2-diméthylbutyl)-3-(3-chloro-2-fluorophényl)-4-(4-chloro-2-fluorophényl)-4-cyanopyrrolidine-2-carboxylique,
le ((S)-3,4-dihydroxybutyl)amide de l'acide rac-(2R,3S,4R,5S)-5-(4-acétylamino-2,2-diméthylbutyl)-3-(3-chloro-2-fluorophényl)-4-(4-chloro-2-fluorophényl)-4-cyanopyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5-(4-méthanesulfonylamino-2,2-diméthylbutyl)pyrrolidine-2-carboxylique,
le ((S)-3,4-dihydroxybutyl)amide de l'acide rac-(2R,3S,4R,5S)-5-(4-benzoylamino-2,2-diméthylbutyl)-3-(3-chloro-2-fluorophényl)-4-(4-chloro-2-fluorophényl)-4-cyanopyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5-[2-méthyl-2-(5-méthylfuran-2-yl)propyl]pyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5[3-(4-méthoxyphényl)-2,2-diméthylpropyl]pyrrolidine-2-carboxylique,
le ((S)-3,4-dihydroxybutyl)amide de l'acide rac-(2R,3S,4R,5S)-5-[2-(1-benzyl-1,2,3,6-tétrahydropyridin-4-yl)-2-méthylpropyl]-3-(3-chloro-2-fluorophényl)-4-(4-chloro-2-fluorophényl)-4-cyanopyrrolidine-2-carboxylique,
le ((S)-3,4-dihydroxybutyl)amide de l'acide rac-(2R,3S,4R,5S)-5-[2-(1-benzylpipéridin-4-yl)-2-méthylpropyl]-3-(3-chloro-2-fluorophényl)-4-(4-chloro-2-fluorophényl)-4-cyanopyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5-[2-(3,6-dihydro-2H-pyran-4-yl)-2-méthylpropyl]pyrrolidine-2-carboxylique,
le ((S)-3,4-dihydroxybutyl)amide de l'acide rac-(2R,3S,4R,5S)-3-(3-chloro-2-fluorophényl)-4-(4-chloro-2-fluorophényl)-4-cyano-5-[2-méthyl-2-(tétrahydropyran-4-yl)propyl]pyrrolidine-2-carboxylique,
le ((S)-3,4-dihydroxy-4-méthylpentyl)amide de l'acide rac-(2R,3S,4R,5S)-3-(3-chloro-2-fluorophényl)-4-(4-chloro-2-fluorophényl)-4-cyano-5-(3-hydroxy-2,2-diméthylpropyl)pyrrolidine-2-carboxylique,
le ((S)-3,4-dihydroxy-4-méthylpentyl)amide de l'acide (2R,3S,4R,5S)-3-(3-chloro-2-fluorophényl)-4-(4-chloro-2-fluorophényl)-4-cyano-5-(3-hydroxy-2)-diméthylpropyl)pyrrolidine-2-carboxylique,
le ((S)-3,4-dihydroxy-4-méthylpentyl)amide de l'acide rac-(2R,3S,4R,5S)-3-(3-chloro-2-fluorophényl)-4-(4-chloro-2-fluorophényl)-4-cyano-5-(2,2-diméthylpropyl)pyrrolidine-2-carboxylique,
le ((S)-3,4-dihydroxy-4-méthylpentyl)amide de l'acide (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le ((S)-3,4-dihydroxy-4-méthylpentyl)amide de l'acide rac-(2R,3S,4R,5S)-3-(3-chloro-2-fluorophényl)-4-(4-chloro-2-fluorophényl)-4-cyano-5-(1-méthylcyclohexyl-méthyl)pyrrolidine-2-carboxylique,
le ((S)-3,4-dihydroxy-4-méthylpentyl)amide de l'acide (2R,3S,4R,5S)-3-(3-chloro-2-fluorophényl)-4-(4-chloro-2-fluorophényl)-4-cyano-5-(1-méthylcyclohexylméthyl)-pyrr olidine-2-carboxylique,
le rac-(2S,3R,4S,5R)-4-(3-chloro-2-fluorophényl)-3-(4-chloro-2-fluorophényl)-2-(2,2-diméthylpropyl)-5-(3-hydroxyazétidine-1-carbonyl)pyrrolidine-3-carbonitrile,
le [1-(2-hydroxy-2-méthylpropyl)-1H-pyrazol-3-yl]amide de l'acide rac-(2R,3S,4R,5S)-3-(3-chloro-2-fluorophényl)-4-(4-chloro-2-fluorophényl)-4-cyano-5-(3-hydroxy-2,2-diméthylpropyl)pyrrolidine-2-carboxylique,
le [1-(2-hydroxy-2-méthylpropyl)-1H-pyrazol-3-yl]amide de l'acide (2R,3S,4R,5S)-3-(3-chloro-2-fluorophényl)-4-(4-chloro-2-fluorophényl)-4-cyano-5-(3-hydroxy-2,2-diméthylpropyl)pyrrolidine-2-carboxylique,
le [1-(2-hydroxy-2-méthylpropyl)-1H-pyrazol-3-yl]amide de l'acide rac-(2R,3S,4R,5S)-3-(3-chloro-2-fluorophényl)-4-(4-chloro-2-fluorophényl)-4-cyano-5-(4-hydroxy-2,2-diméthylbutyl)pyrrolidine-2-carboxylique,
le [1-(2-hydroxy-2-méthylpropyl)-1H-pyrazol-3-yl]amide de l'acide (2R,3S,4R,5S)-3-(3-chloro-2-fluorophényl)-4-(4-chloro-2-fluorophényl)-4-cyano-5-(4-hydroxy-2,2-diméthylbutyl)pyrrolidine-2-carboxylique,
le rac-(2R,3S,4R,5S)-3-(3-chloro-2-fluorophényl)-4-(4-chloro-2-fluorophényl)-4-cyano-5-(2,2-diméthylpropyl)pyrrolidine-2-carboxamide,
le rac-6-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}nicotinate de méthyle,
le rac-6-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}-N-(2-hydroxy-1,1-diméthyléthyl)nicotinamide,
le (6-acétylaminopyridin-3-yl)amide de l'acide rac-(2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le (1-méthyl-2-oxo-1,2-dihydropyridin-4-y1)amide de l'acide rac-(2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le (1-méthyl-2-oxo-1,2-dihydropyridin-4-yl)amide de l'acide (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le [5-((S)-1,2-dihydroxyéthyl)pyrazin-2-yl]amide de l'acide rac-(2R,3S,4R,5S)-3-(3-chloro-2-fluorophényl)-4-(4-chloro-2-fluorophényl)-4-cyano-5-(2,2-diméthylpropyl)pyrrolidine-2-carboxylique,
le (1-méthyl-2-oxo-1,2-dihydropyridin-4-yl)amide de l'acide rac-(2R,3S,4R,5S)-3-(3-chloro-2-fluorophényl)-4-(4-chloro-2-fluorophényl)-4-cyano-5-(4-hydroxy-2,2-diméthylbutyl)pyrrolidine-2-carboxylique,
le rac-5-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}furane-2-carboxylate de méthyle,
l'acide rac-5-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}furane-2-carboxylique,
le rac-5-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}furane-2-carboxamide,
le (6-chloropyridazin-3-yl)amide de l'acide rac-(2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le (2-méthylpyridin-3-yl)amide de l'acide rac-(2R,3S,4R,5S)-3-(3-chloro-2-fluorophényl)-4-(4-chloro-2-fluorophényl)-4-cyano-5-(2,2-diméthylpropyl)-pyrrolidine-2- carboxylique,
le [4-(2-hydroxyéthoxy)phényl]amide de l'acide rac-(2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le [4-(2-hydroxyéthoxy)phényl]amide de l'acide (2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le rac-5-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}thiophéne-2-carboxylate de méthyle,
l'acide rac-5-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}-thiophène-2-carboxylique,
l'acide 5-{[(2R,35,4R,5S)-3-(3-chloro-2-fluorophényl)-4-(4-chloro-2-fluorophényl)-4-cyano-5-(2,2-diméthylpropyl)pyrrolidine-2-carbonyl]amino}thiophène-2-carboxylique,
le (2-méthoxypyridin-4-yl)amide de l'acide rac-(2R,3S,4R,5S)-3-(3-chloro-2-fluorophényl)-4-(4-ehloro-2-fluorophényl)-4-cyano-5-(2,2-diméthylpropyl)-pyrrolidine-2-carboxylique,
le (2-hydroxypyridin-4-yl)amide de l'acide rac-(2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le (4-acétylphényl)amide de l'acide rac-(2R,3S,4R,SS)-3-(3-chloro-2-fluorophényl)-4-(4-chloro-2-fluorophényl)-4-cyano-5-(2,2-diméthylpropyl)pyrrolidine-2-carboxylique,
le [4-(2-bromoacétyl)phényl]amide de l'acide rac-(2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le [4-(2-diméthylaminoacétyl)phényl]amide de l'acide rac-(2R,3S,4R,5S)-3-(3-chloro-2-fluorophényl)-4-(4-chloro-2-fluorophényl)-4-cyano-5-(2,2-diméthylpropyl)pyrrolidine-2-carboxylique,
l'acide rac-(5-{[(2R,35,4R,5S)-3-(3-chloro-2-fluorophényl)-4-(4-chloro-2-fluorophényl)-4-cyano-5-(2,2-diméthylpropyl)pyrrolidine-2-carbonyl]amino}-4H-[1,2,4]triazol-3-yl)acétique,
l'acide rac-(3-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}-pyrazol-1-yl)acétique,
le (1H-imidazol-4-ylméthyl)amide de l'acide rac-(2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le rac-(2S,3R,4S,5R)-4-(3-chloro-2-fluorophényl)-3-(4-chloro-2-fluorophényl)-2-(2,2-diméthylpropyl)-5-(2-oxa-6-azaspiro[3.3]heptane-6-carbonyl)pyrrolidine-3-carbonitrile,
l'acide rac-1-[(2R,35,4R,5S)-3-(3-chloro-2-fluorophényl)-4-(4-chloro-2-fluorophényl)-4-cyano-5-(2,2-diméthylpropyl)pyrrolidine-2-carbonyl]azétidine-3-carboxylique,
le (2-[1,2,3]triazol-1-yléthyl)amide de l'acide rac-(2R,3S,4R,5S)-3-(3-chloro-2-fluorophényl)-4-(4-chloro-2-fluorophényl)-4-cyano-5-(2,2-diméthylpropyl)-pyrrol idine-2-carboxylique,
le (1-carbamoylméthyl-1H-pyrazol-3-yl)amide de l'acide rac-(2R,3S,4R,SS)-3-(3-chloro-2-fluorophényl)-4-(4-chloro-2-fluorophényl)-4-cyano-5-(2,2-diméthylpropyl)pyrrolidine-2-carboxylique,
le [1-(2-hydroxy-2-méthylpropyl)-1H-pyrazol-3-yl]amide de l'acide rac-(2R,3R,4R,5S)-3-(3-chlorophényl)-4-(4-chlorophényl)-4-cyano-5-(2,2-diméthylpropyl)pymolidine-2-carboxylique,
le (3-méthanesulfonylaminopropyl)amide de l'acide rac-(2R,3R,4R,5S)-3-(3-chlorophényl)-4-(4-chlorophényl)-4-cyano-5-(2,2-diméthylpropyl)pyrrolidine-2-carboxylique,
le {1-[2-((S)-3-diméthylamino-2-hydroxypropoxy)-2-méthylpropyl]-1H-pyrazol-3-yl}amide de l'acide (2R,3S,4R,5S)-3-(3-chloro-2-fluorophényl)-4-(4-chloro-2-fluorophényl)-4-cyano-5-(2,2-diméthylpropyl)pyrrolidine-2-carboxylique chiral,
le {1-[2-((S)-3-diméthylamino-2-hydroxypropoxy)-2-méthylpropyl]-1H-pyrazol-3-yl}amide de l'acide (2S,3R,4S,5R)-3-(3-chloro-2-fluorophényl)-4-(4-chloro-2-fluorophényl)-4-cyano-5-(2,2-diméthylpropyl)pyrrolidine-2-carboxylique chiral,
l'acide rac-1-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}-cyclopropanecarboxylique,
le [1-(4-hydroxypipéridin-4-ylméthyl)-1H-pyrazol-3-yl]amide de l'acide rac-(2R,3S,4R,5S)-3-(3-chloro-2-fluorophényl)-4-(4-chloro-2-fluorophényl)-4-cyano-5-(2,2-diméthylpropyl)pyrrolidine-2-carboxylique,
le (2-acétylthiophén-3-yl)amide de l'acide rac-(2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le (2-carbamoylthiophén-3-yl)amide de l'acide rac-(2R,3S,4R,5S)-3-(3-chloro-2-fluorophényl)-4-(4-chloro-2-fluorophényl)-4-cyano-5-(2,2-diméthylpropyl)-pyrrolidine-2-carboxylique,
le [1-((S)-3-diméthylamino-2-hydroxypropyl)-1H-pyrazol-3-yl]amide de l'acide rac-(2R,3S,4R,5S)-3-(3-chloro-2-fluorophényl)-4-(4-chloro-2-fluorophényl)-4-cyano-5-(2,2-diméthylpropyl)pyrrolidine-2-carboxylique,
l'acide rac-4-{[(2R,35,4R,5S)-3-(3-chloro-2-fluorophényl)-4-(4-chloro-2-fluorophenyl)-4-cyano-5-(1-méthylcyclohexylméthyl)pyrrolidine-2-carbonyl]amino}-benzoïque,
l'acide rac-[4-(3-{[(2R,3S,4R,5S)-3-(3-chloro-2-fluorophényl)-4-(4-chloro-2-fluorophényl)-4-cyano-5-(2,2-diméthylpropyl)pyrrolidine-2-carbonyl]amino}-pyrazol-1-ylméthyl)-4-hydroxypipéridin-1-yl]acétique,
l'acide rac-4-{[(2R,3S,4R,5S)-3-(3-chloro-2-fluorophényl)-4-(4-chloro-2-fluorophényl)-4-cyano-5-(1-méthylcyclohexylméthyl)pyrrolidine-2-carbonyl]amino}benzoïque,
le rac-4-{[(2R,3S,4R,5S)-3-(5-chloro-2-fluorophényl)-4-(4-chloro-2-fluorophényl)-4-cyano-5-(2,2-diméthylpropyl)pyrrolidine-2-carbonyl]amino}benzoate de méthyle,
l'acide rac-4-{[(2R,3S,4R,5S)-3-(5-chloro-2-fluorophényl)-4-(4-chloro-2-fluorophényl)-4-cyano-5-(2,2-diméthylpropyl)pyrrolidine-2-carbonyl]amino}-benzoïque,
le rac-4-{[(2R,3R,4R,5S)-3-(3-chloro-4-fluorophényl)-4-(4-chloro-2-fluorophényl)-4-cyano-5-(2,2-diméthylpropyl)pyrrolidine-2-carbonyl]amino}benzoate de méthyle,
l'acide rac-4-{[(2R,3R,4R,5S)-3-(3-chloro-4-fluorophényl)-4-(4-chloro-2-fluorophényl)-4-cyano-5-(2,2-diméthylpropyl)pyrrolidine-2-carbonyl]amino}-benzoïque,
le rac-4-{[(2R,3R,4R,5S)-3-(3-bromophényl)-4-(4-chloro-2-fluorophényl)-4-cyano-5-(2,2-diméthylpropyl)pyrrolidine-2-carbonyl]amino}benzoate de méthyle,
l'acide rac-4-{[(2R,3R,4R,5S)-3-(3-bromophényl)-4-(4-chloro-2-fluorophényl)-4-cyano-5-(2,2-diméthylpropyl)pyrrolidine-2-carbonyl]amino}benzoïque,
le rac-4-{[(2R,3S,4R,5S)-3-(3-chloro-2-fluorophényl)-4-(4-chlorophényl)-4-cyano-5-(2,2-diméthylpropyl)pyrrolidine-2-carbonyl]amino}benzoate de méthyle,
l'acide rac-4-{[(2R,3S,4R,5S)-3-(3-chloro-2-fluorophényl)-4-(4-chlorophényl)-4-cyano-5-(2,2-diméthylpropyl)pyrrolidine-2-carbonyl]amino}benzoïque, et
le rac-4-{[(2R,3S,4R,5S)-3-(3-chloro-2-fluorophényl)-4-(4-chloro-2-fluorophényl)-4-cyano-5-(1-méthylcyclohexylméthyl)pyrrolidine-2-carbonyl]amino}benzoate de méthyle.

8. Composition pharmaceutique comprenant un composé selon les revendications 1 à 7 avec des excipients ou des supports pharmaceutiquement acceptables.

9. Composé selon l'une quelconque des revendications 1 à 7, à utiliser comme médicament.

10. Composé selon l'une quelconque des revendications 1 à 7, à utiliser comme médicament destiné au traitement du cancer.

11. Composé selon l'une quelconque des revendications 1 à 7, à utiliser comme médicament destiné au traitement de tumeurs solides.

12. Composé selon l'une quelconque des revendications 1 à 7, à utiliser comme médicament destiné au traitement de tumeurs du sein, du côlon, du poumon et de la prostate.
